# EUROPÄISCHE PATENTANMELDUNG

(11) **EP 2 025 675 A1**
(43) Veröffentlichungstag der Anmeldung: **18.02.2009**
(21) Anmeldenummer: 08102047.1
(22) Anmeldetag: 26.02.2008
(51) Int. Cl.: C07D 401/02, C07D 311/16, C07D 205/04, C07D 207/06, C07D 223/04, C07D 403/04, C07D 403/06

(54) **Arylsulfonamide mit analgetischer Wirkung**

(30) Priorität: 14.08.2007 WO PCT/EP2007/058408
(71) Anmelder: Boehringer Ingelheim International GmbH, 55218 Ingelheim am Rhein (DE)
(72) Erfinder: Die Erfindernennung liegt noch nicht vor
(74) Vertreter: Hammann, Heinz

(57) **Zusammenfassung**

Gegenstand der vorliegenden Erfindung Verbindungen der allgemeinen Formel **I** 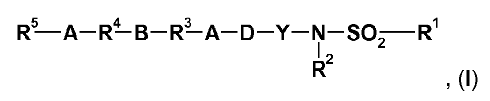 in der **A, B, D, Y**, **R**¹, **R**², **R**³, **R**⁴ und **R**⁵ wie in Anspruch 1 definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

## Beschreibung

Gegenstand der vorliegenden Erfindung sind Verbindungen der allgemeinen Formel **I** 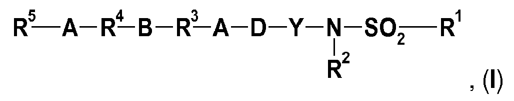 in der **A**, **B**, **D**, **Y**, **R¹**, **R²**, **R³**, **R⁴** und **R⁵** wie in Anspruch 1 definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

### DETAILLIERTE BESCHREIBUNG DER ERFINDUNG

In der obigen allgemeinen Formel **I** bedeuten in einer ersten Ausführungsform
- **A**: eine Bindung, C₁₋₄-Alkylen oder -CH₂-C(O)-,
- **B**: eine Bindung, C₁₋₃-Alkylen, -O- oder-C(O)-,
- **D**: eine Gruppe der allgemeinen Formeln **II** 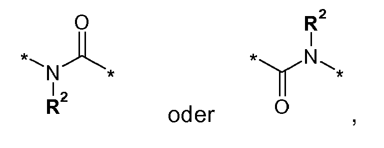
- **Y**: eine gegebenenfalls durch den Rest **R²** substituierte C₁₋₆-Alkylen-Gruppe, wobei eine Methylengruppe zusätzlich durch **Y¹** ersetzt sein kann und
**Y¹** -O-, -S-, -S(O)-, -N(**R²**)-, -N(**R²**)-C(O)-, -C(O)-N(**R²**)-, -C(O)-, -CH(Aryl)-, C₃₋₆-Cycloalkylen oder -S(O)₂- darstellt,
- **R¹**: C₃₋₇-Cycloalkyl- oder Aryl-, Heteroaryl- oder Aryl-C₁₋₃-alkyl-, die jeweils durch einen, zwei, drei oder vier Reste **R^{1.1}** substituiert sein können, wobei die Reste **R^{1.1}** gleich oder verschieden sein können und
**R^{1.1}** H, F, Cl, Br, I, C₁₋₃-Alkyl-, F₃C-, HO-, C₁₋₃-Alkyl-O- oder C₁₋₃-Alkyl-O-C₂₋₄-alkylen-O- darstellt,
- **R²**: H oder C₁₋₃-Alkyl-, wobei jede Methylengruppe mit bis zu zwei und jede Methylgruppe mit bis zu drei Fluoratomen substituiert sein kann, oder auch H₃C-C(O)-,
- **R³**: a) C₁₋₆-Alkylen,
b) eine durch **R^{3.1}** einfach, zweifach oder dreifach substituierte C₃₋₆-Cycloalkylengruppe,
c) eine durch **R^{3.1}** einfach oder zweifach substituierte C₅₋₇-Cycloalkenylengruppe, die über die ungesättigte Bindung mit einem Phenylring kondensiert ist,
d) -N(**R²**)-,
e) eine durch **R^{3.1}** einfach oder zweifach substituierte Arylengruppe,
f) eine durch **R^{3.1}** einfach oder zweifach substituierte Heteroarylengruppe,
g) einen durch **R^{3.1}** einfach oder zweifach substituierten gesättigten 4- bis 7-gliedrigen heterocyclischen Ring,
h) einen durch **R^{3.1}** einfach oder zweifach substituierten ungesättigten 5- bis 7-gliedrigen heterocyclischen Ring, der über die ungesättigten Bindungen mit einem oder zwei Phenylringen kondensiert ist, oder
i) einen durch **R^{3.1}** einfach oder zweifach substituierten gesättigten 8- bis 10-gliedrigen Aza-Heterobicyclus,
wobei die Reste **R^{3.1}** jeweils gleich oder verscheiden sein können und
**R^{3.1}** H, F, Cl, Br, I, C₁₋₃-Alkyl-, HO-, C₁₋₃-Alkyl-O- oder C₁₋₃-Alkyl-O-C₂₋₄-alkylen-O- darstellt, oder
- **R³**: auch -O-, wenn **B** nicht die Gruppe -O- darstellt,
- **R⁴**: a) -O-,
b) -C(O)O-,
c) -C(O)N**R²**-,
d) -N**R²**-,
e) -N**R²**-N**R²**-,
f) C₃₋₇-Cycloalkylen,
g) C₁₋₆-Alkylen,
h) eine durch **R^{4.1}** einfach oder zweifach substituierte Arylengruppe,
i) eine durch **R^{4.1}** einfach oder zweifach substituierte Heteroarylengruppe,
j) einen durch **R^{4.1}** einfach oder zweifach substituierten 4- bis 7-gliedrigen
gesättigten heterocyclischen Ring,
k) einen durch **R^{4.1}** einfach oder zweifach substituierten gesättigten 8- bis 10-gliedrigen Diaza-Heterobicyclus,
I) einen durch **R^{4.1}** einfach oder zweifach substituierten 5- bis 7-gliedrigen ungesättigten heterocyclischen Ring, der über die ungesättigten Bindungen mit einem oder zwei Phenylringen kondensiert ist, oder
m) einen gesättigten 9- bis 11-gliedrigen Diaza-Spirocyclus,
wobei die Reste **R^{4.1}** jeweils gleich oder verscheiden sein können und
**R^{4.1}** H, F, Cl, Br, I, C₁₋₃-Alkyl-, HO-, C₁-₃-Alkyl-O- oder C₁₋₃-Alkyl-O-C₂₋₄-alkylen-O- darstellt,
- **R⁵**: H, HO-, C₁₋₈-Alkyl-, eine gegebenenfalls durch C₁₋₃-Alkyl- substituierte C₃₋₇-Cycloalkylgruppe, H₂N-, C₁₋₄-Alkyl-NH-, (C₃₋₆-Cycloalkyl)-NH-, (C₁₋₄-Alkyl)₂N-, (C₁₋₄-Alkyl)(C₃₋₆-Cycloalkyl)N-, (Cyclopropylmethyl)(methyl)N-, H₂N-C(O)-, eine durch **R^{5.1}** einfach oder zweifach substituierte Phenylgruppe, eine durch **R^{5.1}** einfach oder zweifach substituierte Heteroarylgruppe oder einen durch **R^{5.1}** einfach oder zweifach substituierten 4- bis 7-gliedrigen gesättigten heterocyclischen Ring, wobei die Reste **R^{5.1}** jeweils gleich oder verscheiden sein können und
**R^{5.1}** H, F, Cl, Br, I, C₁₋₃-Alkyl-, HO-, C₁₋₃-Alkyl-O-, (C₁₋₃-Alkyl)₂N- oder C₁₋₃-Alkyl-O-C₂₋₄-alkylen-O- darstellt,
deren Enantiomere, deren Diastereomere, deren Gemische und deren Salze, insbesondere deren physiologisch verträgliche Salze mit organischen oder anorganischen Säuren oder Basen.

Eine zweite Ausführungsform der vorliegenden Erfindung besteht in den Verbindungen der obigen allgemeinen Formel **I**, in denen
- **A**: eine Bindung oder C₁₋₄-Alkylen,
- **B**: eine Bindung, C₁₋₃-Alkylen, -O- oder-C(O)-,
- **D**: eine Gruppe der allgemeinen Formeln **II** 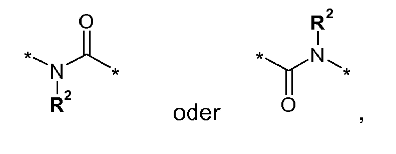 **Y** eine gegebenenfalls durch den Rest **R²** substituierte C₁₋₄-Alkylen-Gruppe, wobei eine Methylengruppe zusätzlich durch **Y¹** ersetzt sein kann und
**Y¹** -O-, -S-, -S(O)-, -N(**R²**)-, -N(**R²**)-C(O)-, -C(O)-N(**R²**)-, -C(O)-, -CH(Aryl)- oder -S(O)₂- darstellt,
- **R¹**: C₃₋₇-Cycloalkyl- oder Aryl-, Heteroaryl- oder Aryl-C₁₋₃-alkyl-, die jeweils durch einen, zwei, drei oder vier Reste **R^{1.1}** substituiert sein können, wobei die Reste **R^{1.1}** gleich oder verschieden sein können und
**R^{1.1}** H, F, Cl, Br, I, C₁₋₃-Alkyl-, HO-, C₁₋₃-Alkyl-O- oder C₁₋₃-Alkyl-O-C₂₋₄-alkylen-O- darstellt,
- **R²**: H oder C₁₋₃-Alkyl-, wobei jede Methylengruppe mit bis zu zwei und jede Methylgruppe mit bis zu drei Fluoratomen substituiert sein kann,
- **R³**: C₁₋₆-Alkylen, eine durch **R^{3.1}** einfach oder zweifach substituierte Arylengruppe, eine durch **R^{3.1}** einfach oder zweifach substituierte Heteroarylengruppe, einen durch **R^{3.1}** einfach oder zweifach substituierten gesättigten 4- bis 7-gliedrigen heterocyclischen Ring oder einen durch **R^{3.1}** einfach oder zweifach substituierten ungesättigten 5- bis 7-gliedrigen heterocyclischen Ring, wobei die Reste **R^{3.1}** jeweils gleich oder verscheiden sein können und
**R^{3.1}** H, F, Cl, Br, I, C₁₋₃-Alkyl-, HO-, C₁₋₃-Alkyl-O- oder C₁₋₃-Alkyl-O-C₂₋₄-alkylen-O- darstellt,
- **R⁴**: -O-, -C(O)O-, -C(O)N**R²**-, -N**R²**-, -N**R²**-N**R²**-, C₃₋₇-Cycloalkylen, C₁₋₆-Alkylen, eine durch **R^{4.1}** einfach oder zweifach substituierte Arylengruppe, eine durch **R^{4.1}** einfach oder zweifach substituierte Heteroarylengruppe, einen durch **R^{4.1}** einfach oder zweifach substituierten 4- bis 7-gliedrigen gesättigten heterocyclischen Ring oder einen durch **R^{4.1}** einfach oder zweifach substituierten 5- bis 7-gliedrigen ungesättigten heterocyclischen Ring, wobei die Reste **R^{4.1}** jeweils gleich oder verscheiden sein können und
**R^{4.1}** H, F, Cl, Br, I, C₁₋₃-Alkyl-, HO-, C₁₋₃-Alkyl-O- oder C₁₋₃-Alkyl-O-C₂₋₄-alkylen-O- darstellt,
- **R⁵**: H, C₁₋₈-Alkyl-, eine gegebenenfalls durch C₁₋₃-Alkyl- substituierte C₃₋₇-Cycloalkylgruppe, H₂N-, C₁₋₄-Alkyl-NH-, (C₁₋₄-Alkyl)₂N-, H₂N-C(O)-, eine durch **R^{5.1}** einfach oder zweifach substituierte Heteroarylgruppe oder einen durch **R^{5.1}** einfach oder zweifach substituierten 4- bis 7-gliedrigen gesättigten heterocyclischen Ring, wobei die Reste **R^{5.1}** jeweils gleich oder verscheiden sein können und
**R^{5.1}** H, F, Cl, Br, I, C₁₋₃-Alkyl-, HO-, C₁₋₃-Alkyl-O- oder C₁₋₃-Alkyl-O-C₂₋₄-alkylen-O- darstellt,
deren Enantiomere, deren Diastereomere, deren Gemische und deren Salze, insbesondere deren physiologisch verträgliche Salze mit organischen oder anorganischen Säuren oder Basen.

Eine dritte Ausführungsform der vorliegenden Erfindung besteht in den Verbindungen der obigen allgemeinen Formel **I**, in denen
- **A**: eine Bindung, C₁₋₄-Alkylen oder -CH₂-C(O)-,
- **B**: eine Bindung, C₁₋₂-Alkylen, -O- oder -C(O)-,
- **D**: eine Gruppe der allgemeinen Formel **II**

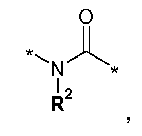

- **Y**: C₁₋₄-Alkylen oder eine Gruppe ausgewählt aus 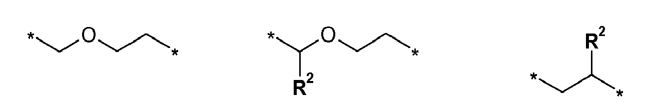 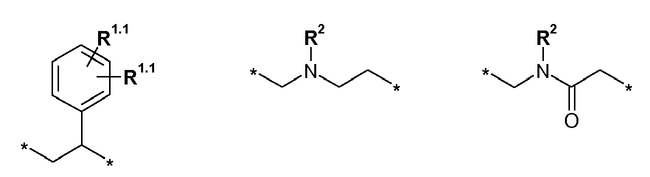 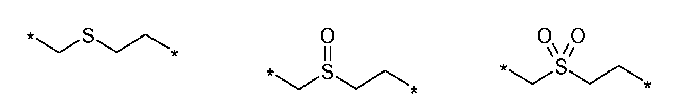 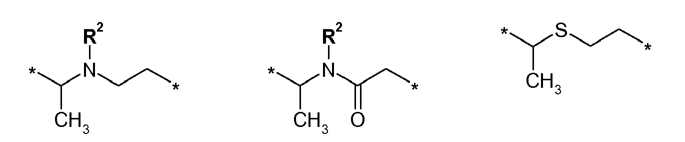 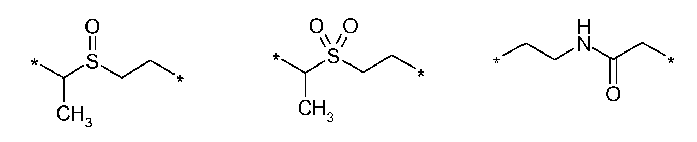 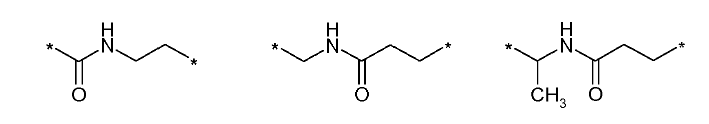 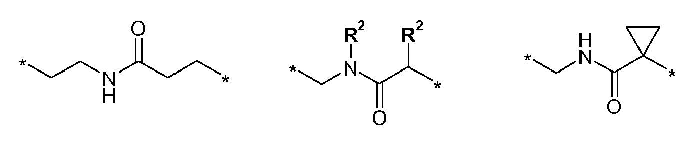 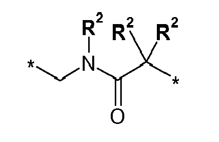
- **R¹**: Aryl- oder Heteroaryl-, die jeweils durch einen, zwei, drei oder vier Reste **R^{1.1}** substituiert sein können, wobei die Reste **R^{1.1}** gleich oder verschieden sein können und
**R^{1.1}** H, F, Cl, Br, I, C₁₋₃-Alkyl-, F₃C-, HO-, C₁₋₃-Alkyl-O- oder C₁₋₃-Alkyl-O-C₂₋₄-alkylen-O- darstellt,
- **R²**: H, H₃C-, H₅C₂-, Isopropyl, F₃C-CH₂-, F₂CH-CH₂- oder FH₂C-H₂C-,
- **R³**: C₁₋₄-Alkylen, -N(**R²**)- oder eine Gruppe ausgewählt aus 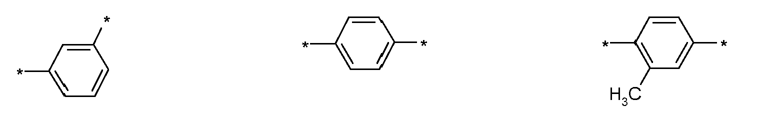 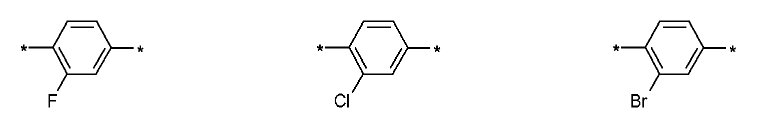 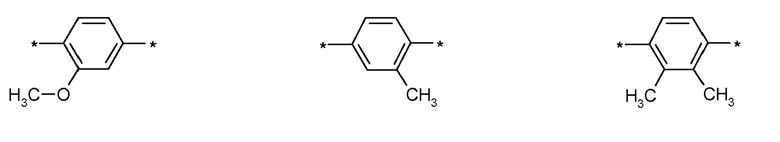 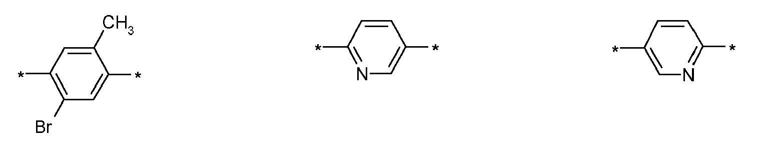 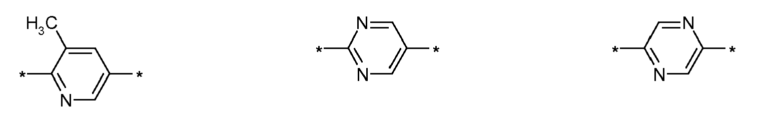 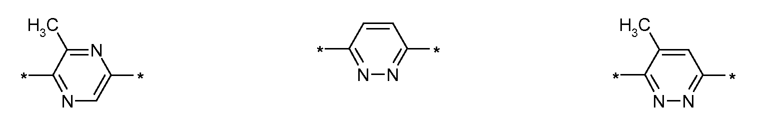 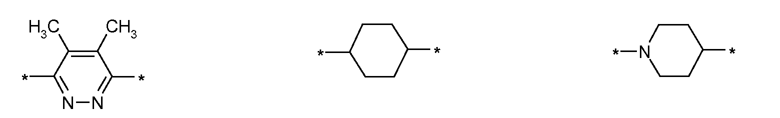 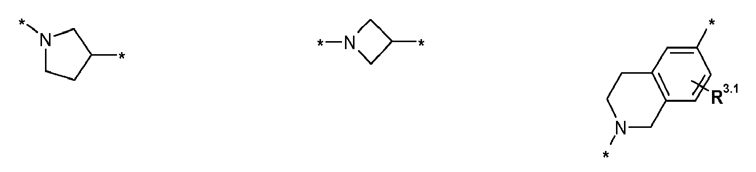 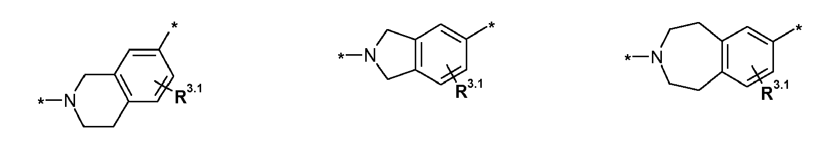 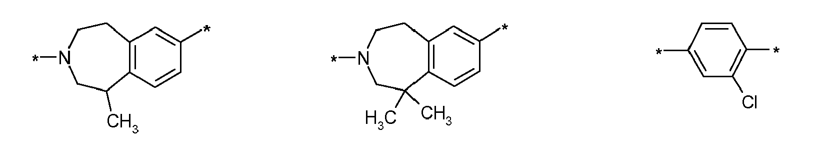 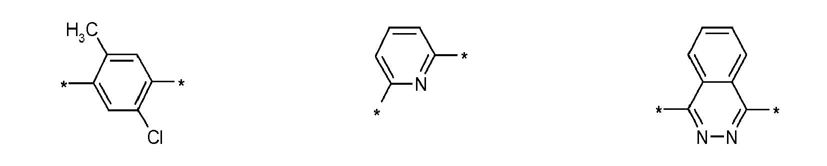 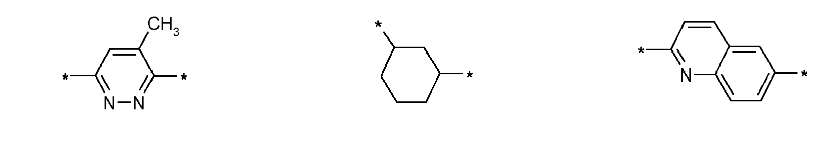 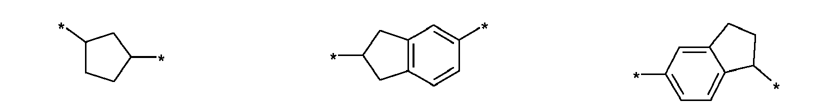 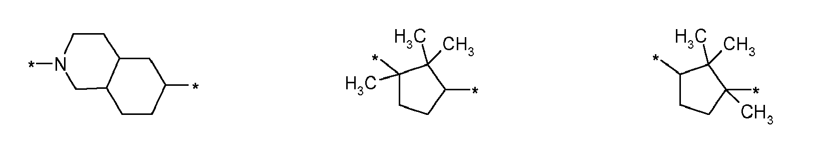 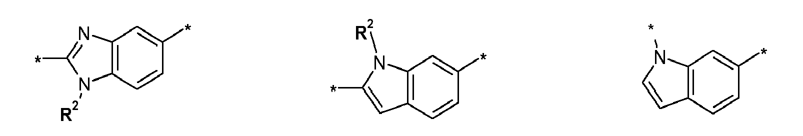 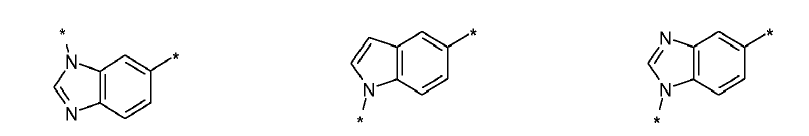 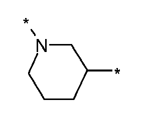 worin
**R^{3.1}** H, F, Cl, Br, I, C₁₋₃-Alkyl-, HO-, C₁₋₃-Alkyl-O- oder C₁₋₃-Alkyl-O-C₂₋₄-alkylen-O- darstellt, oder
- **R³**: auch -O-, wenn **B** nicht die Gruppe -O- darstellt,
- **R⁴**: C₁₋₄-Alkylen, C₃₋₇-Cycloalkylen, -O- oder eine Gruppe ausgewählt aus 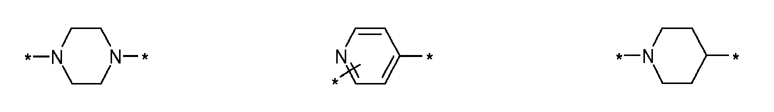 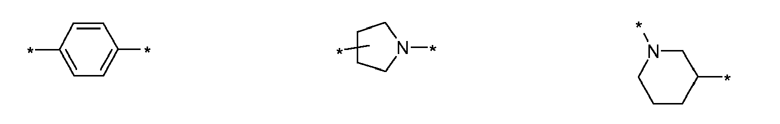 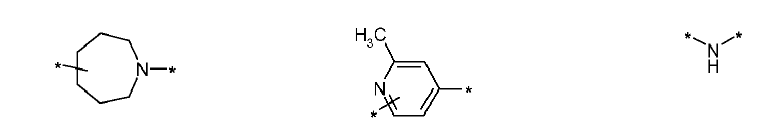 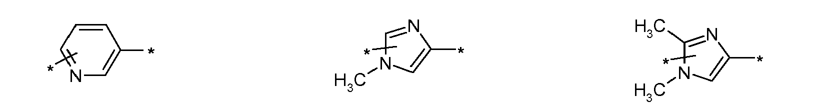 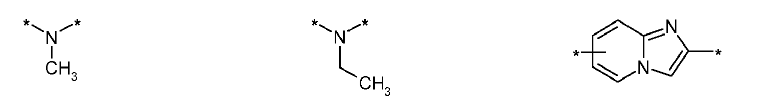 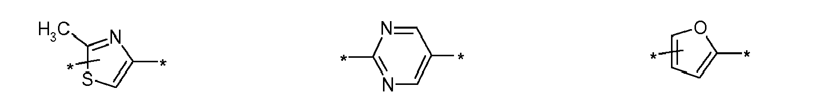 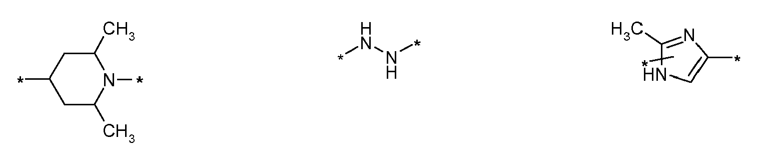 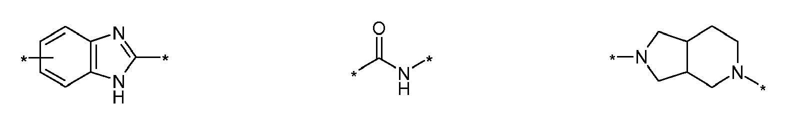 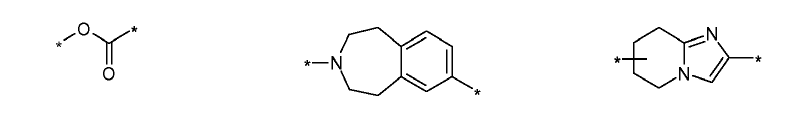 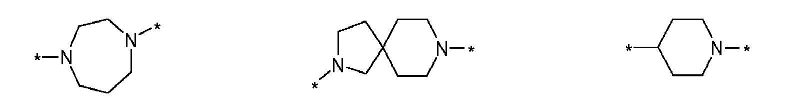 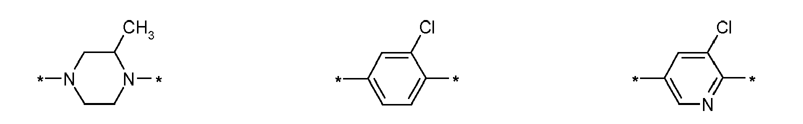 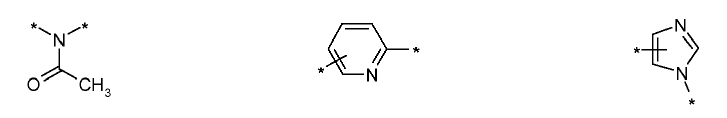 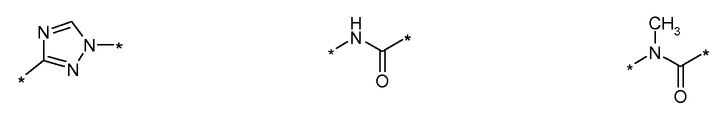 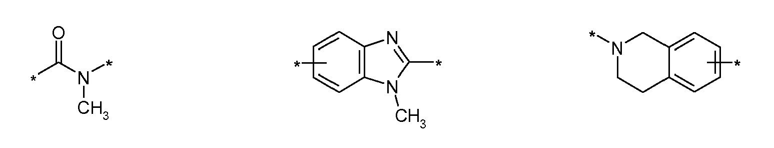
- **R⁵**: H, C₁₋₈-Alkyl, C₃₋₇-Cycloalkyl-, HO-, (C₁₋₃-Alkyl)-O-, (C₁₋₄-Alkyl)-NH-, (C₃₋₆-Cycloalkyl)-NH-, (C₁₋₄-Alkyl)₂N-, (C₁₋₄-Alkyl)(C₃₋₆-Cycloalkyl)N-, (Cyclopropylmethyl)(methyl)N-, H₂N-C(O)-, oder
- **R⁵**: eine Gruppe ausgewählt aus 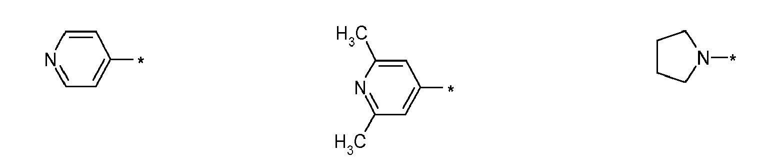 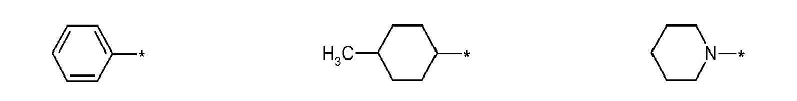 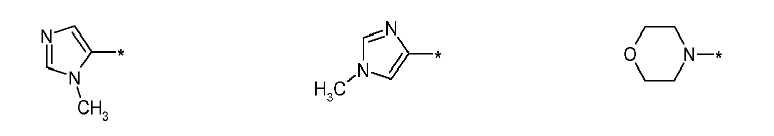 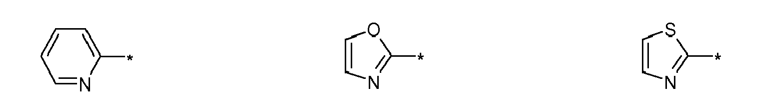 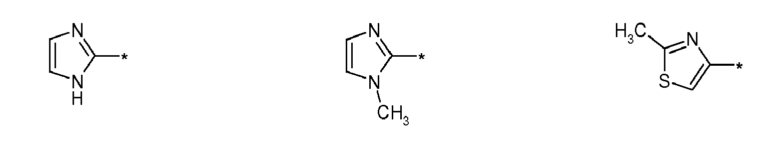 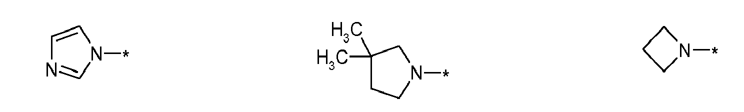 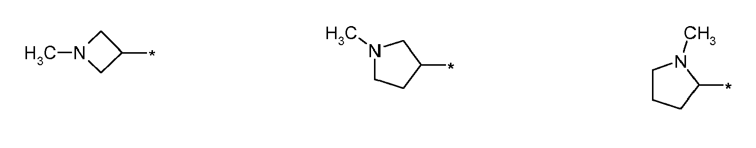 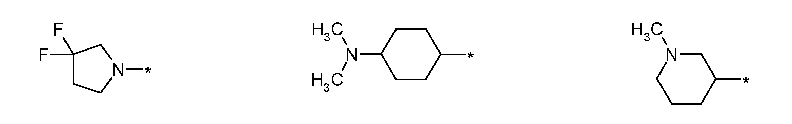 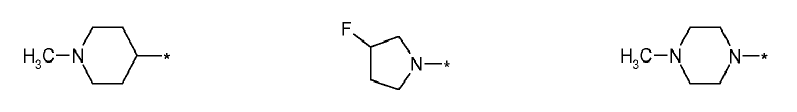 bedeutet,
deren Enantiomere, deren Diastereomere, deren Gemische und deren Salze, insbesondere deren physiologisch verträgliche Salze mit organischen oder anorganischen Säuren oder Basen.

Eine vierte Ausführungsform der vorliegenden Erfindung besteht in den Verbindungen der obigen allgemeinen Formel **I**, in denen
- **A**: eine Bindung oder C₁₋₃-Alkylen,
- **B**: eine Bindung, C₁₋₂-Alkylen, -O- oder -C(O)-,
- **D**: eine Gruppe der allgemeinen Formel **II** 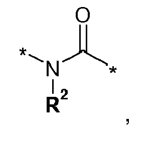 **Y** C₁₋₄-Alkylen oder eine Gruppe ausgewählt aus 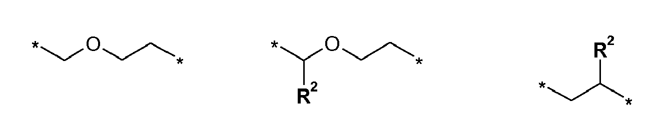 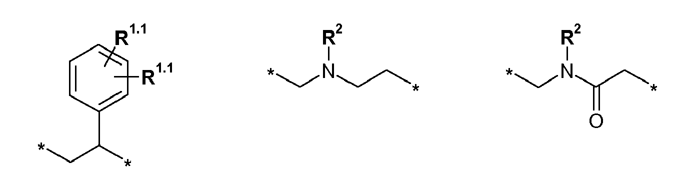 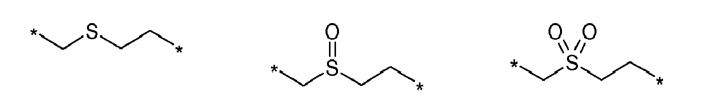 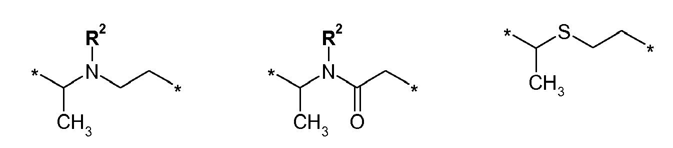 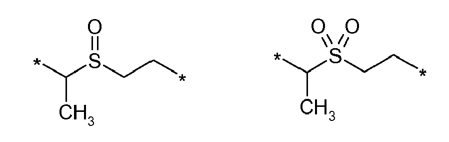
- **R¹**: Aryl- oder Heteroaryl-, die jeweils durch einen, zwei, drei oder vier Reste **R^{1.1}** substituiert sein können, wobei die Reste **R^{1.1}** gleich oder verschieden sein können und
**R^{1.1}** H, F, Cl, Br, I, C₁₋₃-Alkyl-, HO-, C₁₋₃-Alkyl-O- oder C₁₋₃-Alkyl-O-C₂₋₄-alkylen-O- darstellt,
- **R²**: H, H₃C-, H₅C₂-, Isopropyl, F₃C-CH₂-, F₂CH-CH₂- oder FH₂C-H₂C-,
- **R³**: C₁₋₄-Alkylen oder eine Gruppe ausgewählt aus 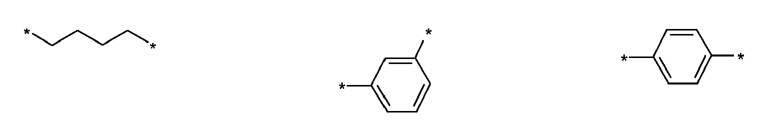 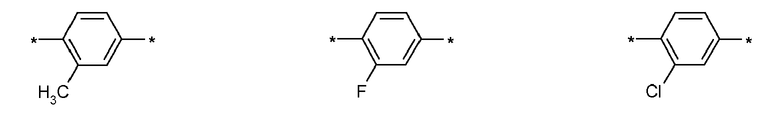 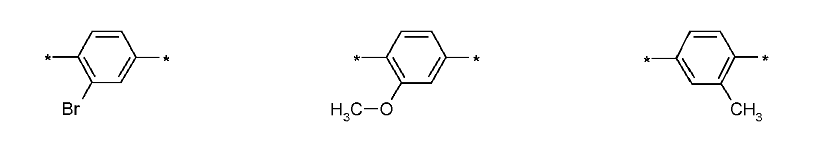 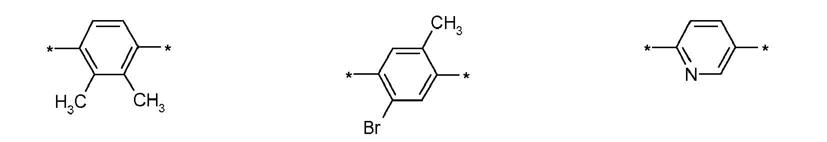 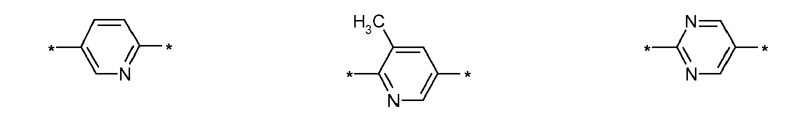 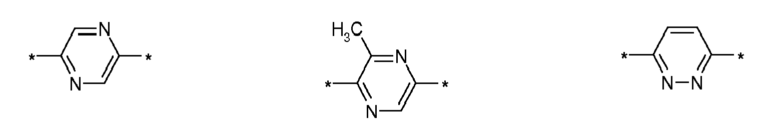 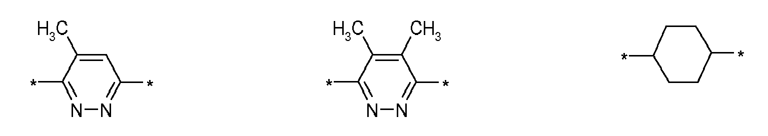 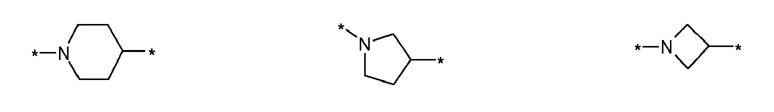 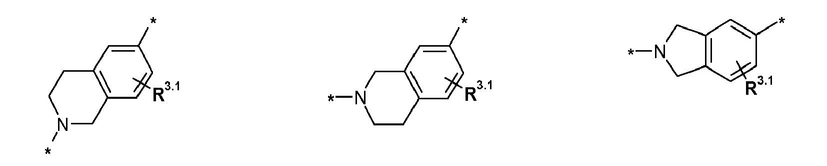 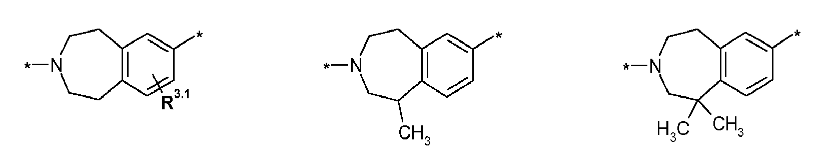 worin
**R^{3.1}** H, F, Cl, Br, I, C₁₋₃-Alkyl-, HO-, C₁₋₃-Alkyl-O- oder C₁₋₃-Alkyl-O-C₂₋₄-alkylen-O- darstellt,
- **R⁴**: C₁₋₄-Alkylen, -O- oder eine Gruppe ausgewählt aus 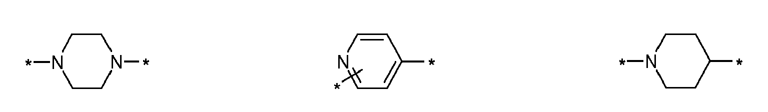 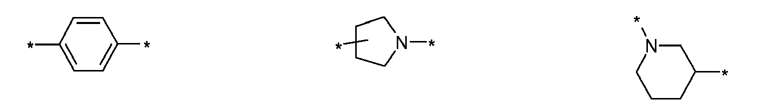 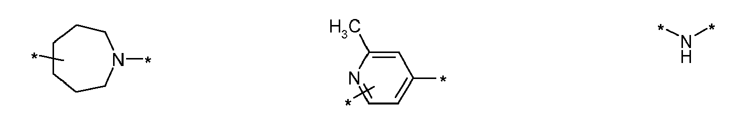 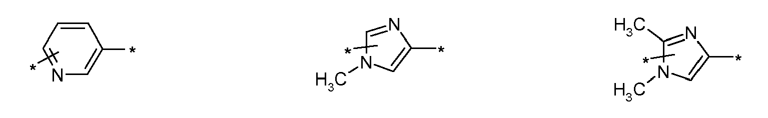 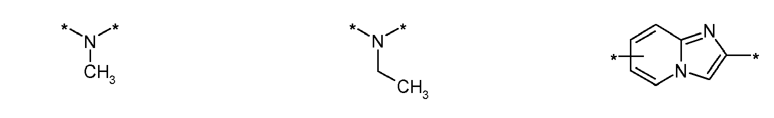 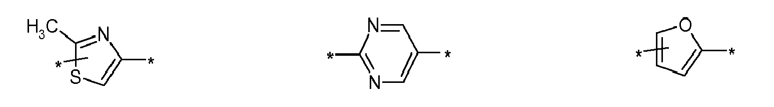 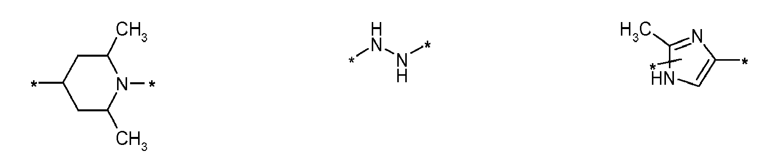 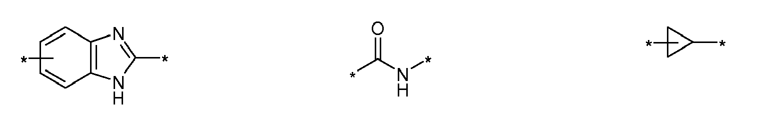 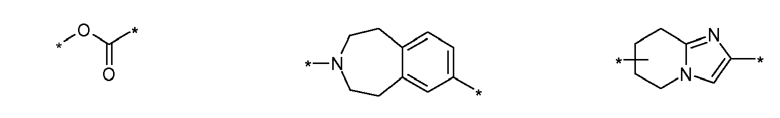
- **R⁵**: H, C₁₋₈-Alkyl, C₃₋₇-Cycloalkyl-, (C₁₋₄-Alkyl)-NH-, (C₁₋₄-Alkyl)₂N-, H₂N-C(O)-, oder
- **R⁵**: eine Gruppe ausgewählt aus 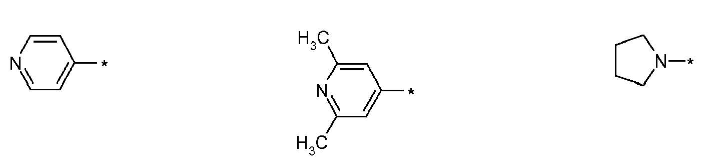 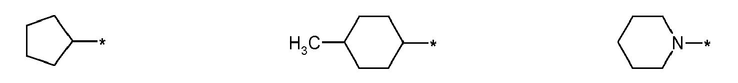 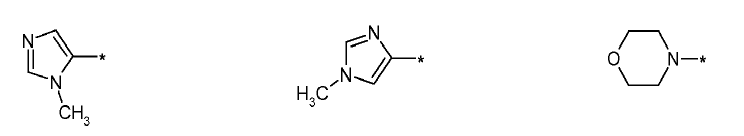 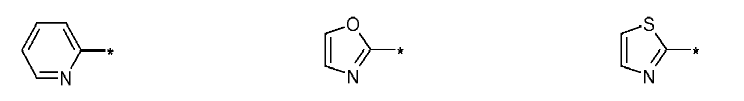 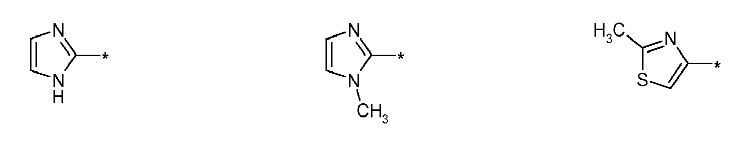
bedeutet,
deren Enantiomere, deren Diastereomere, deren Gemische und deren Salze, insbesondere deren physiologisch verträgliche Salze mit organischen oder anorganischen Säuren oder Basen.

Eine fünfte Ausführungsform der vorliegenden Erfindung besteht in den Verbindungen der obigen allgemeinen Formel **I**, in denen
- **A**: eine Bindung, C₁₋₃-Alkylen oder -CH₂-C(O)-,
- **B**: eine Bindung, C₁₋₂-Alkylen, -O- oder -C(O)-,
- **D**: eine Gruppe der allgemeinen Formel **II** 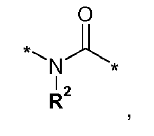
- **Y**: eine Gruppe ausgewählt aus 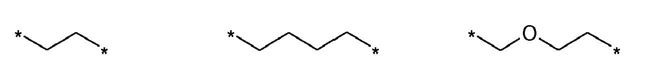 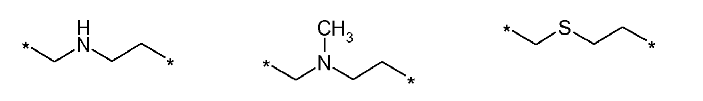 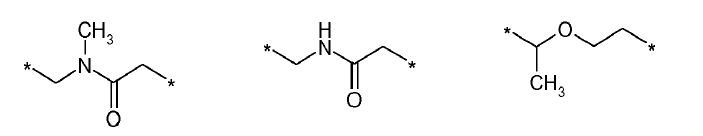 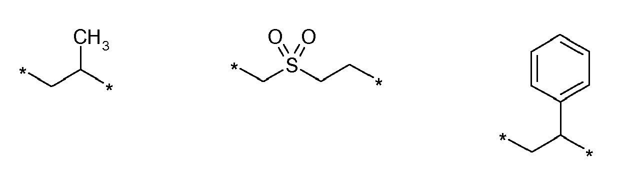 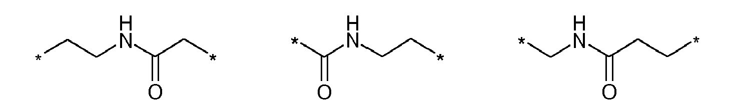 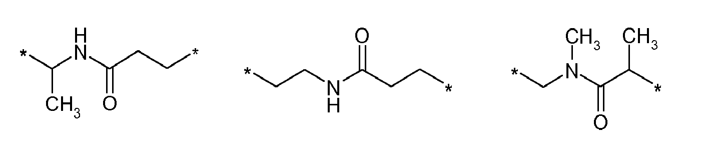 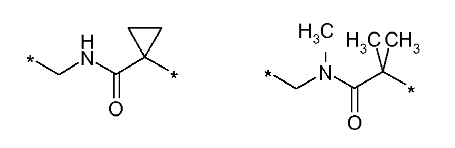 **R¹** eine Gruppe ausgewählt aus 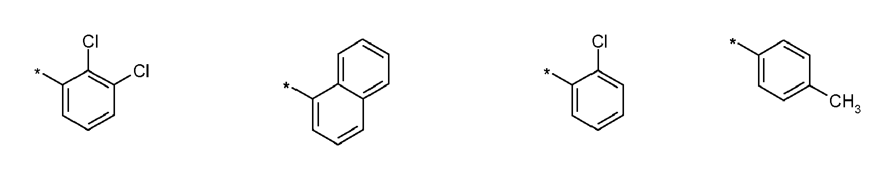 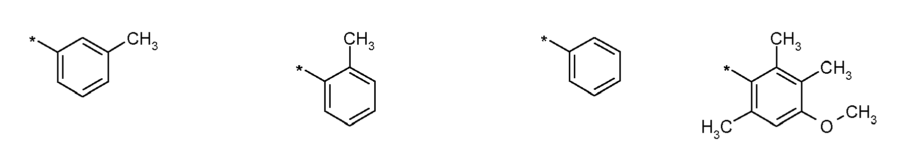 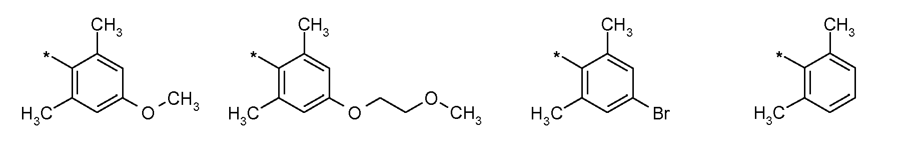 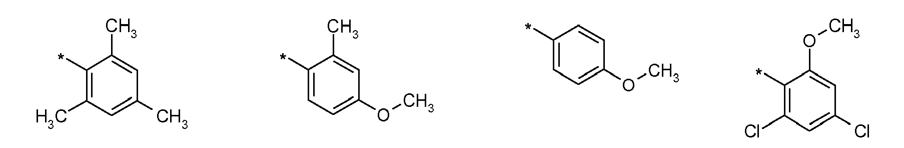 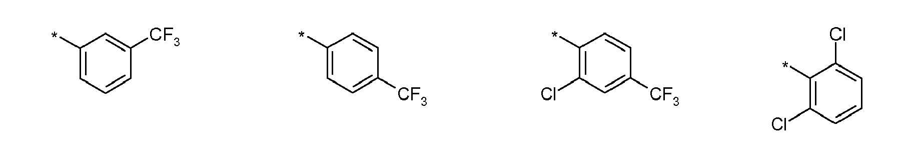
- **R²**: H, H₃C-, H₅C₂- oder FH₂C-H₂C-,
- **R³**: C₁₋₄-Alkylen, -NH-, -N(CH₃)- oder eine Gruppe ausgewählt aus 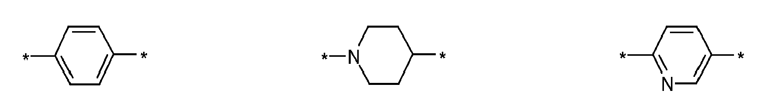 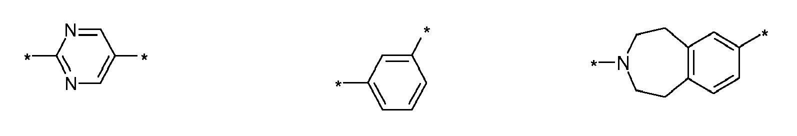 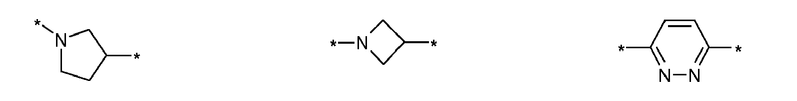 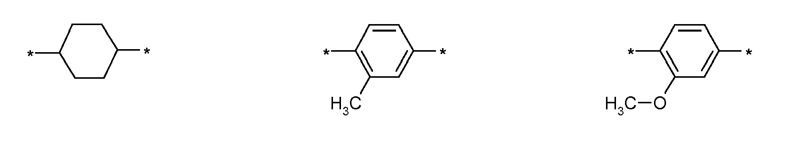 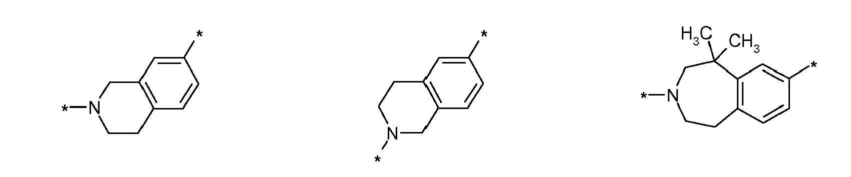 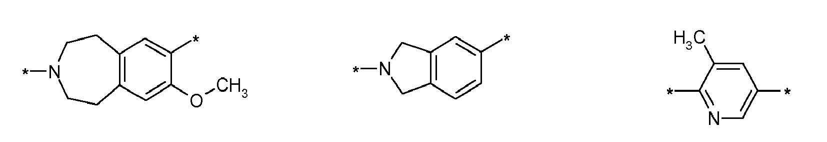 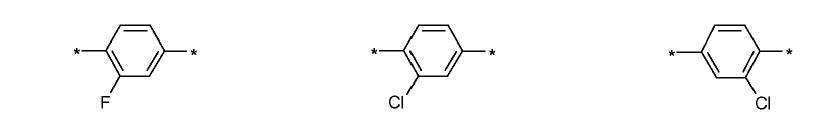 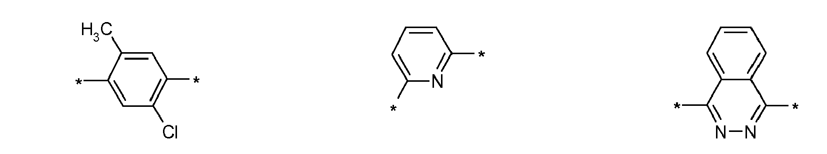 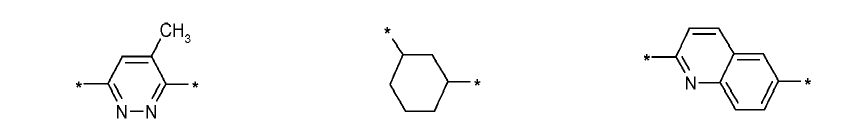 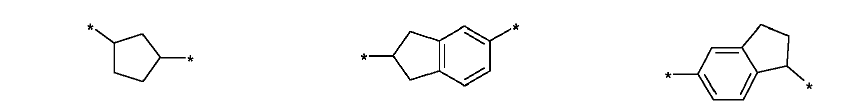 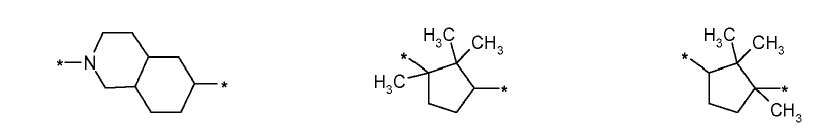 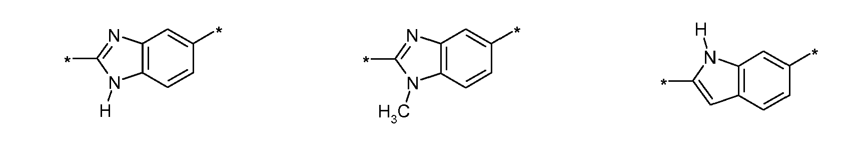 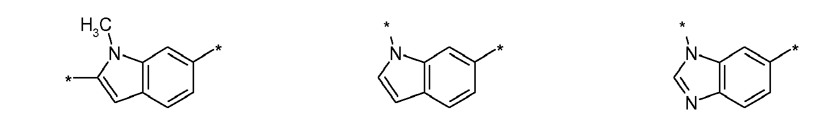 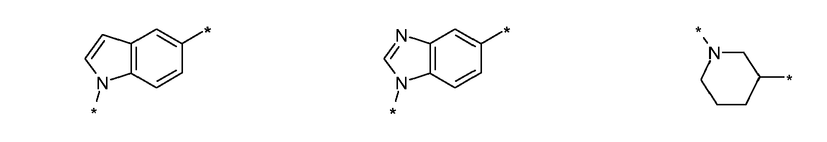 oder
- **R³**: auch -O-, wenn B nicht die Gruppe -O- darstellt,
- **R⁴**: C₁₋₄-Alkylen, C₃₋₇-Cycloalkylen, -O- oder eine Gruppe ausgewählt aus 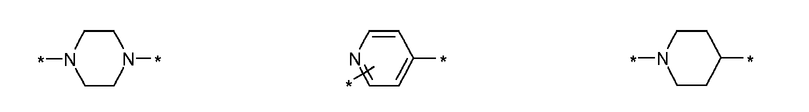 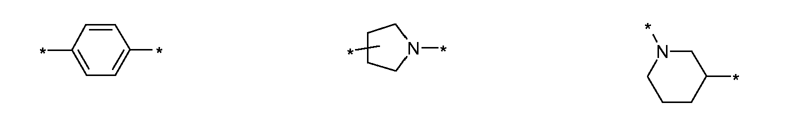 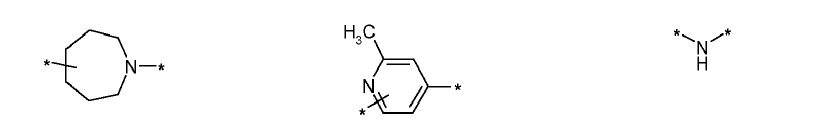 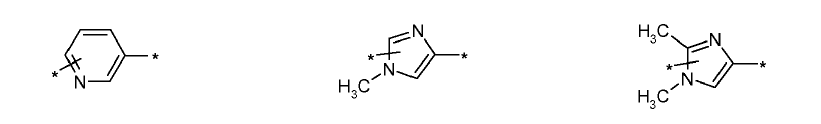 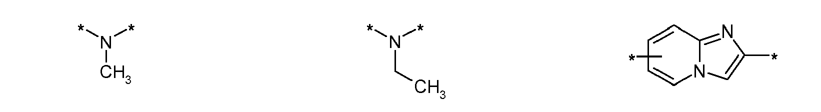 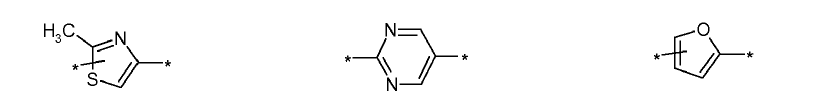 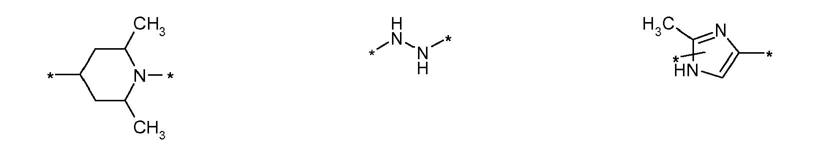 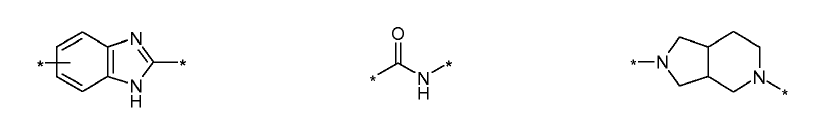 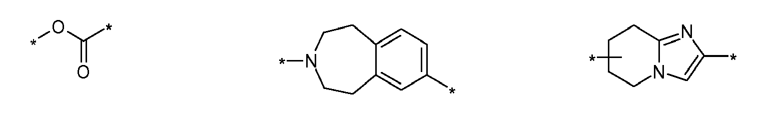 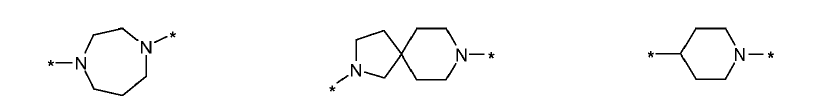 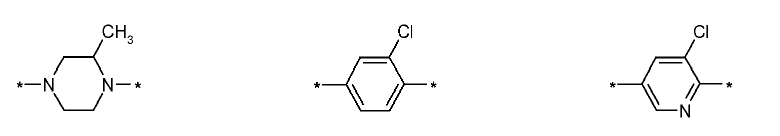 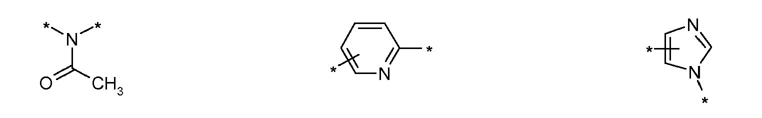 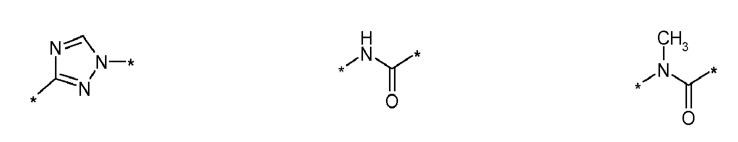 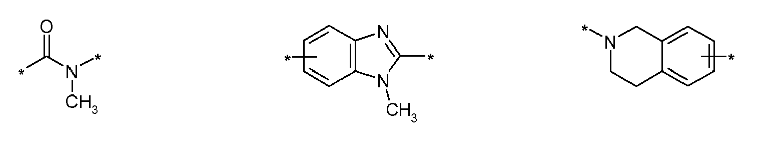
- **R⁵**: H, HO-, C₁₋₅-Alkyl, C₃₋₅-Cycloalkyl-, H₂N-, (C₁₋₂-Alkyl)-NH-, (C₃₋₆-Cycloalkyl)-NH-, (C₁₋₂-Alkyl)₂N-, (C₁₋₄-Alkyl)(C₃₋₆-Cycloalkyl)N-, (Cyclopropylmethyl)(methyl)N-, H₂N-C(O)-, oder
- **R⁵**: eine Gruppe ausgewählt aus 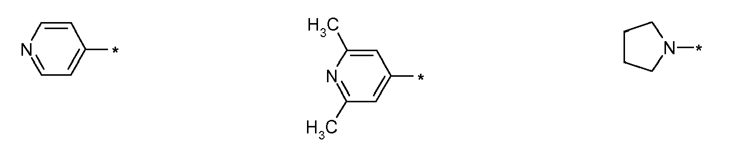 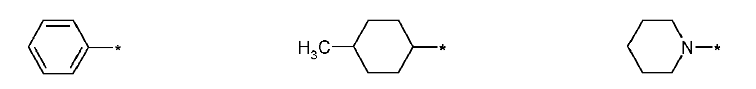 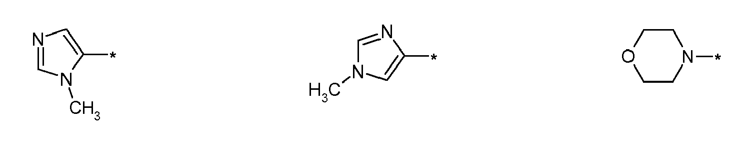 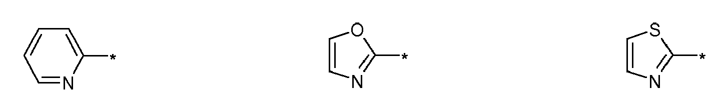 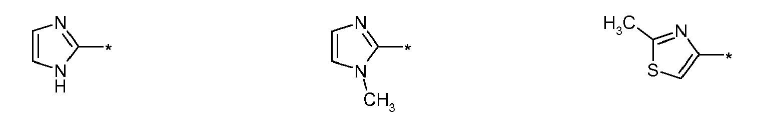 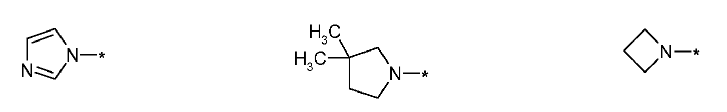 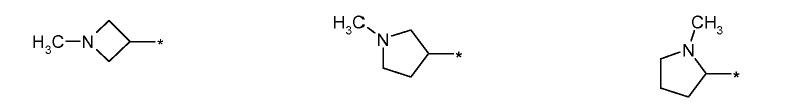 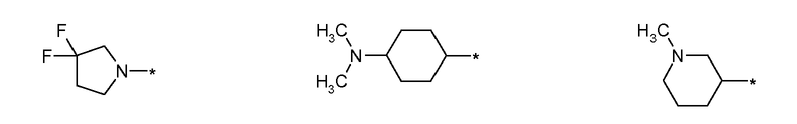 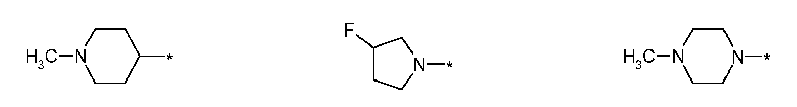
bedeutet,
deren Enantiomere, deren Diastereomere, deren Gemische und deren Salze, insbesondere deren physiologisch verträgliche Salze mit organischen oder anorganischen Säuren oder Basen.

Eine sechste Ausführungsform der vorliegenden Erfindung besteht in den Verbindungen der obigen allgemeinen Formel **I**, in denen
- **A**: eine Bindung oder C₁₋₃-Alkylen,
- **B**: eine Bindung, C₁₋₂-Alkylen, -O- oder -C(O)-,
- **D**: eine Gruppe der allgemeinen Formel **II**

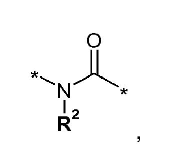

- **Y**: eine Gruppe ausgewählt aus 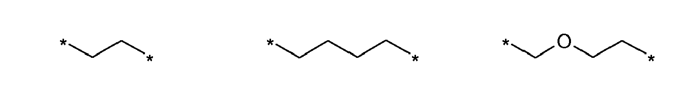 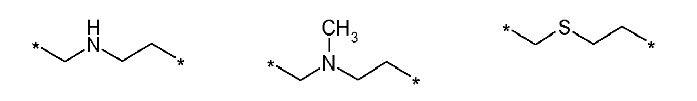 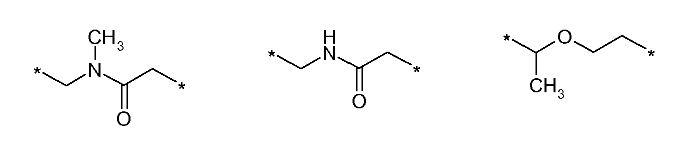 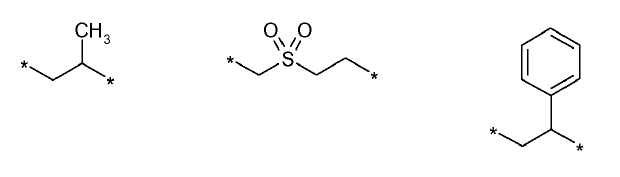
- **R¹**: eine Gruppe ausgewählt aus 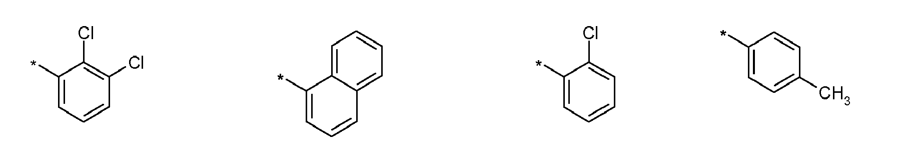 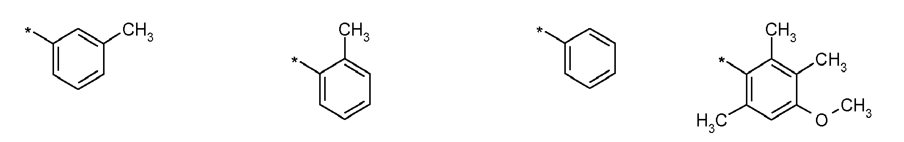 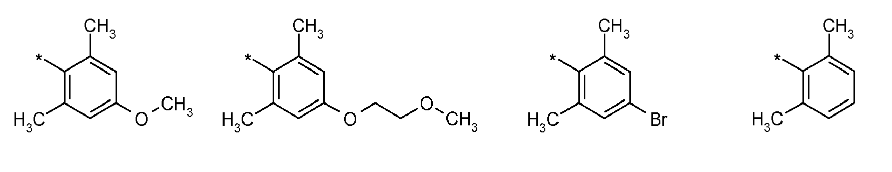 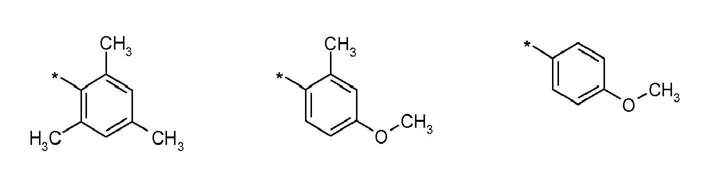
- **R²**: H, H₃C-, H₅C₂- oder FH₂C-H₂C-,
- **R³**: C₁₋₄-Alkylen oder eine Gruppe ausgewählt aus 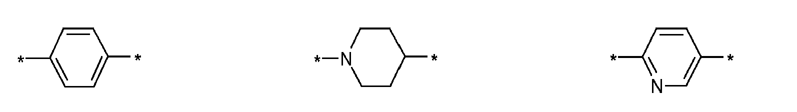 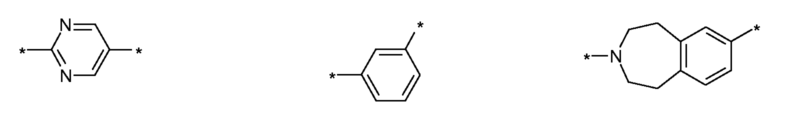 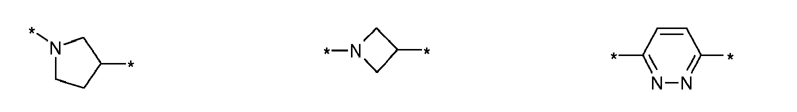 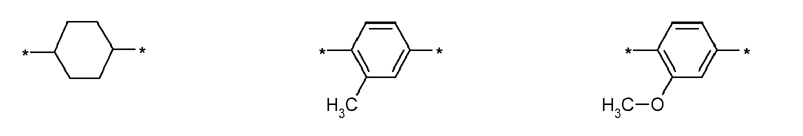 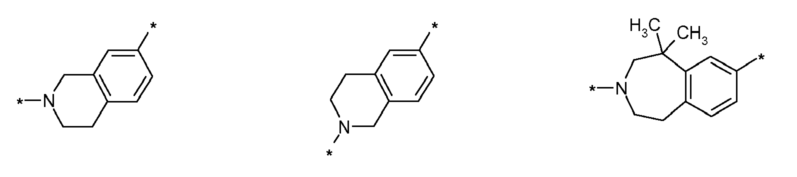 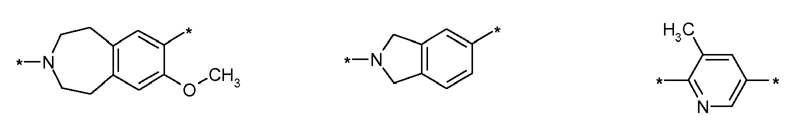 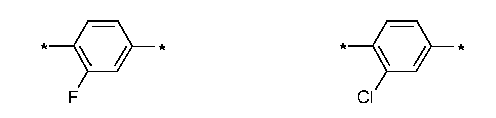
- **R⁴**: C₁₋₄-Alkylen, -O- oder eine Gruppe ausgewählt aus 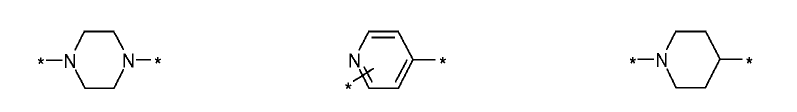 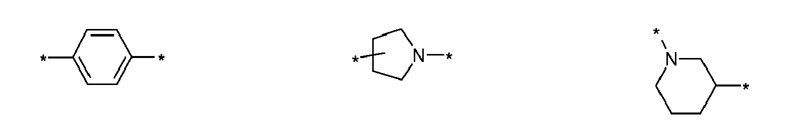 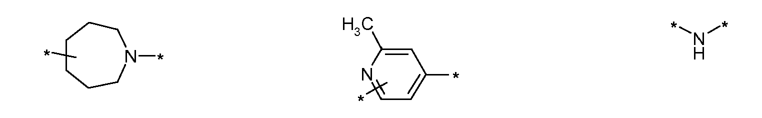 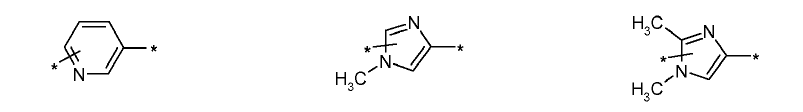 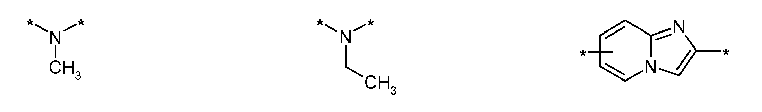 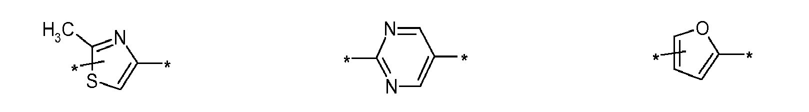 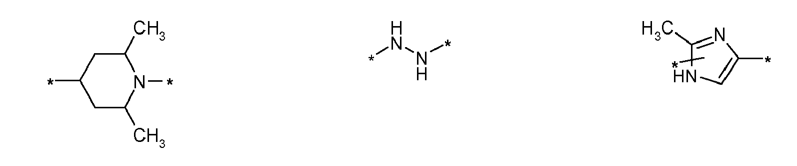 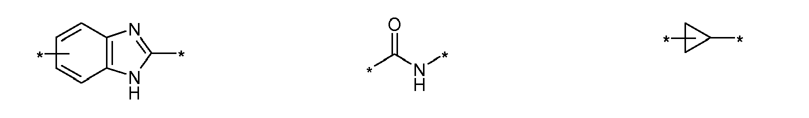 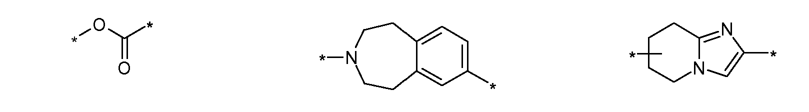
- **R⁵**: H, C₁₋₅-Alkyl, H₂N-, (C₁₋₂-Alkyl)-NH-, (C₁₋₂-Alkyl)₂N-, H₂N-C(O)-, oder
- **R⁵**: eine Gruppe ausgewählt aus 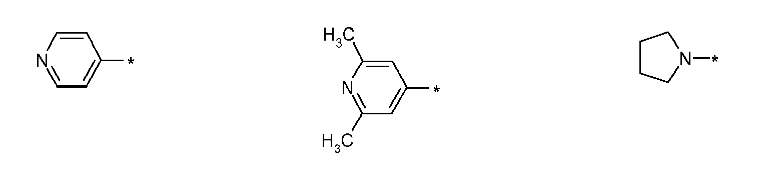 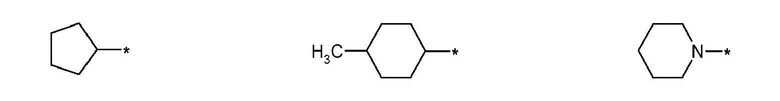 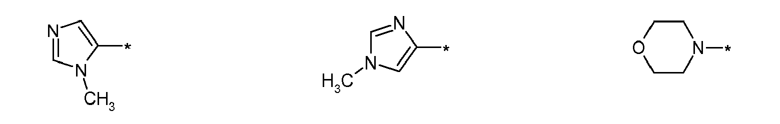 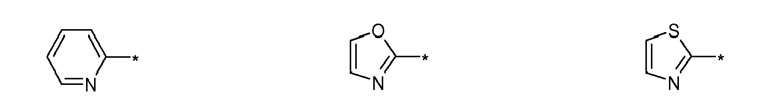 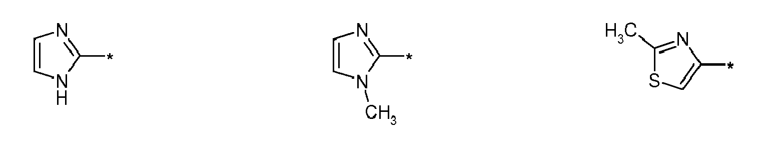
bedeutet,
deren Enantiomere, deren Diastereomere, deren Gemische und deren Salze, insbesondere deren physiologisch verträgliche Salze mit organischen oder anorganischen Säuren oder Basen.

Eine siebte Ausführungsform der vorliegenden Erfindung besteht in den Verbindungen der obigen allgemeinen Formel **I**, in denen **A**, **B**, **D**, **Y**, **R²**, **R³**, **R⁴** und **R⁵** wie voranstehend unter der ersten bis sechsten Ausführungsform definiert sind und
- **R¹**: die Gruppe 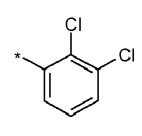 bedeutet,
deren Enantiomere, deren Diastereomere, deren Gemische und deren Salze, insbesondere deren physiologisch verträgliche Salze mit organischen oder anorganischen Säuren oder Basen.

Eine achte Ausführungsform der vorliegenden Erfindung besteht in den Verbindungen der obigen allgemeinen Formel **I**, in denen **A**, **B**, **D**, **Y**, **R²**, **R³**, **R⁴** und **R⁵** wie voranstehend unter der ersten bis sechsten Ausführungsform definiert sind und
- **R¹**: die Gruppe 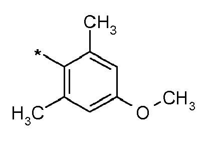 bedeutet,
deren Enantiomere, deren Diastereomere, deren Gemische und deren Salze, insbesondere deren physiologisch verträgliche Salze mit organischen oder anorganischen Säuren oder Basen.

Eine neunte Ausführungsform der vorliegenden Erfindung besteht in den Verbindungen der obigen allgemeinen Formel **I**, in denen **A**, **B**, **R¹**, **R³**, **R⁴** und **R⁵** wie voranstehend unter der ersten bis achten Ausführungsform definiert sind und
- **-D-Y-**: zusammen eine Gruppe ausgewählt aus 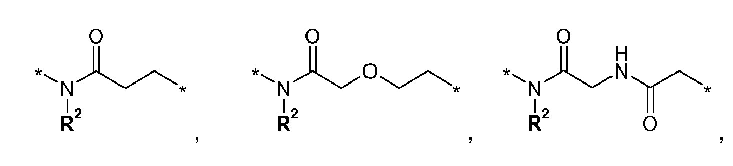 und
- **R²**: H oder C₁₋₃-Alkyl- bedeutet,
deren Enantiomere, deren Diastereomere, deren Gemische und deren Salze, insbesondere deren physiologisch verträgliche Salze mit organischen oder anorganischen Säuren oder Basen.

Eine zehnte Ausführungsform der vorliegenden Erfindung besteht in den Verbindungen der obigen allgemeinen Formel **I**, in denen
- **A**: eine Bindung,
- **B**: eine Bindung,
- **-D-Y-**: zusammen eine Gruppe ausgewählt aus 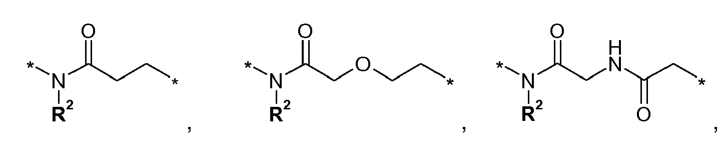 und
- **R¹**: die Gruppe 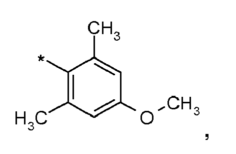
- **R²**: H oder C₁₋₃-Alkyl-, wobei jede Methylengruppe mit bis zu zwei und jede Methylgruppe mit bis zu drei Fluoratomen substituiert sein kann,
- **R³**: eine C₄₋₆-Cycloalkylengruppe,
- **R⁴**: einen gesättigten 6- oder 7-gliedrigen Diaza-Heterocyclus und
- **R⁵**: C₁₋₃-Alkyl oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel **I** seien beispielsweise folgende genannt:

| **Beispiel** | **Struktur** |
|---|---|
| (1) | 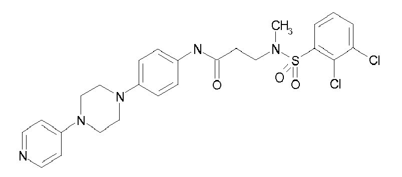 |
| (2) | 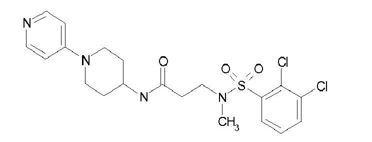 |
| (3) | 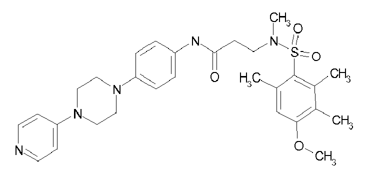 |
| (4) | 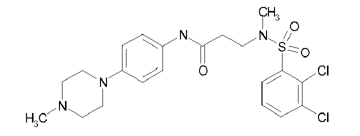 |
| (5) | 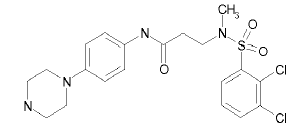 |
| (6) | 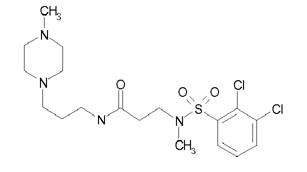 |
| (7) | 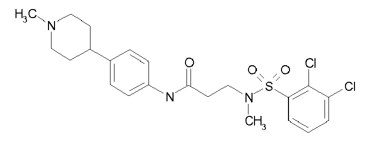 |
| (8) | 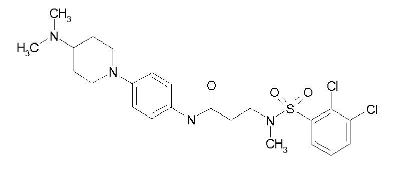 |
| (9) | 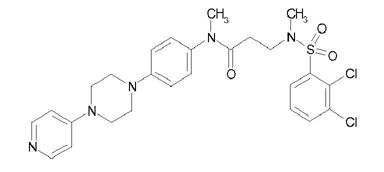 |
| (10) | 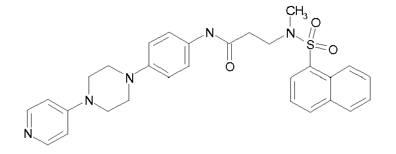 |
| (11) | 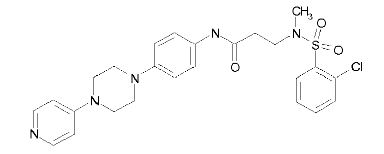 |
| (12) | 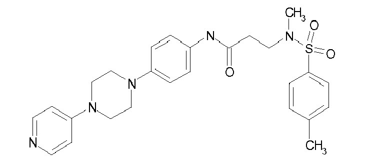 |
| (13) | 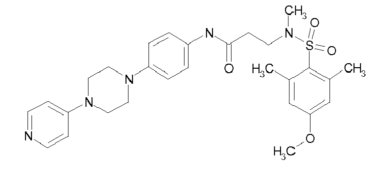 |
| (14) | 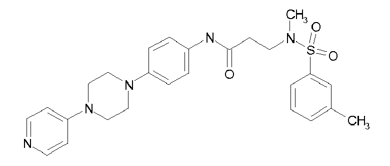 |
| (15) | 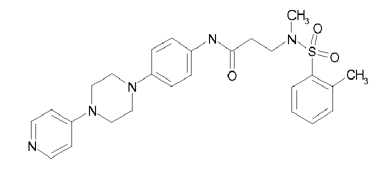 |
| (16) | 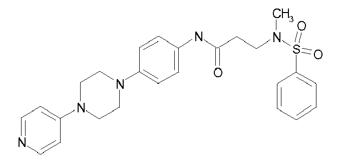 |
| (17) | 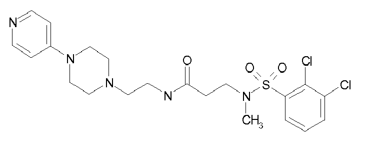 |
| (18) | 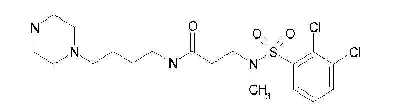 |
| (19) | 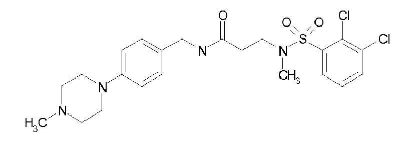 |
| (20) | 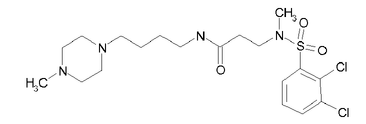 |
| (21) | 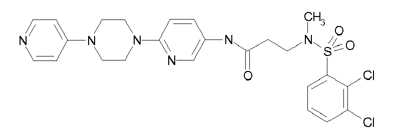 |
| (22) | 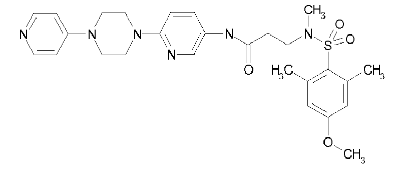 |
| (23) | 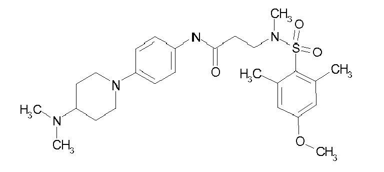 |
| (24) | 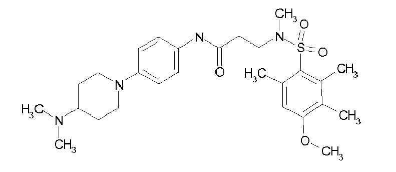 |
| (25) | 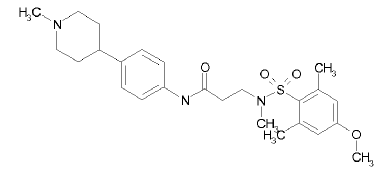 |
| (26) | 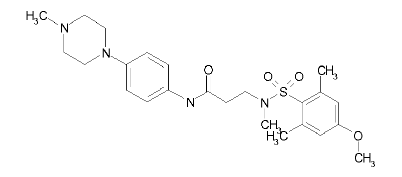 |
| (27) | 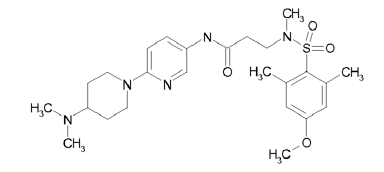 |
| (28) | 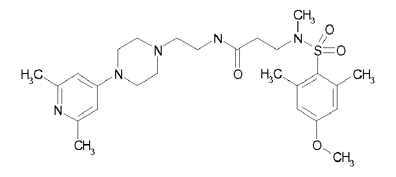 |
| (29) | 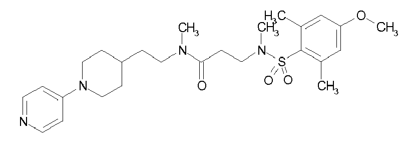 |
| (30) | 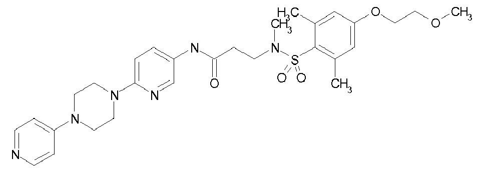 |
| (31) | 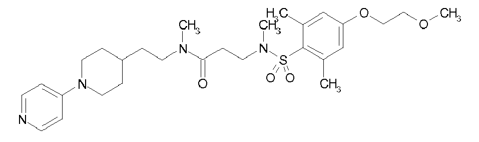 |
| (32) | 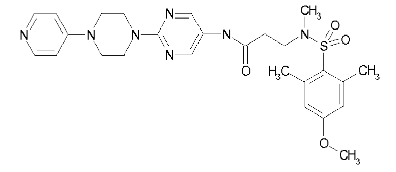 |
| (33) | 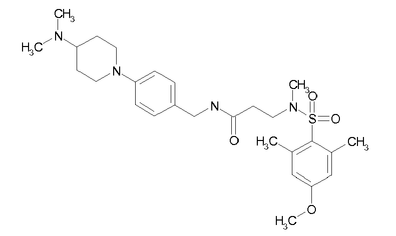 |
| (34) | 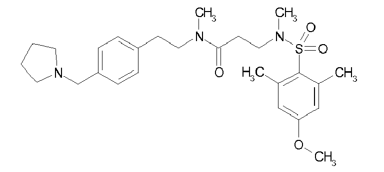 |
| (35) | 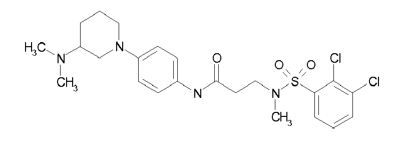 |
| (36) | 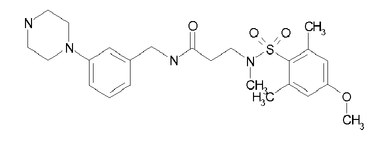 |
| (37) | 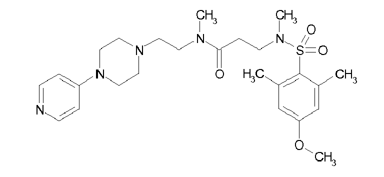 |
| (38) | 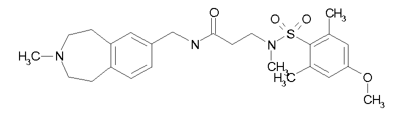 |
| (39) | 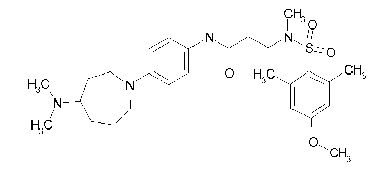 |
| (40) | 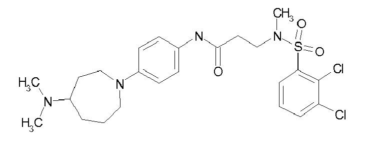 |
| (41) | 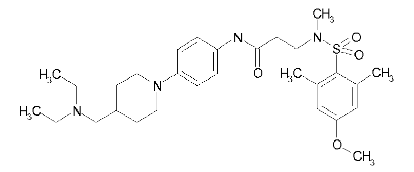 |
| (42) | 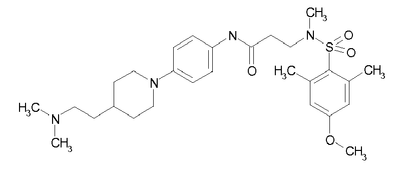 |
| (43) | 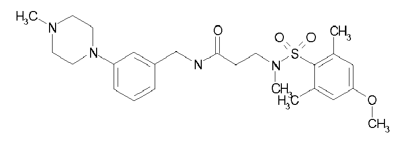 |
| (44) | 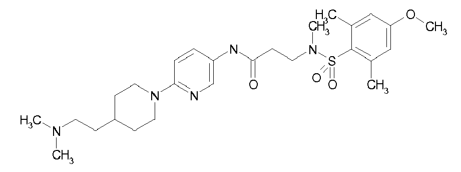 |
| (45) | 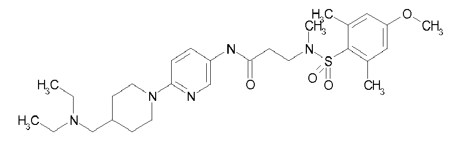 |
| (46) | 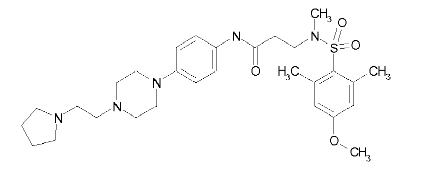 |
| (47) | 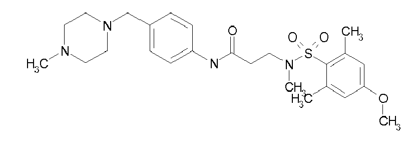 |
| (48) | 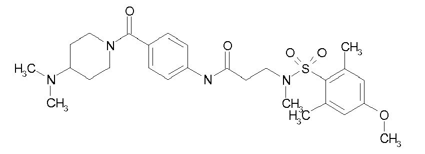 |
| (49) | 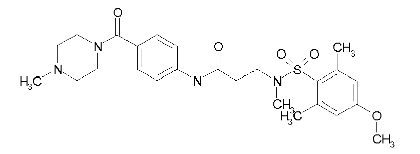 |
| (50) | 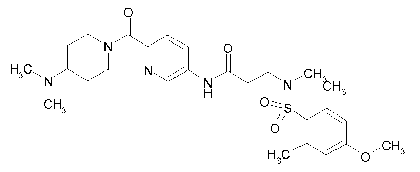 |
| (51) | 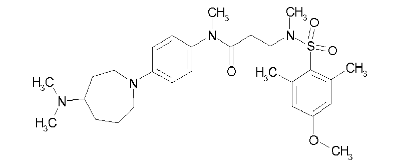 |
| (52) | 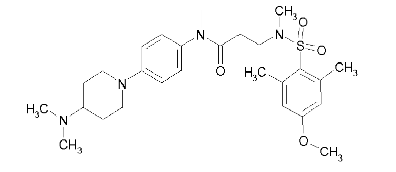 |
| (53) | 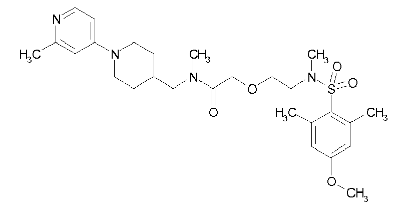 |
| (54) | 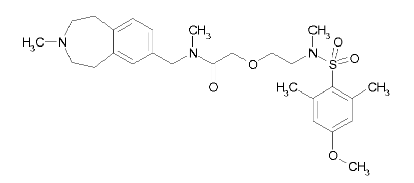 |
| (55) | 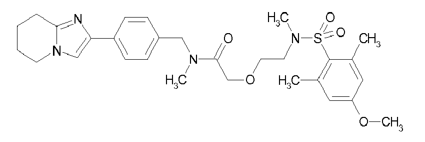 |
| (56) | 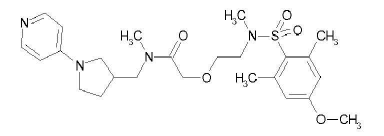 |
| (57) | 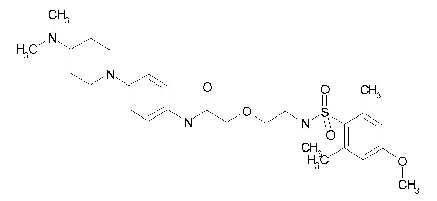 |
| (58) | 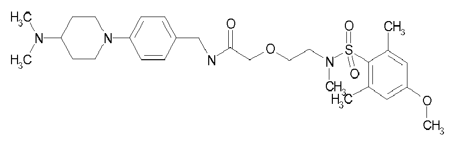 |
| (59) | 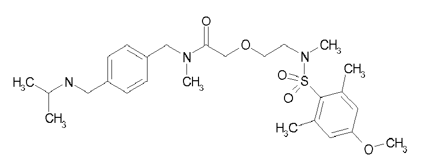 |
| (60) | 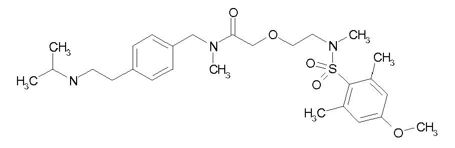 |
| (61) | 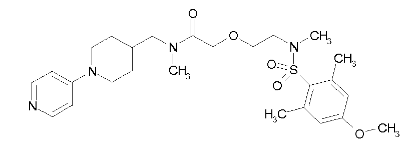 |
| (62) | 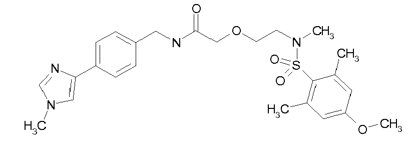 |
| (63) | 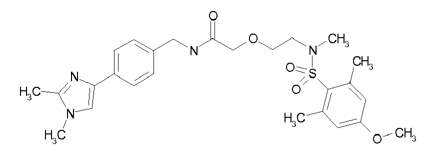 |
| (64) | 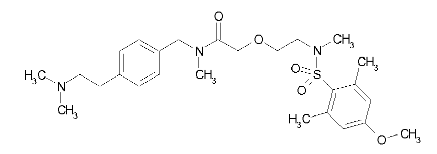 |
| (65) | 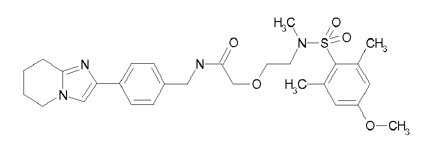 |
| (66) | 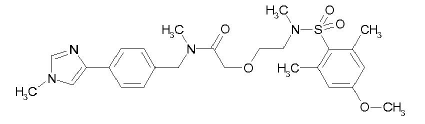 |
| (67) | 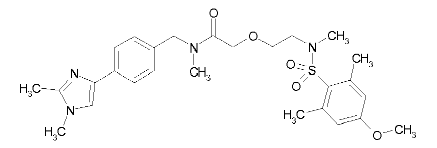 |
| (68) | 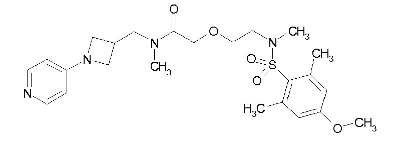 |
| (69) | 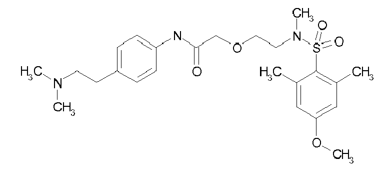 |
| (70) | 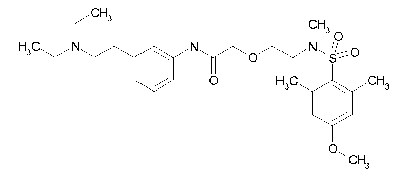 |
| (71) | 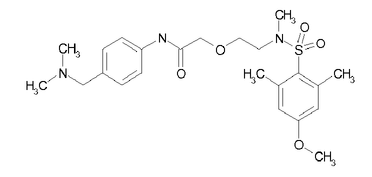 |
| (72) | 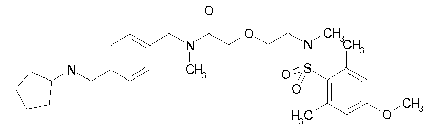 |
| (73) | 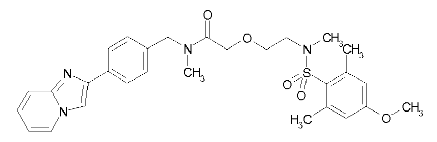 |
| (74) | 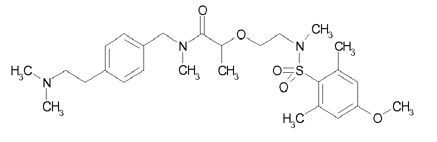 |
| (75) | 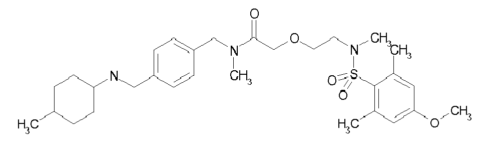 |
| (76) | 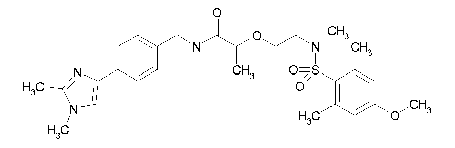 |
| (77) | 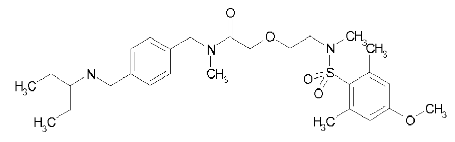 |
| (78) | 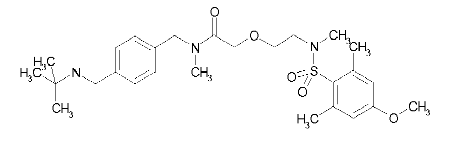 |
| (79) | 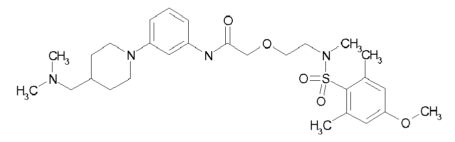 |
| (80) | 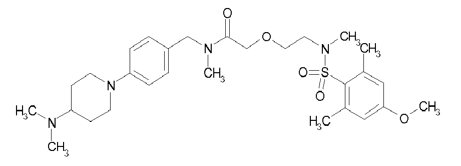 |
| (81) | 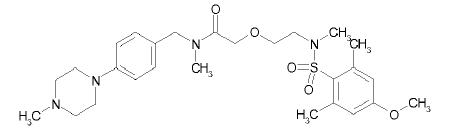 |
| (82) | 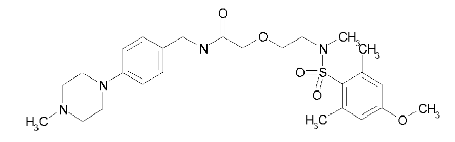 |
| (83) | 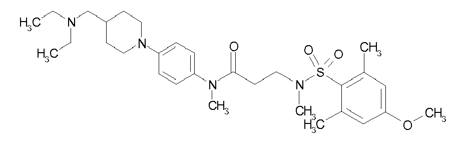 |
| (84) | 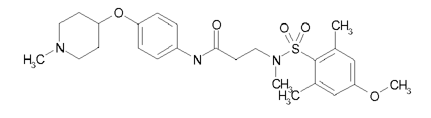 |
| (85) | 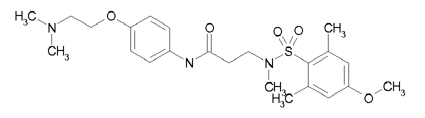 |
| (86) | 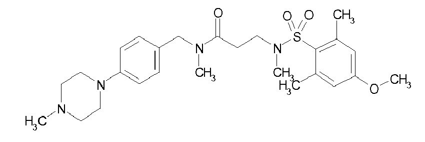 |
| (87) | 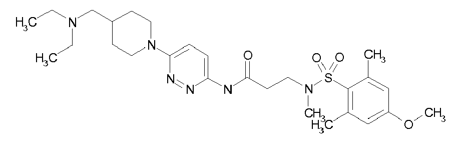 |
| (88) | 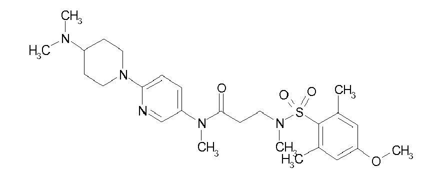 |
| (89) | 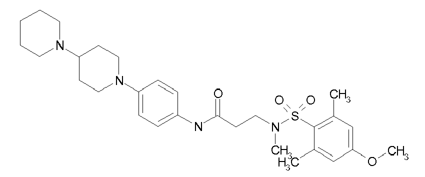 |
| (90) | 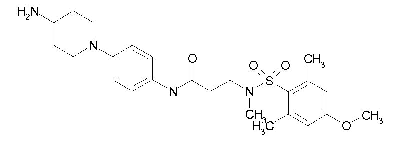 |
| (91) | 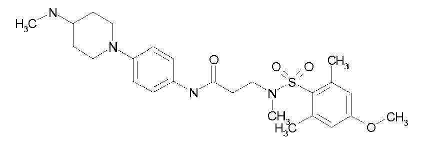 |
| (92) | 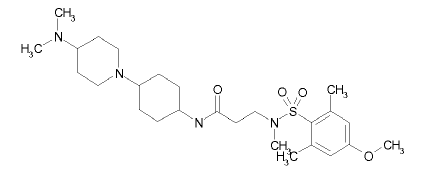 |
| (93) | 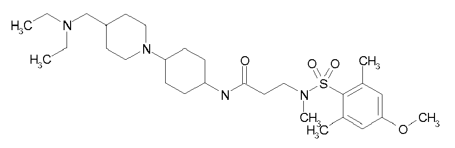 |
| (94) | 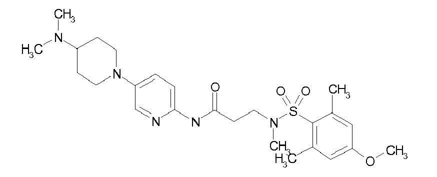 |
| (95) | 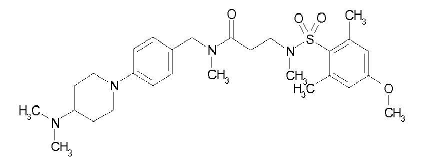 |
| (96) | 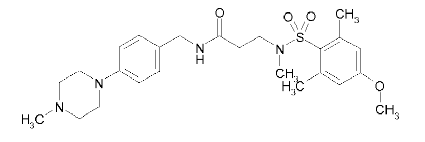 |
| (97) | 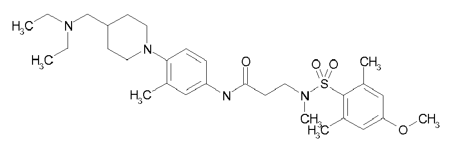 |
| (98) | 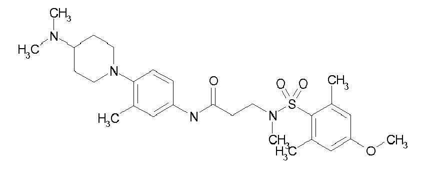 |
| (99) | 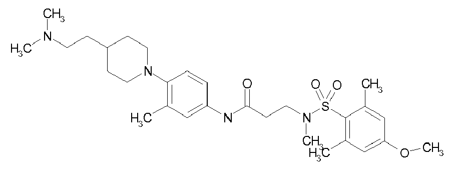 |
| (100) | 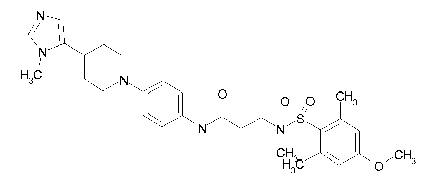 |
| (101) | 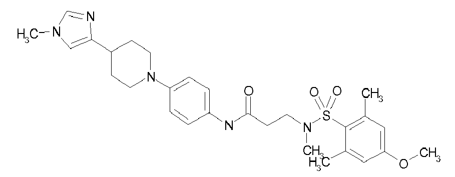 |
| (102) | 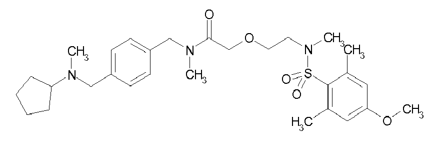 |
| (103) | 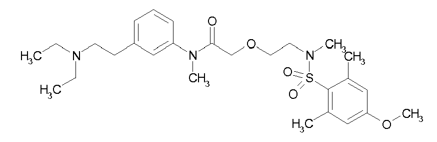 |
| (104) | 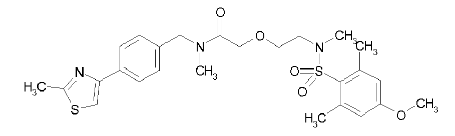 |
| (105) | 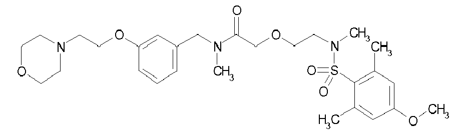 |
| (106) | 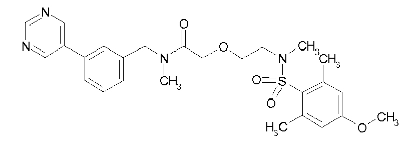 |
| (107) | 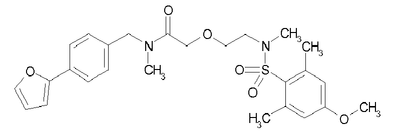 |
| (108) | 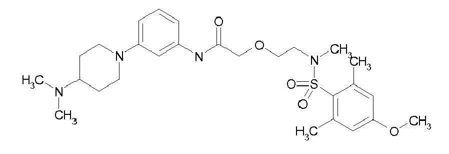 |
| (109) | 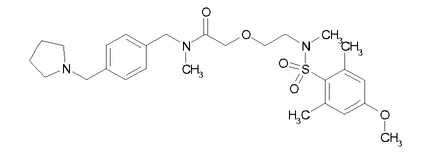 |
| (110) | 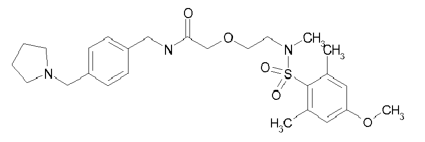 |
| (111) | 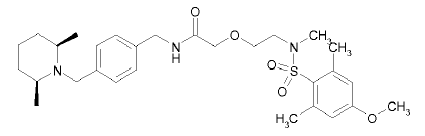 |
| (112) | 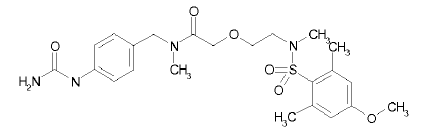 |
| (113) | 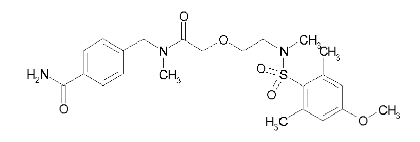 |
| (114) | 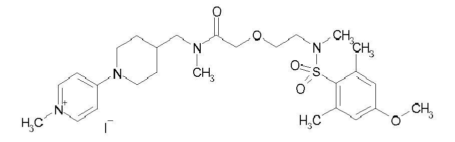 |
| (115) | 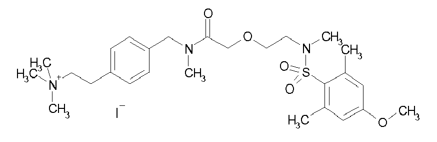 |
| (116) | 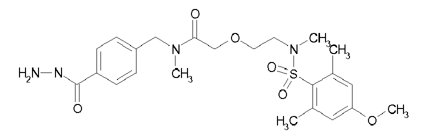 |
| (117) | 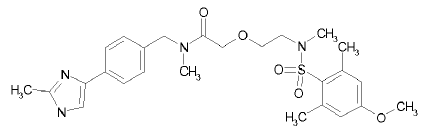 |
| (118) | 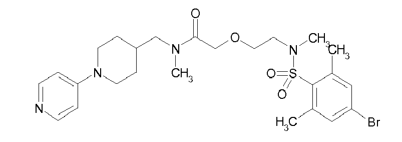 |
| (119) | 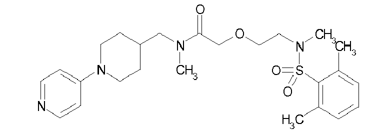 |
| (120) | 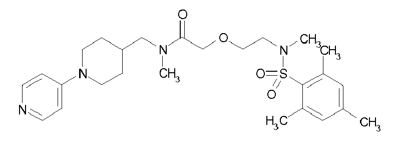 |
| (121) | 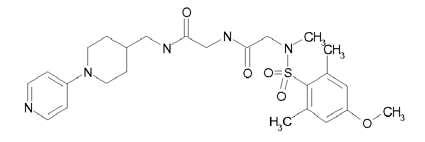 |
| (122) | 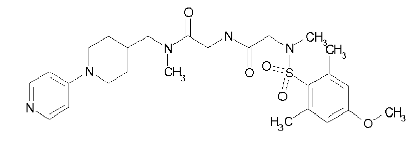 |
| (123) | 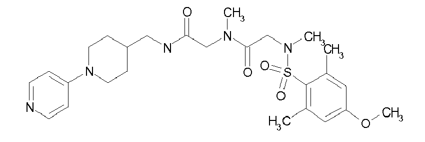 |
| (124) | 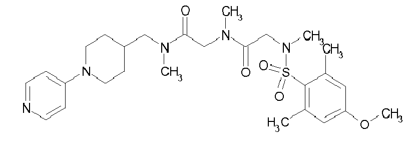 |
| (125) | 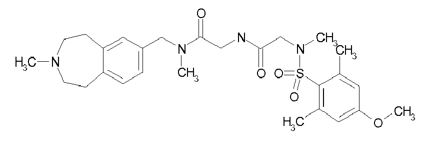 |
| (126) | 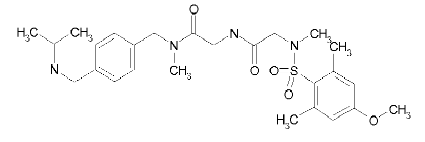 |
| (127) | 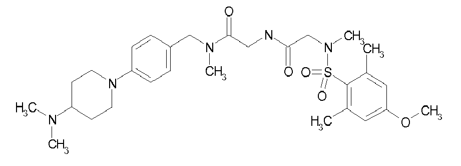 |
| (128) | 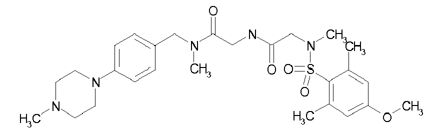 |
| (129) | 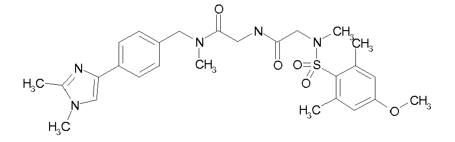 |
| (130) | 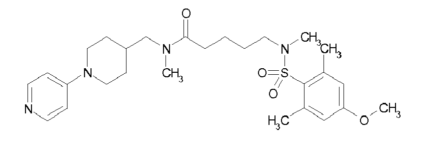 |
| (131) | 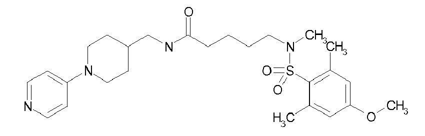 |
| (132) | 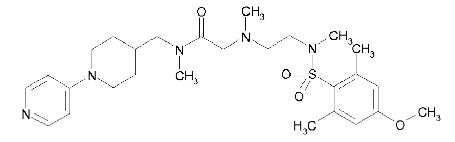 |
| (133) | 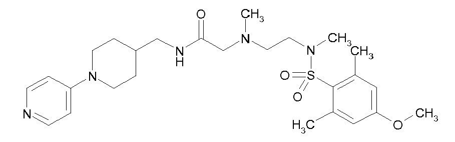 |
| (134) | 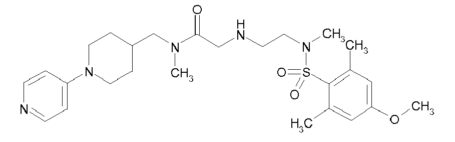 |
| (135) | 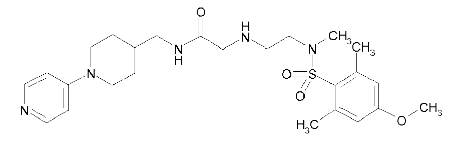 |
| (136) | 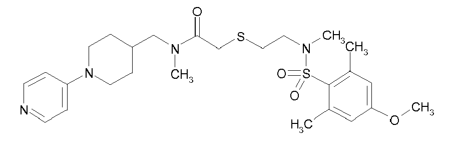 |
| (137) | 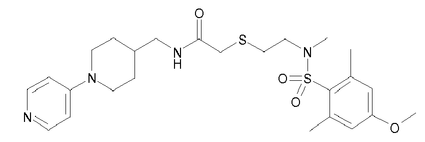 |
| (138) | 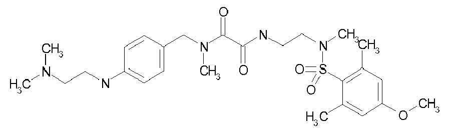 |
| (139) | 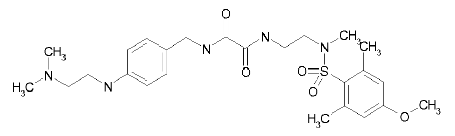 |
| (140) | 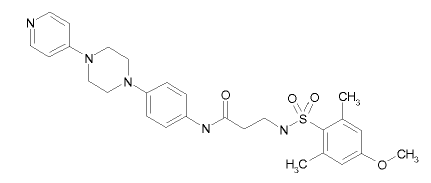 |
| (141) | 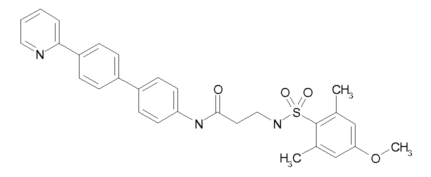 |
| (142) | 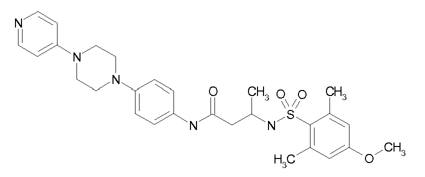 |
| (143) | 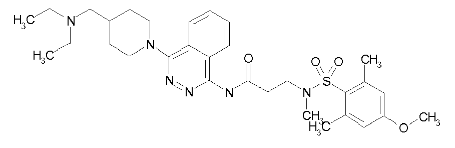 |
| (144) | 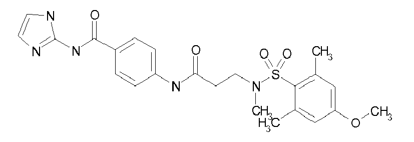 |
| (145) | 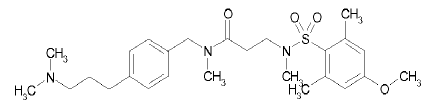 |
| (146) | 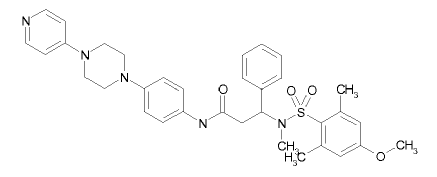 |
| (147) | 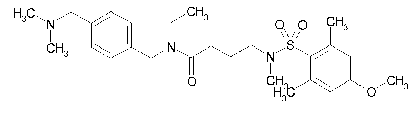 |
| (148) | 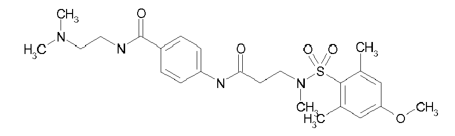 |
| (149) | 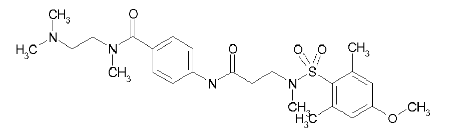 |
| (150) | 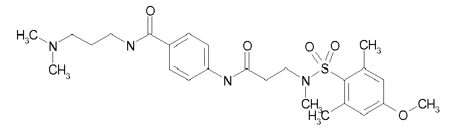 |
| (151) | 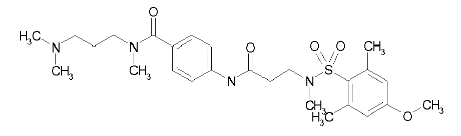 |
| (152) | 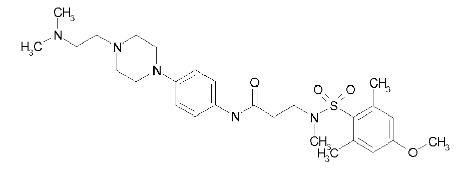 |
| (153) | 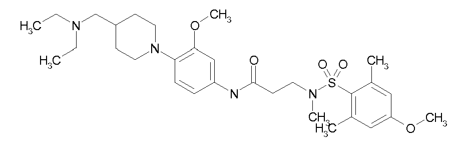 |
| (154) | 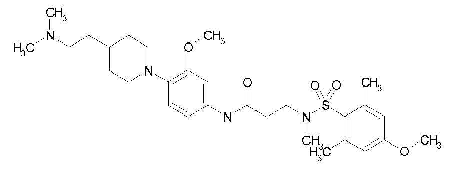 |
| (155) | 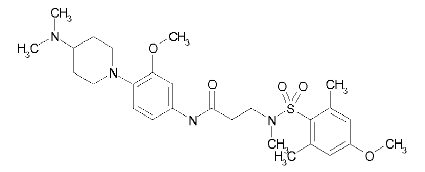 |
| (156) | 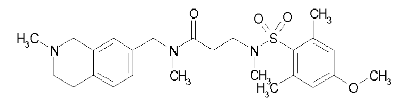 |
| (157) | 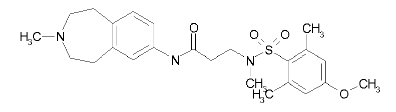 |
| (158) | 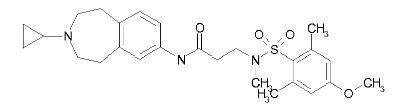 |
| (159) | 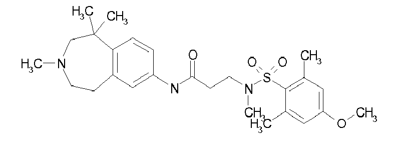 |
| (160) | 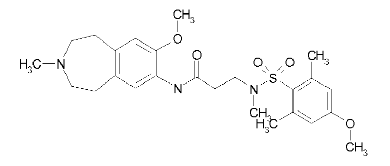 |
| (161) | 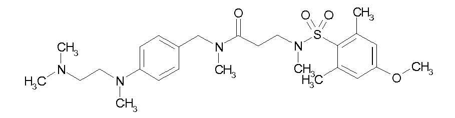 |
| (162) | 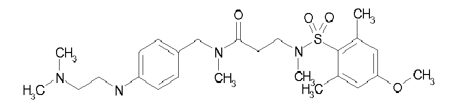 |
| (163) | 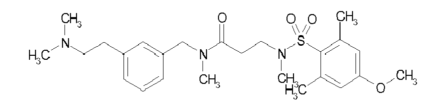 |
| (164) | 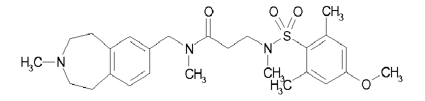 |
| (165) | 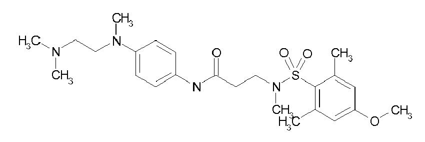 |
| (166) | 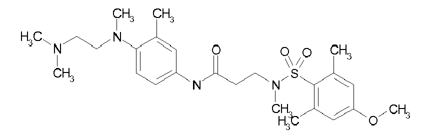 |
| (167) | 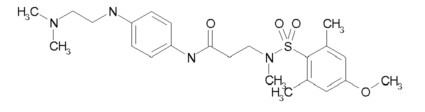 |
| (168) | 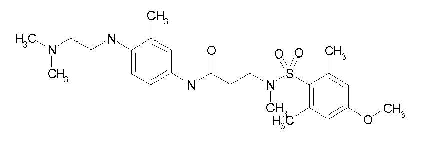 |
| (169) | 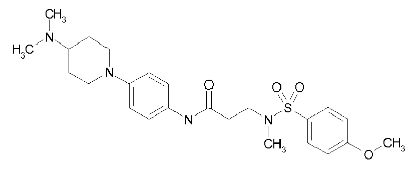 |
| (170) | 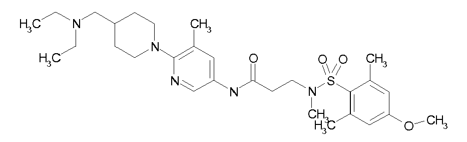 |
| (171) | 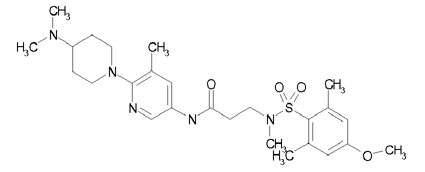 |
| (172) | 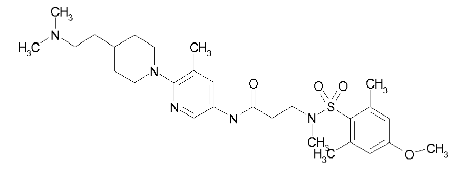 |
| (173) | 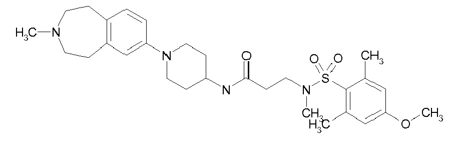 |
| (174) | 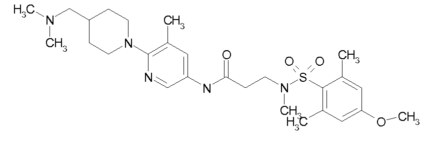 |
| (175) | 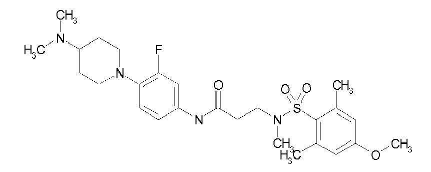 |
| (176) | 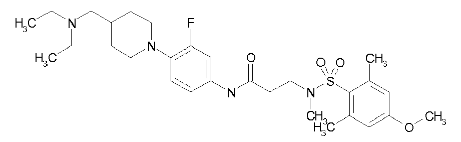 |
| (177) | 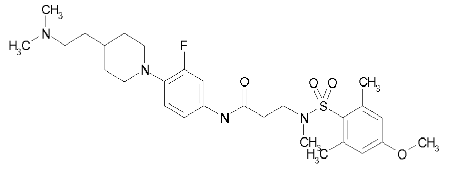 |
| (178) | 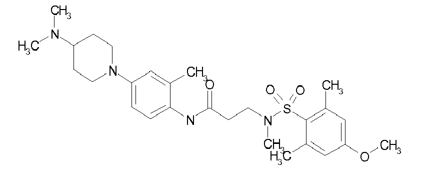 |
| (179) | 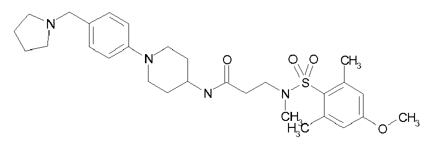 |
| (180) | 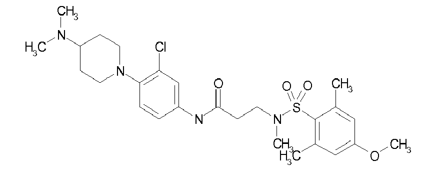 |
| (181) | 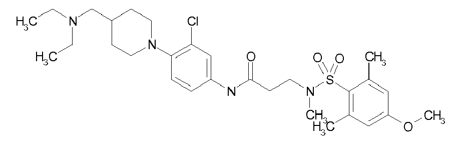 |
| (182) | 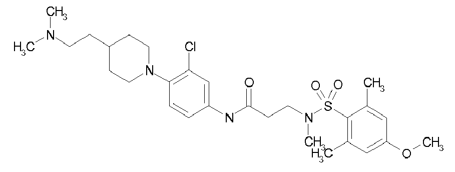 |
| (183) | 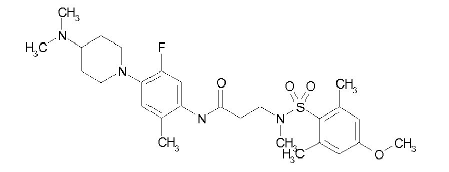 |
| (184) | 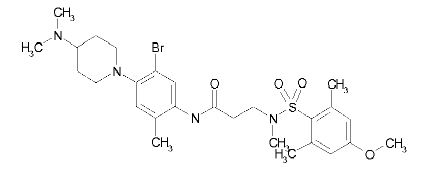 |
| (185) | 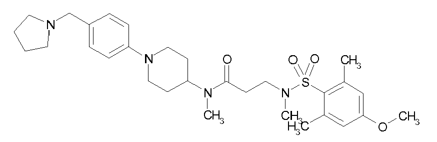 |
| (186) | 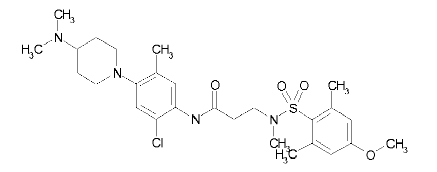 |
| (187) | 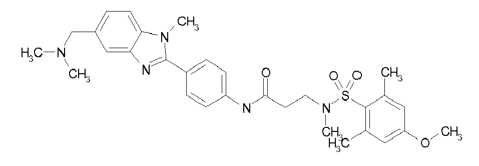 |
| (188) | 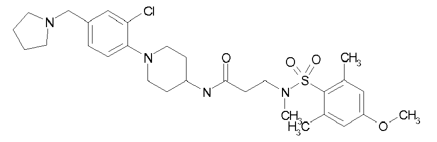 |
| (189) | 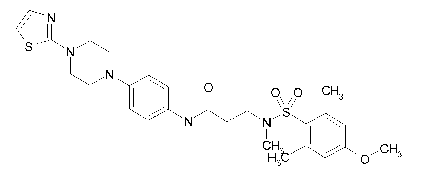 |
| (190) | 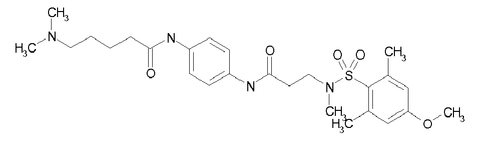 |
| (191) | 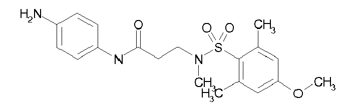 |
| (192) | 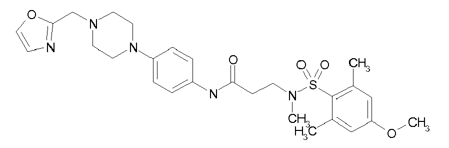 |
| (193) | 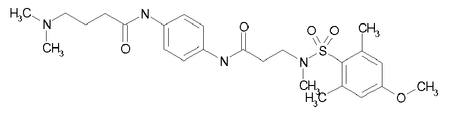 |
| (194) | 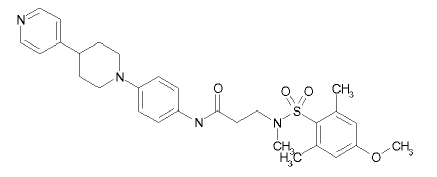 |
| (195) | 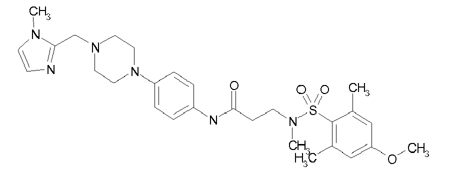 |
| (196) | 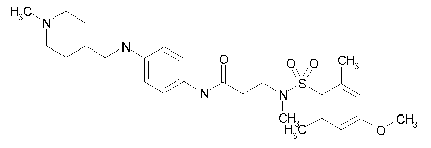 |
| (197) | 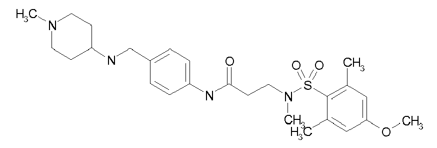 |
| (198) | 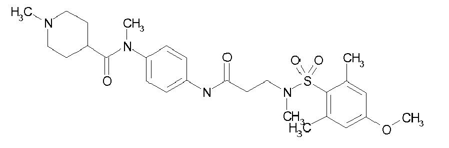 |
| (199) | 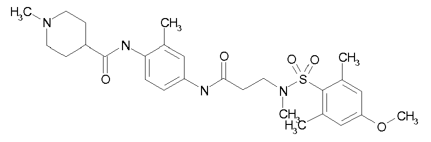 |
| (200) | 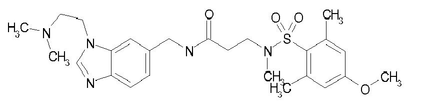 |
| (201) | 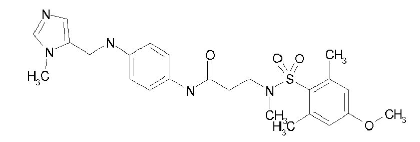 |
| (202) | 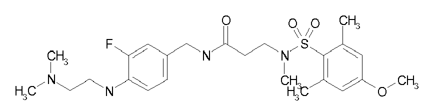 |
| (203) | 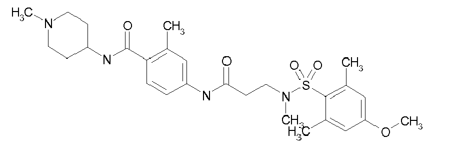 |
| (204) | 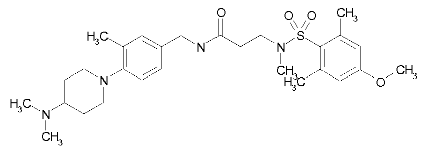 |
| (205) | 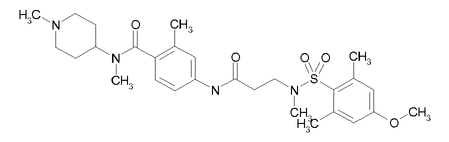 |
| (206) | 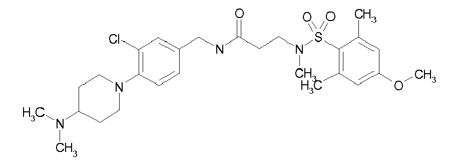 |
| (207) | 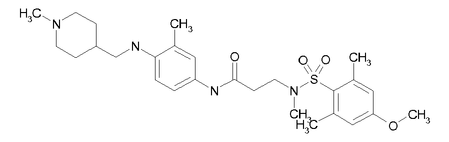 |
| (208) | 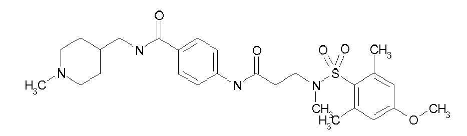 |
| (209) | 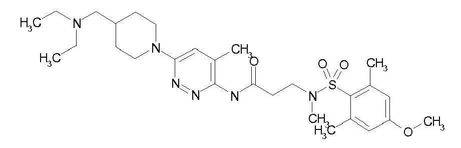 |
| (210) | 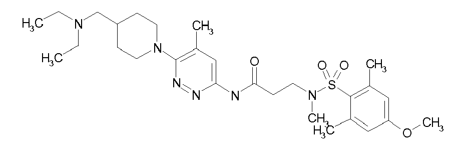 |
| (211) | 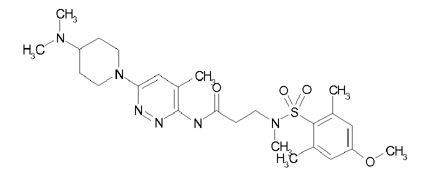 |
| (212) | 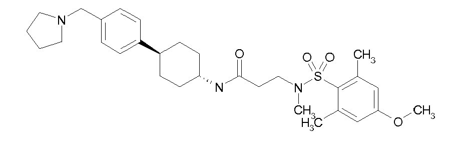 |
| (213) | 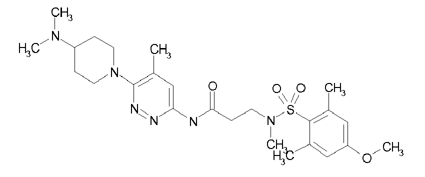 |
| (214) | 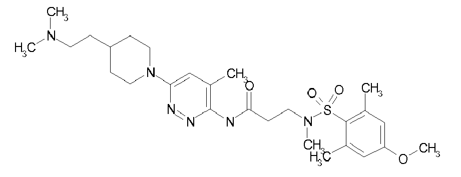 |
| (215) | 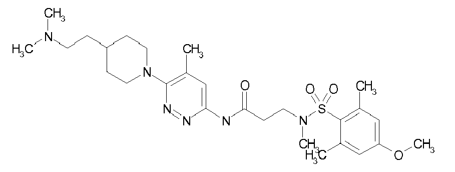 |
| (216) | 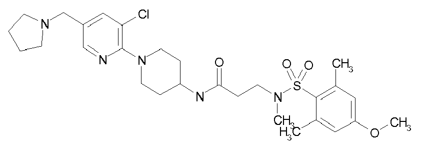 |
| (217) | 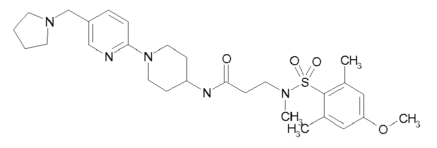 |
| (218) | 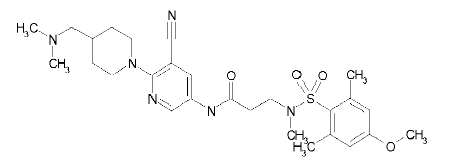 |
| (219) | 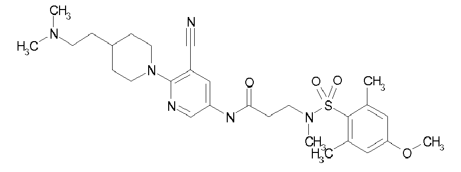 |
| (220) | 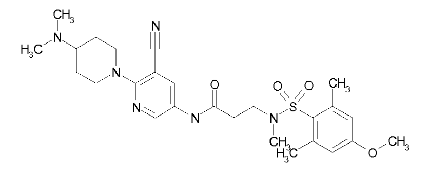 |
| (221) | 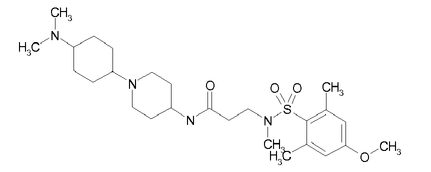 |
| (222) | 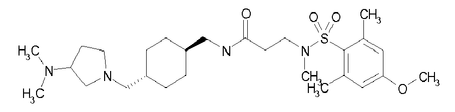 |
| (223) | 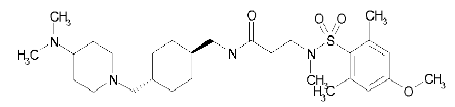 |
| (224) | 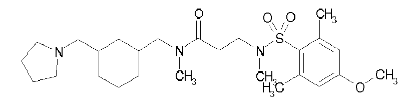 |
| (225) | 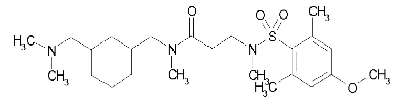 |
| (226) | 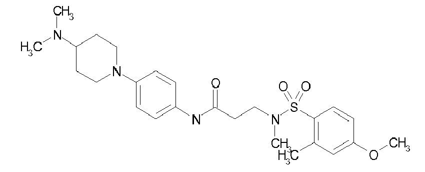 |
| (227) | 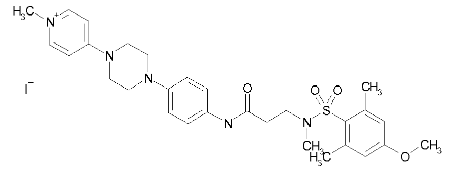 |
| (228) | 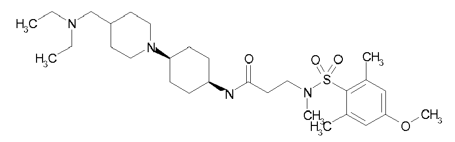 |
| (229) | 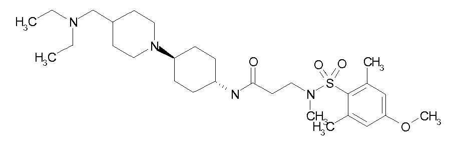 |
| (230) | 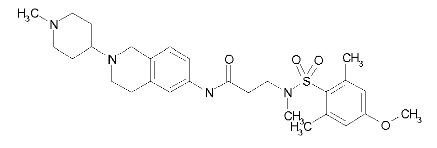 |
| (231) | 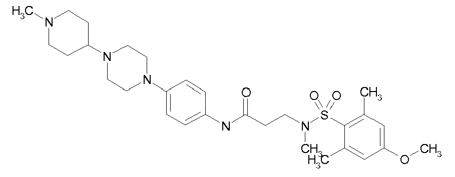 |
| (232) | 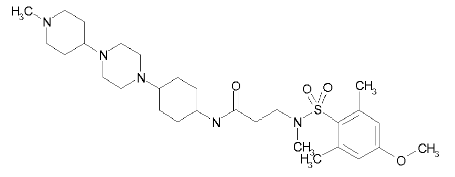 |
| (233) | 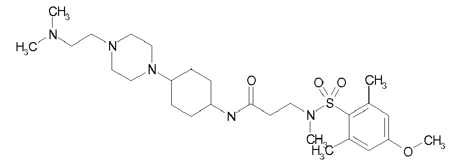 |
| (234) | 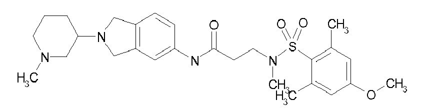 |
| (235) | 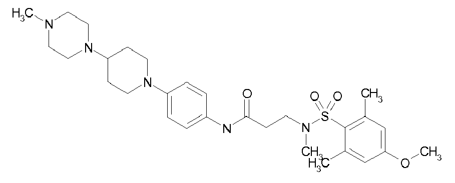 |
| (236) | 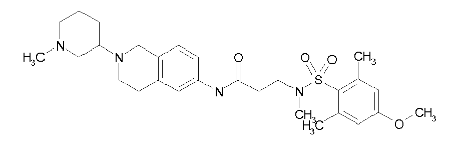 |
| (237) | 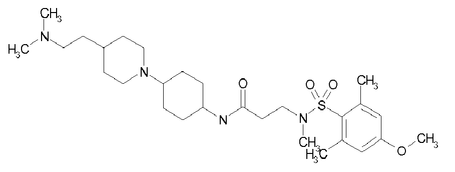 |
| (238) | 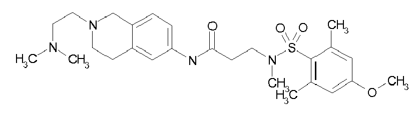 |
| (239) | 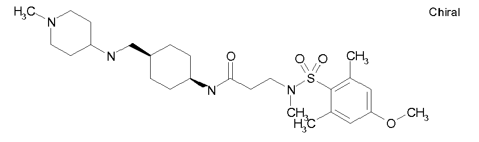 |
| (240) | 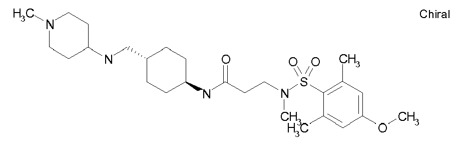 |
| (241) | 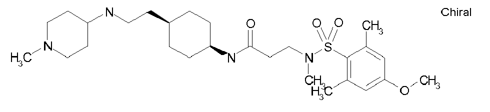 |
| (242) | 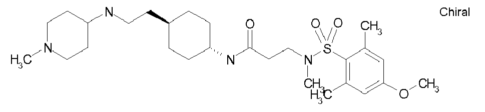 |
| (243) | 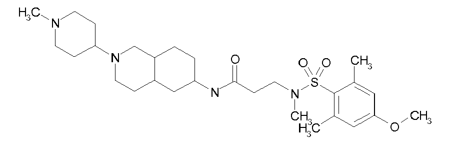 |
| (244) | 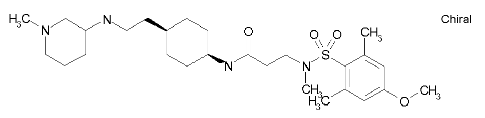 |
| (245) | 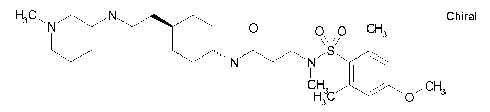 |
| (246) | 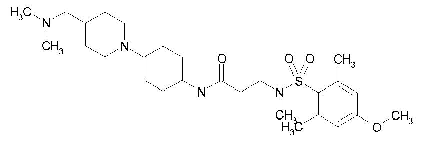 |
| (247) | 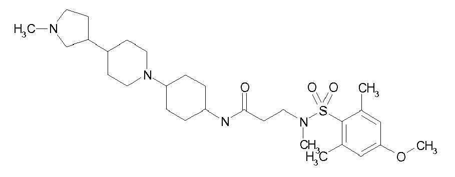 |
| (248) | 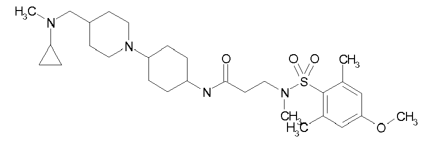 |
| (249) | 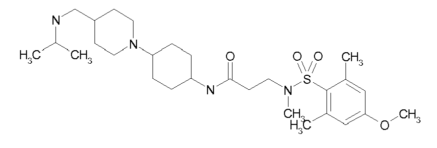 |
| (250) | 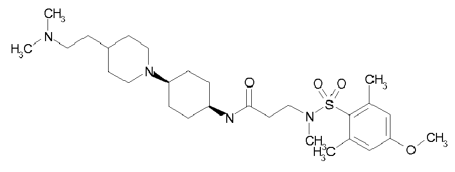 |
| (251) | 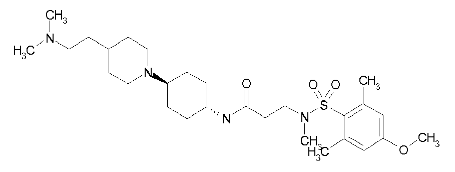 |
| (252) | 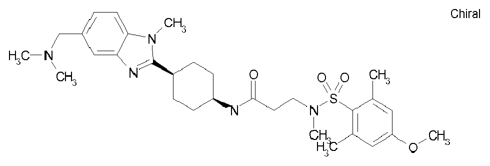 |
| (253) | 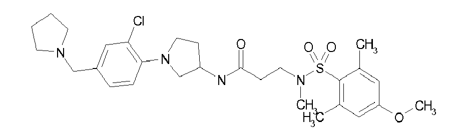 |
| (254) | 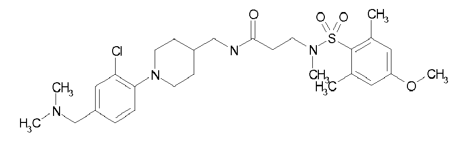 |
| (255) | 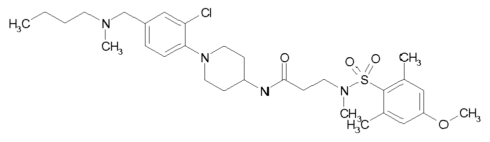 |
| (256) | 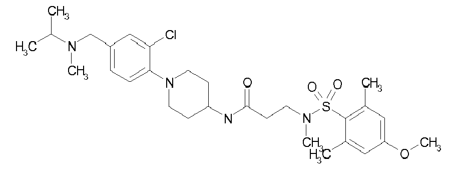 |
| (257) | 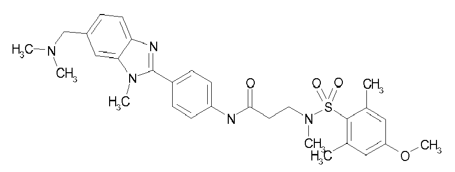 |
| (258) | 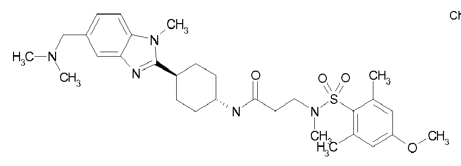 |
| (259) | 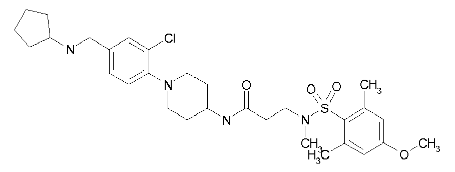 |
| (260) | 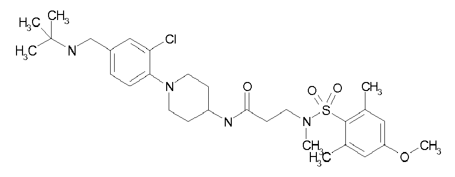 |
| (261) | 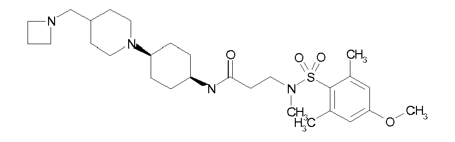 |
| (262) | 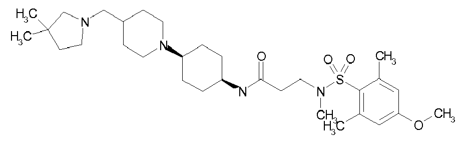 |
| (263) | 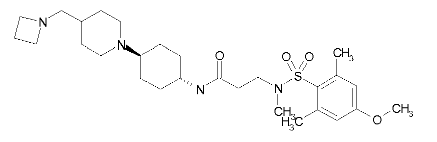 |
| (264) | 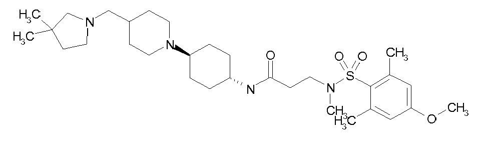 |
| (265) | 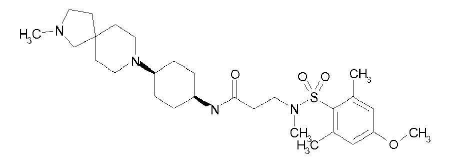 |
| (266) | 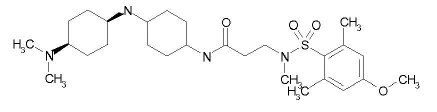 |
| (267) | 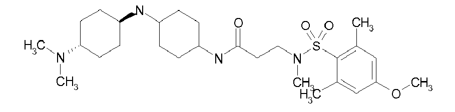 |
| (268) | 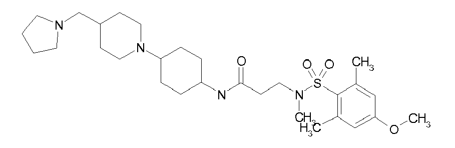 |
| (269) | 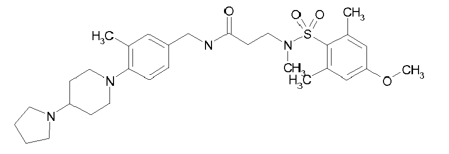 |
| (270) | 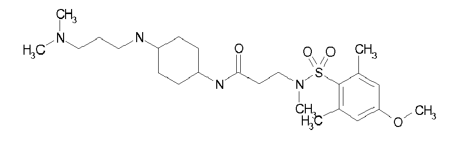 |
| (271) | 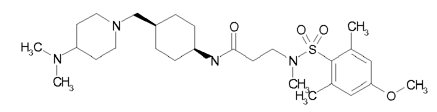 |
| (272) | 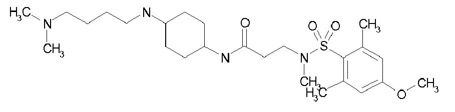 |
| (273) | 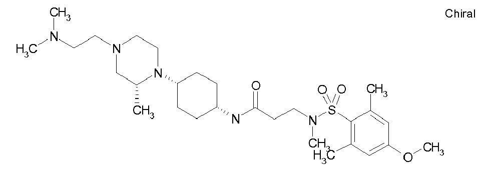 |
| (274) | 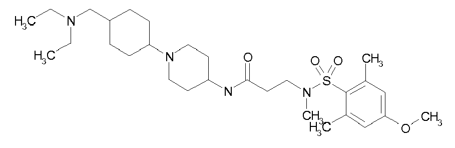 |
| (275) | 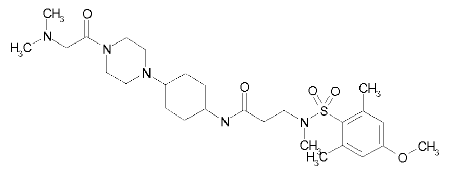 |
| (276) | 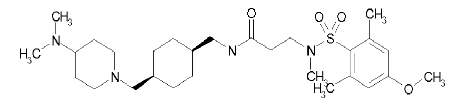 |
| (277) | 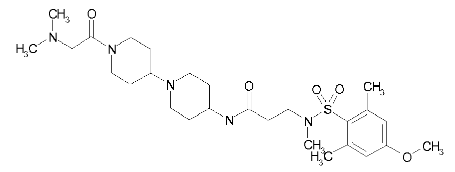 |
| (278) | 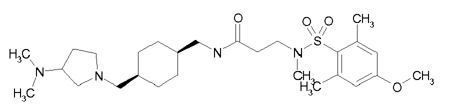 |
| (279) | 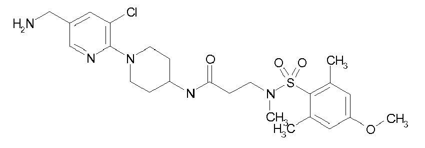 |
| (280) | 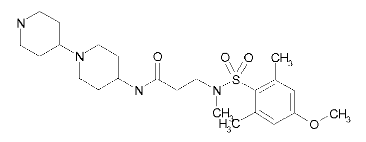 |
| (281) | 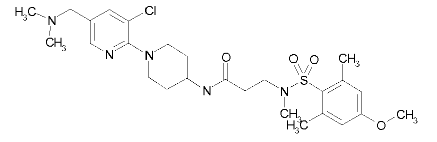 |
| (282) | 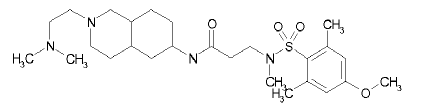 |
| (283) | 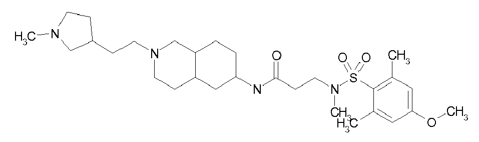 |
| (284) | 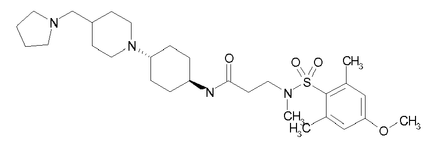 |
| (285) | 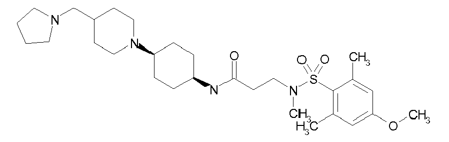 |
| (286) | 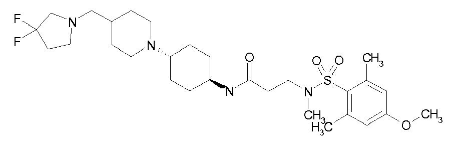 |
| (287) | 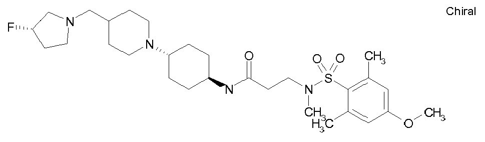 |
| (288) | 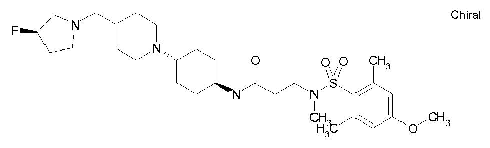 |
| (289) | 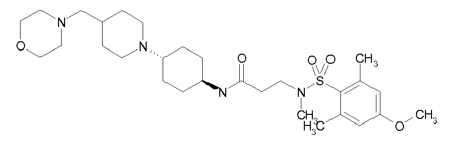 |
| (290) | 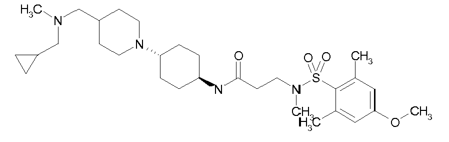 |
| (291) | 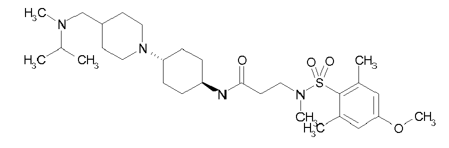 |
| (292) | 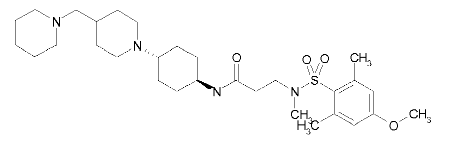 |
| (293) | 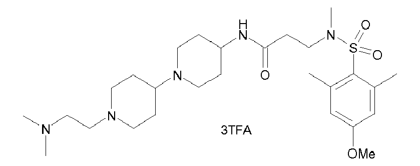 |
| (294) | 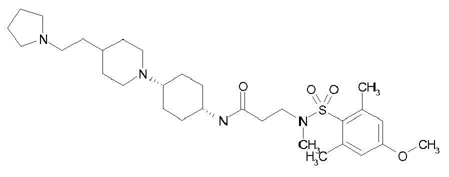 |
| (295) | 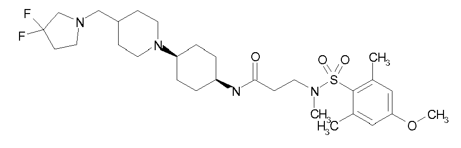 |
| (296) | 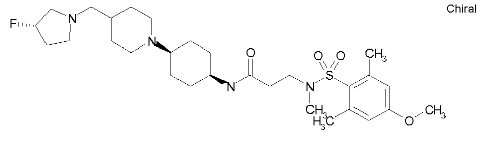 |
| (297) | 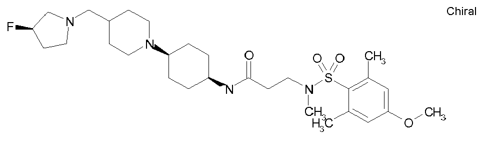 |
| (298) | 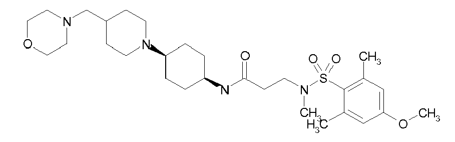 |
| (299) | 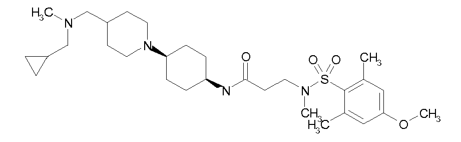 |
| (300) | 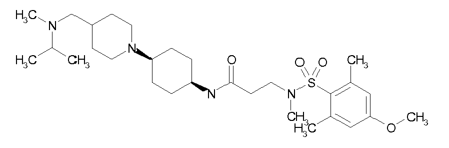 |
| (301) | 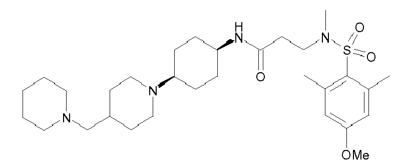 |
| (302) | 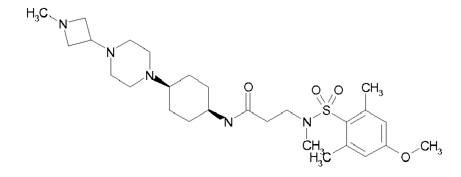 |
| (303) | 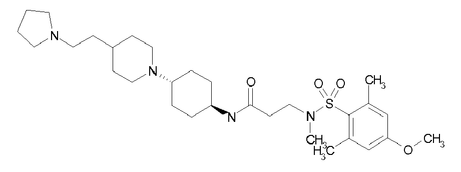 |
| (304) | 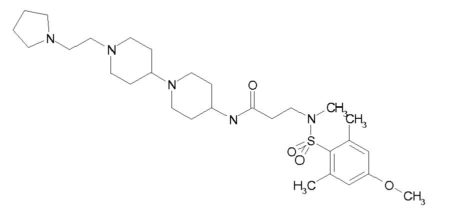 |
| (305) | 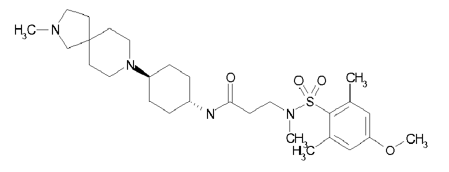 |
| (306) | 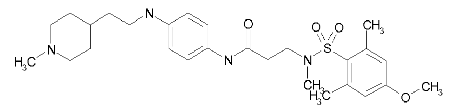 |
| (307) | 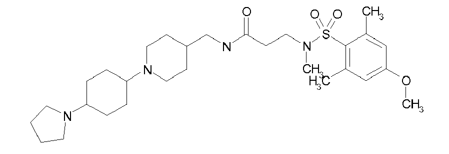 |
| (308) | 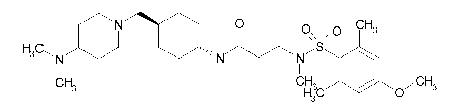 |
| (309) | 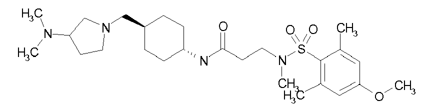 |
| (310) | 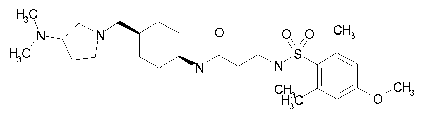 |
| (311) | 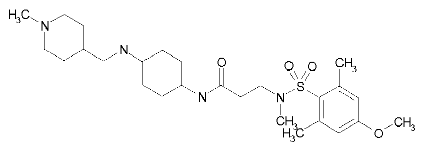 |
| (312) | 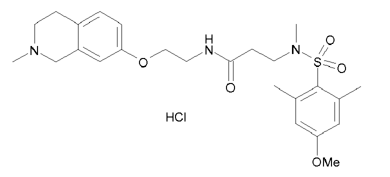 |
| (313) | 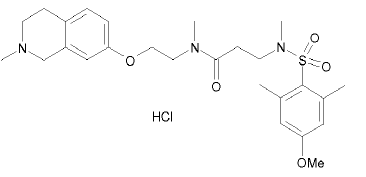 |
| (314) | 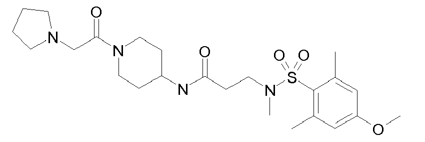 |
| (315) | 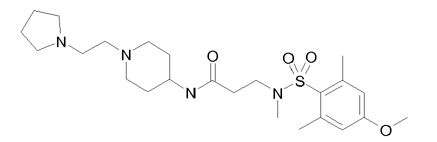 |
| (316) | 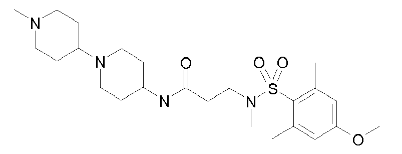 |
| (317) | 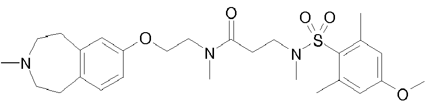 |
| (318) | 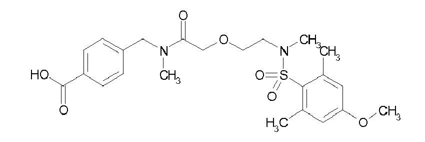 |
| (319) | 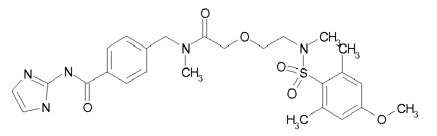 |
| (320) | 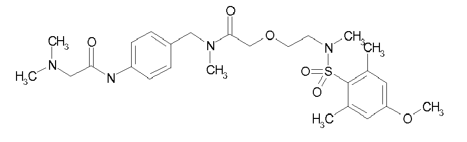 |
| (321) | 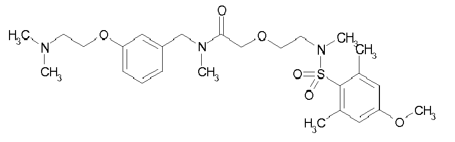 |
| (322) | 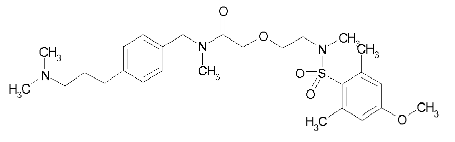 |
| (323) | 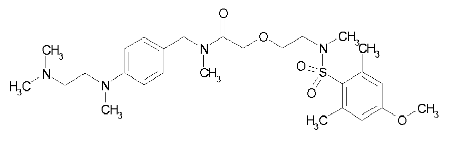 |
| (324) | 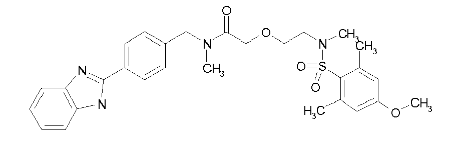 |
| (325) | 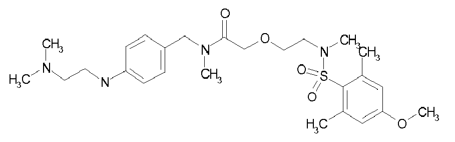 |
| (326) | 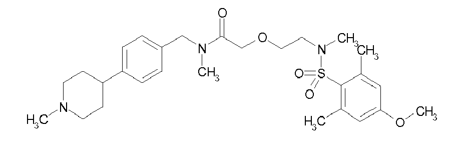 |
| (327) | 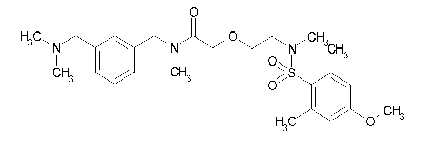 |
| (328) | 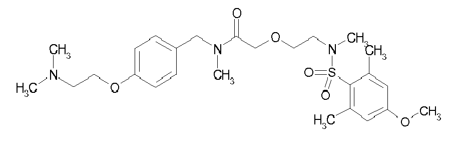 |
| (329) | 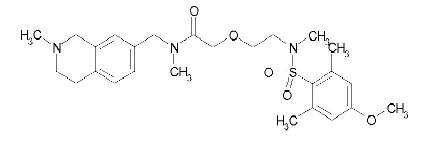 |
| (330) | 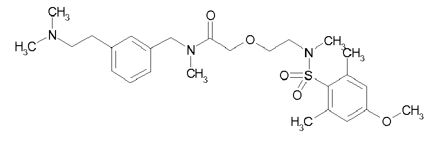 |
| (331) | 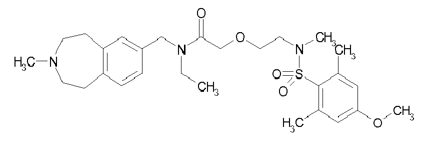 |
| (332) | 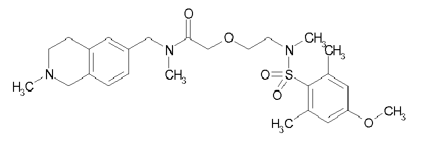 |
| (333) | 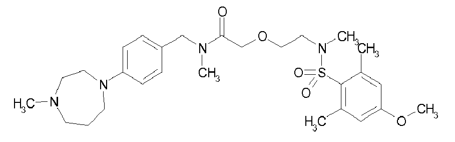 |
| (334) | 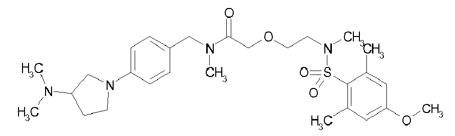 |
| (335) | 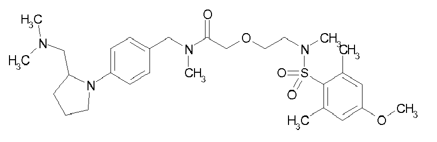 |
| (336) | 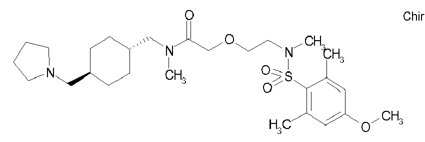 |
| (337) | 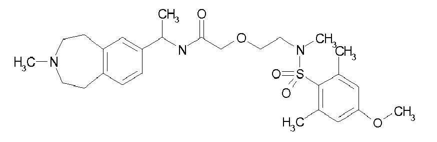 |
| (338) | 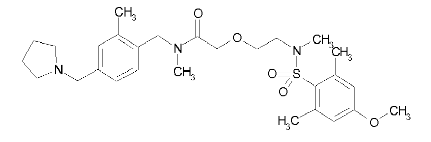 |
| (339) | 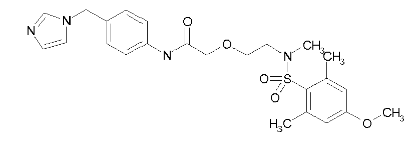 |
| (340) | 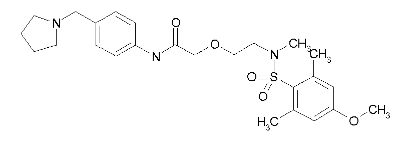 |
| (341) | 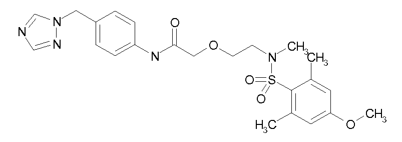 |
| (342) | 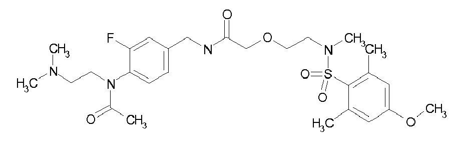 |
| (343) | 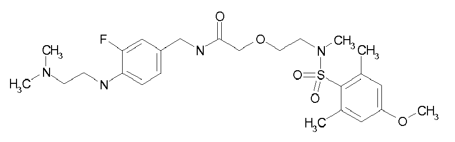 |
| (344) | 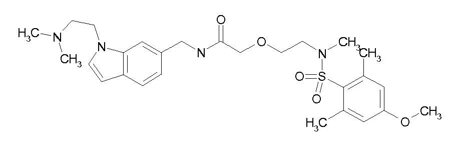 |
| (345) | 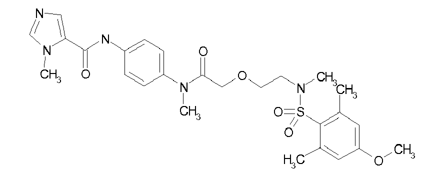 |
| (346) | 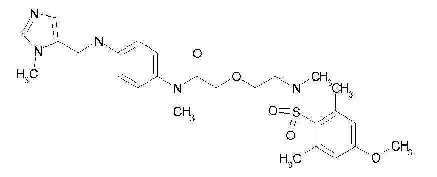 |
| (347) | 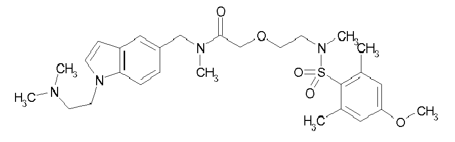 |
| (348) | 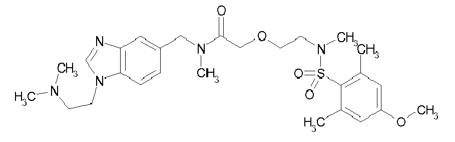 |
| (349) | 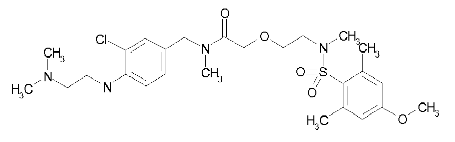 |
| (350) | 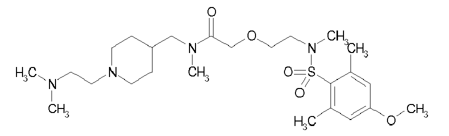 |
| (351) | 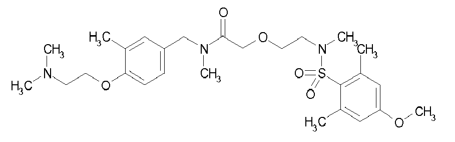 |
| (352) | 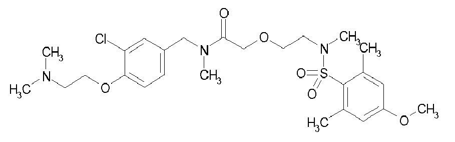 |
| (353) | 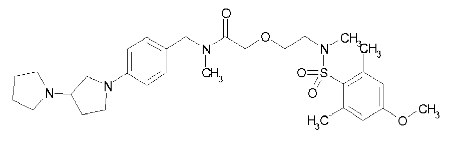 |
| (354) | 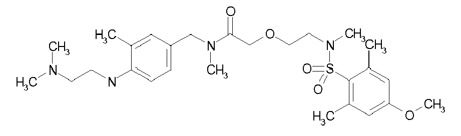 |
| (355) | 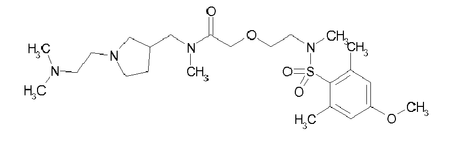 |
| (356) | 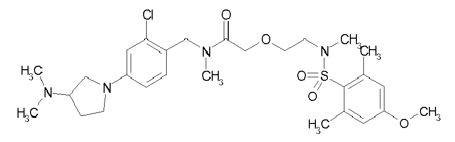 |
| (357) | 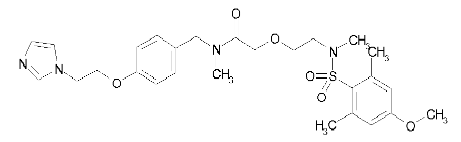 |
| (358) | 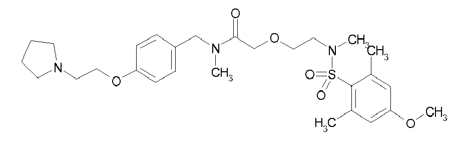 |
| (359) | 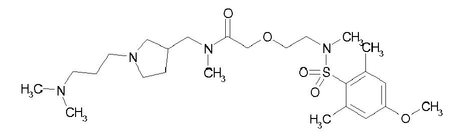 |
| (360) | 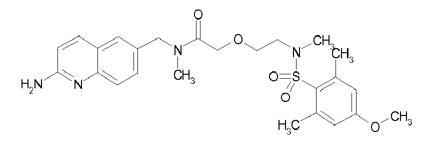 |
| (361) | 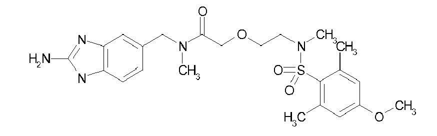 |
| (362) | 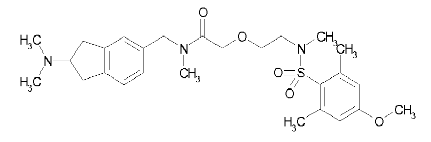 |
| (363) | 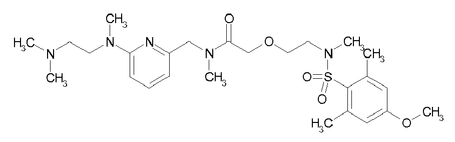 |
| (364) | 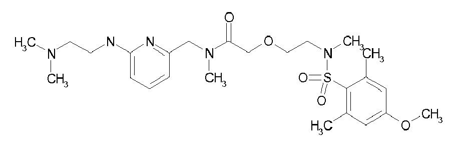 |
| (365) | 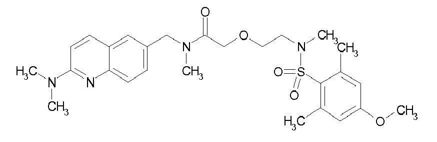 |
| (366) | 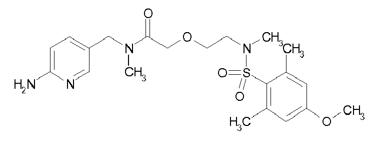 |
| (367) | 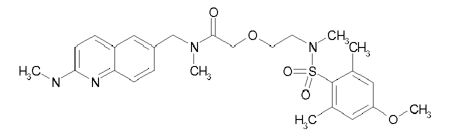 |
| (368) | 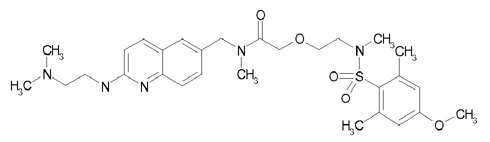 |
| (369) | 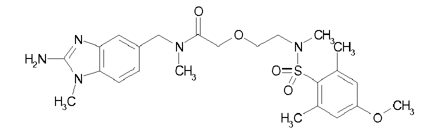 |
| (370) | 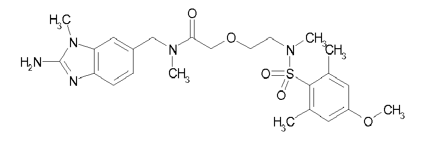 |
| (371) | 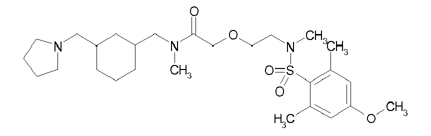 |
| (372) | 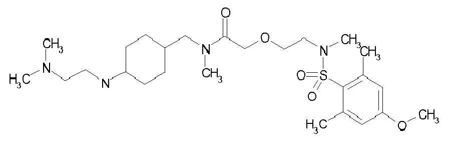 |
| (373) | 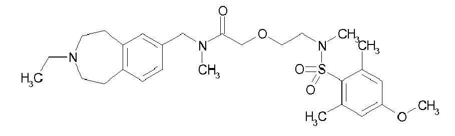 |
| (374) | 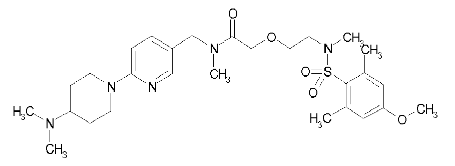 |
| (375) | 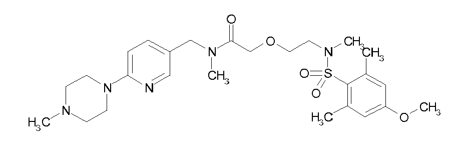 |
| (376) | 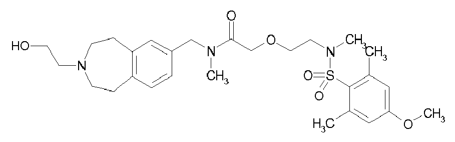 |
| (377) | 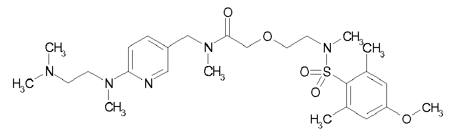 |
| (378) | 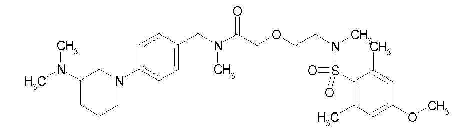 |
| (379) | 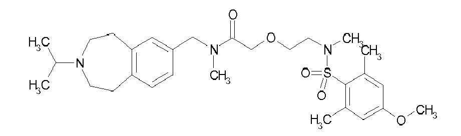 |
| (380) | 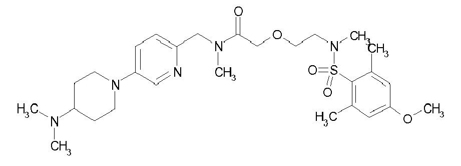 |
| (381) | 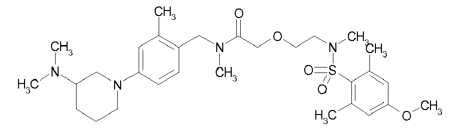 |
| (382) | 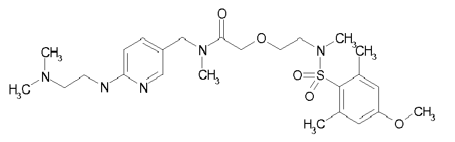 |
| (383) | 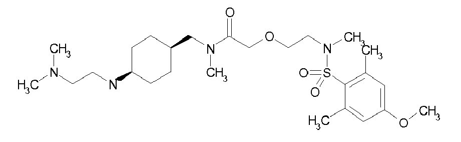 |
| (384) | 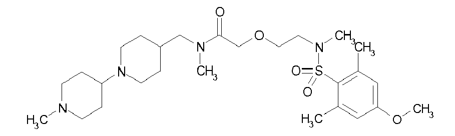 |
| (385) | 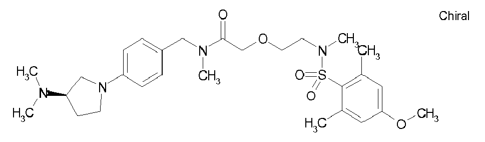 |
| (386) | 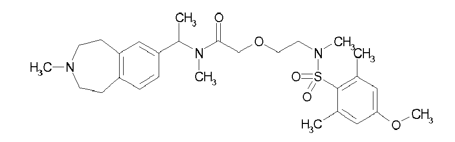 |
| (387) | 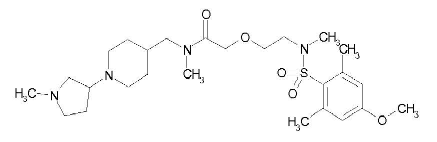 |
| (388) | 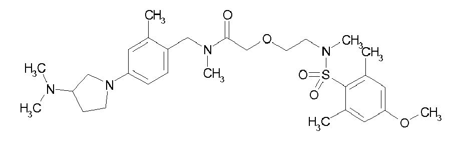 |
| (389) | 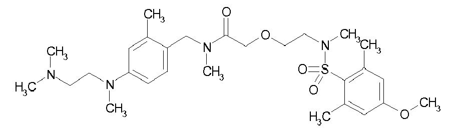 |
| (390) | 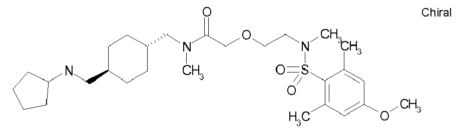 |
| (391) | 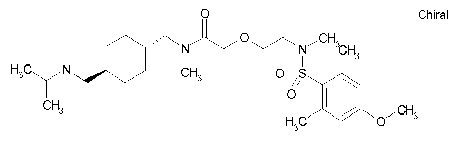 |
| (392) | 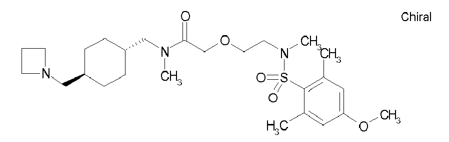 |
| (393) | 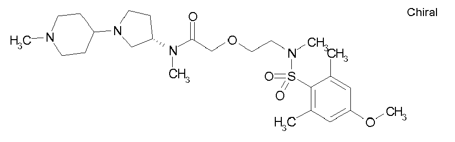 |
| (394) | 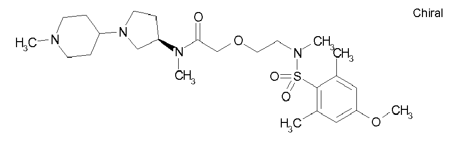 |
| (395) | 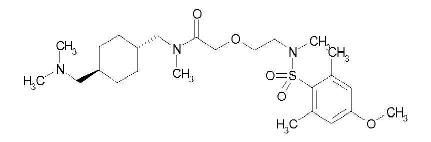 |
| (396) | 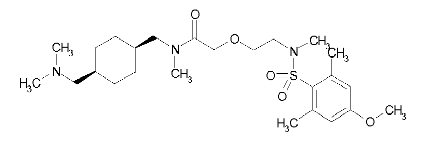 |
| (397) | 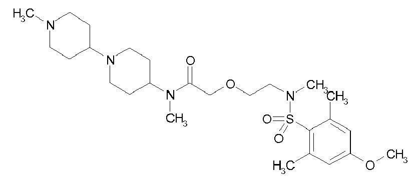 |
| (398) | 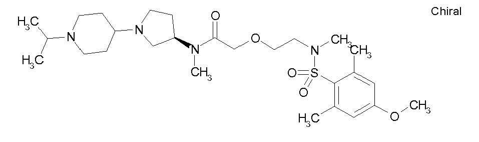 |
| (399) | 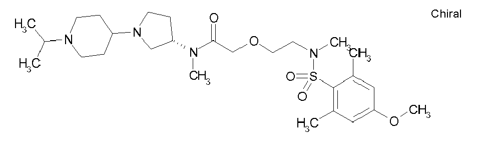 |
| (400) | 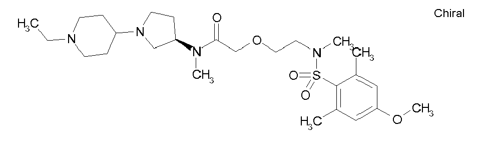 |
| (401) | 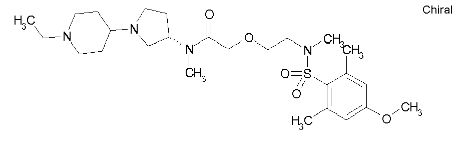 |
| (402) | 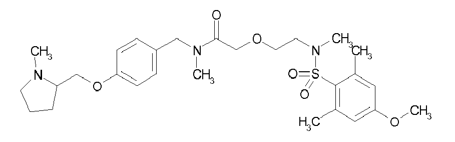 |
| (403) | 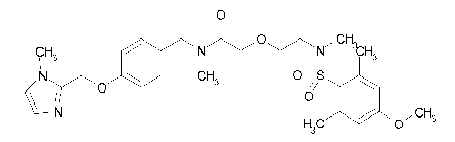 |
| (404) | 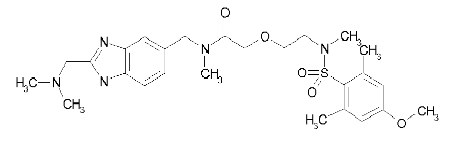 |
| (405) | 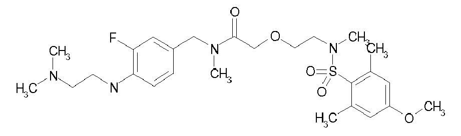 |
| (406) | 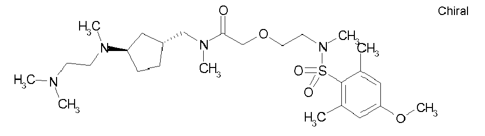 |
| (407) | 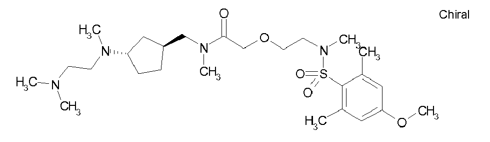 |
| (408) | 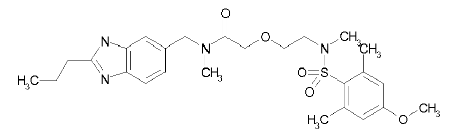 |
| (409) | 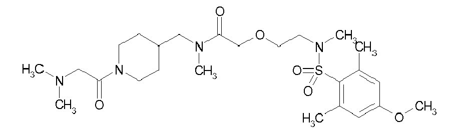 |
| (410) | 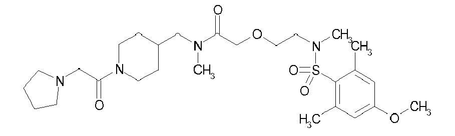 |
| (411) | 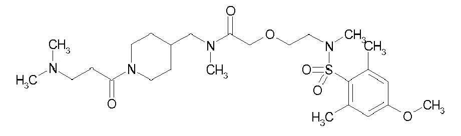 |
| (412) | 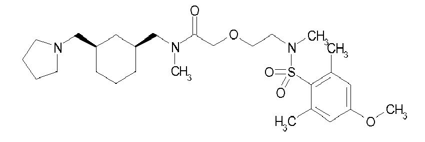 |
| (413) | 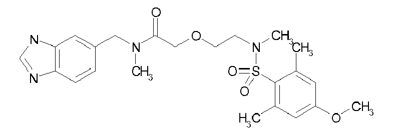 |
| (414) | 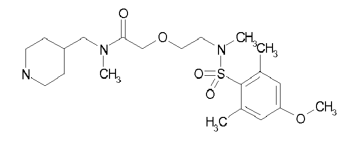 |
| (415) | 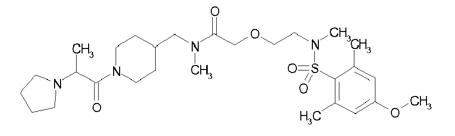 |
| (416) | 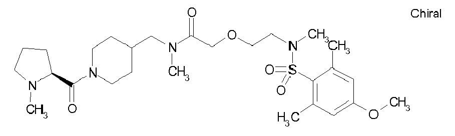 |
| (417) | 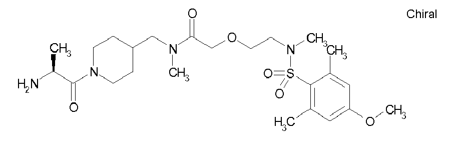 |
| (418) | 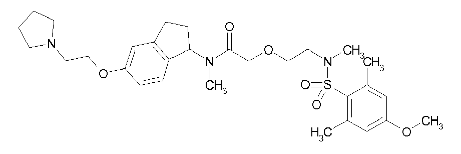 |
| (419) | 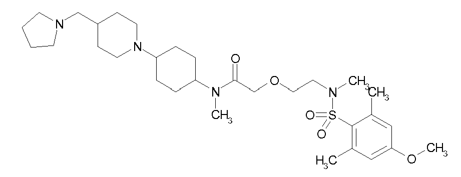 |
| (420) | 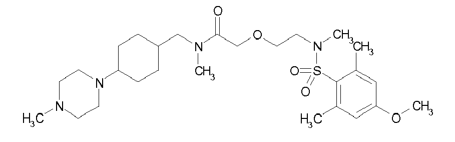 |
| (421) | 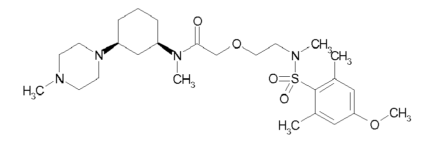 |
| (422) | 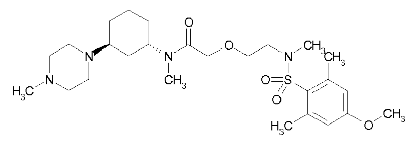 |
| (423) | 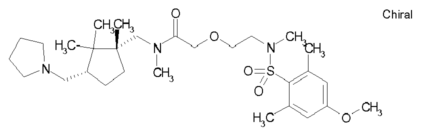 |
| (424) | 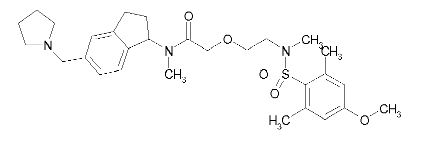 |
| (425) | 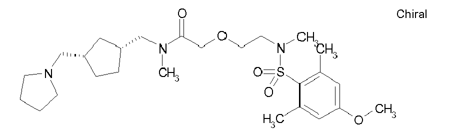 |
| (426) | 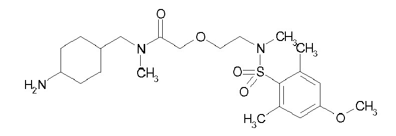 |
| (427) | 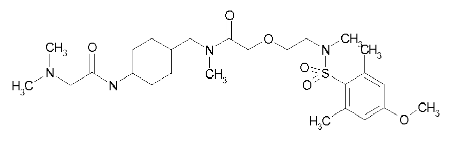 |
| (428) | 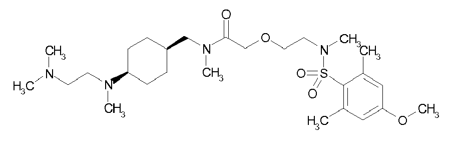 |
| (429) | 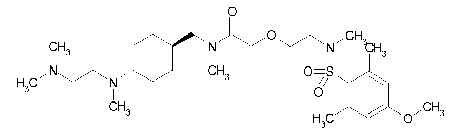 |
| (430) | 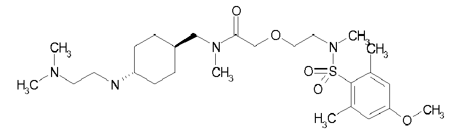 |
| (431) | 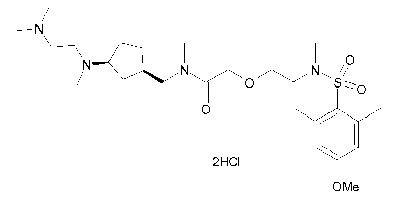 |
| (432) | 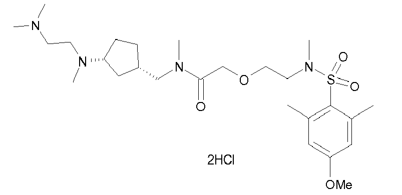 |
| (433) | 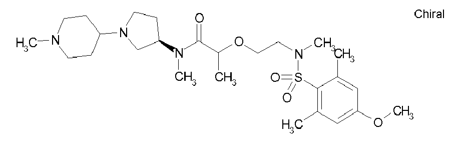 |
| (434) | 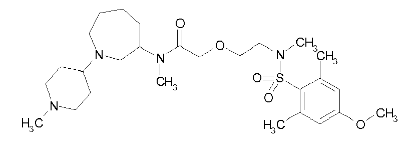 |
| (435) | 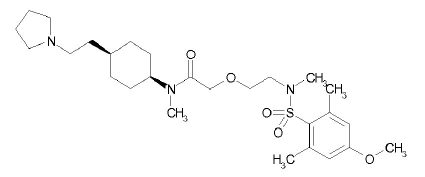 |
| (436) | 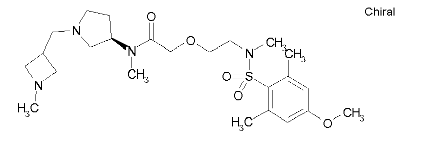 |
| (437) | 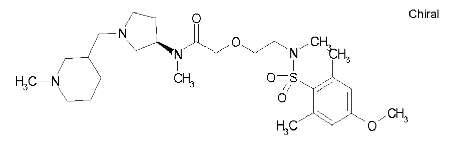 |
| (438) | 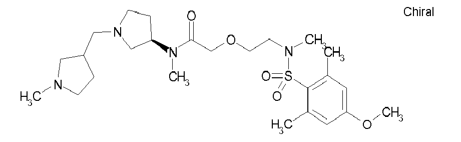 |
| (439) | 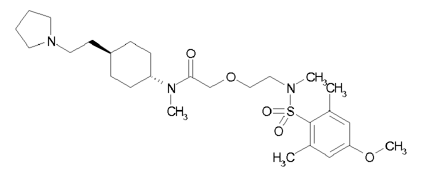 |
| (440) | 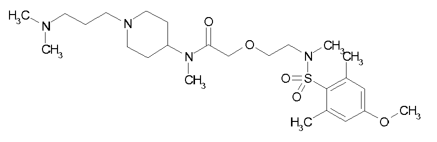 |
| (441) | 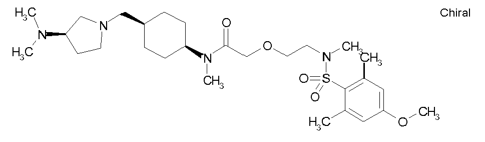 |
| (442) | 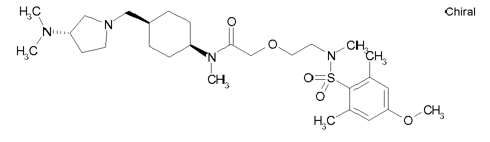 |
| (443) | 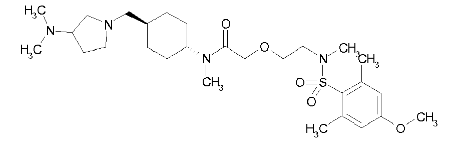 |
| (444) | 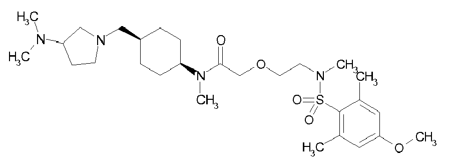 |
| (445) | 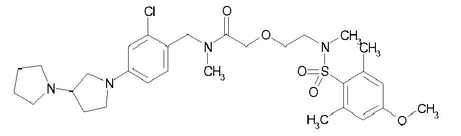 |
| (446) | 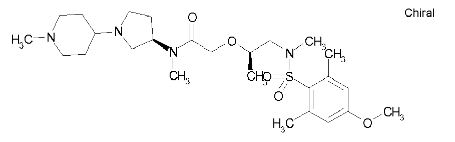 |
| (447) | 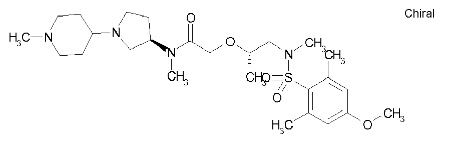 |
| (448) | 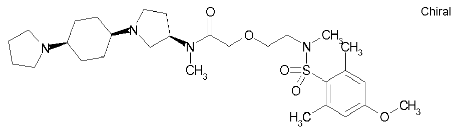 |
| (449) | 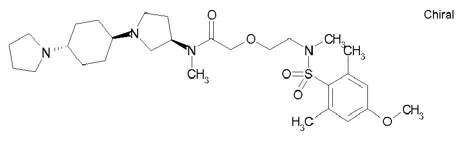 |
| (450) | 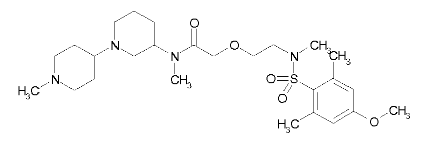 |
| (451) | 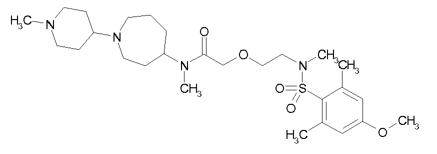 |
| (452) | 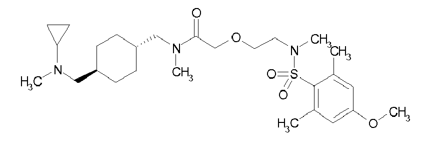 |
| (453) | 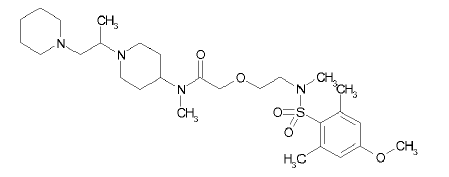 |
| (454) | 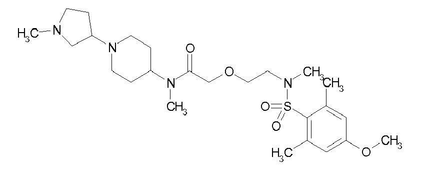 |
| (455) | 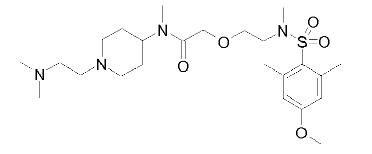 |
| (456) | 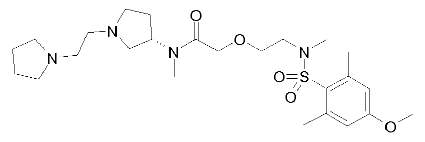 |
| (457) | 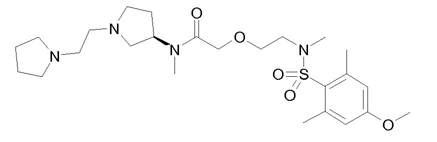 |
| (458) | 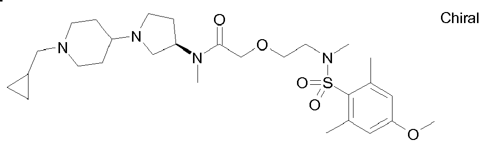 |
| (459) | 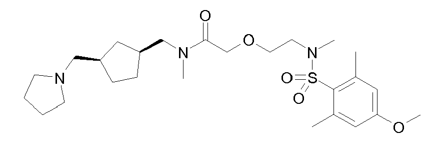 |
| (460) | 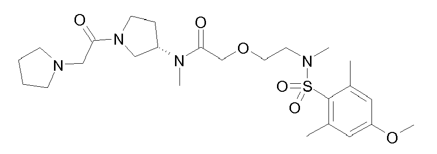 |
| (461) | 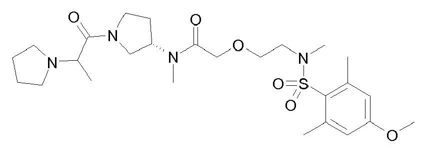 |
| (462) | 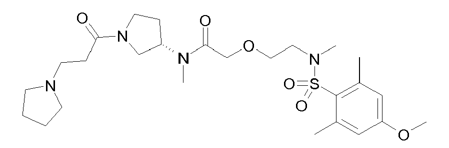 |
| (463) | 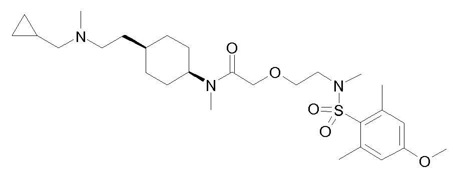 |
| (464) | 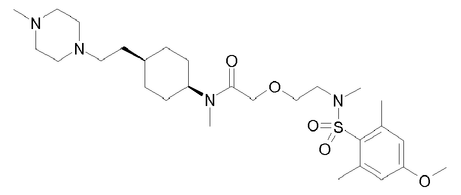 |
| (465) | 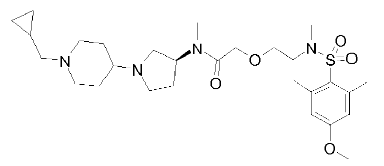 |
| (466) | 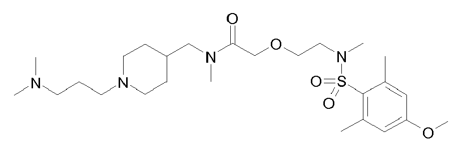 |
| (467) | 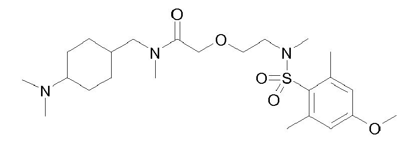 |
| (468) | 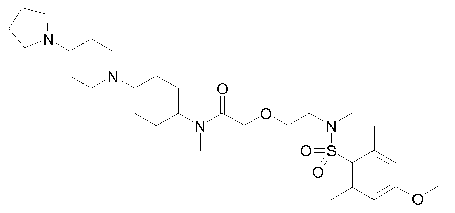 |
| (469) | 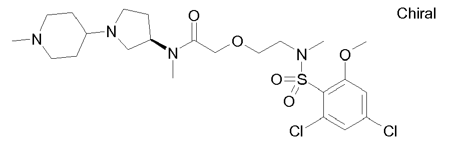 |
| (470) | 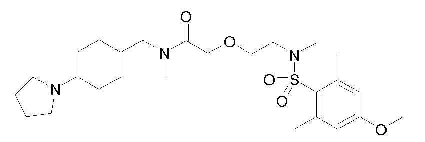 |
| (471) | 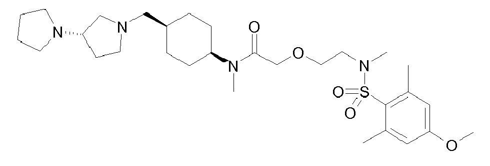 |
| (472) | 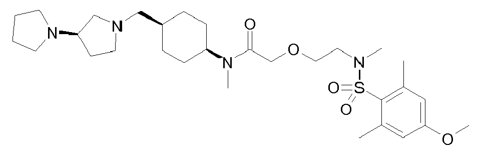 |
| (473) | 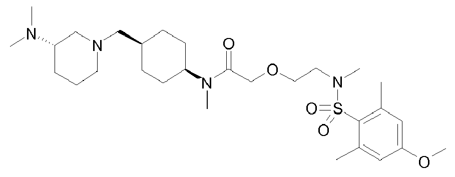 |
| (474) | 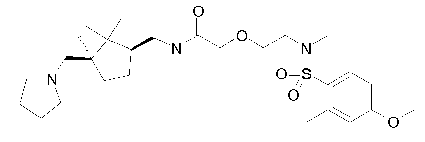 |
| (475) | 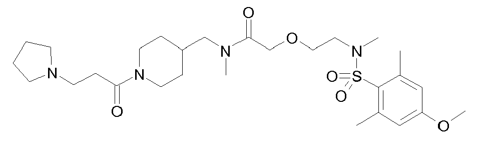 |
| (476) | 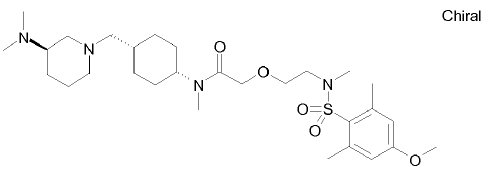 |
| (477) | 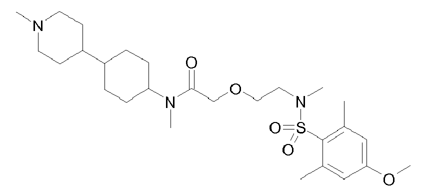 |
| (478) | 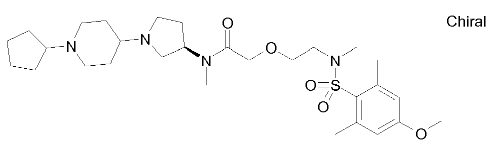 |
| (479) | 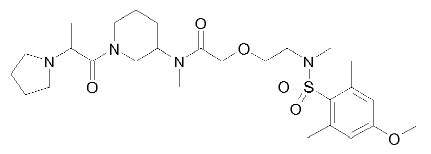 |
| (480) | 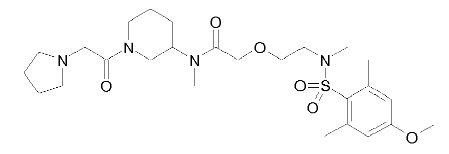 |
| (481) | 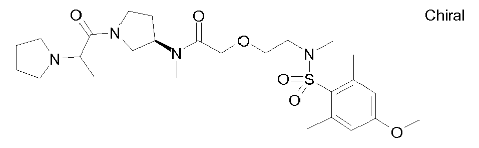 |
| (482) | 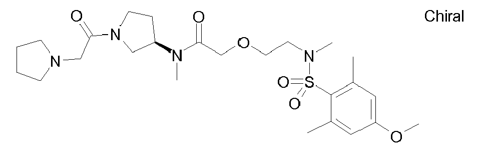 |
| (483) | 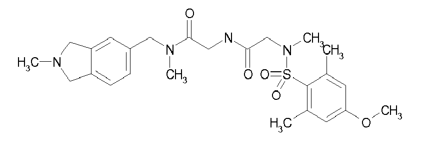 |
| (484) | 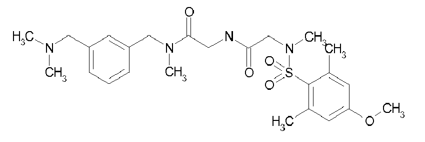 |
| (485) | 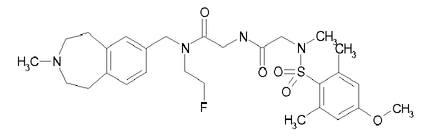 |
| (486) | 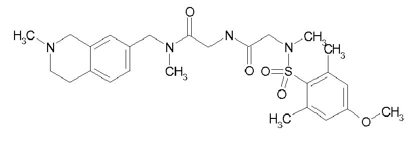 |
| (487) | 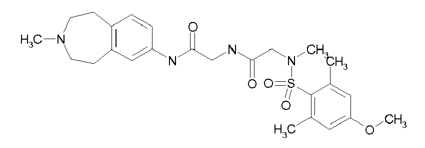 |
| (488) | 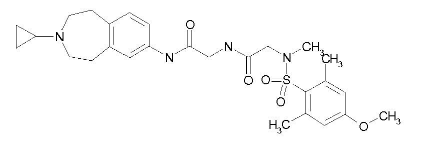 |
| (489) | 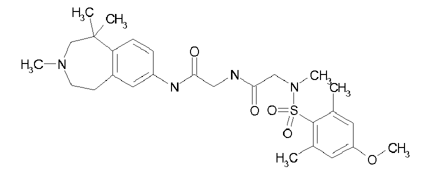 |
| (490) | 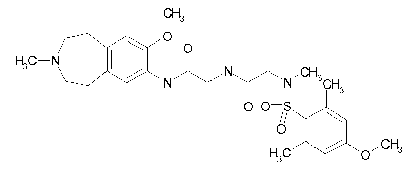 |
| (491) | 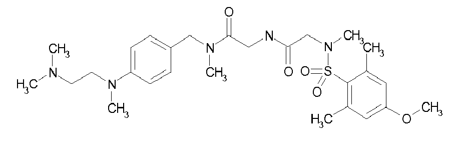 |
| (492) | 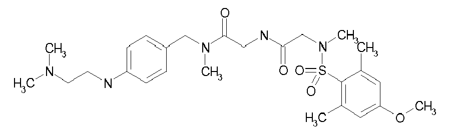 |
| (493) | 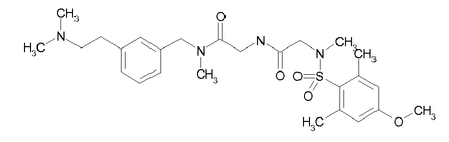 |
| (494) | 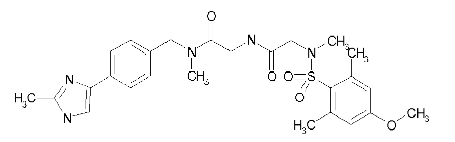 |
| (495) | 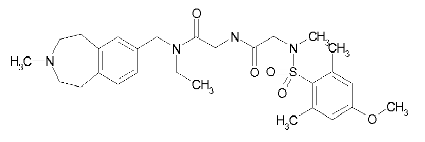 |
| (496) | 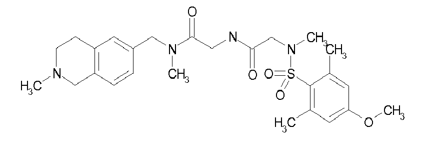 |
| (497) | 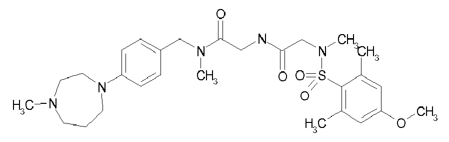 |
| (498) | 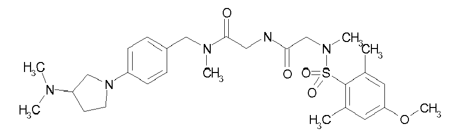 |
| (499) | 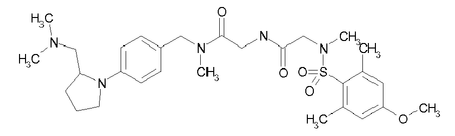 |
| (500) | 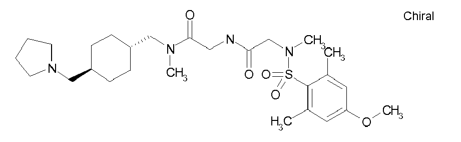 |
| (501) | 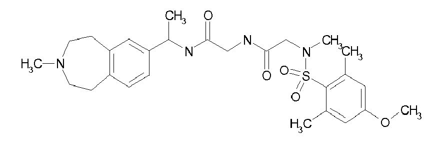 |
| (502) | 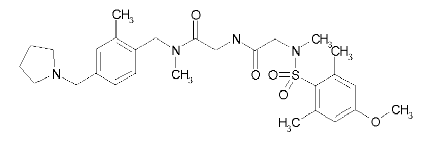 |
| (503) | 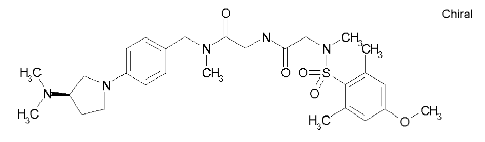 |
| (504) | 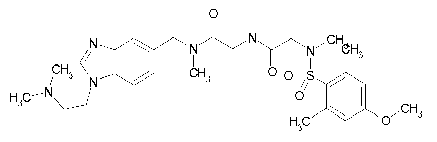 |
| (505) | 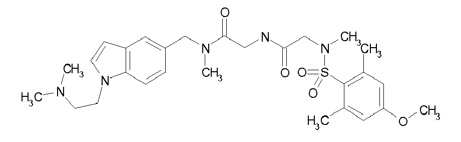 |
| (506) | 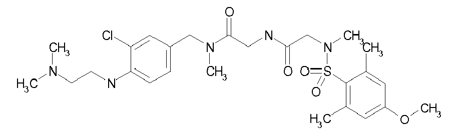 |
| (507) | 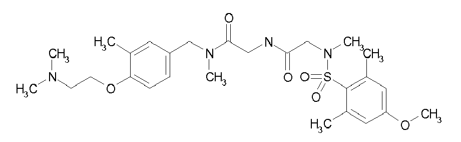 |
| (508) | 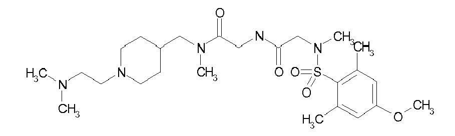 |
| (509) | 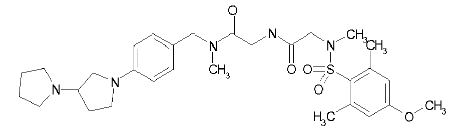 |
| (510) | 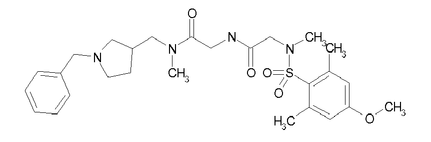 |
| (511) | 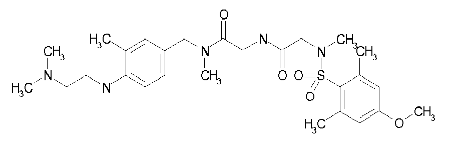 |
| (512) | 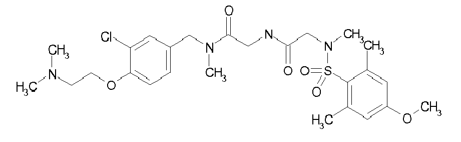 |
| (513) | 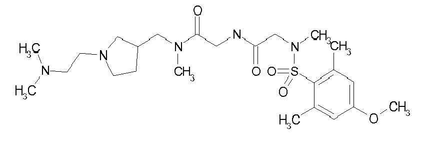 |
| (514) | 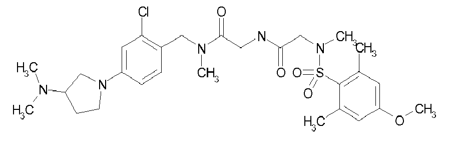 |
| (515) | 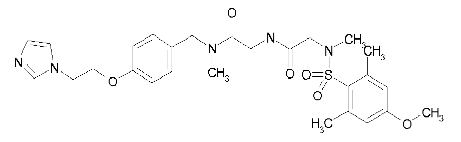 |
| (516) | 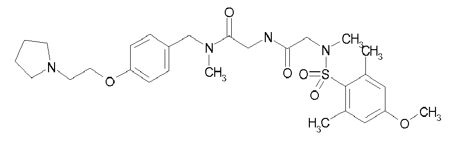 |
| (517) | 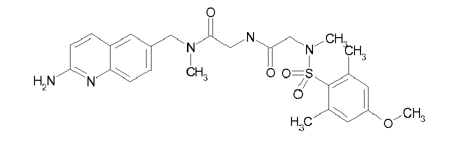 |
| (518) | 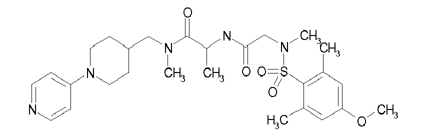 |
| (519) | 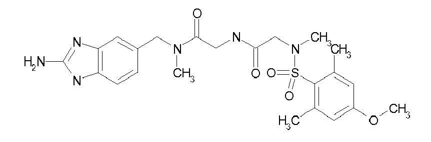 |
| (520) | 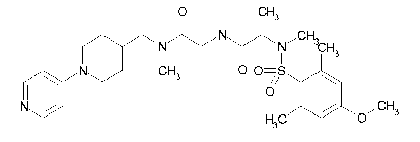 |
| (521) | 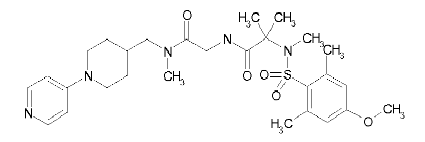 |
| (522) | 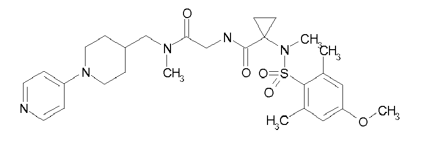 |
| (523) | 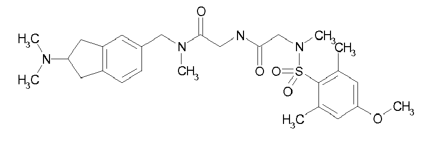 |
| (524) | 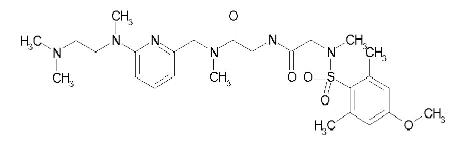 |
| (525) | 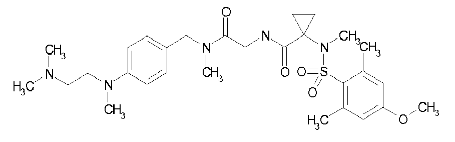 |
| (526) | 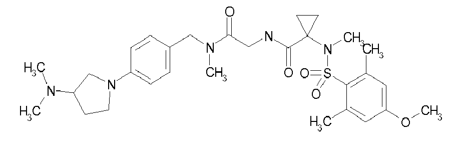 |
| (527) | 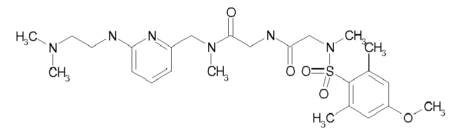 |
| (528) | 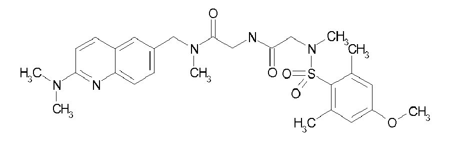 |
| (529) | 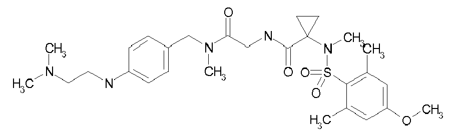 |
| (530) | 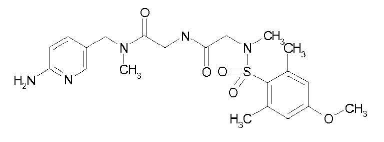 |
| (531) | 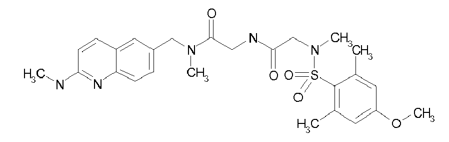 |
| (532) | 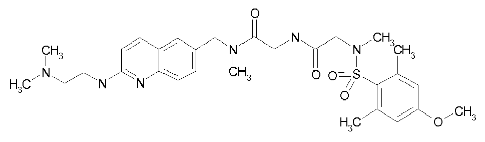 |
| (533) | 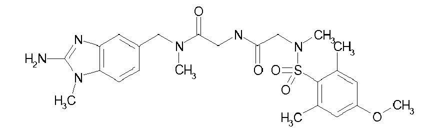 |
| (534) | 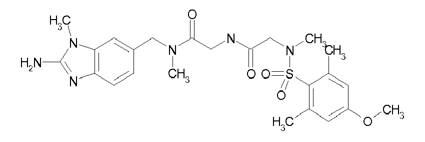 |
| (535) | 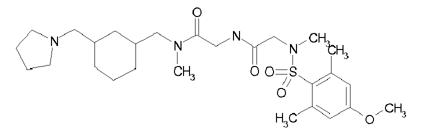 |
| (536) | 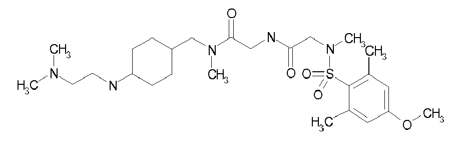 |
| (537) | 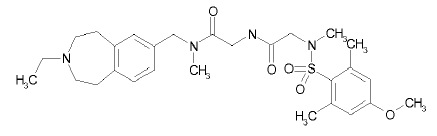 |
| (538) | 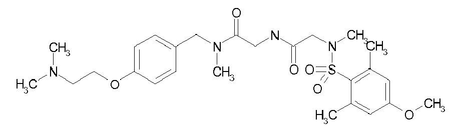 |
| (539) | 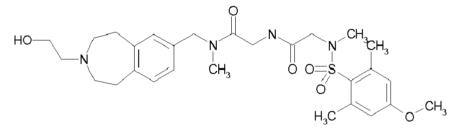 |
| (540) | 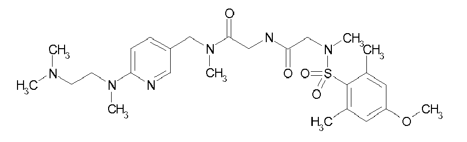 |
| (541) | 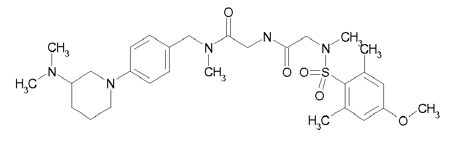 |
| (542) | 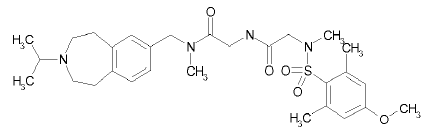 |
| (543) | 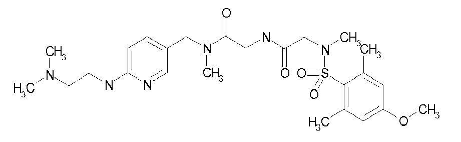 |
| (544) | 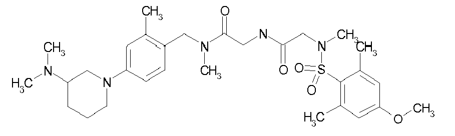 |
| (545) | 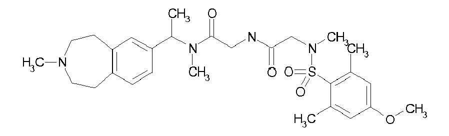 |
| (546) | 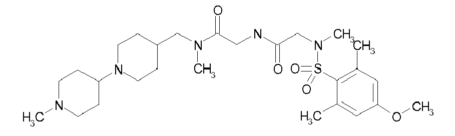 |
| (547) | 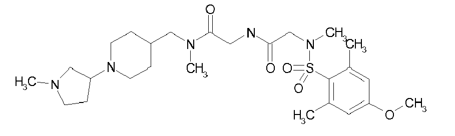 |
| (548) | 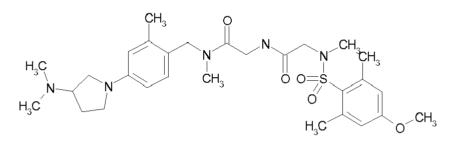 |
| (549) | 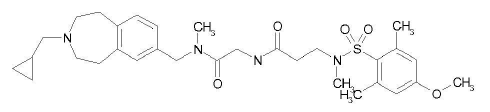 |
| (550) | 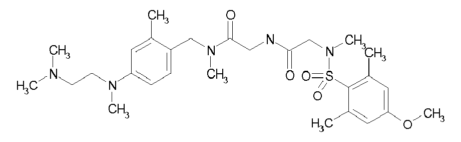 |
| (551) | 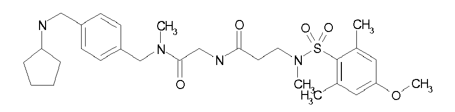 |
| (552) | 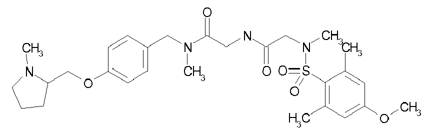 |
| (553) | 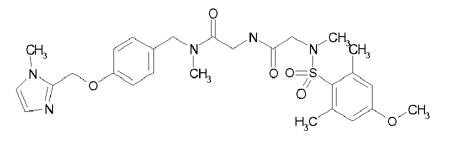 |
| (554) | 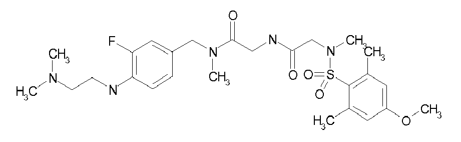 |
| (555) | 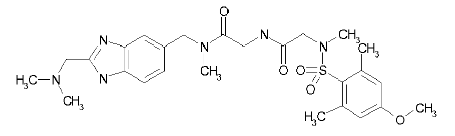 |
| (556) | 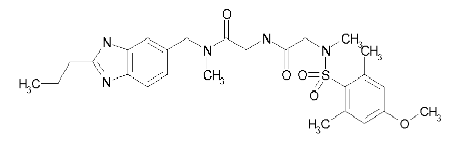 |
| (557) | 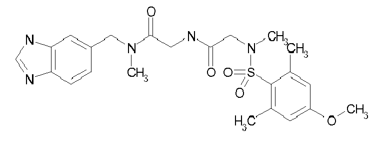 |
| (558) | 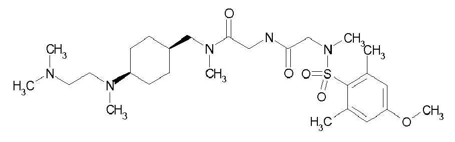 |
| (559) | 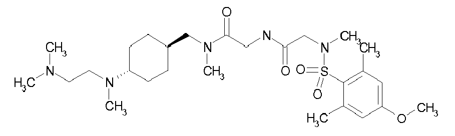 |
| (560) | 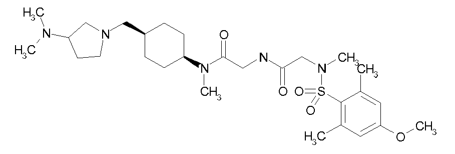 |
| (561) | 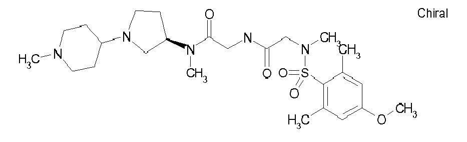 |
| (562) | 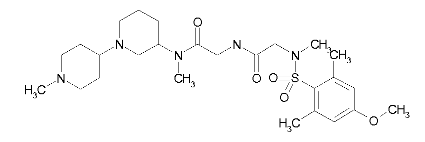 |
| (563) | 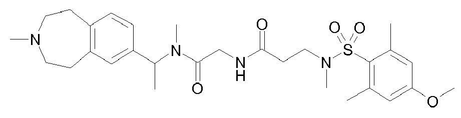 |
| (564) | 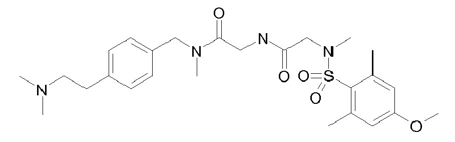 |
| (565) | 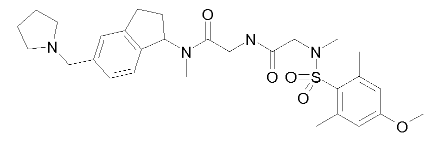 |
| (566) | 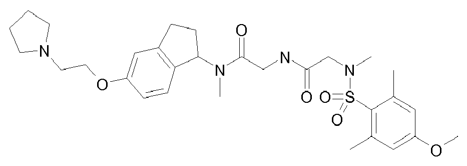 |
| (567) | 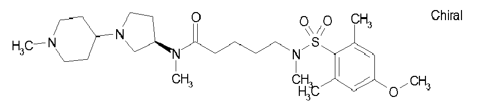 |
| (568) | 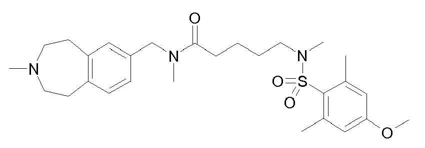 |
| (569) | 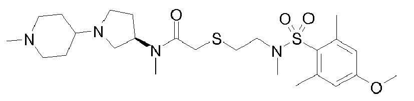 |
| (570) | 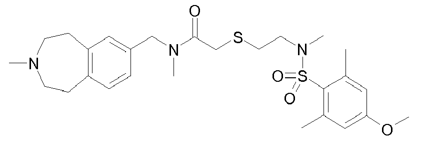 |
| (571) | 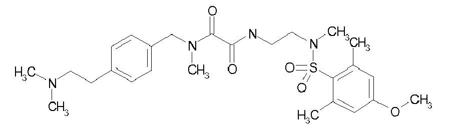 |
| (572) | 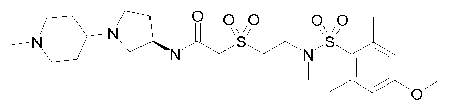 |
| (573) | 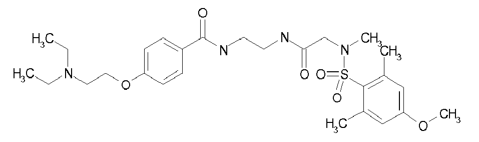 |
| (574) | 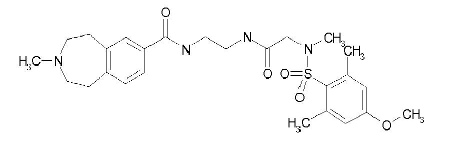 |
| (575) | 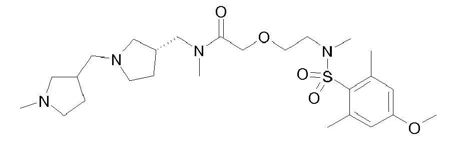 |
| (576) | 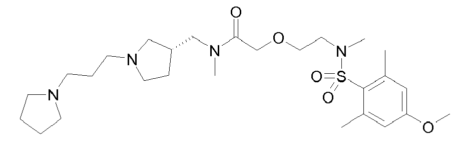 |
| (577) | 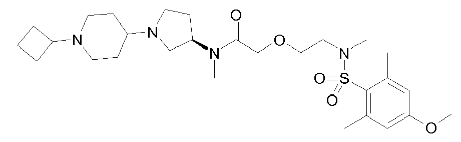 |
| (578) | 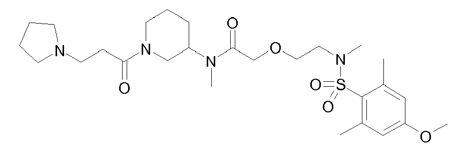 |
| (579) | 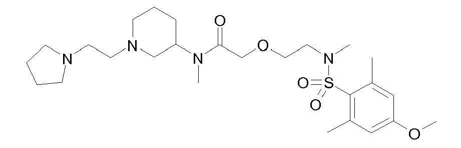 |
| (580) | 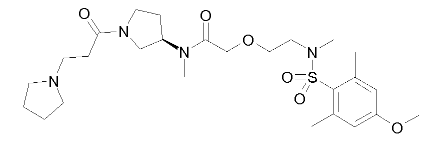 |
| (581) | 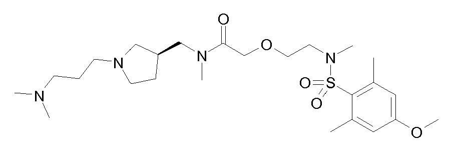 |
| (582) | 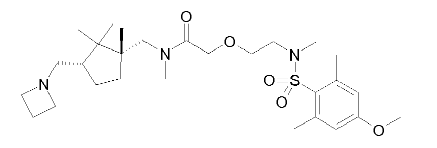 |
| (583) | 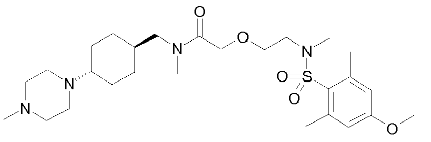 |
| (584) | 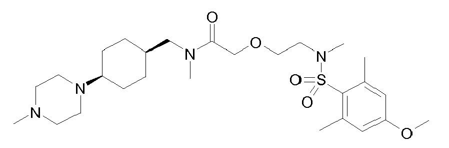 |
| (585) | 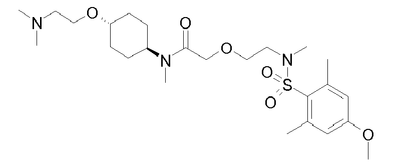 |
| (586) | 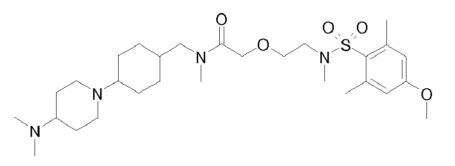 |
| (587) | 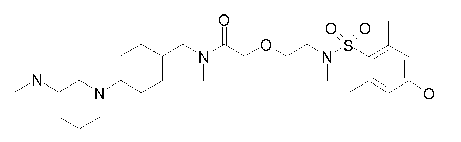 |
| (588) | 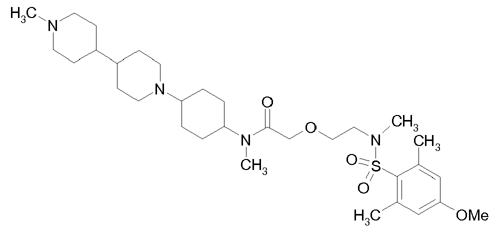 |
| (589) | 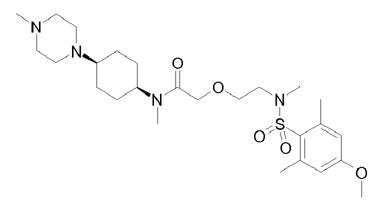 |
| (590) | 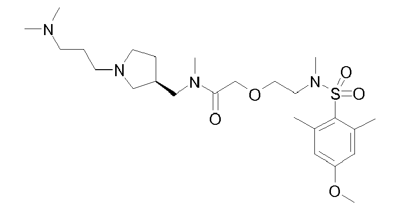 |
| (591) | 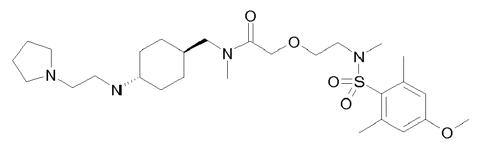 |
| (592) | 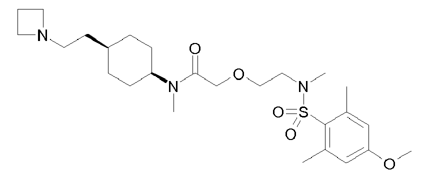 |
| (593) | 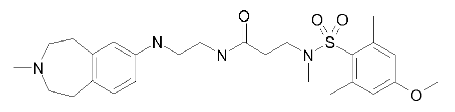 |
| (594) | 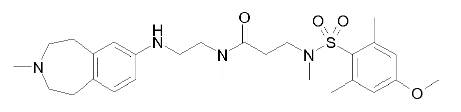 |
| (595) | 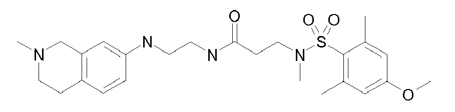 |
| (596) | 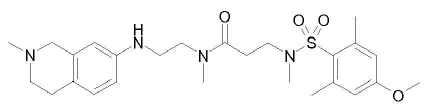 |
| (597) | 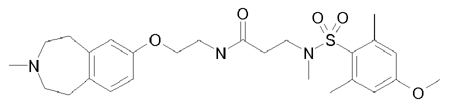 |
| (598) | 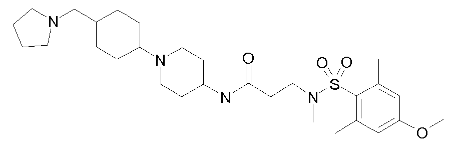 |
| (599) | 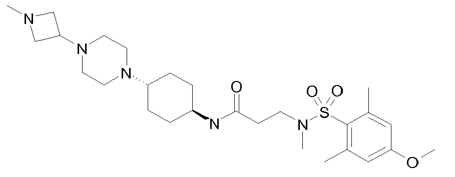 |
| (600) | 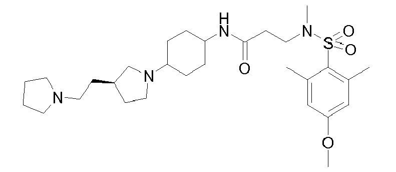 |
| (601) | 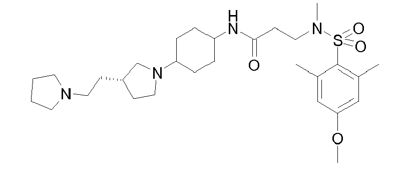 |
| (602) | 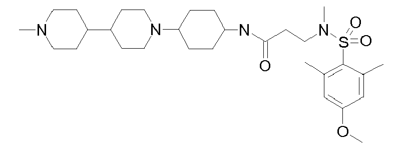 |
| (603) | 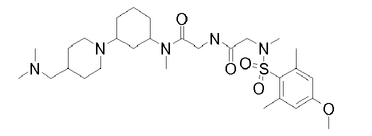 |
| (604) | 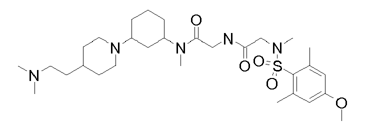 |
| (605) | 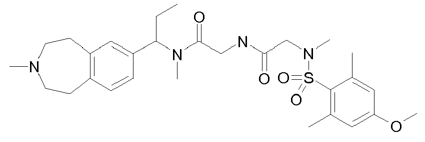 |
| (606) | 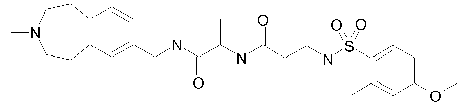 |
| (607) | 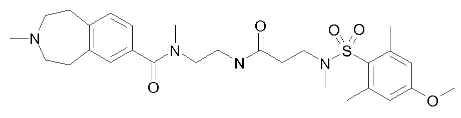 |
| (608) | 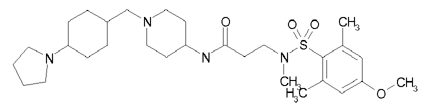 |
| (609) | 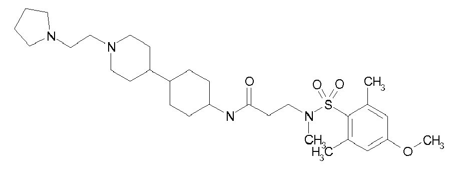 |
| (610) | 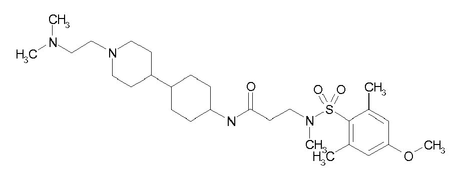 |
| (611) | 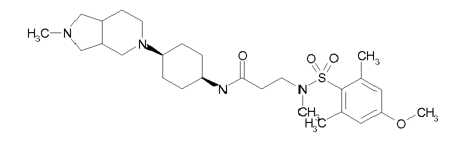 |
| (612) | 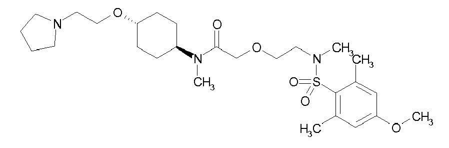 |
| (613) | 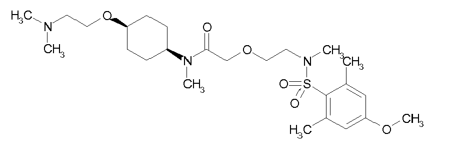 |
| (614) | 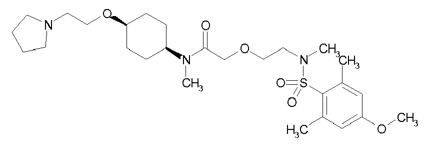 |
| (615) | 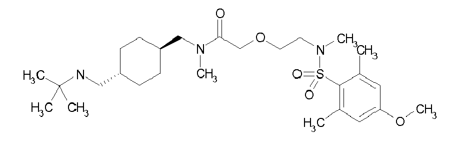 |
| (616) | 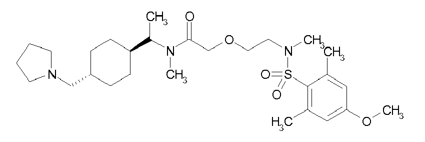 |
| (617) | 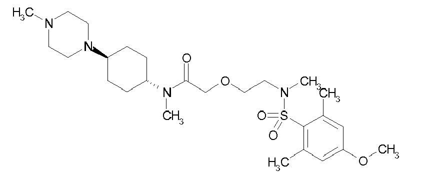 |
| (618) | 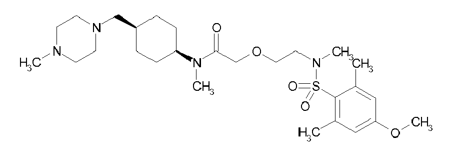 |
| (619) | 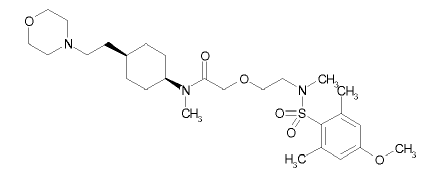 |
| (620) | 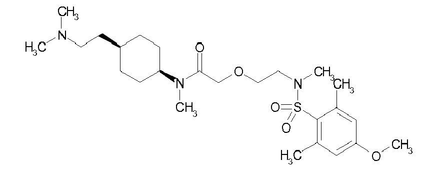 |
| (621) | 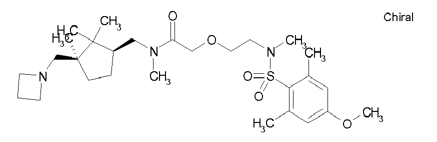 |
| (622) | 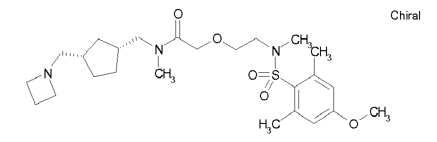 |
| (623) | 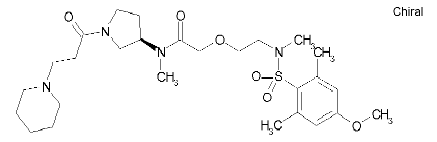 |
| (624) | 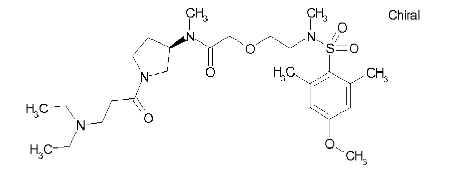 |
| (625) | 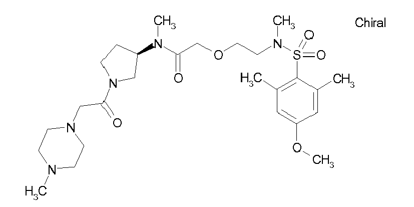 |
| (626) | 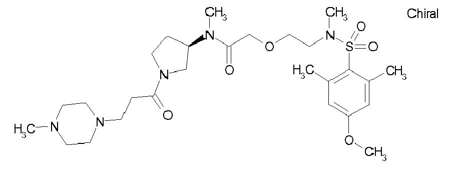 |
| (627) | 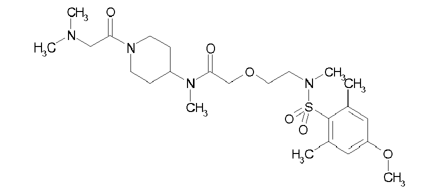 |
| (628) | 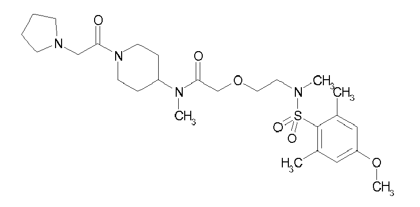 |
| (629) | 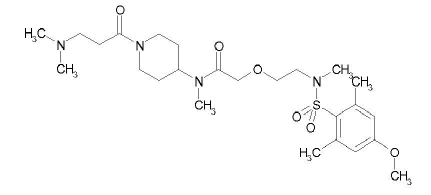 |
| (630) | 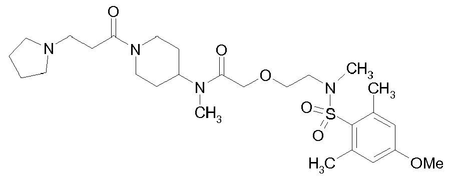 |
| (631) | 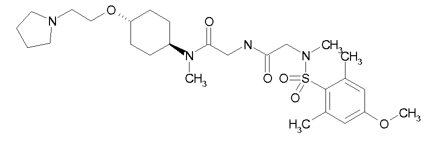 |
| (632) | 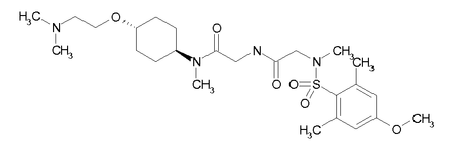 |
| (633) | 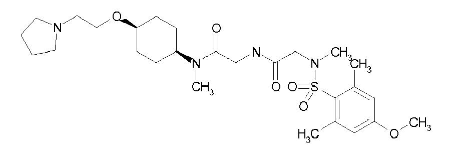 |
| (634) | 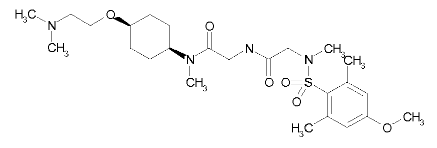 |
| (635) | 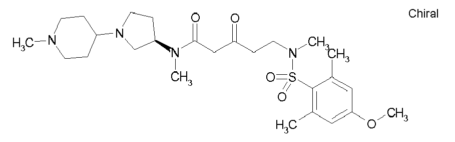 |
| (636) | 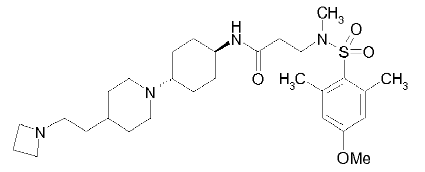 |
| (637) | 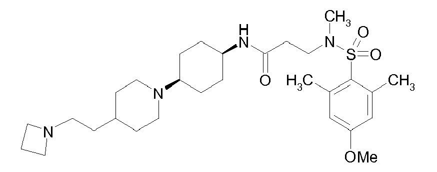 |
| (638) | 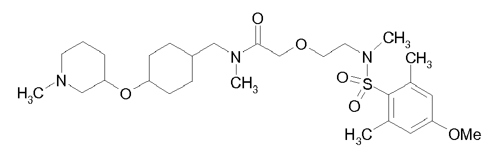 |
| (639) | 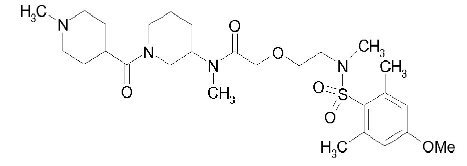 |
| (640) | 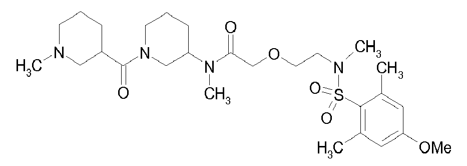 |
| (641) | 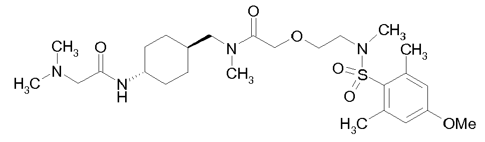 |
| (642) | 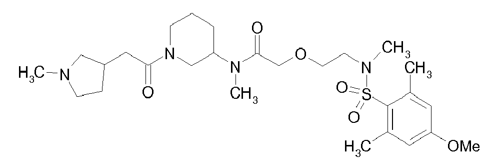 |
| (643) | 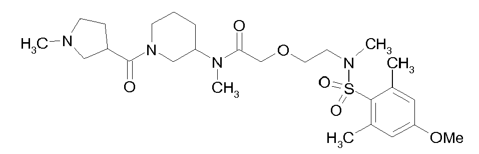 |
| (644) | 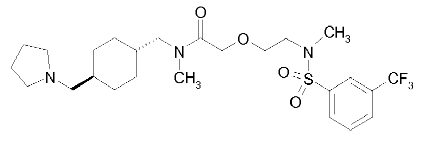 |
| (645) | 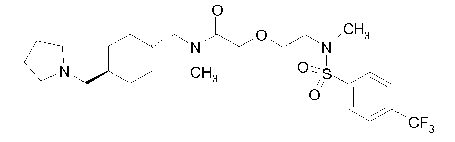 |
| (646) | 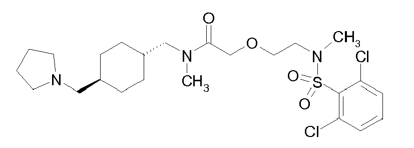 |
| (647) | 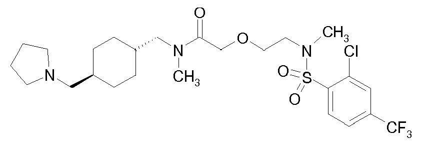 |
| (648) | 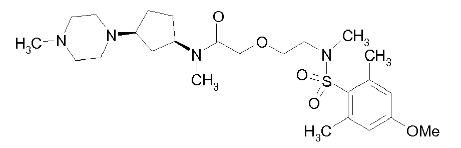 |
| (649) | 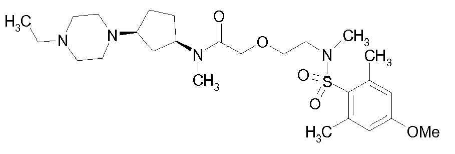 |
| (650) | 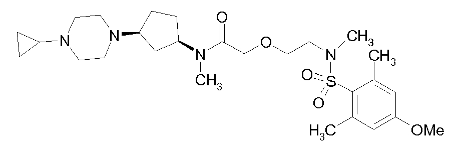 |
| (651) | 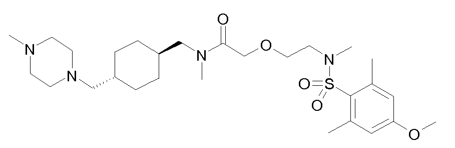 |
| (652) | 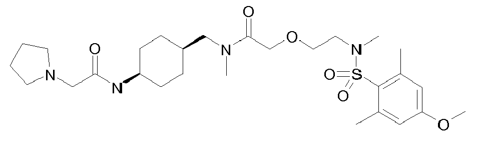 |
| (653) | 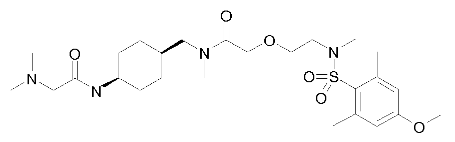 |
| (654) | 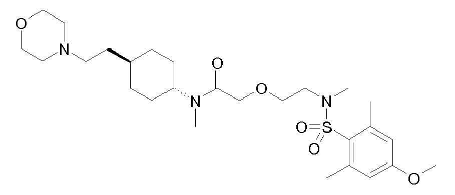 |
| (655) | 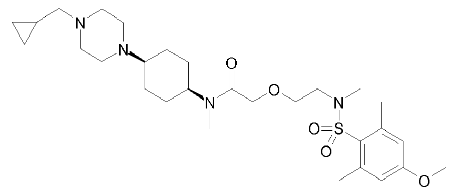 |
| (656) | 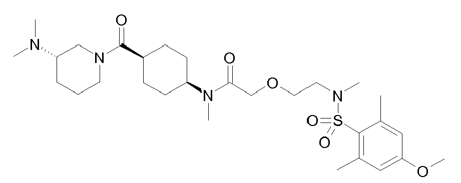 |
| (657) | 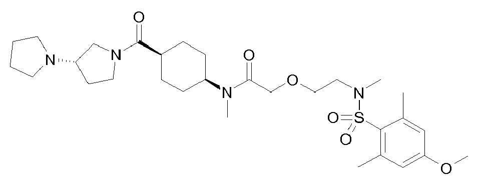 |
| (658) | 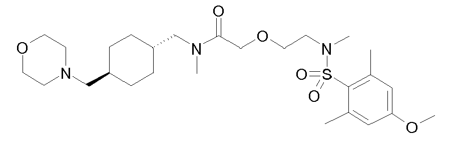 |
| (659) | 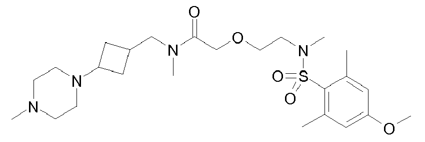 |
| (660) | 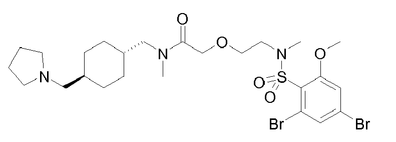 |
| (661) | 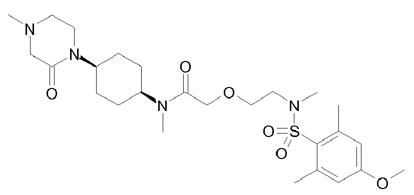 |
| (662) | 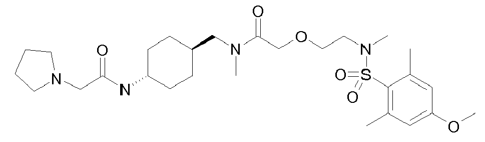 |
| (663) | 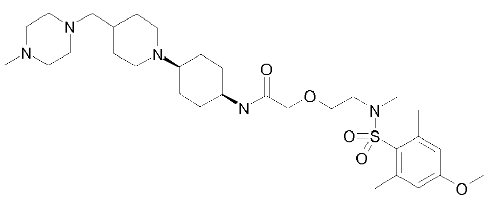 |
| (664) | 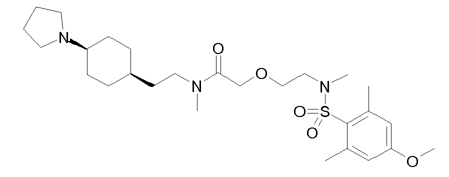 |
| (665) | 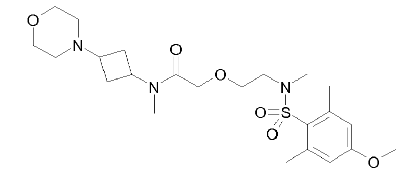 |
| (666) | 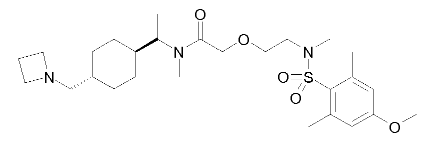 |
| (667) | 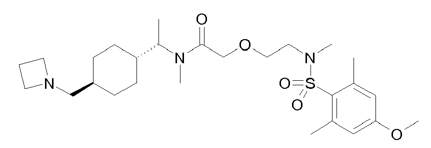 |
| (668) | 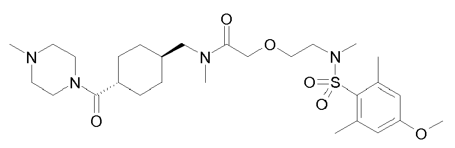 |
| (669) | 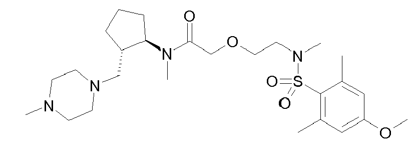 |
| (670) | 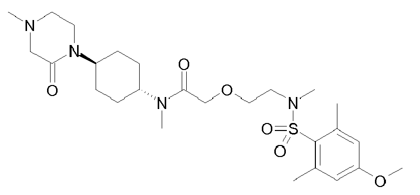 |
| (671) | 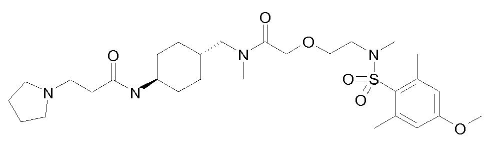 |
| (672) | 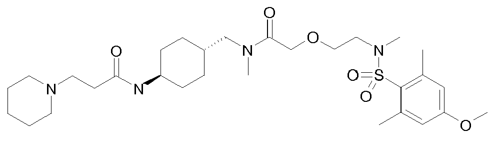 |
| (673) | 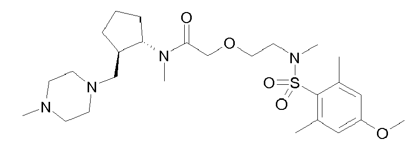 |
| (674) | 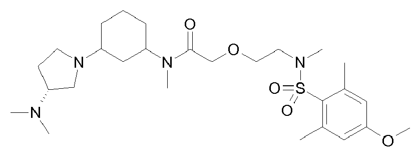 |
| (675) | 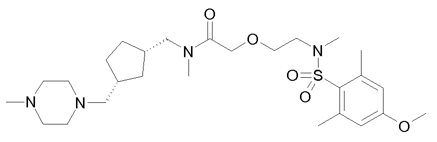 |
| (676) | 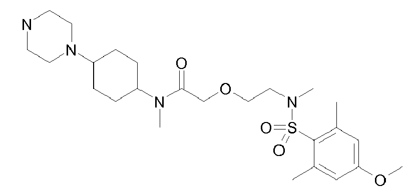 |
| (677) | 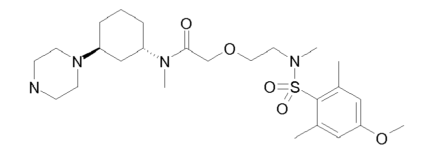 |
| (678) | 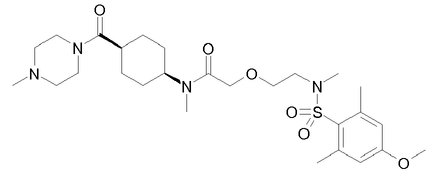 |
| (679) | 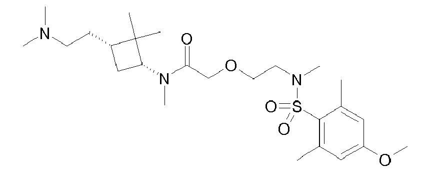 |
| (680) | 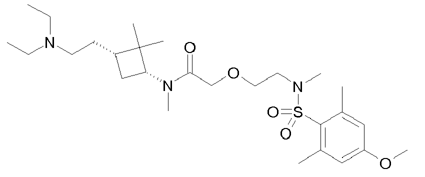 |
| (681) | 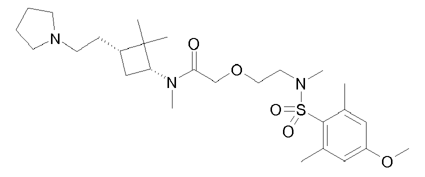 |
| (682) | 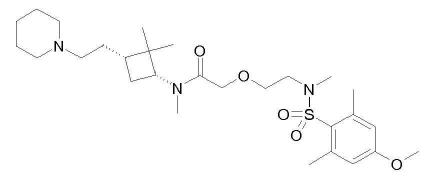 |
| (683) | 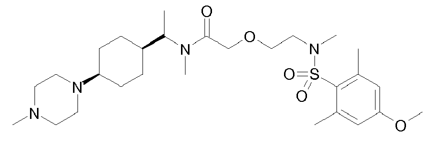 |
| (684) | 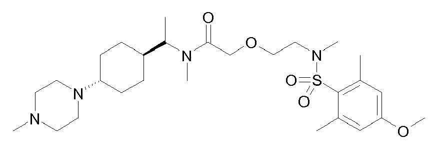 |
| (685) | 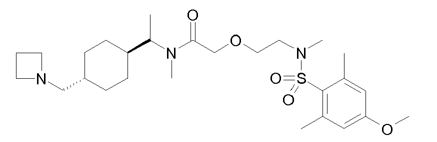 |
| (686) | 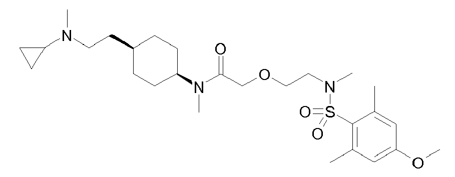 |
| (687) | 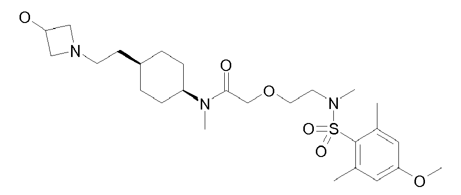 |
| (688) | 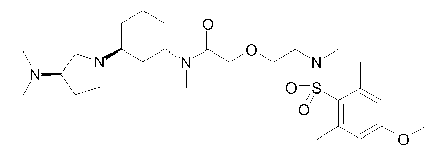 |
| (689) | 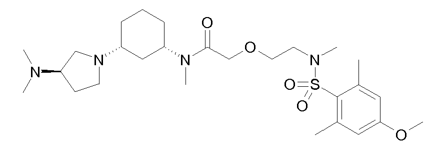 |
| (690) | 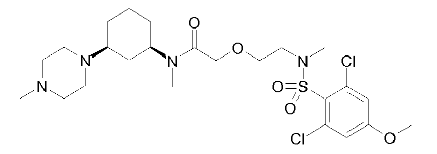 |
| (691) | 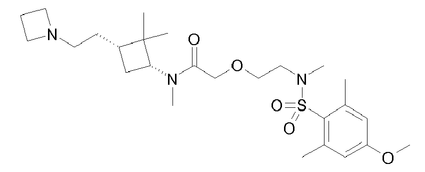 |
| (692) | 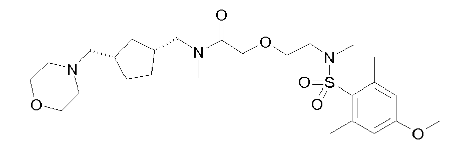 |
| (693) | 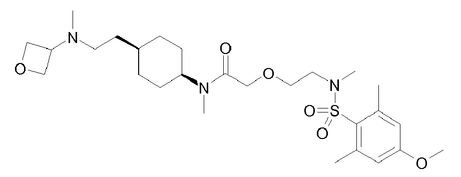 |
| (694) | 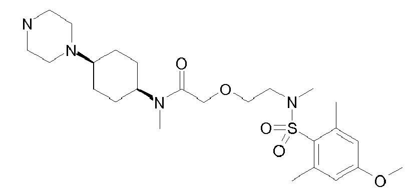 |
| (695) | 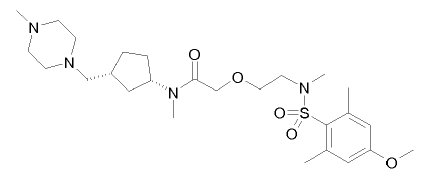 |
| (696) | 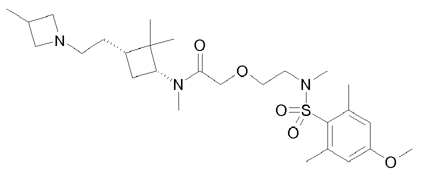 |
| (697) | 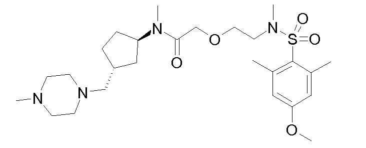 |
| (698) | 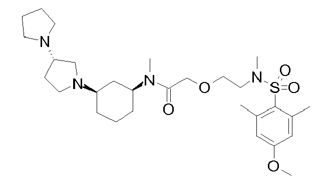 |
| (699) | 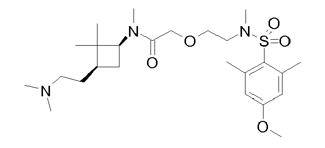 |
| (700) | 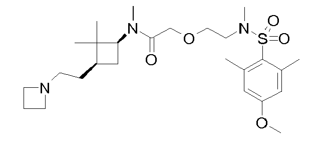 |
| (701) | 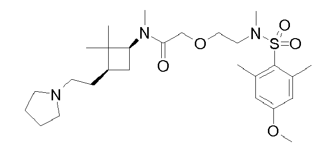 |
| (702) | 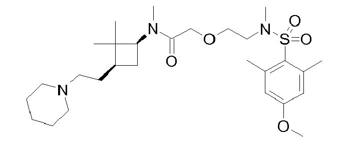 |
| (703) | 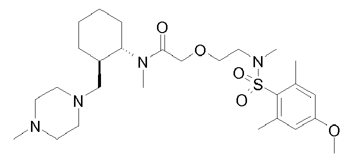 |
| (704) | 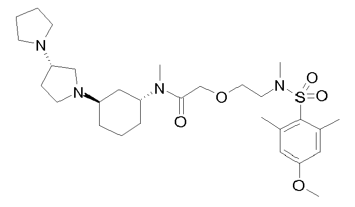 |
| (705) | 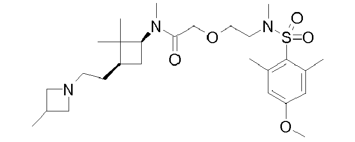 |
| (705) | 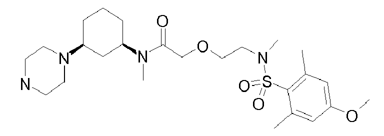 |
| (707) | 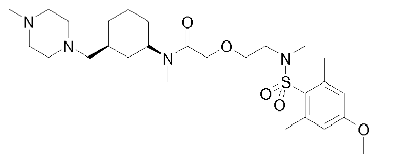 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Soweit nicht anders angegeben, sind alle Substituenten voneinander unabhängig. Sollten an einer Gruppe z.B. mehrere C₁₋₆-Alkylgruppen als Substituenten sein, so könnte im Fall von drei Substituenten C₁₋₆-Alkyl unabhängig voneinander einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₂-Alkyl" (auch soweit sie Bestandteil anderer Reste sind) werden Alkylgruppen mit 1 bis 2 Kohlenstoffatomen, unter dem Begriff "C₁₋₃-Alkyl" verzweigte und unverzweigte Alkylgruppen mit 1 bis 3 Kohlenstoffatomen, unter dem Begriff "C₁₋₄-Alkyl" verzweigte und unverzweigte Alkylgruppen mit 1 bis 4 Kohlenstoffatomen, unter dem Begriff "C₁₋₅-Alkyl" verzweigte und unverzweigte Alkylgruppen mit 1 bis 5 Kohlenstoffatomen, unter dem Begriff "C₁₋₆-Alkyl" verzweigte und unverzweigte Alkylgruppen mit 1 bis 6 Kohlenstoffatomen und unter dem Begriff "C₁₋₈-Alkyl" verzweigte und unverzweigte Alkylgruppen mit 1 bis 8 Kohlenstoffatomen verstanden. Beispielsweise werden hierfür genannt: Methyl, Ethyl, *n*-Propyl, *iso*-Propyl, *n*-Butyl, *iso*-Butyl, sec-Butyl, *tert*-Butyl, *n*-Pentyl, *iso*-Pentyl, *neo*-Pentyl, Hexyl, Heptyl und Octyl. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl, Hexyl, Heptyl und Octyl alle denkbaren isomeren Formen der jeweiligen Reste. So umfasst beispielsweise Propyl *n*-Propyl und *iso*-Propyl, Butyl umfasst *iso*-Butyl, sec-Butyl und *tert*-Butyl etc..
Weiterhin umfassen die voranstehend genannten Begriffe auch solche Reste, in denen jede Methylengruppe mit bis zu zwei und jede Methylgruppe mit bis zu drei Fluoratomen substituiert sein kann.

Unter dem Begriff "C₁₋₂-Alkylen" werden verzweigte und unverzweigte Alkylengruppen mit 1 oder 2 Kohlenstoffatomen, unter dem Begriff "C₁₋₃-Alkylen" (auch soweit sie Bestandteil anderer Reste sind) verzweigte und unverzweigte Alkylengruppen mit 1 bis 3 Kohlenstoffatomen, unter dem Begriff "C₁₋₄-Alkylen" verzweigte und unverzweigte Alkylengruppen mit 1 bis 4 Kohlenstoffatomen, unter dem Begriff "C₁₋₆-Alkylen" verzweigte und unverzweigte Alkylengruppen mit 1 bis 6 Kohlenstoffatomen und unter dem Begriff "C₂₋₄-Alkylen" verzweigte und unverzweigte Alkylengruppen mit 2 bis 4 Kohlenstoffatomen verstanden. Beispielsweise werden hierfür genannt: Methylen, Ethylen, Ethan-1,1-diyl, Propylen, Propan-2,2-diyl, 1-Methylethylen, Butylen, 1-Methylpropylen, 1,1-Dimethylethylen, 1,2-Dimethylethylen. Sofern nicht anders beschrieben, umfassen die Definitionen Propylen und Butylen alle denkbaren isomeren Formen der gleichen Kohlenstoffanzahl. So umfasst beispielsweise Propylen auch 1-Methylethylen und Butylen umfasst 1-Methylpropylen, 1,1-Dimethylethylen, 1,2-Dimethylethylen.
Weiterhin umfassen die voranstehend genannten Begriffe auch solche Reste, in denen jede Methylengruppe mit bis zu zwei Fluoratomen substituiert sein kann.

Unter dem Begriff "C₃₋₅-Cycloalkyl" werden cyclische Alkylgruppen mit 3 bis 5 Kohlenstoffatomen, unter dem Begriff "C₃₋₆-Cycloalkyl" werden cyclische Alkylgruppen mit 3 bis 6 Kohlenstoffatomen und unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₃₋₆-Cycloalkylen" (auch soweit sie Bestandteil anderer Reste sind) werden cyclische Alkylengruppen mit 3 bis 6 Kohlenstoffatomen, unter dem Begriff "C₃₋₇-Cycloalkylen" cyclische Alkylengruppen mit 3 bis 7 Kohlenstoffatomen und unter dem Begriff "C₄₋₆-Cycloalkylen" cyclische Alkylengruppen mit 4 bis 6 Kohlenstoffatomen verstanden. Beispielsweise werden hierfür genannt: Cyclopropylen, Cyclobutylen, Cyclopentylen, Cyclohexylen oder Cycloheptylen. Soweit nicht anders beschrieben, können die cyclischen Alkylengruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Eine C₄- oder eine C₅-Cycloalkylengruppe kann in 1,2-Stellung oder in 1,3-Stellung mit dem restlichen Molekül verknpüft sein, vorzugsweise in 1,3-Stellung. Eine C₆- oder eine C₇-Cycloalkylengruppe kann in 1,2-Stellung, in 1,3- Stellung oder in 1,4-Stellung mit dem restlichen Molekül verknüpft sein, vorzugsweise in 1,3-Stellung.

Unter dem Begriff "C₅₋₇-Cycloalkenylen" (auch soweit sie Bestandteil anderer Reste sind) werden cyclische Alkenylgruppen mit 5 bis 7 Kohlenstoffatomen verstanden, die eine ungesättigte Bindung enthalten und die über diese ungesättigte Bindung mit einem Phenylring kondensiert sind. Beispielsweise werden hierfür genannt: Cyclopentenyl, Cyclohexenyl oder Cyclohepentyl:

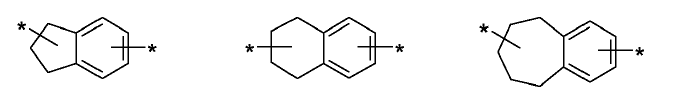

Soweit nicht anders beschrieben, können die cyclischen Alkenylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "gesättigte heterocyclische Ringe" werden vier-, fünf-, sechs- oder siebengliedrige heterocyclische Ringe verstanden, die ein, zwei oder drei Heteroatome, ausgewählt aus der Gruppe Sauerstoff, Schwefel und Stickstoff enthalten können. Dabei kann der Ring über ein Kohlenstoffatom und/oder-falls vorhanden- über ein Stickstoffatom oder auch über zwei Kohlenstoffatome oder über zwei Stickstoffatome mit dem Molekül verknüpft sein. Obwohl unter dem Begriff "heterocyclische Ringe" umfasst, definiert der Begriff "heterocyclische, nichtaromatische Ringe" fünf-, sechs- oder siebengliedrige gesättigte Ringe. Als Beispiele werden genannt:

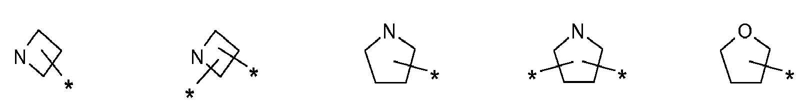

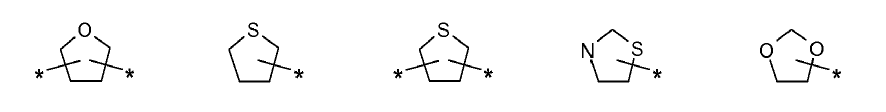

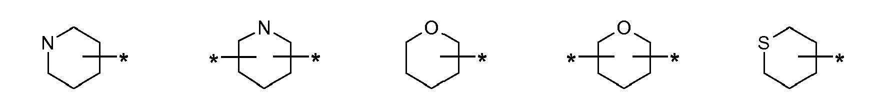

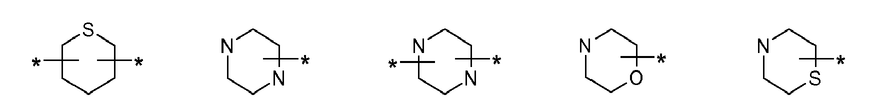

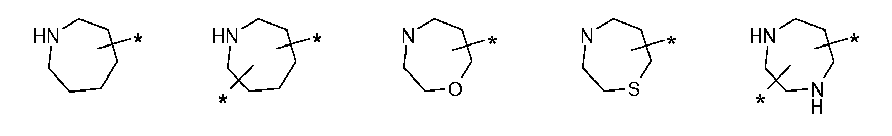

Unter dem Begriff "gesättigte Diaza-Heterocyclen" werden sechs- oder siebengliedrige heterocyclische Ringe verstanden, die zwei Stickstoffatome enthalten. Dabei ist der Ring über beide Stickstoffatome mit dem restlichen Molekül verknüpft. Als Beispiele werden genannt:

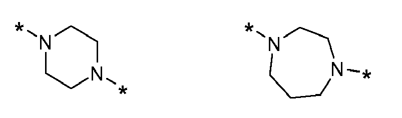

Unter dem Begriff "gesättigte Aza-Heterobicyclen" werden acht-, neun- oder zehngliedrige heterobicyclische Ringe verstanden, die ein Stickstoffatom enthalten. Dabei ist der Ring über ein Kohlenstoffatom und das Stickstoffatom mit dem restlichen Molekül verknüpft. Als Beispiele werden genannt:

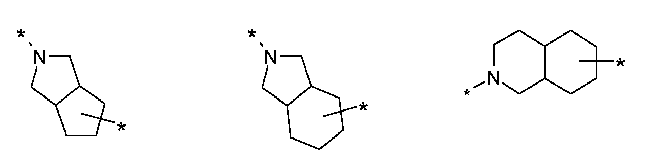

Unter dem Begriff "gesättigte Diaza-Heterobicyclen" werden acht-, neun- oder zehngliedrige heterobicyclische Ringe verstanden, die zwei Stickstoffatom enthalten. Dabei ist der Ring über beide Stickstoffatome mit dem restlichen Molekül verknüpft. Als Beispiele werden genannt:

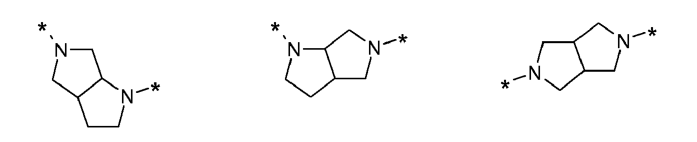

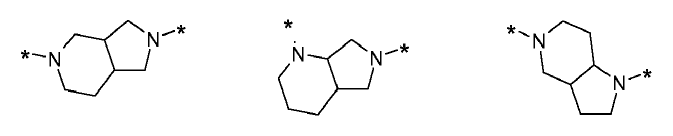

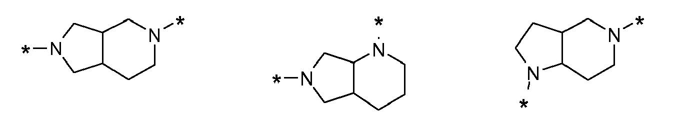

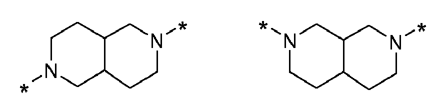

Unter dem Begriff "ungesättigte heterocyclische Ringe" werden fünf-, sechs- oder siebengliedrige, einfach oder zweifach ungesättigte heterocyclische Ringe verstanden, die ein, zwei oder drei Heteroatome, ausgewählt aus der Gruppe Sauerstoff, Schwefel und Stickstoff enthalten können und die über die ungesättigten Bindungen mit einem oder zwei Phenylringen kondensiert sind. Dabei kann der heterocyclische Ring über ein Kohlenstoffatom und/oder -falls vorhanden- über ein Stickstoffatom oder über zwei Kohlenstoffatome oder über zwei Stickstoffatome mit dem Molekül verknüpft sein. Als Beispiele werden genannt:

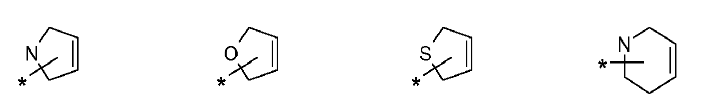

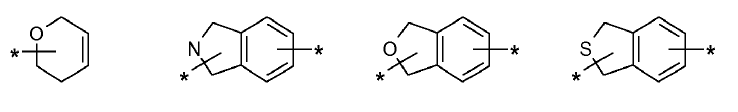

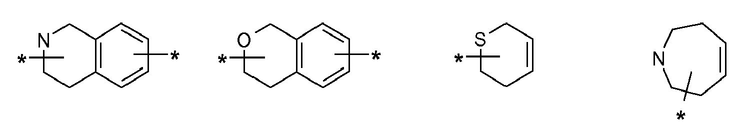

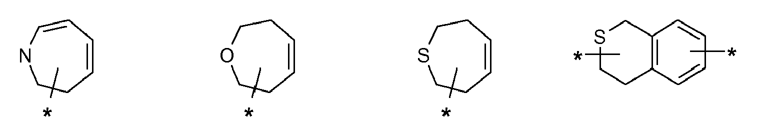

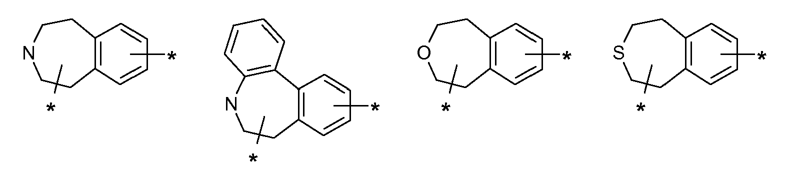

Unter dem Begriff "gesättigte Diaza-Spirocyclen" werden neun-, zehn- oder elfgliedrige spirocyclische Ringe verstanden, die zwei Stickstoffatome enthalten. Dabei ist der Spirocyclus über beide Stickstoffatome mit dem restlichen Molekül verknüpft. Als Beispiele werden genannt:

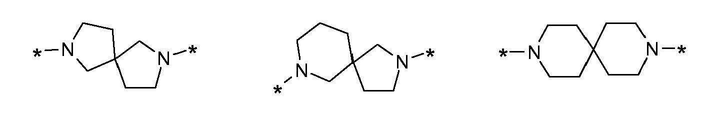

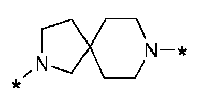

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür Phenyl, 1-Naphthyl oder 2-Naphthyl genannt; bevorzugte Arylreste sind Phenyl und 1-Naphthyl; besonders bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, *n*-Propyl, *iso*-Propyl, *tert*-Butyl, Hydroxy, Methoxy, Trifuormethoxy, Fluor, Chlor, Brom und Jod, wobei die Reste gleich oder verschieden sein können.

Unter dem Begriff "Heteroaryl" werden fünf- oder sechsgliedrige heterocyclische Aromaten oder 9-11 gliedrige bicyclische Heteroarylringe verstanden, die ein, zwei oder drei Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Als Beispiele für fünf- oder sechsgliedrige heterocyclische Aromaten, werden genannt:

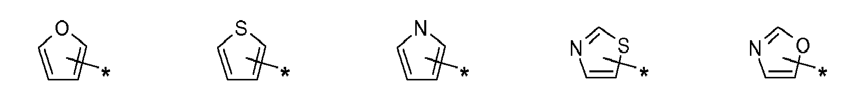

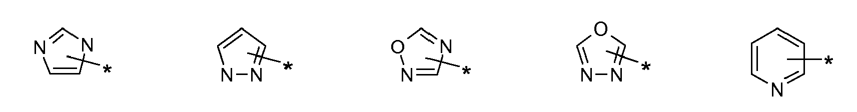

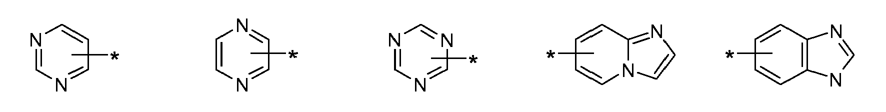

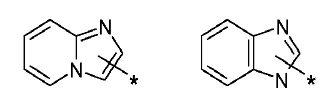

Soweit nicht anders beschrieben, können die voranstehend genannten Heteroaryle substituiert sein mit einem oder mehreren Resten ausgewählt aus der Gruppe bestehend aus Methyl, Ethyl, *n*-Propyl, *iso*-Propyl, *tert*-Butyl, Hydroxy, Methoxy, Trifuormethoxy, Fluor, Chlor, Brom und Jod, wobei die Reste gleich oder verschieden sein können. Bicyclische Heteroarylringe können dabei vorzugsweise im Phenylrest substituiert sein.

Unter dem Begriff "Arylen" (auch soweit sie Bestandteil anderer Reste sind) werden aromatische Ringsysteme mit 6 oder 10 Kohlenstoffatomen verstanden. Beispielsweise werden hierfür genannt: Phenylen, 1-Naphthylen oder 2-Naphthylen, bevorzugter Arylenrest ist Phenylen. Soweit nicht anders beschrieben, können die Aromaten substituiert sein mit einem oder mehreren Resten ausgewählt aus der Gruppe bestehend aus Methyl, Ethyl, *n*-Propyl, *iso*-Propyl, *tert*-Butyl, Hydroxy, Methoxy, Trifluormethoxy, Fluor, Chlor, Brom und Jod.

Diese aromatischen Ringsysteme werden an zwei Stellen unabhängig voneinander über jeweils ein Kohlenstoffatom an das übrige Molekül gebunden.

Unter dem Begriff "Heteroarylen" werden fünf- oder sechsgliedrige heterocyclische Aromaten oder 9-11 gliedrige bicyclische Heteroarylringe verstanden, die ein, zwei oder drei Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Diese heterocyclischen Aromaten werden an zwei Stellen unabhängig voneinander entweder über Kohlenstoff/und oder Stickstoff gebunden. Als Beispiele für fünf- oder sechsgliedrige heterocyclische Aromaten, werden genannt:

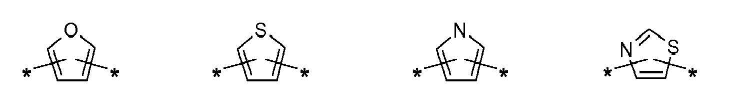

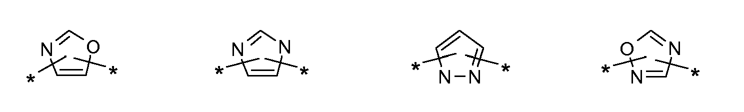

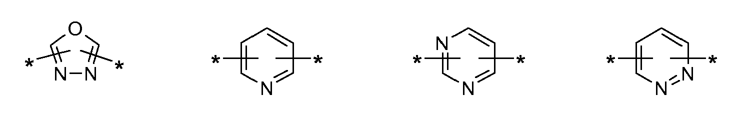

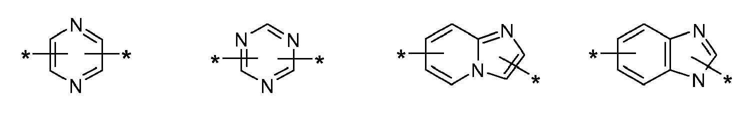

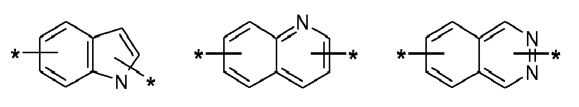

Soweit nicht anders beschrieben, können die Heteroaromaten substituiert sein mit einem oder mehreren Resten ausgewählt aus der Gruppe bestehend aus Methyl, Ethyl, *n*-Propyl, *iso*-Propyl, *tert*-Butyl, Hydroxy, Methoxy, Trifluormethoxy, Fluor, Chlor, Brom und Jod. Vorzugsweise befinden sich die Substituenten bei den voranstehend genannten 5-10 gliedrige bicyclische Heteroarylringen im Phenylring.

Verbindungen der allgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder p-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht. Weiterhin können gegebenenfalls im Molekül vorhandene tertiäre Aminogruppen quarternisiert werden. Zur Umsetzung werden dabei Alkylhalogenide eingesetzt. Erfindungsgemäß bevorzugt wird für die Quartenisierung Methyliodid verwendet.

Weiterhin lassen sich die Verbindungen der allgemeinen Formel **I**, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Erfindungsgemäß erhält man die Verbindungen der allgemeinen Formel **I** nach an sich bekannten Verfahren, beispielsweise nach folgenden Verfahren:

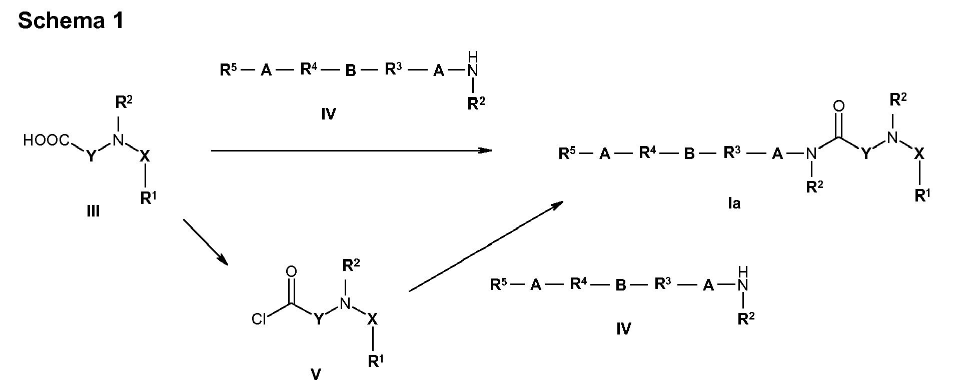

Die in Schema 1 dargestellte Verknüpfung von Carbonsäuren der allgemeinen Formel **III,** in der alle Reste wie voranstehend erwähnt definiert sind, mit Aminen der allgemeinen Formel **IV**, in der alle Reste wie voranstehend erwähnt definiert sind, unter Bildung von Carbonsäureamiden der allgemeinen Formel **Ia**, in der alle Reste wie voranstehend erwähnt definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylamino-propyl)-carbodiimid, O-(1*H*-Benzotriazol-1-yl)-*N,N-N',N'*-tetramethyluronium-hexafluorphosphat (HBTU) oder -tetrafluorborat (TBTU) oder 1*H*-Benzotriazol-1-yl-oxy-tris-(dimethylamino)-phosphonium-hexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran (THF), Acetonitril, Dimethylformamid (DMF), Dimethylacetamid (DMA), N-Methylpyrrolidon (NMP) oder Gemischen aus diesen und bei Temperaturen zwischen -30°C und +30°C, bevorzugt -20°C und +25°C, durchgeführt. Sofern erforderlich, wird als zusätzliche Hilfsbase Diisopropylethylamin (DIPEA) (Hünig-Base) bevorzugt.

Eine alternative Methode zur Verknüpfung besteht in der Überführung einer Carbonsäure der allgemeinen Formel **III,** in der alle Reste wie voranstehend erwähnt definiert sind, in ein Carbonsäurechlorid der allgemeinen Formel **V,** in der alle Reste wie voranstehend erwähnt definiert sind, und anschließender Umsetzung mit einem Amin der allgemeinen Formel **IV,** in der alle Reste wie voranstehend erwähnt definiert sind. Die Synthese eines Carbonsäurechlorids der allgemeinen Formel **V** erfolgt nach literaturbekannten Verfahren (siehe z. B. Houben-Weyl, Methoden der Organischen Chemie, Bd. E5/1).

Die als Ausgangsstoffe verwendeten Carbonsäuren der allgemeinen Formel **III,** in der alle Reste wie voranstehend erwähnt definiert sind, erhält man nach an sich literaturbekannten Verfahren, beispielsweise durch die in Schema 2 bis 7 dargestellten Synthesewege.

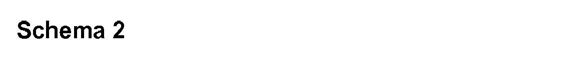

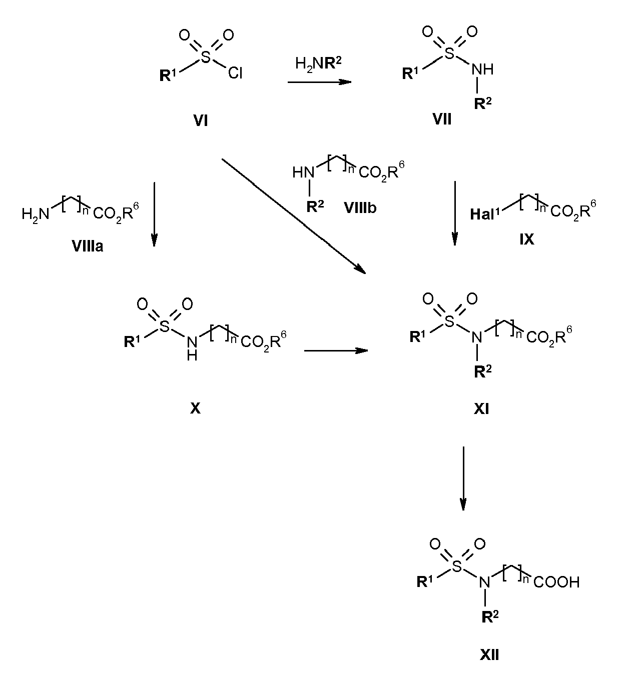

Die Sulfonsäurechloride der allgemeinen Formel **VI,** in der **R¹** wie voranstehend erwähnt definiert ist, sind entweder literaturbekannt oder käuflich erwerbbar. Sie werden nach Standard-Reaktionsbedingungen mit einem Amin der allgemeinen Formeln H₂N-**R²**, **VIIIa** oder Vlllb zu Sulfonsäureamiden der allgemeinen Formeln **VII, X** oder **XI** umgesetzt, wobei **R¹** und **R²** wie voranstehend erwähnt definiert sind und n eine Zahl 1, 2, 3 oder 4 und **R⁶** einen C₁₋₆-Alkylrest bedeuten. Die Umsetzung erfolgt gegebenenfalls in Gegenwart einer Base wie Triethylamin, DIPEA oder Pyridin und einem inerten Lösungsmittel wie Dichlormethan oder Tetrahydrofuran bei einer Temperatur von 0°C bis 100°C mit einer typischen Reaktionsdauer von einer bis 24 Stunden.

Die Umsetzung der Sulfonsäureamide der allgemeinen Formel **VII** mit einem Halogenid der allgemeinen Formel **IX,** in der **Hal¹** Chlor oder Brom bedeutet, erfolgt nach literaturbekannten Verfahren, beispielsweise mit Hilfe einer Base wie Kalium- oder Natriumcarbonat in Dimethylformamid oder Tetrahydrofuran bei 0°C bis 100°C.

Die Hydrolyse der Carbonsäurester der allgemeinen Formel **XI,** in der **R¹** und **R²** wie voranstehend erwähnt definiert sind, n eine Zahl 1, 2, 3 oder 4 und **R⁶** eine C₁₋₃-Alkylgruppe bedeuten, zu Carbonsäuren der allgemeinen Formel **XII,** in der **R¹** und **R²** wie voranstehend erwähnt definiert sind und n eine Zahl 1, 2, 3 oder 4 und **R⁶** eine C₁₋₃-Alkylgruppe bedeutet, wird unter bekannten Bedingungen durchgeführt, beispielsweise mit Lithium- oder Natriumcarbonat und Wasser in Methanol und/oder Tetrahydrofuran.

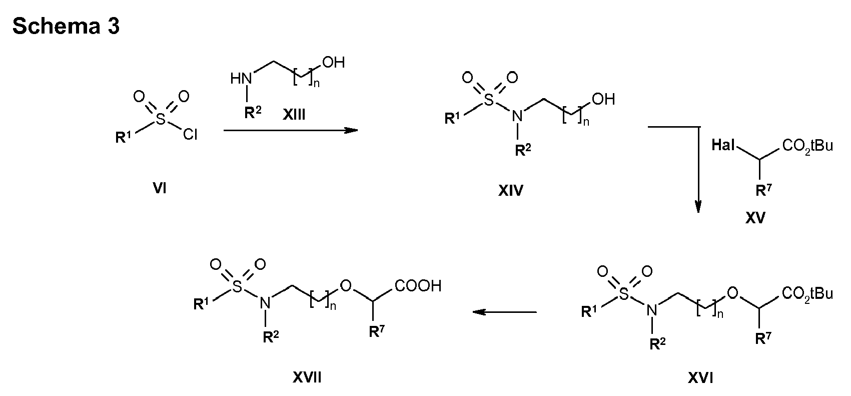

Die Herstellung von Sulfonsäureamiden der allgemeinen Formel **XIV** erfolgt wie unter Schema 2 beschrieben.

Die Alkylierung der Hydroxylfunktion der Sulfonsäureamide der allgemeinen Formel **XIV,** in der **R¹** und **R²** wie voranstehend erwähnt definiert sind mit der Maßgabe, dass **R²** kein Wasserstoffatom darstellt, und n eine Zahl 1, 2, 3 oder 4 und **R⁶** eine C₁₋₃-Alkylgruppe bedeutet, wird unter literaturbekannten Reaktionsbedingungen durchgeführt, beispielsweise unter 2-Phasen-Bedingungen mit Hilfe eines Phasentransfer-Katalysators in Gegenwart einer starken anorganischen Base wie Natronlauge oder Kalilauge und in einem inerten Lösungsmittel wie Toluol bei 0°C bis 100°C.

Die Spaltung des tert-Butylesters der allgemeinen Formel XVI, in der **R¹** und **R²** wie voranstehend erwähnt definiert sind, n eine Zahl 1, 2, 3 oder 4 und **R⁶** eine C₁₋₃-Alkylgruppe und **R⁷** ein Wasserstoffatom oder eine C₁₋₃-Alkylgruppe bedeuten, erfolgt nach literaturbekannten Verfahren (siehe z.B. Philip J. Kocieński, Protecting Groups, 3rd Edition, 2005, Georg Thieme Verlag).

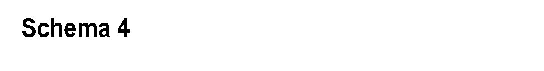

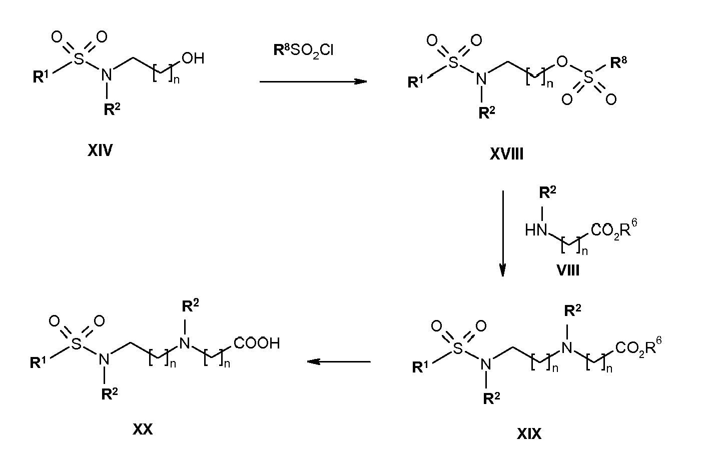

Die Sulfonierung der Hydroxylfunktion einer Verbindung der allgemeinen Formel **XIV,** in der **R¹** und **R²** wie voranstehend erwähnt definiert sind mit der Maßgabe, dass **R²** kein Wasserstoffatom darstellt, und n eine Zahl 1, 2, 3 oder 4 und **R⁶** eine C₁₋₃-Alkylgruppe bedeutet, mit einem Sulfonsäurechlorid der allgemeinen Formel **R⁸**SO₂Cl, in der **R⁸** eine C₁₋₃-Alkylgruppe oder eine ggf. durch C₁₋₃-Alkylgruppe substituierte Phenylgruppe darstellt, zu Verbindungen der allgemeinen Formel **XVIII,** in der alle Rest wie voranstehend erwähnt definiert sind, wird unter Standard-Reaktionsbedingungen durchgeführt, typischerweise in Gegenwart einer Base wie DMAP und/oder Pyridin und einem inerten Lösungsmittel wie Dichlormethan oder THF bei -5°C bis 35°C. Eine flüssige Base wie Pyridin kann als Base und gleichzeitig als Lösungsmittel verwendet werden.

Die nachfolgende Alkylierung der Amine mit der allgemeinen Formel **VII** zu Verbindungen der allgemeinen Formel **XIX,** in der **R¹** und **R²** wie voranstehend erwähnt definiert sind, n eine Zahl 1, 2, 3 oder 4 und **R⁶** eine C₁₋₃-Alkylgruppe und **R⁶** eine C₁₋₆-Alkylgruppe bedeuten, wird zweckmäßigerweise in einem Lösungsmittel wie Toluol, Chlorbenzol, Dimethylformamid, Dimethylsulfoxid (DMSO), Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XIX** zu Carbonsäuren der allgemeinen Formel **XX** erfolgt wie unter Schema 2 beschrieben.

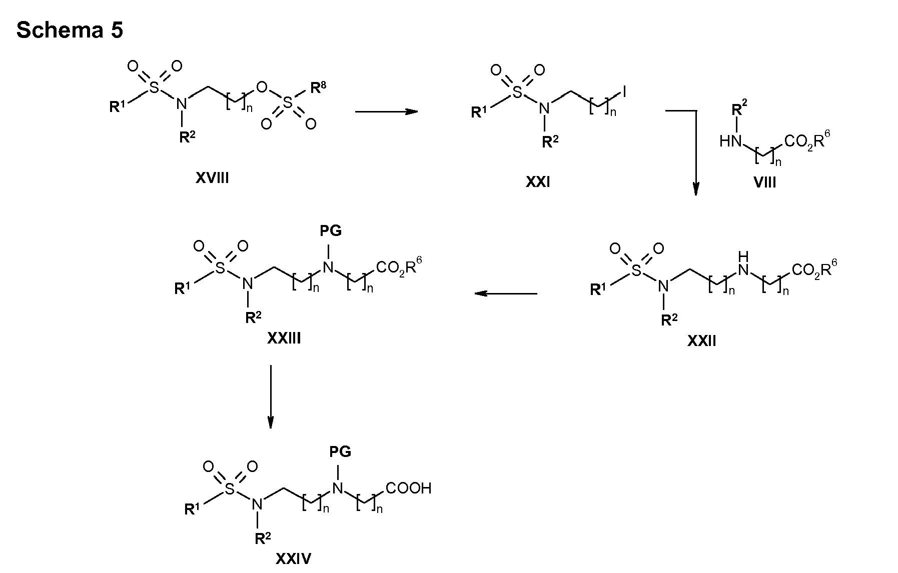

Die Finkelsteinstein-Reaktion von Verbindungen der allgemeinen Formel **XVIII,** in der **R¹** und **R²** wie eingangs erwähnt definiert sind, n eine Zahl 1, 2, 3 oder 4 und **R⁶** eine C₁₋₃-Alkylgruppe und **R⁸** eine C₁₋₃-Alkylgruppe oder eine ggf. durch C₁₋₃-Alkylgruppe substituierte Phenylgruppe bedeuten, zu Halogeniden der allgemeinen Formel XXI, in der **R¹** und **R²** wie eingangs erwähnt definiert sind und n eine Zahl 1, 2, 3 oder 4 und **R⁶** eine C₁₋₃-Alkylgruppe bedeutet, erfolgt unter bekannten Reaktionsbedingungen (siehe z.B. H. Finkelstein, Berichte der Deutschen Chemischen Gesellschaft 43, 1910, 1528).

Die anschließende Alkylierung des Glycinesters wird wie unter Schema 4 beschrieben durchgeführt (**R²** ≠ H).

Die Aminofunktion in den Verbindungen der allgemeinen Formel **XXIII** wird durch eine konventionelle Schutzgruppe PG nach bekannten Verfahren geschützt. Die ausgewählte Schutzgruppe ist eine, die unter nicht-hydrogenolytischen Bedingungen abgespalten werden kann. Eine bevorzugte Schutzgruppe ist die Boc-Gruppe. Eine Übersicht über die Chemie der Schutzgruppen findet sich in Theodora W. Greene und Peter G. M. Wuts, Protective Groups in Organic Synthesis, Second Edition, 1991, Verlag John Wiley and Sons sowie in Philip J. Kocieński, Protecting Groups, 3rd Edition, 2005, Georg Thieme Verlag.

Die Spaltung der Carbonsäureester der allgemeinen Formel **XXIII** zu Carbonsäuren der allgemeinen Formel **XXIV** erfolgt wie unter Schema 2 beschrieben.

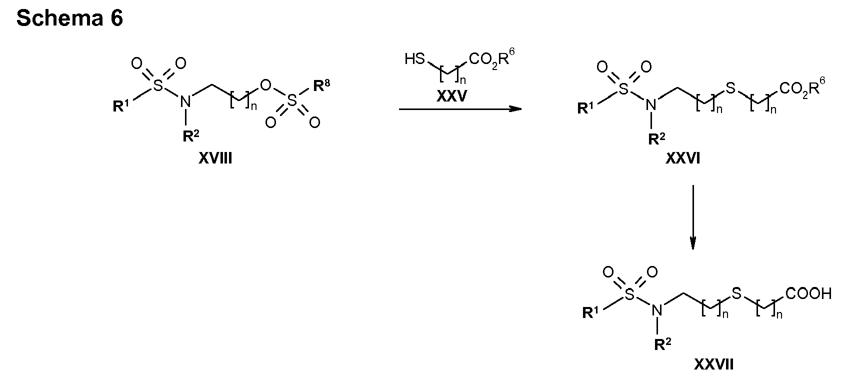

Die Alkylierung eines Thiols der allgemeinen Formel **XXV,** in der n eine Zahl 1, 2, 3 oder 4 und **R⁶** eine C₁₋₆-Alkylgruppe bedeutet, zu Verbindungen der allgemeinen Formel **XXVI,** in der **R¹** und **R²** wie voranstehend erwähnt definiert sind, n eine Zahl 1, 2, 3 oder 4 und **R⁶** eine C₁₋₆-Alkylgruppe bedeuten, wird zweckmäßigerweise in einem Lösungsmittel wie Toluol, Chlorbenzol, DMF, DMSO, Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XXVI** zu Carbonsäuren der allgemeinen Formel **XXVII,** in der alle Reste wie voranstehend erwähnt definiert sind, erfolgt wie unter Schema 2 beschrieben.

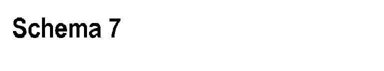

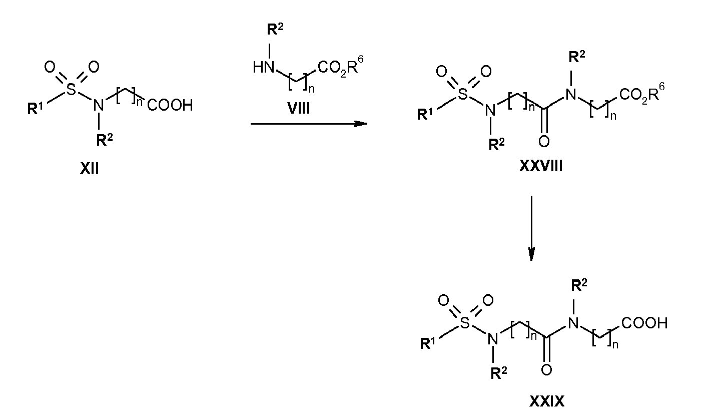

Die Amidknüpfung von Carbonsäuren der allgemeinen Formel **XII,** in der **R¹** und **R²** wie voranstehend erwähnt definiert sind und n eine Zahl 1, 2, 3 oder 4 bedeutet, und Aminosäuren der allgemeinen Formel **VIII,** in der **R¹** und **R²** wie voranstehend erwähnt definiert sind, n eine Zahl 1, 2, 3 oder 4 und **R⁶** eine C₁₋₆-Alkylgruppe bedeuten, zu Carbonsäureamiden der allgemeinen Formel **XXVIII,** in der **R¹** und **R²** wie voranstehend erwähnt definiert sind, n eine Zahl 1, 2, 3 oder 4 und **R⁶** eine C₁₋₆-Alkylgruppe bedeuten, wird wie unter Schema 1 beschrieben durchgeführt.

Wie unter Schema 2 ausgeführt, wird der Carbonsäureester der allgemeinen Formel **XXVIII** zu Carbonsäure der allgemeinen Formel **XXIX,** in der **R¹** und **R²** wie voranstehend erwähnt definiert sind und n eine Zahl 1, 2, 3 oder 4 bedeutet, gespalten.

Die als Ausgangsstoffe verwendeten Amine der allgemeinen Formel **IV** sind entweder käuflich erwerbbar, oder man erhält sie nach an sich literaturbekannten Verfahren, beispielsweise durch die in Schema 8 bis 12 dargestellten Synthesewege, wobei **R^{1.1}** wie voranstehend erwähnt definiert ist, **Hal¹** ein Chlor- oder Bromatom und **Hal²** ein Fluor-, Chlor- oder Bromatom oder einen Rest **R⁹** bedeuten.

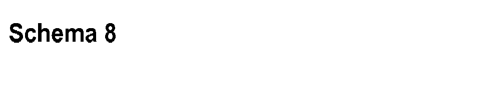

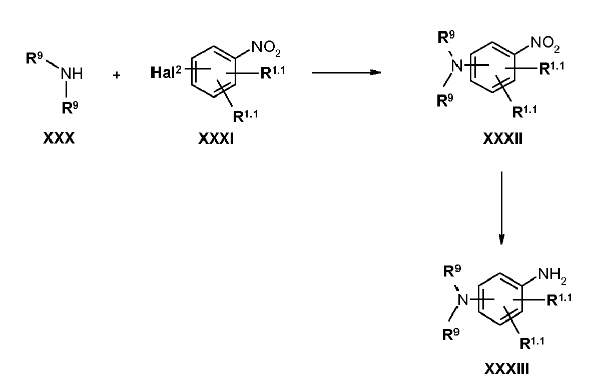

Die Reaktion eines Amins der allgemeinen Formel **XXX,** in der **R⁹** eine C₁₋₃-Alkylgruppe bedeutet, mit einem Halogen-nitrobenzol der allgemeinen Formel **XXXI,** in der **R^{1.1}** wie voranstehend erwähnt definiert ist und **Hal²** ein Fluor-, Chlor- oder Bromatom oder einen Rest **R⁹** bedeutet, erfolgt nach bekannten Verfahren, beispielsweise in einem Lösungsmittel wie Tetrahydrofuran, Dimethylformamid oder Dimethylsulfoxid und zweckmäßigerweise in Gegenwart einer passenden Base wie Triethylamin oder Kaliumcarbonat, bei einer Temperatur von 20°C bis 160°C. Ist das Amin der allgemeinen Formel XXX flüssig, kann die Reaktion auch ohne Lösungsmittel und zusätzlicher Base durchgeführt werden.

Die Reduktion der Nitrogruppe zu Anilinen der allgemeinen Formel **XXXIII,** in der **R^{1.1}** wie voranstehend erwähnt definiert ist und **R⁹** eine C₁₋₃-Alkylgruppe bedeutet, erfolgt nach Standard-Reaktionsbedingungen (siehe z.B. Richard C. Larock, Comprehensive Organic Transformations, 1989, VCH), vorzugsweise nach Standard-Bedingungen der katalytischen Hydrogenolyse mit einem Katalysator wie Palladium auf Kohle oder Raney-Nickel in einem Lösungsmittel wie Methanol oder Ethanol.

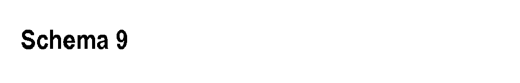

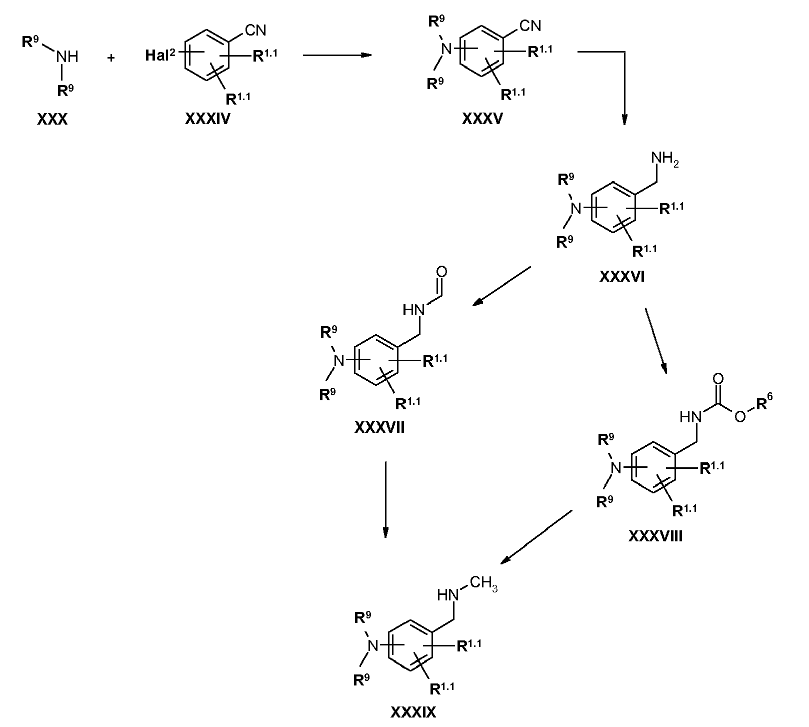

Die Umsetzung von Verbindungen der allgemeinen Formeln **XXX,** in der **R⁹** eine C₁₋₃-Alkylgruppe bedeutet, mit einer Verbindung der allgemeinen Formel **XXXIV,** in der **R^{1.1}** wie voranstehend erwähnt definiert ist und **Hal²** ein Fluor-, Chlor- oder Bromatom oder einen Rest **R⁹** bedeutet, zu Verbindungen mit der allgemeinen Formel **XXXV,** in der R^{1.1} wie voranstehend erwähnt definiert ist und **R⁹** eine C₁₋₃-Alkylgruppe bedeutet, wird wie unter Schema 8 beschrieben durchgeführt.

Die Reduktion eines Nitrils der allgemeinen Formel **XXXV** zu einem Amin der allgemeinen Formel **XXXVI,** in der **R^{1.1}** wie voranstehend erwähnt definiert ist und **R⁹** eine C₁₋₃-Alkylgruppe bedeutet, kann unter Standard-Bedingungen der katalytischen Hydrogenolyse mit einem Katalysator wie beispielsweise Raney-Nickel in einem Lösungsmittel wie ammoniakalischem Methanol oder Ethanol oder mit einem Reduktionsmittel wie Lithiumaluminiumhydrid oder Natriumborhydrid in einem Lösungmittel wie Tetrahydrofuran, gegebenenfalls in Gegenwart von Aluminiumchlorid, durchgeführt werden.

Die Formylierung eines Amins der allgemeinen Formel **XXXVI** zu einer Verbindung der allgemeinen Formel **XXXVII,** in der **R^{1.1}** wie voranstehend erwähnt definiert ist und **R⁹** eine C₁₋₃-Alkylgruppe bedeutet, erfolgt zweckmäßigerweise in einem Lösungsmittel wie Dichlormethan, beispielsweise bei Temperaturen von 40°C bis 70°C und in Gegenwart von Essigsäureanhydrid und Ameisensäure.

Die Carbamatbildung zu Verbindungen der allgemeinen Formel **XXXVIII,** in der **R^{1.1}** wie voranstehend erwähnt definiert ist, **R⁶** eine C₁₋₆-Alkyl- und **R⁹** eine C₁₋₃-Alkylgruppe bedeutet, wird nach bekannten Verfahren durchgeführt, beispielsweise mit einem Chlorameisensäureester oder Boc-Anhydrid in Gegenwart einer Base wie Triethylamin oder Natronlauge und einem Lösungsmittel wie THF oder Dioxan.

Die Reduktion des Formyls bzw. des Carbamates zu Verbindungen der allgemeinen Formel **XXXIX,** in der **R^{1.1}** wie voranstehend erwähnt definiert ist und **R⁹** eine C₁₋₃-Alkylgruppe bedeutet, erfolgt unter Standard-Reaktionsbedingungen, vorzugsweise mit einem Reduktionsmittel wie Lithiumaluminiumhydrid und in einem Lösungsmittel wie Tetrahydrofuran bei einer Temperatur von 50°C bis 100°C.

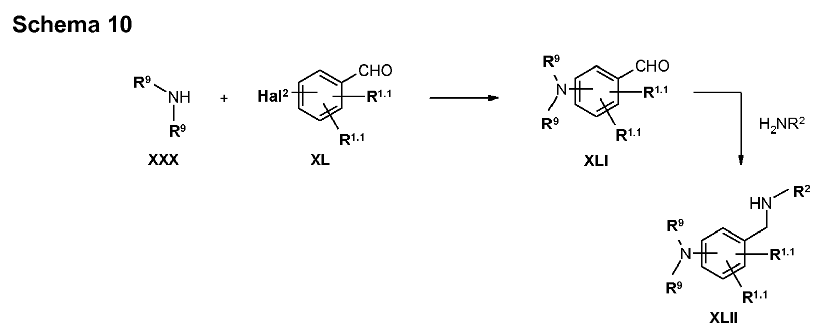

Der Halogen-Stickstoff-Austausch in Verbindungen der allgemeinen Formeln **XXX**, in der **R⁹** eine C₁₋₃-Alkylgruppe bedeutet, und **XL,** in der **R^{1.1}** wie voranstehend erwähnt definiert ist und **Hal²** ein Fluor-, Chlor- oder Bromatom oder einen Rest **R⁹** bedeutet, zur Herstellung von Verbindungen der allgemeinen Formel **XLI**, in der **R^{1.1}** wie voranstehend erwähnt definiert ist und **R⁹** eine C₁₋₃-Alkylgruppe bedeutet, wird wie unter Schema 8 beschrieben durchgeführt.

Die Reaktion von Benzaldehyden der allgemeinen Formel **XLI,** in der **R^{1.1}** wie voranstehend erwähnt definiert ist und **R⁹** eine C₁₋₃-Alkylgruppe bedeutet, mit einem Amin der allgemeinen Formel H₂N**R²**, in der **R²** wie voanstehend erwähnt definiert ist, zu einer Verbindung der allgemeinen Formel **XLII,** in der **R^{1.1}** und **R²** wie voranstehend erwähnt definiert sind und **R⁹** eine C₁₋₃-Alkylgruppe bedeutet, ist eine reduktive Aminierung. Sie erfolgt nach bekannten Verfahren, beispielsweise mit einem Reduktionsmittel wie Natriumtriacetoxyborhydrid, Natriumborhydrid oder Natriumcyanoborhydrid, zweckmäßigerweise in einem Lösungsmittel wie Tetrahydrofuran oder Dichlormethan, gegebenenfalls unter Zusatz von Essigsäure.

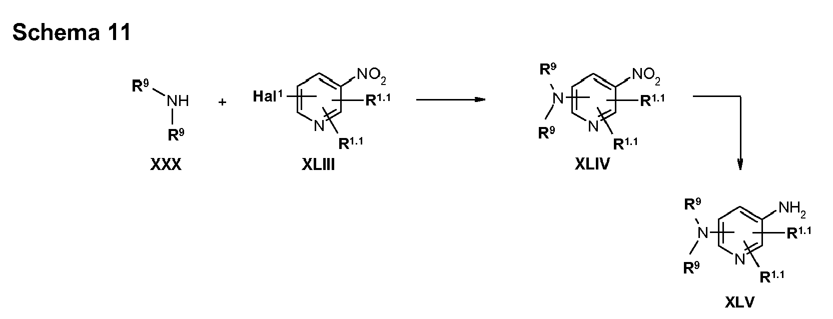

Die Reaktion eines Amins der allgemeinen Formel **XXX**, in der **R⁹** eine C₁₋₃-Alkylgruppe bedeutet, mit einem Halogen-nitropyridin der allgemeinen Formel **XLIII,** in der **R^{1.1}** wie voranstehend erwähnt definiert ist und **Hal¹** ein Chlor- oder Bromatom bedeutet, erfolgt nach bekannten Verfahren, beispielsweise in einem Lösungsmittel wie Tetrahydrofuran, Dichlormethan, Methanol oder DMSO und zweckmäßigerweise in Gegenwart einer passenden Base wie Triethylamin, Natronlauge oder Kaliumcarbonat und bei einer Temperatur von 20°C bis 100°C.

Die anschließende Reduktion der Nitrogruppe einer Verbindung der allgemeinen Formel **XLIV,** in der **R^{1.1}** wie voranstehend erwähnt definiert ist und **R⁹** eine C₁₋₃-Alkylgruppe bedeutet, zu Verbindungen der allgemeinen Formel **XLV**, in der **R^{1.1}** wie voranstehend erwähnt definiert ist und **R⁹** eine C₁₋₃-Alkylgruppe bedeutet, wird wie unter Schema 8 beschrieben durchgeführt.

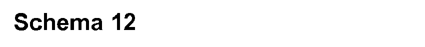

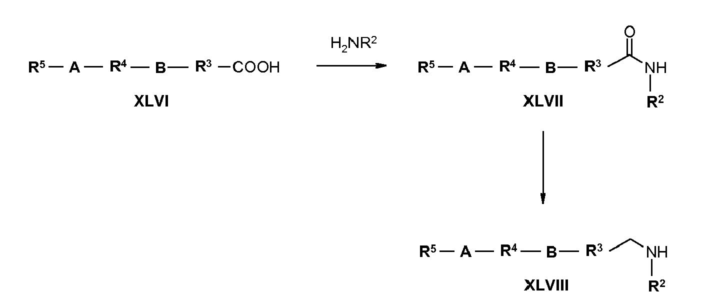

Die Amidknüpfung von Carbonsäuren der allgemeinen Formel **XLVI,** in der alle Reste wie voranstehend erwähnt definiert sind, und Aminen der allgemeinen Formel H₂N**R²**, in der **R²** wie voranstehend erwähnt definiert ist, zu Carbonsäureamiden der allgemeinen Formel **XLVII,** in der alle Reste wie voranstehend erwähnt definiert sind, wird wie unter Schema 1 beschrieben durchgeführt.

Die Reduktion von Carbonsäureamiden der allgemeinen Formel **XLVII** zu Aminen der allgemeinen Formel **XLVIII,** in der alle Reste wie voranstehend erwähnt definiert sind, erfolgt nach Standard-Reaktionsbedingungen, vorzugsweise in Gegenwart eines Reduktionsmittels wie Lithiumaluminiumhydrid und einem Lösungsmittel wie Tetrahydrofuran bei 40°C bis 100°C.

### Methodenbeschreibung zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

Um zu zeigen, dass die Verbindungen der allgemeinen Formel **I** mit unterschiedlichen Strukturelementen gute bis sehr gute Bradikinin-B1-Rezeptor antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht um spezifische Verbindungen hervorzuheben:

| **Beispiel** | **Kᵢ [nM]** |
|---|---|
| (1) | 6.2 |
| (13) | 2.1 |
| (22) | 7 |
| (53) | 2.4 |
| (55) | 0.7 |
| (59) | 6.3 |
| (61) | 3.3 |
| (66) | 4.6 |
| (67) | 0.4 |
| (72) | 2.8 |
| (73) | 6.8 |
| (77) | 8.7 |
| (78) | 5.8 |
| (97) | 6.7 |
| (102) | 5.0 |
| (109) | 6.0 |
| (114) | 4.4 |
| (117) | 0.99 |
| (130) | 5.7 |
| (180) | 5.2 |
| (181) | 7.1 |
| (182) | 4.8 |
| (183) | 6.6 |
| (184) | 1.3 |
| (186) | 3.4 |
| (188) | 9.4 |
| (216) | 4.9 |
| (227) | 4.8 |
| (269) | 7.8 |
| (303) | 6.32 |
| (323) | 2.8 |
| (325) | 0.94 |
| (326) | 6.5 |
| (334) | 8.65 |
| (335) | 9.37 |
| (338) | 1.1 |
| (352) | 9.2 |
| (353) | 6.1 |
| (356) | 8.8 |
| (358) | 3.5 |
| (360) | 4.4 |
| (361) | 7.4 |
| (365) | 2.4 |
| (367) | 2.7 |
| (368) | 1.52 |
| (369) | 3.8 |
| (372) | 2.39 |
| (381) | 8.1 |
| (383) | 6.2 |
| (384) | 9.3 |
| (385) | 6.4 |
| (386) | 6.3 |
| (389) | 3.7 |
| (392) | 8.3 |
| (393) | 1.6 |
| (394) | 1.04 |
| (397) | 7.5 |
| (398) | 0.74 |
| (399) | 3 |
| (400) | 0.79 |
| (401) | 2.7 |
| (402) | 9.3 |
| (404) | 2.8 |
| (418) | 1.2 |
| (419) | 0.65 |
| (420) | 9.1 |
| (421) | 8.7 |
| (423) | 3.4 |
| (424) | 1.4 |
| (425) | 8.3 |
| (428) | 6.3 |
| (435) | 1.5 |
| (439) | 7.5 |
| (441) | 4.6 |
| (444) | 6.9 |
| (445) | 5.6 |
| (448) | 0.82 |
| (451) | 9 |
| (458) | 4.3 |
| (463) | 2 |
| (464) | 1.5 |
| (465) | 3.8 |
| (468) | 1 |
| (469) | 8 |
| (471) | 4.1 |
| (472) | 0.68 |
| (473) | 1.8 |
| (474) | 1.4 |
| (475) | 2.4 |
| (476) | 2.35 |
| (477) | 5.8 |
| (478) | 1 |
| (492) | 3.2 |
| (576) | 0.85 |
| (577) | 0.34 |
| (580) | 6.2 |
| (582) | 9.3 |
| (584) | 9.0 |
| (586) | 5.1 |
| (587) | 9.5 |
| (588) | 1.5 |
| (589) | 5.1 |
| (591) | 1.9 |
| (592) | 2.8 |
| (613) | 9.7 |
| (614) | 2 |
| (616) | 3.7 |
| (619) | 1.2 |
| (620) | 5.9 |
| (621) | 5.8 |
| (623) | 3.4 |
| (624) | 8 |
| (630) | 5.5 |

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.

Aufgrund ihrer pharmakologischen Wirkung eignen sich die Substanzen zur Behandlung
(a) von akuten Schmerzen wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen / Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und
   Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz.
   Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
   chronisch obstruktiver Lungen-Erkrankung (COPD) einschließlich Lungenemphysem, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson schen Erkrankung und der Alzheimer Erkrankung;
(k) von Sepsis und septischem Schock nach bakteriellen Infektionen oder nach Trauma;
(l) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Neben der Eignung als Humantherapeutika, sind diese Substanzen auch nützlich in der veterinärmedizinischen Therapie von Haustieren, exotischen Tieren und Nutztieren.

Zur Behandlung von Schmerzen kann es vorteilhaft sein, die erfindungsgemässen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemässen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemässen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, warum nicht auch Mesalazin, Olsalazin, und Pyrazolone (Apazon, Bezpiperylon, Feprazon, Mofebutazon, Oxyphenbutazon, Phenylbutazon, warum nicht auch Propyphenazon und Metamizol, und Coxibe (Celecoxib, Valecoxib, Rofecoxib, Etoricoxib).
Opiat Rezeptor Agonisten wie z. B. Morphin, Propoxyphen (Darvon), Tramadol, Buprenorphin.
Cannabinoid Agonisten wie z.B. GW-1000, KDS-2000, SAB-378, SP-104, NVP001-GW-843166, GW-842166X, PRS-211375.
Natriumkanalblocker wie z.B. Carbamazepin, Mexiletin, Lamotrigin, Pregabalin, Tectin, NW-1029, CGX-1002.
N-Typ Calciumkanalblocker wie z.B. Ziconitid, NMED-160, SP1-860.
Serotonerge und noradrenerge Modulatoren wie z.B. SR-57746, Paroxetin, Duloxetin, Clonidin, Amitriptylin, Citalopram.
Corticosteroide wie z.B. Betamethason, Budesonid, Cortison, Dexamethason, Hydrocortison, Methylprednisolon, Prednisolon, Prednison und Triamcinolon.
Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin,
Cetirizin, Desloratadin, fexofenadin, Levocetirizin.
Histamin H2-Rezeptor Antagonisten wie z.B. Cimetidin, Famotidin, und Ranitidin. Protonenpumpenhemmer wie z.B. Omeprazol, Pantoprazol, Esomeprazol.
Leukotrien Antagonisten und 5-Lipoxygenasehemmer wie z.B. Zafirlukast, Montelukast, Pranlukast und Zileuton.
Lokalanästhetika wie z.B. Ambroxol, Lidocain.
VR1 Agonisten und Antagonisten wie z.B. NGX-4010, WL-1002, ALGRX-4975, WL-10001, AMG-517.
Nikotinrezeptor Agonisten wie z.B. ABT-202, A-366833, ABT-594, BTG-102, A-85380, CGX1204.
P2X3-Rezeptor Antagonisten wie z.B. A-317491, ISIS-13920, AZD-9056.
NGF Agonisten und Antagonisten wie z.B. RI-724, RI-1024, AMG-819, AMG-403, PPH 207.
NK1 und NK2 Antagonisten wie z.B. DA-5018, R-116301, CP-728663, ZD-2249.
NMDA Antagonisten wie z.B. NER-MD-11, CNS-5161, EAA-090, AZ-756, CNP-3381.
Kaliumkanal Modulatoren wie z.B. CL-888, ICA-69673, Retigabin.
GABA Modulatoren wie z.B. Lacosamid.
Serotonerge und noradrenerge Modulatoren wie z.B. SR-57746, Paroxetin, Duloxetin, Clonidin, Amitriptylin, Citalopram, Flibanserin.
Anti-Migräne Therapeutika wie z.B. Sumatriptan, Zolmitriptan, Naratriptan, Eletriptan.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel IR-, ¹H-NMR und/oder Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser. Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX™, 35-70 µm) oder Alox (E. Merck, Darmstadt, Aluminiumoxid 90 standardisiert, 63-200 µm, Artikel-Nr: 1.01097.9050) verwendet.

In den Versuchsbeschreibungen werden die folgenden Abkürzungen verwendet:
- CDI: 1,1'-Carbonyldiimidazol
- DC: Dünnschichtchromatogramm
- DIPEA: Diisopropylethylamin
- DMAP: 4-Dimethylaminopyridin
- DMF: Dimethylformamid
- DMSO: Dimethylsulfoxid
- HATU: *O*-(7-Azabenzotriazol-1-yl)-*N*,*N*,*N*'*,*N'-tetramethyluroniumhexafluorphosphat

- *tert*: tertiär
- TBTU: 2-(1 *H*-Benzotriazol-1-yl)-1,1,3,3-tetramethyluronium-tetrafluorborat
- THF: Tetrahydrofuran

Folgende analytische HPLC-Methoden wurden verwendet:

| | | |
|---|---|---|
| Methode 1: | Säule: | XTerra^{™} MS C18, 2.5 µM, 4.6 x 30 mm |
| | Detektion: | 210 - 420 nm |
| | Fließmittel A: | Wasser / 0.1 % Ameisensäure |
| | Fließmittel B: | Acetonitril / 0.1 % Ameisensäure |
| | Gradient: | |

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 3.1 | 2.0 | 98.0 | 1.0 |
| 4.5 | 2.0 | 98.0 | 1.0 |
| 5.0 | 95.0 | 5.0 | 1.0 |

| | | |
|---|---|---|
| Methode 2: | Säule: | Microsorb C18, 3 µM, 4.6 x 50 mm |
| | Detektion: | 220 - 320 nm |
| | Fließmittel A: | Wasser / 0.1 % TFA |
| | Fließmittel B: | Acetonitril / 0.1 % TFA |
| | Gradient: | |

| Zeit in min | %A | %B | Flussrate in mL/min |
|---|---|---|---|
| 0.0 | 95.0 | 5.0 | 1.5 |
| 0.5 | 95.0 | 5.0 | 1.5 |
| 3.8 | 2.0 | 98.0 | 1.5 |
| 4.3 | 2.0 | 98.0 | 1.5 |
| 4.35 | 95.0 | 5.0 | 1.5 |
| 4.6 | 95.0 | 5.0 | 1.5 |

| | | |
|---|---|---|
| Methode 3: | Säule: | XTerra^{™} MS C18, 3.5 µM, 4.6 x 50 mm |
| | Detektion: | 210 - 420 nm |
| | Fließmittel A: | Wasser / 0.1 % Ameisensäure |
| | Fließmittel B: | Acetonitril / 0.1 % Ameisensäure |
| | Gradient: | |

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 7.1 | 2.0 | 98.0 | 1.0 |
| 7.9 | 2.0 | 98.0 | 1.0 |
| 8.0 | 95.0 | 5.0 | 1.0 |

| | | |
|---|---|---|
| Methode 4: | Säule: | Zorbax Stable Bond C18, 3.5 µM, 4.6 x 75 mm |
| | Detektion: | 230 - 360 nm |
| | Fließmittel A: | Wasser / 0.1 % Ameisensäure |
| | Fließmittel B: | Acetonitril / 0.1 % Ameisensäure |
| | Gradient: | |

| Zeit in min | %A | %B | Flussrate in mL/min |
|---|---|---|---|
| 0.0 | 95.0 | 5.0 | 1.6 |
| 0.1 | 95.0 | 5.0 | 1.6 |
| 4.5 | 10.0 | 90.0 | 1.6 |
| 5.09 | 10.0 | 90.0 | 1.6 |
| 5.5 | 90.0 | 10.0 | 1.6 |

| | | |
|---|---|---|
| Methode 5: | Säule: | Interchim Strategy C18, 5 µM, 4.6 x 50 mm |
| | Detektion: | 220 - 320 nm |
| | Fließmittel A: | Wasser / 0.1 % TFA |
| | Fließmittel B: | Acetonitril |
| | Gradient: | |

| Zeit in min | %A | %B | Flussrate in mL/min |
|---|---|---|---|
| 0.0 | 95.0 | 5.0 | 3.0 |
| 0.3 | 95.0 | 5.0 | 3.0 |
| 2.0 | 2.0 | 98.0 | 3.0 |
| 2.4 | 2.0 | 98.0 | 3.0 |
| 2.45 | 95.0 | 5.0 | 3.0 |
| 2.8 | 95.0 | 5.0 | 3.0 |

| | | |
|---|---|---|
| Methode 6: | Säule: | Merck Cromolith Speed ROD RP18e, 4.6 x 50 mm |
| | Detektion: | 190 - 400 nm |
| | Fließmittel A: | Wasser / 0.1 % Ameisensäure |
| | Fließmittel B: | Acetonitril / 0.1 % Ameisensäure |
| | Gradient: | |

| Zeit in min | %A | %B | Flussrate in mL/min |
|---|---|---|---|
| 0.0 | 90.0 | 10.0 | 1.5 |
| 4.5 | 10.0 | 90.0 | 1.5 |
| 5.0 | 10.0 | 90.0 | 1.5 |
| 5.5 | 90.0 | 10.0 | 1.5 |

| | | |
|---|---|---|
| Methode 7: | Säule: | Waters SunFire C18, 3.5 µM, 4.6 x 50 mm |
| | Detektion: | 210 - 500 nm |
| | Fließmittel A: | Wasser / 0.1 % TFA |
| | Fließmittel B: | Acetonitril / 0.1 % TFA |
| | Gradient: | |

| Zeit in min | %A | %B | Flussrate in mL/min |
|---|---|---|---|
| 0.0 | 95.0 | 5.0 | 1.5 |
| 2.0 | 2.0 | 98.0 | 1.5 |
| 3.0 | 2.0 | 98.0 | 1.5 |
| 3.4 | 95.0 | 5.0 | 1.5 |

| | | |
|---|---|---|
| Methode 8: | Säule: | Waters XBridge C18, 3.5 µM, 4.6 x 50 mm |
| | Detektion: | 210 - 500 nm |
| | Fließmittel A: | Wasser / 0.1 % TFA |
| | Fließmittel B: | Acetonitril / 0.1 % TFA |
| | Gradient: | |

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 5.1 | 2.0 | 98.0 | 1.0 |
| 6.5 | 2.0 | 98.0 | 1.0 |
| 7.0 | 95.0 | 5.0 | 1.0 |

| | | |
|---|---|---|
| Methode 9: | Säule: | Merck Chromolith^{™} Flash RP18e, 4.6 x 25 mm |
| | Detektion: | 190 - 400 nm |
| | Fließmittel A: | Wasser / 0.1 % Ameisensäure |
| | Fließmittel B: | Acetonitril / 0.1 % Ameisensäure |
| | Gradient: | |

| Zeit in min | %A | %B | Flussrate in mL/min |
|---|---|---|---|
| 0.0 | 90.0 | 10.0 | 1.6 |
| 2.7 | 10.0 | 90.0 | 1.6 |
| 3.0 | 10.0 | 90.0 | 1.6 |
| 3.3 | 90.0 | 10.0 | 1.6 |

| | | |
|---|---|---|
| Methode 10: | Säule: | Merck Chromolith^{™} Flash RP18e, 4.6 x 25 mm |
| | Detektion: | 210 - 400 nm |
| | Fließmittel A: | Wasser / 0.1 % TFA |
| | Fließmittel B: | Acetonitril / 0.1 % TFA |
| | Gradient: | |

| Zeit in min | %A | %B | Flussrate in mL/min |
|---|---|---|---|
| 0.0 | 95.0 | 5.0 | 2.5 |
| 0.2 | 95.0 | 5.0 | 2.5 |
| 1.5 | 2.0 | 98.0 | 2.5 |
| 1.7 | 2.0 | 98.0 | 2.5 |
| 1.9 | 95.0 | 5.0 | 2.5 |
| 2.2 | 95.0 | 5.0 | 2.5 |

| | | |
|---|---|---|
| Methode 11: | Säule: | Waters XBridge C18, 3.5 µM, 4.6 x 50 mm |
| | Detektion: | 210 - 500 nm |
| | Fließmittel A: | Wasser / 0.1 % TFA |
| | Fließmittel B: | Acetonitril / 0.1 % TFA |
| | Gradient: | |

| Zeit in min | %A | %B | Flussrate in mL/min |
|---|---|---|---|
| 0.0 | 95.0 | 5.0 | 1.5 |
| 2.0 | 0.0 | 100.0 | 1.5 |
| 3.0 | 0.0 | 100.0 | 1.5 |
| 3.4 | 95.0 | 5.0 | 1.5 |

| | | |
|---|---|---|
| Methode 12: | Säule: | YMC-Pack ODS-AQ, 3.0 µM, 4.6 x 75 mm |
| | Detektion: | 230 - 360 nm |
| | Fließmittel A: | Wasser / 0.1 % Ameisensäure |
| | Fließmittel B: | Acetonitril / 0.1 % Ameisensäure |
| | Gradient: | |

| Zeit in min | %A | %B | Flussrate in mL/min |
|---|---|---|---|
| 0.0 | 95.0 | 5.0 | 1.6 |
| 4.5 | 10.0 | 90.0 | 1.6 |
| 5.0 | 10.0 | 90.0 | 1.6 |
| 5.5 | 90.0 | 10.0 | 1.6 |

Folgende Mikrowellen-Apparatur wurde verwendet: Biotage EmrysOptimizer^{™}

### Herstellung der Endverbindungen

### Beispiel 1

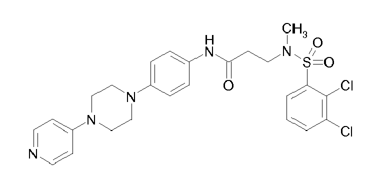

1a) 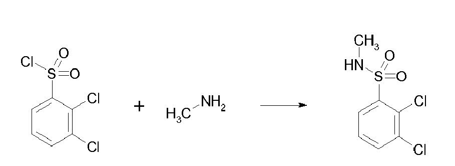 Eine Mischung aus 1.0 g (4.07 mMol) 2,3-Dichlorbenzolsulfonsäurechlorid, 0.33 g (4.89 mMol) Methylamin Hydrochlorid, 2.73 ml (19.55 mMol) Triethylamin und 20 ml Dichlormethan wird über Nacht bei Raumtemperatur gerührt. Die Reaktionsmischung wird anschließend je einmal mit 1N HCl, gesättigter Natriumhydrogencarbonat-Lösung, Wasser und gesättigter Natriumchloridlösung gewaschen, über Natriumsulfat getrocknet und dann bis zur Trockne eingeengt.
   C₇H₇Cl₂NO₂S (240.11)
   [M+H]+ = 240/242/244
   DC: Kieselgel, Petrolether / Ethylacetat 2:1, Rf-Wert = 0.50
1b) 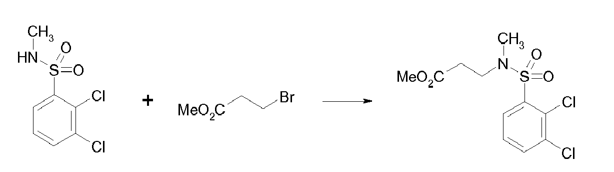 Eine Mischung aus 0.9 g (3.75 mMol) Produkt aus 1a und 20 ml DMF wird vorgelegt und mit 1.55 g (11.24 mMol) Kaliumcarbonat und 0.49 ml (4.50 mMol) 3-Brompropionsäureethylester versetzt. Die Reaktionsmischung wird über Nacht bei Raumtemperatur gerührt und dann mit Wasser versetzt. Man extrahiert zweimal mit Ethylacetat. Die organischen Extrakte werden dreimal mit Wasser und einmal mit gesättigter Natriumchloridlösung gewaschen, über Natriumsulfat getrocknet und zur Trockne eingeengt.
   C₁₁H₁₃Cl₂NO₄S (326.20)
   [M+H]+ = 326/328/330
   DC: Kieselgel, Petrolether / Ethylacetat 2:1, Rf-Wert = 0.45
1c) 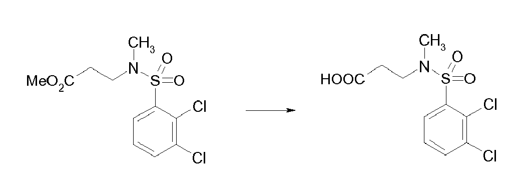 Eine Mischung aus 1.15 g (3.53 mMol) Produkt aus 1b, 0.74 g (17.63 mMol) Lithiumhydroxid Monohydrat, 15 ml THF und 15 ml Wasser wird eine Stunde bei Raumtemperatur gerührt. Danach wird das THF im Vakuum entfernt und der Rückstand mit konzentrierter HCl sauer gestellt. Die Reaktionsmischung wird anschließend dreimal mit Ethylacetat extrahiert. Die organischen Extrakte werden mit gesättigter Natriumchlorid-lösung gewaschen, über Natriumsulfat getrocknet und zur Trockne eingeengt. Das Rohprodukt wird mit Diethylether verrieben und abgesaugt.
   C₁₀H₁₁Cl₂NO₄S (312.17)
   [M+H]+ = 310/312/314
   DC: Kieselgel, Petrolether / Ethylacetat 2:1, Rf-Wert = 0.03
1d) 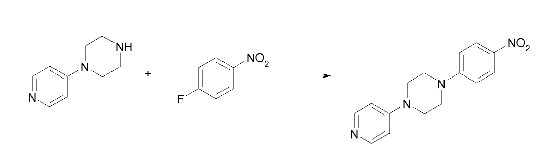 Eine Mischung aus 5.0 g (30.63 mMol) 1-Pyridin-4-yl-piperazin, 4.32 g (30.63 mMol) 1-Fluor-4-nitrobenzol (Aldrich), 10.62 ml (76.59 mMol) Triethylamin und 100 ml DMF wird 50 min unter Rückfluß erhitzt und anschließend bis zur Trockene eingeengt. Das so erhaltene Rohprodukt wird durch Säulenchromatographie über Kieselgel (Elutionsmittel: Dichlormethan / Ethanol / Ammoniak 12:1:0.1 bis 10:1:0.1) gereinigt.
   C₁₅H₁₆N₄O₂ (284.31)
   [M+H]+ = 285
   DC: Kieselgel, Dichlormethan / Methanol / Ammoniak 9:1:0.1, Rf-Wert = 0.52
1e) 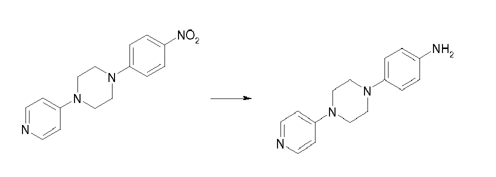 Eine Mischung aus 4.95 g (17.41 mMol) Produkt aus 1d, 0.6 g Palladium auf Kohle (10%), 120 ml Dichlormethan und 20 ml Methanol wird fünf Stunden im Autoklaven bei Raumtemperatur hydriert. Anschließend wird abgesaugt und der Filterkuchen noch sechsmal mit Dichlormethan / Methanol 1:1 aufgekocht und wieder abgesaugt. Die vereinigten Filtrate werden im Vakuum bis zur Trockene eingeengt.
   C₁₅H₁₈N₄ (254.33)
   [M+H]+ = 255
1f) 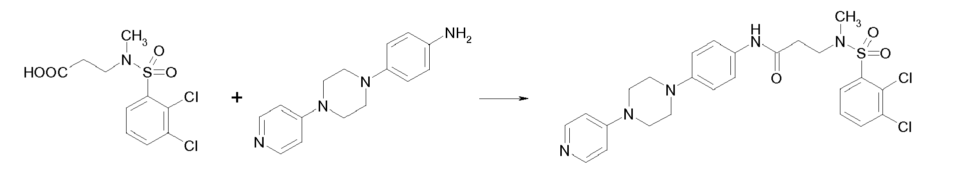 Eine Mischung aus 1.25 g (4.00 mMol) Produkt aus 1c, 2.0 ml (14.34 mMol) Triethylamin, 1.28 g (4.00 mMol) TBTU und 7 ml DMF wird 45 min bei Raumtemperatur gerührt.
   Danach wird 1.0 g (3.93 mMol) Produkt aus 1e hinzugefügt und über Nacht weiter bei Raumtemperatur gerührt. Anschließend wird die Reaktionsmischung in Wasser geschüttet und mit Dichlormethan extrahiert. Die organischen Extrakte werden mit Wasser gewaschen, über Na₂SO₄ getrocknet und bis zur Trockene eingeengt. Das so erhaltene Rohprodukt wurde durch Säulenchromatographie über Kieselgel (Elutionsmittel:
   Dichlormethan mit 5-20 % Methanol) gereinigt.
   C₂₅H₂₇Cl₂N₅O₃S (548.49)
   [M+H]+ = 548/550/552
   DC: Kieselgel, Dichlormethan / Methanol 4:1, Rf-Wert = 0.65

### Beispiel 2

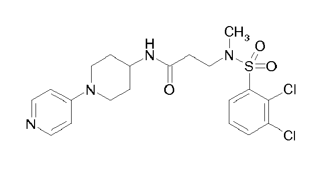

2a) 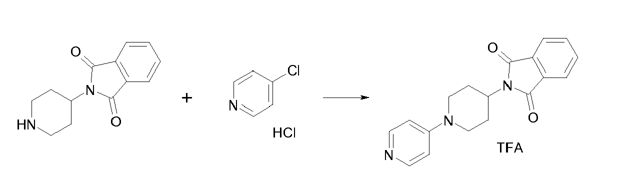 Eine Mischung aus 0.5 g (2.17 mMol) N-(1-Benzylpiperidin-4-yl)-phthalimid (Bioorg. Med. Chem. Lett. 11, 2001, 2325-2330), 0.33 g (2.17 mMol) 4-Chlorpyridin Hydrochlorid, 1.2 ml (8.69 mMol) Triethylamin und 2.4 ml Ethanol absolut wird eine Stunde in der Mikrowelle auf 150°C erhitzt. Die Reaktionsmischung wird anschließend mit Ethanol verdünnt, der entstandene Niederschlag wird abfiltriert. Man engt das Filtrat bis zur Trockene ein und reinigt das Rohprodukt durch präparative HPLC.
   C₁₈H₁₇N₃O₂ x C₂HF₃O₂ (421.37)
   [M+H]+ = 308
   HPLC (Methode 1): Retentionszeit = 2.07 min
2b) 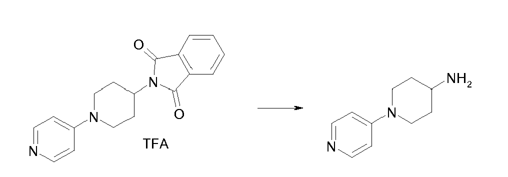 Eine Mischung aus 0.3 g (0.71 mMol) Produkt aus 2a, 0.09 g (1.42 mMol) Hydrazinhydrat 80%ig und 6 ml Ethanol absolut wird vier Stunden unter Rückfluß erhitzt. Die Reaktionsmischung wird anschließend auf 0°C abgekühlt, der entstandene Niederschlag wird abfiltriert. Man engt das Filtrat bis zur Trockene ein.
   C₁₀H₁₅N₃ (177.25)
   [M+H]+ = 178
2c) 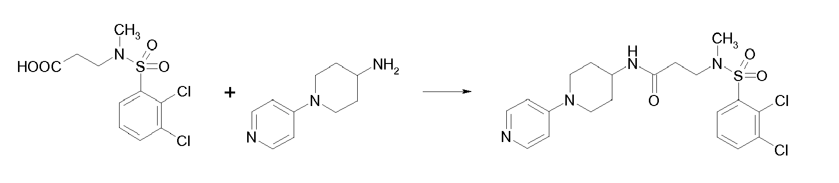 Beispiel 2 wird analog zu 1f aus 0.22 g (0.71 mMol) Produkt aus 1c, 0.24 g (1.35 mMol) Produkt aus 2b, 0.3 ml (2.13 mMol) Triethylamin und 0.23 g (0.71 mMol) TBTU in 5.5 ml DMF hergestellt.
   C₂₀H₂₄Cl₂N₄O₃S (471.40)
   [M+H]+ = 471/473/475
   DC: Kieselgel, Dichlormethan / Methanol 9:1, Rf-Wert = 0.2

### Beispiel 3

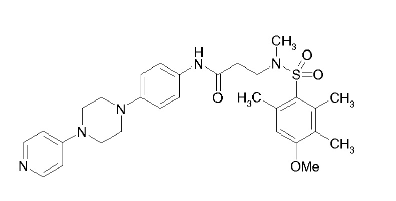

3a) 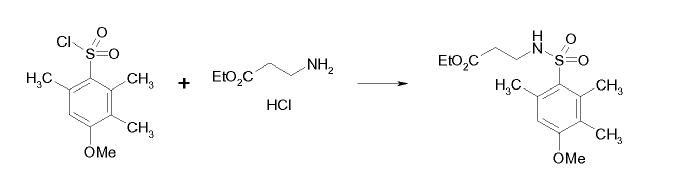 Eine Mischung aus 0.99 g (4.00 mMol) 4-Methoxy-2,3,6-trimethyl-benzolsulfonylchlorid, 0.69 g (4.51 mMol) β-Alaninethylester Hydrochlorid, 2.23 ml (15.98 mMol) Triethylamin und 20 ml Dichlormethan wird über Nacht bei Raumtemperatur gerührt. Die Reaktionsmischung wird anschließend mit 0.5 M HCl, gesättigter Natriumhydrogencarbonatlösung, Wasser und gesättigter Natrium-chloridlösung gewaschen, über Natriumsulfat getrocknet und im Vakuum bis zur Trockene eingeengt.
   C₁₅H₂₃NO₅S (329.41)
   [M+H]+ = 330
   DC: Kieselgel, Petrolether / Ethylacetat 2:1, Rf-Wert = 0.43
3b) 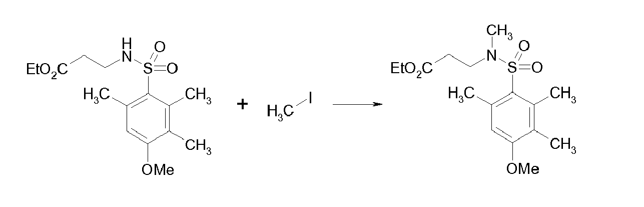 Eine Mischung aus 1.24 g (3.76 mMol) Produkt aus 3a, 0.84 ml (13.55 mMol) Methyliodid, 1.04 g (7.53 mMol) Kaliumcarbonat wasserfrei und 10 ml DMF wird fünf Stunden bei Raumtemperatur gerührt. Die Reaktionsmischung wird anschließend im Vakuum bis zur Trockene eingeengt, der Rückstand wird in Ethylacetat aufgenommen. Man wäscht mit Wasser, gesättigter Natriumhydrogencarbonatlösung und gesättigter Natriumchloridlösung, trocknet über Natriumsulfat und engt im Vakuum bis zur Trockene ein.
   C₁₆H₂₅NO₅S (343.44)
   [M+H]+ = 344
   DC: Kieselgel, Petrolether / Ethylacetat 2:1, Rf-Wert = 0.52
3c) 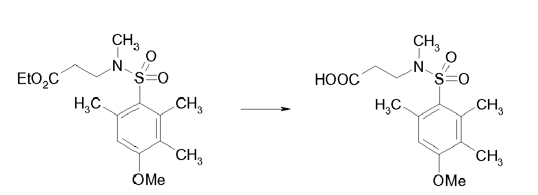 Die Säure wird analog zu 1c aus 1.29 g (3.76 mMol) Produkt aus 3b, 0.79 g (18.80 mMol) Lithiumhydroxid Monohydrat, 15 ml THF und 15 ml Wasser hergestellt.
   C₁₄H₂₁NO₅S (315.39)
   [M+H]+ = 316
   DC: Kieselgel, Petrolether / Ethylacetat 2:1, Rf-Wert = 0.07
3d) 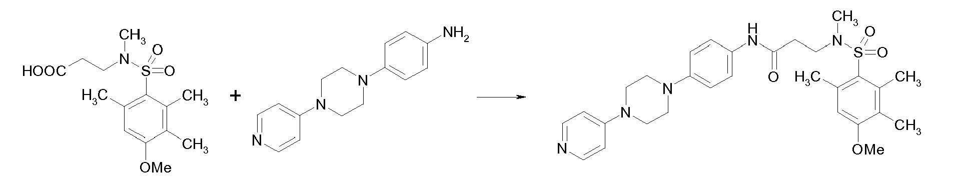 Beispiel 3 wird analog zu 1f aus 0.15 g (0.47 mMol) Produkt aus 3c, 0.12 g (0.47 mMol) Produkt aus 1e, 0.2 ml (1.43 mMol) Triethylamin und 0.15 g (0.48 mMol) TBTU in 8 ml
   DMF hergestellt.
   C₂₉H₃₇N₅O₄S (551.70)
   [M+H]+ = 552
   DC: Kieselgel, Dichlormethan / Methanol 9:1, Rf-Wert = 0.38

### Beispiel 4

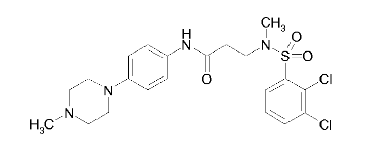

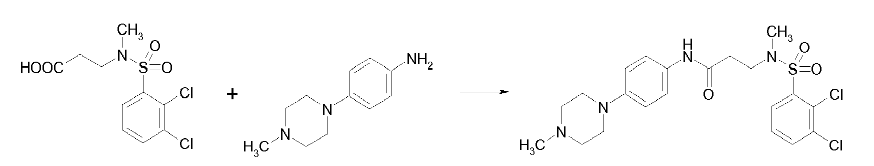

Beispiel 4 wird analog zu 1f aus 0.39 g (1.26 mMol) Produkt aus 1c, 0.24 g (1.26 mMol) 4-(4-Methylpiperazin-1-yl)-anilin (J. Med. Chem. SIR 48, 7, 2005, 2371-2387), 0.35 ml (2.51 mMol) Triethylamin und 0.50 g (1.32 mMol) HATU in 5 ml DMF hergestellt.
C₂₁H₂₆CL₂N₄O₃S (485.43)
[M+H]+ = 485/487/489
HPLC (Methode 2): Retentionszeit = 2.64 min

### Beispiel 5

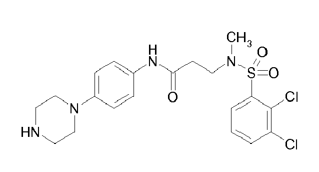

5a) 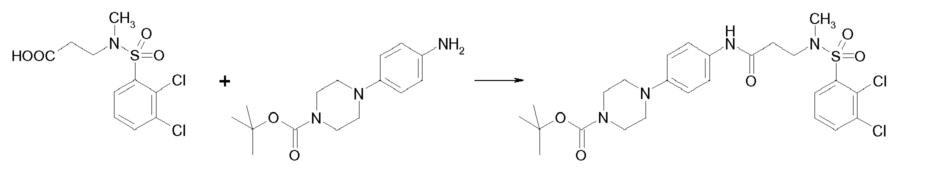 5a wird analog zu 1f aus 0.39 g (1.26 mMol) Produkt aus 1 c, 0.24 g (1.26 mMol) 4-(4-Methylpiperazin-1-yl)-anilin (J. Med. Chem. SIR 48, 7, 2005, 2371-2387), 0.35 ml (2.51 mMol) Triethylamin und 0.50 g (1.32 mMol) HATU in 5 ml DMF hergestellt.
   C₂₅H₃₂CL₂N₄O₅S (571.52)
5b) 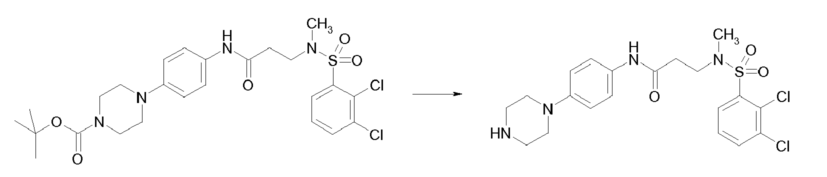 Eine Mischung aus 0.60 g (1.05 mMol) Produkt aus 5a, 3 ml TFA und 3 ml Dichlormethan wird zwei Stunden bei Raumtemperatur gerührt. Man engt die Reaktionsmischung bis zur Trockene ein und reinigt das Rohprodukt durch präparative HPLC.
   C₂₀H₂₄Cl₂N₄O₃S (471.40)
   [M+H]+ = 471/473/475
   HPLC (Methode 2): Retentionszeit = 2.58 min

### Beispiel 6

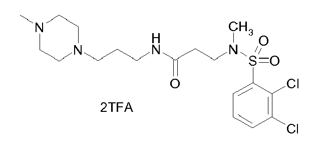

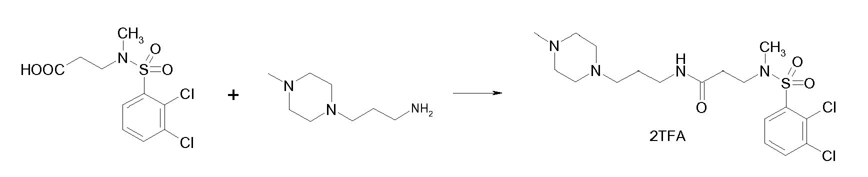

Beispiel 6 wird analog zu 1f aus 0.22 g (0.71 mMol) Produkt aus 1c, 0.12 g (0.78 mMol) 3-(4-Methylpiperazin-1-yl)-propylamin (Bioorg. Med. Chem. Lett. 13, 2003, 2131-2136), 0.30 ml (2.13 mMol) Triethylamin und 0.23 g (0.71 mMol) TBTU in 5.5 ml THF hergestellt. C₁₈H₂₈Cl₂N₄O₃S x 2C₂HF₃O₂ (679.46)
[M+H]+ = 451/453/455
HPLC (Methode 5): Retentionszeit = 1.37 min

### Beispiel 7

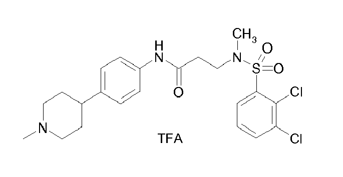

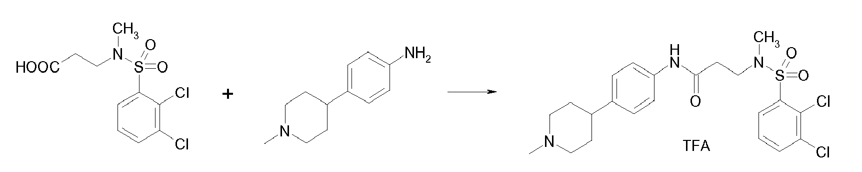

Beispiel 7 wird analog zu 1f aus 0.22 g (0.71 mMol) Produkt aus 1c, 0.14 g (0.71 mMol) 4-(1-Methylpiperidin-4-yl)-anilin (JW Pharmlab), 0.30 ml (2.13 mMol) Triethylamin und 0.23 g (0.71 mMol) TBTU in 5.5 ml THF hergestellt.

C₂₂H₂₇Cl₂N₃O₃S x C₂HF₃O₂ (598.46)
[M+H]+ = 484/486/488
HPLC (Methode 5): Retentionszeit = 1.57 min

### Beispiel 8

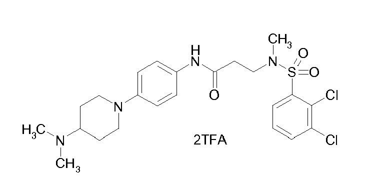

8a) 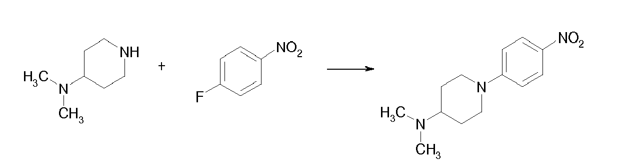 8a wird analog zu 1d aus 0.5 g (3.90 mMol) 4-Dimethylamino-piperidin (Alfa Aesar), 0.44 g (4.18 mMol) 1-Fluor-4-nitrobenzol (Aldrich) und 1.33 ml (76.59 mMol) Triethylamin in 12 ml DMF hergestellt.
   C₁₃H₁₉N₃O₂ (249.31)
   [M+H]+ = 250
8b) 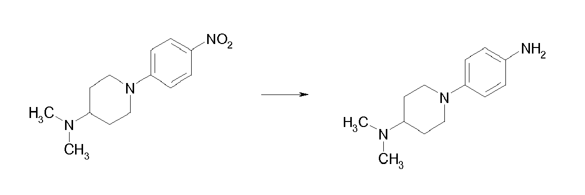 Eine Mischung aus 1.66 g (6.67 mMol) Produkt aus 8a, 0.17 g Palladium auf Kohle (5%) und 132 ml Ethanol wird im Autoklaven bei Raumtemperatur hydriert. Anschließend wird der Katalysator abgesaugt und das Filtrat im Vakuum bis zur Trockene eingeengt.
   C₁₃H₂₁N₃ (219.33)
   [M+H]+ = 220
   DC: Kieselgel, Dichlormethan / Methanol 9:1, Rf-Wert = 0.1
8c) 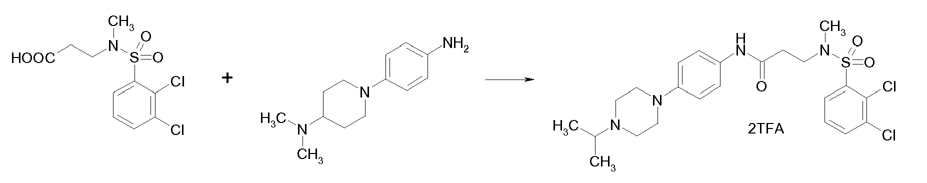 Beispiel 8 wird analog zu 1f aus 0.22 g (0.71 mMol) Produkt aus 1c, 0.16 g (0.71 mMol) Produkt aus 8b, 0.30 ml (2.13 mMol) Triethylamin und 0.23 g (0.71 mMol) TBTU in 5.5 ml THF hergestellt.
   C₂₃H₃₀Cl₂N₄O₃S x 2C₂HF₃O₂ (741.53)
   [M+H]+ = 513/515/517
   HPLC (Methode 5): Retentionszeit = 1.46 min

### Beispiel 9

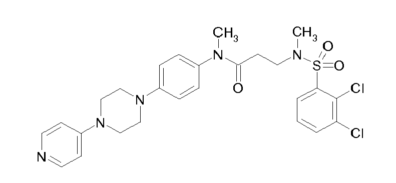

9a) 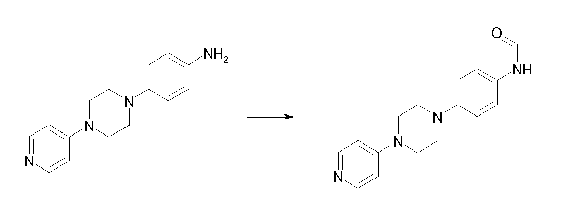 13 ml Essigsäureanhydrid werden vorgelegt und langsam mit 8 ml Ameisensäure versetzt. Die Reaktionsmischung wird 1.5 Stunden auf 50°C erhitzt und dann mit 80 ml Dichlormethan versetzt. Unter Eisbadkühlung werden anschließend 5.0 g (19.66 mMol) hinzugegeben. Es wird eine Stunde bei Raumtemperatur gerührt und danach bis zur Trockene eingeengt. Der Rückstand wird mit halbgesättigter Natriumhydrogencarbonat-Lösung versetzt und mit Dichlormethan zweimal extrahiert. Die organischen Extrakte werden mit gesättigter Natriumchlorid-Lösung gewaschen, über Natriumsulfat getrocknet und bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
   C₁₆H₁₈N₄O (282.34)
   [M+H]+ = 283
9b) 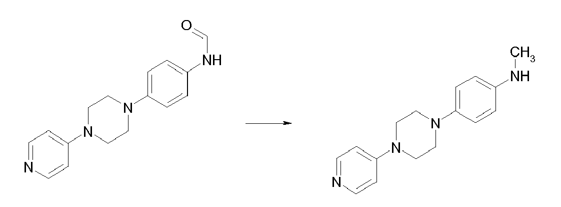 Bei 60°C wird eine Mischung aus 10.63 ml Lithiumaluminiumhydrid 2 M in THF (21.25 mMol) und 50 ml THF langsam mit 3.0 g (10.63 mMol) Produkt aus 9a versetzt. Die Reaktionsmischung wird acht Stunden bei 60°C und vier Stunden bei Raumtemperatur gerührt. Unter Eisbadkühlung werden anschließend 20 ml Wasser hinzugegeben. Es wird über Celite filtriert und mit THF und Dichlormethan nachgewaschen. Das Filtrat wird bis zur Trockene eingeengt. Der Rückstand wird mit Dichlormethan versetzt, mit Wasser und 1 M Natronlauge gewaschen, über Natriumsulfat-Lösung getrocknet und bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
   C₁₆H₂₀N₄ (268.36)
9c) 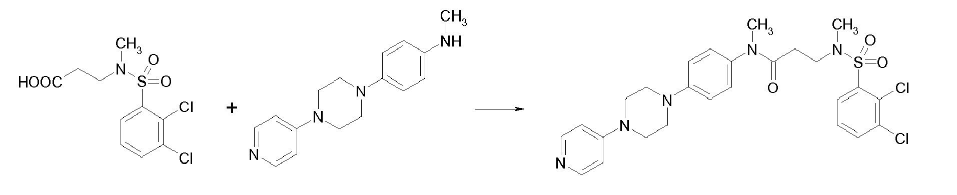 Beispiel 9 wird analog zu 1f aus 0.15 g (0.48 mMol) Produkt aus 1c, 0.14 g (0.51 mMol) Produkt aus 9b, 0.13 ml (0.96 mMol) Triethylamin und 0.19 g (0.51 mMol) HATU in 5 ml DMF hergestellt.
   C₂₆H₂₉Cl₂N₅O₃S (562.51)
   [M+H]+ = 562/564/566
   HPLC (Methode 2): Retentionszeit = 2.86 min

### Beispiel 10

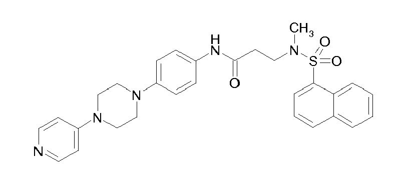

10a) 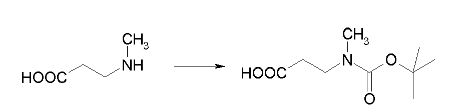 Eine Mischung aus 1.0 g (9.70 mMol) N-Methyl-β-alanin (Convertex), 24 ml Dioxan, 12 ml Wasser und 2.68 g (19.38 mMol) Kaliumcarbonat wasserfrei wird unter Eisbadkühlung mit 2.33 g (10.66 mMol) Boc-Anhydrid versetzt. Die Reaktionsmischung wird drei Tage bei Raumtemperatur gerührt. Danach wird das Dioxan im Vakuum entfernt. Der wässrige Rückstand wird mit Ethylacetat extrahiert (Ethylacetat-Phasen verwerfen), anschließend mit 1 M Salzsäure leicht sauer gestellt und dann mit Dichlormethan extrahiert. Die organischen Dichlormethan-Extrakte werden mit gesättigter Natriumchlorid-Lösung gewaschen, über Natriumsulfat getrocknet und bis zur Trockene eingeengt.
   C₉H₁₇NO₄ (203.24)
   [M+H]+ = 204
10b) 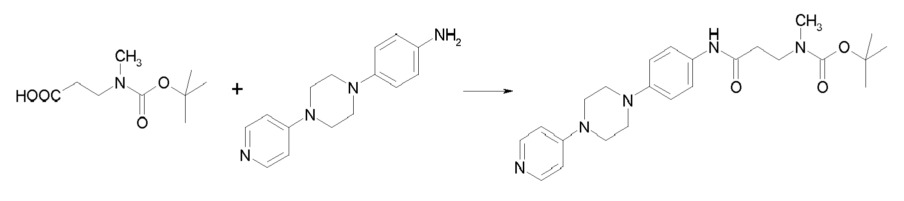 10b wird analog zu 1f aus 1.85 g (9.10 mMol) Produkt aus 10a, 2.32 g (9.10 mMol) Produkt aus 1 e, 3.81 ml (27.31 mMol) Triethylamin und 2.92 g (9.10 mMol) TBTU in 80 ml DMF hergestellt.
   C₂₄H₃₃N₅O₃ (439.55)
   [M+H]+ = 440
   DC: Kieselgel, Dichlormethan / Methanol 9:1, Rf-Wert = 0.49
10c) 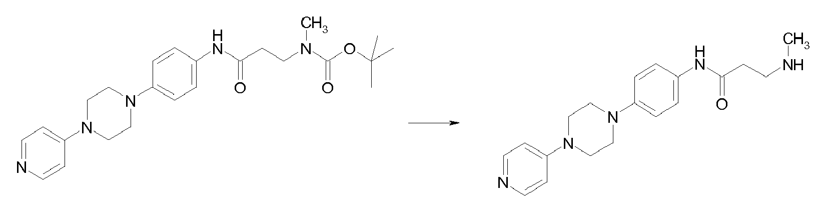 Eine Mischung aus 3.20 g (7.28 mMol) Produkt aus 10b, 20 ml TFA und 60 ml Dichlormethan wird 30 min bei Raumtemperatur gerührt. Danach wird die Reaktionsmischung im Vakuum bis zur Trockene eingeengt. Das so erhaltene Rohprodukt wird durch Säulen-chromatographie über Kieselgel (Elutionsmittel: Dichlormethan / Ethanol / Ammoniak 9:1:0.1 bis 4:1:0.1) gereinigt.
   C₁₉H₂₅N₅O (339.43)
   [M+H]+ = 340
   DC: Kieselgel, Dichlormethan / Methanol / Ammoniak 9:1:0.1, Rf-Wert = 0.25
10d) 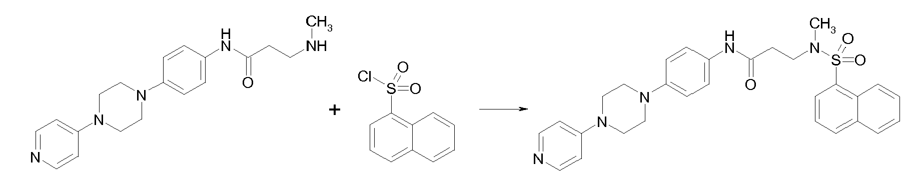 Eine Mischung aus 0.1 g (0.30 mMol) Produkt aus 10c, 0.056 g (0.25 mMol) 1-Naphthylsulfonsäurechlorid, 0.137 ml (0.98 mMol) Triethylamin und 5 ml Dichlormethan wird über Nacht bei Raumtemperatur gerührt. Danach wird die Reaktionsmischung im Vakuum bis zur Trockene eingeengt. Das so erhaltene Rohprodukt wird durch Säulenchromatographie über Kieselgel (Elutionsmittel: Dichlormethan / Ethanol / Ammoniak 12:1:0.1) gereinigt.
   C₂₉H₃₁N₅O₃S (529.65)
   [M+H]+ = 530
   DC: Kieselgel, Dichlormethan / Methanol / Ammoniak 9:1:0.1, Rf-Wert = 0.44

### Beispiel 11

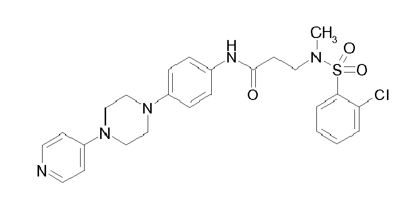

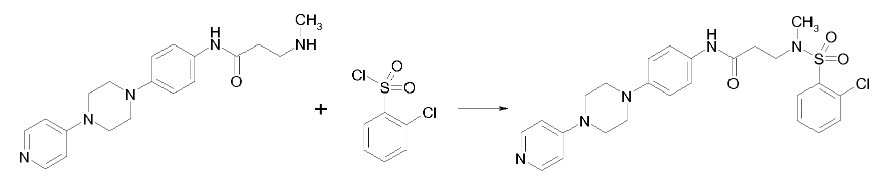

Beispiel 11 wird analog zu 10d aus 0.10 g (0.30 mMol) Produkt aus 10c, 0.052 g (0.25 mMol) 2-Chlorbenzolsulfonsäurechlorid, 0.14 ml (98 mMol) Triethylamin in 5 ml Dichlormethan hergestellt.
C₂₅H₂₈ClN₅O₃S (514.04)
[M+H]+ = 514/516
DC: Kieselgel, Dichlormethan / Methanol / Ammoniak 9:1:0.1, Rf-Wert = 0.47

### Beispiel 12

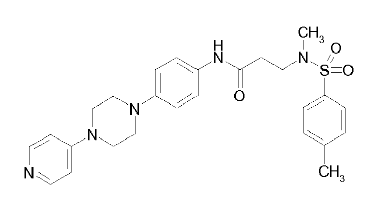

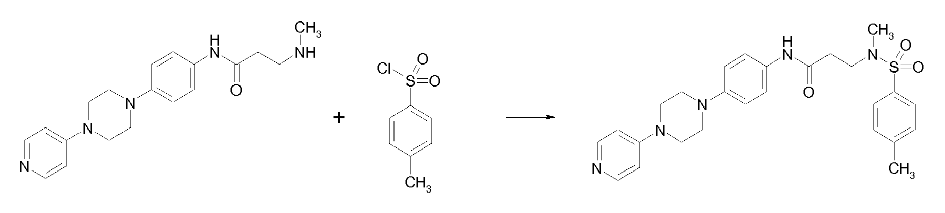

Beispiel 12 wird analog zu 10d aus 0.10 g (0.30 mMol) Produkt aus 10c, 0.047 g (0.25 mMol) p-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
DC: Kieselgel, Dichlormethan / Methanol / Ammoniak 9:1:0.1, Rf-Wert = 0.43

### Beispiel 13

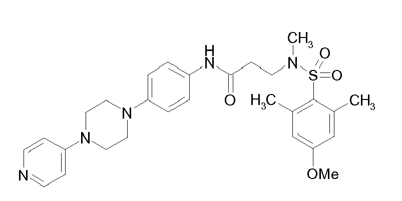

13a) 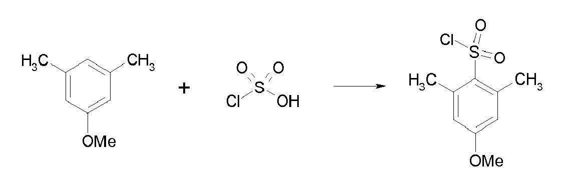 Eine Mischung aus 2.0 g (14.69 mMol) 3,5-Dimethylanisol und 20 ml Dichlormethan wird unter Eisbadkühlung mit 5.85 ml (88.0 mMol) Chlorsulfonsäure versetzt. Die Reaktionsmischung wird danach 20 min bei Raumtemperatur gerührt und anschließend auf 50 ml Eiswasser gegossen. Man extrahiert mit 100 ml Dichlormethan. Die organischen Extrakte werden mit 5%iger Natriumhydrogencarbonat-Lösung gewaschen, über Natriumsulfat getrocknet und bis zur Trockene eingeengt.
   C₉H₁₁ClO₃S (234.70)
   [M+H]+ = 234/236
   DC: Kieselgel, Petrolether / Ethylacetat 9:1, Rf-Wert = 0.46
13b) 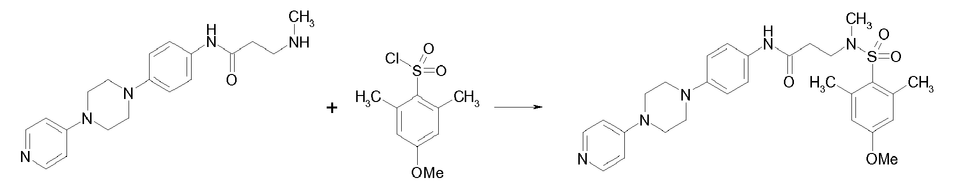 Beispiel 13 wird analog zu 10d aus 0.10 g (0.30 mMol) Produkt aus 10c, 0.058 g (0.25 mMol) Produkt aus 13a, 0.14 ml (98 mMol) Triethylamin in 5 ml Dichlormethan hergestellt.
   C₂₈H₃₅N₅O₄S (537.67)
   [M+H]+ = 538
   DC: Kieselgel, Dichlormethan / Methanol / Amoniak 9:1:0.1, Rf-Wert = 0.62

### Beispiel 14

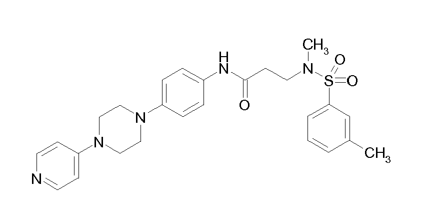

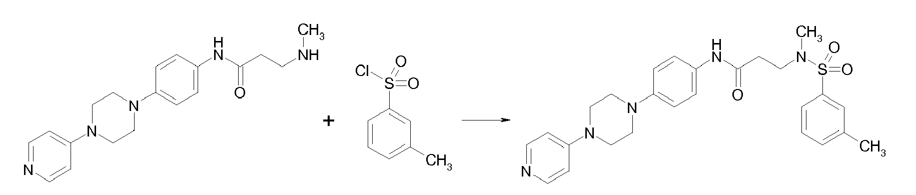

Beispiel 14 wird analog zu 10d aus 0.10 g (0.30 mMol) Produkt aus 10c, 0.047 g (0.25 mMol) m-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
DC: Kieselgel, Dichlormethan / Methanol / Ammoniak 9:1:0.1, Rf-Wert = 0.47

### Beispiel 15

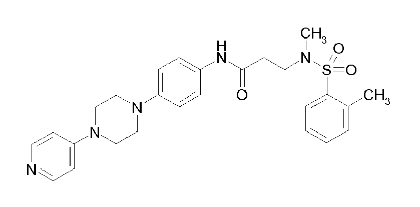

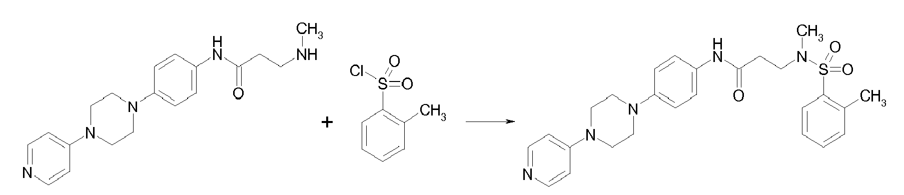

Beispiel 15 wird analog zu 10d aus 0.10 g (0.30 mMol) Produkt aus 10c, 0.047 g (0.25 mMol) o-Toluolsulfonsäurechlorid, 0.14 ml (98 mMol) Triethylamin in 5 ml Dichlormethan hergestellt.
C₂₆H₃₁N₅O₃S (493.62)
[M+H]+ = 494
HPLC (Methode 1): Retentionszeit = 2.37 min

### Beispiel 16

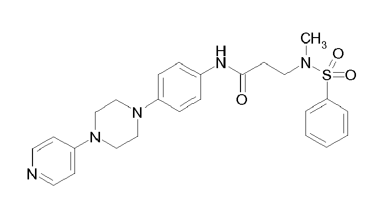

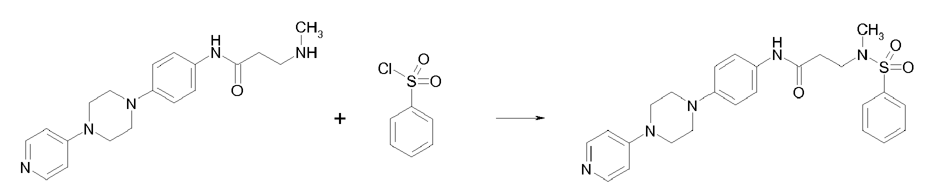

Beispiel 16 wird analog zu 10d aus 0.10 g (0.30 mMol) Produkt aus 10c, 0.043 g (0.25 mMol) Benzolsulfonsäurechlorid, 0.14 ml (98 mMol) Triethylamin in 5 ml Dichlormethan hergestellt.
C₂₅H₂₉N₅O₃S (479.60)
[M+H]+ = 480
HPLC (Methode 1): Retentionszeit = 2.40 min

### Beispiel 17

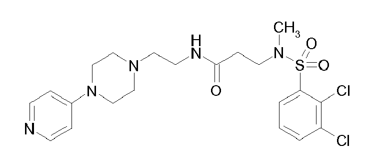

17a) 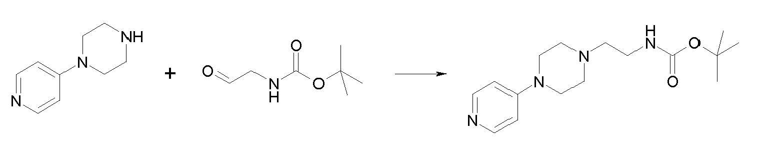 Eine Mischung aus 1.03 g (6.28 mMol) 1-(4-Pyridyl)-piperazin (Girindus) und 50 ml Dichlormethan wird mit 1.0 g (6.28 mMol) *tert*-Butyl-N-(2-oxoethyl)-carbamat (Aldrich) versetzt. Die Reaktionsmischung wird danach 30 min bei Raumtemperatur gerührt, anschließend unter Eisbadkühlung mit 2.66 g (12.56 mMol) Natrium-triacetoxyborhydrid versetzt und danach über Nacht bei Raumtemperatur gerührt. Man gibt weitere 60 ml Dichlormethan hinzu und wäscht die Reaktionsmischung mit gesättigter Natriumhydrogencarbonat-Lösung und gesättigter Natriumchlorid-Lösung. Die organische Phase wird über Natriumsulfat getrocknet und bis zur Trockene eingeengt. Das so erhaltene Rohprodukt wird durch Säulenchromatographie über Kieselgel (Elutionsmittel: Dichlormethan / Ethanol / Ammoniak 14:1:0.1 bis 10:1:0.1) gereinigt.
   C₁₆H₂₆N₄O₂ (306.40)
   [M+H]+ = 307
17b) 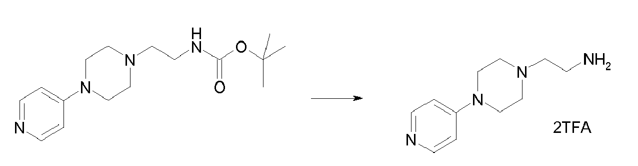 Eine Mischung aus 0.36 g (1.19 mMol) Produkt aus 17a, 1.19 ml (15.50 mMol) TFA und 2 ml Dichlormethan wird zwei Stunden bei Raumtemperatur gerührt. Danach wird die Reaktionsmischung im Vakuum bis zur Trockene eingeengt.
   C₁₁H₁₈N₄x 2C₂HF₃O₂ (434.33)
   [M+H]+ = 207
   HPLC (Methode 2): Retentionszeit = 0.98 min
17c) 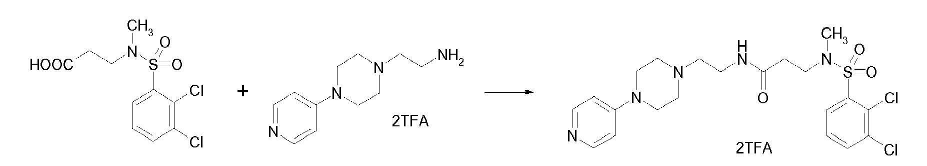 Beispiel 17 wird analog zu 1f aus 0.22 g (0.71 mMol) Produkt aus 1c, 0.34 g (0.78 mMol) Produkt aus 17b, 0.50 ml (3.56 mMol) Triethylamin und 0.23 g (0.71 mMol) TBTU in 5.5 ml THF hergestellt.
   C₂₁H₂₇Cl₂N₅O₃S x 2C₂HF₃O₂ (728.49)
   [M+H]+ = 500/502/504
   HPLC (Methode 2): Retentionszeit = 3.14 min

### Beispiel 18

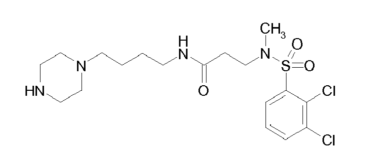

18a) 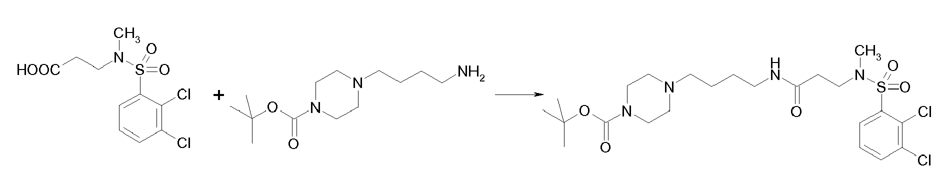 18a wird analog zu 1f aus 0.20 g (0.64 mMol) Produkt aus 1c, 0.17 g (0.64 mMol) 4-(4-Aminobutyl)-piperazin-1-carbonsäure-tert-butylester (J. Med. Chem. 47, 2004, 4300-4315), 0.27 ml (1.92 mMol) Triethylamin und 0.21 g (0.64 mMol) TBTU in 5 ml THF hergestellt.
   C₂₃H₃₆CL₂N₄O₅S (551.53)
   M+H]+ = 551/553/555
   DC: Kieselgel, Dichlormethan / Methanol 30:1, Rf-Wert = 0.1
18b) 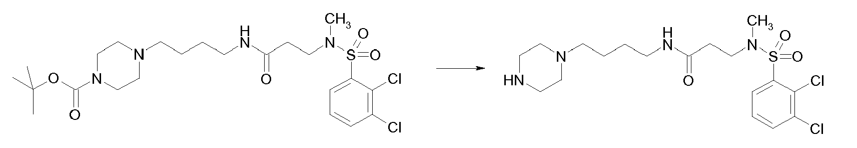 Eine Mischung aus 0.29 g (0.53 mMol) Produkt aus 18a, 0.53 ml TFA und 1 ml Dichlormethan wird zwei Stunden bei Raumtemperatur gerührt. Die Reaktionsmischung wird mit gesättigter Natriumhydrogencarbonat-Lösung gewaschen. Nach der Phasentrennung wird die wässrige Phase noch dreimal mit Dichlormethan extrahiert. Die organischen Extrakte werden über Natriumsulfat getrocknet und bis zur Trockene im Vakuum eingeengt.
   C₁₈H₂₈Cl₂N₄O₃S (451.41)
   [M+H]+ = 451/453/455
   HPLC (Methode 2): Retentionszeit = 2.22 min

### Beispiel 19

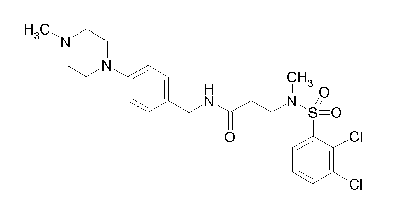

19a) 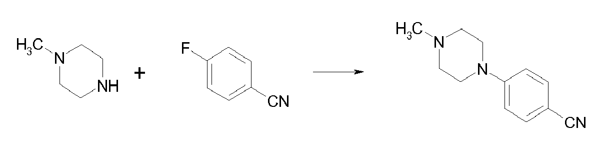 Eine Mischung aus 5.0 ml (45.13 mMol) N-Methylpiperazin und 0.73 g (6.00 mMol) 4-Fluorbenzonitril (Aldrich) wird 12 Stunden auf 80°C erhitzt. Danach wird bis zur Trockene eingeengt und der Rückstand mit Wasser versetzt. Es wird dreimal mit Ethylacetat extrahiert. Die organischen Extrakte werden über Natriumsulfat getrocknet und im Vakuum bis zur Trockene eingeengt.
   C₁₂H₁₅N₃ (201.27)
   M+H]+ = 202
   DC: Kieselgel, Dichlormethan / Ethanol 95:5, Rf-Wert = 0.31
19b) 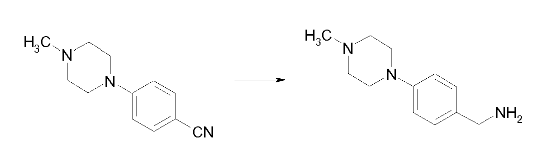 Eine Mischung aus 1.17 g (5.81 mMol) Produkt aus 19a, 0.3 g Raney-Nickel und 50 ml methanolische Ammoniaklösung wird bei 50°C im Autoklaven hydriert. Danach wird der Katalysator abfiltriert und das Filtrat im Vakuum bis zur Trockene eingeengt.
   C₁₂H₁₉N₃ (205.30)
   M+H]+ = 206
19c) 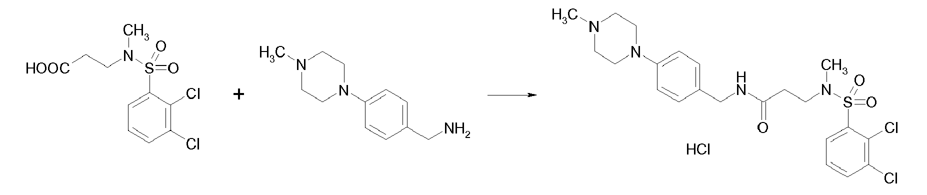 Beispiel 19 wird analog zu 1f aus 0.16 g (0.50 mMol) Produkt aus 1c, 0.10 g (0.50 mMol) Produkt aus 19b, 0.14 ml (1.00 mMol) Triethylamin und 0.16 g (0.50 mMol) TBTU in 3 ml DMF hergestellt.
   C₂₂H₂₈Cl₂N₄O₃S x HCl (535.91)
   [M+H]+ = 499/501/503
   HPLC (Methode 3): Retentionszeit = 3.49 min

### Beispiel 20

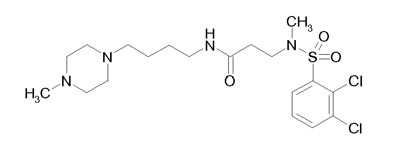

20a) 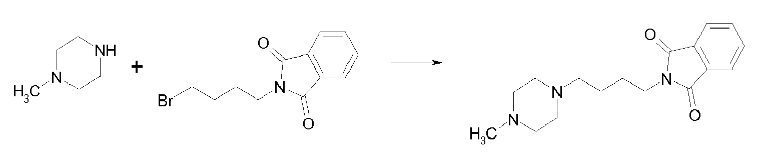 Eine Mischung aus 0.5 g (4.99 mMol) *N*-Methylpiperazin (Aldrich), 1.41 g (4.99 mMol) N-(4-Brombutyl)-phthalimid (Fluka), 0.86 ml (4.99 mMol) DIPEA und 9.3 ml Acetonitril wird 45 min in der Mikrowelle auf 120°C erhitzt. Danach wird bis zur Trockene eingeengt. Das so erhaltene Rohprodukt wird durch Säulenchromatographie über Kieselgel (Elutionsmittel: Dichlormethan / Methanol 98:2) gereinigt.
   C₁₇H₂₃N₃O₂ (301.38)
   M+H]+ = 302
20b) 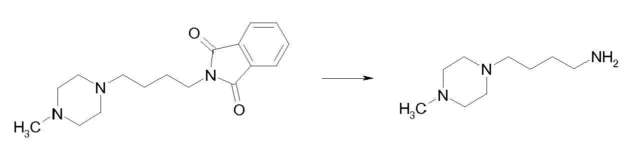 Eine Mischung aus 1.94 g (6.44 mMol) Produkt aus 20a, 1.61 g (25.75 mMol) Hydrazinhydrat Hydrat und 15 ml Ethanol absolut wird 5.5 Stunden im Autoklaven auf 120°C erhitzt. Der entstandene Niederschlag wird abfiltriert. Man engt danach das Filtrat bis zur Trockene ein.
   C₉H₂₁N₃ (171.28)
20c) 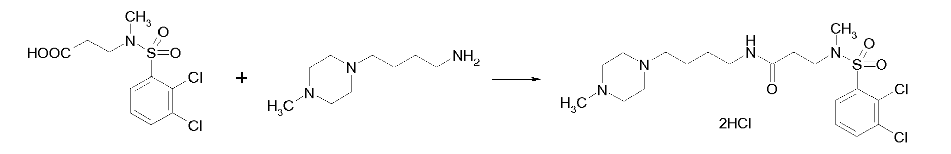 Beispiel 20 wird analog zu 1f aus 0.50 g (1.61 mMol) Produkt aus 1c, 0.55 g (3.22 mMol) Produkt aus 20b, 0.67 ml (4.83 mMol) Triethylamin und 0.52 g (1.61 mMol) TBTU in 30 ml DMF hergestellt.
   C₁₉H₃₀Cl₂N₄O₃S x 2HCl (538.36)
   [M+H]+ = 465/467/469
   HPLC (Methode 1): Retentionszeit = 2.15 min

### Beispiel 21

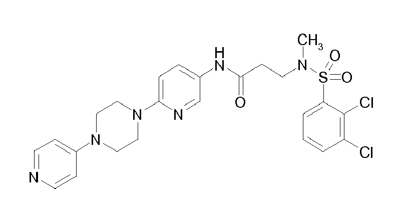

21a) 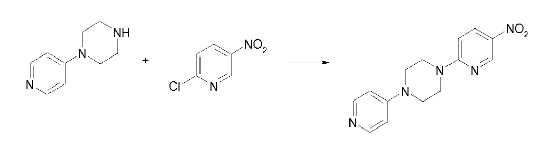 Eine Mischung aus 2.06 g (12.62 mMol) 1-Pyridin-4-yl-piperazin (Girindus), 2.00 g (12.62 mMol) 2-Chlor-5-nitropyridin (Fluka) und 50 ml Dichlormethan wird 15 min bei Raumtemperatur gerührt und anschließend mit 6.31 ml (12.62 mMol) 2 M Natronlauge versetzt. Die Reaktionsmischung wird 20 Stunden bei Raumtemperatur gerührt und danach mit 300 ml Dichlormethan und 100 ml 5%iger Natriumhydrogencarbonat-Lösung versetzt. Nach der Phasentrennung wird die organische Phase über Natriumsulfat getrocknet und im Vakuum bis zur Trockene eingeengt. Das Rohprodukt wird mit 100 ml Diethylether / Ethanol 2:1 verrührt, abfiltriert und getrocknet.
   C₁₄H₁₅N₅O₂ (285.30)
   [M+H]+ = 286
   DC: Kieselgel, Dichlormethan / Ethanol 9:1, Rf-Wert = 0.10
21b) 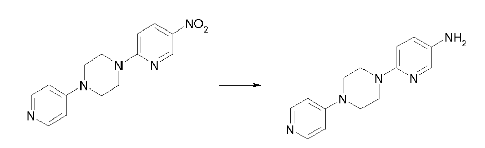 Eine Mischung aus 1.75 g (6.13 mMol) Produkt aus 21 a, 0.4 g Palladium auf Kohle (10%), 100 ml Dichlormethan und 50 ml Methanol wird fünf Stunden im Autoklaven bei Raumtemperatur hydriert. Anschließend wird der Katalysator abgesaugt und das Filtrat im Vakuum bis zur Trockene eingeengt. Der Rückstand wird mit 100 ml Diethylether / Ethanol 2:1 verrührt und abgesaugt. Das so erhaltene Rohprodukt wird durch Säulenchromatographie über Kieselgel (Elutionsmittel: Dichlormethan / Methanol / Ammoniak 95:5:0.5) gereinigt.
   C₁₄H₁₇N₅ (255.32)
   [M+H]+ = 256
   DC: Kieselgel, Dichlormethan / Methanol / Ammoniak 9:1:0.1, Rf-Wert = 0.37
21c) 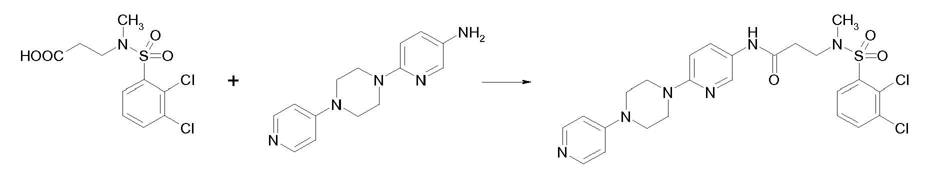 Beispiel 21 wird analog zu 1f aus 0.11 g (0.35 mMol) Produkt aus 1c, 0.089 g (0.35 mMol) Produkt aus 21b, 0.098 ml (0.70 mMol) Triethylamin und 0.13 g (0.42 mMol) TBTU in 15 ml THF hergestellt.
   C₂₄H₂₆Cl₂N₆O₃S (549.47)
   [M+H]+ = 549/551/553
   HPLC (Methode 4): Retentionszeit = 2.7 min

### Beispiel 22

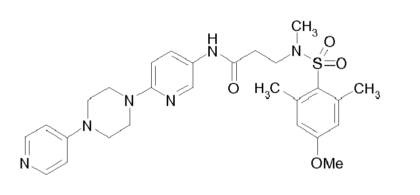

22a) 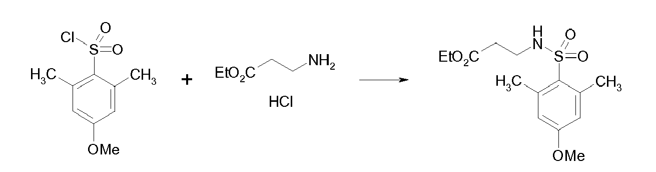 22a wird analog zu 3a aus 3.00 g (12.78 mMol) Produkt aus 13a, 2.16 g (14.06 mMol) β-Alaninethylester Hydrochlorid, 7.13 ml (51.13 mMol) Triethylamin in 70 ml Dichlormethan hergestellt.
   C₁₄H₂₁NO₅S (315.39)
   [M+H]+ = 316
   DC: Kieselgel, Petrolether / Ethylacetat 2:1, Rf-Wert = 0.23
22b) 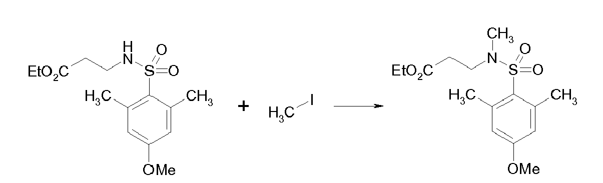 22b wird analog zu 3b aus 4.06 g (12.87 mMol) Produkt aus 22a, 2.40 ml (38.62 mMol) Methyliodid, 3.56 g (25.75 mMol) Kaliumcarbonat wasserfrei in 40 ml DMF hergestellt.
   C₁₅H₂₃NO₅S (329.41)
   [M+H]+ = 330
   DC: Kieselgel, Petrolether / Ethylacetat 2:1, Rf-Wert = 0.36
22c) 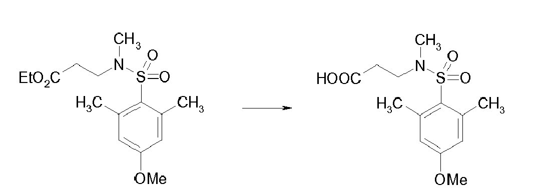 Die Säure wird analog zu 1c aus 3.83 g (11.63 mMol) Produkt aus 22b, 2.44 g (58.13 mMol) Lithiumhydroxid Monohydrat in 30 ml THF und 30 ml Wasser hergestellt.
   C₁₃H₁₉NO₅S (301.36)
   [M+H]+ = 302
   DC: Kieselgel, Petrolether / Ethylacetat 1:1, Rf-Wert = 0.12
22d) 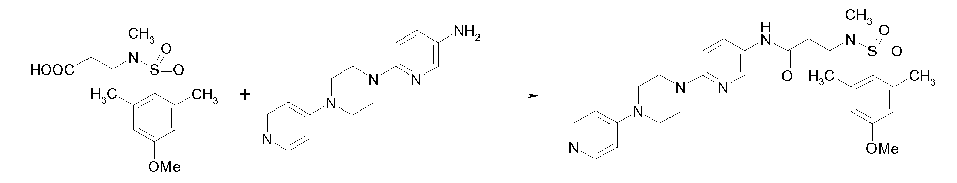 Beispiel 22 wird analog zu 1f aus 0.13 g (0.42 mMol) Produkt aus 22c, 0.089 g (0.35 mMol) Produkt aus 21 b, 0.098 ml (0.70 mMol) Triethylamin und 0.13 g (0.42 mMol) TBTU in 15 ml THF hergestellt.
   C₂₇H₃₄N₆O₄S (538.66)
   [M+H]+ = 539
   HPLC (Methode 4): Retentionszeit = 2.6 min

### Beispiel 23

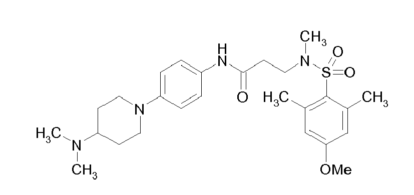

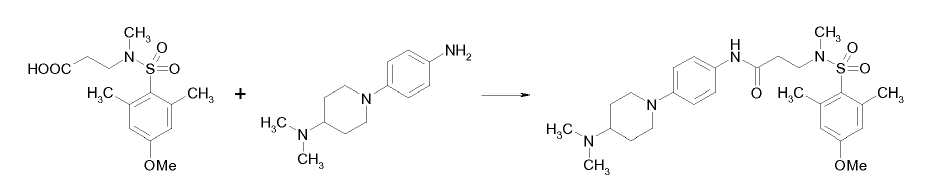

Beispiel 23 wird analog zu 1f aus 0.30 g (1.00 mMol) Produkt aus 22c, 0.22 g (1.00 mMol) Produkt aus 8b, 0.42 ml (2.99 mMol) Triethylamin und 0.32 g (1.00 mMol) TBTU in 15 ml

DMF hergestellt.
C₂₆H₃₈N₄O₄S (502.67)
[M+H]+ = 503
HPLC (Methode 1): Retentionszeit = 2.47 min

### Beispiel 24

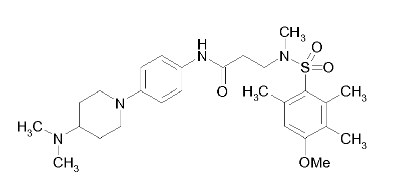

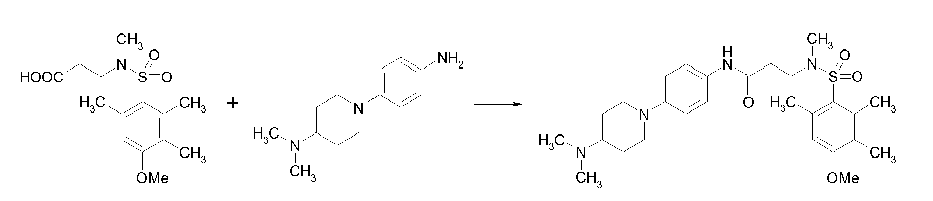

Beispiel 24 wird analog zu 1f aus 0.25 g (0.80 mMol) Produkt aus 3c, 0.18 g (0.80 mMol) Produkt aus 8b, 0.33 ml (2.39 mMol) Triethylamin und 0.26 g (0.80 mMol) TBTU in 10 ml

DMF hergestellt.
C₂₇H₄₀N₄O₄S (516.70)
[M+H]+ = 517
HPLC (Methode 1): Retentionszeit = 2.50 min

### Beispiel 25

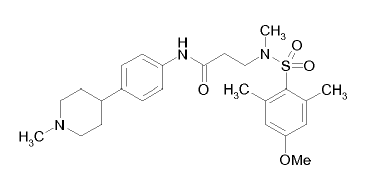

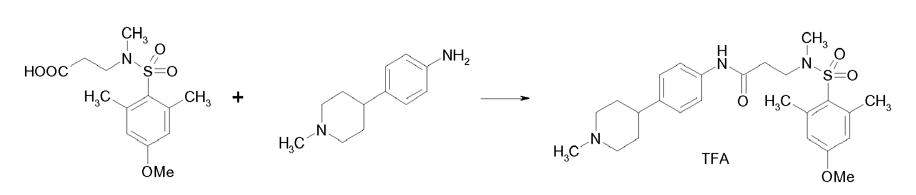

Beispiel 25 wird analog zu 1f aus 0.20 g (0.66 mMol) Produkt aus 22c, 0.14 g (0.73 mMol) 4-(1-Methylpiperidin-4-yl)-anilin (JW Pharmlab), 0.28 ml (1.99 mMol) Triethylamin und 0.21 g (0.66 mMol) TBTU in 50 ml THF hergestellt.
C₂₇H₄₀N₄O₄S x C₂HF₃O₂ (587.65)
[M+H]+ = 474
HPLC (Methode 2): Retentionszeit = 3.03 min

### Beispiel 26

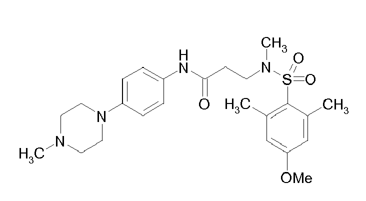

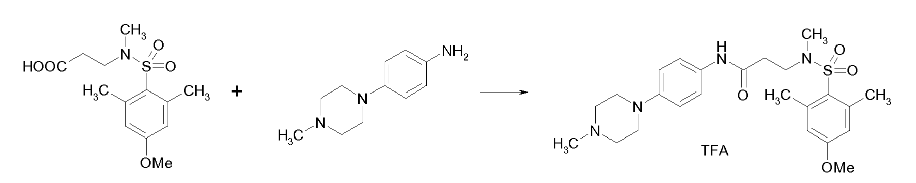

Beispiel 26 wird analog zu 1f aus 0.20 g (0.66 mMol) Produkt aus 22c, 0.14 g (0.73 mMol) 4-(4-Methylpiperazin-1-yl)-anilin (J. Med. Chem. SIR 48, 7, 2005, 2371-2387), 0.28 ml (1.99 mMol) Triethylamin und 0.21 g (0.66 mMol) TBTU in 5 ml THF hergestellt. C₂₄H₃₄N₄O₄S x C₂HF₃O₂ (588.65)
[M+H]+ = 475
HPLC (Methode 5): Retentionszeit = 1.50 min

### Beispiel 27

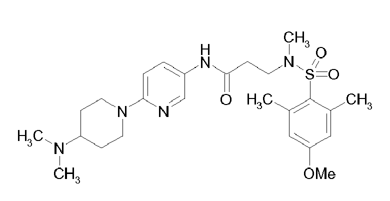

27a) 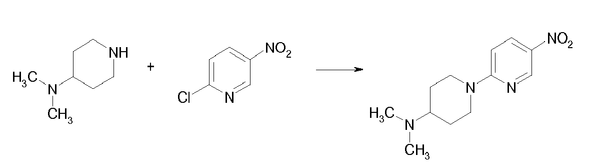 Eine Mischung aus 1.00 g (6.31 mMol) 2-Chlor-5-nitropyridin (Fluka), 1.32 ml (9.46 mMol) Triethylamin und 2 ml Methanol wird vorgelegt und langsam mit 1.13 g (8.83 mMol) 4-Dimethylamino-piperidin (Alfa Aesar) versetzt. Die Reaktionslösung wird anschließend mit halbgesättigter Natriumchlorid gequencht, der entstandene Niederschlag wird abgesaugt und getrocknet.
   C₁₂H₁₈N₄O₂ (250.30)
27b) 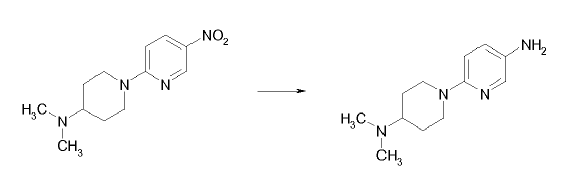 27b wird analog zu 8b aus 1.50 g (5.99 mMol) Produkt aus 27a, 0.20 g Palladium auf Kohle (10%) und 15 ml Methanol hergestellt.
   C₁₂H₂₀N₄ (220.31)
27c) 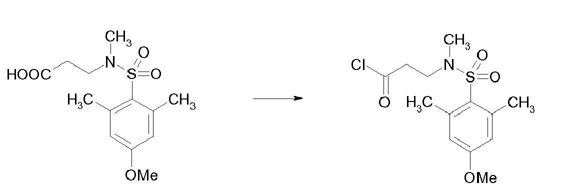 Eine Mischung aus 0.11 g (0.35 mMol) Produkt aus 22c und 2.0 ml Thionylchlorid wird zwei Stunden bei Raumtemperatur gerührt. Die Reaktionsmischung wird danach im Vakuum bis zur Trockene eingeengt.
   C₁₃H₁₈ClNO₄S (319.81)
27d) 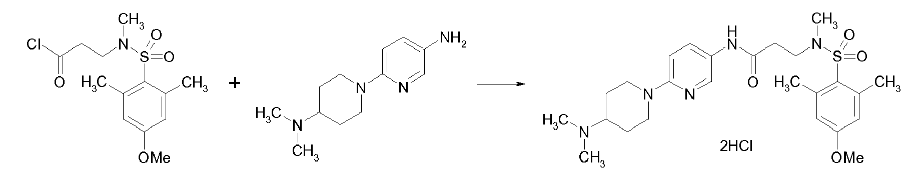 Eine Mischung aus 0.11 g (0.34 mMol) Produkt aus 27c, 0.091 g (0.41 mMol) Produkt aus 27b, 0.18 ml (1.03 mMol) DIPEA und 45 ml THF wird zwei Stunden bei Raumtemperatur gerührt. Die Reaktionsmischung wird anschließend im Vakuum bis zur Trockene eingeengt. Das Rohprodukt wird durch präparative HPLC gereinigt. Danach wird mit Hilfe von 4 M HCl in Dioxan das Hydrochlorid hergestellt.
   C₂₅H₃₇N₅O₄S x 2HCl (576.58)
   [M+H]+ = 504
   HPLC (Methode 2): Retentionszeit = 2.73 min

### Beispiel 28

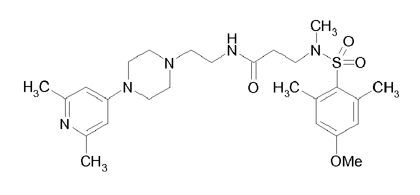

28a) 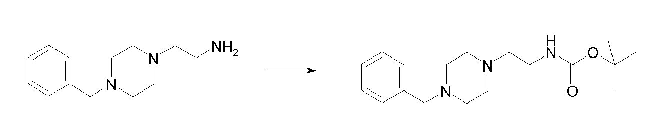 Eine Mischung aus 1.50 g (6.84 mMol) 1-(2-Aminoethyl)-4-benzylpiperazin (Maybridge), 1.64 g (7.52 mMol) Boc-Anhydrid und 30 ml Dichlormethan wird eine Stunde bei Raumtemperatur gerührt. Danach wird die Reaktionslösung mit 100 ml Dichlormethan verdünnt und mit 1 M Natronlauge und Wasser gewaschen. Die organische Phase wird über Natriumsulfat getrocknet und im Vakuum bis zur Trockene eingeengt.
   C₁₈H₂₉N₃O₂ (319.44)
   HPLC (Methode 4): Retentionszeit = 2.6 min
28b) 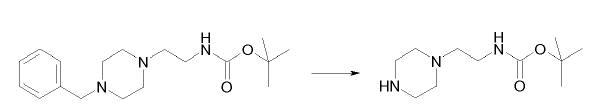 Eine Mischung aus 2.10 g (6.57 mMol) Produkt aus 28a, 0.25 g Palladium auf Kohle (10%) und 30 ml Methanol wird 15 Stunden bei Raumtemperatur im Autoklaven hydriert. Anschließend wird der Katalysator abgesaugt und das Filtrat im Vakuum bis zur Trockene eingeengt.
   C₁₁H₂₃N₃O₄ (229.32)
   [M+H]+ = 230
   DC: Kieselgel, Dichlormethan / Ethanol 9:1, Rf-Wert = 0.08
28c) 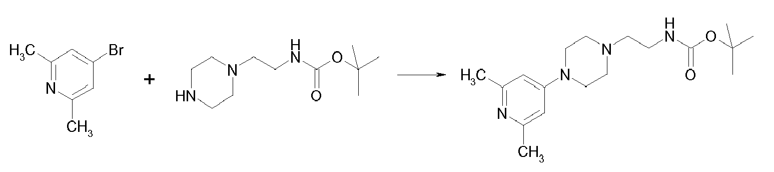 Eine Mischung aus 1.18 g (6.32 mMol) 4-Brom-2,6-dimethylpyridin (Acta Chem. Scand. Ser. B 42, 1988, 373-377), 1.45 g (6.32 mMol) Produkt aus 28b und 2.2 ml DIPEA wird 50 min auf 130°C in der Mikrowelle erhitzt. Man versetzt die Reaktionsmischung mit Ethylacetat und halbgesättigter Kaliumcarbonat-Lösung und trennt danach die Phasen. Die organische Phase wird über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 95:5:0.5) gereinigt.
   C₁₈H₃₀N₄O₂S (334.46)
   [M+H]+ = 335
   DC: Kieselgel, Dichlormethan / Methanol / Ammoniak 9:1:0.1, Rf-Wert = 0.37
28d) 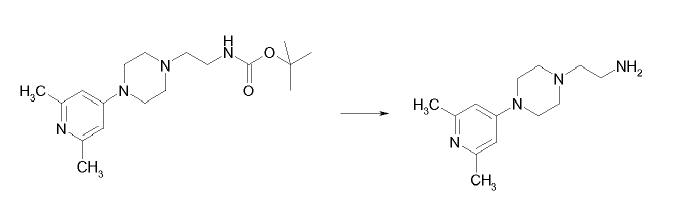 Eine Mischung aus 1.61 g (4.81 mMol) Produkt aus 28c, 3.70 ml TFA und 30 ml Dichlormethan wird sechs Stunden bei Raumtemperatur gerührt. Die Reaktionsmischung wird anschließend mit Dichlormethan verdünnt und mit 5%iger Natriumhydrogencarbonat-Lösung gewaschen. Die wässrige Phase wird noch zweimal mit Ethylacetat extrahiert. Die vereinigten organischen Phasen werden über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 90:10:1) gereinigt.
   C₁₃H₂₂N₄ (234.34)
   [M+H]+ = 235
   DC: Kieselgel, Dichlormethan / Methanol / Ammoniak 9:1:0.1, Rf-Wert = 0.10
28e) 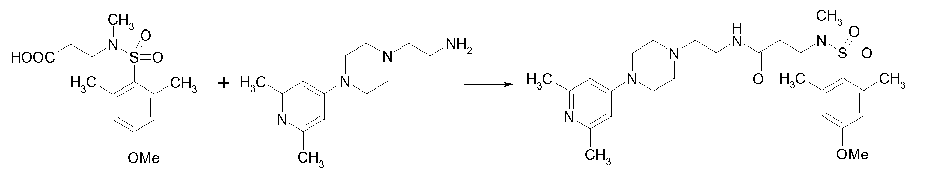 Beispiel 28 wird analog zu 1f aus 0.11 g (0.35 mMol) Produkt aus 22c, 0.082 g (0.35 mMol) Produkt aus 28d, 0.098 ml (0.70 mMol) Triethylamin und 0.13 g (0.42 mMol) TBTU in 7 ml THF und 1 ml DMF hergestellt.
   C₂₆H₃₉N₅O₄S (517.69)
   [M+H]+ = 518
   HPLC (Methode 4): Retentionszeit = 2.4 min

### Beispiel 29

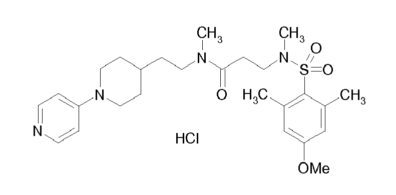

29a) 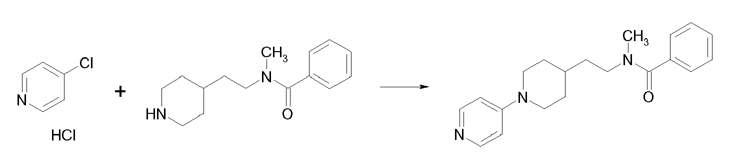 29a wird analog zu 28c aus 0.12 g (0.80 mMol) 4-Chlorpyridin Hydrochlorid (Aldrich), 0.20 g (0.80 mMol) N-Methyl-N-(2-piperidin-4-yl-ethyl)-benzamid (J. Med. Chem. 33, 1990, 1880-1887), 0.23 ml (1.68 mMol) Triethylamin in 5 ml Ethanol hergestellt.
   C₂₀H₂₅N₃O (323.43)
   [M+H]+ = 324
29b) 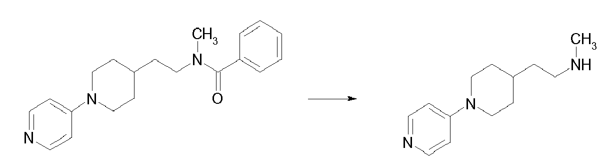 Eine Mischung aus 0.52 g (1.61 mMol) Produkt aus 29a, 10 ml 2 M Kalilauge und 10 ml Ethanol wird 30 Stunden unter Rückfluss erhitzt. Die Reaktionsmischung wird im Vakuum bis auf die Hälfte eingeengt und danach mit Dichlormethan extrahiert. Die organischen Extrakte werden über Natriumsulfat getrocknet und im Vakuum bis zur Trockene eingeengt.
   C₁₃H₂₁N₃ (219.33)
   [M+H]+ = 220
   DC: Kieselgel, Dichlormethan / Methanol / Ammoniak 8:2:0.01, Rf-Wert = 0.46
29c) 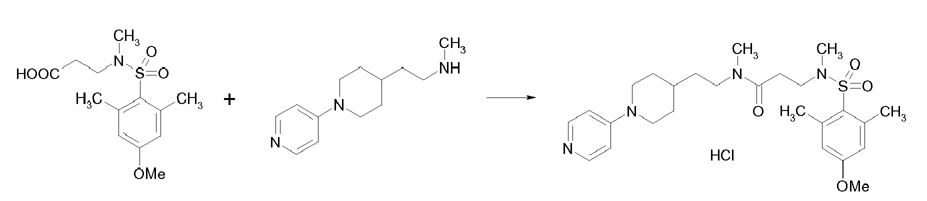 Beispiel 29 wird analog zu 1f aus 0.14 g (0.46 mMol) Produkt aus 22c, 0.10 g (0.46 mMol) Produkt aus 29b, 0.15 ml (1.09 mMol) Triethylamin und 0.16 g (0.50 mMol) TBTU in 30 ml THF und 5 ml DMF hergestellt.
   C₂₆H₃₈N₄O₄S x HCl (539.13)
   [M+H]+ = 503
   DC: Kieselgel, Dichlormethan / Methanol / Ammoniak 8:2:0.01, Rf-Wert = 0.41

### Beispiel 30

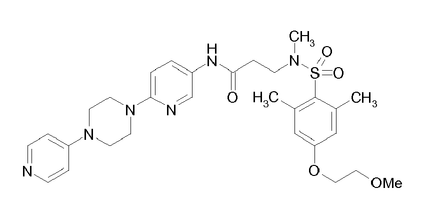

30a) 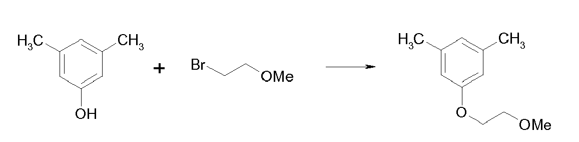 Eine Mischung aus 3.25 g (26.60 mMol) 3,5-Dimethylphenol (Aldrich), 3.20 g (28.52 mMol) Kalium-*tert*-butylat und 40 ml DMSO wird eine Stunde bei Raumtemperatur gerührt. Danach tropft man 3.80 g (27.34 mMol) Bromethylmethylether (Aldrich) hinzu und rührt weitere zwei Stunden bei Raumtemperatur. Die Reaktionsmischung wird auf Wasser gegossen und mit Diethylether extrahiert. Die organischen Extrakte werden mit Wasser und gesättigter Natriumchlorid-Lösung gewaschen, über Natriumsulfat getrocknet und im Vakuum bis zur Trockene eingeengt.
   C₁₁H₁₆O₂ (180.24)
   [M+H]+ = 181
   DC: Kieselgel, Petrolether/Ethylacetat 9:1, Rf-Wert = 0.31
30b) 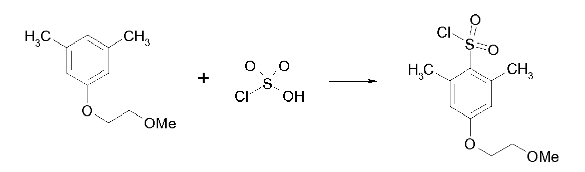 30b wird analog zu 13a aus 4.30 g (23.86 mMol) Produkt aus 30a und 5.60 g (48.06 mMol) Chlorsulfonsäure in 100 ml Dichlormethan hergestellt.
   C₁₁H₁₅ClO₄S (278.75)
   DC: Kieselgel, Petrolether/Ethylacetat 9:1, Rf-Wert = 0.06
30c) 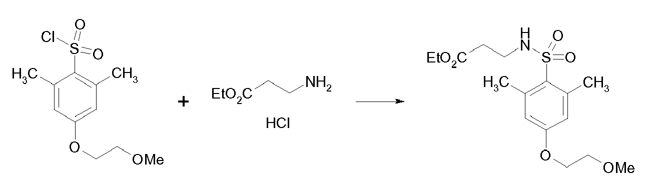 30a wird analog zu 3a aus 1.70 g (6.10 mMol) Produkt aus 30b, 1.20 g (7.81 mMol) β-Alaninethylester Hydrochlorid, 2.60 ml (18.65 mMol) Triethylamin in 30 ml Dichlormethan hergestellt.
   C₁₆H₂₅NO₆S (359.44)
   [M+H]+ = 360
   DC: Kieselgel, Dichlormethan / Methanol 19:1, Rf-Wert = 0.51
30d) 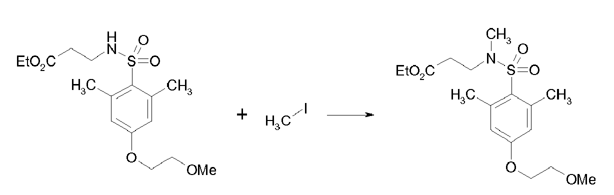 30d wird analog zu 3b aus 1.90 g (5.29 mMol) Produkt aus 30c, 1.10 g (7.75 mMol) Methyliodid, 1.50 g (10.85 mMol) Kaliumcarbonat wasserfrei in 30 ml DMF hergestellt.
   C₁₇H₂₇NO₆S (373.47)
   [M+H]+ = 374
30e) 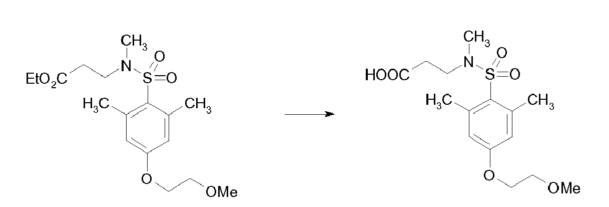 Die Säure wird analog zu 1 c aus 1.70 g (4.55 mMol) Produkt aus 30d, 0.80 g (20.00 mMol) Natriumhydroxid in 30 ml Ethanol und 10 ml Wasser hergestellt.
   C₁₅H₂₃NO₆S (345.41)
   [M+H]+ = 346
30f) 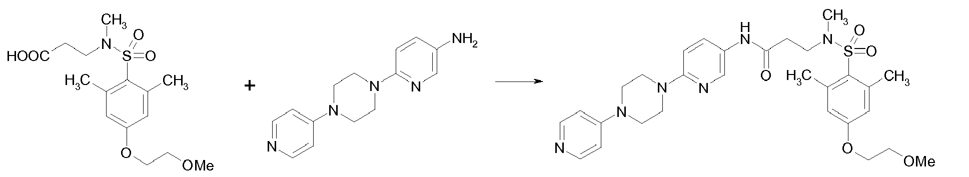 Beispiel 30 wird analog zu 1f aus 0.14 g (0.39 mMol) Produkt aus 30e, 0.10 g (0.39 mMol) Produkt aus 21 b, 0.10 ml (0.99 mMol) Triethylamin und 0.14 g (0.44 mMol) TBTU in 30 ml THF und 5 ml DMF hergestellt.
   C₂₉H₃₈N₆O₅S (582.72)
   [M+H]+ = 583
   DC: Kieselgel, Dichlormethan / Methanol / Ammoniak 8:2:0.01, Rf-Wert = 0.44

### Beispiel 31

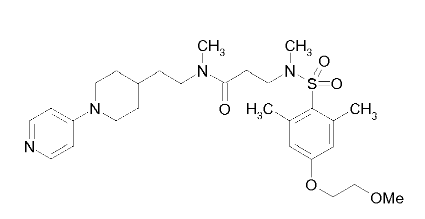

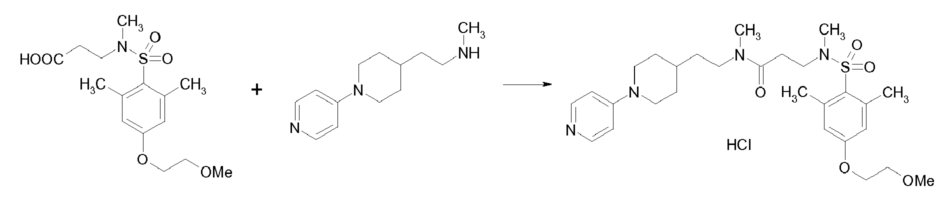

Beispiel 31 wird analog zu 1f aus 0.16 g (0.46 mMol) Produkt aus 30e, 0.10 g (0.46 mMol) Produkt aus 29b, 0.11 ml (1.09 mMol) Triethylamin und 0.16 g (0.50 mMol) TBTU in 30 ml THF und 5 ml DMF hergestellt.
C₂₈H₄₂N₄O₅S x HCl (583.18)
[M+H]+ = 547
DC: Kieselgel, Dichlormethan / Methanol / Ammoniak 8:2:0.01, Rf-Wert = 0.52

### Beispiel 32

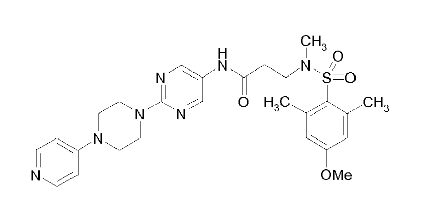

32a) 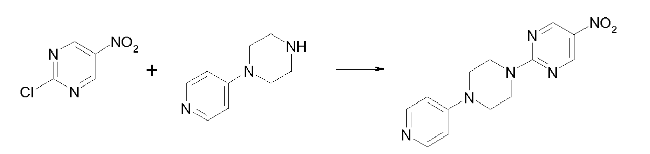 Eine Mischung aus 3.00 g (18.81 mMol) 2-Chlor-5-nitropyrimidin (Apin), 3.07 g (18.81 mMol) 1-(4-Pyridyl)-piperazin (Girindus), 9.40 ml (18.81 mMol) 2 M Natronlauge in 80 ml Dichlormethan wird 2.5 Stunden bei Raumtemperatur gerührt. Danach wird die Reaktionsmischung mit 100 ml Dichlormethan verdünnt und mit 5%iger Natriumhydrogencarbonat-Lösung gewaschen. Die organische Phase wird über Natriumsulfat getrocknet und im Vakuum bis zur Trockene eingeengt. Das Rohprodukt wird mit einer Mischung aus 50 ml Wasser und 30 ml Ethylacetat verrieben, abfiltriert und getrocknet.
   C₁₃H₁₄N₆O₂ (286.29)
   [M+H]+ = 287
   HPLC (Methode 4): Retentionszeit = 2.6 min
32b) 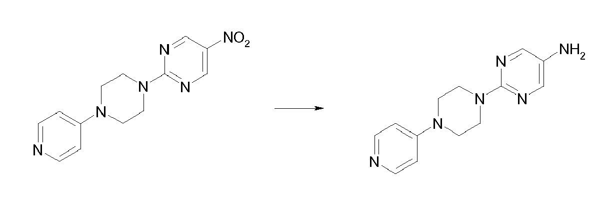 32b wird analog zu 21 b aus 1.93 g (6.74 mMol) Produkt aus 32a und 0.3 g Palladium auf Kohle (10 %) in 60 ml Dichlormethan und 30 ml Methanol hergestellt.
   C₁₃H₁₆N₆ (256.31)
   [M+H]+ = 257
   DC: Kieselgel, Dichlormethan / Methanol 8:2, Rf-Wert = 0.11
32c) 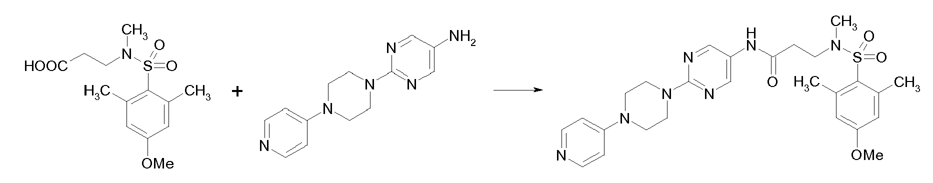 Beispiel 32 wird analog zu 1f aus 0.11 g (0.35 mMol) Produkt aus 22c, 0.090 g (0.35 mMol) Produkt aus 32b, 0.098 ml (0.70 mMol) Triethylamin und 0.13 g (0.42 mMol) TBTU in 7 ml THF und 1 ml DMF hergestellt.
   C₂₆H₃₃N₇O₄S (539.65)
   [M+H]+ = 540
   HPLC (Methode 4): Retentionszeit = 2.9 min

### Beispiel 33

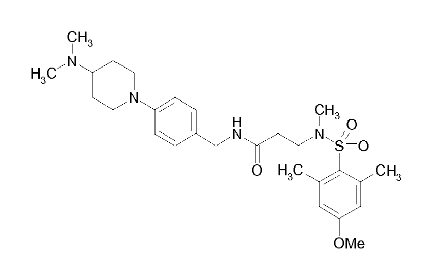

33a) 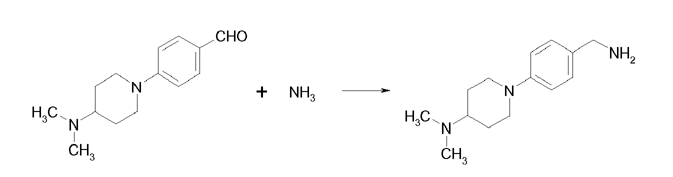 Eine Mischung aus 0.68 g (2.91 mMol) 4-(4-Dimethylamino-piperidin-1-yl)-benzaldehyd (Tetrahedron 57, 2001, 4781-4785), 15 ml 2 M Ammoniak in Ethanol und 0.10 g Raney-Nickel wird bei Raumtemperatur im Autoklaven hydriert. Danach wird der Katalysator abfiltriert und das Filtrat im Vakuum bis zur Trockene eingeengt.
   C₁₄H₂₃N₃ (233.35)
33b) 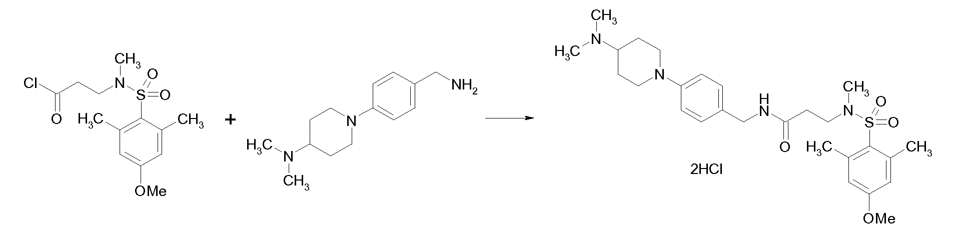 Beispiel 33 wird analog zu 27d aus 0.27 g (0.84 mMol) Produkt aus 27c, 0.63 g (2.68 mMol) Produkt aus 33a, 0.22 ml (1.26 mMol) DIPEA in 3 ml Dichlormethan hergestellt. C₂₇H₄₀N₄O₄S x 2HCl (589.62)
   [M+H]+ = 517
   HPLC (Methode 5): Retentionszeit = 1.46 min

### Beispiel 34

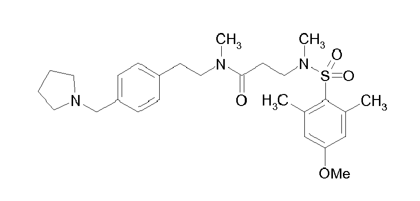

34a) 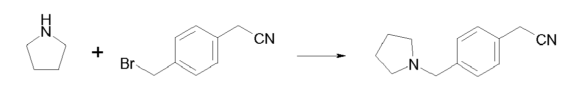 Eine Mischung aus 4.97 ml (59.50 mMol) Pyrrolidin und 100 ml Dichlormethan wird unter Eisbadkühlung langsam mit 5.00 g (23.80 mMol) (4-Brommethyl-phenyl)-acetonitril (Tetrahedron 47, 1991, 3969-3980) versetzt. Danach wird die Reaktionsmischung auf Raumtemperatur erwärmt, mit Wasser gewaschen, über Magnesiumsulfat getrocknet und im Vakuum bis zur Trockene eingeengt.
   C₁₃H₁₆N₂ (200.28)
   [M+H]+ = 201
   DC: Kieselgel, Dichlormethan / Methanol / Ammoniak 9:1:0.1, Rf-Wert = 0.58
34b) 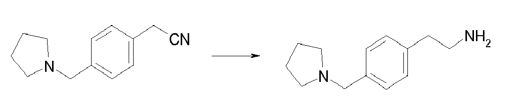 Eine Mischung aus 4.70 g (23.47 mMol) Produkt aus 34a, 0.5 g Raney-Nickel und 50 ml methanolische Ammoniak-Lösung wird im Autoklaven bei 50°C hydriert. Anschließend wird der Katalysator abfiltriert und das Filtrat im Vakuum bis zur Trockene eingeengt.
   C₁₃H₂₀N₂ (204.31)
   [M+H]+ = 205
34c) 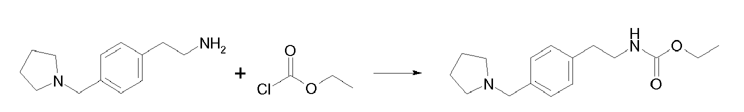 Eine Mischung aus 4.09 g (20.00 mMol) Produkt aus 34b, 5.62 ml (40.00 mMol) Triethylamin und 100 ml Dichlormethan wird unter Eisbadkühlung langsam mit 2.17 ml (22.00 mMol) Chlorameisensäureethylester (Aldrich) versetzt. Danach wird fünf Stunden bei Raumtemperatur gerührt. Die Reaktionsmischung wird anschließend mit Wasser gequencht und mit MTB-Ether extrahiert. Die organischen Extrakte werden zweimal mit Wasser gewaschen, über Magnesiumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
   C₁₆H₂₄N₂O₂ (276.37)
   [M+H]+ = 277
34d) 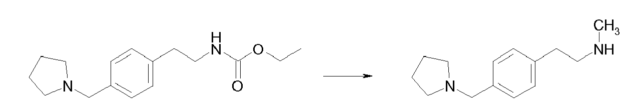 Eine Mischung aus 3.60 g (13.03 mMol) Produkt aus 34c und 25 ml THF wird langsam mit 51.05 ml (51.05 mMol) 1 M Lithiumaluminiumhydrid in THF (Aldrich) versetzt. Danach wird zwei Stunden bei Raumtemperatur und zwei Stunden bei 70°C gerührt. Die Reaktionsmischung wird anschließend mit Wasser und 15%iger Natronlauge gequencht und noch eine Stunde bei Raumtemperatur gerührt. Der entstandene Niederschlag wird abfiltriert und das Filtrat im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
   C₁₄H₂₂N₂ (218.34)
   [M+H]+ = 219
34e) 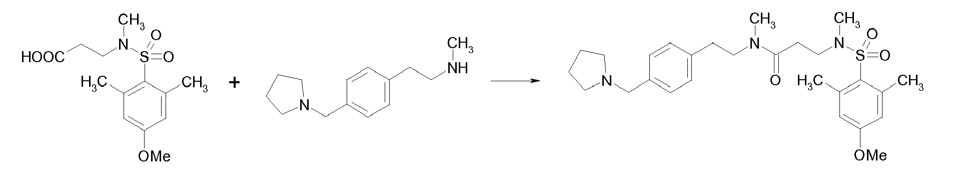 Beispiel 34 wird analog zu 1f aus 0.11 g (0.35 mMol) Produkt aus 22c, 0.076 g (0.35 mMol) Produkt aus 34d, 0.098 ml (0.70 mMol) Triethylamin und 0.13 g (0.42 mMol) TBTU in 7 ml THF und 1 ml DMF hergestellt.
   C₂₇H₃₉N₃O₄S (501.68)
   [M+H]+ = 502
   HPLC (Methode 4): Retentionszeit = 3.1 min

### Beispiel 35

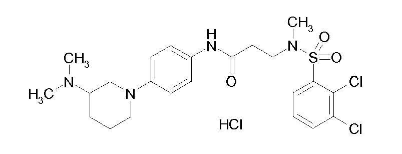

35a) 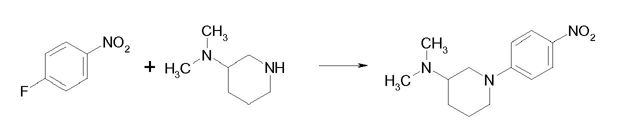 Eine Mischung aus 0.33 g (2.34 mMol) 1-Fluor-4-nitrobenzol (Aldrich), 0.30 g (2.34 mMol) 3-Dimethylamino-piperidin (Chess), 0.46 ml (3.27 mMol) Triethylamin und 4 ml DMF wird sechs Stunden bei Raumtemperatur gerührt. Die Reaktionsmischung wird danach mit Wasser gequencht und mit Dichlormethan extrahiert. Die organischen Extrakte werden mit gesättigter Natriumchlorid-Lösung gewaschen, über Natriumsulfat getrocknet und im Vakuum bis zur Trockene eingeengt.
   C₁₃H₁₉N₃O₂ (249.31)
   [M+H]+ = 250
   DC: Kieselgel, Dichlormethan / Methanol / Ammoniak 9:1:0.1, Rf-Wert = 0.56
35b) 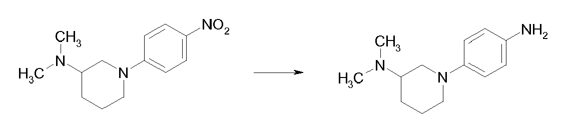 Eine Mischung aus 0.25 g (1.00 mMol) Produkt aus 35a, 30 mg Raney-Nickel und 10 ml Ethylacetat wird im Autoklaven bei Raumtemperatur hydriert. Der Katalysator wird abfiltriert und das Filtrat im Vakuum bis zur Trockene eingeengt.
   C₁₃H₂₁N₃ (219.33)
   DC: Kieselgel, Dichlormethan / Methanol / Ammoniak 9:1:0.1, Rf-Wert = 0.25
35c) 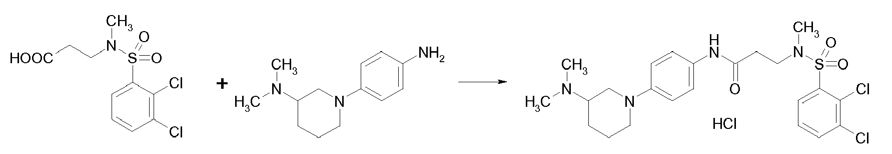 Beispiel 35 wird analog zu 1f aus 0.16 g (0.50 mMol) Produkt aus 1c, 0.11 g (0.50 mMol) Produkt aus 35b, 0.14 ml (1.00 mMol) Triethylamin und 0.16 g (0.50 mMol) TBTU in 3 ml DMF hergestellt.
   C₂₃H₃₀Cl₂N₄O₃S x HCl (539.14)
   [M+H]+ = 513/515/517
   HPLC (Methode 1): Retentionszeit = 2.43 min

### Beispiel 36

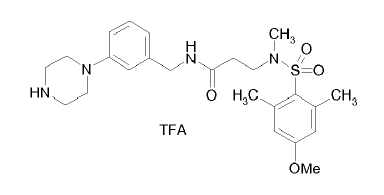

36a) 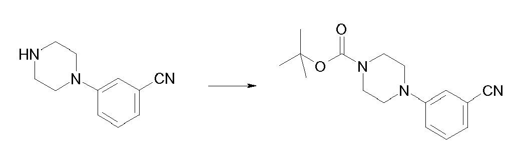 Eine Mischung aus 2.50 g (13.35 mMol) 3-Piperazin-1-yl-benzonitril (Tetrahedron 55, 1999, 13285-13300), 3.00 g (13.75 mMol) Boc-Anhydrid, 2.40 ml (13.78 mMol) DIPEA und 50 ml THF wird vier Stunden bei Raumtemperatur gerührt und danach im Vakuum bis zur Trockene eingeengt. Der Rückstand wird in Wasser aufgenommen und mit Diethylether extrahiert. Die organischen Extrakte werden über Natriumsulfat getrocknet und im Vakuum bis zur Trockene eingeengt.
   C₁₆H₂₁N₃O₂ (287.36)
   [M+Na]+ = 310
36b) 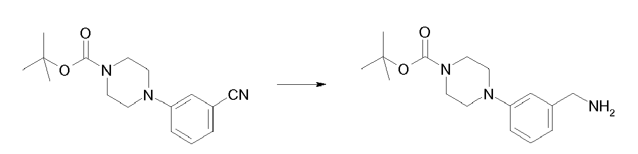 36b wird analog zu 34b aus 4.40 g (15.31 mMol) Produkt aus 36a, 0.7 g Raney-Nickel und 45 ml methanolischer Ammoniak-Lösung hergestellt.
   C₁₆H₂₅N₃O₂ (291.39)
   [M+H]+ = 292
36c) 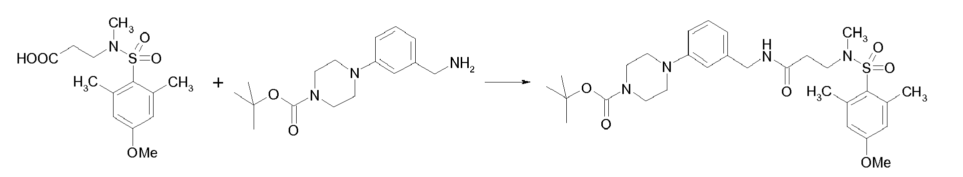 36c wird analog zu 1f aus 0.40 g (1.33 mMol) Produkt aus 22c, 0.43 g (1.46 mMol) Produkt aus 36b, 0.56 ml (3.98 mMol) Triethylamin und 0.43 g (1.33 mMol) TBTU in 10 ml THF hergestellt.
   C₂₉H₄₂N₄O₆S (574.73)
   M+H]+ = 575
   DC: Kieselgel, Dichlormethan / Methanol 9:1, Rf-Wert = 0.53
36d) 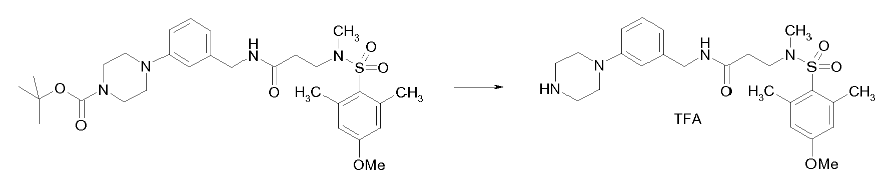 Beispiel 36 wird analog zu 18b aus 0.50 g (0.57 mMol) Produkt aus 36c, 0.44 ml TFA in 5 ml Dichlormethan hergestellt.
   C₁₈H₂₈Cl₂N₄O₃S x C₂HF₃O₂ (588.64)
   [M+H]+ = 475
   HPLC (Methode 2): Retentionszeit = 2.95 min

### Beispiel 37

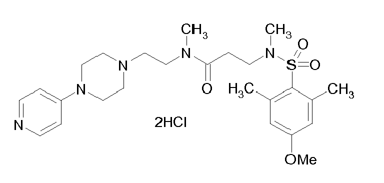

37a) 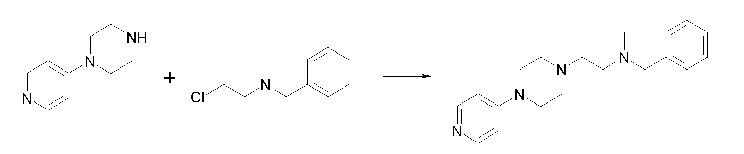 Eine Mischung aus 2.00 g (12.25 mMol) 1-(4-Pyridyl)-piperazin (Girindus), 1.65 g (14.70 mMol) Kalium-tert-butylat und 50 ml DMSO wird 30 min bei Raumtemperatur gerührt und danach mit 2.25 g (12.25 mMol) 1-Benzylmethylamino-2-chlorethan (Chem. Pharm. Bull. 45, 1997, 996-1007) versetzt. Die Reaktionsmischung wird über Nacht bei Raumtemperatur gerührt und anschließend auf Eiswasser gegossen. Man extrahiert viermal mit Dichlormethan. Die organischen Extrakte werden über Natriumsulfat getrocknet und im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
   C₁₉H₂₆N₄ (310.44)
   [M+H]+ = 311
   DC: Kieselgel, Dichlormethan / Methanol / Ammoniak 9:1:0.1, Rf-Wert = 0.22
37b) 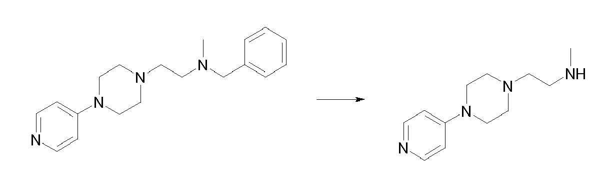 Eine Mischung aus 1.78 g (5.73 mMol) Produkt aus 37a, 0.40 g Palladiumhydroxid und 50 ml Methanol wird bei 40°C im Autoklaven hydriert. Der Katalysator wird abfiltriert und das Filtrat im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
   C₁₂H₂₀N₄ (220.31)
   [M+H]+ = 221
   DC: Kieselgel, Dichlormethan / Methanol / Ammoniak 9:1:0.1, Rf-Wert = 0.13
37c) 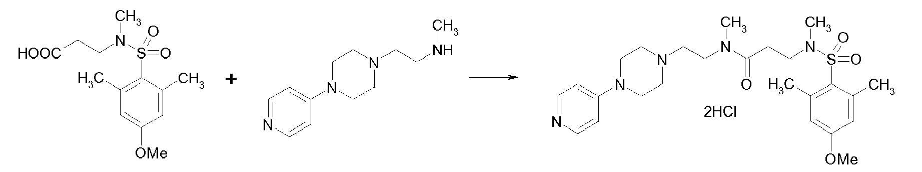 Beispiel 37 wird analog zu 1f aus 0.11 g (0.35 mMol) Produkt aus 22c, 0.072 g (0.35 mMol) Produkt aus 37b, 0.098 ml (0.70 mMol) Triethylamin und 0.13 g (0.42 mMol) TBTU in 7 ml THF hergestellt.
   C₂₅H₃₇N₅O₄S x 2HCl (576.58)
   [M+H]+ = 504
   HPLC (Methode 4): Retentionszeit = 2.5 min

### Beispiel 38

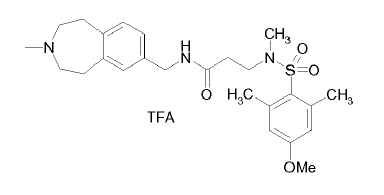

38a) 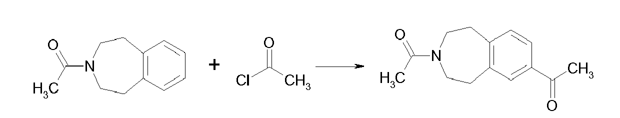 Eine Mischung aus 4.44 g (33.29 mMol) Aluminiumchlorid (Merck) und 16 ml Dichlorethan wird vorgelegt und unter Eisbadkühlung langsam mit 1.24 ml (17.44 mMol) Acetylchlorid (Aldrich) versetzt. Man lässt 30 min bei Raumtemperatur rühren. Anschließend werden 3.00 g (15.85 mMol) 1-(1,2,4,5-Tetrahydrobenzo[d]azepin-3-yl)-ethanon (J. Med.Chem. 46, 2003, 4952-4964) in 7 ml Dichlorethan langsam zur Reaktionsmischung gegeben. Nach zweistündigem Rühren bei Raumtemperatur wird die Reaktionsmischung auf eine Mischung aus 6 M HCl und Eis gegossen. Nach der Phasentrennung wird die wässrige Phase noch dreimal mit Dichlormethan extrahiert. Die vereinigten organischen Phasen werden mit Wasser gewaschen, über Natriumsulfat getrocknet und im Vakuum bis zur Trockene eingeengt. Das so erhaltene Rohprodukt wird mit Diethylether verrieben, abfiltriert und getrocknet.
   C₁₄H₁₇NO₂ (231.29)
   [M+H]+ = 232
38b) 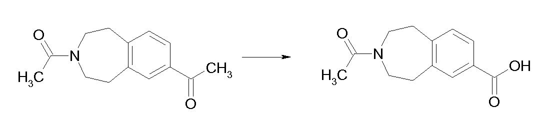 Eine Mischung aus 2.86 g (12.37 mMol) Produkt aus 38a und 79 ml 2.5 M Natronlauge wird bei Raumtemperatur langsam mit 2.48 ml (48.23 mMol) Brom versetzt. Die Reaktionsmischung wird danach eine Stunde bei Raumtemperatur gerührt. Der entstandene Niederschlag wird abfiltriert und das Filtrat mit MTB-Ether extrahiert. Die wässrige Phase wird anschließend unter Eisbadkühlung mit konzentrierter HCl und wenig Natriumdisulfit-Lösung versetzt. Der entstandene Niederschlag wird abfiltriert und im Umlufttrockenschrank bei 30°C getrocknet.
   C₁₃H₁₅NO₃ (233.26)
   [M+H]+ = 234
38c) 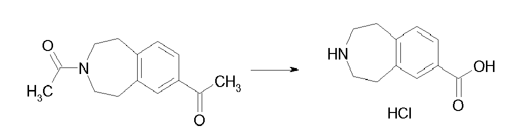 Eine Mischung aus 2.12 g (9.09 mMol) Produkt aus 38b und 20 ml 6 M HCl wird 3.5 Tage auf 100°C erhitzt. Die Reaktionsmischung wird danach mit einer Mischung aus Eis und
   Ethanol gekühlt. Der entstandene Niederschlag wird abfiltriert, mit wenig gekühltem Aceton und Diethylether gewaschen und im Exsikkator getrocknet.
   C₁₁H₁₃NO₂ x HCl (227.69)
   [M+H]+ = 192
38d) 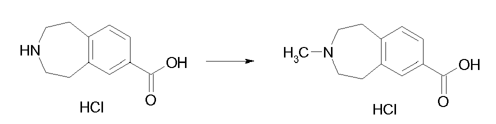 Eine Mischung aus 2.03 g (8.92 mMol) Produkt aus 38c und 3.36 ml (89.16 mMol) Ameisensäure wird erst langsam mit 0.94 ml (17.83 mMol) 50%iger Natronlauge, dann mit 2.66 ml (35.66 mMol) 37%iger Formalin-Lösung versetzt. Die Reaktionsmischung wird zwei Stunden auf 70°C erhitzt und danach im Vakuum bis zur Trockene eingeengt. Der Rückstand wird mit Wasser und konzentrierter HCl versetzt und nochmals bis zur Trockene eingeengt. Das Rohprodukt wird mit wenig eiskaltem Wasser verrieben, abfiltriert und im Umlufttrockenschrank bei 60°C getrocknet.
   C₁₂H₁₅NO₂ x HCl (241.71)
   [M+H]+ = 206
38e) 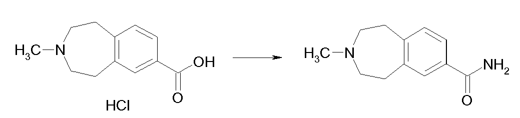 38e wird analog zu 1f aus 2.00 g (8.27 mMol) Produkt aus 38d, 18.20 ml (9.10 mMol) Ammoniak 0.5 M in Dioxan (Aldrich), 3.46 ml (24.82 mMol) Triethylamin und 3.19 g (9.93 mMol) TBTU in 30 ml THF hergestellt.
   C₁₂H₁₆N₂O (204.27)
   [M+H]+ = 205
   HPLC (Methode 2): Retentionszeit = 1.54 min
38f) 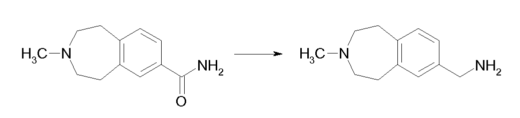 Eine Mischung aus 5.20 ml (5.20 mMol) Lithiumaluminiumhydrid 1 M in THF (Aldrich) und 18 ml THF wird auf 50°C erhitzt und langsam mit 0.84 g (2.64 mMol) Produkt aus 38e versetzt. Die Reaktionsmischung wird danach 30 min bei 50°C gerührt. Man kühlt anschließend auf -20°C ab und quencht die Reaktionsmischung erst mit einer Mischung aus Wasser und THF, dann mit 2 M Natronlauge. Es wird eine Stunde bei Raumtemperatur gerührt. Der entstandene Niederschlag wird abfiltriert, das Filtrat im Vakuum bis zur Trockene eingeengt. Der Rückstand wird in Dichlormethan aufgenommen und mit gesättigter Natriumhydrogensulfat-Lösung gewaschen. Die organische Phase wird über Natriumsulfat getrocknet und im Vakuum bis zur Trockene eingeengt.
   C₁₂H₁₈N₂ (190.28)
   [M+H]+ = 191
   HPLC (Methode 2): Retentionszeit = 2.21 min
38g) 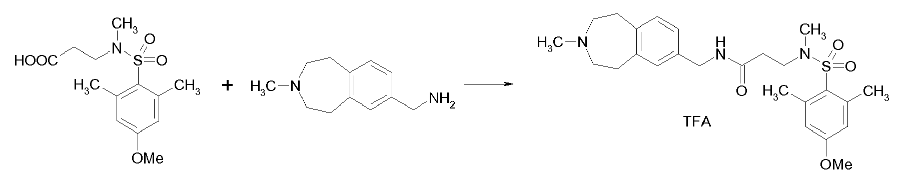 Beispiel 38 wird analog zu 1f aus 0.19 g (0.63 mMol) Produkt aus 22c, 0.12 g (0.63 mMol) Produkt aus 38f, 0.26 ml (1.89 mMol) Triethylamin und 0.20 g (0.63 mMol) TBTU in 5 ml
   THF hergestellt.
   C₂₅H₃₅N₃O₄S x C₂HF₃O₂ (587.65)
   [M+H]+ = 474
   HPLC (Methode 4): Retentionszeit = 2.98 min

### Beispiel 39

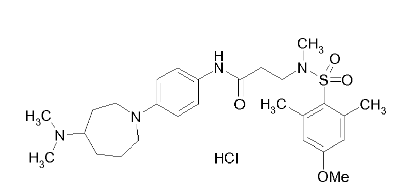

39a) 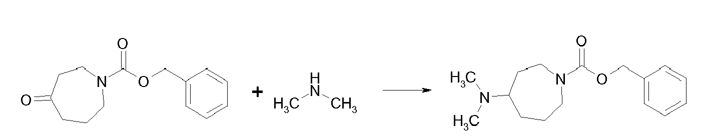 Eine Mischung aus 1.50 g (6.07 mMol) 4-Oxo-azepan-1-carbonsäurebenzylester (Tyger), 20 ml (40.00 mMol) Dimethylamin 2 M in THF (Aldrich) und 0.34 ml (6.07 mMol) Essigsäure wird 20 min bei Raumtemperatur gerührt und danach mit 3.82 g (18.00 mMol) Natriumtriacetoxyborhydrid (Aldrich) versetzt. Man lässt über Nacht bei Raumtemperatur rühren. Die Reaktionsmischung wird anschließend mit gesättigter Natriumhydrogencarbonat-Lösung versetzt und mit Ethylacetat extrahiert. Die organischen Extrakte werden mit gesättigter Natriumchlorid-Lösung gewaschen, über Natriumsulfat getrocknet und im Vakuum bis zur Trockene eingeengt. Das so erhaltene Rohprodukt wird durch Säulenchromatographie über Kieselgel (Elutionsmittel: Dichlormethan / 4-12 % (Methanol + 10% Ammoniak)) gereinigt.
   C₁₆H₂₄N₂O₂ (276.37)
   [M+H]+ = 277
   HPLC (Methode 1): Retentionszeit = 2.12 min
39b) 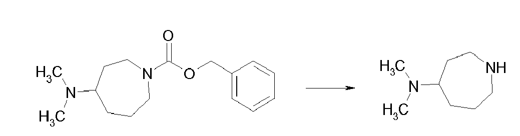 Eine Mischung aus 1.00 g (3.62 mMol) Produkt aus 39a, 0.10 g Palladium auf Kohle (10%) und 30 ml Methanol wird bei Raumtemperatur im Autoklaven hydriert. Der
   Katalysator wird danach abfiltriert, das Filtrat im Vakuum bis zur Trockene eingeengt.
   C₈H₁₈N₂ (142.24)
   [M+H]+ = 143
   DC: Kieselgel, Dichlormethan / Methanol / Ammoniak 9:1:0.1, Rf-Wert = 0.12
39c) 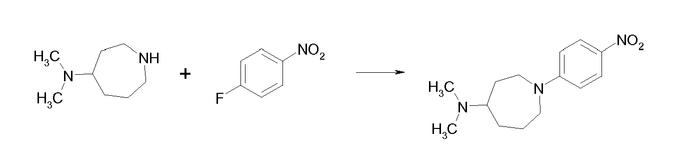 39c wird analog zu 1d aus 0.56 g (4.37 mMol) Produkt aus 39b, 0.64 g (4.50 mMol) 4-Fluor-nitrobenzol (Aldrich), 0.65 ml (4.60 mMol) Triethylamin in 5 ml DMF hergestellt.
   C₁₄H₂₁N₃O₂ (263.34)
   [M+H]+ = 264
   DC: Kieselgel, Dichlormethan / Methanol / Ammoniak 9:1:0.1, Rf-Wert = 0.60
39d) 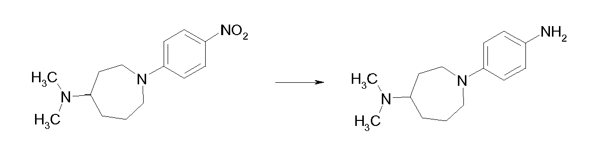 39d wird analog zu 8b aus 0.94 g (3.55 mMol) Produkt aus 39c und 0.10 g Palladium auf Kohle (10%) in 30 ml Methanol hergestellt.
   C₁₄H₂₃N₃O₂ (233.35)
   DC: Kieselgel, Dichlormethan / Methanol / Ammoniak 9:1:0.1, Rf-Wert = 0.15
39e) 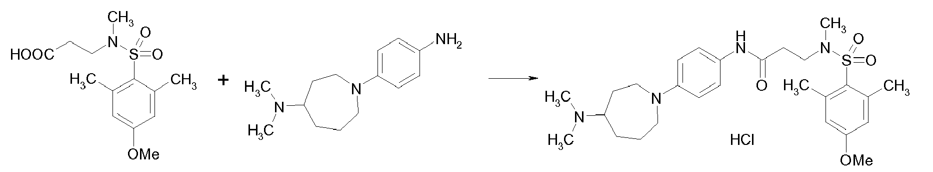 Beispiel 39 wird analog zu 1f aus 0.30 g (1.00 mMol) Produkt aus 22c, 0.23 g (1.00 mMol) Produkt aus 39d, 0.42 ml (3.00 mMol) Triethylamin und 0.32 g (1.00 mMol) TBTU in 15 ml DMF hergestellt.
   C₂₇H₄₀N₄O₄S x HCl (553.16)
   [M+H]+ = 517
   DC: Kieselgel, Dichlormethan / Methanol / Ammoniak 9:1:0.1, Rf-Wert = 0.42
   HPLC (Methode 5): Retentionszeit = 1.50 min

### Beispiel 40

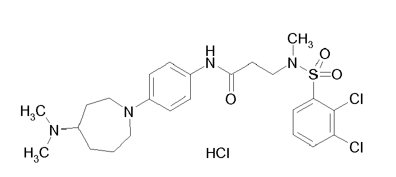

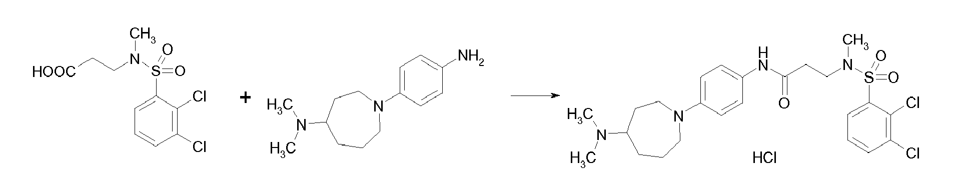

Beispiel 40 wird analog zu 1f aus 0.16 g (0.50 mMol) Produkt aus 1c, 0.12 g (0.50 mMol) Produkt aus 39d, 0.21 ml (1.50 mMol) Triethylamin und 0.16 g (0.50 mMol) TBTU in 5 ml DMF hergestellt.
C₂₄H₃₂Cl₂N₄O₃S x HCl (563,97)
[M+H]+ = 527/529/531
DC: Kieselgel, Dichlormethan / Methanol / Ammoniak 6:1:0.2, Rf-Wert = 0.48
HPLC (Methode 5): Retentionszeit = 1.53 min

### Beispiel 41

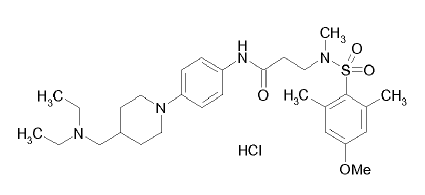

41a) 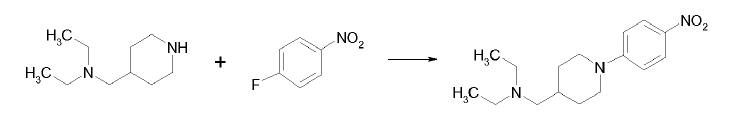 41 a wird analog zu 1d aus 1.00 g (5.87 mMol) Diethyl-piperidin-4-ylmethyl-amin (Chem. Pharm. Bull. 42, 1994, 74-84), 0.83 g (5.87 mMol) 4-Fluor-nitrobenzol (Aldrich), 1.14 ml (8.20 mMol) Triethylamin in 12 ml DMF hergestellt.
   C₁₆H₂₅N₃O₂ (291.39)
   [M+H]+ = 292
   DC: Kieselgel, Dichlormethan / Ethanol 9:1, Rf-Wert = 0.5
41b) 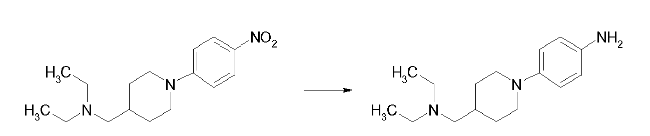 Eine Mischung aus 0.40 g (1.37 mMol) Produkt aus 41 a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
   C₁₆H₂₇N₃ (261.41)
   DC: Kieselgel, Dichlormethan / Ethanol 9:1, Rf-Wert = 0.1
41c) 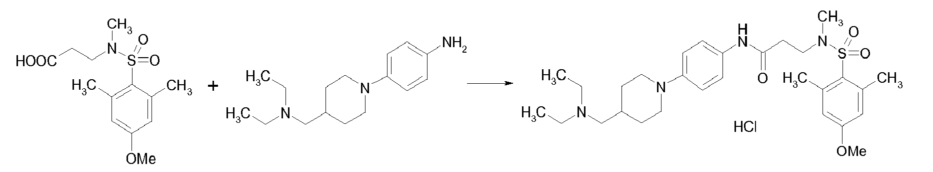 Beispiel 41 wird analog zu 1f aus 0.40 g (1.34 mMol) Produkt aus 22c, 0.35 g (1.34 mMol) Produkt aus 41 b, 0.47 ml (3.35 mMol) Triethylamin und 0.43g (1.34 mMol) TBTU in 7 ml THF und 1 ml DMF hergestellt.
   C₂₉H₄₄N₄O₄S x HCl (581.21)
   [M+H]+ = 545
   HPLC (Methode 5): Retentionszeit = 1.42 min

### Beispiel 42

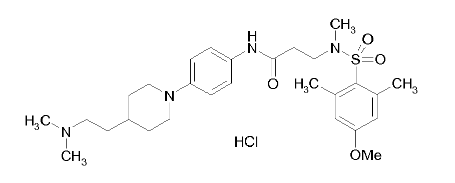

42a) 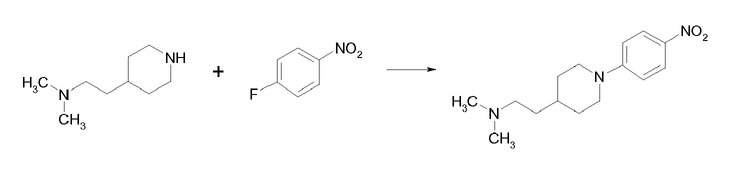 42a wird analog zu 1 d aus 1.88 g (12.00 mMol) Dimethyl-(2-piperidin-4-yl-ethyl)-amin (J. Med. Chem. 36, 1993, 162-165), 1.69 g (12.00 mMol) 4-Fluor-nitrobenzol (Aldrich), 2.37 ml (17.00 mMol) Triethylamin in 15 ml DMF hergestellt.
   C₁₅H₂₃N₃O₂ (277.36)
   [M+H]+ = 278
   DC: Kieselgel, Dichlormethan / Ethanol 9:1, Rf-Wert = 0.2
42b) 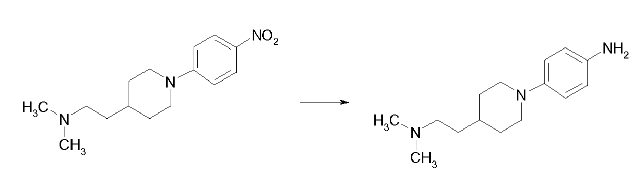 Eine Mischung aus 0.30 g (1.08 mMol) Produkt aus 42a, 0.10 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
   C₁₅H₂₅N₃ (247.38)
42c) 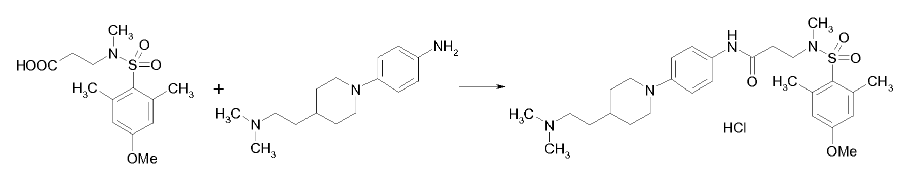 Beispiel 42 wird analog zu 1f aus 0.33 g (1.08 mMol) Produkt aus 22c, 0.27 g (1.08 mMol) Produkt aus 42b, 0.38 ml (2.70 mMol) Triethylamin und 0.35g (1.08 mMol) TBTU in 7 ml THF und 1 ml DMF hergestellt.
   C₂₈H₄₂N₄O₄S (530.72)
   [M+H]+ = 531
   HPLC (Methode 5): Retentionszeit = 1.41 min

### Beispiel 43

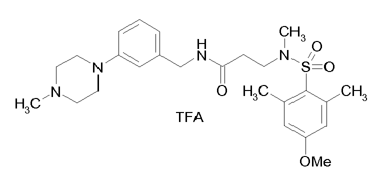

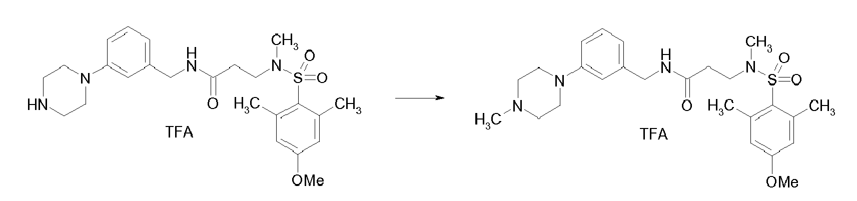

Eine Mischung aus 0.15 g (0.25 mMol) Produkt aus 36d, 0.025 ml (0.40 mMol) Methyliodid (Aldrich), 0.10 ml (0.75 mMol) Kaliumcarbonat und 5 ml Acetonitril wird über Nacht bei Raumtemperatur gerührt. Die Reaktionsmischung wird danach mit 10%iger TFA versetzt, das Produkt durch präparative HPLC abgetrennt.
C₂₅H₃₆N₄O₄S x C₂HF₃O₂ (602.67)
[M+H]+ = 489
HPLC (Methode 2): Retentionszeit = 2.99 min

### Beispiel 44

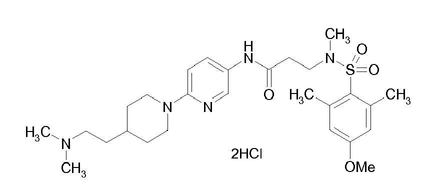

44a) 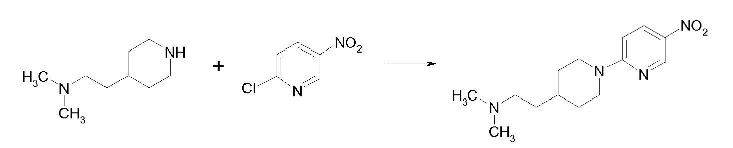 Eine Mischung aus 0.72 g (4.61 mMol) Dimethyl-(2-piperidin-4-yl-ethyl)-amin (J. Med. Chem. 36, 1993, 162-165), 0.73 g (4.61 mMol) 2-Chlor-5-nitropyridin (Fluka), 1.30 g (9.41 mMol) Kaliumcarbonat und 100 ml THF wird über das Wochenende bei Raumtemperatur gerührt. Der Niederschlag wird abfiltriert, das Filtrat wird im Vakuum bis zur Trockene eingeengt. Das so erhaltene Rohprodukt wird durch Säulenchromatographie über Kieselgel (Elutionsmittel: Dichlormethan / Methanol 19:1 bis 4:1) gereinigt.
   C₁₄H₂₂N₄O₂ (278.35)
   [M+H]+ = 279
44b) 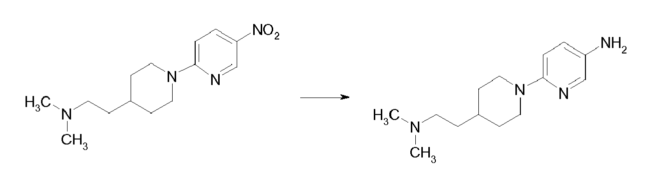 Eine Mischung aus 0.26 g (0.93 mMol) Produkt aus 44a, 0.05 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
   C₁₄H₂₄N₄ (248.37)
   [M+H]+ = 249
44c) 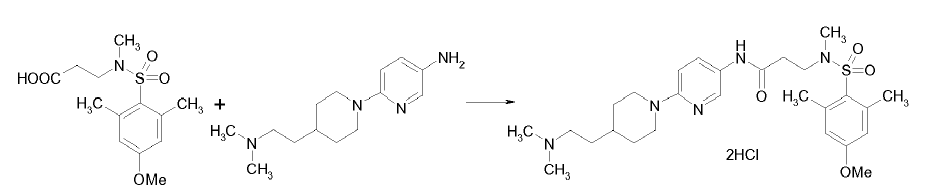 Beispiel 44 wird analog zu 1f aus 0.12 g (0.40 mMol) Produkt aus 22c, 0.10 g (0.40 mMol) Produkt aus 44b, 0.069 ml (0.50 mMol) Triethylamin und 0.14g (0.44 mMol) TBTU in 40 ml THF und 5 ml DMF hergestellt.
   C₂₇H₄₁N₅O₄S x 2HCl (604.63)
   [M+H]+ = 532
   HPLC (Methode 5): Retentionszeit = 1.40 min

### Beispiel 45

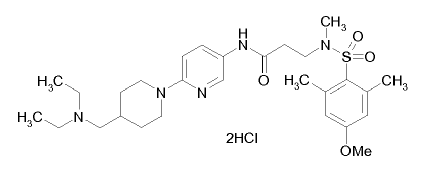

45a) 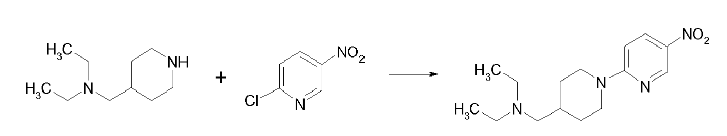 45a wird analog zu 44a aus 1.00 g (5.87 mMol) Diethyl-piperidin-4-ylmethyl-amin (Chem. Pharm. Bull. 42, 1994, 74-84), 0.93 g (5.87 mMol) 2-Chlor-5-nitropyridin (Fluka) und 1.70 g (12.30 mMol) Kaliumcarbonat in 100 ml THF hergestellt.
   C₁₅H₂₄N₄O₂ (292.38)
   [M+H]+ = 293
45b) 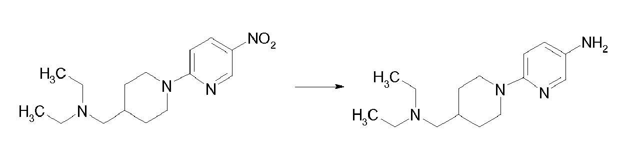 Eine Mischung aus 0.20 g (0.68 mMol) Produkt aus 45a, 0.03 g Palladium auf Kohle (10%) und 30 ml Methanol wird bei Raumtemperatur im Autoklaven hydriert. Der Katalysator wird danach abfiltriert, das Filtrat im Vakuum bis zur Trockene eingeengt.
   C₁₅H₂₆N₄ (262.39)
   [M+H]+ = 263
45c)

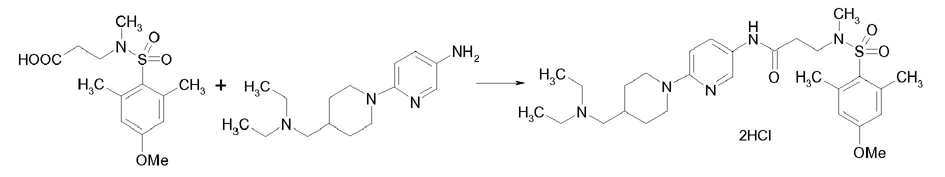

Beispiel 45 wird analog zu 1f aus 0.16 g (0.53 mMol) Produkt aus 22c, 0.14 g (0.53 mMol) Produkt aus 45b, 0.096 ml (0.69 mMol) Triethylamin und 0.19 g (0.58 mMol) TBTU in 40 ml THF und 5 ml DMF hergestellt.
C₂₈H₄₃N₅O₄S x 2HCl (618.66)
[M+H]+ = 546
HPLC (Methode 5): Retentionszeit = 1.40 min

### Beispiel 46

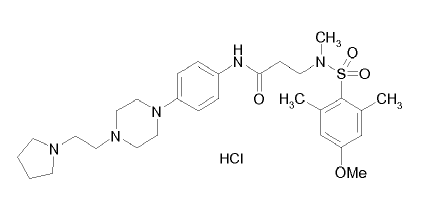

46a) 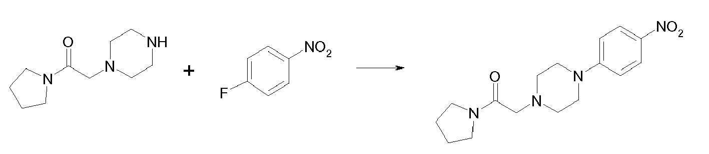 46a wird analog zu 1d aus 3.00 g (15.21 mMol) 2-Piperazin-1-yl-1-pyrrolidin-1-yl-ethanon (Chess), 2.15 g (15.21 mMol) 1-Fluor-4-nitrobenzol (Aldrich) und 3.07 ml (22.00 mMol) Triethylamin in 25 ml DMF hergestellt.
   C₁₆H₂₂N₄O₃ (318.37)
   [M+H]+ = 319
   DC: Kieselgel, Dichlormethan / Ethanol 9:1, Rf-Wert = 0.4
46b) 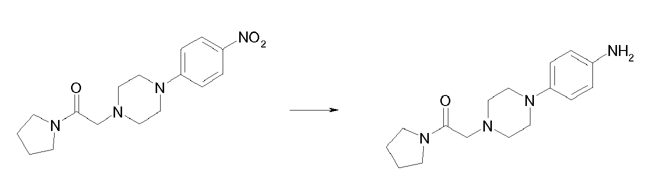 Eine Mischung aus 3.00 g (9.42 mMol) Produkt aus 46a, 0.30 g Palladium auf Kohle (10%) und 200 ml Methanol wird bei Raumtemperatur im Autoklaven hydriert. Der Katalysator wird danach abfiltriert, das Filtrat im Vakuum bis zur Trockene eingeengt.
   C₁₆H₂₄N₄O (288.39)
   DC: Kieselgel, Dichlormethan / Ethanol 9:1, Rf-Wert = 0.42
46c) 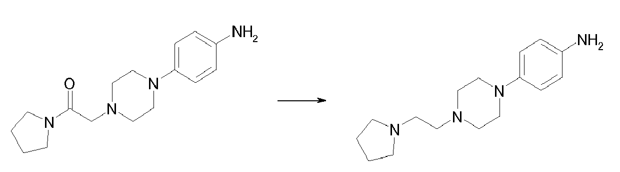 30.00 ml (30.00 mMol) Lithiumaluminiumhydrid 1 M in THF (Aldrich) wird in 50 ml THF vorgelegt und bei Raumtemperatur mit einer Mischung aus 2.70 g (9.36 mMol) Produkt aus 46b und 20 ml THF versetzt. Die Reaktionsmischung wird danach drei Stunden bei Raumtemperatur gerührt und anschließend unter Eisbadkühlung mit 20%iger Natronlauge versetzt. Der entstandene Niederschlag wird abfiltriert, das Filtrat im Vakuum bis zur Trockene eingeengt.
   C₁₆H₂₆N₄ (274.40)
46d) 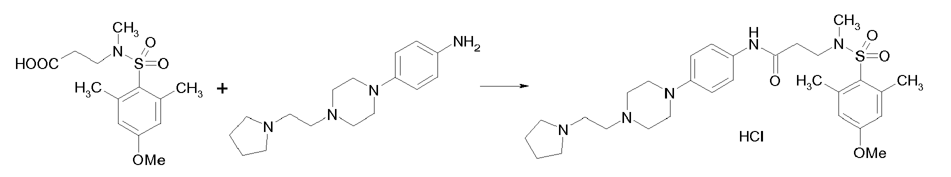 Beispiel 46 wird analog zu 1f aus 0.30 g (1.00 mMol) Produkt aus 22c, 0.27 g (1.00 mMol) Produkt aus 46c, 0.35 ml (2.50 mMol) Triethylamin und 0.32 g (1.00 mMol) TBTU in 7 ml THF und 1 ml DMF hergestellt.
   C₂₉H₄₃N₅O₄S x HCl (594.21)
   [M+H]+ = 558
   HPLC (Methode 5): Retentionszeit = 1.43 min

### Beispiel 47

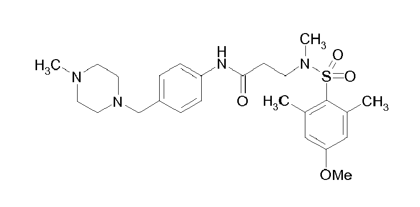

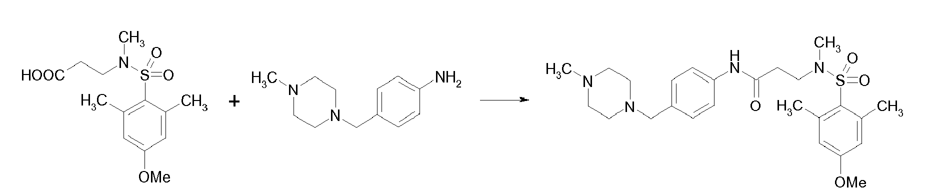

Beispiel 47 wird analog zu 1f aus 0.20 g (0.66 mMol) Produkt aus 22c, 0.14 g (0.66 mMol) 4-(4-Methyl-piperazin-1-ylmethyl)-phenylamin (Med. Chem. Res. 9, 1999, 149-161), 0.28 ml (1.99 mMol) Triethylamin und 0.21 g (0.66 mMol) TBTU in 5 ml THF hergestellt.
C₂₅H₃₆N₄O₄S (488.64)
[M+H]+ = 489
HPLC (Methode 5): Retentionszeit = 1.42 min

### Beispiel 48

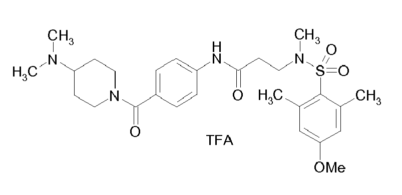

48a) 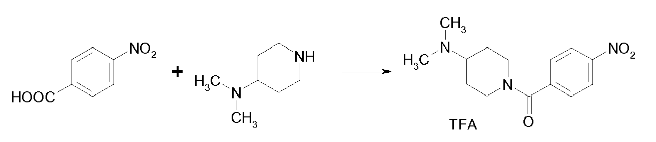
48a wird analog zu 1f aus 0.24 g (1.45 mMol) 4-Nitrobenzoesäure (Aldrich), 0.19 g (1.45 mMol) 4-Dimethylamino-piperidin (Alfa Aesar), 0.21 ml (1.52 mMol) Triethylamin und 0.49 g (1.52 mMol) TBTU in 8 ml DMF hergestellt.
   C₁₄H₁₉N₃O₃ x C₂HF₃O₂ (391.34)
   [M+H]+ = 278
   HPLC (Methode 2): Retentionszeit = 2.29 min
48b) 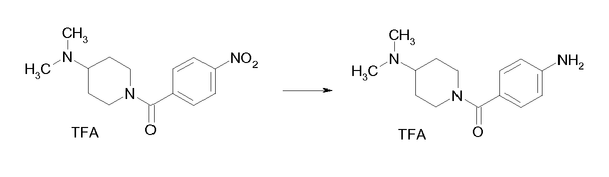 Eine Mischung aus 0.36 g (0.92 mMol) Produkt aus 48a, 0.092 g Palladium auf Kohle (10%) und 5 ml Methanol wird bei Raumtemperatur im Autoklaven hydriert. Der Katalysator wird danach abfiltriert, das Filtrat im Vakuum bis zur Trockene eingeengt. C₁₄H₂₁N₃0 x C₂HF₃O₂ (361.36)
   [M+H]+ = 248
   HPLC (Methode 2): Retentionszeit = 0.66 min
48c) 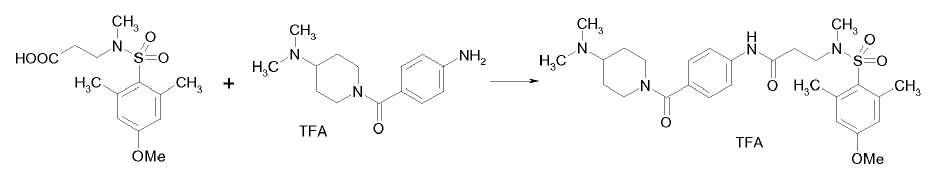 Beispiel 48 wird analog zu 1f aus 0.15 g (0.50 mMol) Produkt aus 22c, 0.18 g (0.50 mMol) Produkt aus 48b, 0.21 ml (1.49 mMol) Triethylamin und 0.16 g (0.50 mMol) TBTU in 3 ml
   THF hergestellt.
   C₂₇H₃₈N₄O₅S x C₂HF₃O₂ (644.70)
   [M+H]+ = 531
   HPLC (Methode 5): Retentionszeit = 1.48 min

### Beispiel 49

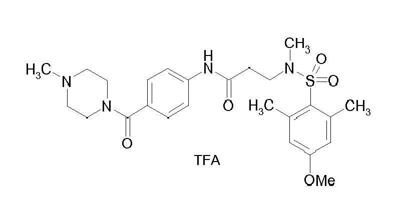

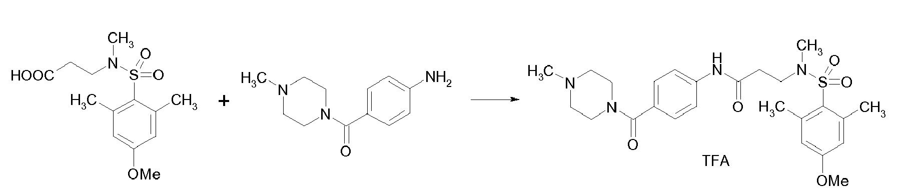

Beispiel 49 wird analog zu 1f aus 0.15 g (0.50 mMol) Produkt aus 22c, 0.11 g (0.50 mMol) (4-Aminophenyl)-(4-methylpiperazin-1-yl)-methanon (J. Org. Chem. 24, 1959, 459-463), 0.21 ml (1.49 mMol) Triethylamin und 0.16 g (0.50 mMol) TBTU in 3 ml THF hergestellt. C₂₅H₃₄N₄O₅S x C₂HF₃O₂ (616.65)
[M+H]+ = 503
HPLC (Methode 5): Retentionszeit = 1.47 min

### Beispiel 50

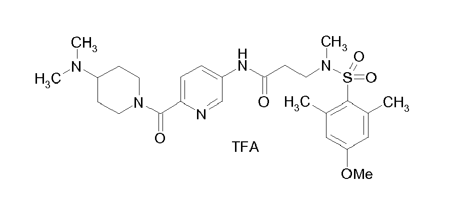

50a) 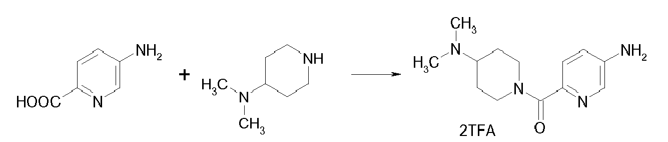 50a wird analog zu 1f aus 0.60 g (4.34 mMol) 5-Amino-pyridin-2-carbonsäure (Pharm. Acta Helv. 44, 1969, 637-643), 0.56 g (4.34 mMol) 4-Dimethylamino-piperidin (Alfa Aesar), 0.64 ml (4.56 mMol) Triethylamin und 1.46 g (4.56 mMol) TBTU in 24 ml DMF hergestellt.
   C₁₃H₂₀N₄O x 2C₂HF₃O₂ (476.37)
   HPLC (Methode 2): Retentionszeit = 0.65 min
50b) 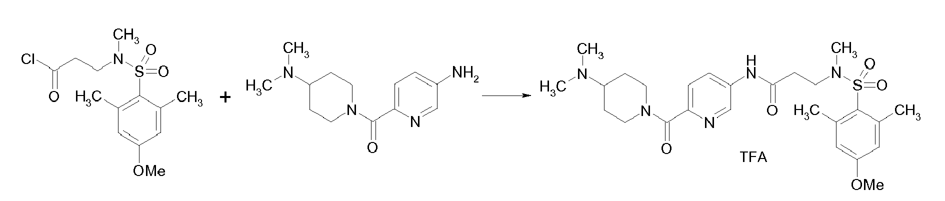 Eine Mischung aus 0.64 g (1.99 mMol) Produkt aus 27c, 1.90 g (2.39 mMol) Produkt aus 50a, 0.08 g (0.33 mMol) DMAP und 16 ml Chlorbenzol wird 39 Stunden auf 15°C erhitzt. Die Reaktionsmischung wird danach im Vakuum bis zur Trockene eingeengt. Das Produkt wird durch präparative HPLC erhalten.
   C₂₆H₃₇N₅O₅S x C₂HF₃O₂ (645.69)
   [M+H]+ = 532
   HPLC (Methode 5): Retentionszeit = 1.44 min

### Beispiel 51

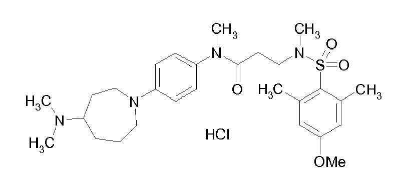

51a) 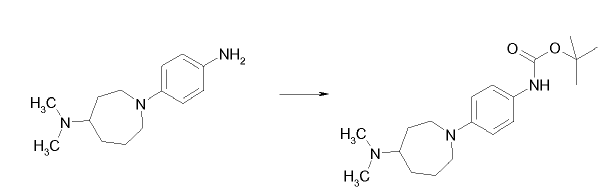 Eine Mischung aus 0.26 g (1.11 mMol) Produkt aus 39d, 0.27 g (1.22 mMol) Boc-Anhydrid, 0.17 ml (1.22 mMol) Triethylamin und 15 ml Dichlormethan wird über Nacht bei Raumtemperatur gerührt. Danach wird die Reaktionsmischung mit Dichlormethan verdünnt, mit gesättigter Natriumhydrogencarbonat-Lösung und gesättigter Natriumchlorid-Lösung gewaschen, über Natriumsulfat getrocknet und im Vakuum bis zur Trockene eingeengt.
   C₁₉H₃₁N₃O₂ (333.47)
   [M+H]+ = 334
   HPLC (Methode 1): Retentionszeit = 2.40 min
51b) 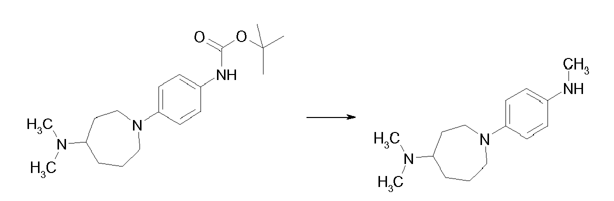 0.13 g (3.40 mMol) Lithiumaluminiumhydrid werden in 5 ml THF vorgelegt, auf 60°C erhitzt und mit 0.38 g (1.14 mMol) Produkt aus 51a in 5 ml THF versetzt. Die Reaktionsmischung wird danach vier Stunden unter Rückfluß erhitzt und über Nacht bei Raumtemperatur gerührt. Anschließend wird die Reaktionsmischung mit Wasser und 1 M Natronlauge gequencht. Der entstandene Niederschlag wird über Celite abfiltriert, das Filtrat im Vakuum bis zur Trockene eingeengt.
   C₁₅H₂₅N₃ (247.38)
   DC: Kieselgel, Dichlormethan / Methanol / Ammoniak 4:1:0.2, Rf-Wert = 0.68
51c) 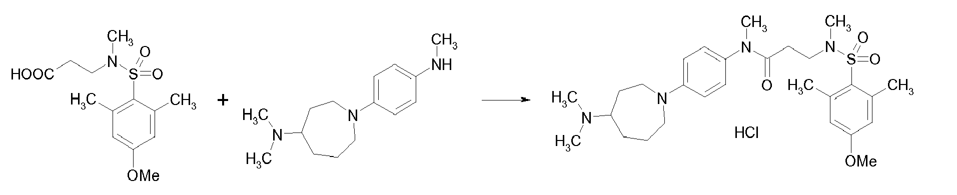 Beispiel 51 wird analog zu 1f aus 0.15 g (0.50 mMol) Produkt aus 22c, 0.12 g (0.50 mMol) Produkt aus 51b, 0.21 ml (1.50 mMol) Triethylamin und 0.18 g (0.55 mMol) TBTU in 8 ml DMF hergestellt.
   C₂₈H₄₂N₄O₄S x HCl (567.18)
   [M+H]+ = 531
   HPLC (Methode 1): Retentionszeit = 2.5 min

### Beispiel 52

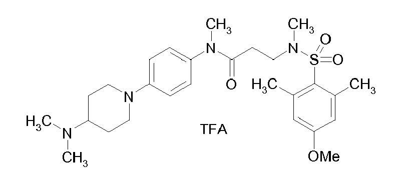

52a) 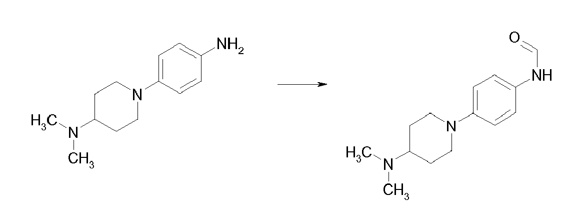 0.70 ml (7.36 mMol) Essigsäureanhydrid werden unter Stickstoff-Atmosphäre vorgelegt und unter Eisbadkühlung langsam mit 0.42 ml (9.06 mMol) Ameisensäure versetzt. Die Reaktionsmischung wird zwei Stunden auf 50-60°C erhitzt und anschließend unter Eisbadkühlung mit 0.50 g (2.28 mMol) Produkt aus 8b in 7 ml Dichlormethan versetzt. Nach 20-minütigem Rühren bei Raumtemperatur wird die Reaktionsmischung im Vakuum bis zur Trockene eingeengt. Das so erhaltene Rohprodukt wird durch Säulenchromatographie über Kieselgel (Elutionsmittel: Dichlormethan / Methanol / Ammoniak 9:1:0.1) gereinigt.
   C₁₄H₂₁N₃O (247.34)
   [M+H]+ = 248
   HPLC (Methode 5): Retentionszeit = 0.50 min
52b) 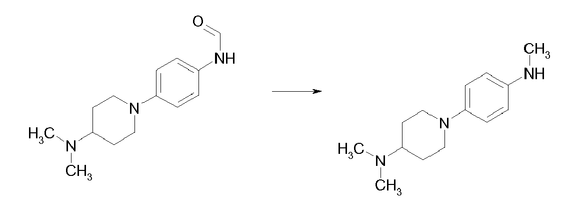 52b wird analog zu 51 b aus 0.17 g (4.51 mMol) Lithiumaluminiumhydrid und 0.58 g (2.34 mMol) Produkt aus 52a in 10 ml THF hergestellt.
   C₁₄H₂₃N₃ (233.35)
   DC: Kieselgel, Dichlormethan / Methanol / Ammoniak 9:1:0.1, Rf-Wert = 0.5
52c) 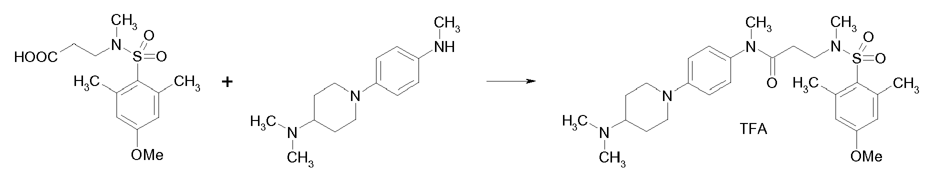 Beispiel 52 wird analog zu 1f aus 0.21 g (0.68 mMol) Produkt aus 22c, 0.24 g (0.68 mMol) Produkt aus 52b, 0.28 ml (2.04 mMol) Triethylamin und 0.22 g (0.68 mMol) TBTU in 4 ml
   THF hergestellt.
   C₂₇H₄₀N₄O₄S x C₂HF₃O₂ (630.72)
   [M+H]+ = 517
   HPLC (Methode 5): Retentionszeit = 1.50 min

### Beispiel 53

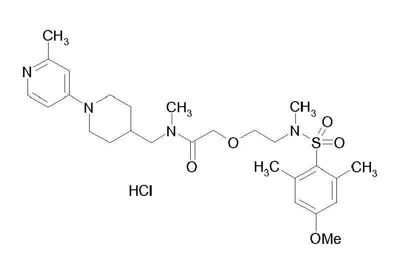

53a) 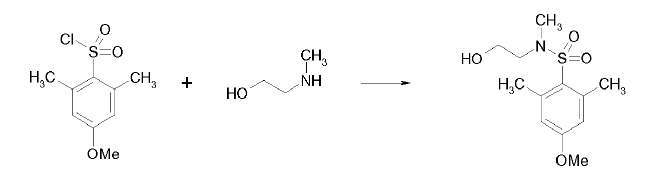 53a wird analog zu 3a aus 4.50 g (19.17 mMol) Produkt aus 13a, 1.69 g (21.10 mMol) N-Methylaminoethanol (BASF), 6.68 ml (47.90 mMol) Triethylamin in 150 ml Dichlormethan hergestellt.
   C₁₂H₁₉NO₄S (273.35)
   [M+H]+ = 274
   DC: Kieselgel, Dichlormethan / Ethanol 19:1, Rf-Wert = 0.43
53b) 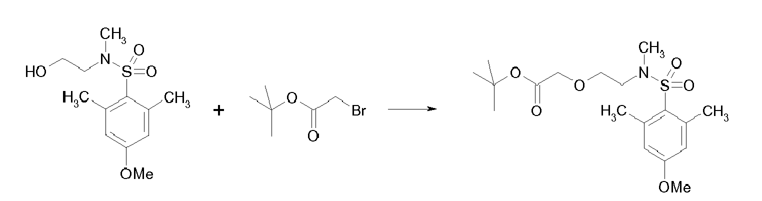 Eine Mischung aus 5.15 g (18.84 mMol) Produkt aus 53a, 1.75 g (6.60 mMol) Tetrabutylammoniumchlorid (Fluka) und 80 ml Toluol wird bei 0°C erst mit 100 ml 35%iger Natronlauge, dann mit 4.18 ml (28.26 mMol) Bromessigsäure-tert-butylester in 20 ml Toluol versetzt. Die Reaktionsmischung wird anschließend 1.5 Stunden bei Raumtemperatur gerührt, dann mit Diethylether verdünnt. Nach der Phasentrennung wird die organische Phase viermal mit Wasser neutral gewaschen, über Natriumsulfat getrocknet und im Vakuum bis zur Trockene eingeengt. Das so erhaltene Rohprodukt wird durch Säulen-chromatographie über Kieselgel (Elutionsmittel: Petrolether /Ethylacetat 4:1) gereinigt. C₁₈H₂₉NO₆S (387.49)
   [M+H]+ = 388
   DC: Kieselgel, Petrolether / Ethylacetat 7:3, Rf-Wert = 0.59
53c) 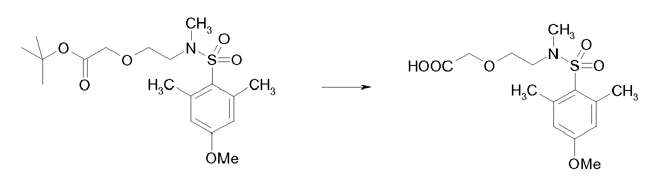 Eine Mischung aus 6.80 g (17.55 mMol) Produkt aus 53b, 8 ml TFA und 100 ml Dichlormethan wird 2.5 Stunden bei Raumtemperatur gerührt. Die Reaktionsmischung wird danach im Vakuum bis zur Trockene eingeengt. Man versetzt den Rückstand mit 1 M Natronlauge und extrahiert zweimal mit Ethylacetat (organische Extrakte verwerfen). Die wässrige Phase wird mit 2 M HCl angesäuert, dann nochmals mit Ethylacetat extrahiert. Die organischen Extrakte werden mit Wasser gewaschen, über Natriumsulfat getrocknet und im Vakuum bis zur Trockene eingeengt.
   C₁₄H₂₁NO₆S (331.29)
   [M+H]+ = 332
   HPLC (Methode 4): Retentionszeit = 3.4 min
53d) 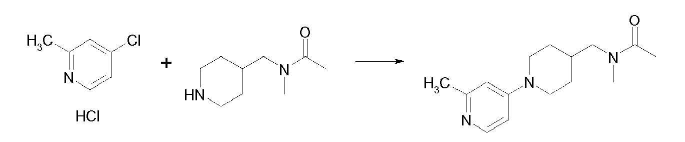 53d wird analog zu 28c aus 1.00 g (6.10 mMol) 4-Chlor-2-methylpyridin Hydrochlorid (Alfa Aesar) und 2.08 g (12.20 mMol) N-Methyl-N-piperidin-4-ylmethyl-acetamid (DE 1100635, Rhöne-Poulenc, 1961) hergestellt.
   C₁₅H₂₃N₃O (261.36)
   [M+H]+ = 262
   HPLC (Methode 4): Retentionszeit = 1.9 min
53e) 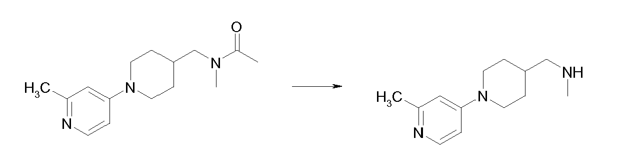 Eine Mischung aus 1.37 g (5.24 mMol) Produkt aus 53d und 15 ml halbkonzentrierte HCl wird vier Tage unter Rückfluss erhitzt. Die Reaktionsmischung wird mit 20%iger Natronlauge alkalisch gestellt und mit Dichlormethan extrahiert. Die organischen Extrakte werden über Natriumsulfat getrocknet und im Vakuum bis zur Trockene eingeengt.
   C₁₃H₂₁N₃ (219.33)
   [M+H]+ = 220
   DC: Kieselgel, Dichlormethan / Methanol / Ammoniak 8:2:0.2, Rf-Wert = 0.48
53f) 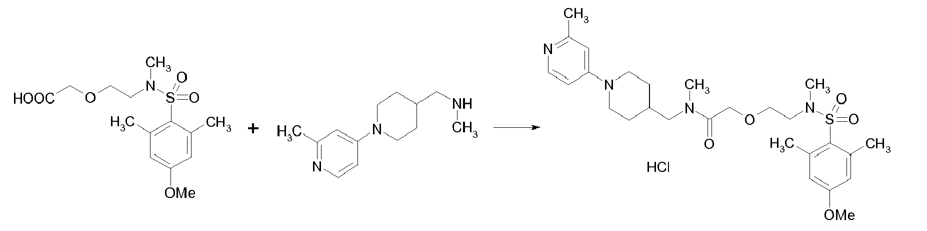 Beispiel 53 wird analog zu 1f aus 0.099 g (0.30 mMol) Produkt aus 53c, 0.066 g (0.30 mMol) Produkt aus 53e, 0.10 ml (0.75 mMol) Triethylamin und 0.12 g (0.36 mMol) TBTU in 8 ml THF und 1 ml DMF hergestellt.
   C₂₇H₄₀N₄O₅S x HCl (569.16)
   [M+H]+ = 533
   HPLC (Methode 4): Retentionszeit = 3.1 min

### Beispiel 54

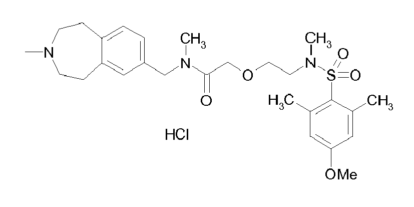

54a) 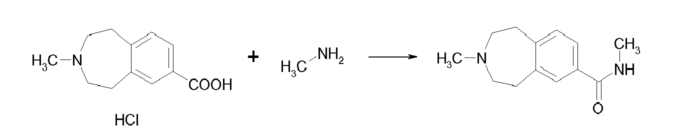 54a wird analog zu 1f aus 2.00 g (8.27 mMol) Produkt aus 38d, 8.28 ml (16.55 mMol) Methylamin 2 M in THF (Aldrich), 3.46 ml (24.82 mMol) Triethylamin und 3.19 g (9.93 mMol) TBTU in 30 ml THF hergestellt.
   C₁₃H₁₈N₂O (218.29)
   [M+H]+ = 219
   DC: Kieselgel, Dichlormethan / Methanol 8:2, Rf-Wert = 0.14
54b) 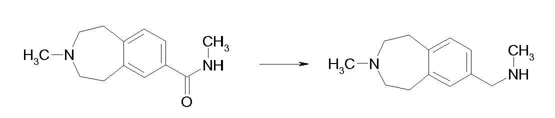 54b wird analog zu 38f aus 1.00 g (4.58 mMol) Produkt aus 54a und 9.00 ml (9.00 mMol) Lithiumaluminiumhydrid 1 M in THF (Aldrich) in 30 ml THF hergestellt.
   C₁₃H₂₀N₂ (204.31)
   [M+H]+ = 205
   DC: Kieselgel, Dichlormethan / Methanol 8:2, Rf-Wert = 0.07
54c) 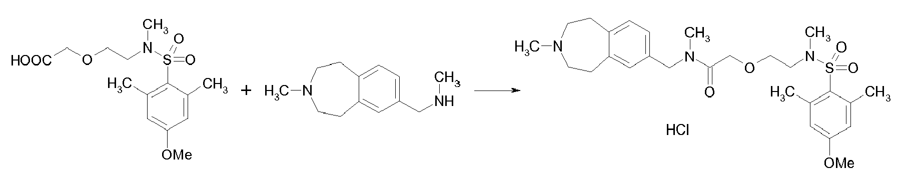 Beispiel 54 wird analog zu 1f aus 0.099 g (0.30 mMol) Produkt aus 53c, 0.088 g (0.30 mMol) Produkt aus 54b, 0.10 ml (0.75 mMol) Triethylamin und 0.12 g (0.36 mMol) TBTU in 7 ml THF und 1 ml DMF hergestellt.
   C₂₇H₃₉N₃O₅S x HCl (554.14)
   [M+H]+ = 518
   HPLC (Methode 4): Retentionszeit = 3.1 min

### Beispiel 55

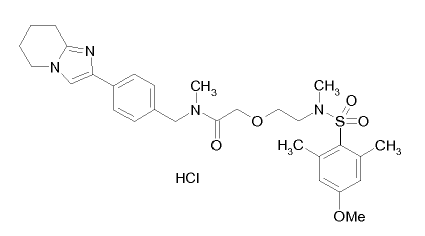

55a) 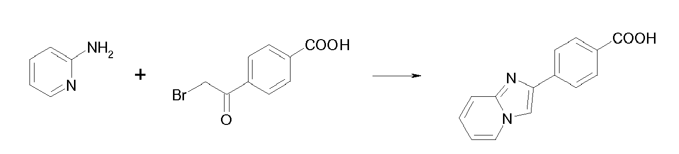 Eine Mischung aus 3.20 g (34.00 mMol) 2-Aminopyridin (Aldrich), 2.75 g (11.31 mMol) 4-Bromacetylbenzoesäure (Fluorochem) und 100 ml Ethanol wird sechs Stunden unter Rückfluß erhitzt und über Nacht bei Raumtemperatur gerührt. Der entstandene Niederschlag wird abfiltriert und getrocknet.
   C₁₄H₁₀N₂O₂ (238.24)
   [M+H]+ = 239
55b) 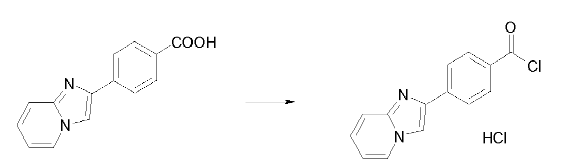 55b wird analog zu 27c aus 1.7 g (7.14 mMol) Produkt aus 55a und 30 ml Thionylchlorid hergestellt.
   C₁₄H₉ClN₂O x HCl (293.15)
55c) 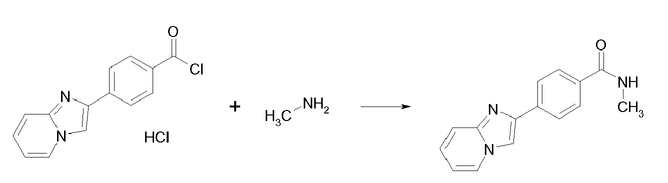 2.10 g (7.14 mMol) Produkt aus 55b in 100 ml Dichlormethan werden unter Eisbadkühlung mit 25 ml (50.00 mMol) Methylamin 2 M in THF (Aldrich) versetzt. Die Reaktionsmischung wird danach zwei Stunden bei Raumtemperatur gerührt und anschließend im Vakuum bis zur Trockene eingeengt. Der Rückstand wird mit Wasser verrieben, abfiltriert und getrocknet. Das so erhaltene Rohprodukt wird durch Säulenchromatographie über Kieselgel (Elutionsmittel: Dichlormethan / Methanol 97:3) gereinigt.
   C₁₅H₁₃N₃O (251.28)
   [M+H]+ = 252
55d) 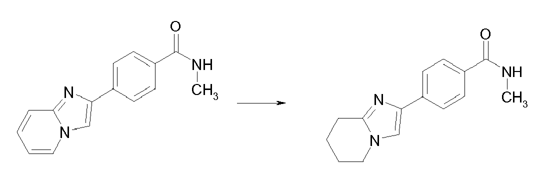 Eine Mischung aus 0.70 g (2.79 mMol) Produkt aus 55c, 0.15 g Palladium auf Kohle (20%), 100 ml Methanol und 30 ml Dichlormethan wird bei Raumtemperatur im Autoklaven hydriert. Der Katalysator wird abfiltriert, das Filtrat im Vakuum bis zur Trockene eingeengt.
   C₁₅H₁₇N₃O (255.32)
   [M+H]+ = 256
55e) 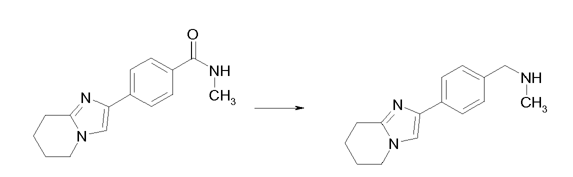 55e wird analog zu 38f aus 0.80 g (3.13 mMol) Produkt aus 55d und 20.00 ml (20.00 mMol) Lithiumaluminiumhydrid 1 M in THF (Aldrich) in 50 ml Pyridin hergestellt.
   C₁₅H₁₉N₃ (241.33)
   [M+H]+ = 242
55f) 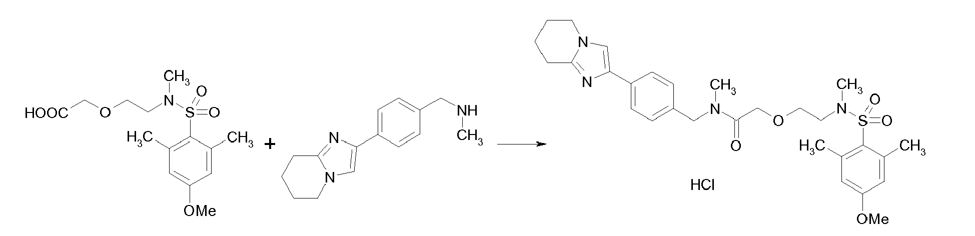 Beispiel 55 wird analog zu 1f aus 0.14 g (0.42 mMol) Produkt aus 53c, 0.10 g (0.41 mMol) Produkt aus 55e, 0.14 ml (0.99 mMol) Triethylamin und 0.15 g (0.46 mMol) TBTU in 30 ml THF und 5 ml DMF hergestellt.
   C₂₉H₃₈N₄O₅S x HCl (591.16)
   [M+H]+ = 555
   DC: Kieselgel, Dichlormethan / Methanol 9:1, Rf-Wert = 0.26

### Beispiel 56

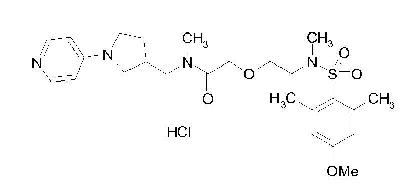

56a) 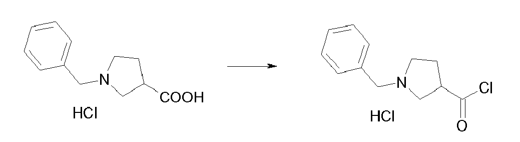 56a wird analog zu 27c aus 1.35 g (5.59 mMol) 1-Benzyl-pyrrolidine-3-carbonsäure (J. Org. Chem. 33, 1968, 3637-3639) und 10 ml Thionylchlorid hergestellt. C₁₂H₁₄CINO x HCl (260.16)
56b) 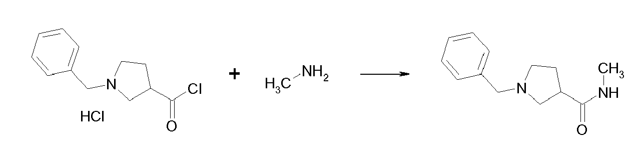 56b wird analog zu 55c aus 1.45 g (5.57 mMol) Produkt aus 56a, 10 ml (50.00 mMol) Methylamin 2 M in THF (Aldrich) in 50 ml THF hergestellt.
   C₁₃H₁₈N₂O (218.29)
   [M+H]+ = 219
56c) 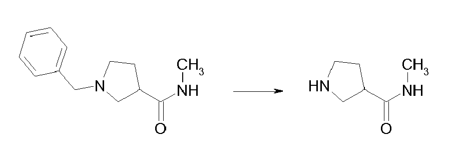 Eine Mischung aus 1.10 g (5.04 mMol) Produkt aus 56b, 0.20 g Palladiumhydroxid und 40 ml Methanol wird bei 50°C im Autoklaven hydriert. Der Katalysator wird abfiltriert, das Filtrat im Vakuum bis zur Trockene eingeengt.
   C₆H₁₂N₂O (128.17)
   [M+H]+ = 129
56d) 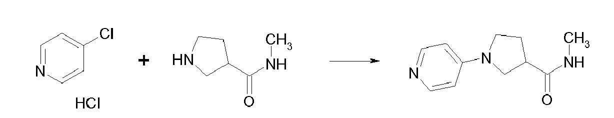 56d wird analog zu 28c aus 0.76 g (5.08 mMol) 4-Chlorpyridin Hydrochlorid (Aldrich), 0.65 g (5.07 mMol) Produkt aus 56c und 1.52 ml (10.88 mMol) Triethylamin in 10 ml Ethanol hergestellt.
   C₁₁H₁₅N₃O (205.26)
   [M+H]+ = 206
56e) 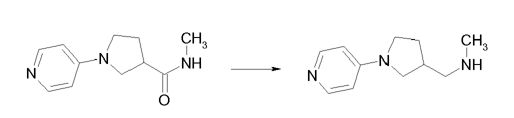 56e wird analog zu 38f aus 0.45 g (2.19 mMol) Produkt aus 56d und 7.00 ml (7.00 mMol) Lithiumaluminiumhydrid 1 M in THF (Aldrich) in 30 ml THF hergestellt.
   C₁₁H₁₇N₃ (191.27)
   [M+H]+ = 192
56f) 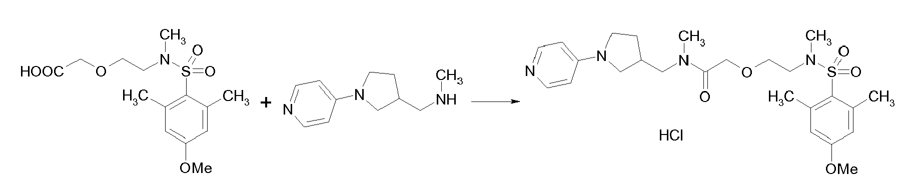 Beispiel 56 wird analog zu 1f aus 0.14 g (0.42 mMol) Produkt aus 53c, 0.10 g (0.42 mMol) Produkt aus 56e, 0.18 ml (1.29 mMol) Triethylamin und 0.18 g (0.56 mMol) TBTU in 30 ml THF und 5 ml DMF hergestellt.
   C₂₅H₃₆N₄O₅S x HCl (541.10)
   [M+H]+ = 505
   HPLC (Methode 5): Retentionszeit = 1.51 min

### Beispiel 57

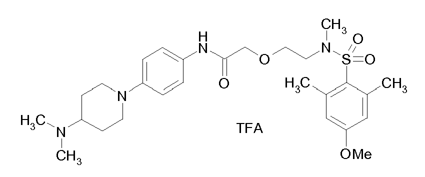

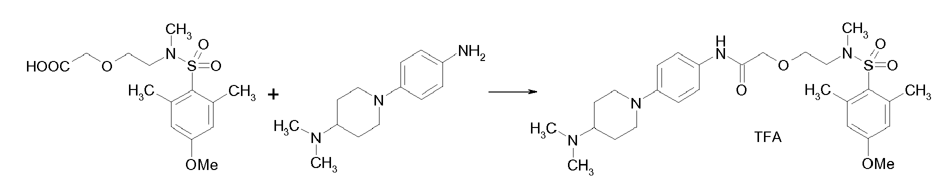

Beispiel 57 wird analog zu 1f aus 0.10 g (0.30 mMol) Produkt aus 53c, 0.066 g (0.30 mMol) Produkt aus 8b, 0.13 ml (0.91 mMol) Triethylamin und 0.097 g (0.30 mMol) TBTU in 5 ml DMF hergestellt.
C₂₇H₄₀N₄O₅S x C₂HF₃O₂ (646.72)
[M+H]+ = 533
HPLC (Methode 5): Retentionszeit = 1.51 min

### Beispiel 58

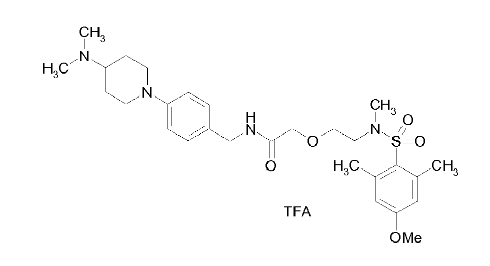

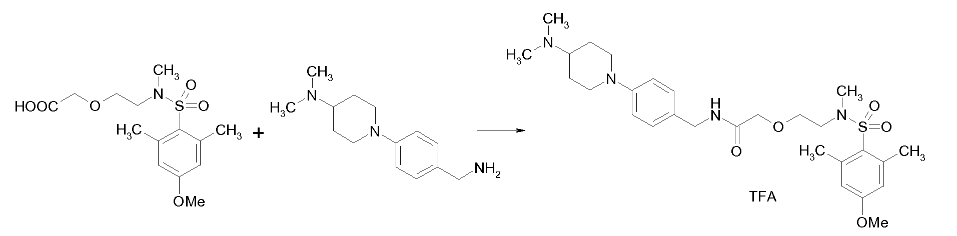

Beispiel 58 wird analog zu 1f aus 0.10 g (0.30 mMol) Produkt aus 53c, 0.07 g (0.30 mMol) Produkt aus 33a, 0.13 ml (0.91 mMol) Triethylamin und 0.097 g (0.30 mMol) TBTU in 5 ml DMF hergestellt.
C₂₈H₄₂N₄O₅S x C₂HF₃O₂ (660.75)
[M+H]+ = 547
HPLC (Methode 5): Retentionszeit = 1.48 min

### Beispiel 59

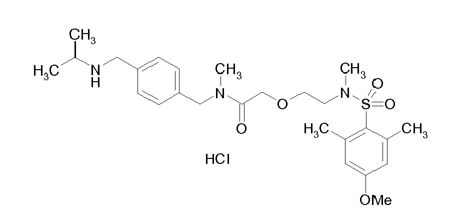

59a) 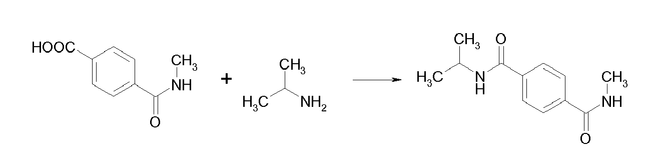 59a wird analog zu 1f aus 2.00 g (11.16 mMol) Terephthalsäuremonomethylamid (EMKA), 1.90 ml (22.32 mMol) Isopropylamin (Aldrich), 3.11 ml (22.32 mMol) Triethylamin und 4.30 g (13.40 mMol) TBTU in 60 ml THF hergestellt.
   C₁₂H₁₆N₂O₂ (220.27)
   [M+H]+ = 221
   HPLC (Methode 4): Retentionszeit = 2.3 min
59b) 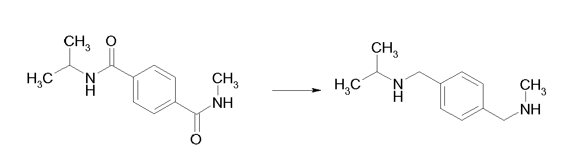 59b wird analog zu 38f aus 1.34 g (6.08 mMol) Produkt aus 59a und 25.00 ml (25.00 mMol) Lithiumaluminiumhydrid 1 M in THF (Aldrich) in 150 ml THF hergestellt.
   C₁₂H₂₀N₂ (192.30)
   [M+H]+ = 193
   DC: Kieselgel, Dichlormethan / Methanol / Ammoniak 9:1:0.1, Rf-Wert = 0.17
59c) 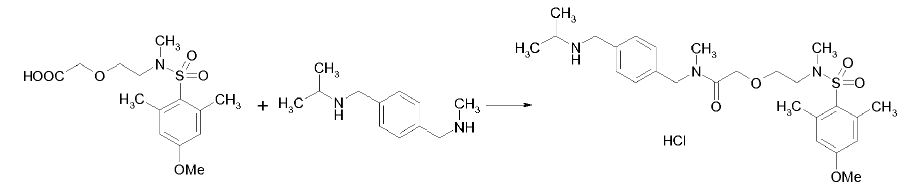 Beispiel 59 wird analog zu 1f aus 0.099 g (0.30 mMol) Produkt aus 53c, 0.058 g (0.30 mMol) Produkt aus 59b, 0.10 ml (0.75 mMol) Triethylamin und 0.12 g (0.36 mMol) TBTU in 8 ml THF hergestellt.
   C₂₆H₃₉N₃O₅S x HCl (542.13)
   [M+H]+ = 506
   HPLC (Methode 4): Retentionszeit = 3.1 min

### Beispiel 60

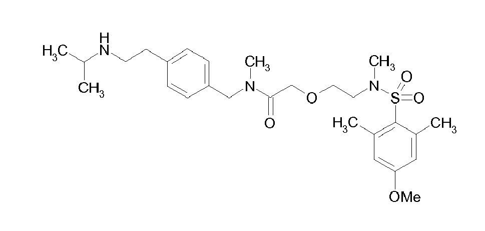

60a) 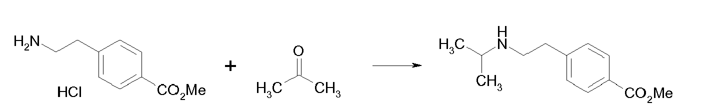 Eine Mischung aus 3.00 g (13.91 mMol) 4-Aminoethylbenzoesäuremethylester (EMKA), 1.94 ml (13.91 mMol) Triethylamin und 50 ml THF wird 10 min bei Raumtemperatur gerührt und danach mit 1.13 ml (15.30 mMol) Aceton versetzt. Die Reaktionsmischung wird weitere 30 min bei Raumtemperatur gerührt, dann werden 3.24 g (15.30 mMol) Natriumtriacetoxyborhydrid und 1.19 ml (20.86 mMol) Essigsäure hinzugefügt. Man lässt 16 Stunden bei Raumtemperatur rühren. Die Reaktionsmischung wird im Vakuum bis zur Trockene eingeengt, der Rückstand wird in 1 M HCl aufgenommen und mit Ethylacetat extrahiert (organische Phase verwerfen). Die wässrige Phase wird mit gesättigter Kaliumcarbonat-Lösung alkalisch gestellt und mit Ethylacetat extrahiert. Die organischen Extrakte werden über Natriumsulfat getrocknet und im Vakuum bis zur Trockene eingeengt.
   C₁₃H₁₉NO₂ (221.30)
   [M+H]+ = 222
   HPLC (Methode 4): Retentionszeit = 2.2 min
60b) 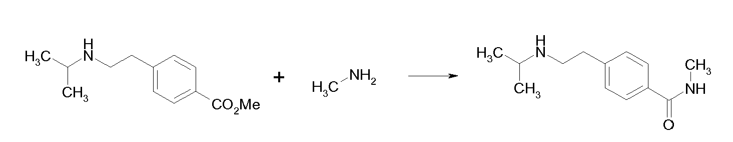 Eine Mischung aus 2.52 g (11.39 mMol) Produkt aus 60a, 11.40 ml (22.80 mMol) Methylamin 2 M in THF (Aldrich), 0.54 g (5.70 mMol) Magnesiumchlorid (Aldrich) und 100 ml THF wird 17 Stunden bei 120°C im Autoklaven gerührt. Die Reaktionsmischung wird filtriert, das Filtrat im Vakuum bis zur Trockene eingeengt. Das so erhaltene Rohprodukt wird durch Säulenchromatographie über Kieselgel (Elutionsmittel: Dichlormethan /
   Methanol / Ammoniak 9:1:0.1) gereinigt.
   C₁₃H₂₀N₂O (220.31)
   [M+H]+ = 221
   DC: Kieselgel, Dichlormethan / Methanol / Ammoniak 9:1:0.1, Rf-Wert = 0.21
60c) 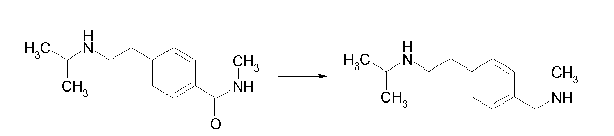 60c wird analog zu 38f aus 1.49 g (6.76 mMol) Produkt aus 60b und 10.00 ml (10.00 mMol) Lithiumaluminiumhydrid 1 M in THF (Aldrich) in 60 ml THF hergestellt.
   C₁₃H₂₂N₂ (206.33)
   [M+H]+ = 207
   DC: Kieselgel, Dichlormethan / Methanol / Ammoniak 9:1:0.1, Rf-Wert = 0.10
60d) 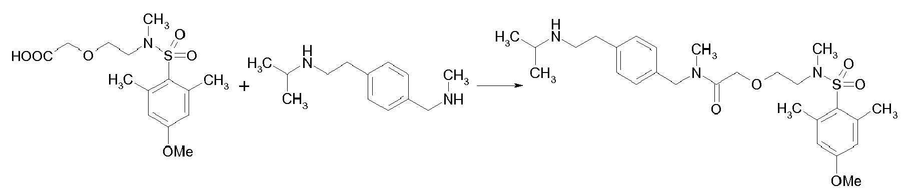 Beispiel 60 wird analog zu 1f aus 0.099 g (0.30 mMol) Produkt aus 53c, 0.062 g (0.30 mMol) Produkt aus 60c, 0.083 ml (0.60 mMol) Triethylamin und 0.12 g (0.36 mMol) TBTU in 8 ml THF hergestellt.
   C₂₇H₄₁N₃O₅S (519.70)
   [M+H]+ = 520
   HPLC (Methode 4): Retentionszeit = 3.2 min

### Beispiel 61

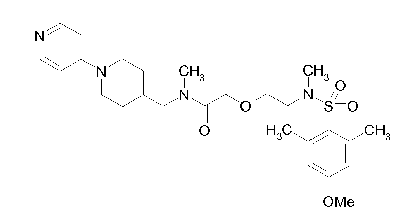

61a) 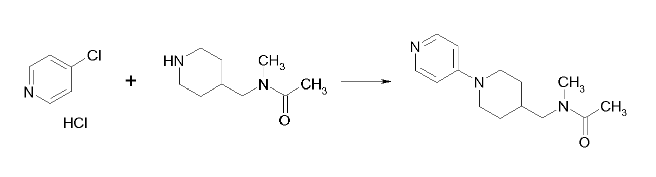 61 a wird analog zu 28c aus 1.00 g (6.67 mMol) 4-Chlorpyridin Hydrochlorid (Aldrich) und 2.55 g (15.00 mMol) N-Methyl-N-piperidin-4-ylmethyl-acetamid (DE 110635, Rhöne-Poulenc, 1961) in 1 ml Wasser hergestellt.
   C₁₄H₂₁N₃O (247.34)
   [M+H]+ = 248
61b) 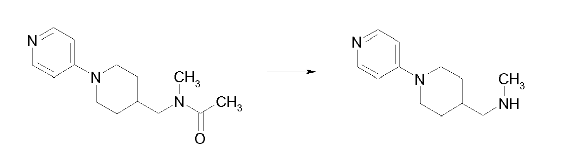 Eine Mischung aus 1.00 g (4.04 mMol) Produkt aus 61 a und 10 ml halbkonzentrierte HCl wird drei Tage unter Rückfluss erhitzt. Die Reaktionsmischung wird danach mit Wasser verdünnt, mit 20%iger Natronlauge alkalisch gestellt und mit Ethylacetat extrahiert. Die organischen Extrakte werden über Natriumsulfat getrocknet und im Vakuum bis zur Trockene eingeengt.
   C₁₂H₁₉N₃ (205.30)
   DC: Kieselgel, Dichlormethan / Ethanol 9:1, Rf-Wert = 0.2
61c) 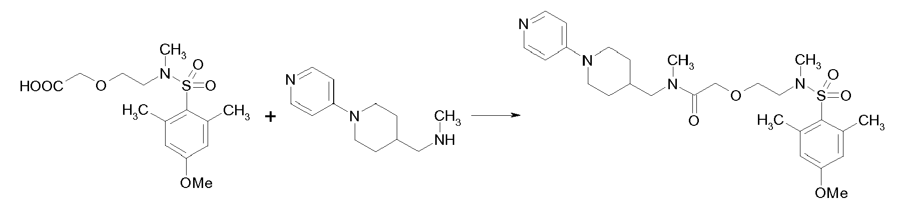 Beispiel 61 wird analog zu 1f aus 0.099 g (0.30 mMol) Produkt aus 53c, 0.062 g (0.30 mMol) Produkt aus 61 b, 0.083 ml (0.60 mMol) Triethylamin und 0.12 g (0.36 mMol) TBTU in 8 ml THF und 1 ml DMF hergestellt.
   C₂₆H₃₈N₄O₅S (518.67)
   [M+H]+ = 519
   HPLC (Methode 4): Retentionszeit = 3.2 min

### Beispiel 62

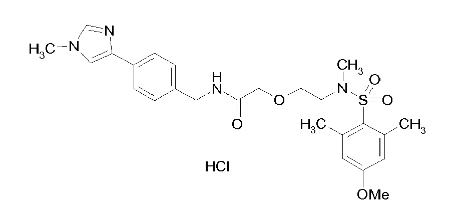

62a) 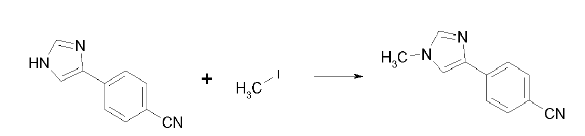 Eine Mischung aus 1.69 g (10.00 mMol) 4-(1 H-Imidazol-4-yl)-benzonitril (J. Am. Chem. Soc. 93, 1971, 4256-4263), 1.12 g (10.00 mMol) Kalium-tert-butylat und 25 ml DMSO wird erst 30 min bei Raumtemperatur gerührt, dann mit 0.62 ml (10.00 mMol) Methyliodid langsam versetzt und anschließend noch weitere 2.5 Stunden bei Raumtemperatur gerührt. Die Reaktionsmischung wird danach in Wasser gegeben, der entstandene Niederschlag wird abfiltriert und im Vakuum getrocknet. Das so erhaltene Rohprodukt wird durch Säulenchromatographie über Kieselgel (Elutionsmittel: Dichlormethan / Ethanol 19:1) gereinigt.
   C₁₁H₉N₃ (183.21)
   [M+H]+ = 184
   HPLC (Methode 4): Retentionszeit = 1.9 min
62b) 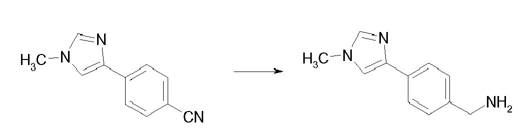 62b wird analog zu 34b aus 1.02 g (5.57 mMol) Produkt aus 62a, 0.20 g Raney-Nickel und 30 ml methanolischer Ammoniak-Lösung hergestellt.
   C₁₁H₁₃N₃ (187.24)
   DC: Kieselgel, Dichlormethan / Methanol / Ammoniak 9:1:0.1, Rf-Wert = 0.27
62c) 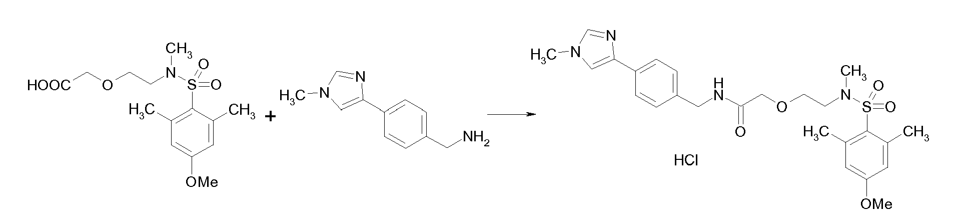 Beispiel 62 wird analog zu 1f aus 0.099 g (0.30 mMol) Produkt aus 53c, 0.056 g (0.30 mMol) Produkt aus 62b, 0.083 ml (0.60 mMol) Triethylamin und 0.12 g (0.36 mMol) TBTU in 7 ml THF und 1 ml DMF hergestellt.
   C₂₅H₃₂N₄O₅S x HCl (537.07)
   [M+H]+ = 501
   HPLC (Methode 4): Retentionszeit = 3.2 min

### Beispiel 63

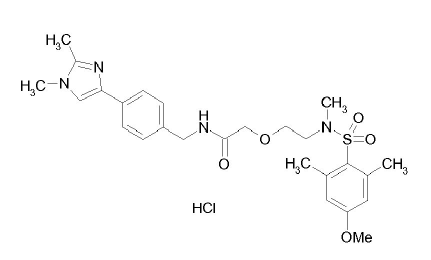

63a) 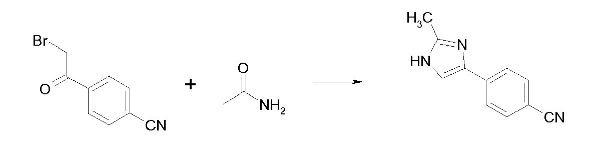 5.00 g (22.32 mMol) 2-Brom-4'-cyano-acetophenon (Aldrich) und 10.00 g (169.29 mMol) Acetamid (Merck) werden zusammen unter Rühren zwei Stunden auf 210°C erhitzt. Die Reaktionsmischung wird nach dem Abkühlen in Wasser eingerührt und mit 2 M HCl angesäuert. Der Niederschlag wird abfiltriert und verworfen. Das Filtrat wird mit konzentrierter Ammoniak-Lösung alkalisch gestellt, der Niederschlag abfiltriert und im Vakuum getrocknet. Das so erhaltene Rohprodukt wird durch Säulenchromatographie über Kieselgel (Elutionsmittel: Dichlormethan / Ethanol 9:1) gereinigt.
   C₁₁H₉N₃ (183.21)
   [M-H]- = 182
   HPLC (Methode 4): Retentionszeit = 1.9 min
63b) 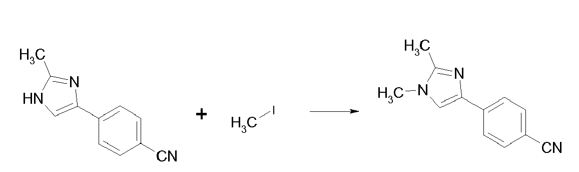 63b wird analog zu 62a aus 2.39 g (13.05 mMol) Produkt aus 63a, 1.46 g (13.05 mMol) Kalium-tert-butylat und 0.81 ml (13.05 mMol) Methyliodid in 50 ml DMSO hergestellt.
   C₁₂H₁₁N₃ (197.24)
   [M+H]+ = 198
   HPLC (Methode 4): Retentionszeit = 1.9 min
63c) 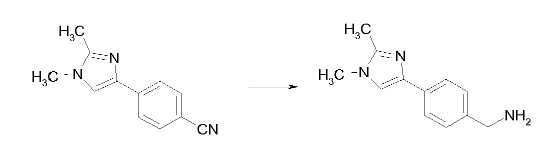 63c wird analog zu 34b aus 2.04 g (10.34 mMol) Produkt aus 63b, 0.40 g Raney-Nickel und 50 ml methanolischer Ammoniak-Lösung hergestellt.
   C₁₂H₁₅N₃ (201.27)
   DC: Kieselgel, Dichlormethan / Methanol / Ammoniak 9:1:0.1, Rf-Wert = 0.19
63d) 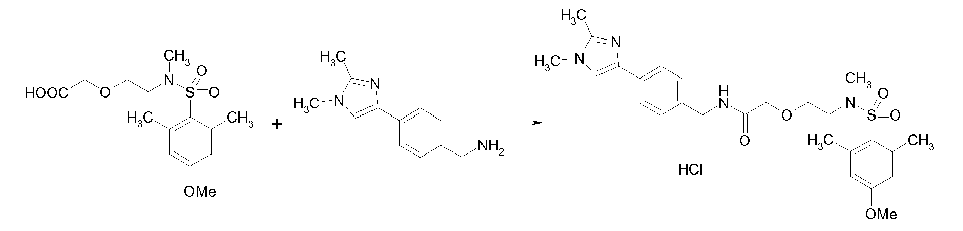 Beispiel 63 wird analog zu 1f aus 0.099 g (0.30 mMol) Produkt aus 53c, 0.060 g (0.30 mMol) Produkt aus 63c, 0.083 ml (0.60 mMol) Triethylamin und 0.12 g (0.36 mMol) TBTU in 7 ml THF und 1 ml DMF hergestellt.
   C₂₆H₃₄N₄O₅S x HCl (551.10)
   [M+H]+ = 515
   HPLC (Methode 4): Retentionszeit = 3.2 min

### Beispiel 64

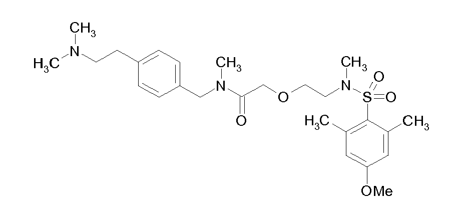

64a) 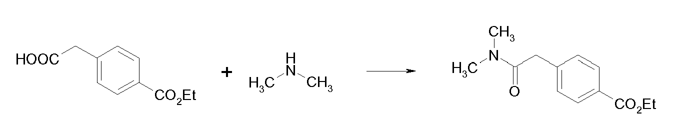 64a wird analog zu 1f aus 4.00 g (19.21 mMol) 4-Carboxymethyl-benzoesäureethylester (J. Med. Chem. 41, 1998, 5219-5246), 19.21 ml (38.42 mMol) Dimethylamin 2 M in THF (Aldrich), 5.36 ml (38.42 mMol) Triethylamin und 7.39 g (23.00 mMol) TBTU in 100 ml THF hergestellt.
   C₁₃H₁₇NO₃ (235.28)
   [M+H]+ = 236
   HPLC (Methode 4): Retentionszeit = 3.3 min
64b) 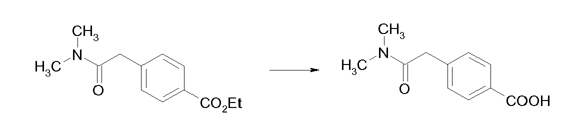 Eine Mischung aus 3.07 g (13.05 mMol) Produkt aus 64a, 9.75 ml (39.00 mMol) 4 M Natronlauge, 9.75 ml Wasser und 50 ml Ethanol wird über Nacht bei Raumtemperatur gerührt. Danach wird das Ethanol im Vakuum entfernt. Der wässrige Rückstand wird mit 4 M HCl sauer gestellt und mit Ethylacetat extrahiert. Die organischen Extrakte werden über Natriumsulfat getrocknet und im Vakuum bis zur Trockene eingeengt. Das so erhaltene Produkt wird mit Diethylether verrieben und getrocknet.
   C₁₁H₁₃NO₃ (207.23)
   [M+H]+ = 208
   HPLC (Methode 4): Retentionszeit = 2.3 min
64c) 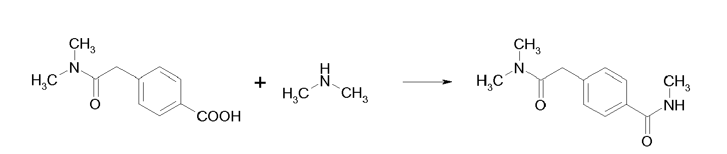 64c wird analog zu 1f aus 2.30 g (11.10 mMol) Produkt aus 64b, 11.10 ml (22.20 mMol) Dimethylamin 2 M in THF (Aldrich), 3.09 ml (22.20 mMol) Triethylamin und 4.28 g (13.32 mMol) TBTU in 70 ml THF hergestellt.
   C₁₂H₁₆N₂O₂ (220.27)
   [M+H]+ = 221
   DC: Kieselgel, Dichlormethan / Methanol / Ammoniak 9:1:0.1, Rf-Wert = 0.44
64d) 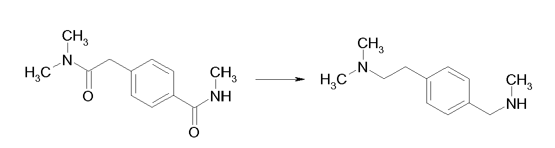 64d wird analog zu 38f aus 1.92 g (8.72 mMol) Produkt aus 64c und 40.00 ml (40.00 mMol) Lithiumaluminiumhydrid 1 M in THF (Aldrich) in 150 ml THF hergestellt.
   C₁₂H₂₀N₂ (192.30)
   [M+H]+ = 193
   DC: Kieselgel, Dichlormethan / Methanol / Ammoniak 9:1:0.1, Rf-Wert = 0.10
64e) 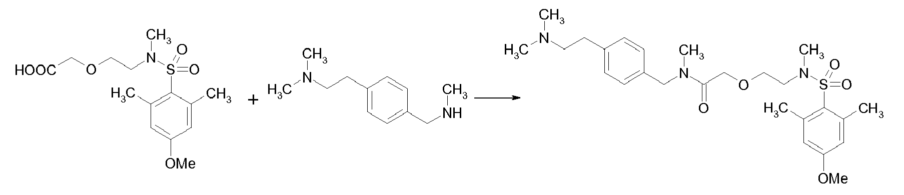 Beispiel 64 wird analog zu 1f aus 0.099 g (0.30 mMol) Produkt aus 53c, 0.058 g (0.30 mMol) Produkt aus 64d, 0.083 ml (0.60 mMol) Triethylamin und 0.12 g (0.36 mMol) TBTU in 7 ml THF und 1 ml DMF hergestellt.
   C₂₆H₃₉N₃O₅S (505.67)
   [M+H]+ = 508
   HPLC (Methode 4): Retentionszeit = 3.0 min

### Beispiel 65

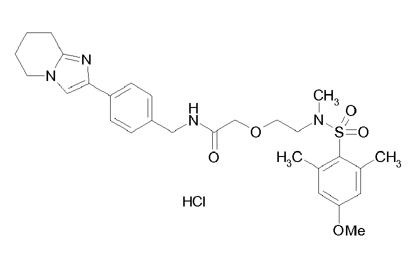

65a) 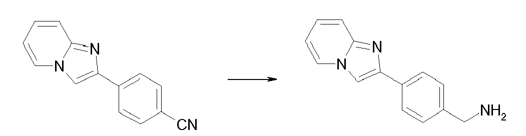 65a wird analog zu 34b aus 0.40 g (1.82 mMol) 4-Imidazo[1,2-a]pyridin-2-yl-benzonitril (J. Med. Chem. 41, 1998, 4317-4328), 0.10 g Raney-Nickel und 40 ml methanolischer Ammoniak-Lösung hergestellt.
   C₁₄H₁₃N₃ (223.27)
   [M+H]+ = 224
   DC: Kieselgel, Dichlormethan / Methanol 9:1, Rf-Wert = 0.06
65b) 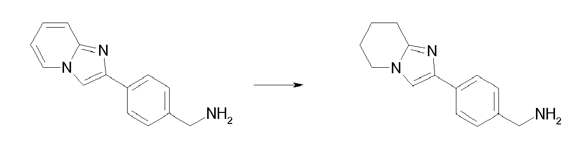 Eine Mischung aus 0.40 g (1.79 mMol) Produkt aus 65a, 0.05 g Platinoxid und 40 ml Methanol wird bei 50°C im Autoklaven hydriert. Der Katalysator wird abfiltriert, das Filtrat im Vakuum bis zur Trockene eingeengt.
   C₁₄H₁₇N₃ (227.30)
   [M+H]+ = 228
65c) 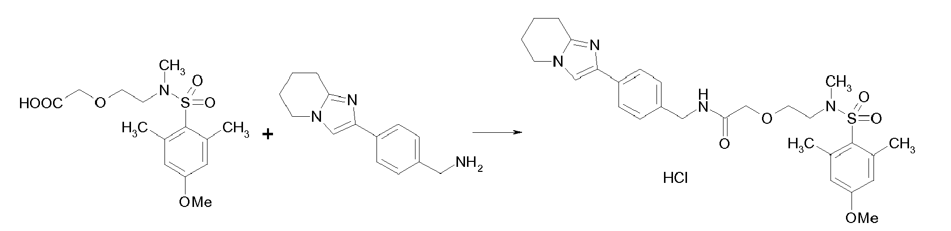 Beispiel 65 wird analog zu 1f aus 0.15 g (0.44 mMol) Produkt aus 53c, 0.10 g (0.44 mMol) Produkt aus 65b, 0.15 ml (1.09 mMol) Triethylamin und 0.16 g (0.48 mMol) TBTU in 30 ml THF und 10 ml DMF hergestellt.
   C₂₈H₃₆N₄O₅S x HCl (577.14)
   [M+H]+ = 541
   HPLC (Methode 5): Retentionszeit = 1.57 min

### Beispiel 66

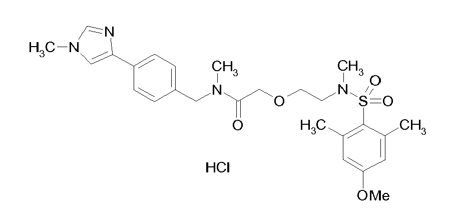

66a) 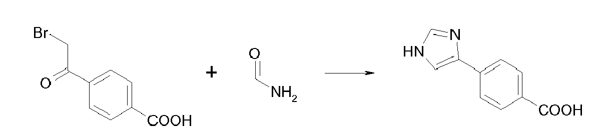 Eine Mischung aus 0.50 g (2.06 mMol) 4-Bromacetylbenzoesäure (Fluorochem) und 5 ml Formamid wird eine Stunde bei 150°C in der Mikrowelle gerührt. Nach dem Abkühlen wird das ausgefallene Produkt abfiltriert, mit Diethylether gewaschen und getrocknet.
   C₁₀H₈N₂O₂ (188.18)
   [M+H]+ = 189
66b) 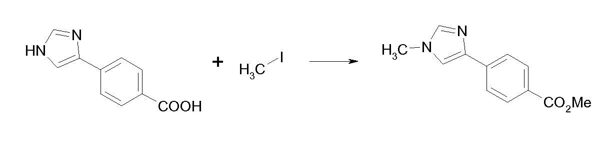 Eine Mischung aus 1.60 g (8.50 mMol) Produkt aus 66a, 5.40 g (38.04 mMol) Methyliodid, 7.00 g (25.33 mMol) Kaliumcarbonat und 30 ml DMF wird über Nacht bei Raumtemperatur gerührt. Die Reaktionsmischung wird filtriert, das Filtrat wird im Vakuum bis zur Trockene eingeengt. Das so erhaltene Rohprodukt wird durch Säulenchromatographie über Kieselgel (Elutionsmittel: Dichlormethan / Methanol 100:1 bis 75:1) gereinigt.
   C₁₂H₁₂N₂O₂ (216.24)
   [M+H]+ = 217
66c) 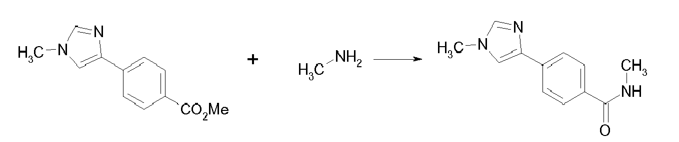 Eine Mischung aus 0.75 g (3.47 mMol) Produkt aus 66b und 20 ml Methylamin 33%ig in Ethanol (Fluka) über Nacht im Autoklaven auf 160°C erhitzt. Die Reaktionsmischung wird im Vakuum bis zur Trockene eingeengt. Das so erhaltene Rohprodukt wird durch Säulenchromatographie über Kieselgel (Elutionsmittel: Dichlormethan / Methanol 50:1 bis 25:1) gereinigt.
   C₁₂H₁₃N₃O (215.25)
   [M+H]+ = 216
66d) 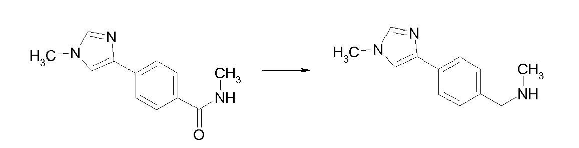 66d wird analog zu 38f aus 0.41 g (1.91 mMol) Produkt aus 66c und 3.00 ml (3.00 mMol) Lithiumaluminiumhydrid 1 M in THF (Aldrich) in 70 ml THF hergestellt.
   C₁₂H₁₅N₃ (201.27)
   [M+H]+ = 202
   DC: Kieselgel, Dichlormethan / Methanol / Ammoniak 8:2:0.01, Rf-Wert = 0.16
66e) 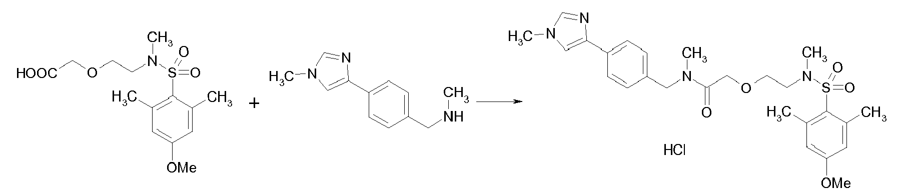 Beispiel 66 wird analog zu 1f aus 0.15 g (0.45 mMol) Produkt aus 53c, 0.09g (0.45 mMol) Produkt aus 66d, 0.11 ml (1.09 mMol) Triethylamin und 0.16 g (0.50 mMol) TBTU in 30 ml THF und 5 ml DMF hergestellt.
   C₂₆H₃₄N₄O₅S x HCl (551.10)
   [M+H]+ = 515
   DC: Kieselgel, Dichlormethan / Methanol 9:1, Rf-Wert = 0.52

### Beispiel 67

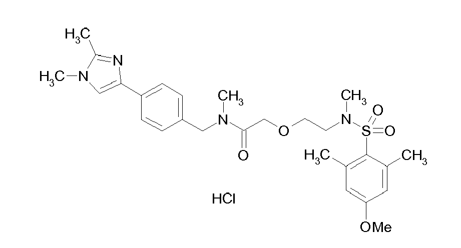

67a) 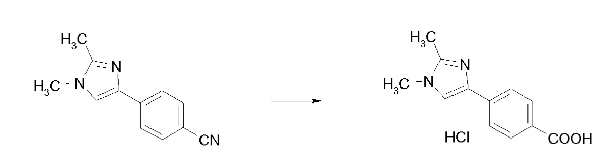 Eine Mischung aus 1.21 g (6.14 mMol) Produkt aus 63b, 20 ml 20%iger Natronlauge und 40 ml Ethanol wird über Nacht unter Rückfluss gerührt. Das Ethanol wird im Vakuum entfernt. Der Rückstand wird mit Wasser verdünnt und mit 4 M HCl sauer gestellt. Das ausgefallene Produkt wird abfiltriert und im Umlufttrockenschrank bei 50°C getrocknet.
   C₁₂H₁₂N₂O₂ (252.70)
   [M+H]+ = 217
   HPLC (Methode 4): Retentionszeit = 1.7 min
67b) 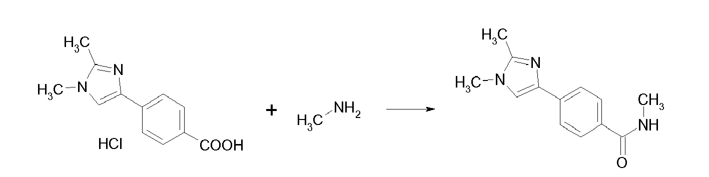 67b wird analog zu 1f aus 1.55 g (6.13 mMol) Produkt aus 67a, 4.60 ml (9.20 mMol) Methylamin 2 M in THF (Aldrich), 1.71 ml (12.30 mMol) Triethylamin und 2.38 g (7.40 mMol) TBTU in 50 ml THF hergestellt.
   C₁₃H₁₅N₃O (229.28)
   [M+H]+ = 230
   DC: Kieselgel, Dichlormethan / Methanol 9:1, Rf-Wert = 0.47
67c) 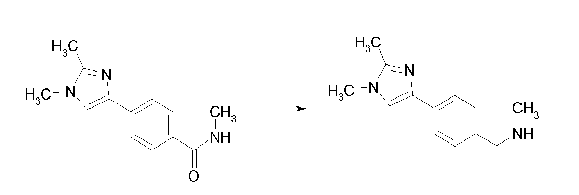 67c wird analog zu 38f aus 1.33 g (5.80 mMol) Produkt aus 67b und 15.00 ml (15.00 mMol) Lithiumaluminiumhydrid 1 M in THF (Aldrich) in 80 ml THF hergestellt.
   C₁₃H₁₇N₃ (215.29)
   [M+H]+ = 216
   DC: Kieselgel, Dichlormethan / Methanol / Ammoniak 9:1:0.1, Rf-Wert = 0.29
67d) 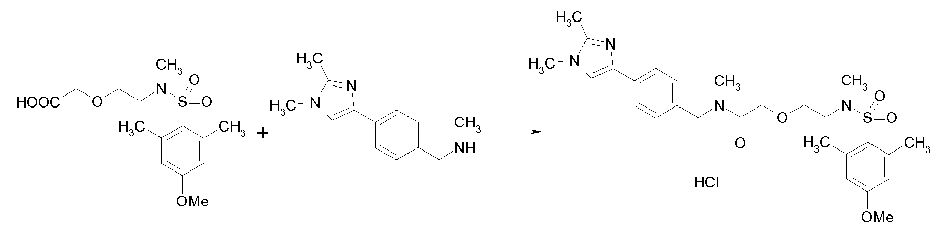 Beispiel 67 wird analog zu 1f aus 0.099 g (0.30 mMol) Produkt aus 53c, 0.065 g (0.30 mMol) Produkt aus 67c, 0.083 ml (0.60 mMol) Triethylamin und 0.12 g (0.36 mMol) TBTU in 7 ml THF und 1 ml DMF hergestellt.
   C₂₇H₃₆N₄O₅S x HCl (565.13)
   [M+H]+ = 529
   HPLC (Methode 4): Retentionszeit = 3.1 min

### Beispiel 68

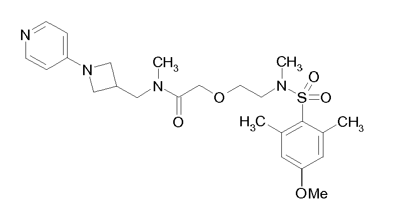

68a) 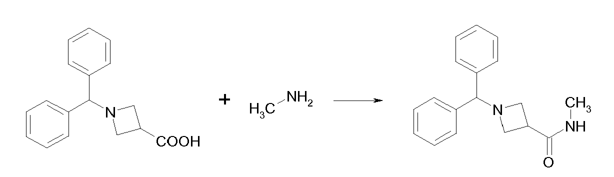 68a wird analog zu 1f aus 5.39 g (20.16 mMol) 1-Benzhydryl-azetidine-3-carbonsäure (Acros), 15 ml (30.00 mMol) Methylamin 2 M in THF (Aldrich), 5.58 ml (40.00 mMol) Triethylamin und 7.71 g (24.00 mMol) TBTU in 150 ml THF hergestellt.
   C₁₈H₂₀N₂O (280.36)
   HPLC (Methode 4): Retentionszeit = 2.5 min
68b) 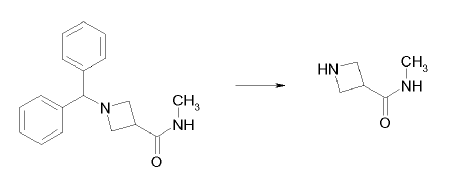 Eine Mischung aus 5.32 g (18.98 mMol) Produkt aus 68a, 0.50 g Palladium auf Kohle (10%) und 100 ml Methanol wird 24 Stunden bei Raumtemperatur im Autoklaven hydriert. Der Katalysator wird abfiltriert, das Filtrat wird im Vakuum bis zur Trockene eingeengt. Das so erhaltene Rohprodukt wird durch Säulenchromatographie über Kieselgel (Elutionsmittel: Dichlormethan / Methanol / Ammoniak 70:30:3) gereinigt.
   C₅H₁₀N₂O (114.15)
   DC: Kieselgel, Dichlormethan / Methanol / Ammoniak 7:3:0.3, Rf-Wert = 0.17
68c) 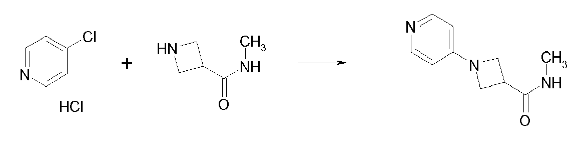 68c wird analog zu 28c aus 1.31 g (8.76 mMol) 4-Chlorpyridin Hydrochlorid (Aldrich), 1.00 g (8.76 mMol) Produkt aus 68b und 2.40 ml (17.22 mMol) Triethylamin in 5 ml Ethanol hergestellt.
   C₁₀H₁₃N₃O (191.23)
   [M+H]+ = 192
   DC: Kieselgel, Dichlormethan / Methanol / Ammoniak 9:1:0.1, Rf-Wert = 0.25
68d) 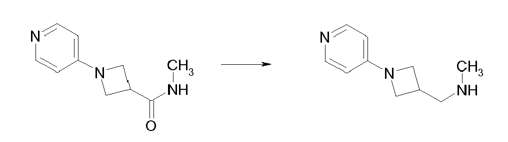 68d wird analog zu 38f aus 0.46 g (2.41 mMol) Produkt aus 68c und 5.00 ml (5.00 mMol) Lithiumaluminiumhydrid 1 M in THF (Aldrich) in 20 ml THF hergestellt.
   C₁₀H₁₅N₃ (177.25)
   [M+H]+ = 178
   DC: Kieselgel, Dichlormethan / Methanol / Ammoniak 8:2:0.2, Rf-Wert = 0.40
68e) 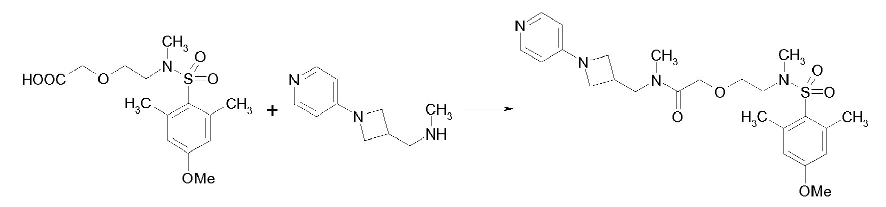 Beispiel 68 wird analog zu 1f aus 0.083 g (0.25 mMol) Produkt aus 53c, 0.044 g (0.25 mMol) Produkt aus 68d, 0.070 ml (0.50 mMol) Triethylamin und 0.096 g (0.30 mMol) TBTU in 7 ml THF und 1 ml DMF hergestellt.
   C₂₄H₃₄N₄O₅S (490.62)
   [M+H]+ = 491
   HPLC (Methode 4): Retentionszeit = 2.9 min

### Beispiel 69

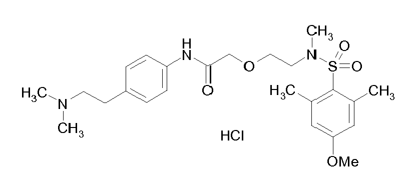

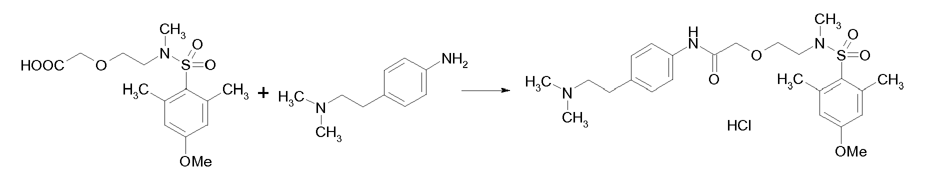

Beispiel 69 wird analog zu 1f aus 0.083 g (0.25 mMol) Produkt aus 53c, 0.041 g (0.25 mMol) 4-(2-Dimethylamino-ethyl)-phenylamin (JW Pharmlab), 0.070 ml (0.50 mMol) Triethylamin und 0.096 g (0.30 mMol) TBTU in 7 ml THF und 1 ml DMF hergestellt. C₂₄H₃₅N₃O₅S x HCl (514.08)
[M+H]+ = 478
HPLC (Methode 4): Retentionszeit = 3.1 min

### Beispiel 70

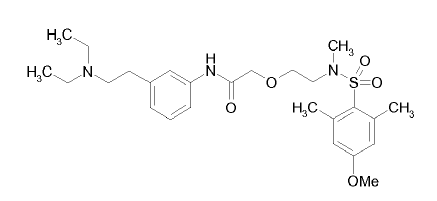

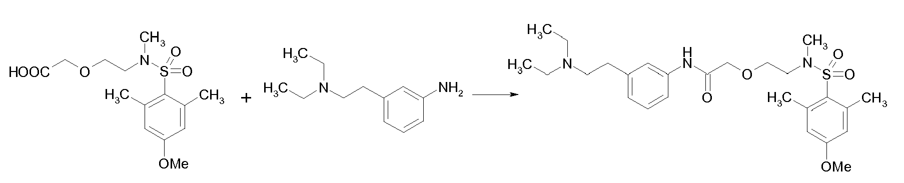

Beispiel 70 wird analog zu 1f aus 0.083 g (0.25 mMol) Produkt aus 53c, 0.048 g (0.25 mMol) 3-(2-Diethylamino-ethyl)-phenylamin (analog J. Med. Chem. 28, 1985, 1533-1536), 0.070 ml (0.50 mMol) Triethylamin und 0.096 g (0.30 mMol) TBTU in 7 ml THF und 1 ml DMF hergestellt.
C₂₆H₃₉N₃O₅S (505.67)
[M+H]+ = 506
HPLC (Methode 4): Retentionszeit = 3.4 min

### Beispiel 71

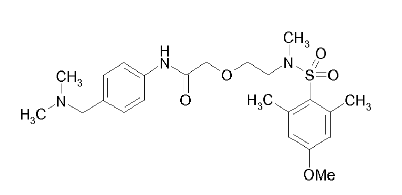

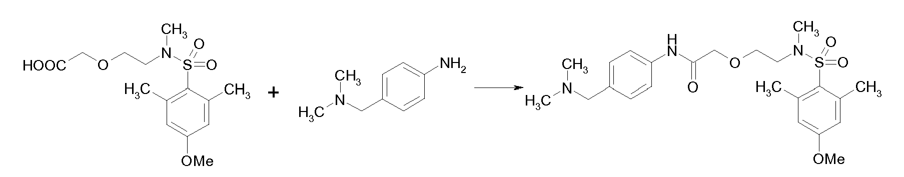

Beispiel 71 wird analog zu 1f aus 0.083 g (0.25 mMol) Produkt aus 53c, 0.038 g (0.25 mMol) 4-Dimethylaminomethyl-phenylamin (J. Chem. Soc. 1935, 871), 0.070 ml (0.50 mMol) Triethylamin und 0.096 g (0.30 mMol) TBTU in 7 ml THF und 1 ml DMF hergestellt.
C₂₃H₃₃N₃O₅S (463.59)
[M+H]+ = 464
HPLC (Methode 4): Retentionszeit = 3.1 min

### Beispiel 72

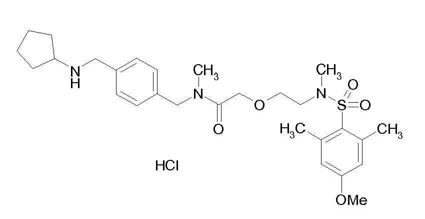

72a) 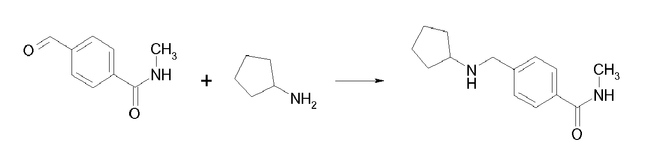 72a wird analog zu 60a aus 0.82 g (5.00 mMol) 4-Formyl-benzoesäuremethylamid (EMKA), 0.50 ml (5.00 mMol) Cyclopentylamin (Aldrich), 0.37 ml (6.50 mMol) Essigsäure und 1.59 g (7.50 mMol) Natriumtriacetoxyborhydrid in 30 ml THF hergestellt.
   C₁₄H₂₀N₂O (232.32)
   DC: Kieselgel, Dichlormethan / Ethanol 9:1, Rf-Wert = 0.15
72b) 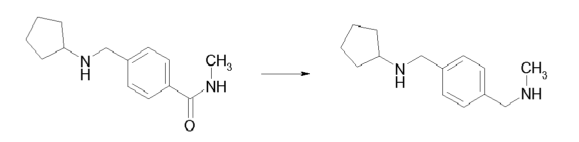 72b wird analog zu 38f aus 1.00 g (4.30 mMol) Produkt aus 72a und 9.00 ml (9.00 mMol) Lithiumaluminiumhydrid 1 M in THF (Aldrich) in 40 ml THF hergestellt.
   C₁₄H₂₂N₂ (218.34)
   [M+H]+ = 219
   DC: Kieselgel, Dichlormethan / Ethanol 9:1, Rf-Wert = 0.2
72c) 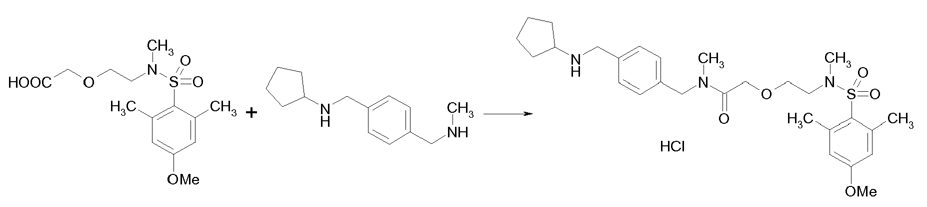 Beispiel 72 wird analog zu 1f aus 0.20 g (0.60 mMol) Produkt aus 53c, 0.13 g (0.60 mMol) Produkt aus 72b, 0.21 ml (1.50 mMol) Triethylamin und 0.23 g (0.72 mMol) TBTU in 10 ml THF hergestellt.
   C₂₈H₄₁N₃O₅S x HCl (568.17)
   [M+H]+ = 532
   HPLC (Methode 5): Retentionszeit = 1.60 min

### Beispiel 73

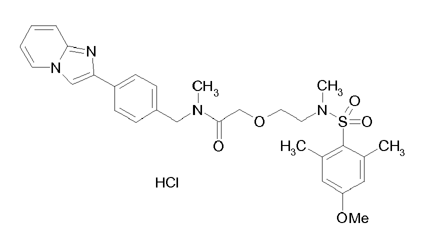

73a) 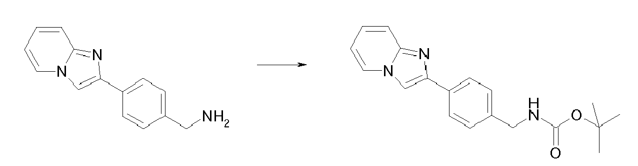 Eine Mischung aus 2.60 g (11.65 mMol) Produkt aus 65a, 2.55 g (11.68 mMol) Boc-Anhydrid und 100 ml DMF wird zwei Stunden bei Raumtemperatur gerührt. Danach gibt man langsam 100 ml Wasser hinzu. Das ausgefallene Produkt wird abfiltriert, mit Wasser und
   Petrolether gewaschen und getrocknet.
   C₁₉H₂₁N₃O₂ (323.39)
   [M+H]+ = 324
   DC: Kieselgel, Dichlormethan / Methanol 9:1, Rf-Wert = 0.78
73b) 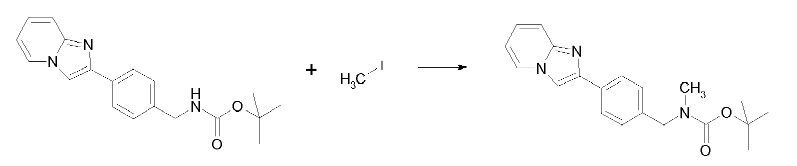 73b wird analog zu 62a aus 1.00 g (3.09 mMol) Produkt aus 73a, 0.70 g (6.24 mMol) Kalium-tert-butylat und 0.98 ml (6.90 mMol) Methyliodid in 30 ml DMSO hergestellt.
   C₁₇H₁₇N₃O₂ (295.34)
   [M+H]+ = 296
73c) 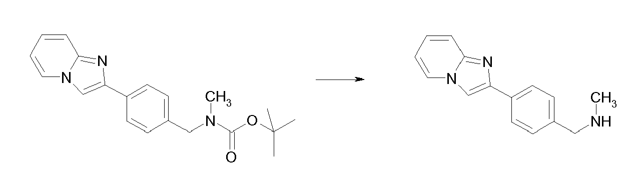 Eine Mischung aus 0.59 g (1.75 mMol) Produkt aus 73b, 2 ml TFA und 30 ml Dichlormethan wird drei Stunden bei Raumtemperatur gerührt. Die Reaktionsmischung wird danach mit Wasser gewaschen, über Natriumsulfat getrocknet und im Vakuum bis zur Trockene eingeengt.
   C₁₅H₁₅N₃ (237.30)
   [M+H]+ = 238
73d) 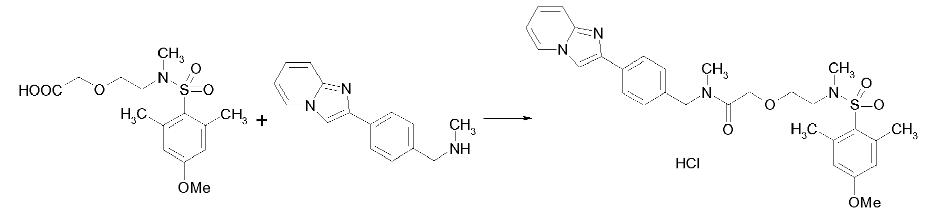 Beispiel 73 wird analog zu 1f aus 0.14 g (0.42 mMol) Produkt aus 53c, 0.10 g (0.42 mMol) Produkt aus 73c, 0.10 ml (0.99 mMol) Triethylamin und 0.15 g (0.46 mMol) TBTU in 30 ml THF und 5 ml DMF hergestellt.
   C₂₉H₃₄N₄O₅S x HCl (587.13)
   [M+H]+ = 551
   HPLC (Methode 5): Retentionszeit = 1.58 min

### Beispiel 74

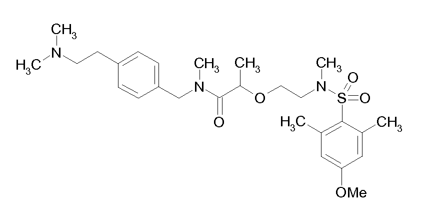

74a) 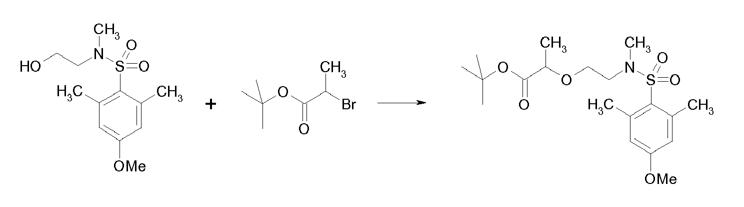 74a wird analog zu 53b aus 4.08 g (14.93 mMol) Produkt aus 53a, 4.68 g (22.39 mMol) 2-Brompropionsäure-tert-butylester (TCI), 1.38 g (4.98 mMol) Tetrabutylammoniumchlorid (Fluka) und 70 ml 35%iger Natronlauge in 70 ml Toluol hergestellt.
   C₁₉H₃₁NO₆S (401.52)
   [M+H]+ = 402
   DC: Kieselgel, Petrolether / Ethylacetat 7:3, Rf-Wert = 0.69
74b) 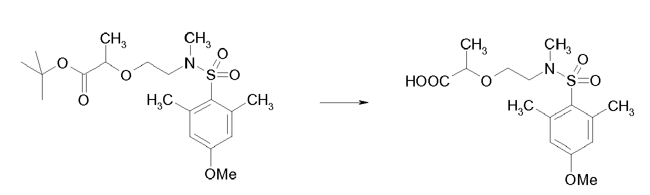 74b wird analog zu 53c aus 5.12 g (12.75 mMol) Produkt aus 74a und 5.89 ml TFA in 80 ml Dichlormethan hergestellt.
   C₁₅H₂₃NO₆S (345.41)
   [M+H]+ = 346
   DC: Kieselgel, Dichlormethan / Ethanol 19:1, Rf-Wert = 0.25
74c) 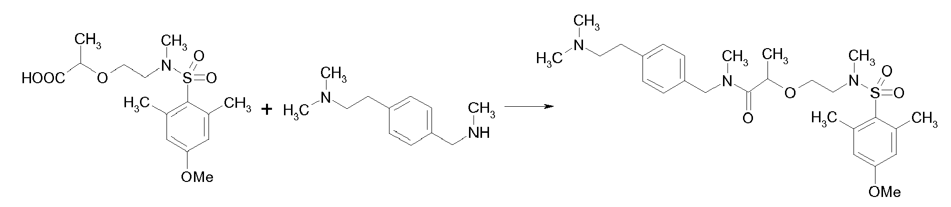 Beispiel 74 wird analog zu 1f aus 0.10 g (0.30 mMol) Produkt aus 74b, 0.058 g (0.30 mMol) Produkt aus 64d, 0.084 ml (0.60 mMol) Triethylamin und 0.12 g (0.36 mMol) TBTU in 7 ml THF und 1 ml DMF hergestellt.
   C₂₇H₄₁N₃O₅S (519.70)
   [M+H]+ = 520
   HPLC (Methode 4): Retentionszeit = 3.1 min

### Beispiel 75

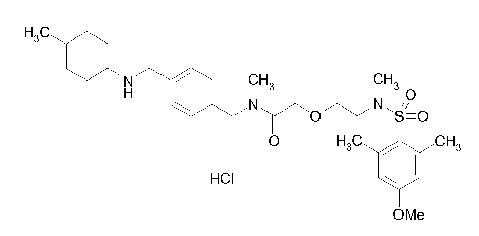

75a) 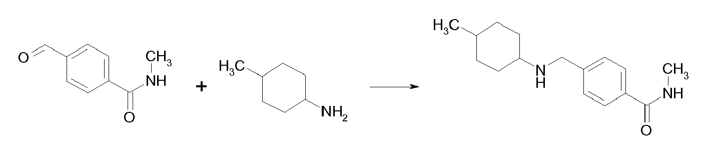 75a wird analog zu 60a aus 0.82 g (5.00 mMol) 4-Formyl-benzoesäuremethylamid (EMKA), 0.67 ml (5.00 mMol) 4-Methylcyclohexylamin cis/trans-Gemisch (Acros), 0.37 ml (6.50 mMol) Essigsäure und 1.59 g (7.50 mMol) Natriumtriacetoxyborhydrid in 30 ml THF hergestellt.
   C₁₆H₂₄N₂O (260.37)
   DC: Kieselgel, Dichlormethan / Ethanol 9:1, Rf-Wert = 0.25
75b) 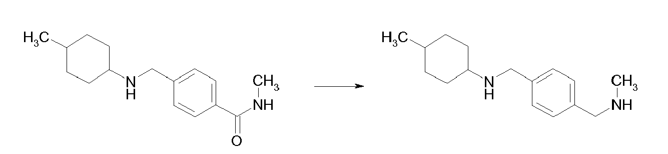 75b wird analog zu 38f aus 1.30 g (4.99 mMol) Produkt aus 75a und 10.00 ml (10.00 mMol) Lithiumaluminiumhydrid 1 M in THF (Aldrich) in 50 ml THF hergestellt.
   C₁₆H₂₆N₂ (246.39)
   DC: Kieselgel, Dichlormethan / Ethanol 9:1, Rf-Wert = 0.2
75c) 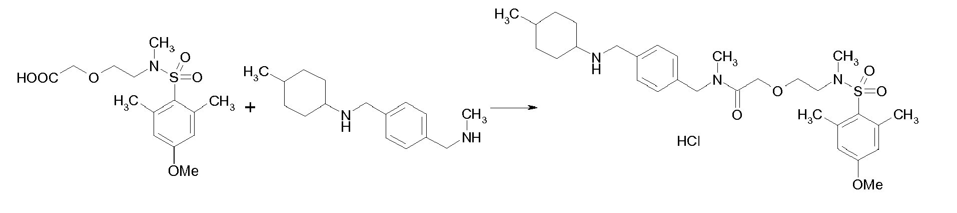 Beispiel 75 wird analog zu 1f aus 0.20 g (0.60 mMol) Produkt aus 53c, 0.15 g (0.60 mMol) Produkt aus 75b, 0.21 ml (1.50 mMol) Triethylamin und 0.23 g (0.72 mMol) TBTU in 10 ml THF hergestellt.
   C₃₀H₄₅N₃O₅S x HCl (596.22)
   [M+H]+ = 560
   HPLC (Methode 5): Retentionszeit = 1.65 min

### Beispiel 76

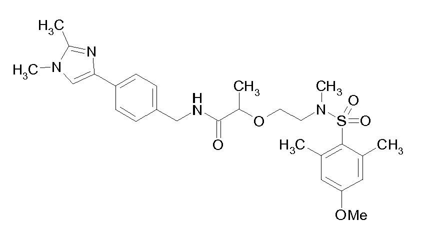

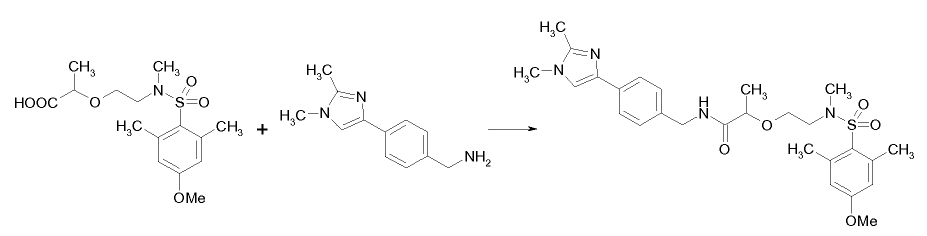

Beispiel 76 wird analog zu 1f aus 0.10 g (0.30 mMol) Produkt aus 74b, 0.060 g (0.30 mMol) Produkt aus 63c, 0.084 ml (0.60 mMol) Triethylamin und 0.12 g (0.36 mMol) TBTU in 7 ml THF und 1 ml DMF hergestellt.
C₂₇H₃₆N₄O₅S (528.66)
[M+H]+ = 529
HPLC (Methode 4): Retentionszeit = 3.2 min

### Beispiel 77

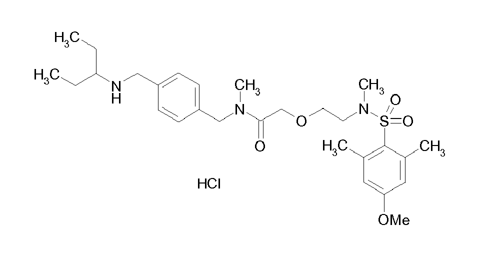

77a) 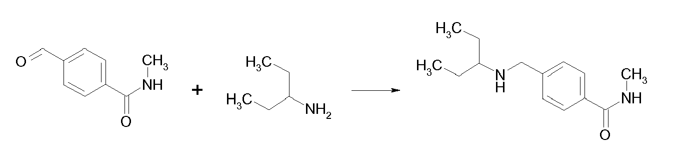 77a wird analog zu 60a aus 0.82 g (5.00 mMol) 4-Formyl-benzoesäuremethylamid (EMKA), 0.58 ml (5.00 mMol) 3-Pentylamin (Aldrich), 0.37 ml (6.50 mMol) Essigsäure und 1.59 g (7.50 mMol) Natriumtriacetoxyborhydrid in 30 ml THF hergestellt.
   C₁₄H₂₂N₂O (234.34)
   [M+H]+ = 235
   DC: Kieselgel, Dichlormethan / Ethanol 9:1, Rf-Wert = 0.4
77b) 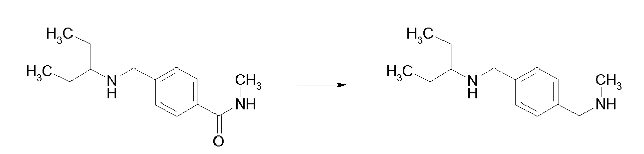 77b wird analog zu 38f aus 1.10 g (4.69 mMol) Produkt aus 77a und 9.40 ml (9.40 mMol) Lithiumaluminiumhydrid 1 M in THF (Aldrich) in 40 ml THF hergestellt.
   C₁₄H₂₄N₂ (220.35)
   DC: Kieselgel, Dichlormethan / Ethanol 9:1, Rf-Wert = 0.1
77c) 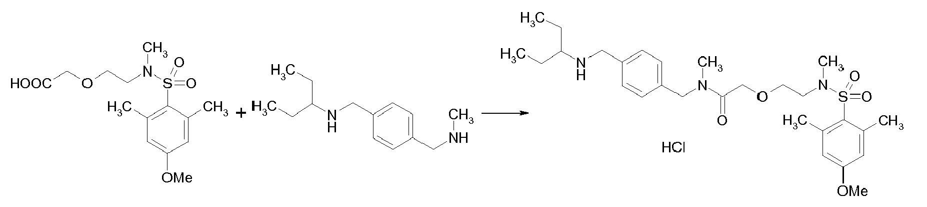 Beispiel 77 wird analog zu 1f aus 0.20 g (0.60 mMol) Produkt aus 53c, 0.13 g (0.60 mMol) Produkt aus 77b, 0.21 ml (1.50 mMol) Triethylamin und 0.23 g (0.72 mMol) TBTU in 10 ml THF hergestellt.
   C₂₈H₄₃N₃O₅S x HCl (570.18)
   [M+H]+ = 534
   DC: Kieselgel, Dichlormethan / Ethanol 9:1, Rf-Wert = 0.45

### Beispiel 78

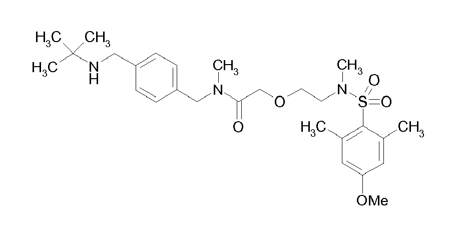

78a) 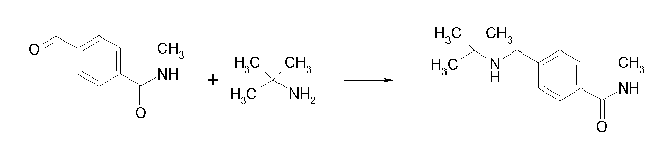 78a wird analog zu 60a aus 0.82 g (5.00 mMol) 4-Formyl-benzoesäuremethylamid (EMKA), 0.53 ml (5.00 mMol) tert-Butylamin (Fluka), 0.37 ml (6.50 mMol) Essigsäure und 1.59 g (7.50 mMol) Natriumtriacetoxyborhydrid in 30 ml THF hergestellt.
   C₁₃H₂₀N₂O (220.31)
   [M+H]+ = 221
   DC: Kieselgel, Dichlormethan / Ethanol 9:1, Rf-Wert = 0.15
78b) 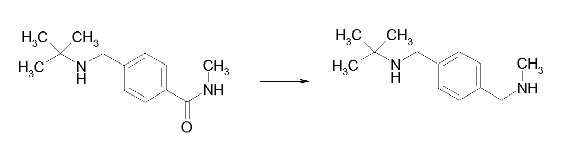 78b wird analog zu 38f aus 1.00 g (4.54 mMol) Produkt aus 78a und 9.10 ml (9.10 mMol) Lithiumaluminiumhydrid 1 M in THF (Aldrich) in 40 ml THF hergestellt.
   C₁₃H₂₂N₂ (206.33)
78c) 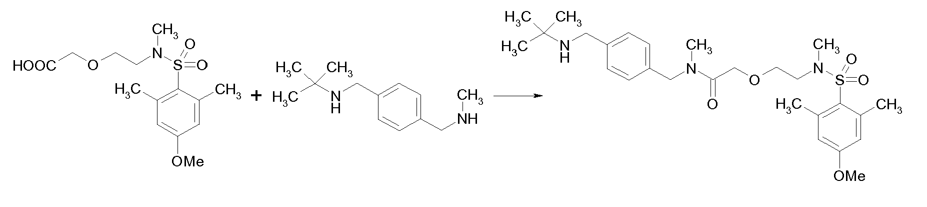 Beispiel 78 wird analog zu 1f aus 0.20 g (0.60 mMol) Produkt aus 53c, 0.12 g (0.60 mMol) Produkt aus 78b, 0.21 ml (1.50 mMol) Triethylamin und 0.23 g (0.72 mMol) TBTU in 10 ml THF hergestellt.
   C₂₇H₄₁N₃O₅S (519.70)
   [M+H]+ = 520
   HPLC (Methode 5): Retentionszeit = 1.58 min

### Beispiel 79

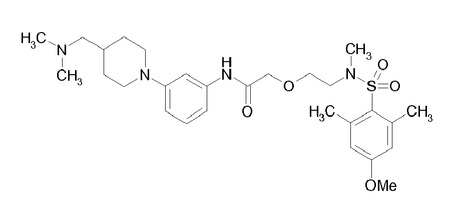

79a) 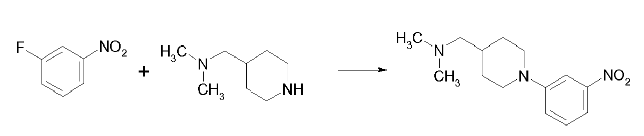 Eine Mischung aus 0.50 ml (4.70 mMol) 1-Fluor-3-nitrobenzol (Fluka), 1.34 g (9.39 mMol) 4-(N,N-Dimethylaminomethyl)-piperidin (Eur. J. Med.Chem. Chim. Ther. 37, 2002, 487-502), 0.65 g (4.70 mMol) Kaliumcarbonat und 6.6 ml DMSO wird fünf Tage bei 110°C gerührt. Die Reaktionsmischung wird danach auf Eis gegossen, der entstandene Niederschlag wird abfiltriert. Das so erhaltene Produkt wird über Nacht im Vakuum-Exsikkator getrocknet.
   C₁₄H₂₁N₃O₂ (263.34)
   [M+H]+ = 264
   DC: Kieselgel, Dichlormethan / Methanol 9:1, Rf-Wert = 0.20
79b) 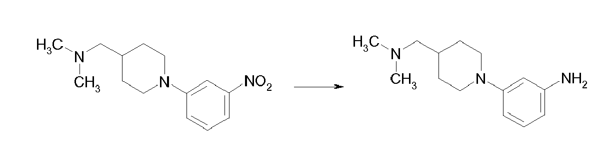 Eine Mischung aus 0.95 ml (3.60 mMol) Produkt aus 79a, 0.095 g Palladium auf Kohle (5%) und 72 ml Ethanol wird bei Raumtemperatur im Autoklaven hydriert. Der Katalysator wird abfiltriert, das Filtrat wird im Vakuum bis zur Trockene eingeengt.
   C₁₄H₂₃N₃ (233.35)
   [M+H]+ = 234
79c) 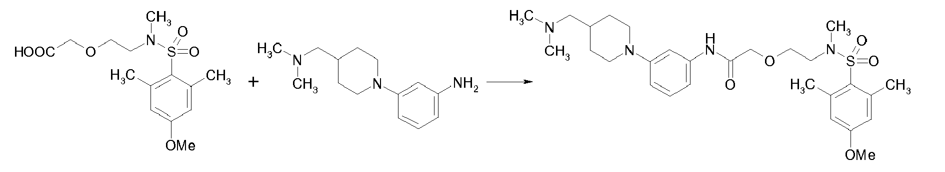 Beispiel 79 wird analog zu 1f aus 0.15 g (0.46 mMol) Produkt aus 53c, 0.11 g (0.46 mMol) Produkt aus 79b, 0.084 ml (0.60 mMol) Triethylamin und 0.18 g (0.56 mMol) TBTU in 3 ml DMF hergestellt.
   C₂₈H₄₂N₄O₅S (546.72)
   [M+H]+ = 547
   HPLC (Methode 5): Retentionszeit = 1.49 min

### Beispiel 80

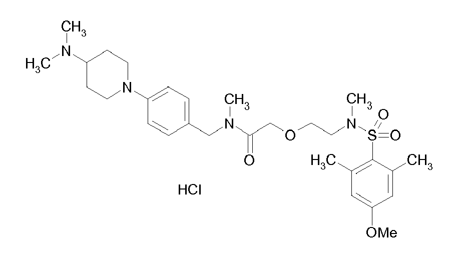

80a) 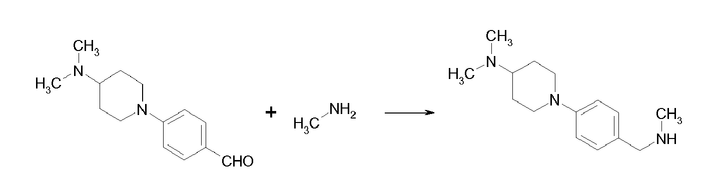 80a wird analog zu 33a aus 0.70 g (3.01 mMol) 4-(4-Dimethylamino-piperidin-1-yl)-benzaldehyd (Tetrahedron 57, 2001, 4781-4785), 3.00 ml (31.88 mMol) Methylamin 33% in Ethanol (Aldrich), 0.20 g Raney-Nickel in 25 ml Ethanol hergestellt.
   C₁₅H₂₅N₃ (247.38)
   [M+H]+ = 248
   DC: Kieselgel, Dichlormethan / Methanol / Ammoniak 9:1:0.1, Rf-Wert = 0.16
80b) 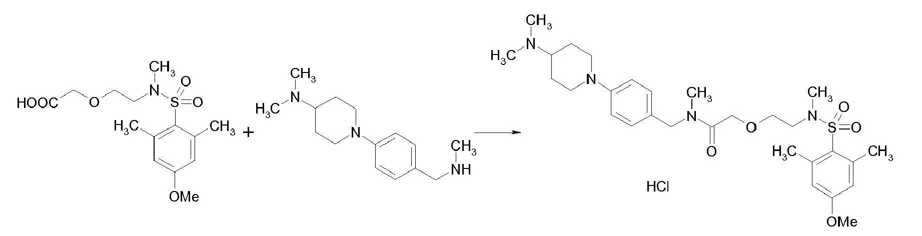 Beispiel 80 wird analog zu 1f aus 0.15 g (0.45 mMol) Produkt aus 53c, 0.11 g (0.45 mMol) Produkt aus 80a, 0.13 ml (0.90 mMol) Triethylamin und 0.17 g (0.54 mMol) TBTU in 5 ml
   DMF hergestellt.
   C₂₉H₄₄N₄O₅S x HCl (597.21)
   [M+H]+ = 561
   HPLC (Methode 1): Retentionszeit = 2.33 min

### Beispiel 81

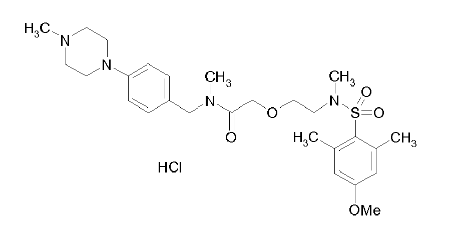

81a) 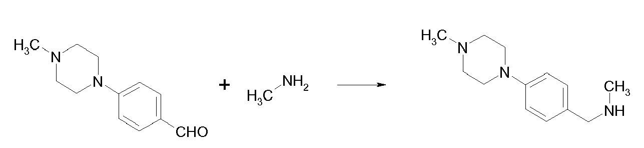 81 a wird analog zu 33a aus 1.00 g (4.90 mMol) 4-(4-Methyl-piperazin-1-yl)-benzaldehyd (Chem. Pharm Bull. 45, 1997, 1458-1469), 4.00 ml (42.50 mMol) Methylamin 33% in Ethanol (Aldrich), 0.20 g Raney-Nickel in 30 ml Ethanol hergestellt.
   C₁₃H₂₁N₃ (219.33)
   [M+H]+ = 220
   DC: Kieselgel, Dichlormethan / Methanol / Ammoniak 9:1:0.1, Rf-Wert = 0.15
81b) 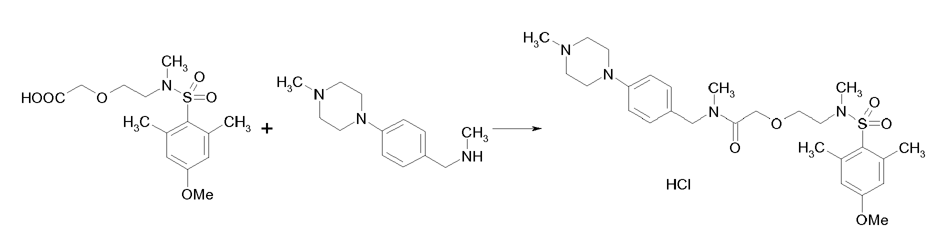 Beispiel 81 wird analog zu 1f aus 0.15 g (0.45 mMol) Produkt aus 53c, 0.099 g (0.45 mMol) Produkt aus 81a, 0.13 ml (0.90 mMol) Triethylamin und 0.17 g (0.54 mMol) TBTU in 5 ml DMF hergestellt.
   C₂₇H₄₀N₄O₅S x HCl (569.16)
   [M+H]+ = 533
   HPLC (Methode 1): Retentionszeit = 2.28 min

### Beispiel 82

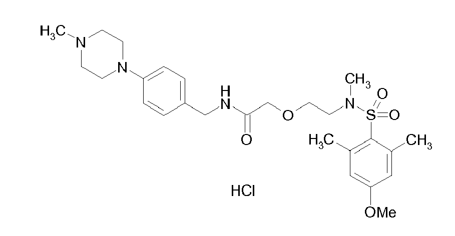

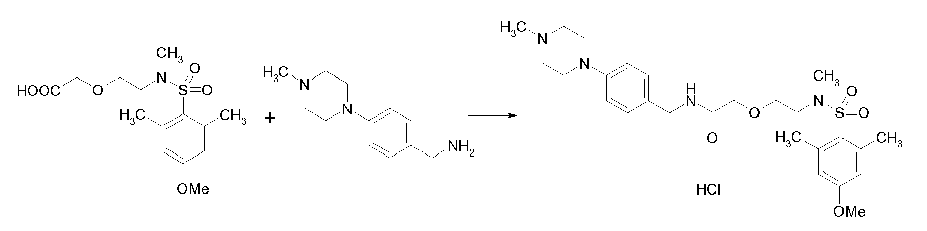

Beispiel 82 wird analog zu 1f aus 0.15 g (0.45 mMol) Produkt aus 53c, 0.092 g (0.45 mMol) Produkt aus 19b, 0.13 ml (0.90 mMol) Triethylamin und 0.17 g (0.54 mMol) TBTU in 5 ml DMF hergestellt.
C₂₆H₃₈N₄O₅S x HCl (555.13)
[M+H]+ = 519
HPLC (Methode 1): Retentionszeit = 2.29 min

### Beispiel 83

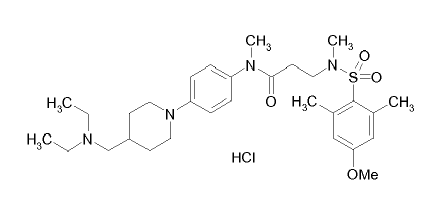

83a) 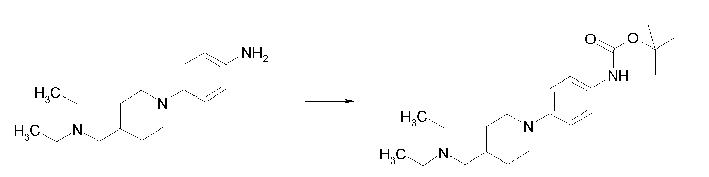 83a wird analog zu 51 a aus 1.35 g (5.16 mMol) Produkt aus 41 b, 1.24 g (5.68 mMol) Boc-Anhydrid und 0.80 ml (5.68 mMol) Triethylamin in 50 ml Dichlormethan hergestellt.
   C₂₁H₃₅N₃O₂ (361.52)
   [M+H]+ = 362
   DC: Kieselgel, Dichlormethan / Methanol / Ammoniak 9:1:0.1, Rf-Wert = 0.42
83b) 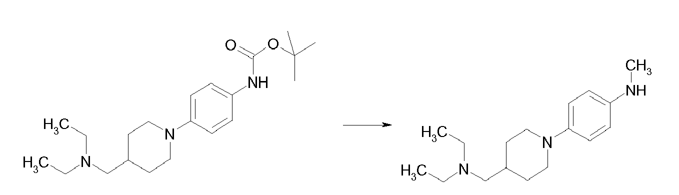 83b wird analog zu 51 b aus 1.80 g (4.98 mMol) Produkt aus 83a und 0.57 g (15.00 mMol) Lithiumaluminiumhydrid (Aldrich) in 25 ml THF hergestellt.
   C₁₇H₂₉N₃ (275.43)
   [M+H]+ = 276
   HPLC (Methode 1): Retentionszeit = 1.77 min
83c) 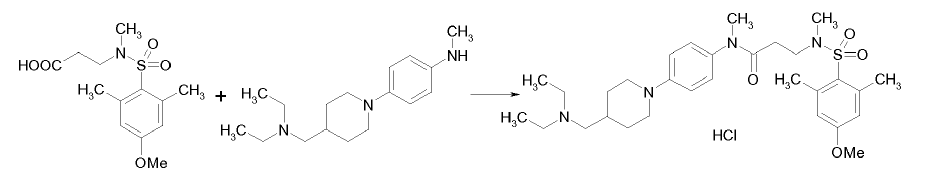 Beispiel 83 wird analog zu 1f aus 0.14 g (0.50 mMol) Produkt aus 22c, 0.15 g (0.50 mMol) Produkt aus 83b, 0.14 ml (1.00 mMol) Triethylamin und 0.18 g (0.55 mMol) TBTU in 6 ml DMF hergestellt.
   C₃₀H₄₆N₄O₄S x HCl (595.24)
   [M+H]+ = 559
   HPLC (Methode 1): Retentionszeit = 2.46 min

### Beispiel 84

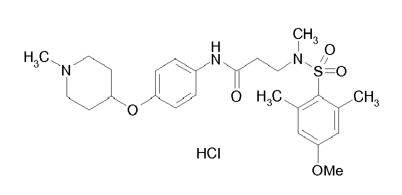

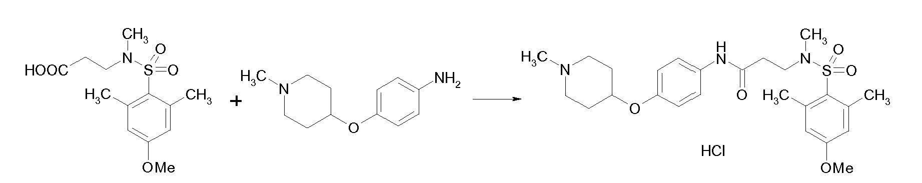

Beispiel 84 wird analog zu 1f aus 0.15 g (0.50 mMol) Produkt aus 22c, 0.10 g (0.50 mMol) 4-(1-Methylpiperidin-4-yloxy)-phenylamin (ART-CHEM), 0.14 ml (1.00 mMol) Triethylamin und 0.18 g (0.55 mMol) TBTU in 6 ml DMF hergestellt.
C₂₅H₃₅N₃O₅S x HCl (526.09)
[M+H]+ = 490
HPLC (Methode 1): Retentionszeit = 2.40 min

### Beispiel 85

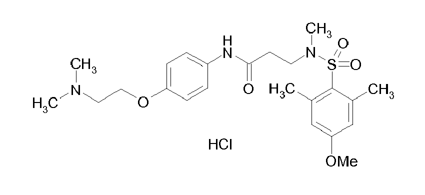

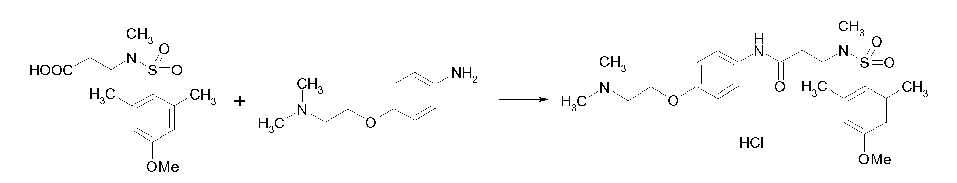

Beispiel 85 wird analog zu 1f aus 0.15 g (0.50 mMol) Produkt aus 22c, 0.09 g (0.50 mMol) 4-(2-Dimethylamino-ethoxy)-phenylamin (Collect. Czech. Chem. Commun. 55, 1990, 282-295), 0.14 ml (1.00 mMol) Triethylamin und 0.18 g (0.55 mMol) TBTU in 6 ml DMF hergestellt.
C₂₃H₃₃N₃O₅S x HCl (500.05)
[M+H]+ = 464
HPLC (Methode 1): Retentionszeit = 2.35 min

### Beispiel 86

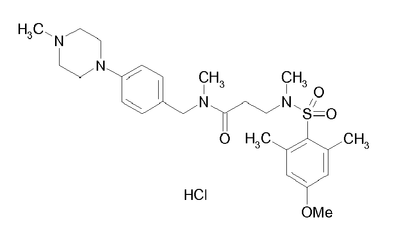

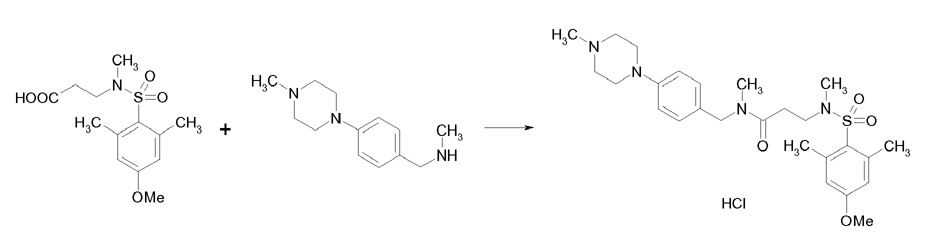

Beispiel 86 wird analog zu 1f aus 0.14 g (0.45 mMol) Produkt aus 22c, 0.099 g (0.45 mMol) Produkt aus 81 a, 0.13 ml (0.90 mMol) Triethylamin und 0.17 g (0.54 mMol) TBTU in 5 ml DMF hergestellt.
C₂₆H₃₈N₄O₅S x HCl (539.13)
[M+H]+ = 503
HPLC (Methode 1): Retentionszeit = 2.43 min

### Beispiel 87

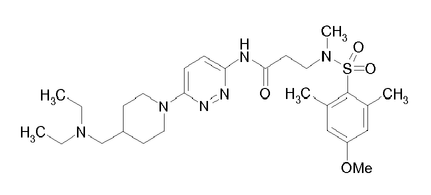

87a) 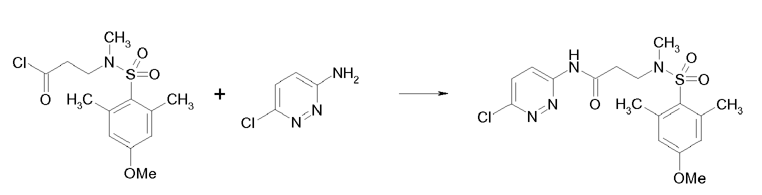 Eine Mischung aus 0.11 g (0.33 mMol) Produkt aus 27c, 0.043 g (0.33 mMol) 3-Amino-6-chlorpyridazin (Acros), 0.12 ml (0.66 mMol) DIPEA und 10 ml Dichlormethan wird drei Tage unter Rückfluss gerührt. Der Niederschlag wird danach abfiltriert. Das Filtrat wird im Vakuum bis zur Trockene eingeengt und das Rohpodukt durch präparative HPLC gereinigt.
   C₁₇H₂₁ClN₄O₄S (412.89)
   [M+H]+ = 413/415
87b) 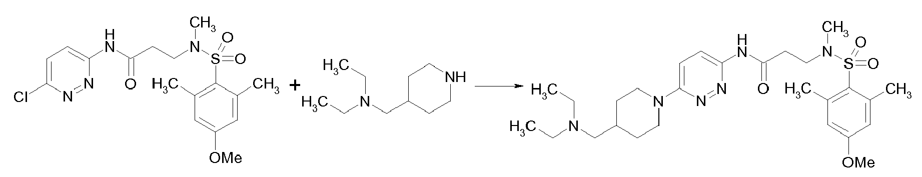 Eine Mischung aus 0.03 g (0.073 mMol) Produkt aus 87a und 0.013 g (0.073 mMol) Diethyl-piperidin-4-ylmethyl-amin (Chem. Pharm. Bull. 42, 1994, 74-84) wird bei 173°C geschmolzen. Das Produkt wird danach durch präparative HPLC aus der Reaktionsmischung gewonnen.
   C₂₇H₄₂N₆O₄S (546.73)
   [M+H]+ = 547
   HPLC (Methode 6): Retentionszeit = 2.12 min

### Beispiel 88

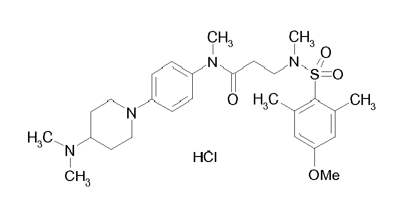

88a) 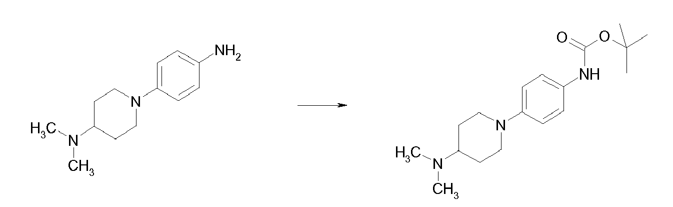 88a wird analog zu 51a aus 0.70 g (3.18 mMol) Produkt aus 27b, 0.80 g (3.65 mMol) Boc-Anhydrid und 5.50 ml (11.00 mMol) 2 M Natronlauge in 40 ml Dioxan und 20 ml Wasser hergestellt.
   C₁₇H₂₈N₄O₂ (320.43)
   [M+H]+ = 321
   DC: Kieselgel, Dichlormethan / Methanol / Ammoniak 4:1:0.2, Rf-Wert = 0.83
88b) 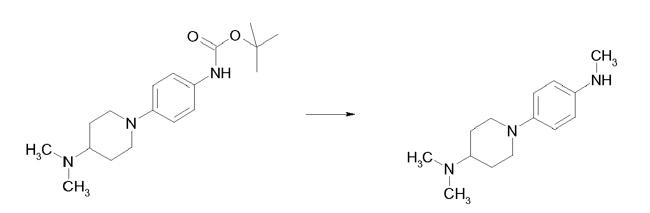 88b wird analog zu 51 b aus 0.60 g (1.87 mMol) Produkt aus 88a und 0.21 g (5.60 mMol) Lithiumaluminiumhydrid (Aldrich) in 20 ml THF hergestellt.
   C₁₃H₂₂N₄ (234.34)
   [M+H]+ = 235
   DC: Kieselgel, Dichlormethan / Methanol / Ammoniak 4:1:0.2, Rf-Wert = 0.62
88c) 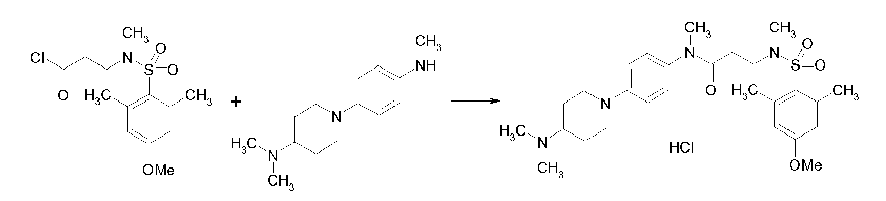 Beispiel 88 wird analog zu 27d aus 0.16 g (0.50 mMol) Produkt aus 27c, 0.12 g (0.50 mMol) Produkt aus 88b und 0.17 ml (1.00 mMol) DIPEA in 5 ml THF hergestellt. C₂₆H₃₉N₅O₄S x HCl (554.15)
   [M+H]+ = 518
   DC: Kieselgel, Dichlormethan / Methanol / Ammoniak 9:1:0.1, Rf-Wert = 0.61

### Beispiel 89

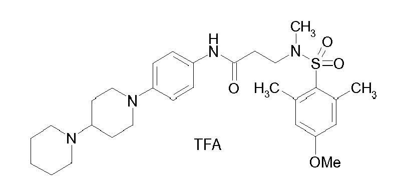

89a) 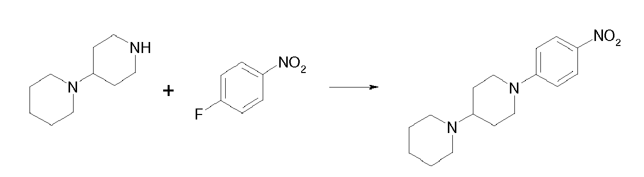 89a wird analog zu 8a aus 0.70 g (4.18 mMol) 4-Piperidinopiperidin (Aldrich), 0.44 ml (4.18 mMol) 1-Fluor-4-nitrobenzol (Acros) und 1.33 ml (9.61 mMol) Triethylamin in 12 ml DMF hergestellt.
   C₁₆H₂₃N₃O₂ (289.37)
   [M+H]+ = 290
   DC: Kieselgel, Dichlormethan / Methanol / Ammoniak 9:1:0.1, Rf-Wert = 0.3
89b) 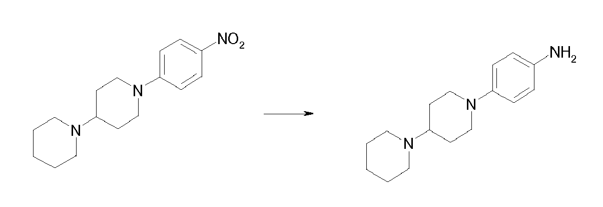 89b wird analog zu 8b aus 0.96 g (3.32 mMol) Produkt aus 89a und 0.093 g Palladium auf Kohle (5%) in 45 ml Ethanol hergestellt.
   C₁₆H₂₅N₃ (259.39)
   [M+H]+ = 260
   DC: Kieselgel, Dichlormethan / Methanol / Ammoniak 9:1:0.1, Rf-Wert = 0.2
89c) 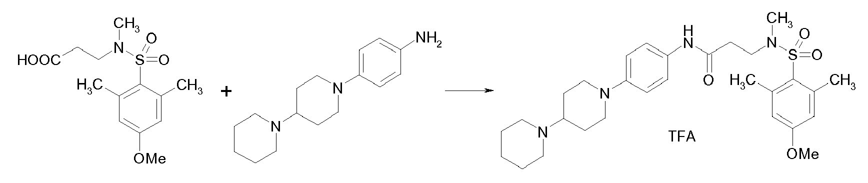 Beispiel 89 wird analog zu 1f aus 0.10 g (0.33 mMol) Produkt aus 22c, 0.086 g (0.33 mMol) Produkt aus 89b, 0.14 ml (1.00 mMol) Triethylamin und 0.11 g (0.33 mMol) TBTU in 2 ml THF hergestellt.
   C₂₉H₄₂N₄O₄S x C₂HF₃O₂ (656.76)
   [M+H]+ = 543
   HPLC (Methode 5): Retentionszeit = 1.47 min

### Beispiel 90

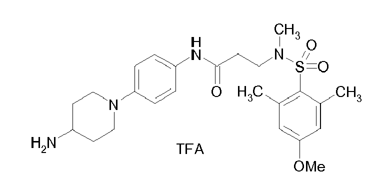

90a) 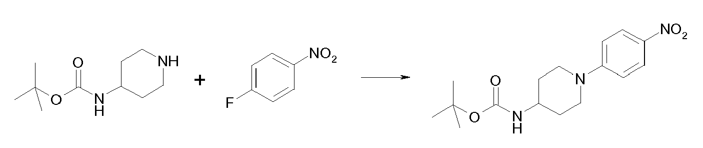 90a wird analog zu 8a aus 0.84 g (4.18 mMol) 4-N-Boc-Aminopiperidin (Acros), 0.44 ml (4.18 mMol) 1-Fluor-4-nitrobenzol (Acros) und 1.33 ml (9.61 mMol) Triethylamin in 12 ml DMF hergestellt.
   C₁₆H₂₃N₃O₄ (321.37)
   [M+H]+ = 322
90b) 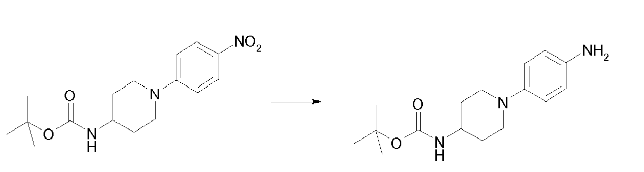 90b wird analog zu 8b aus 1.01 g (3.43 mMol) Produkt aus 90a und 0.11 g Palladium auf Kohle (5%) in 45 ml Ethanol hergestellt.
   C₁₆H₂₅N₃O₂ (291.39)
   [M+H]+ = 292
90c) 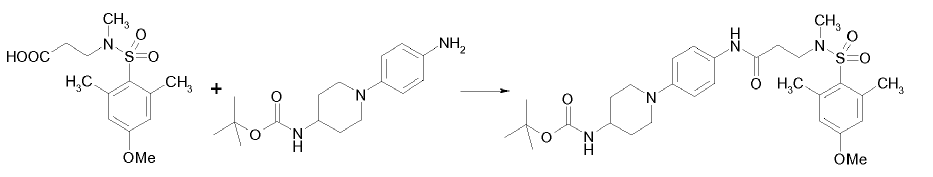 90c wird analog zu 1f aus 0.10 g (0.33 mMol) Produkt aus 22c, 0.097 g (0.33 mMol) Produkt aus 90b, 0.14 ml (1.00 mMol) Triethylamin und 0.11 g (0.33 mMol) TBTU in 2 ml THF hergestellt.
   C₂₉H₄₂N₄O₆S (574.73)
   [M+H]+ = 575
   HPLC (Methode 5): Retentionszeit = 1.62 min
90d) 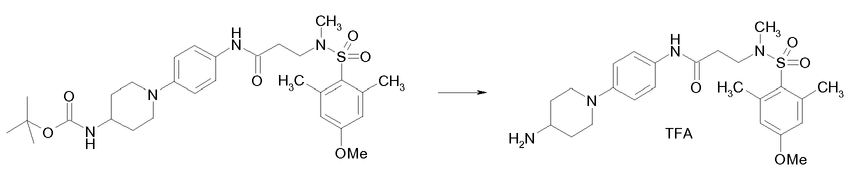 Beispiel 90 wird analog zu 18b aus 0.19 g (0.33 mMol) Produkt aus 90c und 0.33 ml TFA in 0.5 ml Dichlormethan hergestellt.
   C₂₄H₃₄N₄O₄S x C₂HF₃O₂ (588.64)
   [M+H]+ = 475
   HPLC (Methode 5): Retentionszeit = 1.41 min

### Beispiel 91

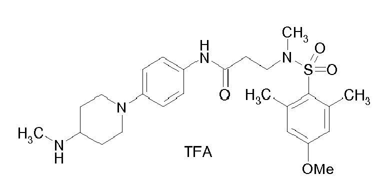

91a) 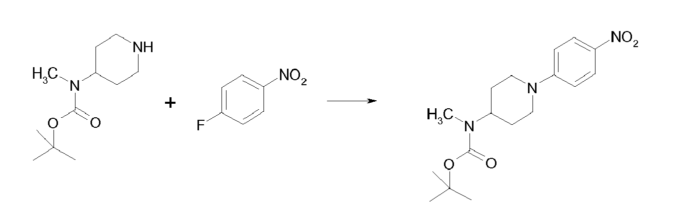 91 a wird analog zu 8a aus 0.90 g (4.18 mMol) Methyl-piperidin-4-yl-carbamatsäure-tert-butylester (Fluorochem), 0.44 ml (4.18 mMol) 1-Fluor-4-nitrobenzol (Acros) und 1.33 ml (9.61 mMol) Triethylamin in 12 ml DMF hergestellt.
   C₁₇H₂₅N₃O₄ (335.40)
   [M+H]+ = 336
   DC: Kieselgel, Dichlormethan / Methanol 30:1, Rf-Wert = 0.6
91b) 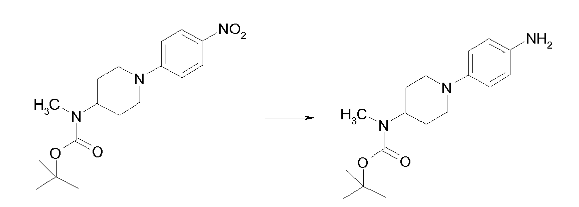 91 b wird analog zu 8b aus 1.08 g (3.22 mMol) Produkt aus 91 a und 0.11 g Palladium auf Kohle (5%) in 45 ml Ethanol hergestellt.
   C₁₇H₂₇N₃O₂ (305.42)
   [M+H]+ = 306
   DC: Kieselgel, Dichlormethan / Methanol 30:1, Rf-Wert = 0.4
91c) 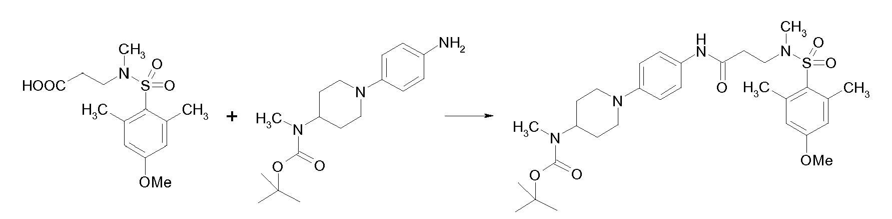 91c wird analog zu 1f aus 0.10 g (0.33 mMol) Produkt aus 22c, 0.10 g (0.33 mMol) Produkt aus 91b, 0.14 ml (1.00 mMol) Triethylamin und 0.11 g (0.33 mMol) TBTU in 2 ml THF hergestellt.
   C₃₀H₄₄N₄O₆S (588.76)
   [M+H]+ = 589
   HPLC (Methode 5): Retentionszeit = 1.69 min
91d) 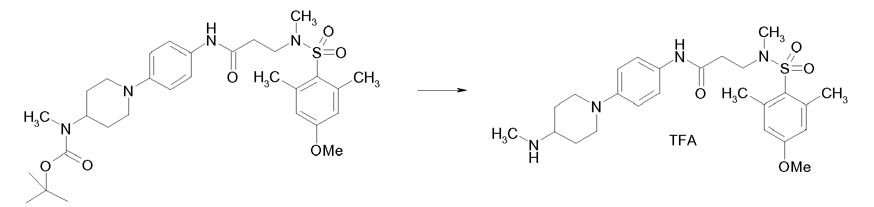 Beispiel 91 wird analog zu 18b aus 0.21 g (0.36 mMol) Produkt aus 91 c und 0.36 ml TFA in 0.6 ml Dichlormethan hergestellt.
   C₂₅H₃₆N₄O₄S x C₂HF₃O₂ (602.67)
   [M+H]+ = 489
   HPLC (Methode 5): Retentionszeit = 1.42 min

### Beispiel 92

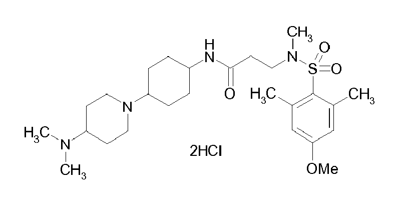

92a) 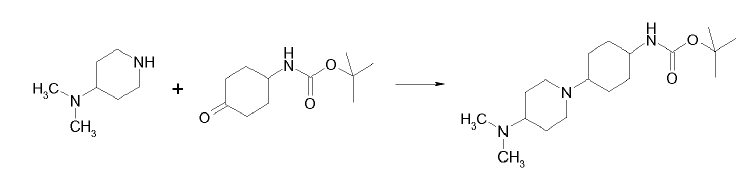 Eine Mischung aus 0.66 g (5.16 mMol) 4-Dimethylamino-piperidin (Alfa Aesar), 1.00 g (4.69 mMol) (4-Oxocyclohexyl)-carbamatsäure-tert-butylester (Fluorochem), 1.19 g (5.16 mMol) Natriumtriacetoxyborhydrid und 20 ml Dichlormethan wird unter Stickstoff vier Stunden bei Raumtemperatur gerührt. Die Reaktionsmischung wird danach mit Dichlormethan verdünnt, mit gesättigter Natriumhydrogencarbonat-Lösung gewaschen, über Natriumsulfat getrocknet und im Vakuum bis zur Trockene eingeengt.
   C₁₈H₃₅N₃O₂ (325.49)
92b) 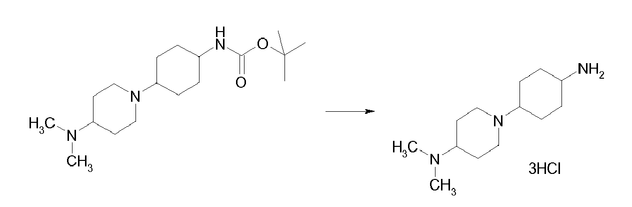 Eine Mischung aus 0.80 g (2.46 mMol) Produkt aus 92a, 4 ml 6 M HCl, 3 ml 37%ige HCl und 3 ml Methanol wird zwei Stunden bei 50°C gerührt. Die Reaktionsmischung wird danach im Vakuum bis zur Trockene eingeengt. C₁₃H₂₇N₃ x 3HCl (334.76)
92c) 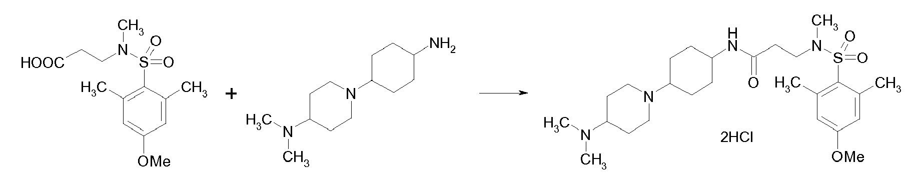 Beispiel 92 wird analog zu 1f aus 0.10 g (0.33 mMol) Produkt aus 22c, 0.13 g (0.40 mMol) Produkt aus 92b, 0.23 ml (1.66 mMol) Triethylamin und 0.13 g (0.40 mMol) TBTU in 8 ml DMF hergestellt.
   C₂₆H₄₄N₄O₄S x 2HCl (581.64)
   [M+H]+ = 509
   HPLC (Methode 5): Retentionszeit = 1.36 min

### Beispiel 93

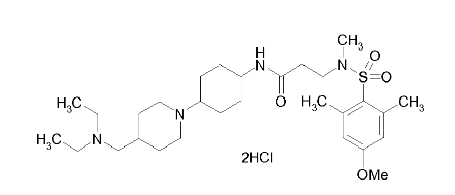

93a) 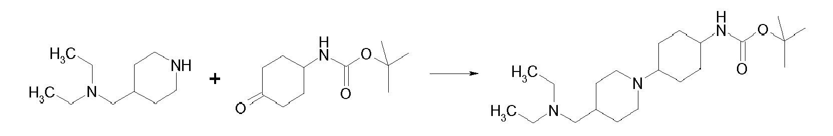 93a wird analog zu 92a aus 0.63 g (3.70 mMol) Diethyl-piperidin-4-ylmethyl-amin (Chem. Pharm. Bull. 42, 1994, 74-84), 0.72 g (3.37 mMol) (4-Oxocyclohexyl)-carbamatsäure-tert-butylester (Fluorochem) und 0.86 g (4.04 mMol) Natriumtriacetoxyborhydrid in 20 ml Dichlormethan durchgeführt.
   C₂₁H₄₁N₃O₂ (367.57)
93b) 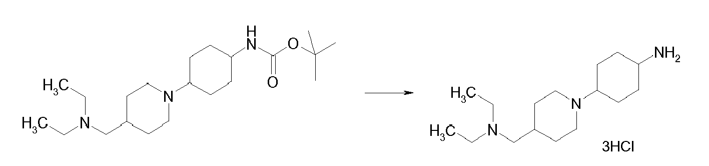 93b wird analog zu 92b aus 0.90 g (2.45 mMol) Produkt aus 93a, 4 ml 6 M HCl und 3 ml 37%ige HCl in 3 ml Methanol hergestellt. C₁₆H₃₃N₃ x 3HCl (376.84)
93c) 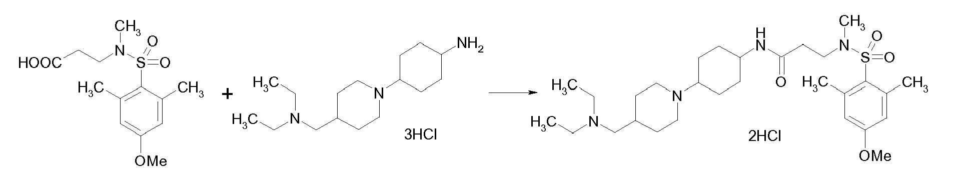 Beispiel 93 wird analog zu 1f aus 0.10 g (0.33 mMol) Produkt aus 22c, 0.14 g (0.37 mMol) Produkt aus 93b, 0.23 ml (1.66 mMol) Triethylamin und 0.13 g (0.40 mMol) TBTU in 8 ml DMF hergestellt.
   C₂₉H₅₀N₄O₄S x 2HCl (623.72)
   [M+H]+ = 551
   HPLC (Methode 5): Retentionszeit = 1.39 min

### Beispiel 94

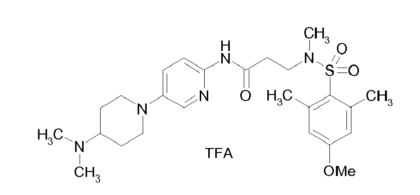

94a) 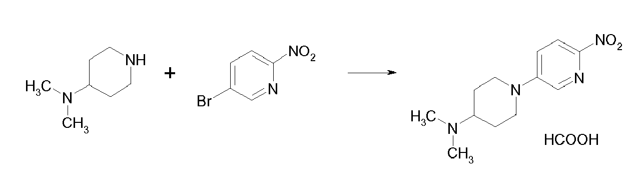 Eine Mischung aus 0.88 g (6.88 mMol) 4-Dimethylamino-piperidin (Alfa Aesar), 1.00 g (4.93 mMol) 3-Brom-6-nitropyriridin (Aldrich), 0.18 g (0.49 mMol) Tetrabutyl-ammoniumiodid, 0.74 g (5.33 mMol) Kaliumcarbonat und 5 ml DMSO wird zwei Stunden bei 80°C gerührt. Danach wird die Reaktionsmischung auf Wasser gegossen und mit Dichlormethan extrahiert. Die organischen Extrakte werden mit gesättigter Natriumhydrogencarbonat-Lösung und gesättigter Natriumchlorid-Lösung gewaschen, über Natriumsulfat getrocknet und im Vakuum bis zur Trockene eingeengt. Das so erhaltene Rohprodukt wird durch präparative HPLC gereinigt.
   C₁₂H₁₈N₄O₂ x CH₂O₂ (296.32)
   HPLC (Methode 1): Retentionszeit = 1.49 min
94b) 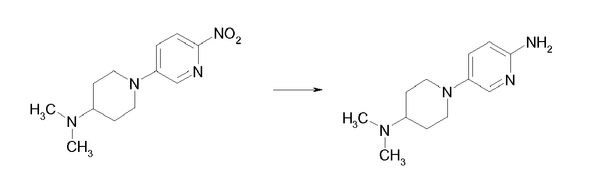 94b wird analog zu 8b aus 0.50 g (2.00 mMol) Produkt aus 94a und 0.08 g Palladium auf Kohle (10%) in 40 ml Methanol hergestellt.
   C₁₂H₂₀N₄ (220.31)
94c) 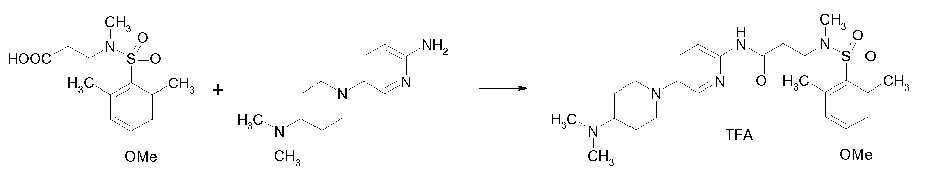 Eine Mischung aus 0.63 g (2.10 mMol) Produkt aus 22c, 0.91 g (9.00 mMol) N-Methylmorpholin, 0.45 g (2.04 mMol) Produkt aus 94b und 50 ml THF wird 10 Minuten bei Raumtemperatur gerührt und danach mit 5.22 ml (9.00 mMol) Propylphosphonsäureanhydrid 50% in Ethylacetat (Fluka) versetzt. Man lässt die Reaktionsmischung über Nacht bei Raumtemperatur rühren und engt sie anschließend im Vakuum bis zur Trockene ein. Der Rückstand wird mit 2 N Kaliumcarbonat-Lösung versetzt und mit Dichlormethan extrahiert. Die organischen Extrakte werden mit Wasser und gesättigter Natriumchlorid-Lösung gewaschen, über Natriumsulfat getrocknet und im Vakuum bis zur Trockene eingeengt. Das so erhaltene Rohprodukt wird durch präparative HPLC gereinigt. C₂₅H₃₇N₅O₄S x C₂HF₃O₂ (617.68)
   [M+H]+ = 504
   HPLC (Methode 5): Retentionszeit = 1.38 min

### Beispiel 95

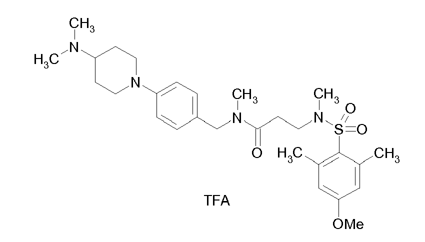

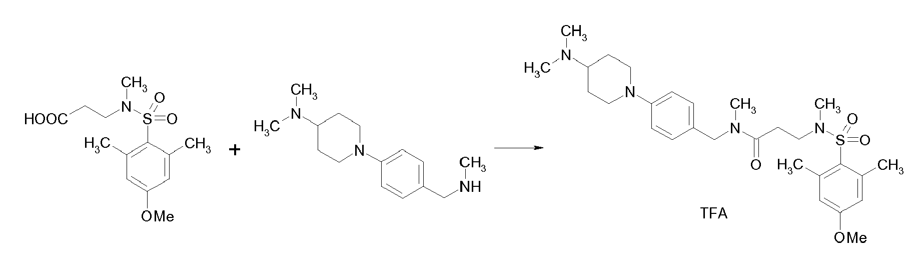

Beispiel 95 wird analog zu 1f aus 0.08 g (0.27 mMol) Produkt aus 22c, 0.066 g (0.27 mMol) Produkt aus 80a, 0.11 ml (0.80 mMol) Triethylamin und 0.085 g (0.27 mMol) TBTU in 2 ml THF hergestellt.
C₂₈H₄₂N₄O₄S x C₂HF₃O₂ (644.75)
[M+H]+ = 531
HPLC (Methode 5): Retentionszeit = 1.50 min

### Beispiel 96

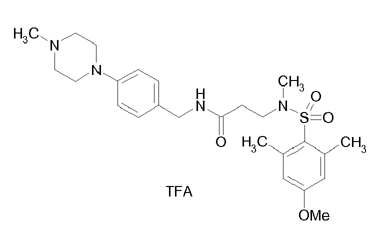

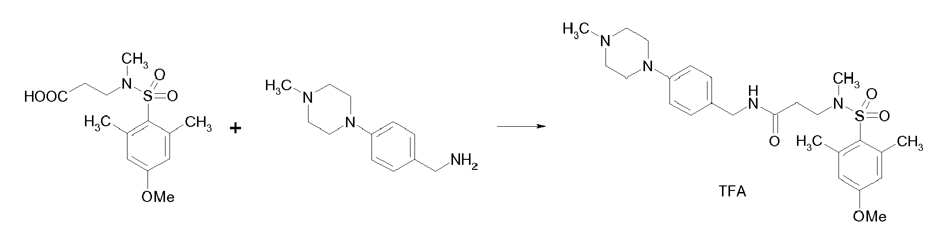

Beispiel 96 wird analog zu 1f aus 0.08 g (0.27 mMol) Produkt aus 22c, 0.054 g (0.27 mMol) Produkt aus 19b, 0.11 ml (0.80 mMol) Triethylamin und 0.085 g (0.27 mMol) TBTU in 2 ml THF hergestellt.
C₂₅H₃₆N₄O₄S x C₂HF₃O₂ (602.67)
[M+H]+ = 489
HPLC (Methode 5): Retentionszeit = 1.49 min

### Beispiel 97

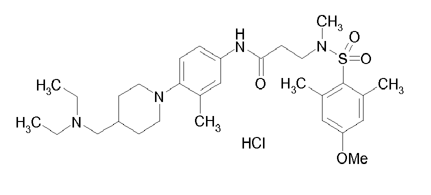

97a) 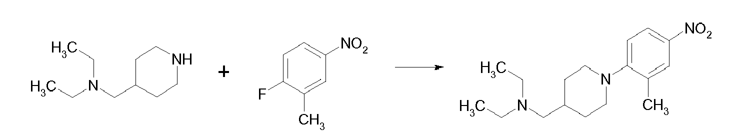 97a wird analog zu 8a aus 1.00 g (5.87 mMol) Diethyl-piperidin-4-ylmethyl-amin (Chem. Pharm. Bull. 42, 1994, 74-84), 0.91 g (5.87 mMol) 1-Fluor-2-methyl-4-nitrobenzol (ABCR) und 1.14 ml (8.20 mMol) Triethylamin in 12 ml DMF hergestellt.
   C₁₇H₂₇N₃O₂ (305.42)
   [M+H]+ = 306
97b) 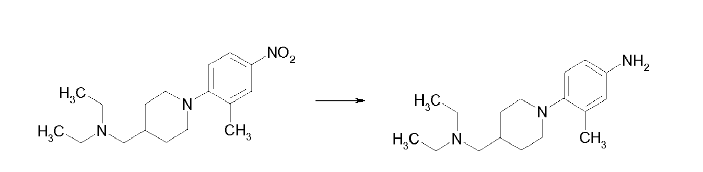 97b wird analog zu 8b aus 0.91 g (2.98 mMol) Produkt aus 97a und 0.20 g Palladium auf Kohle (10%) in 50 ml Methanol hergestellt.
   C₁₇H₂₉N₃ (275.43)
97c) 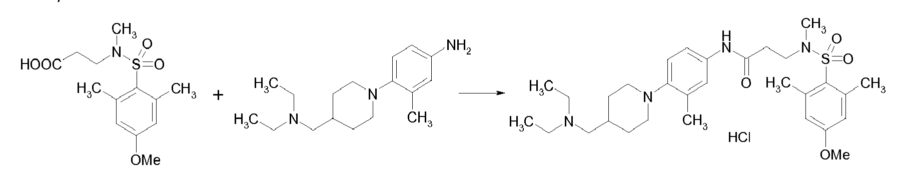 Beispiel 97 wird analog zu 1f aus 0.15 g (0.50 mMol) Produkt aus 22c, 0.14 g (0.50 mMol) Produkt aus 97b, 0.21 ml (1.50 mMol) Triethylamin und 0.16 g (0.50 mMol) TBTU in 7 ml THF und 1 ml DMF hergestellt.
   C₃₀H₄₆N₄O₄S x HCl (595.24)
   [M+H]+ = 559
   HPLC (Methode 5): Retentionszeit = 1.44 min

### Beispiel 98

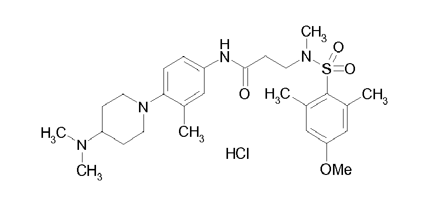

98a) 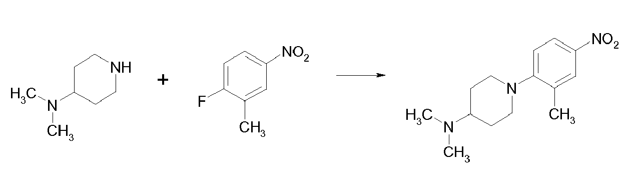 98a wird analog zu 8a aus 0.75 g (5.87 mMol) 4-Dimethylamino-piperidin (Alfar Aesar), 0.91 g (5.87 mMol) 1-Fluor-2-methyl-4-nitrobenzol (ABCR) und 1.14 ml (8.20 mMol) Triethylamin in 12 ml DMF hergestellt.
   C₁₄H₂₁N₃O₂ (263.34)
98b) 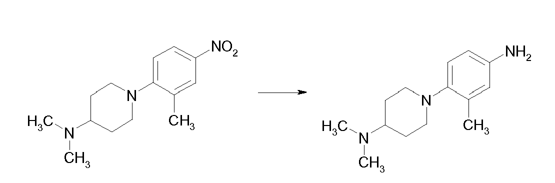 98b wird analog zu 8b aus 0.30 g (1.14 mMol) Produkt aus 98a und 0.10 g Palladium auf Kohle (10%) in 25 ml Methanol hergestellt.
   C₁₄H₂₃N₃ (233.35)
98c)

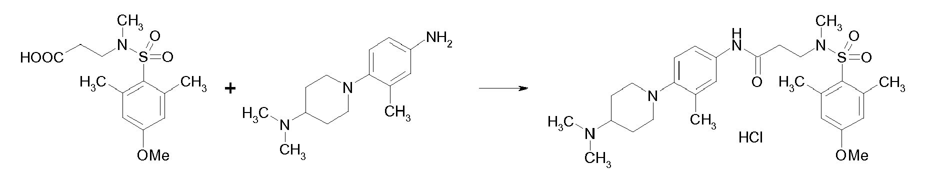

Beispiel 98 wird analog zu 1f aus 0.32 g (1.07 mMol) Produkt aus 22c, 0.25 g (1.07 mMol) Produkt aus 98b, 0.42 ml (3.00 mMol) Triethylamin und 0.34 g (1.07 mMol) TBTU in 7 ml THF und 1 ml DMF hergestellt.
C₂₇H₄₀N₄O₄S x HCl (553.16)
[M+H]+ = 517
HPLC (Methode 5): Retentionszeit = 1.52 min

### Beispiel 99

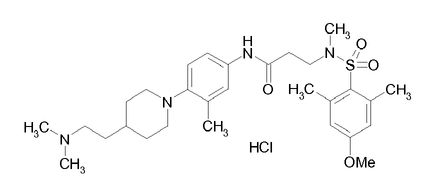

99a) 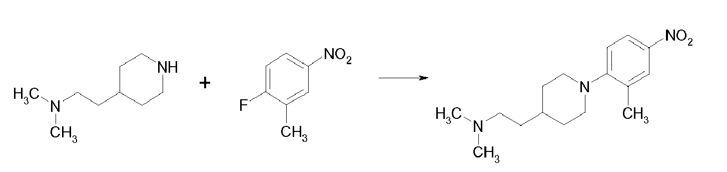 99a wird analog zu 8a aus 0.92 g (5.87 mMol) Dimethyl-(2-piperidin-4-yl-ethyl)-amin (J. Med. Chem. 36, 1993, 162-165), 0.91 g (5.87 mMol) 1-Fluor-2-methyl-4-nitrobenzol (ABCR) und 2.49 g (18.00 mMol) Kaliumcarbonat in 12 ml DMF hergestellt.
   C₁₆H₂₅N₃O₂ (291.39)
   [M+H]+ = 292
99b) 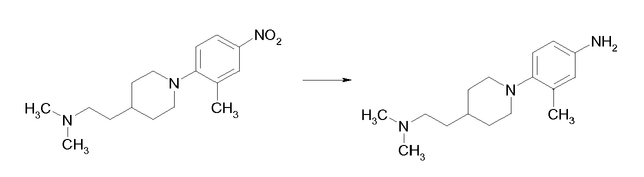 99b wird analog zu 8b aus 0.60 g (1.14 mMol) Produkt aus 99a und 0.20 g Palladium auf Kohle (10%) in 50 ml Methanol hergestellt.
   C₁₆H₂₇N₃ (261.41)
99c) 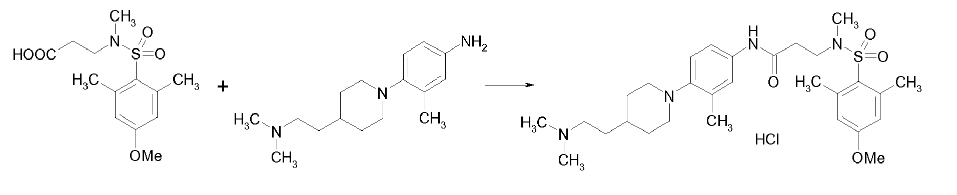 Beispiel 99 wird analog zu 1f aus 0.15 g (0.50 mMol) Produkt aus 22c, 0.13 g (0.50 mMol) Produkt aus 99b, 0.21 ml (1.50 mMol) Triethylamin und 0.16 g (0.50 mMol) TBTU in 7 ml THF und 1 ml DMF hergestellt.
   C₂₉H₄₄N₄O₄S x HCl (581.21)
   [M+H]+ = 545
   HPLC (Methode 5): Retentionszeit = 1.42 min

### Beispiel 100

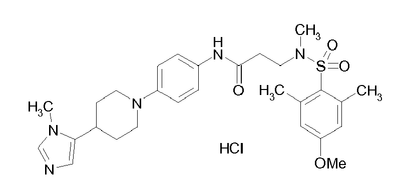

100a) 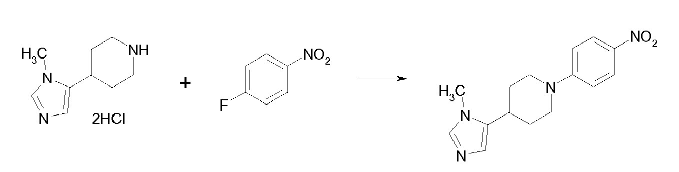 100a wird analog zu 8a aus 1.00 g (4.47 mMol) 4-(3-Methyl-3H-imidazol-4-yl)-piperidin (J. Med. Chem. 46, 2003, 5445-5457), 0.63 g (4.47 mMol) 4-Fluor-nitrobenzol (ABCR) und 2.10 g (15.20 mMol) Kaliumcarbonat in 50 ml DMF hergestellt.
   C₁₄H₁₆N₄O₂ (272.30)
   [M+H]+ = 273
100b) 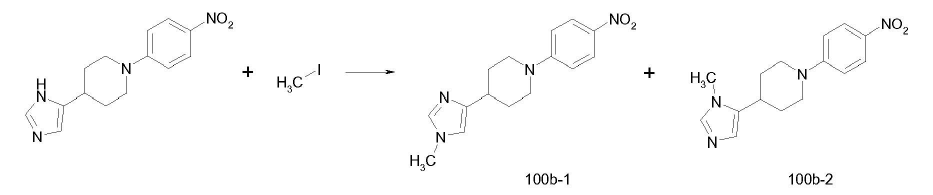 100b-1 und 1 00b-2 werden analog zu 62a aus 1.10 g (4.04 mMol) Produkt aus 100a, 0.60 g (4.23 mMol) Methyliodid und 0.46 g (4.10 mMol) Kalium-tert-butylat in 50 ml DMSO hergestellt. Das so entstandene Isomerengemisch wird durch Säulen-chromatographie über Kieselgel (Elutionsmittel: Dichlormethan / Methanol 100:1 bis 30:1) aufgetrennt.
   - 100b-1:: C₁₅H₁₈N₄O₂ (286.33)
   [M+H]+ = 287
   DC: Kieselgel, Dichlormethan / Methanol 9:1, Rf-Wert = 0.50
   - 100b-2:: C₁₅H₁₈N₄O₂ (286.33)
   [M+H]+ = 287
   DC: Kieselgel, Dichlormethan / Methanol 9:1, Rf-Wert = 0.38
100c) 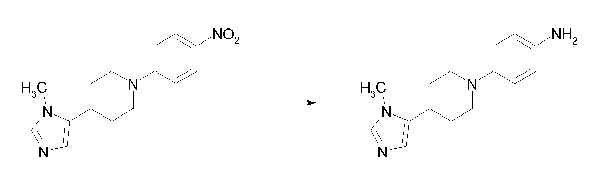 100c wird analog zu 8b aus 0.10 g (0.35 mMol) 100b-2 und 0.20 g Palladium auf Kohle (10%) in 30 ml Methanol hergestellt.
   C₁₅H₂₀N₄ (256.35)
   DC: Kieselgel, Dichlormethan / Methanol 9:1, Rf-Wert = 0.10
100d) 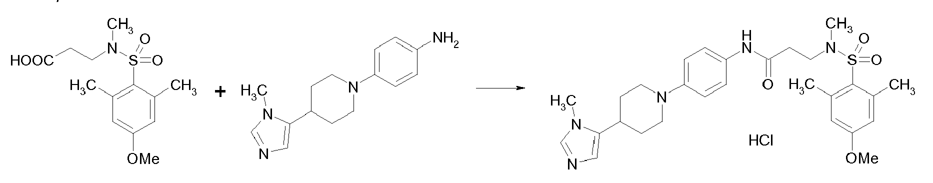 Beispiel 100 wird analog zu 1f aus 0.085 g (0.28 mMol) Produkt aus 22c, 0.070 g (0.27 mMol) Produkt aus 100c, 0.048 ml (0.35 mMol) Triethylamin und 0.095 g (0.30 mMol) TBTU in 20 ml THF und 3 ml DMF hergestellt.
   C₂₈H₃₇N₅O₄S x HCl (576.15)
   [M+H]+ = 540
   HPLC (Methode 5): Retentionszeit = 1.41 min

### Beispiel 101

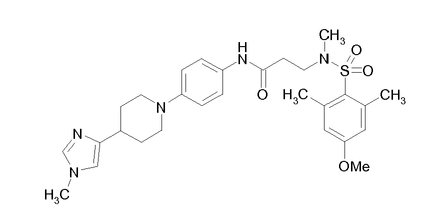

101a) 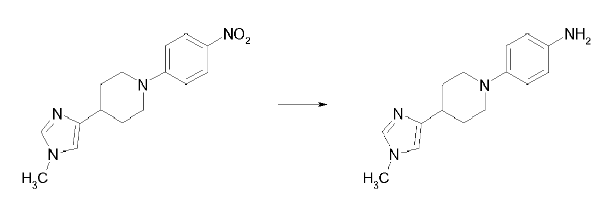 101a wird analog zu 8b aus 0.35 g (1.22 mMol) 100b-1 und 0.50 g Palladium auf Kohle (10%) in 50 ml Methanol hergestellt.
   C₁₅H₂₀N₄ (256.35)
   DC: Kieselgel, Dichlormethan / Methanol 9:1, Rf-Wert = 0.14
101b) 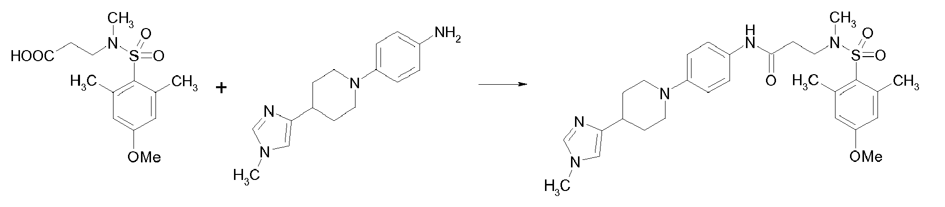 Beispiel 101 wird analog zu 1f aus 0.12 g (0.40 mMol) Produkt aus 22c, 0.10 g (0.39 mMol) Produkt aus 101 a, 0.05 ml (0.50 mMol) Triethylamin und 0.14 g (0.42 mMol) TBTU in 30 ml THF und 5 ml DMF hergestellt.
   C₂₈H₃₇N₅O₄S (539.69)
   [M+H]+ = 540
   DC: Kieselgel, Dichlormethan / Methanol 9:1, Rf-Wert = 0.47

### Beispiel 102

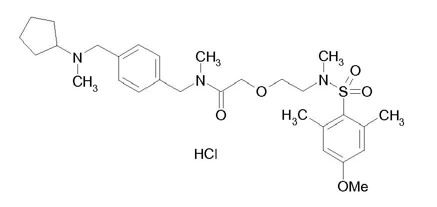

102a) 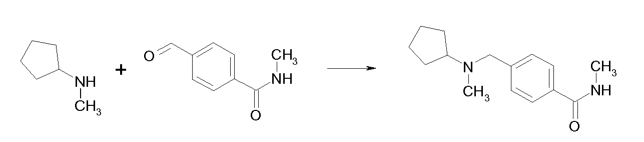 102a wird analog zu 60a aus 0.50 g (5.00 mMol) N-Methylcyclohexylamin (CHESS), 0.82 g (5.00 mMol) 4-Formyl-benzoesäuremethylamid (EMKA), 1.59 g (7.50 mMol) Natrium-triacetoxyborhydrid und 0.37 ml (6.50 mMol) Essigsäure in 30 ml THF hergestellt.
   C₁₅H₂₂N₂ (246.35)
102b) 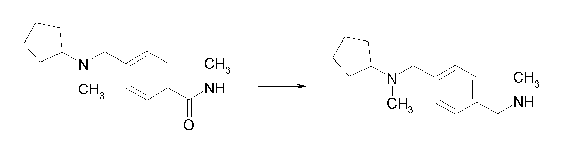 102b wird analog zu 38f aus 1.06 g (4.30 mMol) Produkt aus 102a und 8.60 ml (8.60 mMol) Lithiumaluminiumhydrid 1 M in THF (Aldrich) in 40 ml THF hergestellt.
   C₁₅H₂₄N₂ (232.36)
102c) 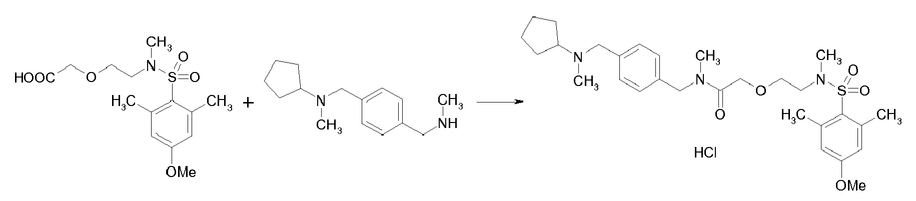 Beispiel 102 wird analog zu 1f aus 0.20 g (0.60 mMol) Produkt aus 53c, 0.14 g (0.60 mMol) Produkt aus 102b, 0.21 ml (1.50 mMol) Triethylamin und 0.23 g (0.72 mMol) TBTU in 10 ml THF hergestellt.
   C₂₉H₄₃N₃O₅S x HCl (582.20)
   [M+H]+ = 546
   HPLC (Methode 5): Retentionszeit = 1.58 min

### Beispiel 103

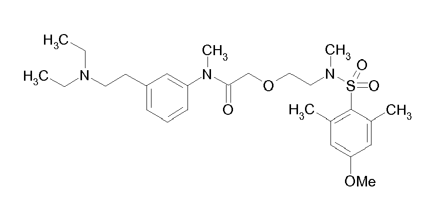

103a) 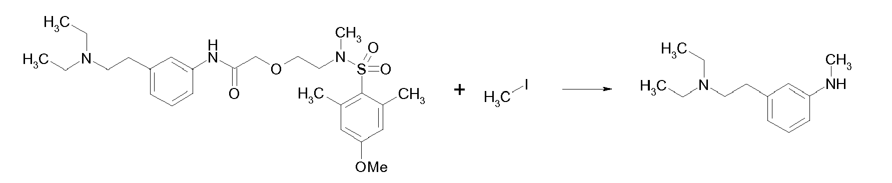 Eine Mischung aus 0.04 g (0.079 mMol) 70, 4.8 mg (0.12 mMol) Natriumhydrid 60%ig, 1 ml THF und 0.5 ml DMF wird 30 Minuten bei Raumtemperatur gerührt. Danach gibt man 4.9 µl (0.079 mMol) Methyliodid hinzu und lässt weitere zwei Stunden bei Raumtemperatur rühren. Die Reaktionsmischung wird anschließend im Vakuum bis zur Trockene eingeengt, der Rückstand mit Wasser versetzt und mit Ethylacetat extrahiert. Die organischen Extrakte werden über Natriumsulfat getrocknet und im Vakuum bis zur Trockene eingeengt. Das so erhaltene Rohprodukt wird durch Säulenchromatographie (Elutionsmittel: Dichlormethan / Methanol / Ammoniak 95:5:0.5) gereinigt.
   C₁₃H₂₂N₂ (206.33)
   [M+H]+ = 207
103b) 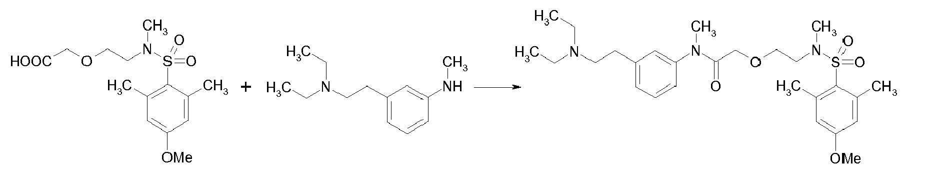 Beispiel 103 wird analog zu 1f aus 0.08 g (0.24 mMol) Produkt aus 53c, 0.05 g (0.24 mMol) Produkt aus 103a, 0.067 ml (0.48 mMol) Triethylamin und 0.093 g (0.29 mMol) TBTU in 7 ml THF und 1 ml DMF hergestellt.
   C₂₇H₄₁N₃O₅S (519.70)
   [M+H]+ = 520
   HPLC (Methode 4): Retentionszeit = 3.2 min

### Beispiel 104

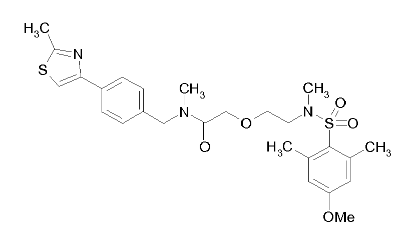

104a) 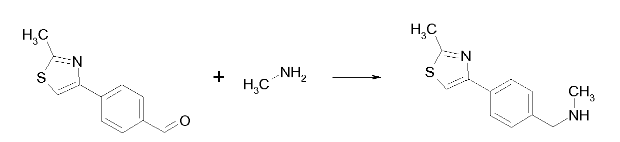 104a wird analog zu 60a aus 0.35 g (1.72 mMol) 4-(2-Methyl-1,3-thiazol-4-yl)-benzaldehyd (Maybridge), 1.50 ml (3.00 mMol) Methylamin 2 M in THF (Acros), 0.70 g (3.30 mMol) Natriumtriacetoxyborhydrid und 0.23 ml (4.00 mMol) Essigsäure in 20 ml THF hergestellt.
   C₁₂H₁₄N₂S (218.32)
   [M+H]+ = 219
104b) 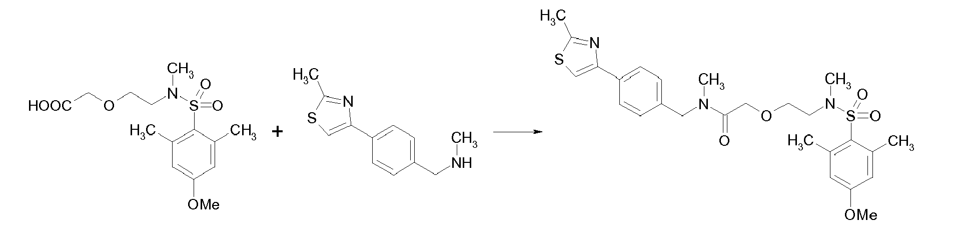 Beispiel 104 wird analog zu 1f aus 0.16 g (0.47 mMol) Produkt aus 53c, 0.10 g (0.46 mMol) Produkt aus 104a, 0.11 ml (1.09 mMol) Triethylamin und 0.16 g (0.48 mMol) TBTU in 30 ml THF und 5 ml DMF hergestellt.
   C₂₆H₃₃N₃O₅S₂ (531.69)
   [M+H]+ = 532
   DC: Kieselgel, Dichlormethan / Methanol 9:1, Rf-Wert = 0.66

### Beispiel 105

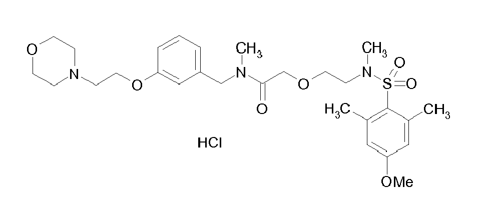

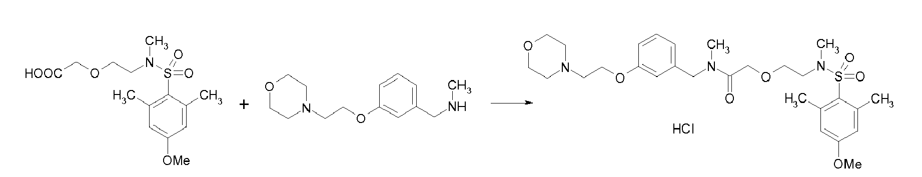

Beispiel 105 wird analog zu 1f aus 0.14 g (0.41 mMol) Produkt aus 53c, 0.10 g (0.40 mMol) Methyl-[3-(2-morpholin-4-yl-ethoxy)-benzyl]-amin (Maybridge), 0.14 ml (0.99 mMol) Triethylamin und 0.14 g (0.44 mMol) TBTU in 30 ml THF und 5 ml DMF hergestellt. C₂₈H₄₁N₃O₇S x HCl (600.17)
[M+H]+ = 564
DC: Kieselgel, Dichlormethan / Methanol 9:1, Rf-Wert = 0.59

### Beispiel 106

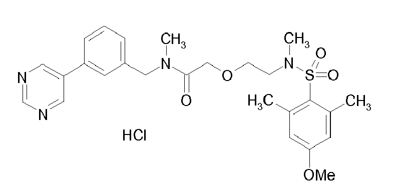

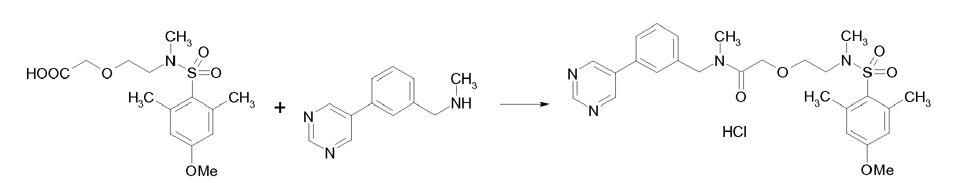

Beispiel 106 wird analog zu 1f aus 0.17 g (0.51 mMol) Produkt aus 53c, 0.10 g (0.50 mMol) Methyl-(3-pyrimidin-5-yl-benzyl)-amin (Maybridge), 0.17 ml (1.19 mMol) Triethylamin und 0.17 g (0.53 mMol) TBTU in 30 ml THF und 5 ml DMF hergestellt.
C₂₆H₃₂N₄O₅S x HCl (549.08)
[M+H]+ = 513
HPLC (Methode 5): Retentionszeit = 1.78 min

### Beispiel 107

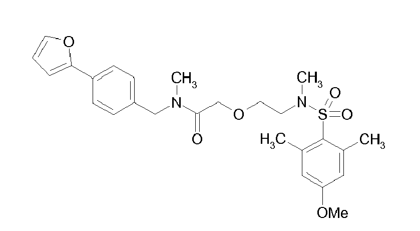

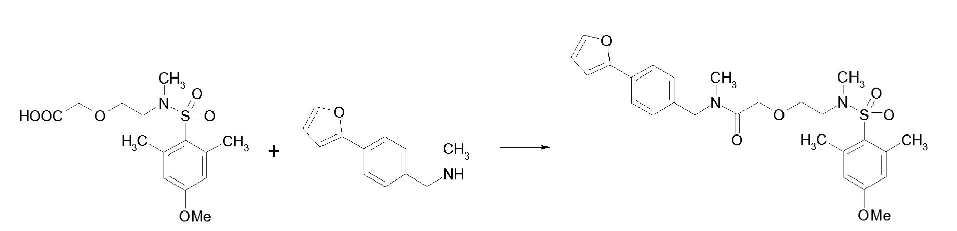

Beispiel 107 wird analog zu 1f aus 0.18 g (0.54 mMol) Produkt aus 53c, 0.10 g (0.53 mMol) (4-Furan-2-yl-benzyl)-methyl-amin (Maybridge), 0.18 ml (1.29 mMol) Triethylamin und 0.18 g (0.56 mMol) TBTU in 30 ml THF und 5 ml DMF hergestellt.
C₂₆H₃₂N₂O₆S (500.61)
[M+H]+ = 501
HPLC (Methode 5): Retentionszeit = 2.09 min

### Beispiel 108

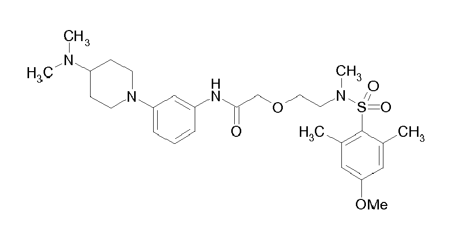

108a) 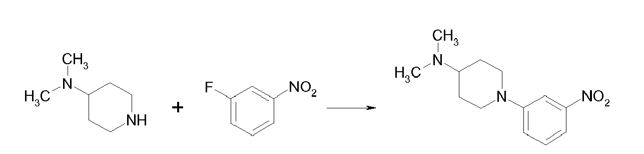 107a wird analog zu 79a aus 2.41 g (18.78 mMol) 4-Dimethylamino-piperidin (Alfa Aesar), 1.00 ml (9.39 mMol) 1-Fluor-3-nitrobenzol (Fluka) und 1.30 g (9.39 mMol) Kaliumcarbonat in 15 ml DMSO hergestellt.
   C₁₃H₁₉N₃O₂ (249.31)
   [M+H]+ = 250
108b) 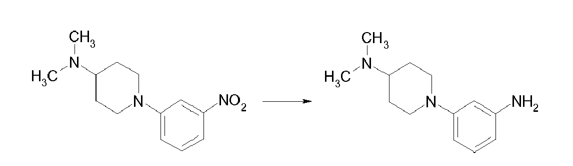 Eine Mischung aus 1.74 g (6.98 mMol) Produkt aus 108a, 12.00 g (68.92 mMol) Natriumdithionit, 10.00 g (72.35 mMol) Kaliumcarbonat, 60 ml THF und 30 ml Wasser sechs Stunden auf 80°C erhitzt. Nach dem Abkühlen wird die organische Phase abgetrennt, mit gesättigter Natriumchlorid-Lösung gewaschen, über Natriumsulfat getrocknet und im Vakuum bis zur Trockene eingeengt.
   C₁₃H₂₁N₃ (219.33)
108c) 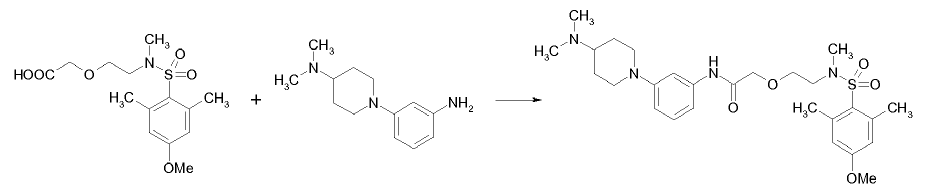 Beispiel 108 wird analog zu 1f aus 0.13 g (0.40 mMol) Produkt aus 53c, 0.10 g (0.47 mMol) Produkt aus 108b, 0.067 ml (0.48 mMol) Triethylamin und 0.15 g (0.48 mMol) TBTU in 5 ml DMF hergestellt.
   C₂₇H₄₀N₄O₅S (532.70)
   [M+H]+ = 533
   HPLC (Methode 5): Retentionszeit = 1.56 min

### Beispiel 109

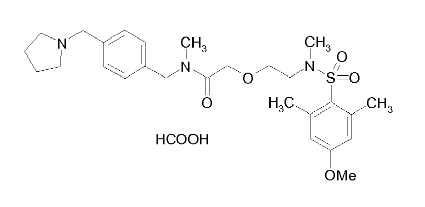

109a) 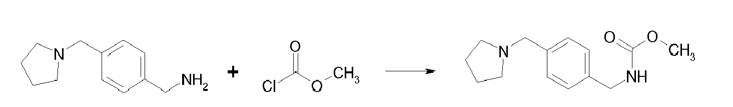 Eine Mischung aus 0.59 g (3.12 mMol) 4-Pyrrolidin-1-ylmethyl-benzylamin (Enamine-BB), 0.29 ml (3.75 mMol) Chlorameisensäuremethylester (Fluka), 0.52 ml (3.75 mMol) Triethylamin und 10 ml Dichlormethan wird zwei Stunden bei Raumtemperatur gerührt. Die Reaktionsmischung wird mit 10 ml Dichlormethan verdünnt, zweimal mit Wasser gewaschen, über Natriumsulfat getrocknet und im Vakuum bis zur Trockene eingeengt.
   C₁₄H₂₀N₂O₂ (248.32)
109b) 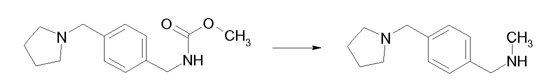 109b wird analog zu 51 b aus 0.61 g (2.44 mMol) Produkt aus 109a und 5.00 ml (5.00 mMol) Lithiumaluminiumhydrid 1 M in THF (Aldrich) in 5 ml THF hergestellt.
   C₁₃H₂₀N₂ (204.31)
109c) 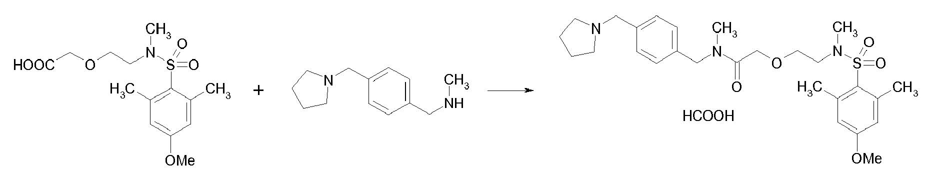 Beispiel 109 wird analog zu 1f aus 0.18 g (0.54 mMol) Produkt aus 53c, 0.17 g (0.81 mMol) Produkt aus 109b, 0.15 ml (1.07 mMol) Triethylamin und 0.21 g (0.65 mMol) TBTU in 4 ml DMF hergestellt.
   C₂₇H₃₉N₃O₅S x CH₂O₂ (631.71)
   [M+H]+ = 518
   HPLC (Methode 5): Retentionszeit = 1.56 min

### Beispiel 110

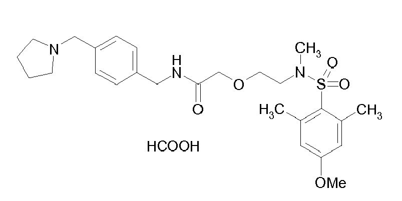

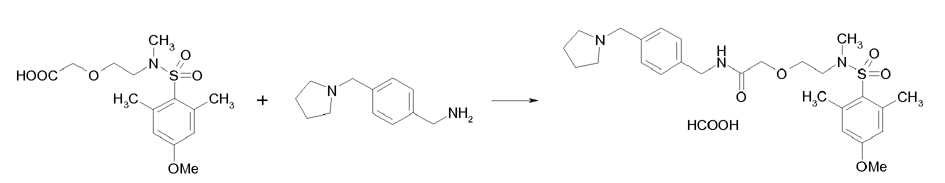

Beispiel 110 wird analog zu 1f aus 0.12 g (0.36 mMol) Produkt aus 53c, 0.096 g (0.51 mMol) 4-Pyrrolidin-1-ylmethyl-benzylamin (Enamine-BB), 0.10 ml (0.72 mMol) Triethylamin und 0.14 g (0.44 mMol) TBTU in 4 ml DMF hergestellt.
C₂₆H₃₇N₃O₅S x CH₂O₂ (617.68)
[M+H]+ = 504
HPLC (Methode 5): Retentionszeit = 1.56 min

### Beispiel 111

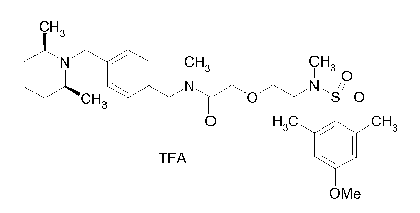

111a) 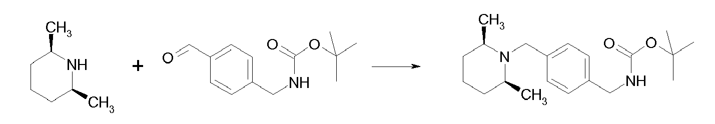 Eine Mischung aus 1.00 g (4.25 mMol) (4-Formylbenzyl)-carbamatsäure-tert-butylester (Acros) und 10 ml Dichlormethan wird unter Eisbadkühlung nacheinander mit 1.15 ml (8.50 mMol) cis-2,6-Dimethylpiperidin (Aldrich) und 1.80 g (8.50 mMol) Natriumtriacetoxyborhydrid versetzt. Die Reaktionsmischung wird drei Tage bei Raumtemperatur gerührt, danach langsam mit gesättigter Natriumhydrogencarbonat-Lösung gequencht und mit Dichlormethan extrahiert. Die organischen Extrakte werden mit Wasser und gesättigter Natriumchlorid-Lösung gewaschen, über Natriumsulfat getrocknet und im Vakuum bis zur Trockene eingeengt. Das so erhaltene Rohprodukt wird durch Säulenchromatographie (Elutionsmittel: Dichlormethan / Methanol 93:7) gereinigt.
   C₂₀H₃₂N₂O₂ (332.48)
   [M+H]+ = 333
   HPLC (Methode 5): Retentionszeit = 1.47 min
111b) 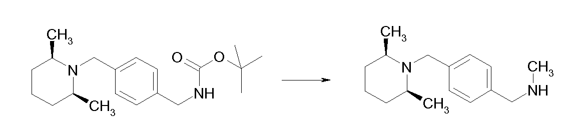 111 b wird analog zu 51 b aus 0.92 g (2.76 mMol) Produkt aus 111 a und 8.27 ml (8.27 mMol) Lithiumaluminiumhydrid 1 M in THF (Aldrich) in 30 ml THF hergestellt.
   C₁₆H₂₆N₂ (246.39)
   [M+H]+ = 247
   HPLC (Methode 5): Retentionszeit = 1.03 min
111c) 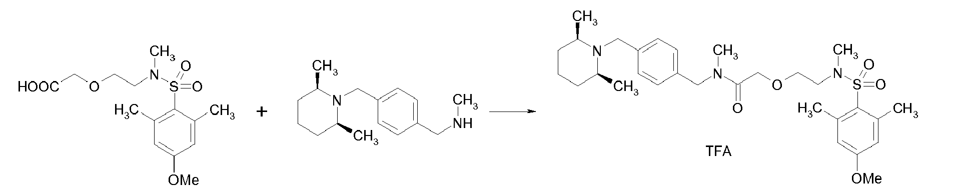 Beispiel 111 wird analog zu 1f aus 0.08 g (0.24 mMol) Produkt aus 53c, 0.059 g (0.24 mMol) Produkt aus 111 b, 0.10 ml (0.72 mMol) Triethylamin und 0.078 g (0.24 mMol) TBTU in 2 ml THF hergestellt.
   C₃₀H₄₅N₃O₅S x C₂HF₃O₂ (673.78)
   [M+H]+ = 560
   HPLC (Methode 5): Retentionszeit = 1.59 min

### Beispiel 112

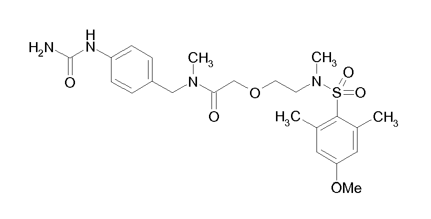

112a) 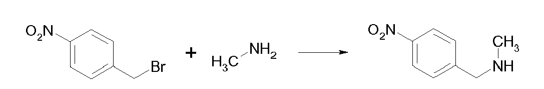 250.00 ml (500.00 mMol) Methylamin 2 M in Methanol (Fluka) werden unter Eisbadkühlung langsam mit 54.00 g (250.00 mMol) 4-Nitrobenzylbromid (Fluka) versetzt. Die Reaktionsmischung wird eine Stunde unter Eisbadkühlung und 30 Minuten bei Raumtemperatur gerührt und danach im Vakuum bis zur Trockene eingeengt. Der Rückstand wird mit Diethylether verrührt und abfiltriert. Das Filtrat wird im Vakuum bis zur Trockene eingeengt, mit Natriumcarbonat-Lösung versetzt und mit Diethylether extrahiert. Die organischen Extrakte werden über Natriumsulfat getrocknet und im Vakuum bis zur Trockene eingeengt. Das so erhaltene Rohprodukt wird durch Säulenchroamtographie (Elutionsmittel: Dichlormethan / Methanol 5:0 bis 5:1) gereinigt.
   C₈H_{1O}N₂O₂ (166.18)
   [M+H]+ = 167
112b) 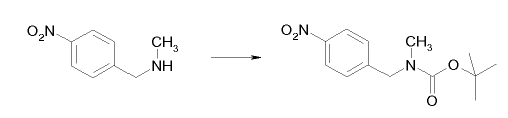 Eine Mischung aus 13.40 g (80.64 mMol) Produkt aus 112a und 25 ml Ethylacetat wird unter Eisbadkühlung langsam mit 17.68 g (81.00 mMol) Boc-Anhydrid versetzt. Die Reaktionsmischung wird drei Stunden bei Raumtemperatur gerührt, danach mit Wasser gewaschen, über Natriumsulfat getrocknet und im Vakuum bis zur Trockene eingeengt.
   C₁₃H₁₈N₂O₄ (266.29)
112c) 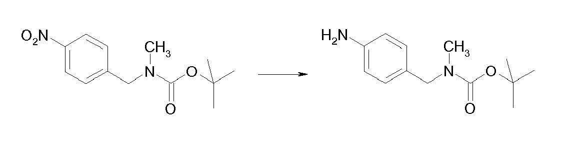 Eine Mischung aus 23.00 g (86.37 mMol) Produkt aus 112b, 2.30 g Raney-Nickel, 230 ml Ethanol und 230 ml Ethylacetat wird im Autoklaven bei Raumtemperatur hydriert. Der
   Katalysator wird abfiltriert, das Filtrat im Vakuum bis zur Trockene eingeengt. Das so erhaltene Rohprodukt wird durch Säulenchromatographie (Elutionsmittel: Petrolether / Ethylacetat 1:1) gereinigt.
   C₁₃H₂₀N₂O₂ (236.31)
   [M+H]+ = 237
   DC: Kieselgel, Petrolether / Ethylacetat 1:1, Rf-Wert = 0.55
112d) 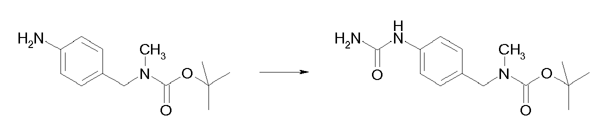 Eine Mischung aus 0.50 g (2.12 mMol) Produkt aus 112c, 0.48 g (3.56 mMol) Trimethylsilylisocyanat (Fluka) und 15 ml THF wird über das Wochenende unter Rückfluss gerührt. Anschließend wird die Reaktionsmischung im Vakuum bis zur Trockene eingeengt.
   C₁₄H₂₁N₃O₃ (279.33)
   [2M+H]+ = 559
112e) 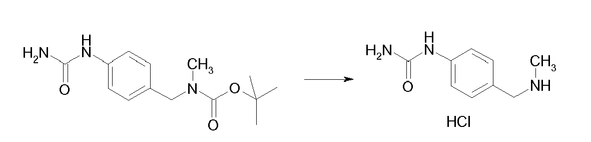 Eine Mischung aus 0.64 g (2.29 mMol) Produkt aus 112d und 10 ml methanolische HCl wird drei Stunden bei Raumtemperatur und zwei Stunden bei 50°C gerührt. Anschließend wird die Reaktionsmischung im Vakuum bis zur Trockene eingeengt. Der Rückstand wird im Vakuum über Nacht getrocknet.
   C₉H₁₃N₃ x HCl (215.68)
   [M+H]+ = 180
112f) 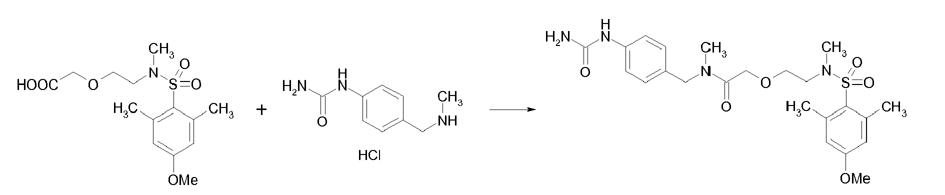 Beispiel 112 wird analog zu 1f aus 0.20 g (0.60 mMol) Produkt aus 53c, 0.13 g (0.60 mMol) Produkt aus 112e, 0.42 ml (3.02 mMol) Triethylamin und 0.22 g (0.66 mMol) TBTU in 4 ml DMF hergestellt.
   C₂₃H₃₂N₄O₆S (492.59)
   [M+H]+ = 493
   HPLC (Methode 6): Retentionszeit = 2.93 min

### Beispiel 113

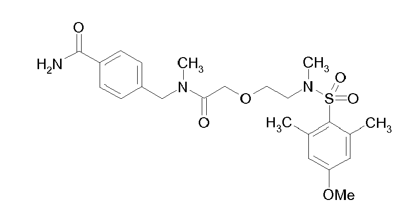

113a) 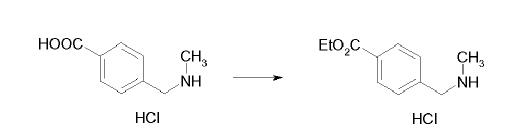 Eine Mischung aus 2.00 g (9.92 mMol) 4-Methylaminomethyl-benzoesäure Hydrochlorid (J. Med. Chem. 26, 1983, 309-312) und 25 ml ethanolischer HCl wird 1.5 Stunden unter Rückfluß gerührt. Danach wird die Reaktionsmischung im Vakuum bis zur Trockene eingeengt.
   C₁₁H₁₅NO₂ x HCl (229.70)
   [M+H]+ = 194
   HPLC (Methode 6): Retentionszeit = 1.39 min
113b) 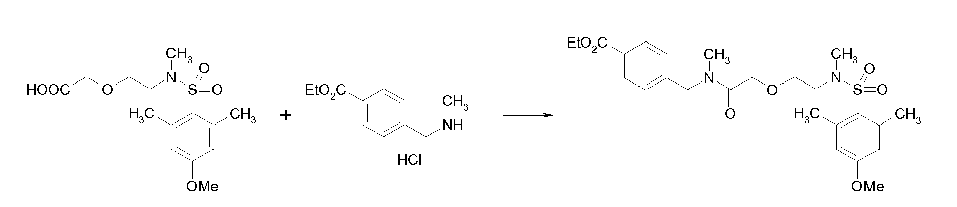 113b wird analog zu 1f aus 0.70 g (2.11 mMol) Produkt aus 53c, 0.49 g (2.11 mMol) Produkt aus 113a, 0.88 ml (6.34 mMol) Triethylamin und 0.78 g (2.32 mMol) TBTU in 12 ml DMF hergestellt.
   C₂₅H₃₄N₂O₇S (506.61)
   [M+H]+ = 507
   HPLC (Methode 6): Retentionszeit = 3.95 min
113c) 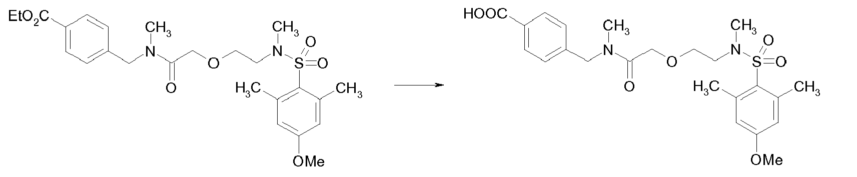 Eine Mischung aus 1.06 g (2.09 mMol) Produkt aus 133b, 7.00 ml (7.00 mMol) 1 M Natronlauge, 15 ml THF und 1.5 ml Ethanol wird vier Stunden bei 50°C gerührt. Die Reaktionsmischung wird danach mit 7 ml 1 M HCl versetzt und im Vakuum bis zur Trockene eingeengt. Der Rückstand wird in Aceton aufgenommen, über Magnesiumsulfat getrocknet und im Vakuum bis zur Trockene eingeengt.
   C₂₃H₃₀N₂O₇S (478.56)
   [M+H]+ = 479
   HPLC (Methode 6): Retentionszeit = 3.21 min
113d) 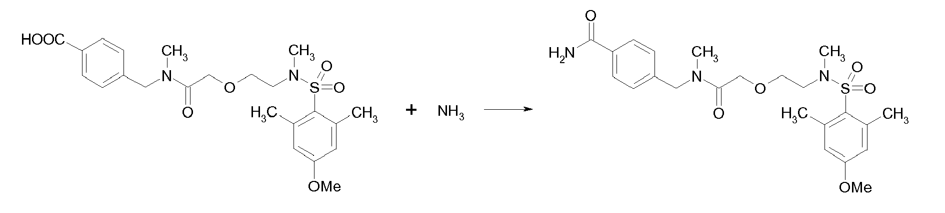 Beispiel 113 wird analog zu 1f aus 0.50 g (1.05 mMol) Produkt aus 113c, 4.00 ml (2.00 mMol) Ammoniak 0.5 M in Dioxan, 0.44 ml (3.14 mMol) Triethylamin und 0.38 g (1.15 mMol) TBTU in 4 ml DMF hergestellt.
   C₂₃H₃₁N₃O₆S (477.57)
   [M+H]+ = 478
   HPLC (Methode 6): Retentionszeit = 2.92 min

### Beispiel 114

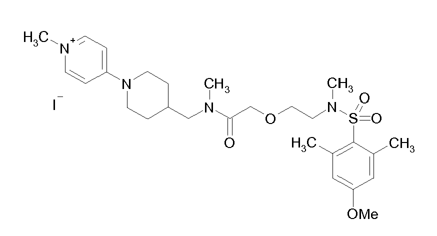

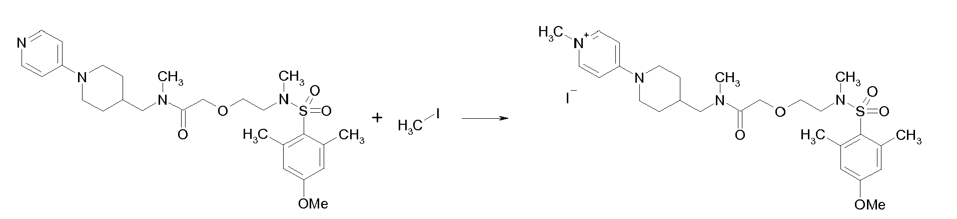

Eine Mischung aus 0.075 g (0.15 mMol) 61, 0.1 g (0.71 mMol) Methyliodid und 5 ml Dichlormethan wird über Nacht bei Raumtemperatur gerührt. Die Reaktionsmischung wird danach im Vakuum bis zur Trockene eingeengt.
C₂₇H₄₁N₄O₅S x I (660.61)
[M+H]+ = 533
HPLC (Methode 5): Retentionszeit = 1.55 min

### Beispiel 115

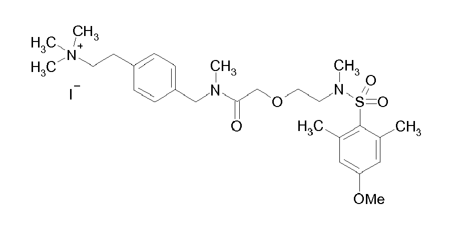

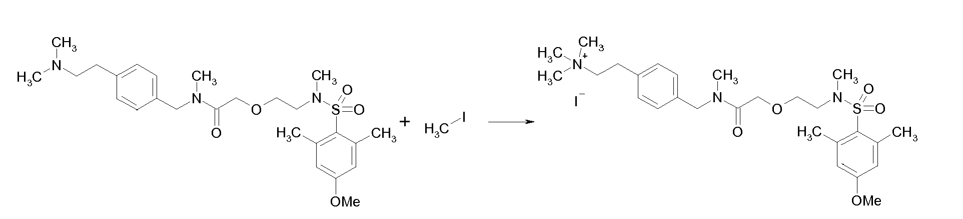

Beispiel 115 wird analog zu 114 aus 0.03 g (0.059 mMol) 64 und 0.05 g (0.35 mMol) Methyliodid in 5 ml Dichlormethan hergestellt.
C₂₇H₄₂N₃O₅S x I (647.61)
[M+H]+ = 520
HPLC (Methode 5): Retentionszeit = 1.55 min

### Beispiel 116

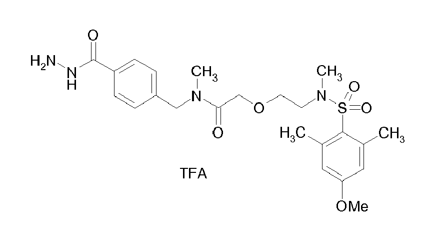

116a) 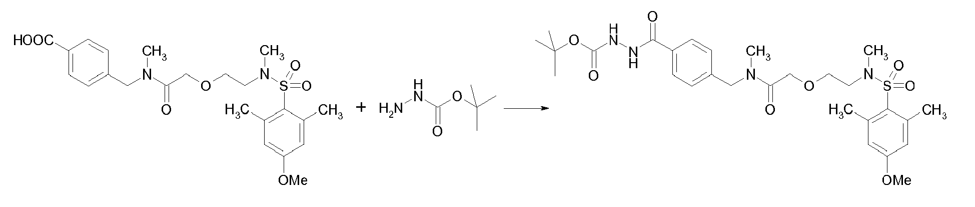 116a wird analog zu 1f aus 0.50 g (1.05 mMol) Produkt aus 113c, 0.14 g (1.05 mMol) Hydrazincarbonsäure-tert-butylester (Aldrich), 0.58 ml (4.18 mMol) Triethylamin und 0.38 g (1.15 mMol) TBTU in 6 ml DMF hergestellt.
   C₂₈H₄₀N₄O₈S (592.71)
   [M+H]+ = 593
   HPLC (Methode 6): Retentionszeit = 3.46 min
116b) 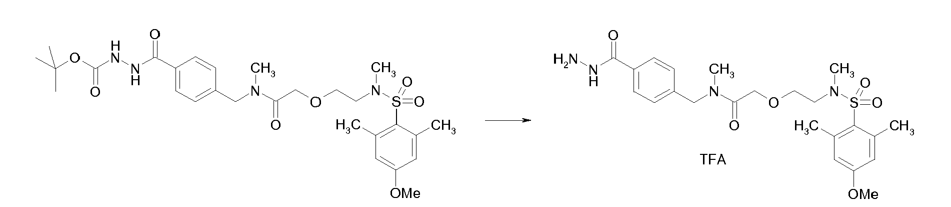 Beispiel 116 wird analog zu 112e aus 0.68 g (1.15 mMol) Produkt aus 116a und 10 ml methanolischer HCl hergestellt.
   C₂₃H₃₂N₄O₆S x C₂HF₃O₂ (606.61)
   [M+H]+ = 493
   HPLC (Methode 6): Retentionszeit = 3.46 min

### Beispiel 117

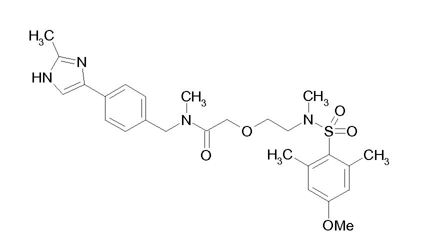

117a) 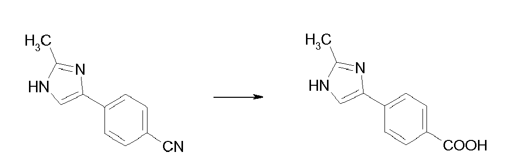 Eine Mischung aus 3.20 g (17.47 mMol) Produkt aus 63a, 50 ml 20%iger Natronlauge und 50 ml Ethanol wird über Nacht unter Rückfluss gerührt. Danach wird das Ethanol im Vakuum entfernt und der wässrige Rückstand mit konzentrierter HCl neutralisiert. Der entstandene Niederschlag wird abfiltriert und getrocknet.
   C₁₁H₁₀N₂O₂ (202.21)
   [M+H]+ = 203
117b) 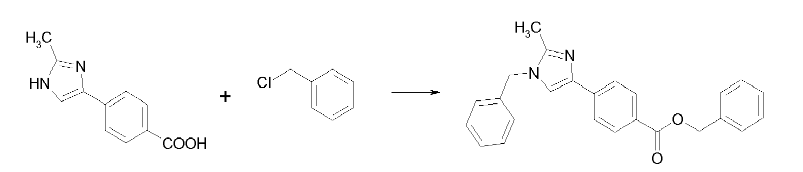 Eine Mischung aus 2.70 g (13.35 mMol) Produkt aus 117a, 8.00 g (57.89 mMol) Kaliumcarbonat und 100 ml DMF wird eine Stunde bei 60°C gerührt. Nach dem Abkühlen wird die Reaktionsmischung mit 3.50 g (27.65 mMol) Benzylchlorid (Aldrich) bei Raumtemperatur versetzt und anschließend über das Wochenende bei 60°C gerührt. Die Reaktionsmischung wird auf Wasser gegossen und eine Stunde bei Raumtemperatur gerührt. Der entstandene Niederschlag wird abfiltriert und getrocknet.
   C₂₅H₂₂N₂O₂ (382.45)
   [M+H]+ = 383
   DC: Kieselgel, Dichlormethan / Methanol 9:1, Rf-Wert = 0.64
117c) 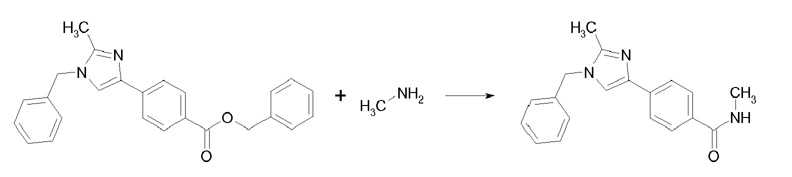 Eine Mischung aus 1.80 g (4.71 mMol) Produkt aus 117b und 100 ml Methylamin 33% in Ethanol (Aldrich) wird sechs Stunden bei 180°C und über Nacht bei 160°C im Autoklaven gerührt. Die Reaktionsmischung wird danach im Vakuum bis zur Trockene eingeengt. Das so erhaltene Rohprodukt wird durch Säulen-chromatographie (Elutionsmittel:
   Dichlormethan / Methanol 19:1) gereinigt.
   C₁₉H₁₉N₃O (305.37)
   [M+H]+ = 306
117d) 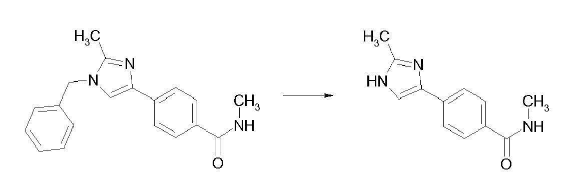 Eine Mischung aus 1.20 g (3.93 mMol) Produkt aus 117c, 0.20 g Palladium auf Kohle (20%) und 50 ml Methanol wird bei 50°C im Autoklaven hydriert. Der Katalysator wird abfiltriert, das Filtrat im Vakuum bis zur Trockene eingeengt.
   C₁₂H₁₃N₃O (215.25)
   [M+H]+ = 216
117e) 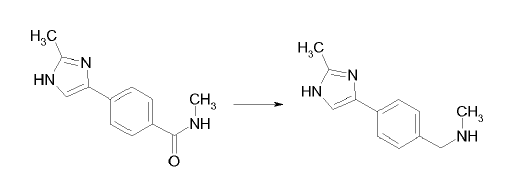 117e wird analog zu 38f aus 0.70 g (3.25 mMol) Produkt aus 117d und 10.00 ml (10.00 mMol) Lithiumaluminiumhydrid 1 M in THF (Aldrich) in 200 ml THF hergestellt.
   C₁₂H₁₅N₃ (201.27)
   [M+H]+ = 202
117f) 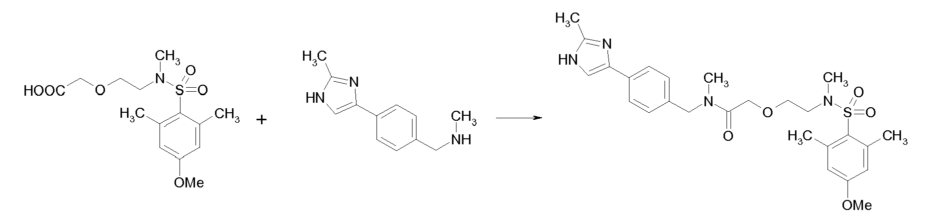 Beispiel 117 wird analog zu 1f aus 0.17 g (0.51 mMol) Produkt aus 53c, 0.10 g (0.50 mMol) Produkt aus 117e, 0.17 ml (1.19 mMol) Triethylamin und 0.17 g (0.53 mMol) TBTU in 30 ml THF und 5 ml DMF hergestellt.
   C₂₆H₃₄N₄O₅S (514.64)
   [M+H]+ = 515
   DC: Kieselgel, Dichlormethan / Methanol 9:1, Rf-Wert = 0.28

### Beispiel 118

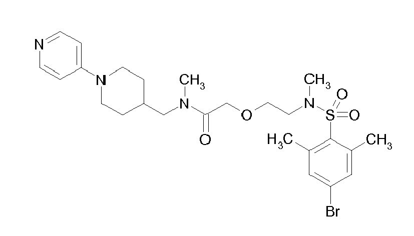

118a) 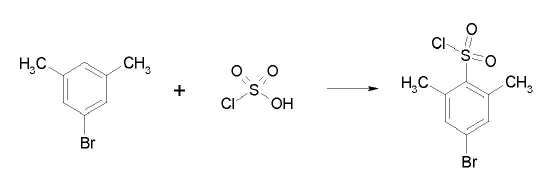 118a wird analog zu 13a aus 0.75 g (4.05 mMol) 5-Brom-m-xylol (Aldrich), 0.55 ml (8.30 mMol) Chlorsulfonsäure (Aldrich) und 10 ml Dichlormethan hergestellt.
   C₈H₈BrClO₂S (283.57)
   HPLC (Methode 6): Retentionszeit = 4.76 min
118b) 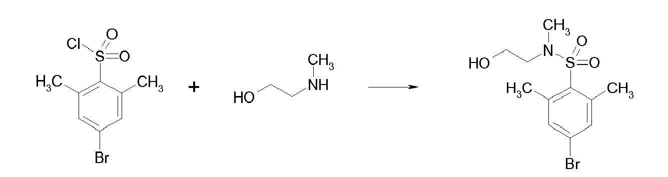 118b wird analog zu 3a aus 0.65 g (2.29 mMol) Produkt aus 118a und 0.28 ml (3.44 mMol) N-Methylaminoethanol (BASF) in 5 ml THF hergestellt.
   C₁₁H₁₆BrNO₃S (322.22)
   HPLC (Methode 6): Retentionszeit = 3.38 min
118c) 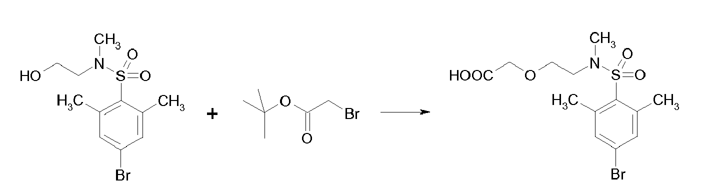 118c wird zuerst analog zu 53b aus 0.74 g (2.29 mMol) Produkt aus 118b, 0.75 g (5.06 mMol) 2-Brompropionsäure-tert-butylester (Fluka), 0.42 g (1.14 mMol) Tetrabutylammoniumiodid (Aldrich) und 8.67 g (75.90 mMol) 35%iger Natronlauge in 40 ml Toluol hergestellt. Der tert-Butylester wird anschließend zusammen mit 2 ml HCl 4 M in Dioxan (Aldrich) in 4 ml Dioxan über Nacht bei Raumtemperatur gerührt. Das Produkt wird danach durch Einengen der Reaktionsmischung im Vakuum erhalten.
   C₁₃H₁₈BrNO₅S (380.26)
   HPLC (Methode 6): Retentionszeit = 3.48 min
118d) 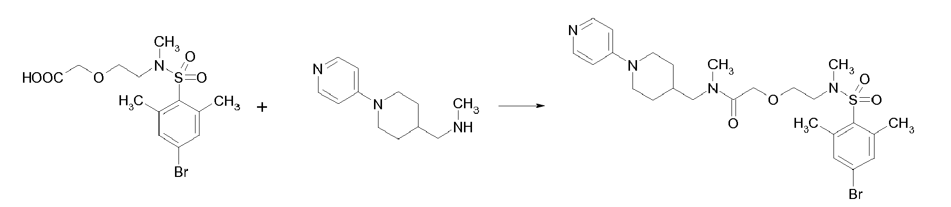 Beispiel 118 wird analog zu 1f aus 0.10 g (0.26 mMol) Produkt aus 118c, 0.054 g (0.26 mMol) Produkt aus 61 b, 0.11 ml (0.79 mMol) Triethylamin und 0.084 g (0.26 mMol) TBTU in 10 ml THF und 3 ml DMF hergestellt.
   C₂₅H₃₅BrN₄O₄S (567.54)
   [M+H]+ = 568/569/571
   HPLC (Methode 6): Retentionszeit = 2.77 min

### Beispiel 119

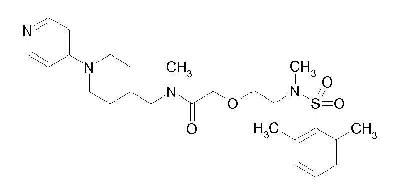

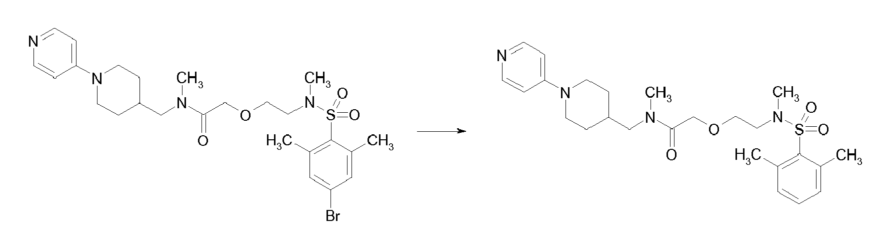

Eine Mischung aus 0.03 g (0.053 mMol) 118 und 0.03 g Palladium auf Kohle in 5 ml Methanol wird im Autoklaven bei Raumtemperatur hydriert. Der Katalysator wird abfiltriert, das Filtrat im Vakuum bis zur Trockene eingeengt. Das so erhaltene Rohprodukt wird durch präparative HPLC gereinigt.
C₂₅H₃₆N₄O₄S (488.64)
[M+H]+ = 489
HPLC (Methode 6): Retentionszeit = 2.45 min

### Beispiel 120

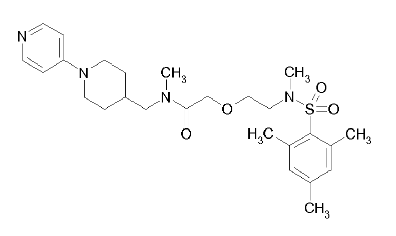

120a) 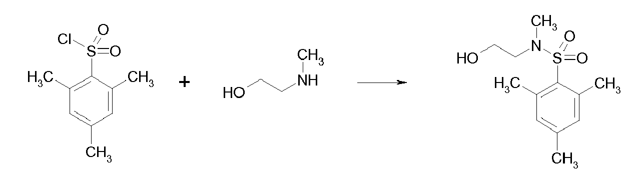 120a wird analog zu 3a aus 0.50 g (2.29 mMol) 2,4,6-Trimethylbenzol-sulfonsäurechlorid (Fluka) und 0.19 g (2.52 mMol) N-Methylaminoethanol (BASF) in 5 ml THF hergestellt.
   C₁₂H₁₉NO₃S (257.35)
   [M+H]+ = 258
120b) 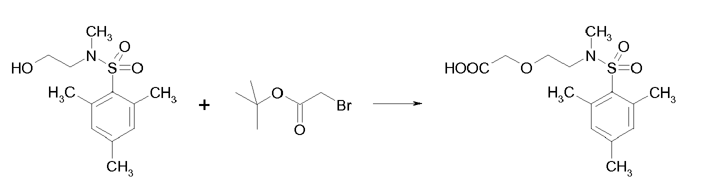 120b wird analog zu 118c aus 0.56 g (2.18 mMol) Produkt aus 120a, 0.48 ml (3.26 mMol) 2-Brompropionsäure-tert-butylester (Fluka), 0.18 g (0.65 mMol) Tetrabutylammoniumchlorid (Fluka) und 7.46 g (65.28 mMol) 35%iger Natronlauge in 20 ml Toluol und anschließendes Rühren in 2 ml HCl 4 M in Dioxan hergestellt.
   C₁₄H₂₁NO₅S (315.39)
120c) 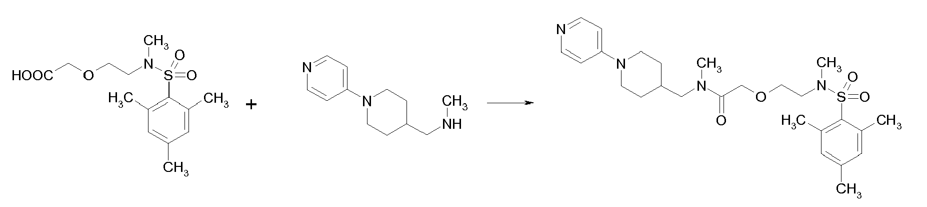 Beispiel 120 wird analog zu 1f aus 0.10 g (0.32 mMol) Produkt aus 120b, 0.065 g (0.32 mMol) Produkt aus 61 b, 0.13 ml (0.95 mMol) Triethylamin und 0.20 g (0.63 mMol) TBTU in 10 ml THF hergestellt.
   C₂₆H₃₈N₄O₄S (502.67)
   [M+H]+ = 503
   HPLC (Methode 5): Retentionszeit = 1.57 min

### Beispiel 121

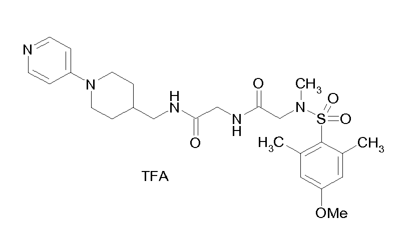

121a) 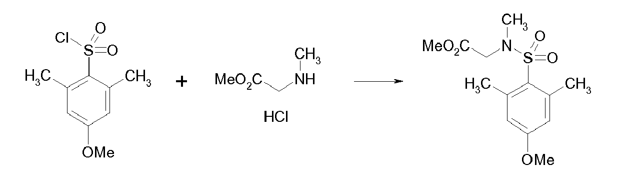 Eine Mischung aus 0.98 g (7.05 mMol) Sarcosin Methylester Hydrochlorid (Fluka), 1.65 g (7.05 mMol) Produkt aus 13a und 50 ml Pyridin wird eine Stunde bei Raumtemperatur gerührt. Die Reaktionsmischung wird danach im Vakuum bis zur Trockene eingeengt. Der Rückstand wird danach in 1 M HCl aufgenommen und mit Ethylacetat extrahiert. Die organischen Extrakte werden über Natriumsulfat getrocknet und im Vakuum bis zur Trockene eingeengt.
   C₁₃H₁₉NO₅S (301.36)
   [M+H]+ = 302
121b) 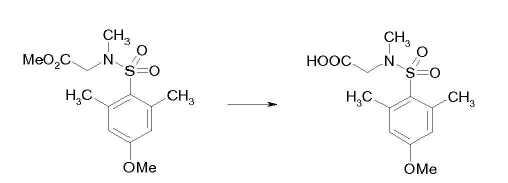 Eine Mischung aus 1.90 g (6.29 mMol) Produkt aus 121a, 6.45 ml (12.90 mMol) 2 M Natronlauge und 9 ml Methanol wird drei Tage bei Raumtemperatur gerührt. Das Methanol wird im Vakuum entfernt, der wässrige Rückstand wird auf 1 M HCl gegossen. Der entstandene Niederschlag wird abfiltriert und im Vakuumexsikkator über Nacht getrocknet.
   C₁₂H₁₇NO₅S (287.33)
   [M+H]+ = 288
121c) 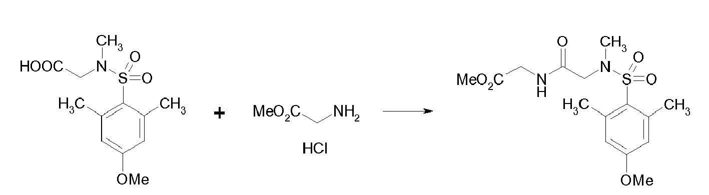 121c wird analog zu 1f aus 0.20 g (0.70 mMol) Produkt aus 121b, 0.087 g (0.70 mMol) Glycin Methylester Hydrochlorid (Aldrich), 0.29 ml (2.09 mMol) Triethylamin und 0.22 g (0.70 mMol) TBTU in 5 ml THF hergestellt.
   C₁₅H₂₂N₂O₆S (358.41)
   [M+H]+ = 359
   HPLC (Methode 5): Retentionszeit = 1.72 min
121d) 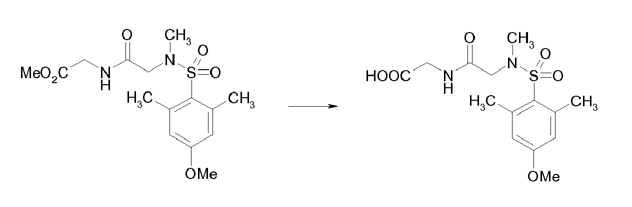 121d wird analog zu 121 b aus 0.23 g (0.63 mMol) Produkt aus 121 c und 0.64ml (1.29 mMol) 2 M Natronlauge in 1 ml Methanol hergestellt.
   C₁₄H₂₀N₂O₆S (344.38)
   [M+H]+ = 345
121e) 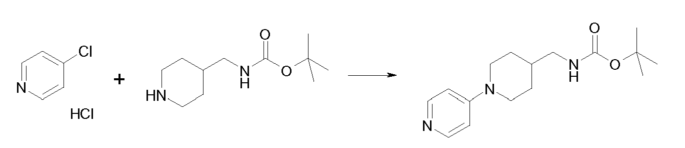 121 e wird analog zu 28c aus 3.00 g (14.00 mMol) Piperidin-4-ylmethyl-carbamatsäure-tert-butylester (EMKA), 2.10 g (14.00 mMol) 4-Chlorpyridin Hydrochlorid (Aldrich) und 7.80 ml (56.32 mMol) Triethlamin in 15 ml Isopropanol hergestellt.
   C₁₆H₂₅N₃O₂ (291.39)
   [M+H]+ = 292
   HPLC (Methode 5): Retentionszeit = 1.40 min
121f) 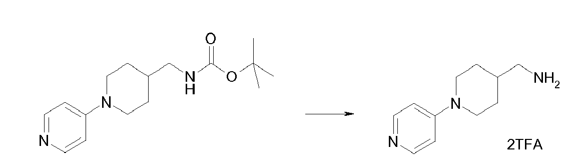 121f wird analog zu 18b aus 1.44 g (4.95 mMol) Produkt aus 121e und 4.95 ml TFA in 8 ml Dichlormethan hergestellt.
   C₁₁H₁₇N₃ x 2C₂HF₃O₂ (419.32)
   [M+H]+ = 192
   HPLC (Methode 5): Retentionszeit = 0.36 min
121g) 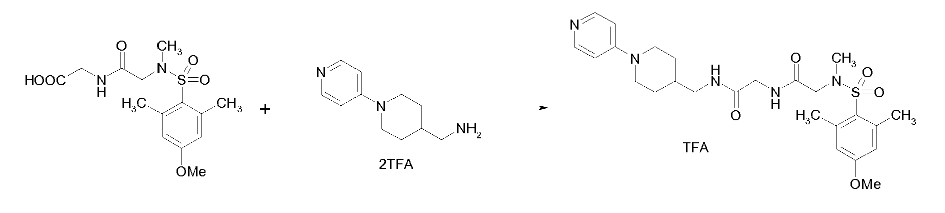 Beispiel 121 wird analog zu 1f aus 0.09 g (0.26 mMol) Produkt aus 121d, 0.11 g (0.26 mMol) Produkt aus 121f, 0.15 ml (1.05 mMol) Triethylamin und 0.084 g (0.26 mMol) TBTU in 1.9 ml THF hergestellt.
   C₂₅H₃₅N₅O₅S x C₂HF₃O₂ (631.67)
   [M+H]+ = 518
   HPLC (Methode 5): Retentionszeit = 1.46 min

### Beispiel 122

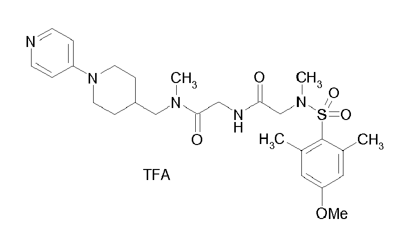

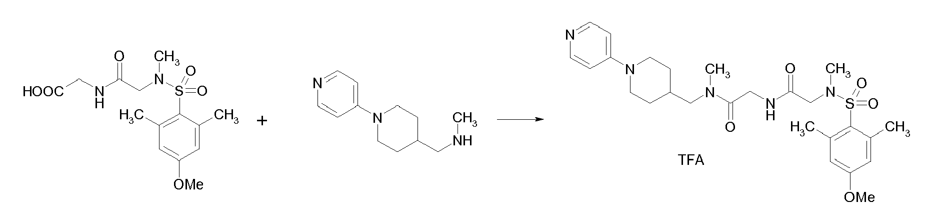

Beispiel 122 wird analog zu 1f aus 0.09 g (0.26 mMol) Produkt aus 121d, 0.054 g (0.26 mMol) Produkt aus 61 b, 0.11 ml (0.78 mMol) Triethylamin und 0.084 g (0.26 mMol) TBTU in 1.9 ml THF hergestellt.
C₂₆H₃₇N₅O₅S x C₂HF₃O₂ (645.69)
[M+H]+ = 532
HPLC (Methode 5): Retentionszeit = 1.50 min

### Beispiel 123

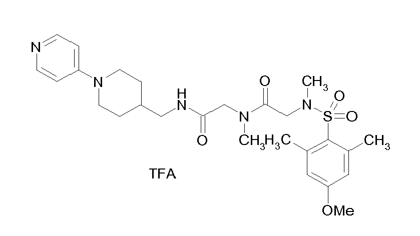

123a) 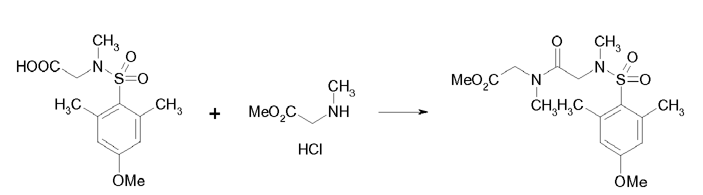 123a wird analog zu 1f aus 0.20 g (0.70 mMol) Produkt aus 121 b, 0.097 g (0.70 mMol) Sarcosin Methylester Hydrochlorid (Fluka), 0.29 ml (2.09 mMol) Triethylamin und 0.22 g (0.70 mMol) TBTU in 5 ml THF hergestellt.
   C₁₆H₂₄N₂O₆S (372.44)
   [M+H]+ = 373
   HPLC (Methode 5): Retentionszeit = 1.78 min
123b) 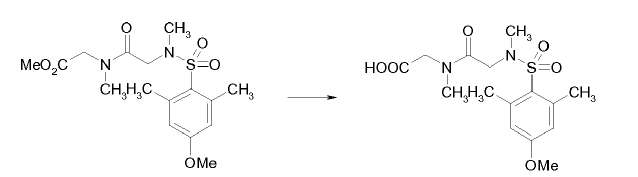 123b wird analog zu 121 b aus 0.23 g (0.60 mMol) Produkt aus 123a und 0.62 ml (1.24 mMol) 2 M Natronlauge in 1 ml Methanol hergestellt.
   C₁₅H₂₂N₂O₆S (258.41)
   [M+H]+ = 359
123c) 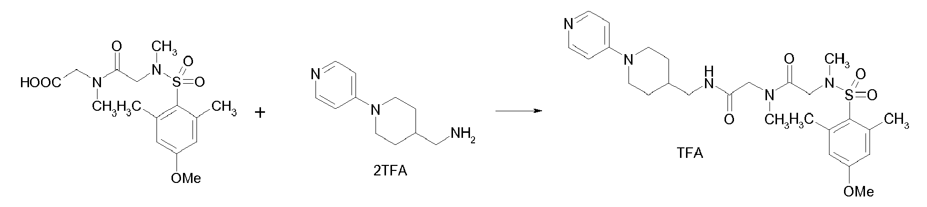 Beispiel 123 wird analog zu 1f aus 0.094 g (0.26 mMol) Produkt aus 123b, 0.11 g (0.26 mMol) Produkt aus 121f, 0.15 ml (1.05 mMol) Triethylamin und 0.084 g (0.26 mMol) TBTU in 1.9 ml THF hergestellt.
   C₂₆H₃₇N₅O₅S x C₂HF₃O₂ (645.69)
   [M+H]+ = 532
   HPLC (Methode 5): Retentionszeit = 1.47 min

### Beispiel 124

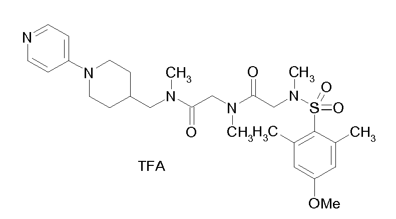

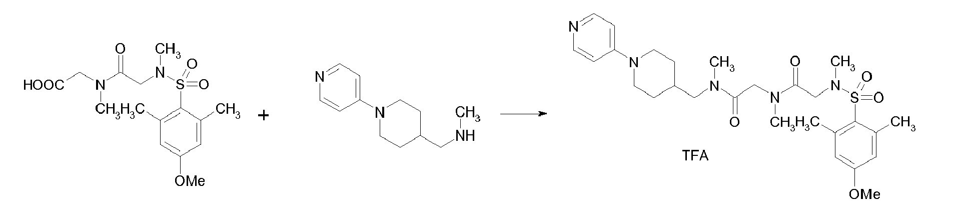

Beispiel 124 wird analog zu 1f aus 0.094 g (0.26 mMol) Produkt aus 123b, 0.054 g (0.26 mMol) Produkt aus 61 b, 0.11 ml (0.78 mMol) Triethylamin und 0.084 g (0.26 mMol) TBTU in 1.9 ml THF hergestellt.
C₂₇H₃₉N₅O₅S x C₂HF₃O₂ (659.72)
[M+H]+ = 548
HPLC (Methode 5): Retentionszeit = 1.49 min

### Beispiel 125

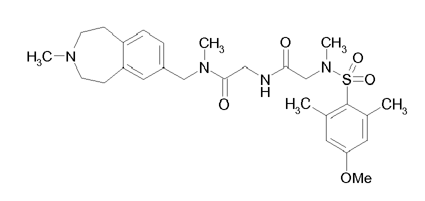

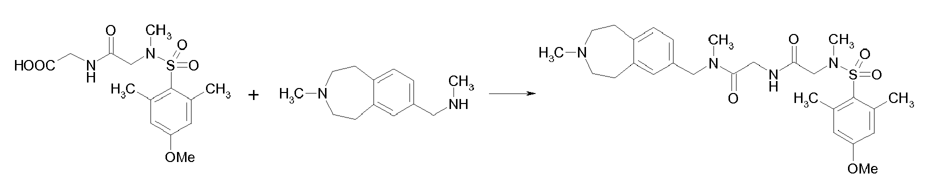

Beispiel 125 wird analog zu 1f aus 0.09 g (0.26 mMol) Produkt aus 121d, 0.076 g (0.26 mMol) Produkt aus 54b, 0.11 ml (0.78 mMol) Triethylamin und 0.084 g (0.26 mMol) TBTU in 1.9 ml THF hergestellt.
C₂₇H₃₈N₄O₅S (530.68)
[M+H]+ = 531
HPLC (Methode 1): Retentionszeit = 2.43 min

### Beispiel 126

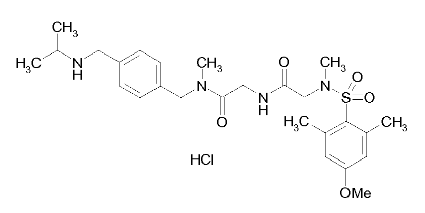

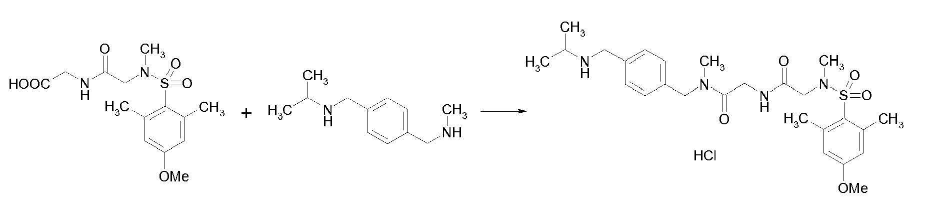

Beispiel 126 wird analog zu 1f aus 0.09 g (0.26 mMol) Produkt aus 121d, 0.05 g (0.26 mMol) Produkt aus 59b, 0.11 ml (0.78 mMol) Triethylamin und 0.084 g (0.26 mMol) TBTU in 1.9 ml THF hergestellt.
C₂₆H₃₈N₄O₅S x HCl (555.13)
[M+H]+ = 519
HPLC (Methode 1): Retentionszeit = 2.42 min

### Beispiel 127

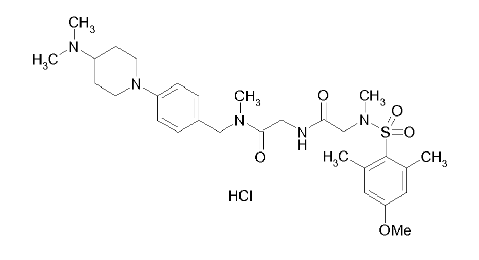

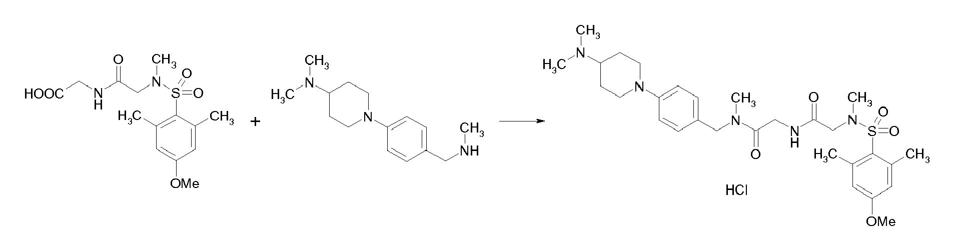

Beispiel 127 wird analog zu 1f aus 0.09 g (0.26 mMol) Produkt aus 121d, 0.065 g (0.26 mMol) Produkt aus 80a, 0.11 ml (0.78 mMol) Triethylamin und 0.084 g (0.26 mMol) TBTU in 1.9 ml THF hergestellt.
C₂₉H₄₃N₅O₅S x HCl (610.21)
[M+H]+ = 574
HPLC (Methode 1): Retentionszeit = 2.38 min

### Beispiel 128

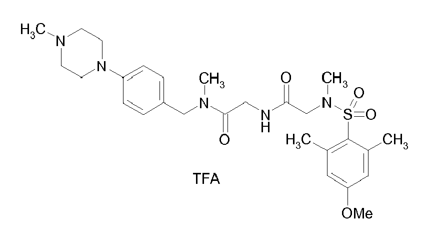

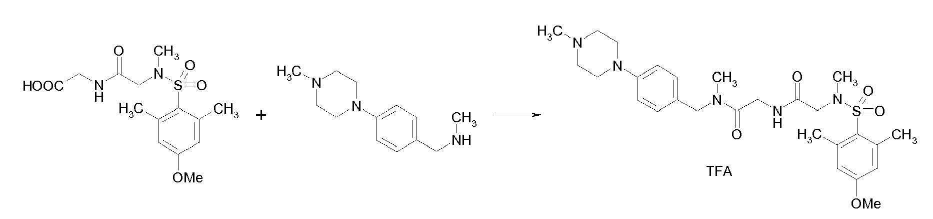

Beispiel 128 wird analog zu 1f aus 0.09 g (0.26 mMol) Produkt aus 121d, 0.057 g (0.26 mMol) Produkt aus 81 a, 0.11 ml (0.78 mMol) Triethylamin und 0.084 g (0.26 mMol) TBTU in 1.9 ml THF hergestellt.
C₂₇H₃₉N₅O₅S x C₂HF₃O₂ (610.21)
[M+H]+ = 546
HPLC (Methode 5): Retentionszeit = 1.51 min

### Beispiel 129

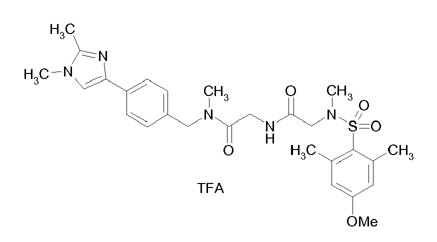

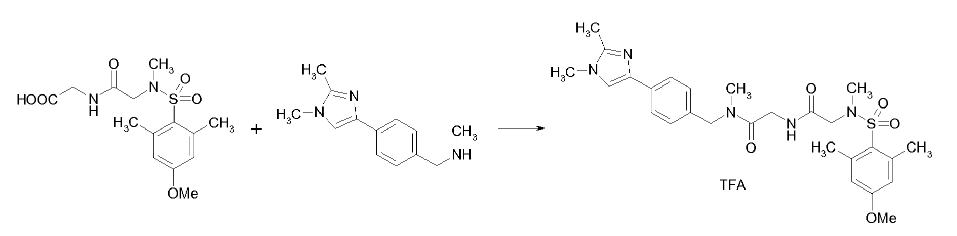

Beispiel 129 wird analog zu 1f aus 0.09 g (0.26 mMol) Produkt aus 121d, 0.056 g (0.26 mMol) Produkt aus 67c, 0.11 ml (0.78 mMol) Triethylamin und 0.084 g (0.26 mMol) TBTU in 1.9 ml THF hergestellt.
C₂₇H₃₅N₅O₅S x C₂HF₃O₂ (655.69)
[M+H]+ = 542
HPLC (Methode 5): Retentionszeit = 1.52 min

### Beispiel 130

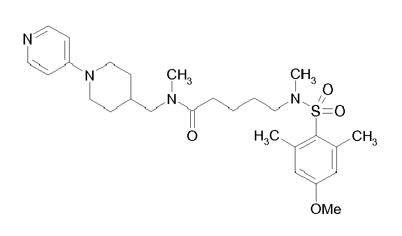

130a) 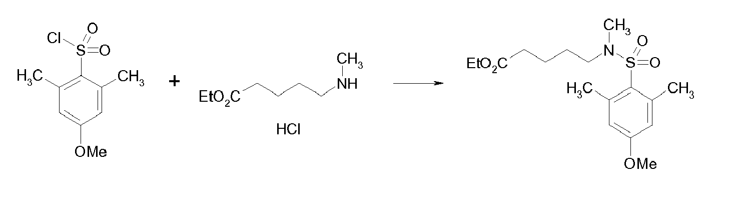 130a wird analog zu 10d aus 0.50 g (2.13 mMol) Produkt aus 13a, 0.42 g (2.13 mMol) 5-Methylaminovaleriansäureethylester (J. Am. Chem. Soc. 55, 1933, 1233-1241) und 1.18 ml (8.52 mMol) Triethylamin in 15 ml THF hergestellt.
   C₁₇H₂₇NO₅S (357.47)
   [M+H]+ = 358
   HPLC (Methode 6): Retentionszeit = 4.10 min
130b) 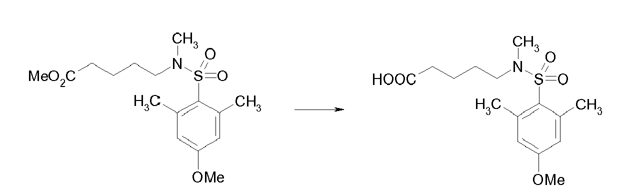 130b wird analog zu 121 b aus 0.62 g (1.73 mMol) Produkt aus 130a und 7.00 ml (7.00 mMol) 1 M Natronlauge in 1.5 ml Methanol und 15 ml THF hergestellt.
   C₁₅H₂₃NO₅S (329.41)
   [M+H]+ = 330
   HPLC (Methode 6): Retentionszeit = 3.24 min
130c) 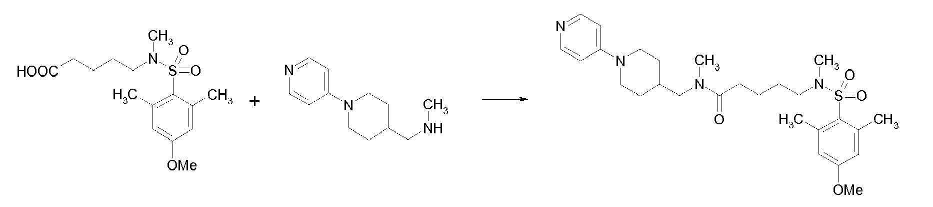 Beispiel 130 wird analog zu 1f aus 0.18 g (0.54 mMol) Produkt aus 130b, 0.10 g (0.49 mMol) Produkt aus 61 b, 0.14 ml (0.97 mMol) Triethylamin und 0.18 g (0.54 mMol) TBTU in 3 ml DMF hergestellt.
   C₂₇H₄₀N₄O₄S (516.70)
   [M+H]+ = 517
   HPLC (Methode 6): Retentionszeit = 2.65 min

### Beispiel 131

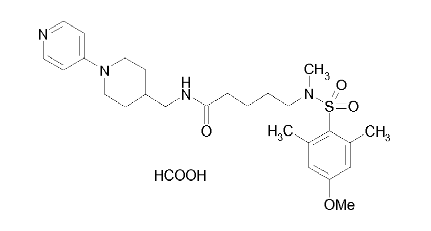

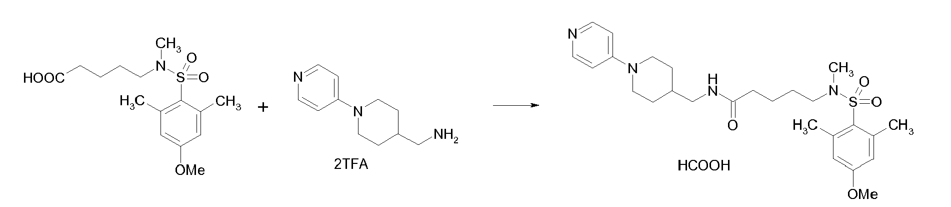

Beispiel 131 wird analog zu 1f aus 0.069 g (0.21 mMol) Produkt aus 130b, 0.088 g (0.21 mMol) Produkt aus 121f, 0.087 ml (0.63 mMol) Triethylamin und 0.077 g (0.23 mMol) TBTU in 1 ml DMF hergestellt.
C₂₆H₃₈N₄O₄S x CH₂O₂ (548.71)
[M+H]+ = 503
HPLC (Methode 6): Retentionszeit = 2.52 min

### Beispiel 132

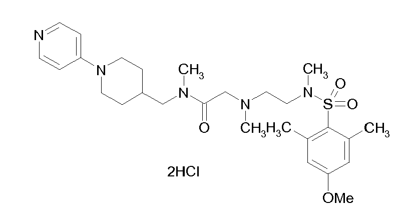

132a) 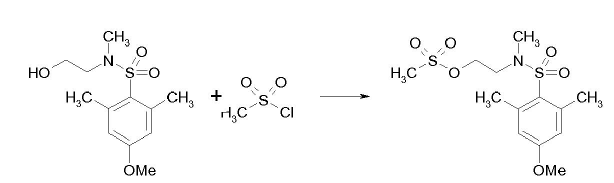 Eine Mischung aus 2.50 g (9.15 mMol) Produkt aus 53a, 1.39 ml (10.00 mMol) Triethylamin und 50 ml THF wird bei Raumtemperatur mit 0.77 ml (10.00 mMol) Methansulfonsäurechlorid (Aldrich) versetzt. Die Reaktionsmischung wird danach über Nacht bei Raumtemperatur gerührt. Der entstandene Niederschlag wird abfiltriert. Das
   Filtrat wird im Vakuum bis zur Trockene eingeengt. Der Rückstand wird in Ethylacetat aufgenommen und mit Wasser und gesättigter Natriumchlorid-Lösung gewaschen, über Natriumsulfat getrocknet und im Vakuum bis zur Trockene eingeengt.
   C₁₃H₂₁NO₆S₂ (351.44)
   [M+H]+ = 352
   DC: Kieselgel, Dichlormethan / Methanol 9.5:0.5, Rf-Wert = 0.95
132b) 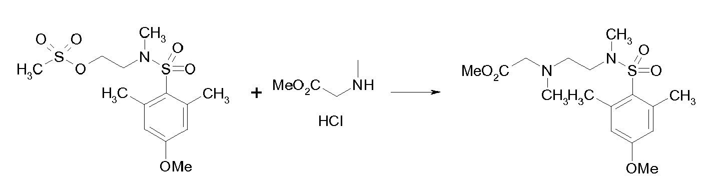 Eine Mischung aus 0.50 g (1.42 mMol) Produkt aus 132a, 0.20 g (1.42 mMol) Sarcosin Methylester Hydrochlorid (Aldrich), 0.52 ml (3.00 mMol) DIPEA und 5 ml DMF wird 24 Stunden bei 80°C gerührt. Die Reaktionsmischung wird im Vakuum eingeengt. Der Rückstand wird in Dichlormethan aufgenommen und mit Wasser und gesättigter Natriumhydrogencarbonat-Lösung gewaschen, über Natriumsulfat getrocknet und im Vakuum bis zur Trockene eingeengt. Das so erhaltene Rohprodukt wird durch Säulenchromatographie (Elutionsmittel: Dichlormethan / 0-3 % Methanol) gereinigt.
   C₁₆H₂₆N₂O₅S (358.45)
   [M+H]+ = 359
132c) 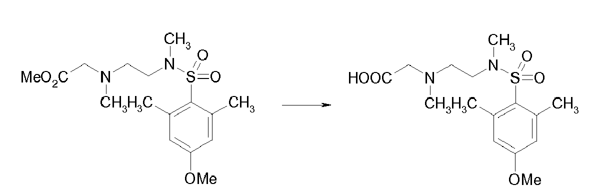 132c wird analog zu 1c aus 0.29 g (0.81 mMol) Produkt aus 132b und 0.17 g (4.00 mMol) Lithiumhydroxid Monohydrat (Aldrich) in 5 ml THF und 4 ml Wasser hergestellt.
   C₁₅H₂₄N₂O₅S (344.43)
   HPLC (Methode 1): Retentionszeit = 2.32 min
132d) 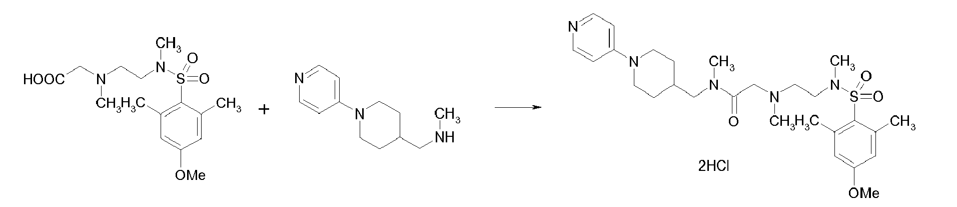 Beispiel 132 wird analog zu 1f aus 0.10 g (0.29 mMol) Produkt aus 132c, 0.06 g (0.29 mMol) Produkt aus 61 b, 0.084 ml (0.60 mMol) Triethylamin und 0.096 g (0.30 mMol) TBTU in 10 ml DMF hergestellt.
   C₂₇H₄₁N₅O₄S x 2HCl (604.63)
   [M+H]+ = 532
   HPLC (Methode 5): Retentionszeit = 1.39 min

### Beispiel 133

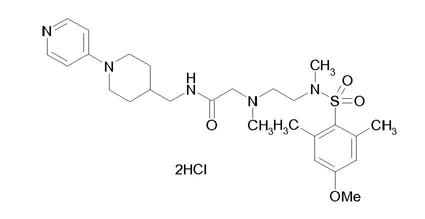

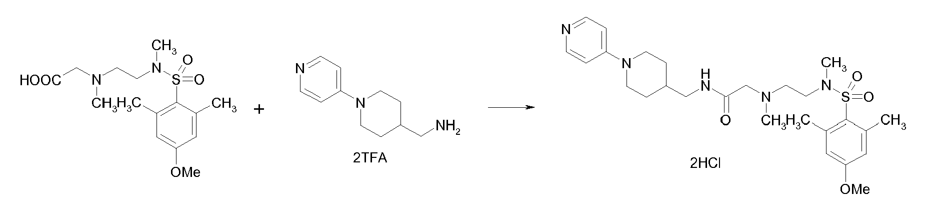

Beispiel 133 wird analog zu 1f aus 0.10 g (0.29 mMol) Produkt aus 132c, 0.12 g (0.29 mMol) Produkt aus 121f, 0.17 ml (1.20 mMol) Triethylamin und 0.096 g (0.30 mMol) TBTU in 60 ml DMF hergestellt.
C₂₆H₃₉N₅O₄S x 2HCl (590.61)
[M+H]+ = 518
HPLC (Methode 5): Retentionszeit = 1.37 min

### Beispiel 134

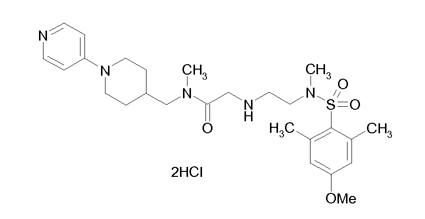

134a) 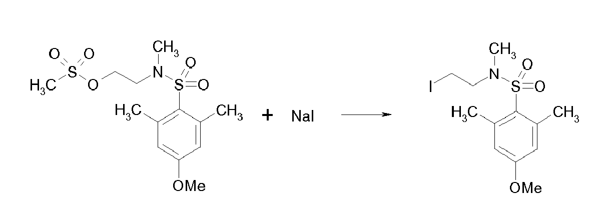 Eine Mischung aus 1.60 g (4.55 mMol) Produkt aus 132a, 2.10 g (14.00 mMol) Natriumiodid und 30 ml Aceton wird acht Stunden unter Rückfluß gerührt. Die Reaktionsmischung wird danach über Kieselgel filtriert. Das Filtrat wird im Vakuum bis zur Trockene eingeengt. Der Rückstand wird in Ethylacetat aufgenommen, mit Wasser gewaschen, über Natriumsulfat getrocknet und im Vakuum bis zur Trockene eingeengt.
   C₁₂H₁₈INO₃S (383.25)
   [M+H]+ = 384
   HPLC (Methode 1): Retentionszeit = 3.75 min
134b) 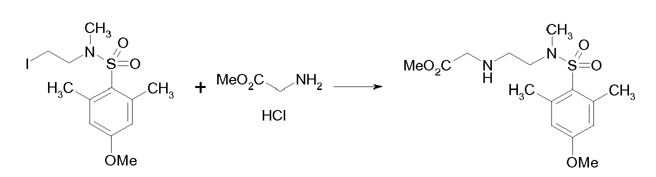 134b wird analog zu 132b aus 1.30 g (3.39 mMol) Produkt aus 134a, 1.28 g (10.20 mMol) Glycin Methylester Hydrochlorid (Aldrich) und 3.48 ml (20.00 mMol) DIPEA in 15 ml Acetonitril hergestellt.
   C₁₅H₂₄N₂O₅S (344.43)
   [M+H]+ = ₃₄₅
   DC: Kieselgel, Dichlormethan / Methanol 9.5:0.5, Rf-Wert = 0.38
134c) 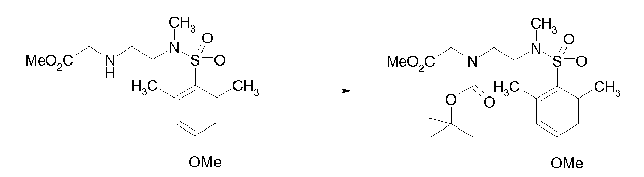 Eine Mischung aus 0.46 g (1.34 mMol) Produkt aus 134b, 0.33 g (1.50 mMol) Boc-Anhydrid, 0.21 ml (1.50 mMol) Triethylamin und 30 ml Dichlormethan wird über Nacht bei Raumtemperatur gerührt. Die Reaktionsmischung wird anschließend mit Dichlormethan verdünnt und mit gesättigter Natriumhydrogencarbonat-Lösung und gesättigter Natriumchlorid-Lösung gewaschen, über Natriumsulfat getrocknet und im Vakuum bis zur Trockene eingeengt.
   C₂₀H₃₂N₂O₇S (444.54)
   [M+H]+ = 445
   DC: Kieselgel, Dichlormethan / Methanol 9.5:0.5, Rf-Wert = 0.45
134d) 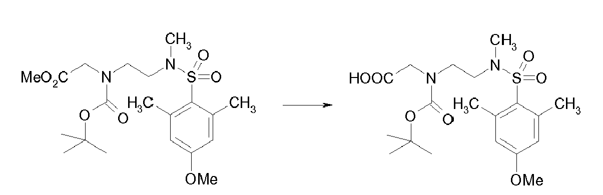 134d wird analog zu 1c aus 0.59 g (1.33 mMol) Produkt aus 134c und 0.28 g (6.60 mMol) Lithiumhydroxid Monohydrat (Aldrich) in 7 ml THF und 6.6 ml Wasser hergestellt.
   C₁₉H₃₀N₂O₇S (430.52)
   [M+H]+ = ₄₃₁
134e) 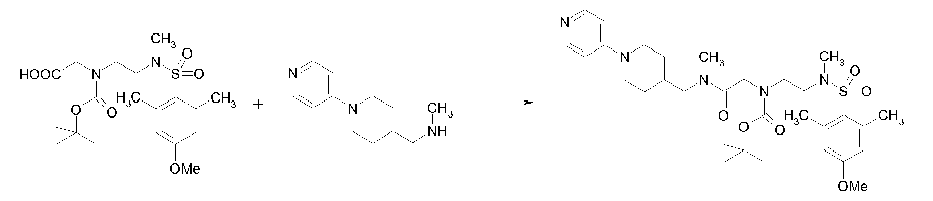 134e wird analog zu 1f aus 0.15 g (0.35 mMol) Produkt aus 134d, 0.072 g (0.35 mMol) Produkt aus 61 b, 0.098 ml (0.70 mMol) Triethylamin und 0.11 g (0.35 mMol) TBTU in 7 ml DMF hergestellt.
   C₃₁H₄₇N₅O₆S (617.80)
   [M+H]+ = 618
   HPLC (Methode 1): Retentionszeit = 2.62 min
134f) 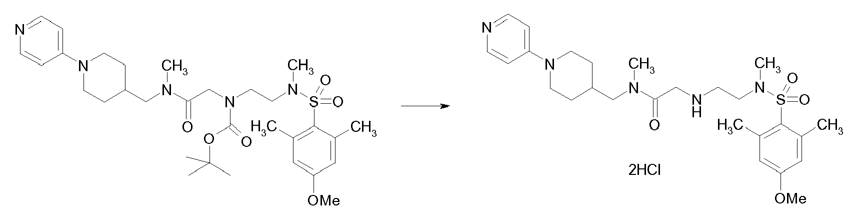 Beispiel 134 wird analog zu 18b aus 0.16 g (0.26 mMol) Produkt aus 134e und 3 ml TFA in 3 ml Dichlormethan hergestellt.
   C₂₆H₃₉N₅O₄S x 2HCl (590.61)
   [M+H]+ = 518
   HPLC (Methode 5): Retentionszeit = 1.40 min

### Beispiel 135

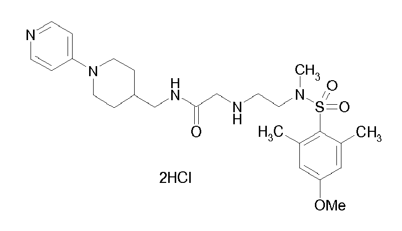

135a) 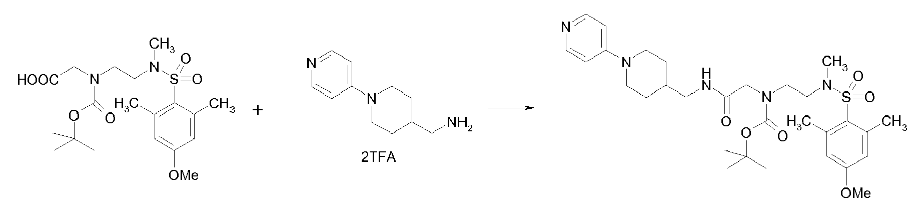 135a wird analog zu 1f aus 0.15 g (0.35 mMol) Produkt aus 134d, 0.15 g (0.35 mMol) Produkt aus 121f, 0.20 ml (1.40 mMol) Triethylamin und 0.11 g (0.35 mMol) TBTU in 7 ml DMF hergestellt.
   C₃₀H₄₅N₅O₆S (603.77)
   [M+H]+ = 604
   DC: Kieselgel, Dichlormethan / Methanol / Ammoniak 9:1:0.1, Rf-Wert = 0.48
135b) 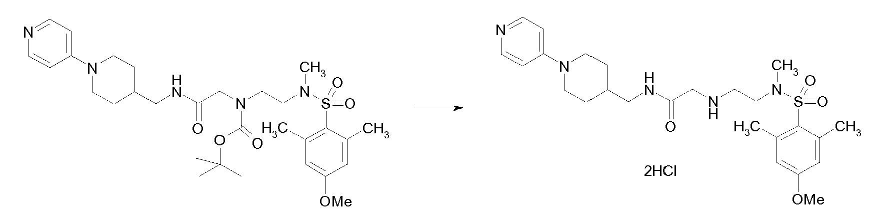 Beispiel 135 wird analog zu 18b aus 0.16 g (0.27 mMol) Produkt aus 135a und 5 ml TFA in 5 ml Dichlormethan hergestellt.
   C₂₅H₃₇N₅O₄S x 2HCl (576.58)
   [M+H]+ = 504
   HPLC (Methode 1): Retentionszeit = 2.17 min

### Beispiel 136

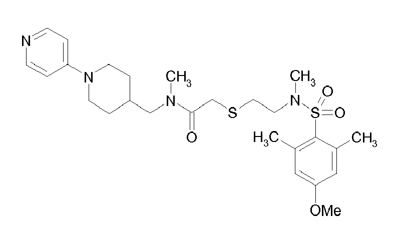

136a) 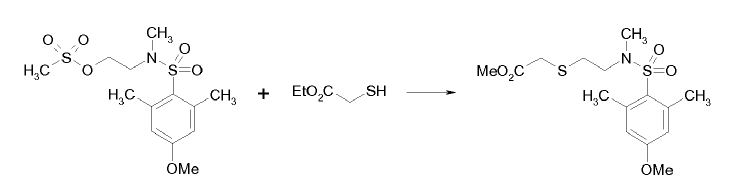 Eine Mischung aus 0.21 g (0.60 mMol) Produkt aus 132a, 0.065 ml (0.60 mMol) Mercaptoessigsäureethylester (Aldrich), 0.17 g (1.20 mMol) Kaliumcarbonat und 10 ml Acetonitril wird 24 Stunden bei Raumtemperatur gerührt. Der Niederschlag wird abfiltriert, das Filtrat wird im Vakuum eingeengt. Das so erhaltene Rohprodukt wird durch präparative HPLC gereinigt.
   C₁₆H₂₅NO₅S₂ (375.51)
   [M+H]+ = 376
136b) 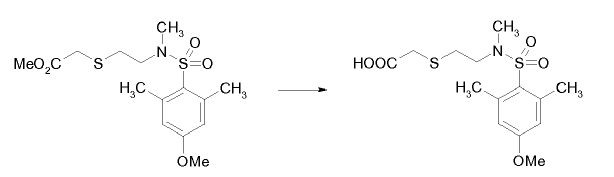 136b wird analog zu 121 b aus 0.070 g (0.19 mMol) Produkt aus 136a und 2.00 ml (2.00 mMol) 1 M Natronlauge in 5 ml THF hergestellt.
   C₁₄H₂₁NO₅S₂ (347.45)
   [M+H]+ = 348
136c) 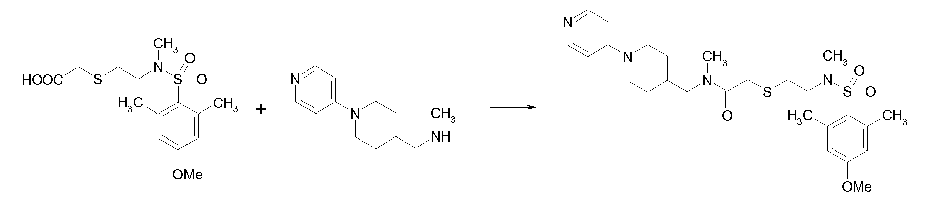 Beispiel 136 wird analog zu 1f aus 0.064 g (0.18 mMol) Produkt aus 136b, 0.038 g (0.18 mMol) Produkt aus 61 b, 0.079 ml (0.46 mMol) DIPEA und 0.059 g (0.18 mMol) TBTU in 5 ml DMF hergestellt.
   C₂₆H₃₈N₄O₄S₂ (534.74)
   [M+H]+ = 535
   HPLC (Methode 6): Retentionszeit = 2.68 min

### Beispiel 137

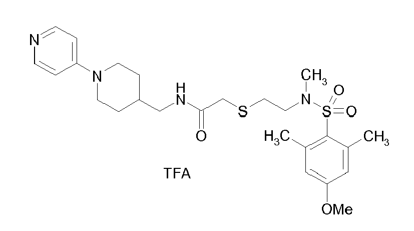

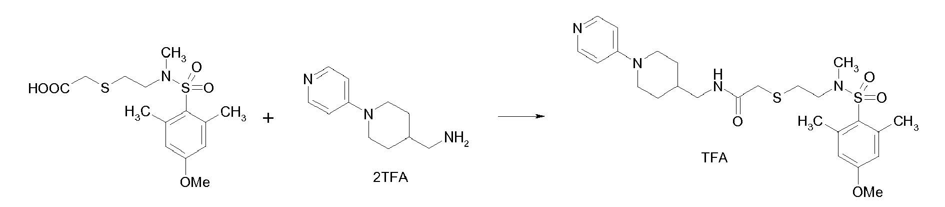

Beispiel 137 wird analog zu 1f aus 0.073 g (0.21 mMol) Produkt aus 136b, 0.088 g (0.21 mMol) Produkt aus 121f, 0.11 ml (0.63 mMol) DIPEA und 0.087 g (0.27 mMol) TBTU in 5 ml DMF hergestellt.
C₂₅H₃₆N₄O₄S₂ x C₂HF₃O₂ (634.73)
[M+H]+ = 521
HPLC (Methode 6): Retentionszeit = 2.57 min

### Beispiel 138

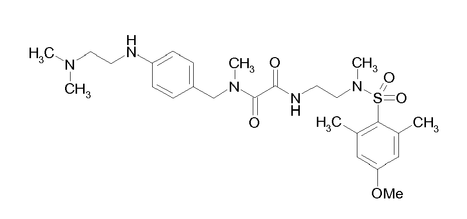

138a) 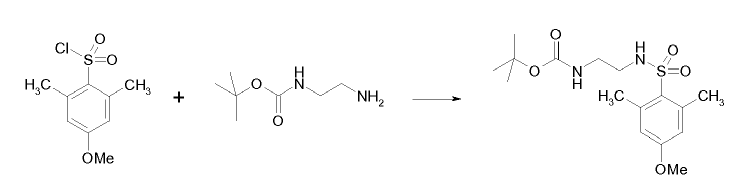 138a wird analog zu 3a aus 2.0 g (8.52 mMol) Produkt aus 13a, 1.37 g (8.55 mMol) N-Boc-Ethylendiamin (Fluka) und 1.0 g (9.89 mMol) Triethylamin in 50 ml THF hergestellt.
   C₁₆H₂₆N₂O₅S (358.45)
   [M-H]- = 357
   HPLC (Methode 6): Retentionszeit = 3.63 min
138b) 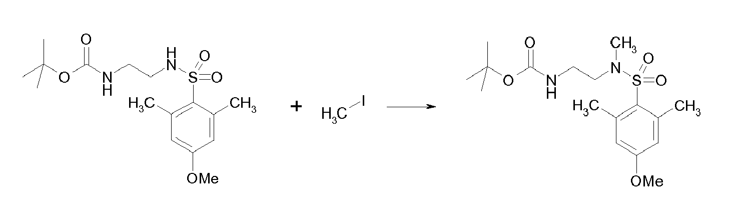 138b wird analog zu 3b aus 3.38 g (9.43 mMol) Produkt aus 138a, 0.53 ml (8.55 mMol) Methyliodid, 1.77 g (12.83 mMol) Kaliumcarbonat wasserfrei in 30 ml DMSO hergestellt.
   C₁₇H₂₈N₂O₅S (372.48)
   [M+H]+ = 373
   HPLC (Methode 6): Retentionszeit = 3.89 min
138c) 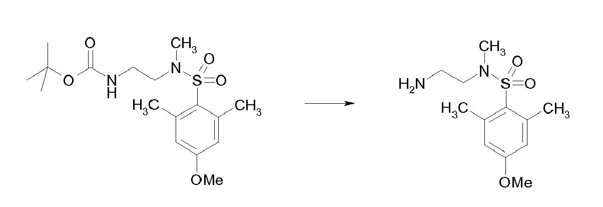 138b wird analog zu 28d aus 3.61 g (9.69 mMol) Produkt aus 138b und 10 ml TFA in 50 ml Dichlormethan hergestellt.
   C₁₂₇H₂₀N₂O₃S (272.36)
   [M+H]+ = 273
   HPLC (Methode 6): Retentionszeit = 1.95 min
138d) 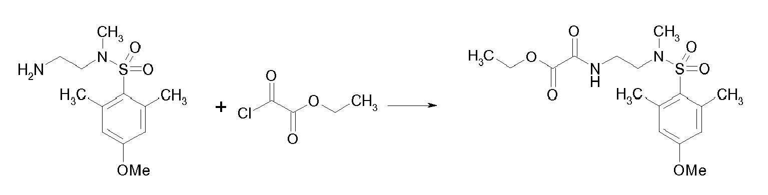 Eine Mischung aus 0.50 g (1.84 mMol) Produkt aus 138c, 0.56 ml (5.51 mMol) Triethylamin und 25 ml Dichlormethan wird unter Eisbadkühlung mit 0.38 ml (2.75 mMol) Oxalsäure-monoethylesterchlorid (Fluka) in 5 ml Dichlormethan versetzt und zwei Stunden bei Raumtemperatur gerührt. Die Reaktionsmischung wird anschließend mit Dichlormethan verdünnt, mit 10%iger wässriger Zitronensäure-Lösung, gesättigter Natriumsulfat-Lösung und mit Wasser gewaschen, über Natriumsulfat getrocknet und im Vakuum bis zur Trockene eingeengt.
   C₁₆H₂₄N₂O₆S (372.44)
   [M+H]+ = 373
138e) 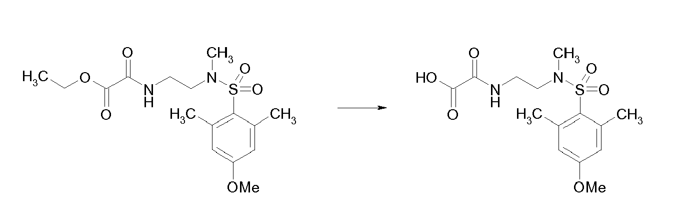 Eine Mischung aus 0.60 g (1.60 mMol) Produkt aus 138d, 5.6 ml 1 M Natronlauge und 6 ml Ethanol wird vier Stunden bei Raumtemperatur gerührt und danach im Vakuum bis zur Trockene eingeengt. Der Rückstand wird mit 8 ml 1 M Salzsäure versetzt und mit Ethylacetat extrahiert. Die vereinigten organischen Extrakte werden mit Wasser und gesättigter Natriumchlorid-Lösung gewaschen, über Natriumsulfat getrocknet und im Vakuum bis zur Trockene eingeengt.
   C₁₄H₂₀N₂O₆S (344.38)
   [M+H]+ = 345
138f) 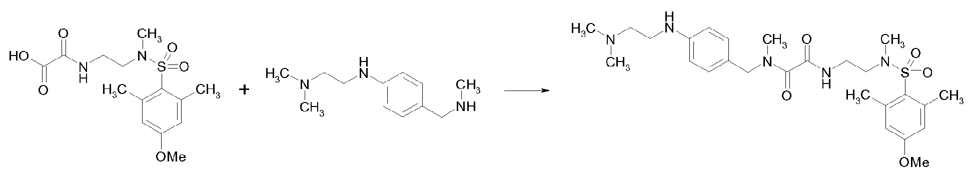 Beispiel 138 wird analog zu 1f aus 40.0 mg (0.12 mMol) Produkt aus 138e, 48.2 mg (0.12 mMol) N,N-Dimethyl-N'-(4-methylaminomethyl-phenyl)-ethan-1,2-diamin (analog J. Chem. Soc 1960, 3163-3165), 0.038 ml (0.29 mMol) DIPEA und 38.9 mg (0.12 mMol) TBTU in 1.5 ml DMF hergestellt.
   C₂₆H₃₉N₅O₅S (533.68)
   [M+H]+ = 534
   HPLC (Methode 6): Retentionszeit = 1.61 min

### Beispiel 139

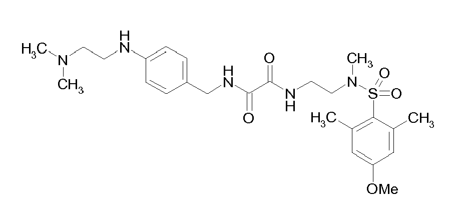

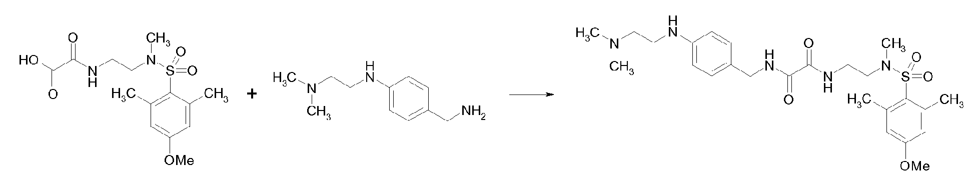

Beispiel 139 wird analog zu 1f aus 40.0 mg (0.12 mMol) Produkt aus 138e, 22.5 mg (0.12 mMol) N-(4-Aminomethyl-phenyl)-N',N'-dimethylethan-1,2-diamin (analog J. Chem. Soc 1960, 3163-3165), 0.038 ml (0.29 mMol) DIPEA und 38.9 mg (0.12 mMol) TBTU in 1.5 ml DMF hergestellt.
C₂₅H₃₇N₅O₅S (519.66)
[M+H]+ = 520
HPLC (Methode 6): Retentionszeit = 1.53 min

Die folgenden Verbindungen wurden analog zu Beispiel 22 hergestellt:

### Beispiel 140

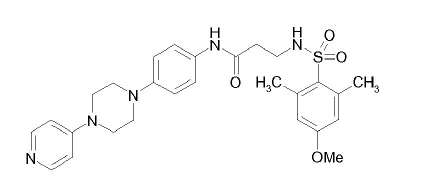

C₂₇H₃₃N₅O₄S x C₂HF₃O₂ (637.67)
[M+H]+ = 524
HPLC (Methode 6): Retentionszeit = 2.43 min

### Beispiel 141

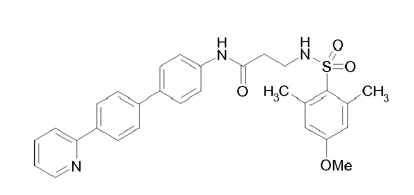

C₂₉H₂₉N₃O₄S x C₂HF₃O₂ (629.65)
[M+H]+ = 516
HPLC (Methode 6): Retentionszeit = 3.39 min

### Beispiel 142

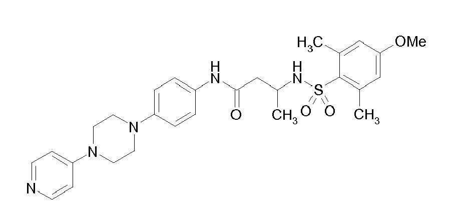

C₂₈H₃₅N₅O₄S (537.67)
[M+H]+ = 538
HPLC (Methode 6): Retentionszeit = 2.45 min

### Beispiel 143

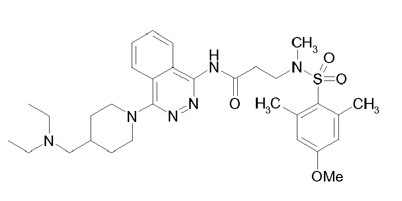

C₃₁H₄₄N₆O₄S x C₂HF₃O₂ (710.81)
[M+H]+ = 597
HPLC (Methode 6): Retentionszeit = 2.30 min

### Beispiel 144

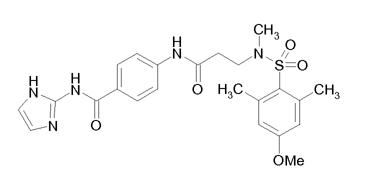

C₂₃H₂₇N₅O₅S x C₂HF₃O₂ (599.58)
[M+H]+ = 486
HPLC (Methode 6): Retentionszeit = 2.46 min

### Beispiel 145

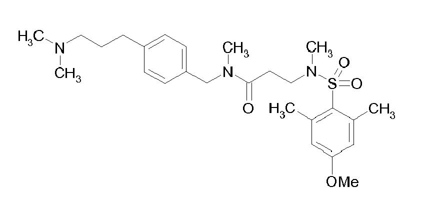

C₂₆H₃₉N₃O₄S (489.67)
[M+H]+ = 490
HPLC (Methode 6): Retentionszeit = 2.66 min

### Beispiel 146

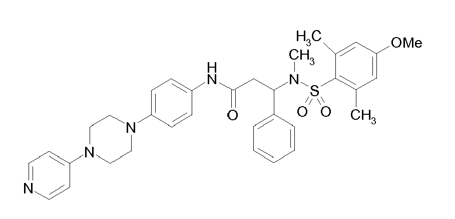

C₃₄H₃₉N₅O₄S (613.77)
[M+H]+ = 614
HPLC (Methode 6): Retentionszeit = 3.07 min

### Beispiel 147

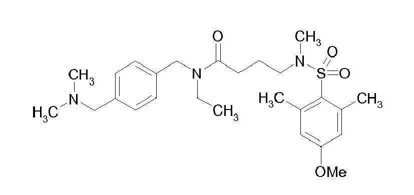

C₂₆H₃₉N₃O₄S x C₂HF₃O₂ (603.70)
[M+H]+ = 490
HPLC (Methode 6): Retentionszeit = 2.60 min

### Beispiel 148

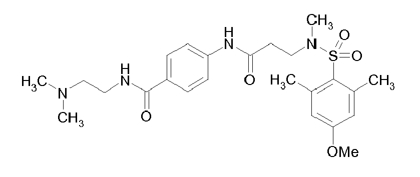

C₂₄H₃₄N₄O₅S x CH₂O₂ (536.64)
[M+H]+ = 491
HPLC (Methode 6): Retentionszeit = 2.29 min

### Beispiel 149

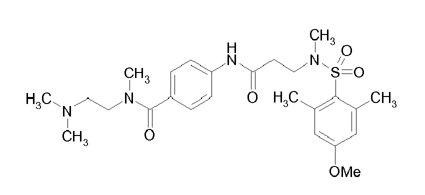

C₂₅H₃₆N₄O₅S x CH₂O₂ (550.67)
[M+H]+ = 505
HPLC (Methode 6): Retentionszeit = 2.32 min

### Beispiel 150

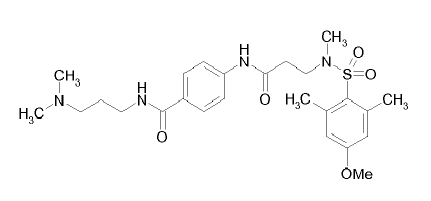

C₂₅H₃₆N₄O₅S (504.64)
[M+H]+ = 505
HPLC (Methode 6): Retentionszeit = 2.31 min

### Beispiel 151

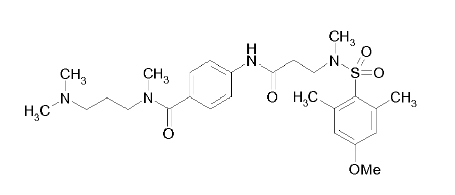

C₂₆H₃₈N₄O₅S x CH₂O₂ (564.70)
[M+H]+ = 519
HPLC (Methode 6): Retentionszeit = 2.34 min

### Beispiel 152

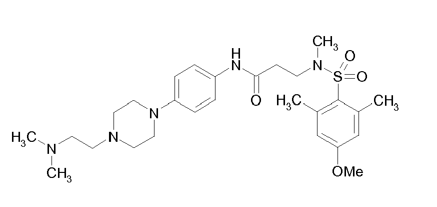

C₂₇H₄₁N₅O₄S x 2C₂HF₃O₂ (759.76)
[M+H]+ = 532
HPLC (Methode 5): Retentionszeit = 1.40 min

### Beispiel 153

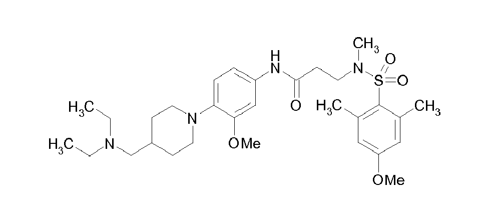

C₃₀H₄₆N₄O₅S x HCl (611.24)
[M+H]+ = 575
HPLC (Methode 1): Retentionszeit = 2.12 min

### Beispiel 154

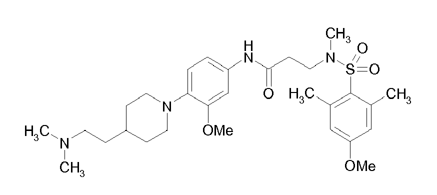

C₂₉H₄₄N₄O₅S x HCl (597.21)
[M+H]+ = 561
HPLC (Methode 8): Retentionszeit = 3.12 min

### Beispiel 155

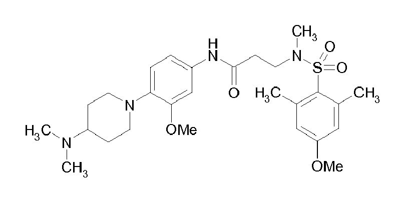

C₂₇H₄₀N₄O₅S x HCl (569.16)
[M+H]+ = 533
HPLC (Methode 11): Retentionszeit = 1.67 min

### Beispiel 156

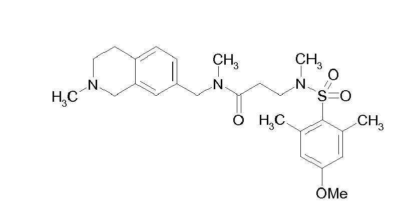

C₂₅H₃₅N₃O₄S x HCl (510.09)
[M+H]+ = 474
HPLC (Methode 7): Retentionszeit = 1.90 min

### Beispiel 157

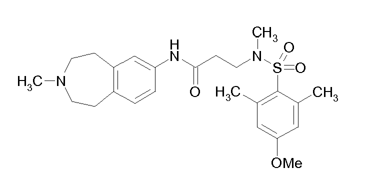

C₂₄H₃₃N₃O₄S x C₂HF₃O₂ (573.63)
[M+H]+ = 460
HPLC (Methode 5): Retentionszeit = 1.52 min

### Beispiel 158

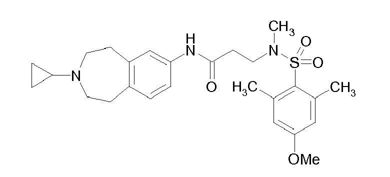

C₂₆H₃₅N₃O₄S x C₂HF₃O₂ (599.66)
[M+H]+ = 486
HPLC (Methode 5): Retentionszeit = 1.55 min

### Beispiel 159

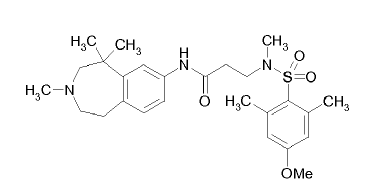

C₂₆H₃₇N₃O₄S x C₂HF₃O₂ (601.68)
[M+H]+ = 488
HPLC (Methode 5): Retentionszeit = 1.54 min

### Beispiel 160

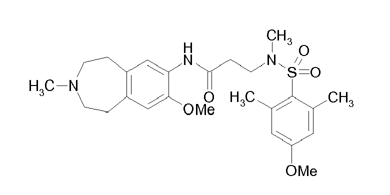

C₂₅H₃₅N₃O₅S x C₂HF₃O₂ (603.65)
[M+H]+ = 490
HPLC (Methode 5): Retentionszeit = 1.54 min

### Beispiel 161

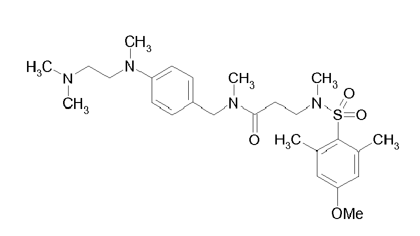

C₂₆H₄₀N₄O₄S x HCl (541.15)
[M+H]+ = 505
HPLC (Methode 5): Retentionszeit = 1.59 min

### Beispiel 162

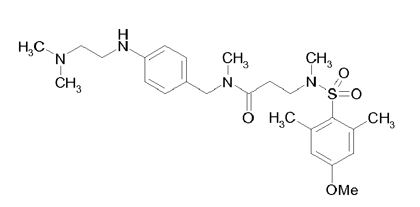

C₂₅H₃₈N₄O₄S x HCl (527.12)
[M+H]+ = 491
HPLC (Methode 5): Retentionszeit = 1.55 min

### Beispiel 163

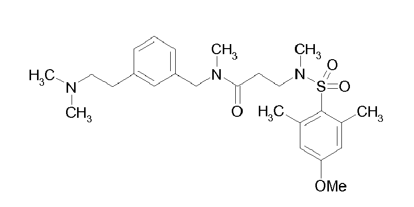

C₂₅H₃₇N₃O₄S x HCl (512.11)
[M+H]+ = 476
HPLC (Methode 5): Retentionszeit = 1.56 min

### Beispiel 164

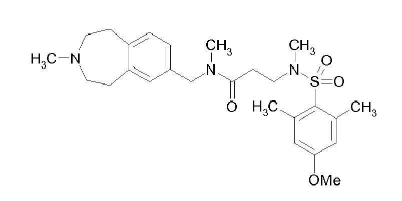

C₂₆H₃₇N₃O₄S x HCl (524.12)
[M+H]+ = 488
HPLC (Methode 5): Retentionszeit = 1.54 min

### Beispiel 165

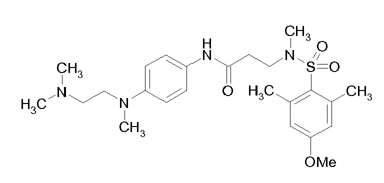

C₂₄H₃₆N₄O₄S x HCl (513.09)
[M+H]+ = 477
HPLC (Methode 7): Retentionszeit = 1.88 min

### Beispiel 166

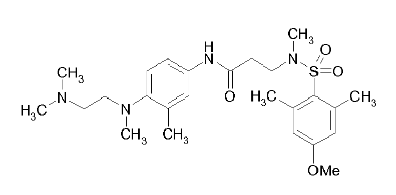

C₂₅H₃₈N₄O₄S x HCl (527.12)
[M+H]+ = 491
HPLC (Methode 7): Retentionszeit = 1.92 min

### Beispiel 167

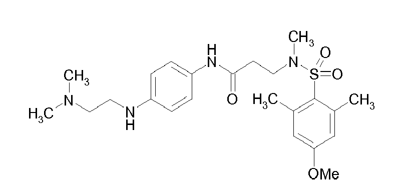

C₂₃H₃₄N₄O₄S x HCl (499.07)
[M+H]+ = 463
HPLC (Methode 7): Retentionszeit = 1.79 min

### Beispiel 168

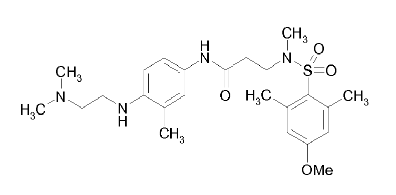

C₂₄H₃₆N₄O₄S x HCl (513.09)
[M+H]+ = 477
HPLC (Methode 7): Retentionszeit = 1.86 min

### Beispiel 169

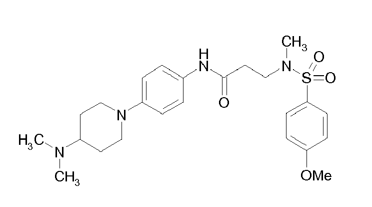

C₂₄H₃₄N₄O₄S x C₂HF₃O₂ (588.64)
[M+H]+ = 475
HPLC (Methode 5): Retentionszeit = 1.39 min

### Beispiel 170

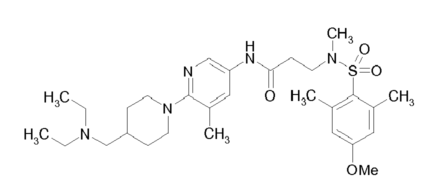

C₂₉H₄₅N₅O₄S x 2HCl (632.69)
[M+H]+ = 560
DC: Kieselgel, Dichlormethan / Ethanol 9:1, Rf-Wert = 0.22

### Beispiel 171

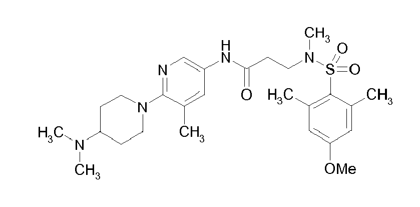

C₂₆H₃₉N₅O₄S x 2HCl (590.61)
[M+H]+ = 518
DC: Kieselgel, Dichlormethan / Ethanol 4:1, Rf-Wert = 0.68

### Beispiel 172

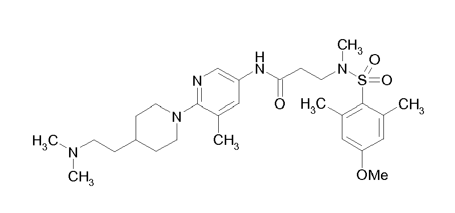

C₂₈H₄₃N₅O₄S x 2HCl (618.66)
[M+H]+ = 546
HPLC (Methode 5): Retentionszeit = 1.26 min

### Beispiel 173

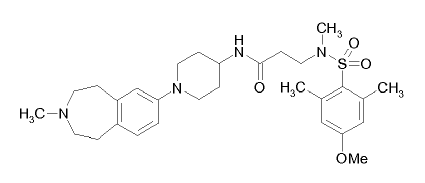

C₂₉H₄₂N₄O₄S (542.73)
[M+H]+ = 543
HPLC (Methode 4): Retentionszeit = 2.8 min

### Beispiel 174

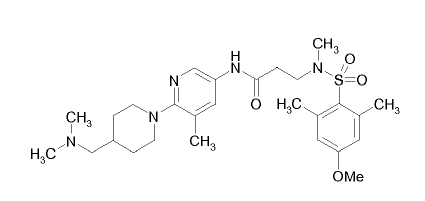

C₂₇H₄₁N₅O₄S x 2HCl (604.63)
[M+H]+ = 532
HPLC (Methode 5): Retentionszeit = 1.39 min

### Beispiel 175

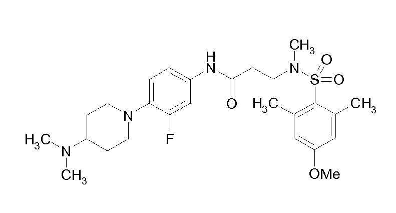

C₂₆H₃₇FN₄O₄S x HCl (557.12)
[M+H]+ = 521
DC: Kieselgel, Dichlormethan / Ethanol 9:1, Rf-Wert = 0.25

### Beispiel 176

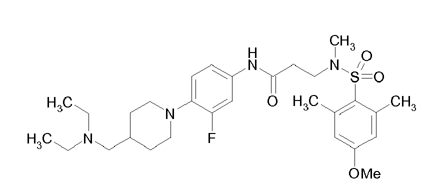

C₂₉H₄₃FN₄O₄S x HCl (599.20)
[M+H]+ = 563
DC: Kieselgel, Dichlormethan / Ethanol 9:1, Rf-Wert = 0.40

### Beispiel 177

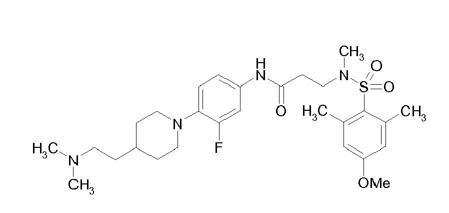

C₂₈H₄₁FN₄O₄S x HCl (585.17)
[M+H]+ = 549
DC: Kieselgel, Dichlormethan / Ethanol 9:1, Rf-Wert = 0.35

### Beispiel 178

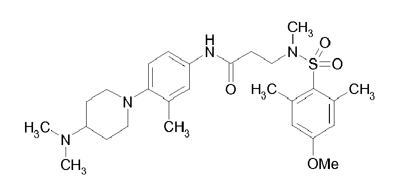

C₂₇H₄₀N₄O₄S (516.70)
[M+H]+ = 517
DC: Kieselgel, Dichlormethan / Ethanol / Ammoniak 8:2:0.01, Rf-Wert = 0.41

### Beispiel 179

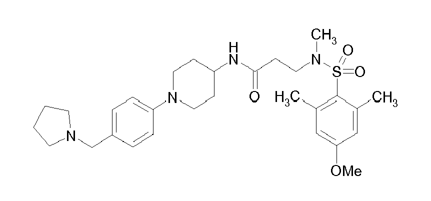

C₂₉H₄₂N₄O₄S x HCl (579.19)
[M+H]+ = 543
DC: Kieselgel, Dichlormethan / Ethanol / Ammoniak 8:2:0.01, Rf-Wert = 0.47

### Beispiel 180

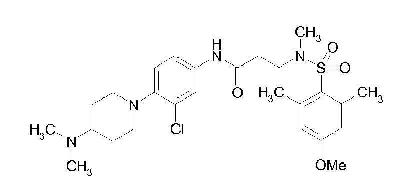

C₂₆H₃₇CIN₄O₄S x HCl (573.58)
[M+H]+ = 537/539
DC: Kieselgel, Dichlormethan / Ethanol 9:1, Rf-Wert = 0.26

### Beispiel 181

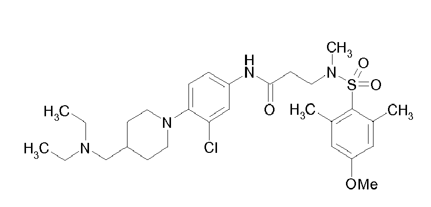

C₂₉H₄₃CIN₄O₄S x HCl (615.66)
[M+H]+ = 579/581
DC: Kieselgel, Dichlormethan / Ethanol 9:1, Rf-Wert = 0.45

### Beispiel 182

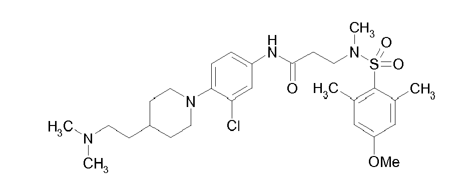

C₂₈H₄₁CIN₄O₄S x HCl (601.63)
[M+H]+ = 565/567
DC: Kieselgel, Dichlormethan / Ethanol 9:1, Rf-Wert = 0.30

### Beispiel 183

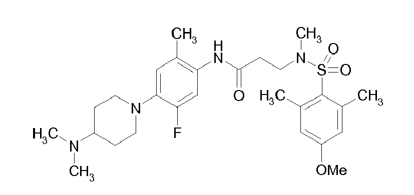

C₂₇H₃₉FN₄O₄S x HCl (571.15)
[M+H]+ = 535
DC: Kieselgel, Dichlormethan / Ethanol / Ammoniak 8:2:0.01, Rf-Wert = 0.26

### Beispiel 184

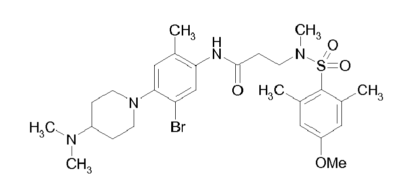

C₂₇H₃₉BrN₄O₄S x HCl (632.05)
[M+H]+ = 595/597
DC: Kieselgel, Dichlormethan / Ethanol / Ammoniak 8:2:0.01, Rf-Wert = 0.51

### Beispiel 185

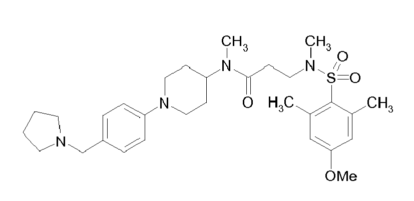

C₃₀H₄₄N₄O₄S x HCl (593.22)
[M+H]+ = 557
DC: Kieselgel, Dichlormethan / Ethanol / Ammoniak 8:2:0.01, Rf-Wert = 0.63

### Beispiel 186

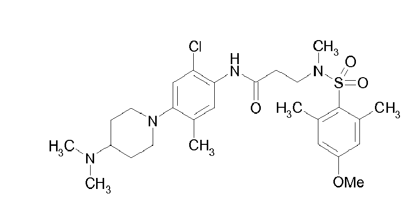

C₂₇H₃₉CIN₄O₄S x HCl (587.60)
[M+H]+ = 551
DC: Kieselgel, Dichlormethan / Ethanol / Ammoniak 8:2:0.01, Rf-Wert = 0.58

### Beispiel 187

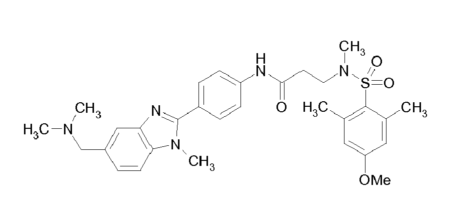

C₃₀H₃₇N₅O₄S x HCl (600.17)
[M+H]+ = 564
HPLC (Methode 4): Retentionszeit = 3.0 min

### Beispiel 188

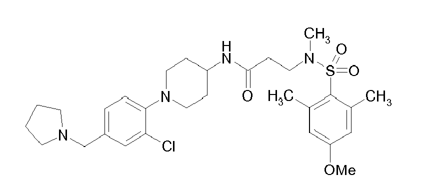

C₂₉H₄₁CIN₄O₄S x HCl (613.64)
[M+H]+ = 577/579
DC: Kieselgel, Dichlormethan / Methanol 9:1, Rf-Wert = 0.24

### Beispiel 189

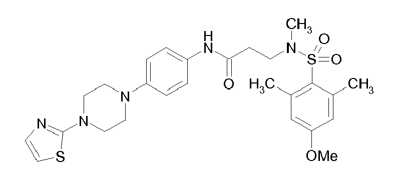

C₂₆H₃₃N₅O₄S₂ (543.70)
[M+H]+ = 544
HPLC (Methode 6): Retentionszeit = 3.11 min

### Beispiel 190

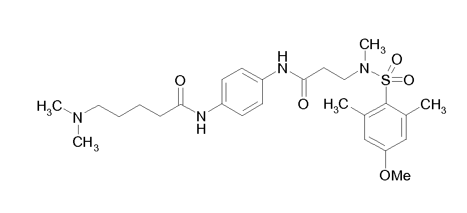

C₂₆H₃₈N₄O₅S (518.67)
[M+H]+ = 519
HPLC (Methode 6): Retentionszeit = 2.44 min

### Beispiel 191

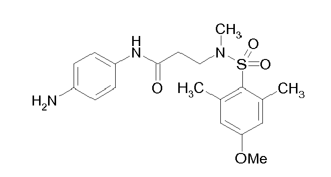

C₁₉H₂₅N₃O₄S x C₂HF₃O₂ (505.51)
[M+H]+ = 392
HPLC (Methode 6): Retentionszeit = 2.04 min

### Beispiel 192

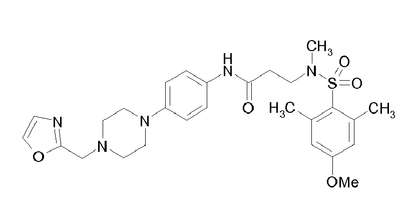

C₂₇H₃₅N₅O₅S (541.66)
[M+H]+ = 542
HPLC (Methode 6): Retentionszeit = 2.51 min

### Beispiel 193

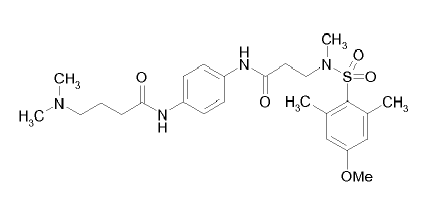

C₂₅H₃₆N₄O₅S (504.64)
[M+H]+ = 505
HPLC (Methode 6): Retentionszeit = 2.43 min

### Beispiel 194

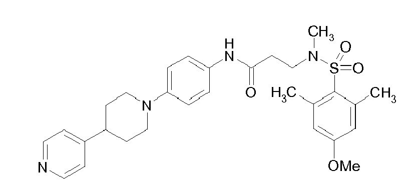

C₂₉H₃₆N₄O₄S (536.69)
[M+H]+ = 537
HPLC (Methode 6): Retentionszeit = 2.19 min

### Beispiel 195

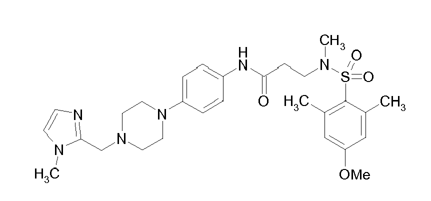

C₂₈H₃₈N₆O₄S (554.71)
[M+H]+ = 555
HPLC (Methode 6): Retentionszeit = 2.37 min

### Beispiel 196

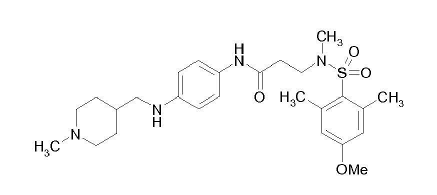

C₂₆H₃₈N₄O₄S (502.67)
[M+H]+ = 503
HPLC (Methode 6): Retentionszeit = 2.35 min

### Beispiel 197

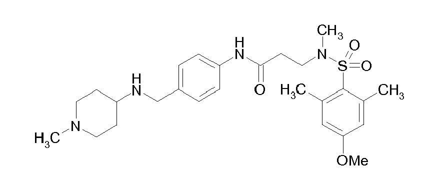

C₂₆H₃₈N₄O₄S (502.67)
[M+H]+ = 503
HPLC (Methode 6): Retentionszeit = 2.00 min

### Beispiel 198

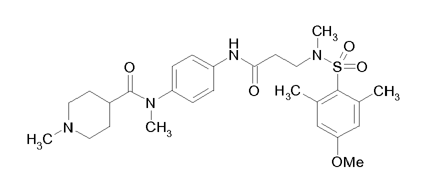

C₂₇H₃₈N₄O₅S x C₂HF₃O₂ (644.70)
[M+H]+ = 531
HPLC (Methode 6): Retentionszeit = 2.45 min

### Beispiel 199

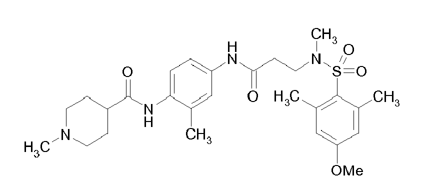

C₂₇H₃₈N₄O₅S (530.68)
[M+H]+ = 531
HPLC (Methode 6): Retentionszeit = 2.53 min

### Beispiel 200

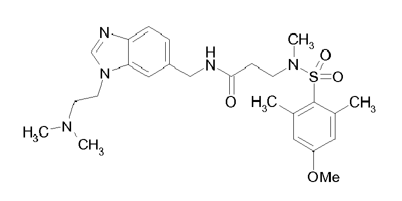

C₂₅H₃₅N₅O₄S (501.64)
[M+H]+ = 502
HPLC (Methode 6): Retentionszeit = 2.05 min

### Beispiel 201

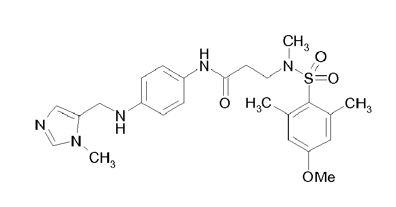

C₂₄H₃₁N₅O₄S (485.60)
[M+H]+ = 486
HPLC (Methode 6): Retentionszeit = 2.41 min

### Beispiel 202

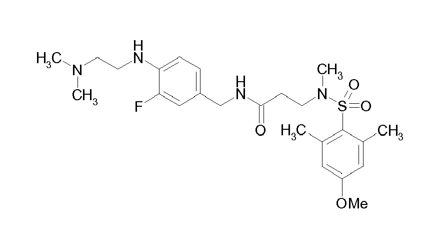

C₂₄H₃₅FN₄O₄S x CH₂O₂ (540.65)
[M+H]+ = 495
HPLC (Methode 6): Retentionszeit = 2.50 min

### Beispiel 203

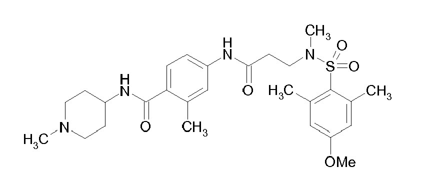

C₂₇H₃₈N₄O₅S (530.68)
[M+H]+ = 531
HPLC (Methode 6): Retentionszeit = 2.36 min

### Beispiel 204

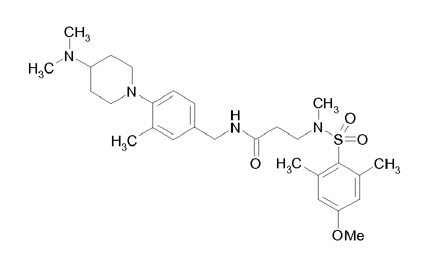

C₂₈H₄₂N₄O₄S (530.72)
[M+H]+ = 531
HPLC (Methode 6): Retentionszeit = 2.60 min

### Beispiel 205

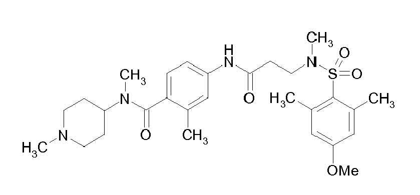

C₂₈H₄₀N₄O₅S (544.71)
[M+H]+ = 545
HPLC (Methode 6): Retentionszeit = 2.21 min

### Beispiel 206

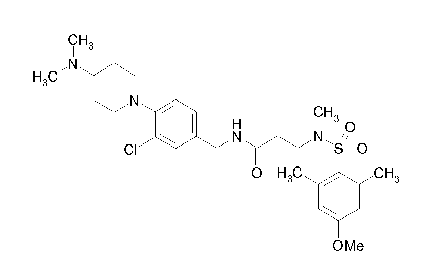

C₂₇H₃₉ClN₄O₄S (551.14)
[M+H]+ = 551/553
HPLC (Methode 6): Retentionszeit = 2.65 min

### Beispiel 207

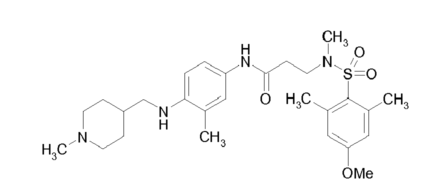

C₂₇H₄₀N₄O₄S x C₂HF₃O₂ (630.72)
[M+H]+ = 517
HPLC (Methode 6): Retentionszeit = 2.51 min

### Beispiel 208

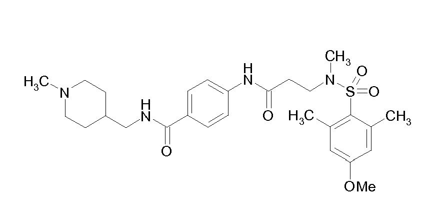

C₂₇H₃₈N₄O₅S x CH₂O₂ (576.71)
[M+H]+ = 531
HPLC (Methode 6): Retentionszeit = 2.33 min

### Beispiel 209

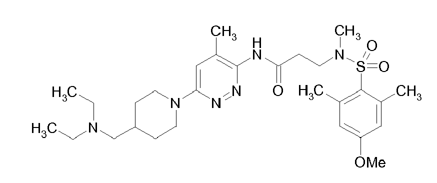

C₂₈H₄₄N₆O₄S x C₂HF₃O₂ (674.78)
[M+H]+ = 561
HPLC (Methode 6): Retentionszeit = 2.14 min

### Beispiel 210

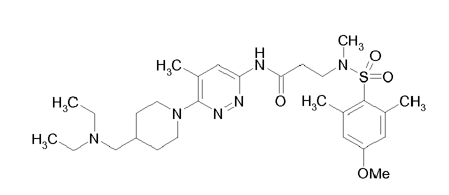

C₂₈H₄₄N₆O₄S x C₂HF₃O₂ (674.78)
[M+H]+ = 561
HPLC (Methode 6): Retentionszeit = 2.43 min

### Beispiel 211

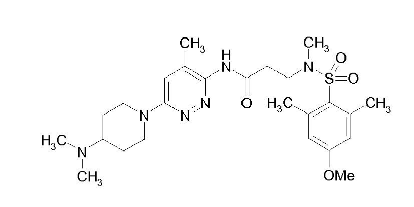

C₂₅H₃₈N₆O₄S x C₂HF₃O₂ (632.70)
[M+H]+ = 519
HPLC (Methode 6): Retentionszeit = 2.11 min

### Beispiel 212

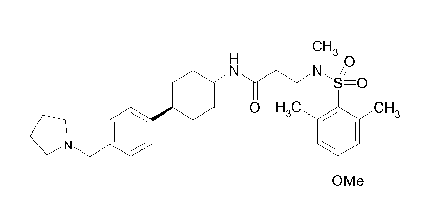

C₃₀H₄₃N₃O₄S x C₂HF₃O₂ (655.77)
[M+H]+ = 542
HPLC (Methode 6): Retentionszeit = 2.75 min

### Beispiel 213

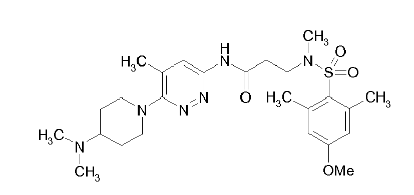

C₂₅H₃₈N₆O₄S x C₂HF₃O₂ (632.70)
[M+H]+ = 519
HPLC (Methode 6): Retentionszeit = 2.30 min

### Beispiel 214

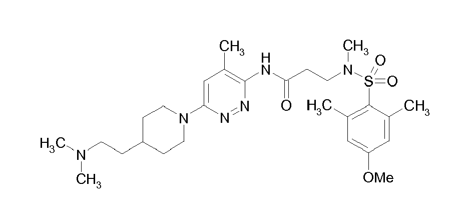

C₂₇H₄₂N₆O₄S x C₂HF₃O₂ (660.75)
[M+H]+ = 547
HPLC (Methode 6): Retentionszeit = 2.08 min

### Beispiel 215

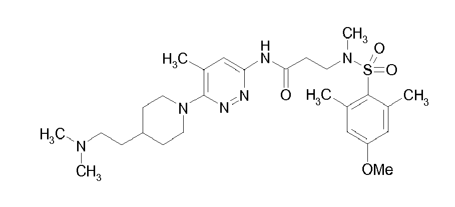

C₂₇H₄₂N₆O₄S x C₂HF₃O₂ (660.75)
[M+H]+ = 547
HPLC (Methode 6): Retentionszeit = 2.34 min

### Beispiel 216

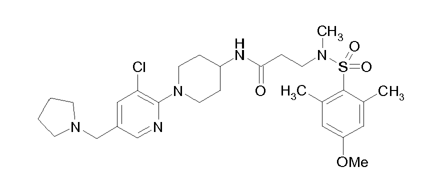

C₂₈H₄₀CIN₅O₄S x CH₂O₂ (624.19)
[M+H]+ = 578/580
HPLC (Methode 6): Retentionszeit = 2.64 min

### Beispiel 217

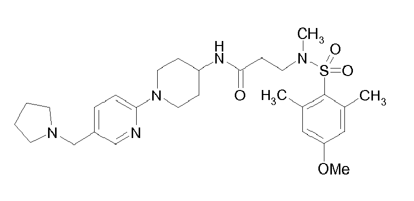

C₂₈H₄₁N₅O₄S x CH₂O₂ (589.75)
[M+H]+ = 544
HPLC (Methode 6): Retentionszeit = 2.09 min

### Beispiel 218

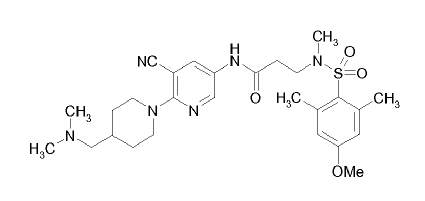

C₂₇H₃₈N₆O₄S x CH₂O₂ (588.72)
[M+H]+ = 543
HPLC (Methode 9): Retentionszeit = 1.67 min

### Beispiel 219

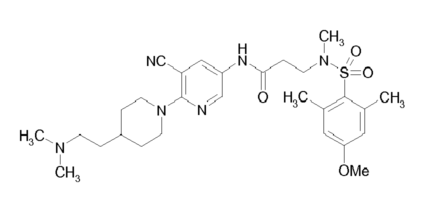

C₂₈H₄₀N₆O₄S (556.72)
[M+H]+ = 557
HPLC (Methode 9): Retentionszeit = 1.71 min

### Beispiel 220

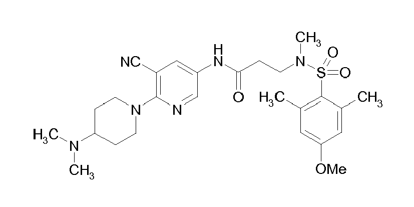

C₂₆H₃₆N₆O₄S x CH₂O₂ (574.69)
[M+H]+ = 529
HPLC (Methode 9): Retentionszeit = 1.61 min

### Beispiel 221

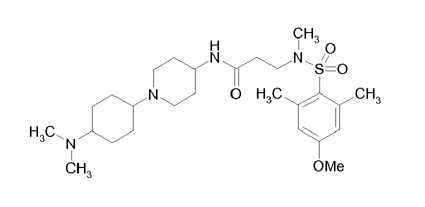

C₂₆H₄₄N₄O₄S (508.72)
[M+H]+ = 509
HPLC (Methode 9): Retentionszeit = 1.23 min

### Beispiel 222

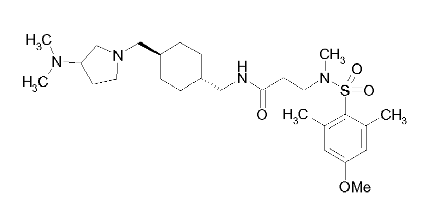

C₂₇H₄₆N₄O₄S x 2C₂HF₃O₂ (750.79)
[M+H]+ = 523
HPLC (Methode 9): Retentionszeit = 1.30 min

### Beispiel 223

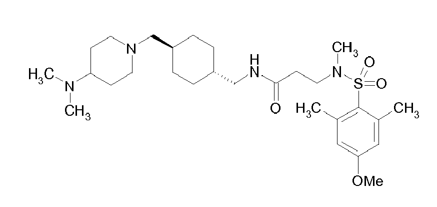

C₂₈H₄₈N₄O₄S x 2C₂HF₃O₂ (764.82)
[M+H]+ = 537
HPLC (Methode 9): Retentionszeit = 1.31 min

### Beispiel 224

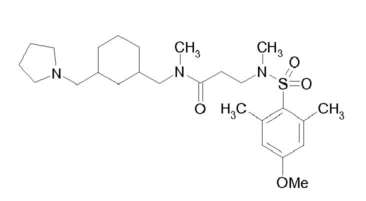

C₂₆H₄₃N₃O₄S (493.70)
[M+H]+ = 494
HPLC (Methode 9): Retentionszeit = 1.72 min

### Beispiel 225

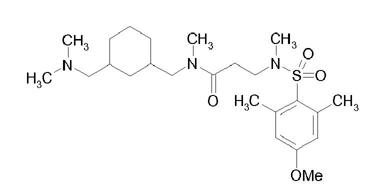

C₂₄H₄₁N₃O₄S x C₂HF₃O₂ (581.69)
[M+H]+ = 468
HPLC (Methode 9): Retentionszeit = 1.69 min

### Beispiel 226

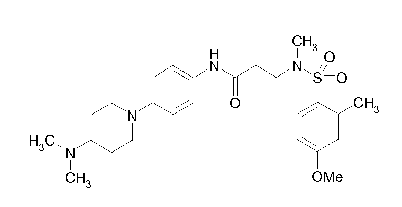

C₂₅H₃₆N₄O₄S x C₂HF₃O₂ (602.67)
[M+H]+ = 489
HPLC (Methode 5): Retentionszeit = 1.39 min

### Beispiel 227

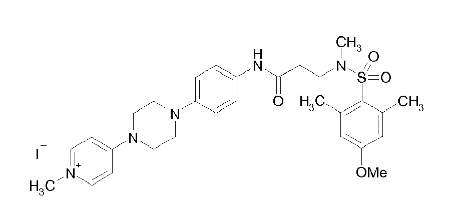

C₂₉H₃₈N₅O₄S x I (679.61)
[M+H]+ = 552
HPLC (Methode 5): Retentionszeit = 1.55 min

### Beispiel 228

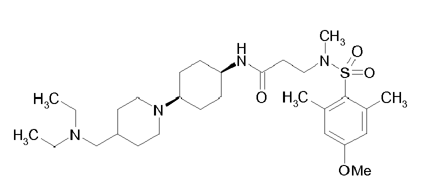

C₂₉H₅₀N₄O₄S (550.80)
[M+H]+ = 551
HPLC (Methode 5): Retentionszeit = 1.38 min

### Beispiel 229

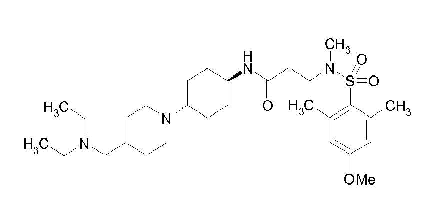

C₂₉H₅₀N₄O₄S (550.80)
[M+H]+ = 551
HPLC (Methode 5): Retentionszeit = 1.40 min

### Beispiel 230

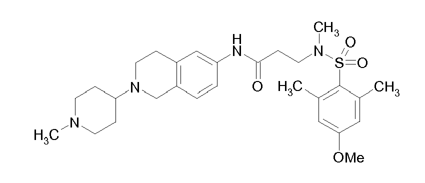

C₂₈H₄₀N₄O₄S x 2HCl (601.63)
[M+H]+ = 529
HPLC (Methode 5): Retentionszeit = 1.41 min

### Beispiel 231

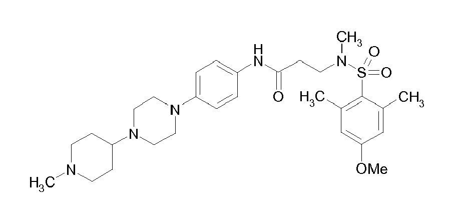

C₂₉H₄₃N₅O₄S (557.75)
[M+H]+ = 558
HPLC (Methode 1): Retentionszeit = 1.90 min

### Beispiel 232

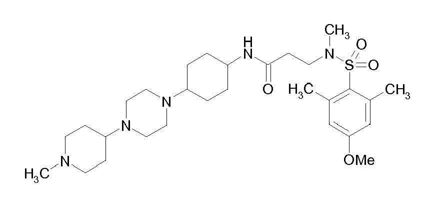

C₂₉H₄₉N₅O₄S (563.80)
[M+H]+ = 564
HPLC (Methode 5): Retentionszeit = 1.33 min

### Beispiel 233

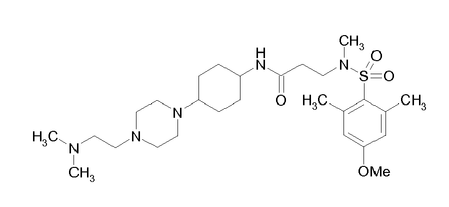

C₂₇H₄₇N₅O₄S x 2HCl (610.68)
[M+H]+ = 538
HPLC (Methode 7): Retentionszeit = 1.74 min

### Beispiel 234

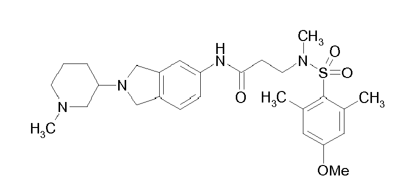

C₂₇H₃₈N₄O₄S (610.68)
[M+H]+ = 515
HPLC (Methode 5): Retentionszeit = 1.41 min

### Beispiel 235

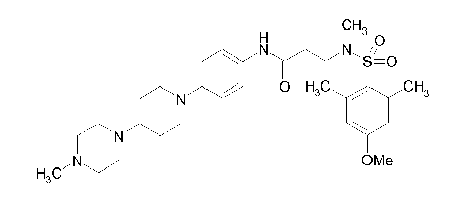

C₂₉H₄₃N₅O₄S (557.75)
[M+H]+ = 558
HPLC (Methode 5): Retentionszeit = 1.43 min

### Beispiel 236

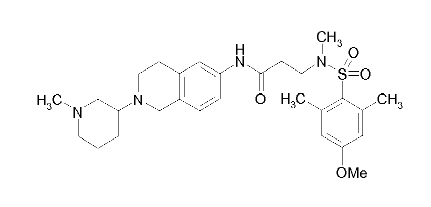

C₂₈H₄₀N₄O₄S x 2C₂HF₃O₂ (756.75)
[M+H]+ = 529
HPLC (Methode 5): Retentionszeit = 1.42 min

### Beispiel 237

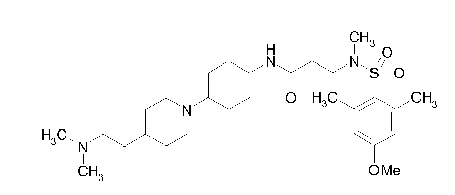

C₂₈H₄₈N₄O₄S x 2HCl (609.69)
[M+H]+ = 537
HPLC (Methode 7): Retentionszeit = 1.70 min

### Beispiel 238

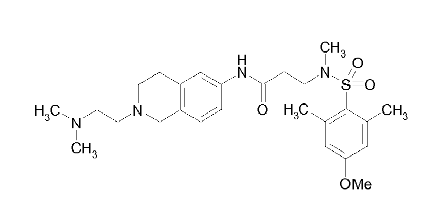

C₂₆H₃₈N₄O₄S x 2C₂HF₃O₂ (730.72)
[M+H]+ = 503
HPLC (Methode 5): Retentionszeit = 1.40 min

### Beispiel 239

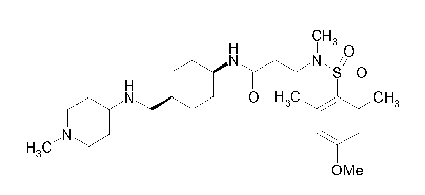

C₂₆H₄₄N₄O₄S x 2HCl (581.64)
[M+H]+ = 509
HPLC (Methode 5): Retentionszeit = 1.38 min

### Beispiel 240

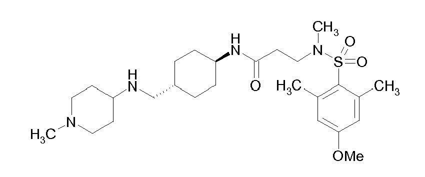

C₂₆H₄₄N₄O₄S x 2HCl (581.64)
[M+H]+ = 509
HPLC (Methode 5): Retentionszeit = 1.40 min

### Beispiel 241

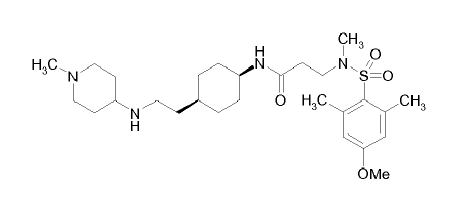

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.13

### Beispiel 242

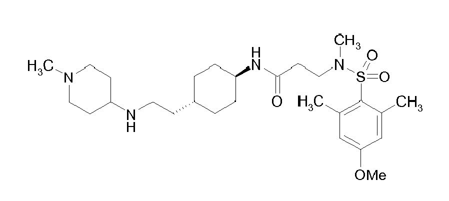

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.13

### Beispiel 243

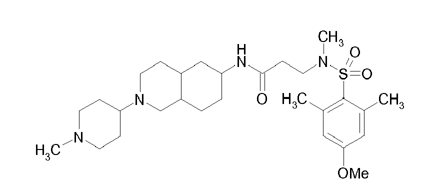

C₂₈H₄₆N₄O₄S x 2HCl (607.68)
[M+H]+ = 535
HPLC (Methode 5): Retentionszeit = 1.10 min

### Beispiel 244

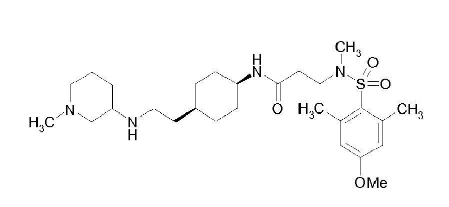

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.25

### Beispiel 245

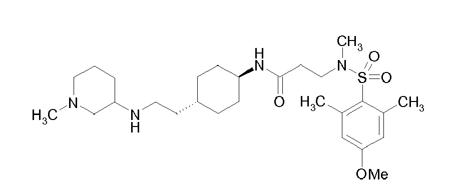

C₂₇H₄₆N₄O₄S x 2HCl (595.67)
[M+H]+ = 523
DC: Kieselgel, Dichlormethan / Methanol / Ammoniak 9:1:0.1, Rf-Wert = 0.25

### Beispiel 246

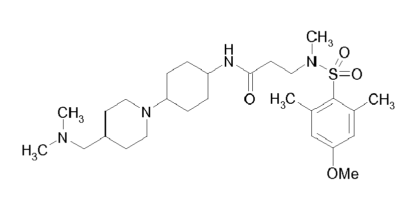

C₂₇H₄₆N₄O₄S x 2C₂HF₃O₂ (750.79)
[M+H]+ = 523
HPLC (Methode 5): Retentionszeit = 1.38 min

### Beispiel 247

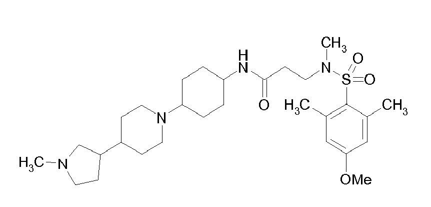

C₂₉H₄₈N₄O₄S x 2C₂HF₃O₂ (776.83)
[M+H]+ = 549
HPLC (Methode 5): Retentionszeit = 1.39 min

### Beispiel 248

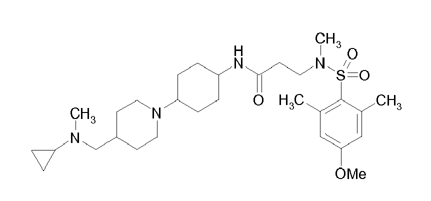

C₂₉H₄₈N₄O₄S x 2C₂HF₃O₂ (776.83)
[M+H]+ = 549
HPLC (Methode 5): Retentionszeit = 1.39 min

### Beispiel 249

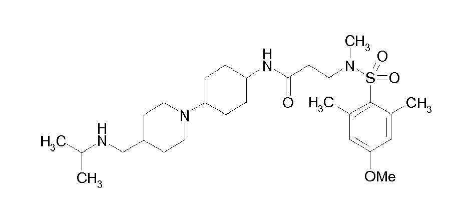

C₂₈H₄₈N₄O₄S x 2HCl (609.69)
[M+H]+ = 537
HPLC (Methode 5): Retentionszeit = 1.38 min

### Beispiel 250

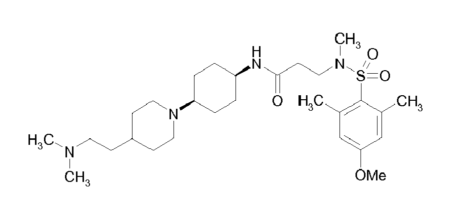

C₂₈H₄₈N₄O₄S x 2HCl (609.69)
[M+H]+ = 537
HPLC (Methode 11): Retentionszeit = 1.60 min

### Beispiel 251

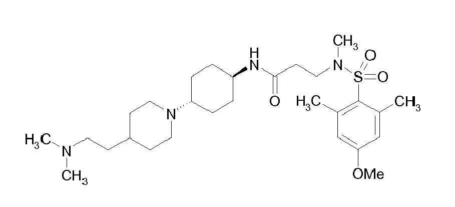

C₂₈H₄₈N₄O₄S x 2HCl (609.69)
[M+H]+ = 537
HPLC (Methode 7): Retentionszeit = 1.71 min

### Beispiel 252

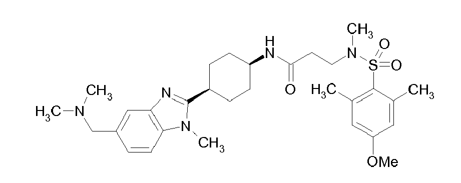

C₃₀H₄₃N₅O₄S x HCl (606.22)
[M+H]+ = 570
DC: Kieselgel, Dichlormethan / Methanol / Ammoniak 9:1:0.1, Rf-Wert = 0.29

### Beispiel 253

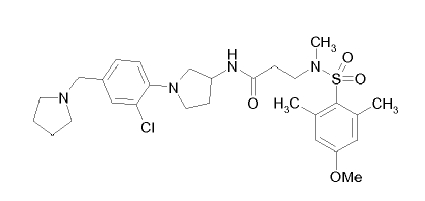

C₂₈H₃₉CIN₄O₄S x HCl (599.61)
[M+H]+ = 563/565
HPLC (Methode 5): Retentionszeit = 1.59 min

### Beispiel 254

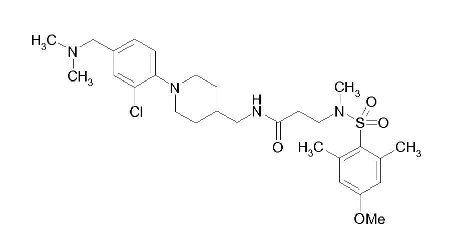

C₂₈H₄₁CIN₄O₄S x HCl (601.63)
[M+H]+ = 565/567
DC: Kieselgel, Dichlormethan / Methanol / Ammoniak 9:1:0.1, Rf-Wert = 0.46

### Beispiel 255

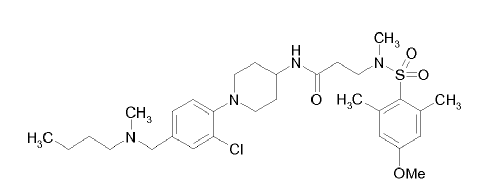

C₃₀H₄₅CIN₄O₄S x HCl (629.68)
[M+H]+ = 593/595
DC: Kieselgel, Dichlormethan / Methanol 9:1, Rf-Wert = 0.42

### Beispiel 256

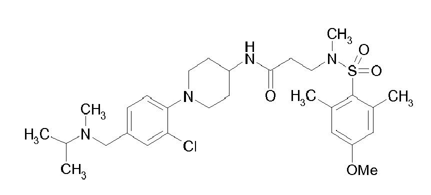

C₂₉H₄₃CIN₄O₄S x HCl (615.66)
[M+H]+ = 579/581
DC: Kieselgel, Dichlormethan / Methanol 9:1, Rf-Wert = 0.23

### Beispiel 257

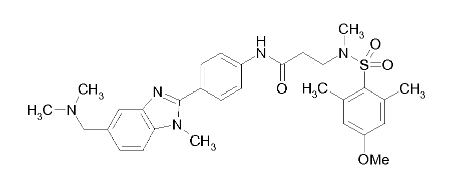

C₃₀H₃₇N₅O₄S x HCl (600.17)
[M+H]+ = 564
DC: Kieselgel, Dichlormethan / Methanol / Ammoniak 8:2:0.1, Rf-Wert = 0.67

### Beispiel 258

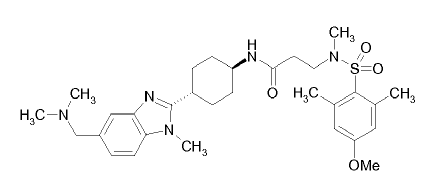

C₃₀H₄₃N₅O₄S (569.76)
[M+H]+ = 570
DC: Kieselgel, Dichlormethan / Methanol / Ammoniak 8:2:0.2, Rf-Wert = 0.65

### Beispiel 259

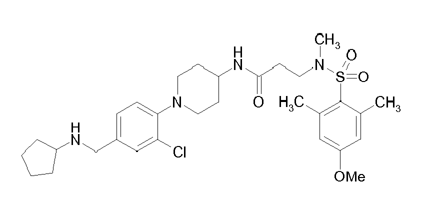

C₃₀H₄₃ClN₄O₄S x HCl (627.67)
[M+H]+ = 591/593
DC: Kieselgel, Dichlormethan / Methanol 9:1, Rf-Wert = 0.20

### Beispiel 260

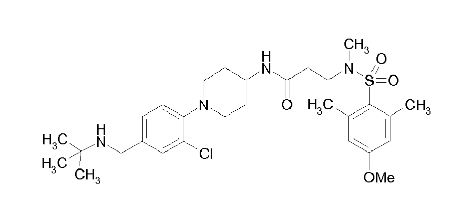

C₂₉H₄₃ClN₄O₄S x HCl (615.66)
[M+H]+ = 579/581
DC: Kieselgel, Dichlormethan / Methanol 9:1, Rf-Wert = 0.17

### Beispiel 261

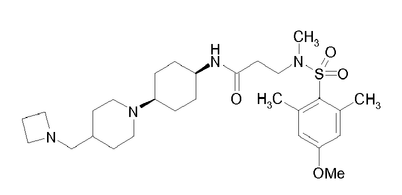

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.23

### Beispiel 262

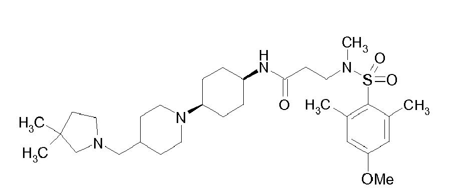

C₃₁H₅₂N₄O₄S x 2HCl (649.76)
[M+H]+ = 577
DC: Kieselgel, Dichlormethan / Methanol / Ammoniak 8:2:0.2, Rf-Wert = 0.69

### Beispiel 263

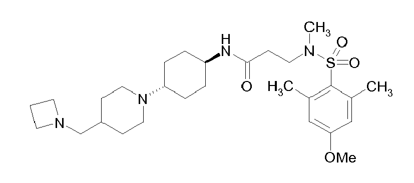

C₂₈H₄₆N₄O₄S (534.76)
[M+H]+ = 535
DC: Kieselgel, Dichlormethan / Methanol / Ammoniak 8:2:0.2, Rf-Wert = 0.58

### Beispiel 264

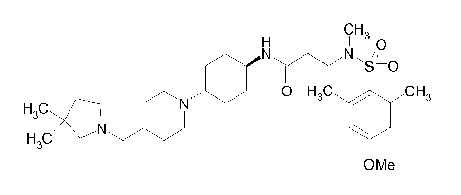

C₃₁H₅₂N₄O₄S (576.84)
[M+H]+ = 577
DC: Kieselgel, Dichlormethan / Methanol / Ammoniak 8:2:0.2, Rf-Wert = 0.56

### Beispiel 265

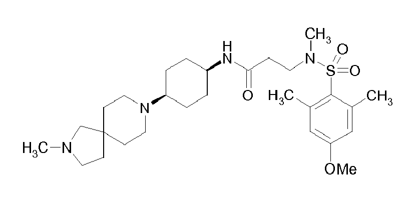

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.58

### Beispiel 266

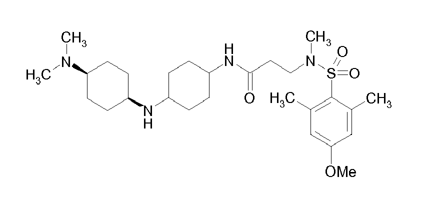

C₂₇H₄₆N₄O₄S (522.74)
[M+H]+ = 523
HPLC (Methode 9): Retentionszeit = 1.30 min

### Beispiel 267

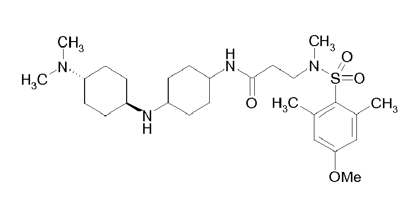

C₂₇H₄₆N₄O₄S (522.74)
[M+H]+ = 523
HPLC (Methode 9): Retentionszeit = 1.28 min

### Beispiel 268

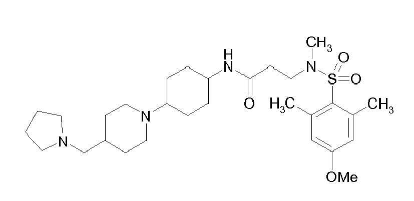

C₂₉H₄₈N₄O₄S x CH₂O₂ (594.81)
[M+H]+ = 549
HPLC (Methode 6): Retentionszeit = 1.96 min

### Beispiel 269

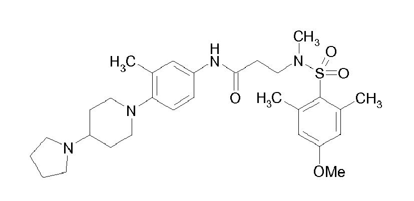

C₃₀₉H₄₄N₄O₄S (556.76)
[M+H]+ = 557
HPLC (Methode 9): Retentionszeit = 1.71 min

### Beispiel 270

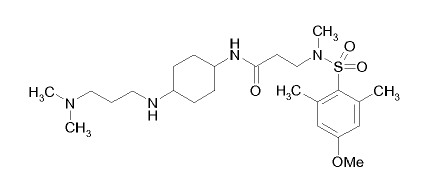

C₂₄H₄₂N₄O₄S x C₂HF₃O₂ (596.70)
[M+H]+ = 483
HPLC (Methode 9): Retentionszeit = 1.23 min

### Beispiel 271

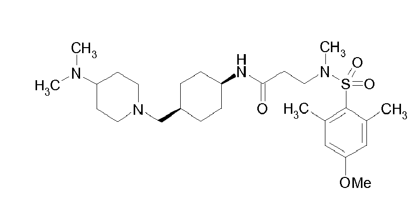

C₂₇H₄₆N₄O₄S (522.74)
[M+H]+ = 523
HPLC (Methode 9): Retentionszeit = 1.23 min

### Beispiel 272

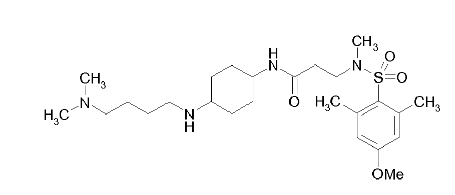

C₂₅H₄₄N₄O₄S (496.71)
[M+H]+ = 497
HPLC (Methode 9): Retentionszeit = 1.25 min

### Beispiel 273

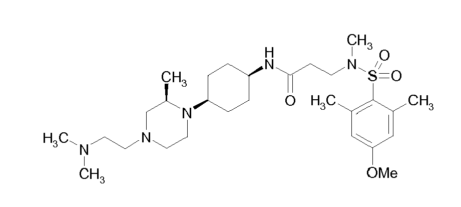

C₂₈H₄₉N₅O₄S x 2HCl (624.71)
[M+H]+ = 552
HPLC (Methode 10): Retentionszeit = 1.06 min

### Beispiel 274

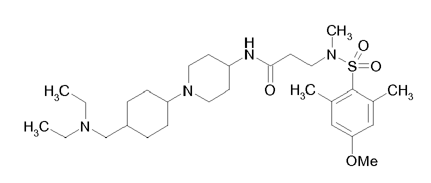

C₂₉H₅₀N4O₄S x CH₂O₂ (596.82)
[M+H]+ = 551
HPLC (Methode 9): Retentionszeit = 1.31 min

### Beispiel 275

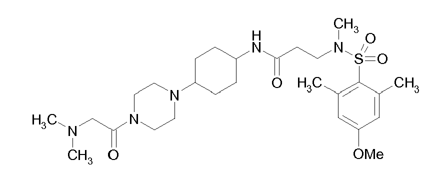

C₂₇H₄₅N₅O₅S x CH₂O₂ (597.77)
[M+H]+ = 552
HPLC (Methode 9): Retentionszeit = 1.20 min

### Beispiel 276

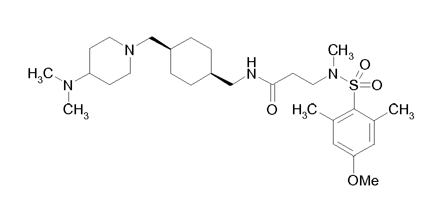

C₂₈H₄₈N₄O₄S (536.77)
[M+H]+ = 537
HPLC (Methode 6): Retentionszeit = 1.32 min

### Beispiel 277

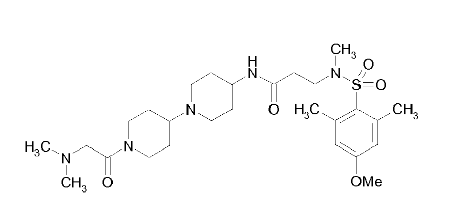

C₂₇H₄₅N₅O₅S x C₂HF₃O₂ (665.77)
[M+H]+ = 552
HPLC (Methode 9): Retentionszeit = 1.18 min

### Beispiel 278

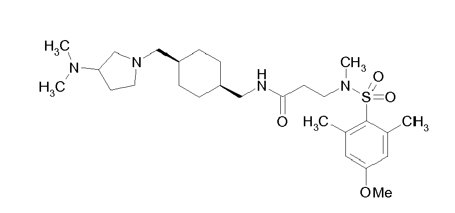

C₂₇H₄₆N₄O₄S x 2 C₂HF₃O₂ (750.79)
[M+H]+ = 523
HPLC (Methode 9): Retentionszeit = 1.29 min

### Beispiel 279

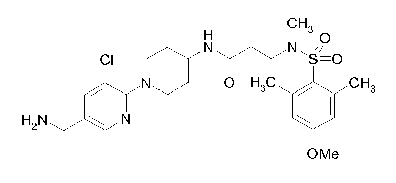

C₂₄H₃₄ClN₅O₄S (524.08)
[M+H]+ = 524/526
HPLC (Methode 9): Retentionszeit = 1.60 min

### Beispiel 280

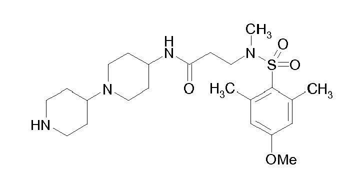

C₂₃H₃₈N₄O₄S x 2C₂HF₃O₂ (694.69)
[M+H]+ = 467
HPLC (Methode 9): Retentionszeit = 1.16 min

### Beispiel 281

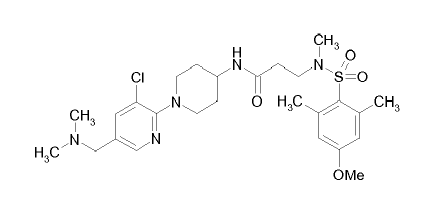

C₂₆H₃₈ClN₅O₄S x C₂HF₃O₂ (666.15)
[M+H]+ = 552/554
HPLC (Methode 9): Retentionszeit = 1.68 min

### Beispiel 282

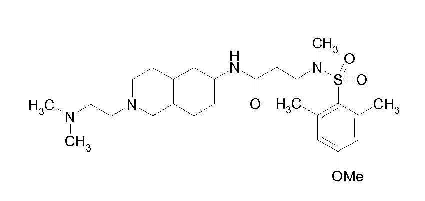

C₂₆H₄₄N₄O₄S x 2HCl (581.64)
[M+H]+ = 509
HPLC (Methode 5): Retentionszeit = 1.36 min

### Beispiel 283

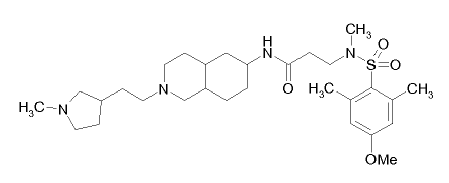

C₂₉H₄₈N₄O₄S x 2HCl (621.70)
[M+H]+ = 549
HPLC (Methode 5): Retentionszeit = 1.38 min

### Beispiel 284

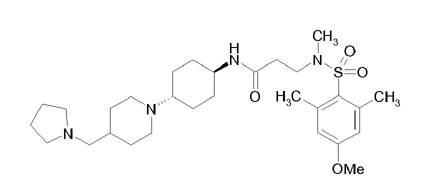

C₂gH₄₈N₄O₄S (548.78)
[M+H]+ = 549
HPLC (Methode 5): Retentionszeit = 1.38 min

### Beispiel 285

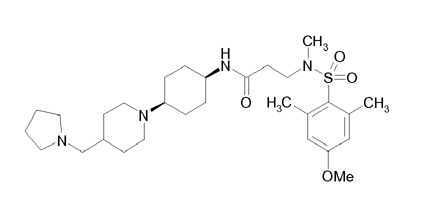

C₂₉H₄₈N₄O₄S (548.78)
[M+H]+ = 549
HPLC (Methode 5): Retentionszeit = 1.36 min

### Beispiel 286

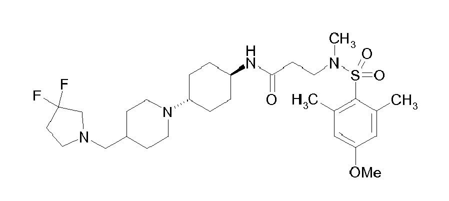

C₂₉H₄₆F₂N₄O₄S (584.76)
[M+H]+ = 585
HPLC (Methode 5): Retentionszeit = 1.38 min

### Beispiel 287

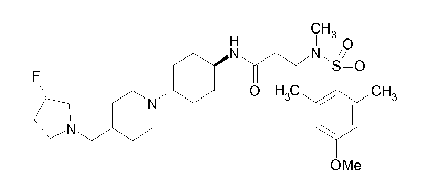

C₂₉H₄₇FN₄O₄S (566.77)
[M+H]+ = 567
HPLC (Methode 5): Retentionszeit = 1.38 min

### Beispiel 288

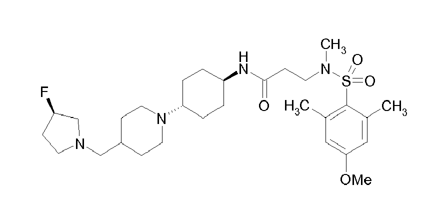

C₂₉H₄₇FN₄O₄S (566.77)
[M+H]+ = 567
HPLC (Methode 5): Retentionszeit = 1.36 min

### Beispiel 289

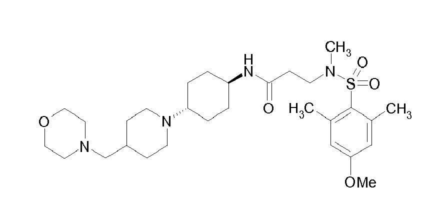

C₂₉H₄₈N₄O₅S (564.78)
[M+H]+ = 565
HPLC (Methode 5): Retentionszeit = 1.37 min

### Beispiel 290

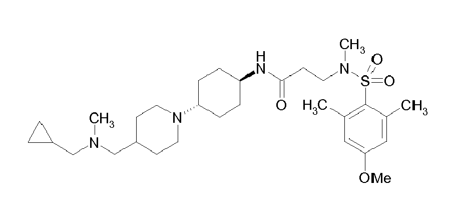

C₃₀H₅₀N₄O₄S (562.81)
[M+H]+ = 563
HPLC (Methode 5): Retentionszeit = 1.39 min

### Beispiel 291

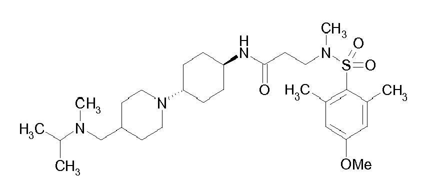

C₂₉H₅₀N₄O₄S (550.80)
[M+H]+ = 551
HPLC (Methode 5): Retentionszeit = 1.36 min

### Beispiel 292

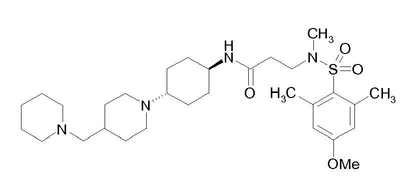

C₃₀H₅₀N₄O₄S (562.81)
[M+H]+ = 563
HPLC (Methode 5): Retentionszeit = 1.28 min

### Beispiel 293

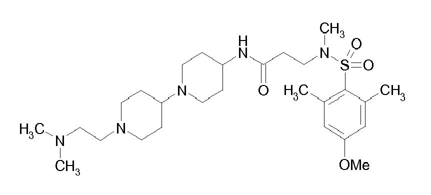

C₂₇H₄₇N₅O₄S x 3C₂HF₃O₂ (879.83)
[M+H]+ = 538
HPLC (Methode 5): Retentionszeit = 1.33 min

### Beispiel 294

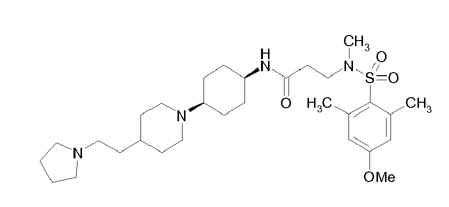

C₃₀H₅₀N₄O₄S x 2HCl (635.73)
[M+H]+ = 563
HPLC (Methode 5): Retentionszeit = 1.37 min

### Beispiel 295

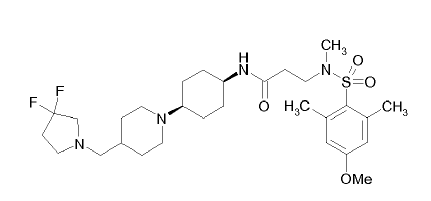

C₂₉H₄₆F₂N₄O₄S x 2C₂HF₃O₂ (584.76)
[M+H]+ = 585
HPLC (Methode 5): Retentionszeit = 1.38 min

### Beispiel 296

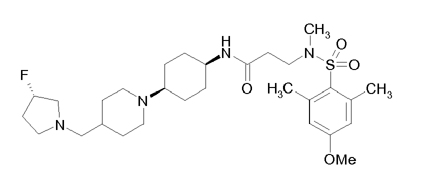

C₂₉H₄₇FN₄O₄S x 2C₂HF₃O₂ (794.82)
[M+H]+ = 567
HPLC (Methode 5): Retentionszeit = 1.35 min

### Beispiel 297

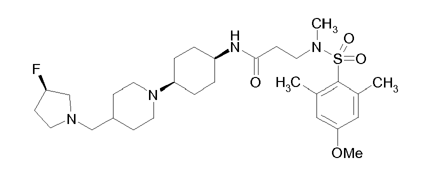

C₂₉H₄₇FN₄O₄S x 2C₂HF₃O₂ (794.82)
[M+H]+ = 567
HPLC (Methode 5): Retentionszeit = 1.36 min

### Beispiel 298

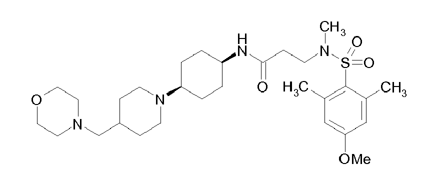

C₂₉H₄₈N₄O₅S (564.78)
[M+H]+ = 565
HPLC (Methode 5): Retentionszeit = 1.36 min

### Beispiel 299

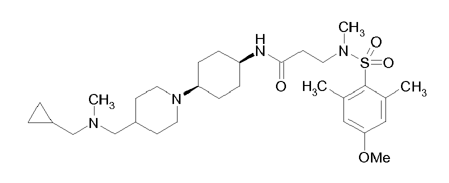

C₃₀H₅₀N₄O₄S (562.81)
[M+H]+ = 563
HPLC (Methode 5): Retentionszeit = 1.37 min

### Beispiel 300

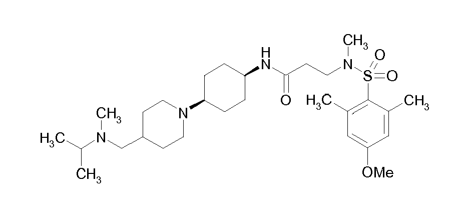

C₂₉H₅₀N₄O₄S (550.80)
[M+H]+ = 551
HPLC (Methode 5): Retentionszeit = 1.37 min

### Beispiel 301

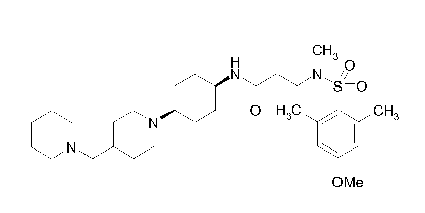

C₃₀H₅₀N₄O₄S (562.81)
[M+H]+ = 563
HPLC (Methode 5): Retentionszeit = 1.37 min

### Beispiel 302

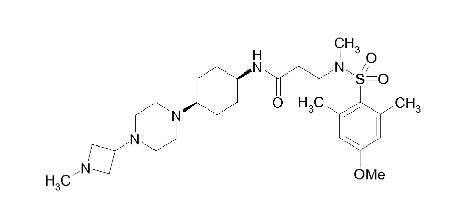

C₃₀H₅₀N₄O₄S x 3C₂HF₃O₂ (877.81)
[M+H]+ = 536
HPLC (Methode 5): Retentionszeit = 1.34 min

### Beispiel 303

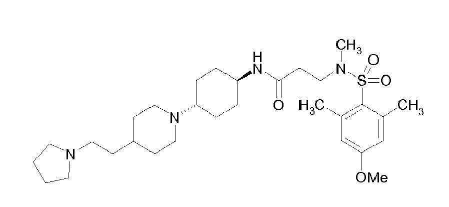

C₃₀H₅₀N₄O₄S (562.81)
[M+H]+ = 563
HPLC (Methode 5): Retentionszeit = 1.37 min

### Beispiel 304

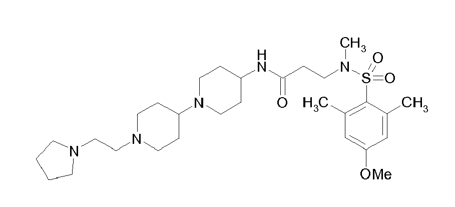

C₂₉H₄₉N₅O₄S x 3C₂HF₃O₂ (905.87)
[M+H]+ = 564
HPLC (Methode 5): Retentionszeit = 1.08 min

### Beispiel 305

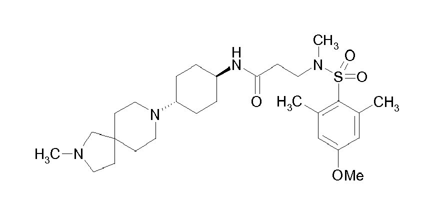

C₂₈H₄₆N₄O₄S x 2HCl (607.68)
[M+H]+ = 535
DC: Kieselgel, Dichlormethan / Methanol / Ammoniak 8:2:0.2, Rf-Wert = 0.19

### Beispiel 306

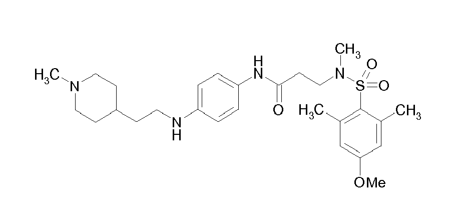

C₂₇H₄₀N₄O₄S x C₂HF₃O₄ (630.72)
[M+H]+ = 517
HPLC (Methode 9): Retentionszeit = 1.40 min

### Beispiel 307

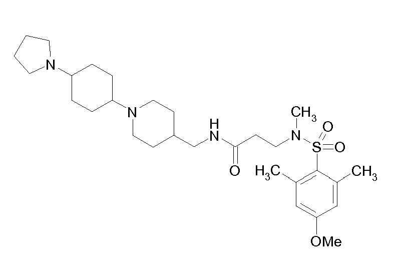

C₂₉H₄₈N₄O₄S (548.78)
[M+H]+ = 549
HPLC (Methode 6): Retentionszeit = 1.24 min

### Beispiel 308

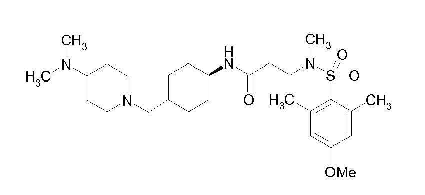

C₂₇H₄₆N₄O₄S (522.74)
[M+H]+ = 523
HPLC (Methode 9): Retentionszeit = 1.29 min

### Beispiel 309

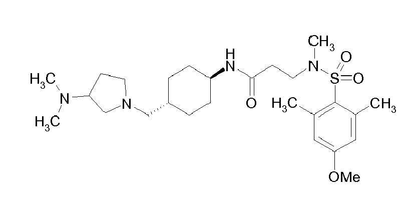

C₂₆H₄₄N₄O₄S (508.72)
[M+H]+ = 509
HPLC (Methode 9): Retentionszeit = 1.30 min

### Beispiel 310

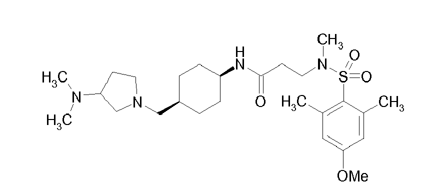

C₂₆H₄₄N₄O₄S (508.72)
[M+H]+ = 509
HPLC (Methode 9): Retentionszeit = 1.23 min

### Beispiel 311

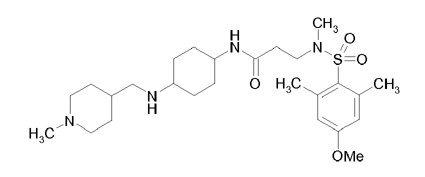

C₂₆H₄₄N₄O₄S (508.72)
[M+H]+ = 509
HPLC (Methode 6): Retentionszeit = 1.20 min

### Beispiel 312

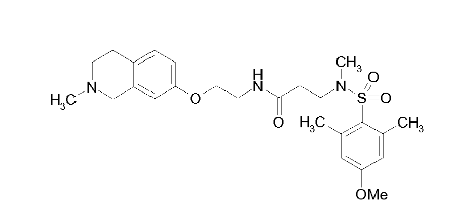

C₂₅H₃₅N₃O₅S x HCl (526.09)
[M+H]+ = 490
HPLC (Methode 10): Retentionszeit = 1.16 min

### Beispiel 313

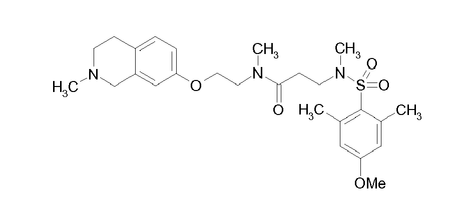

C₂₆H₃₇N₃O₅S x HCl (540.12)
[M+H]+ = 504
HPLC (Methode 10): Retentionszeit = 1.22 min

### Beispiel 314

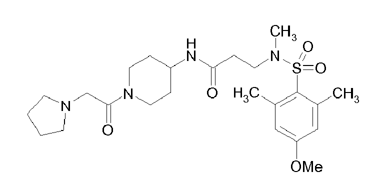

C₂₄H₃₈N₄O₅S x C₂HF₃O₂ (608.67)
[M+H]+ = 495
HPLC (Methode 9): Retentionszeit = 1.47 min

### Beispiel 315

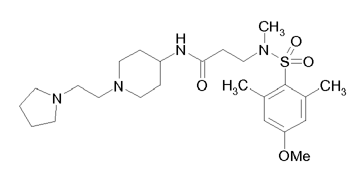

C₂₄H₄₀N₄O₄S (480.66)
[M+H]+ = 481
HPLC (Methode 9): Retentionszeit = 1.21 min

### Beispiel 316

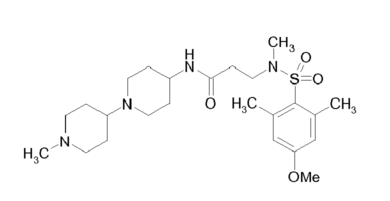

C₂₄H₄₀N₄O₄S x C₂HF₃O₂ (594.69)
[M+H]+ = 481
HPLC (Methode 9): Retentionszeit = 1.19 min

### Beispiel 317

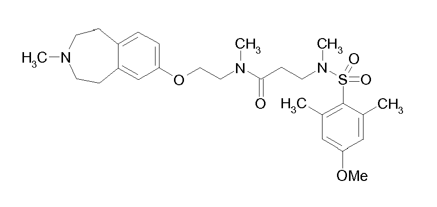

C₂₇H₃₉N₃O₅S x HCl (554.14)
[M+H]+ = 518
HPLC (Methode 5): Retentionszeit = 1.36 min

### Beispiel 593

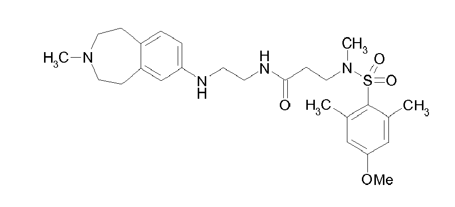

C₂₆H₃₈N₄O₄S x HCl (539.13)
[M+H]+ = 503
HPLC (Methode 5): Retentionszeit = 1.29 min

### Beispiel 594

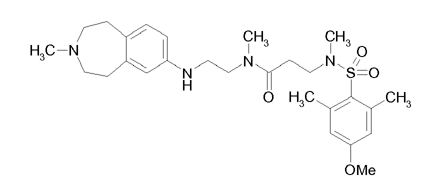

C₂₇H₄₀N₄O₄S x HCl (553.16)
[M+H]+ = 517
HPLC (Methode 5): Retentionszeit = 1.35 min

### Beispiel 595

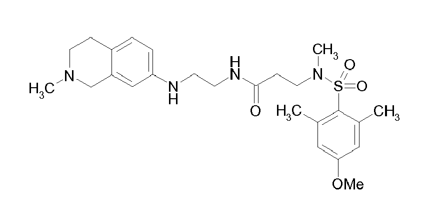

C₂₅H₃₆N₄O₄S x HCl (525.10)
[M+H]+ = 489
HPLC (Methode 5): Retentionszeit = 1.31 min

### Beispiel 596

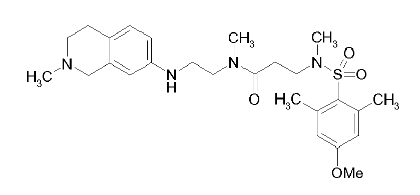

C₂₆H₃₈N₄O₄S x HCl (539.13)
[M+H]+ = 503
HPLC (Methode 5): Retentionszeit = 1.35 min

### Beispiel 597

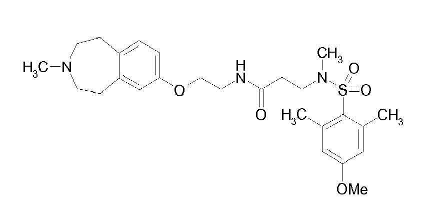

C₂₆H₃₇N₃O₅S x HCl (540.12)
[M+H]+ = 504
HPLC (Methode 10): Retentionszeit = 1.18 min

### Beispiel 598

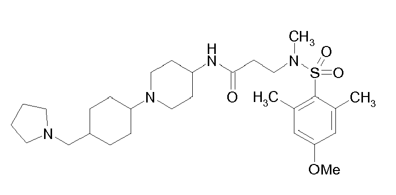

C₂₉H₄₈N₄O₄S x C₂HF₃O₂ (662.81)
[M+H]+ = 549
HPLC (Methode 9): Retentionszeit = 1.27 min

### Beispiel 599

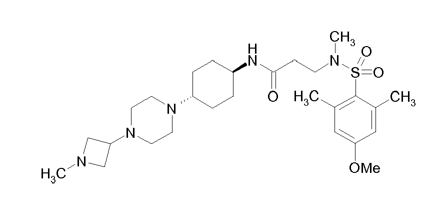

C₂₇H₄₅N₅O₄S x 3HCl (645.13)
[M+H]+ = 536
HPLC (Methode 5): Retentionszeit = 1.14 min

### Beispiel 600

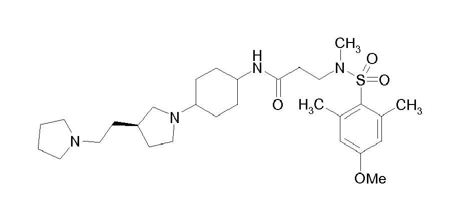

C₂₉H₄₈N₄O₄S x 2HCl (621.70)
[M+H]+ = 549
HPLC (Methode 5): Retentionszeit = 1.16 min

### Beispiel 601

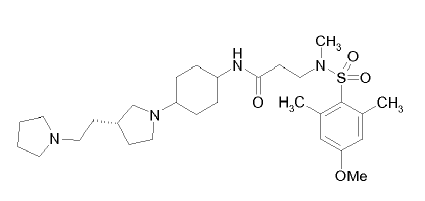

C₂₉H₄₈N₄O₄S (548.78)
[M+H]+ = 549
HPLC (Methode 5): Retentionszeit = 1.16 min

### Beispiel 602

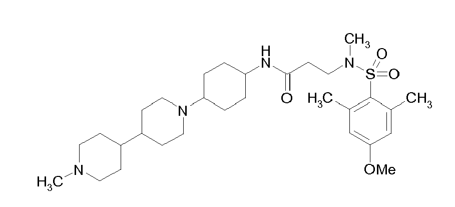

C₃₀H₅₀N₄O₄S x C₂HF₃O₂ (676.83)
[M+H]+ = 563
HPLC (Methode 9): Retentionszeit = 1.14 min

### Beispiel 608

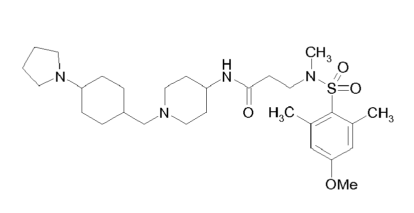

C₂₉H₄₈N₄O₄S x C₂HF₃O₂ (662.81)
[M+H]+ = 549
HPLC (Methode 9): Retentionszeit = 1.30 min

### Beispiel 609

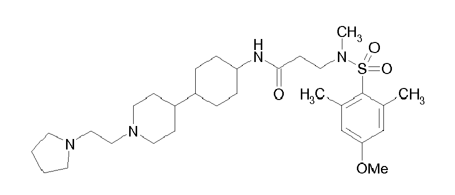

C₃₀H₅₀N₄O₄S x 2HCl (635.73)
[M+H]+ = 563
HPLC (Methode 11): Retentionszeit = 1.70 min

### Beispiel 610

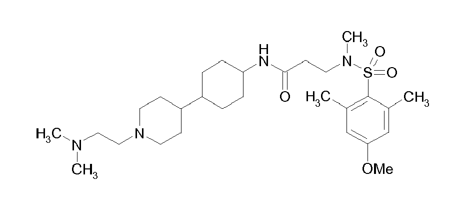

C₂₈H₄₈N₄O₄S x 2HCl (609.69)
[M+H]+ = 537
HPLC (Methode 11): Retentionszeit = 1.67 min

### Beispiel 611

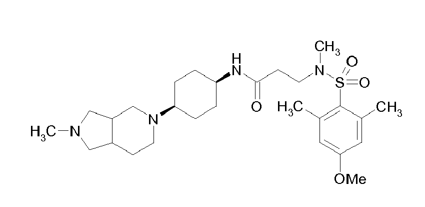

C₂₇H₄₄N₄O₄S x 2HCl (593.65)
[M+H]+ = 521
HPLC (Methode 11): Retentionszeit = 1.61 min

### Beispiel 636

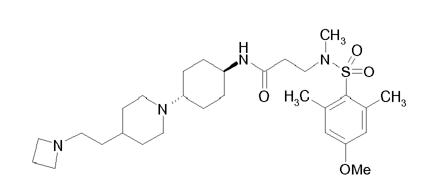

C₂₉H₄₈N₄O₄S x 2HCl (621.70)
[M+H]+ = 549
HPLC (Methode 4): Retentionszeit = 2.39 min

### Beispiel 637

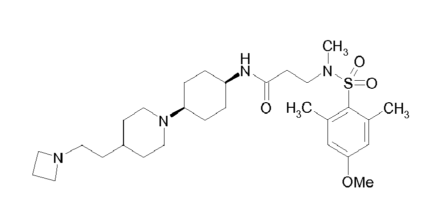

C₂₉H₄₈N₄O₄S x 2HCl (621.70)
[M+H]+ = 549
HPLC (Methode 4): Retentionszeit = 2.34 min

Die folgenden Verbindungen wurden analog zu Beispiel 53 hergestellt: Beispiel 318 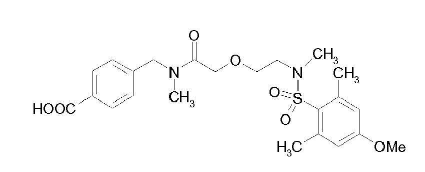 C₂₃H₃₀N₂O₇S (478.56)
[M+H]+ = 479
HPLC (Methode 6): Retentionszeit = 3.21 min

### Beispiel 319

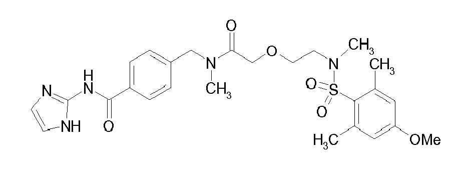

C₂₆H₃₃N₅O₆S (543.64)
[M+H]+ = 544
HPLC (Methode 6): Retentionszeit = 2.51 min

### Beispiel 320

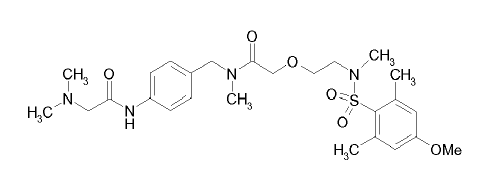

C₂₆H₃₈N₄O₆S (534.67)
[M+H]+ = 535
HPLC (Methode 6): Retentionszeit = 2.48 min

### Beispiel 321

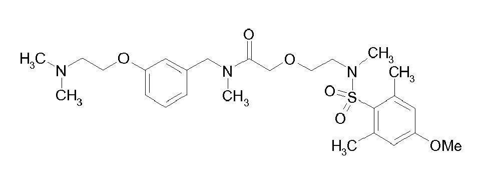

C₂₆H₃₉₈N₃O₆S (521.67)
[M+H]+ = 522
HPLC (Methode 6): Retentionszeit = 2.60 min

### Beispiel 322

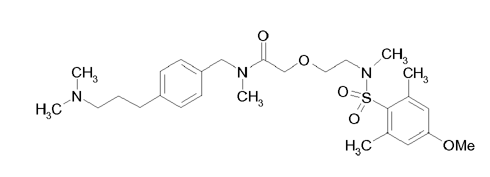

C₂₇H₄₁N₃O₅S (519.70)
[M+H]+ = 520
HPLC (Methode 6): Retentionszeit = 2.61 min

### Beispiel 323

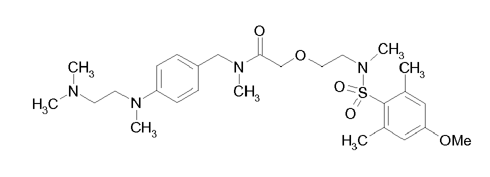

C₂₇H₄₂N₄O₅S x HCl (571.17)
[M+H]+ = 535
HPLC (Methode 5): Retentionszeit = 1.57 min

### Beispiel 324

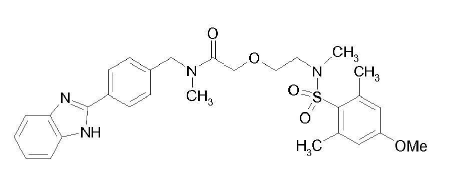

C₂₉H₃₄N₄O₅S x C₂HF₃O₂ (664.69)
[M+H]+ = 551
HPLC (Methode 5): Retentionszeit = 1.60 min

### Beispiel 325

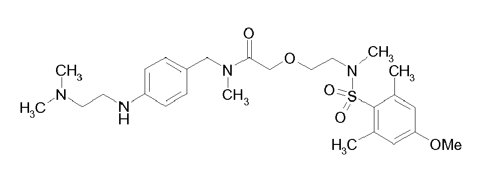

C₂₆H₄₀N₄O₅S x HCl (557.15)
[M+H]+ = 521
HPLC (Methode 5): Retentionszeit = 1.53 min

### Beispiel 326

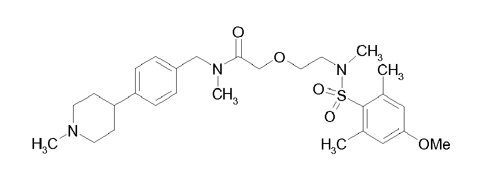

C₂₈H₄₁N₃O₅S x C₂HF₃O₂ (645.73)
[M+H]+ = 532
HPLC (Methode 5): Retentionszeit = 1.54 min

### Beispiel 327

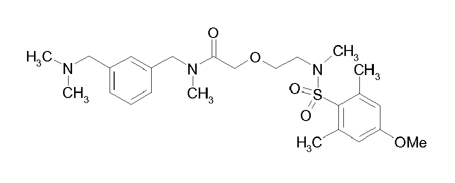

C₂₅H₃₇N₃O₅S x HCl (528.11)
[M+H]+ = 492
HPLC (Methode 5): Retentionszeit = 1.53 min

### Beispiel 328

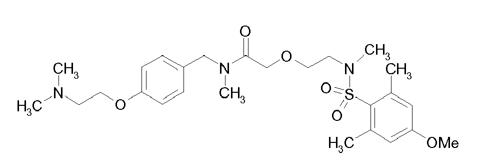

C₂₆H₃₉N₃O₆S x C₂HF₃O₂ (635.69)
[M+H]+ = 522
HPLC (Methode 5): Retentionszeit = 1.53 min

### Beispiel 329

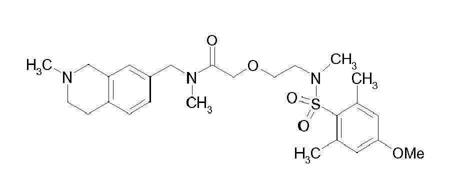

C₂₆H₃₇N₃O₅S x HCl (540.12)
[M+H]+ = 504
HPLC (Methode 7): Retentionszeit = 1.93 min

### Beispiel 330

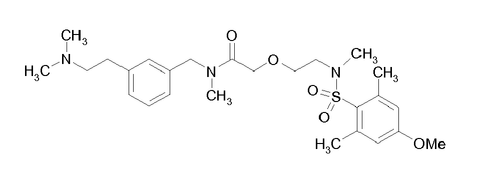

C₂₆H₃₉N₃O₅S x HCl (542.13)
[M+H]+ = 506
HPLC (Methode 5): Retentionszeit = 1.54 min

### Beispiel 331

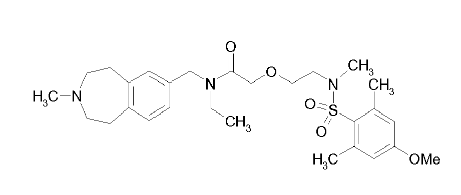

C₂₈H₄₁N₃O₅S x HCl (568.17)
[M+H]+ = 532
DC: Kieselgel, Dichlormethan / Methanol / Ammoniak 9:1:0.1, Rf-Wert = 0.30

### Beispiel 332

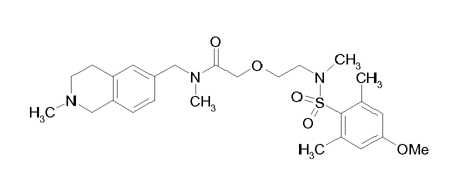

C₂₆H₃₇N₃O₅S x HCl (540.12)
[M+H]+ = 504
HPLC (Methode 5): Retentionszeit = 1.51 min

### Beispiel 333

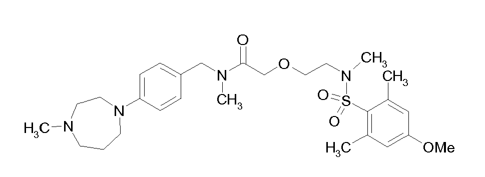

C₂₈H₄₂N₄O₅S x HCl (583.18)
[M+H]+ = 547
HPLC (Methode 5): Retentionszeit = 1.54 min

### Beispiel 334

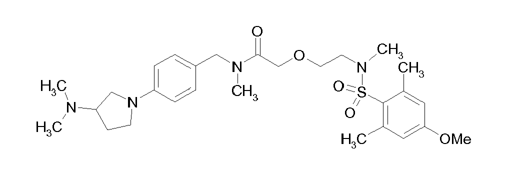

C₂₈H₄₂N₄O₅S x HCl (583.18)
[M+H]+ = 547
HPLC (Methode 10): Retentionszeit = 1.24 min

### Beispiel 335

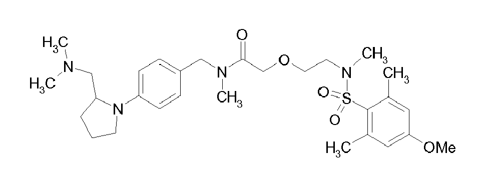

C₂₉H₄₄N₄O₅S x HCl (597.21)
[M+H]+ = 561
HPLC (Methode 5): Retentionszeit = 1.55 min

### Beispiel 336

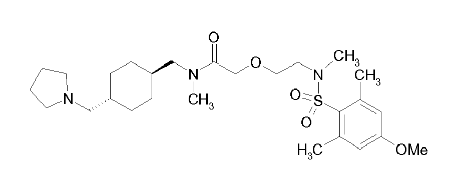

C₂₇H₄₅N₃O₅S x HCl (560.19)
[M+H]+ = 524
DC: Kieselgel, Dichlormethan / Methanol / Ammoniak 8:2:0.01, Rf-Wert = 0.39

### Beispiel 337

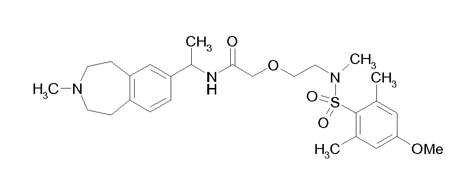

C₂₇H₃₉N₃O₅S x HCl (554.14)
[M+H]+ = 518
HPLC (Methode 4): Retentionszeit = 3.4 min

### Beispiel 338

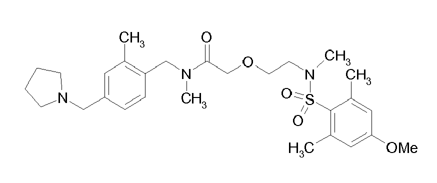

C₂₈H₄₁N₃O₅S x HCl (568.17)
[M+H]+ = 532
DC: Kieselgel, Dichlormethan / Methanol 9:1, Rf-Wert = 0.14

### Beispiel 339

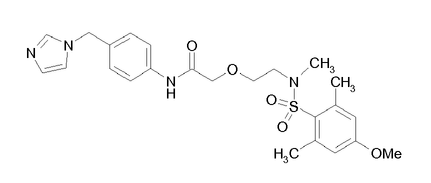

C₂₄H₃₀N₄O₅S x C₂HF₃O₂ (600.61)
[M+H]+ = 487
HPLC (Methode 6): Retentionszeit = 2.58 min

### Beispiel 340

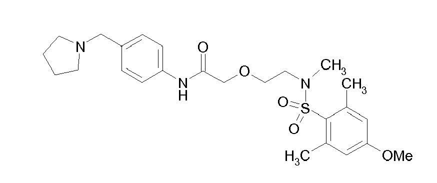

C₂₅H₃₅N₃O₅S x C₂HF₃O₂ (603.65)
[M+H]+ = 490
HPLC (Methode 6): Retentionszeit = 2.61 min

### Beispiel 341

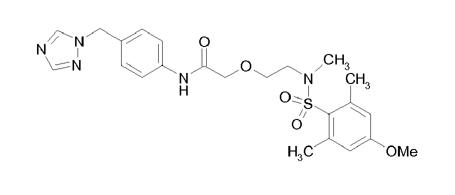

C₂₃H₂₉N₅O₅S x C₂HF₃O₂ (601.60)
[M+H]+ = 488
HPLC (Methode 6): Retentionszeit = 3.28 min

### Beispiel 342

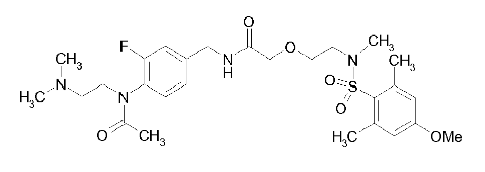

C₂₇H₃₉FN₄O₆S (566.69)
[M+H]+ = 567
HPLC (Methode 6): Retentionszeit = 2.59 min

### Beispiel 343

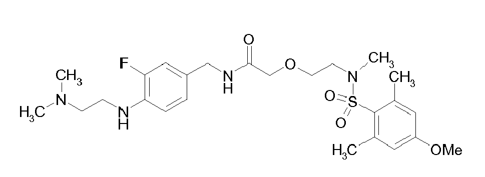

C₂₅H₃₇FN₄O₅S (524.65)
[M+H]+ = 525
HPLC (Methode 6): Retentionszeit = 2.59 min

### Beispiel 344

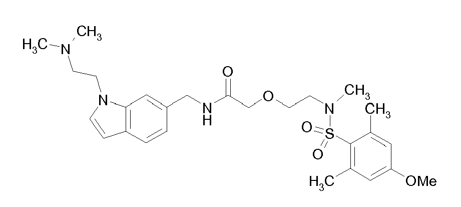

C₂₇H₃₈N₄O₅S (530.68)
[M+H]+ = 531
HPLC (Methode 6): Retentionszeit = 2.65 min

### Beispiel 345

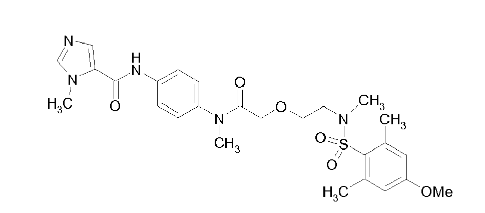

C₂₆H₃₃N₅O₆S (543.64)
[M+H]+ = 544
HPLC (Methode 6): Retentionszeit = 2.39 min

### Beispiel 346

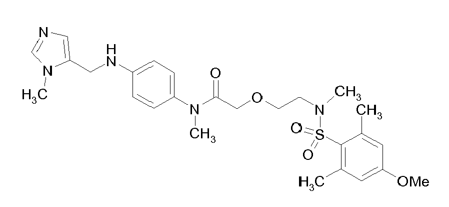

C₂₆H₃₅N₅O₅S (529.65)
[M+H]+ = 530
HPLC (Methode 6): Retentionszeit = 2.43 min

### Beispiel 347

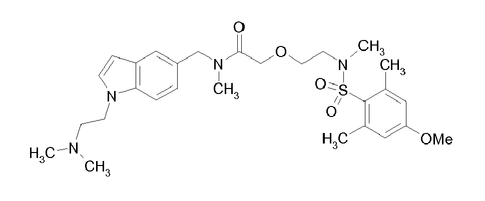

C₂₈H₄₀N₄O₅S (544.71)
[M+H]+ = 545
HPLC (Methode 6): Retentionszeit = 2.65 min

### Beispiel 348

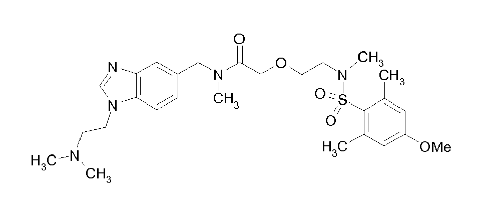

C₂₇H₃₉N₅O₅S (545.70)
[M+H]+ = 546
HPLC (Methode 6): Retentionszeit = 2.19 min

### Beispiel 349

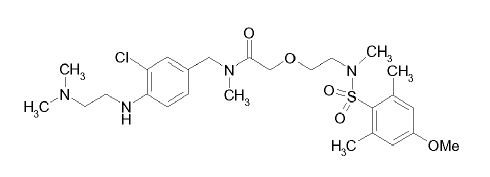

C₂₆H₃₉ClN₄O₅S (555.13)
[M+H]+ = 555/557
HPLC (Methode 6): Retentionszeit = 2.63 min

### Beispiel 350

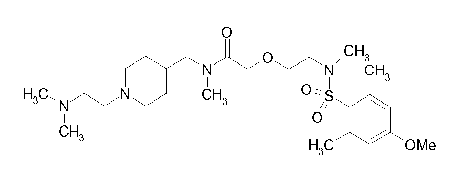

C₂₅H₄₄N₄O₅S (512.71)
[M+H]+ = 513
HPLC (Methode 6): Retentionszeit = 1.94 min

### Beispiel 351

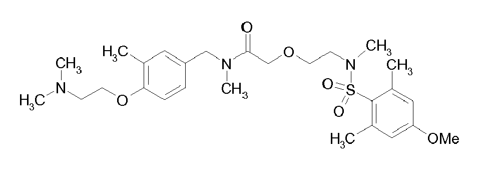

C₂₇H₄₁N₃O₆S (535.70)
[M+H]+ = 536
HPLC (Methode 6): Retentionszeit = 2.56 min

### Beispiel 352

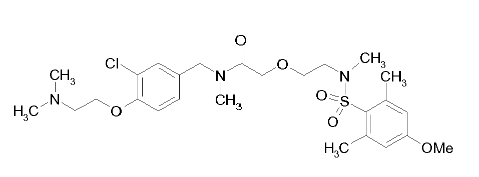

C₂₆H₃₈ClN₃O₆S x CH₂O₂ (602.14)
[M+H]+ = 556/558
HPLC (Methode 6): Retentionszeit = 2.65 min

### Beispiel 353

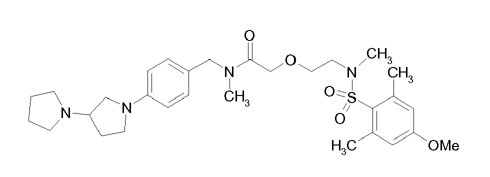

C₃₀H₄₄N₄O₅S (572.76)
[M+H]+ = 573
HPLC (Methode 6): Retentionszeit = 2.69 min

### Beispiel 354

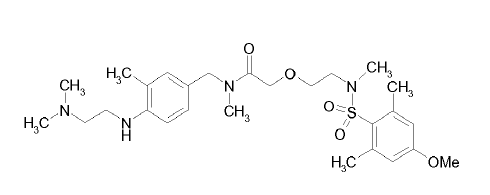

C₂₇H₄₂N₄O₅S (534.71)
[M+H]+ = 535
HPLC (Methode 6): Retentionszeit = 2.54 min

### Beispiel 355

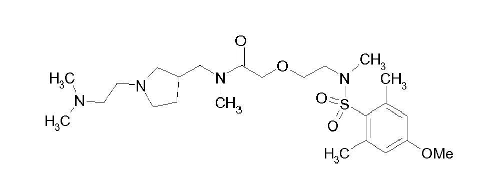

C₂₄H₄₂N₄O₅S (498.68)
[M+H]+ = 499
HPLC (Methode 6): Retentionszeit = 1.95 min

### Beispiel 356

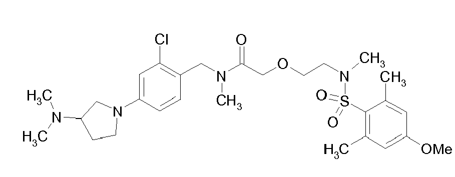

C₂₈H₄₁ClN₄O₅S (581.17)
[M+H]+ = 581/583
HPLC (Methode 6): Retentionszeit = 2.77 min

### Beispiel 357

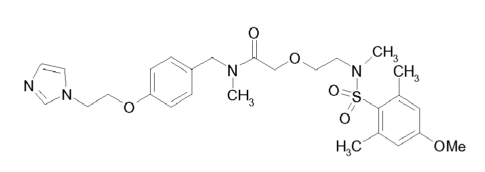

C₂₇H₃₆N₄O₆S (544.66)
[M+H]+ = 545
HPLC (Methode 6): Retentionszeit = 2.59 min

### Beispiel 358

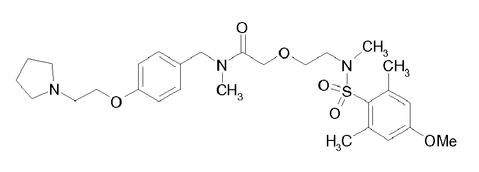

C₂₈H₄₁N₃O₆S (547.71)
[M+H]+ = 548
HPLC (Methode 6): Retentionszeit = 2.59 min

### Beispiel 359

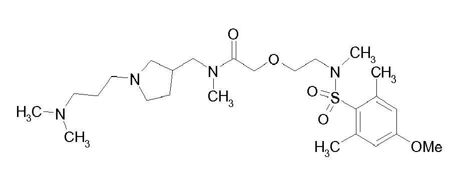

C₂₅H₄₄N₄O₅S (512.71)
[M+H]+ = 513
HPLC (Methode 6): Retentionszeit = 1.94 min

### Beispiel 360

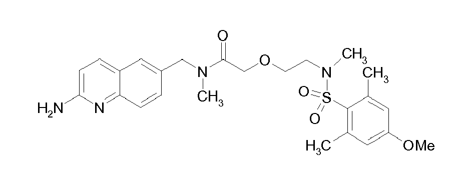

C₂₅H₃₂N₄O₅S (500.61)
[M+H]+ = 501
HPLC (Methode 6): Retentionszeit = 2.41 min

### Beispiel 361

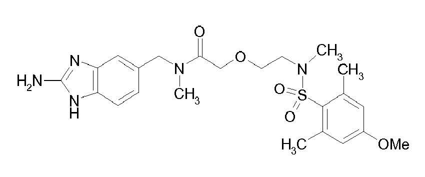

C₂₃H₃₁N₅O₅S (489.59)
[M+H]+ = 490
HPLC (Methode 6): Retentionszeit = 2.46 min

### Beispiel 362

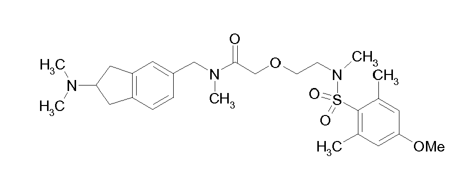

C₂₇H₃₉N₃O₅S x C₂HF₃O₂ (631.71)
[M+H]+ = 518
HPLC (Methode 6): Retentionszeit = 2.54 min

### Beispiel 363

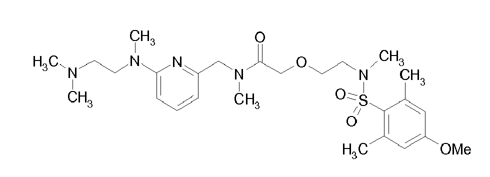

C₂₆H₄₁N₅O₅S x CH₂O₂ (581.73)
[M+H]+ = 536
HPLC (Methode 6): Retentionszeit = 2.58 min

### Beispiel 364

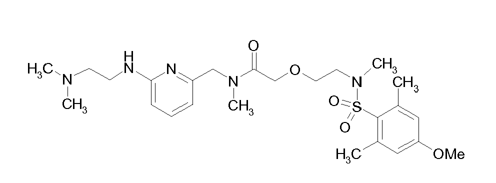

C₂₅H₃₉N₅O₅S x CH₂O₂ (567.70)
[M+H]+ = 522
HPLC (Methode 6): Retentionszeit = 2.31 min

### Beispiel 365

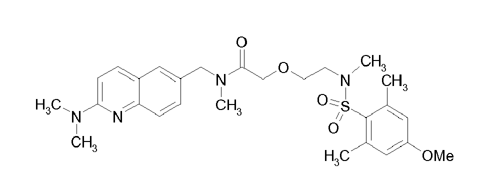

C₂₇H₃₆N₄O₅S x C₂HF₃O₂ (642.69)
[M+H]+ = 529
HPLC (Methode 6): Retentionszeit = 2.56 min

### Beispiel 366

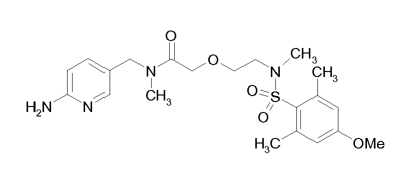

C₂₁H₃₀N₄O₅S (450.55)
[M+H]+ = 451
HPLC (Methode 6): Retentionszeit = 2.31 min

### Beispiel 367

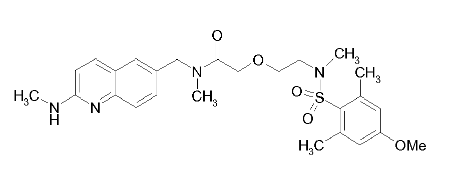

C₂₆H₃₄N₄O₅S (514.64)
[M+H]+ = 515
HPLC (Methode 6): Retentionszeit = 2.51 min

### Beispiel 368

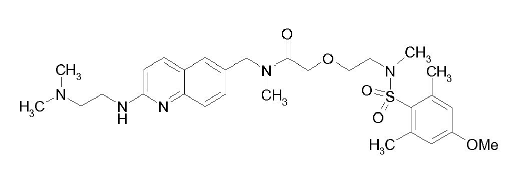

C₂₉H₄₁N₅O₅S x C₂HF₃O₂ (685.76)
[M+H]+ = 572
HPLC (Methode 6): Retentionszeit = 2.14 min

### Beispiel 369

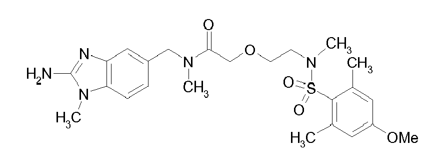

C₂₄H₃₃N₅O₅S x C₂HF₃O₂ (617.64)
[M+H]+ = 504
HPLC (Methode 9): Retentionszeit = 1.61 min

### Beispiel 370

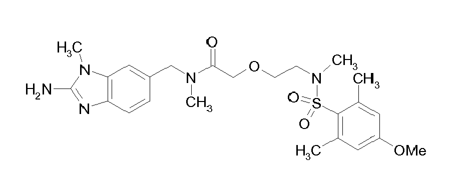

C₂₄H₃₃N₅O₅S x C₂HF₃O₂ (617.64)
[M+H]+ = 504
HPLC (Methode 9): Retentionszeit = 1.59 min

### Beispiel 371

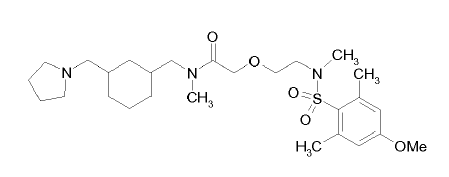

C₂₇H₄₅N₃O₅S x C₂HF₃O₂ (637.75)
[M+H]+ = 524
HPLC (Methode 9): Retentionszeit = 1.70 min

### Beispiel 372

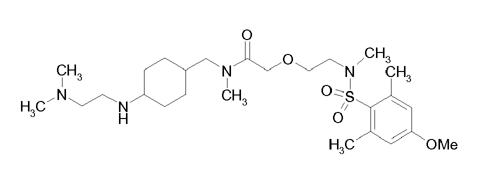

C₂₆H₄₆N₄O₅S (526.73)
[M+H]+ = 527
HPLC (Methode 5): Retentionszeit = 1.43 min

### Beispiel 373

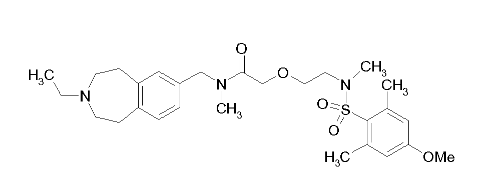

C₂₈H₄₁N₃O₅S x C₂HF₃O₂ (645.73)
[M+H]+ = 532
HPLC (Methode 5): Retentionszeit = 1.56 min

### Beispiel 374

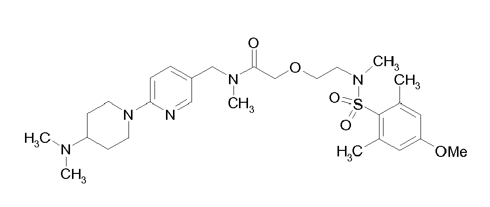

C₂₈H₄₃N₅O₅S x C₂HF₃O₂ (675.76)
[M+H]+ = 562
HPLC (Methode 5): Retentionszeit = 1.41 min

### Beispiel 375

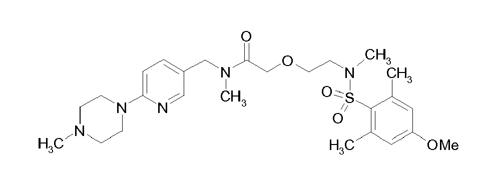

C₂₆H₃₉N₅O₅S x C₂HF₃O₂ (647.71)
[M+H]+ = 534
HPLC (Methode 5): Retentionszeit = 1.42 min

### Beispiel 376

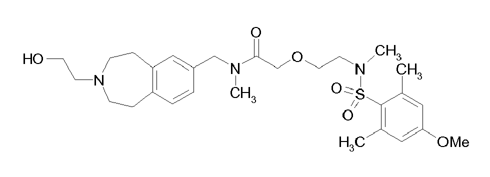

C₂₈H₄₁N₃O₆S (547.71)
[M+H]+ = 548
HPLC (Methode 5): Retentionszeit = 1.53 min

### Beispiel 377

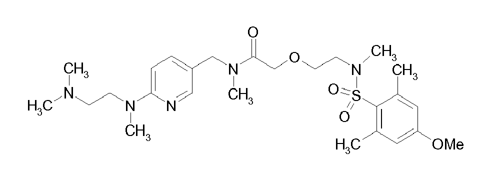

C₂₆H₄₁N₅O₅S (535.70)
[M+H]+ = 536
HPLC (Methode 5): Retentionszeit = 1.42 min

### Beispiel 378

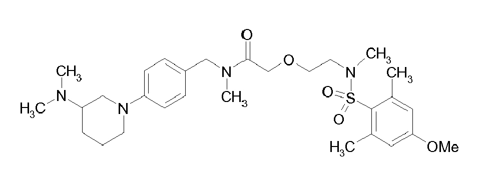

C₂₉H₄₄N₄O₅S x HCl (597.21)
[M+H]+ = 561
HPLC (Methode 7): Retentionszeit = 1.91 min

### Beispiel 379

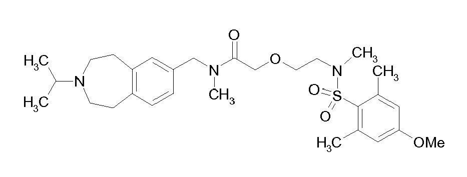

C₂₉H₄₃N₃O₅S x C₂HF₃O₂ (659.76)
[M+H]+ = 546
HPLC (Methode 5): Retentionszeit = 1.59 min

### Beispiel 380

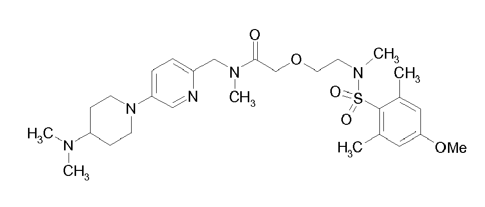

C₂₈H₄₃N₅O₅S x 2C₂HF₃O₂ (789.78)
[M+H]+ = 562
HPLC (Methode 5): Retentionszeit = 1.40 min

### Beispiel 381

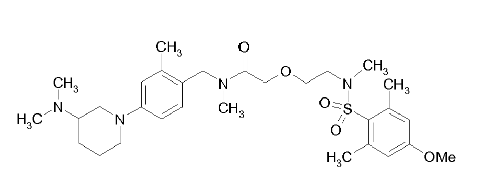

C₃₀H₄₆N₄O₅S x C₂HF₃O₂ (688.80)
[M+H]+ = 575
HPLC (Methode 5): Retentionszeit = 1.56 min

### Beispiel 382

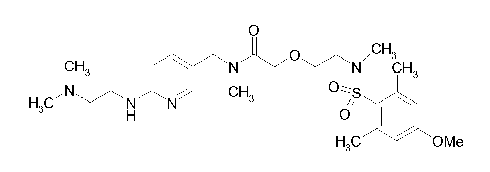

C₂₅H₃₉N₅O₅S x 2HCl (594.60)
[M+H]+ = 522
HPLC (Methode 5): Retentionszeit = 1.40 min

### Beispiel 383

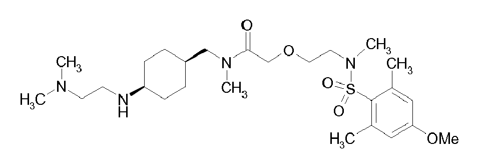

C₂₆H₄₆N₄O₅S x 2HCl (599.65)
[M+H]+ = 527
HPLC (Methode 5): Retentionszeit = 1.41 min

### Beispiel 384

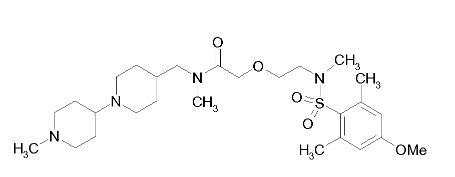

C₂₇H₄₆N₄O₅S x HCl (575.20)
[M+H]+ = 539
DC: Kieselgel, Dichlormethan / Methanol / Ammoniak 8:2:0.01, Rf-Wert = 0.35

### Beispiel 385

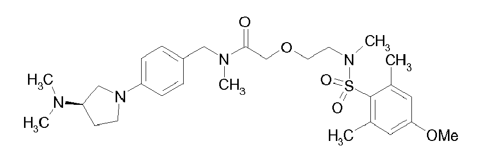

C₂₈H₄₂N₄O₅S x HCl (583.18)
[M+H]+ = 547
DC: Kieselgel, Dichlormethan / Methanol / Ammoniak 9:1:0.1, Rf-Wert = 0.32

### Beispiel 386

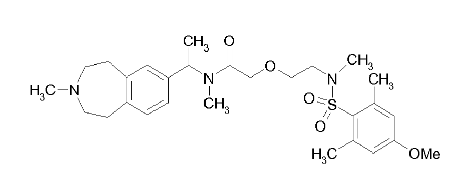

C₂₈H₄₁N₃O₅S x HCl (568.17)
[M+H]+ = 532
DC: Kieselgel, Dichlormethan / Methanol / Ammoniak 9:1:0.1, Rf-Wert = 0.35

### Beispiel 387

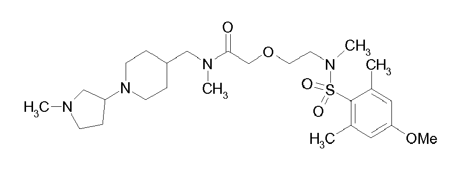

C₂₆H₄₄N₄O₅S x HCl (561.18)
[M+H]+ = 525
DC: Kieselgel, Dichlormethan / Methanol / Ammoniak 8:2:0.01, Rf-Wert = 0.12

### Beispiel 388

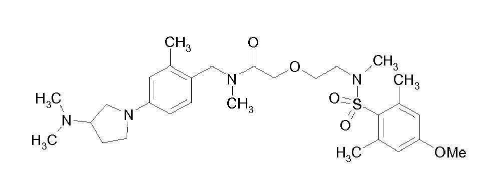

C₂₉H₄₄N₄O₅S x HCl (597.21)
[M+H]+ = 561
DC: Kieselgel, Dichlormethan / Methanol 9:1, Rf-Wert = 0.30

### Beispiel 389

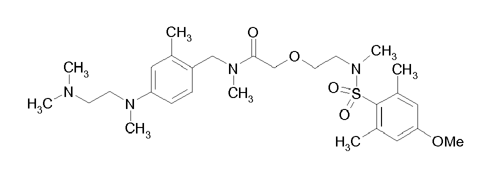

C₂₈H₄₄N₄O₅S x HCl (585.20)
[M+H]+ = 549
DC: Kieselgel, Dichlormethan / Methanol 9:1, Rf-Wert = 0.15

### Beispiel 390

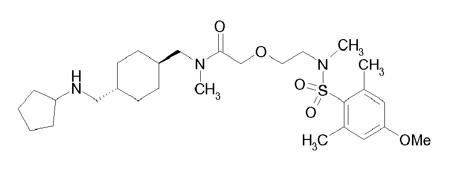

C₂₈H₄₇N₃O₅S x HCl (574.22)
[M+H]+ = 538
DC: Kieselgel, Dichlormethan / Methanol 9:1, Rf-Wert = 0.05

### Beispiel 391

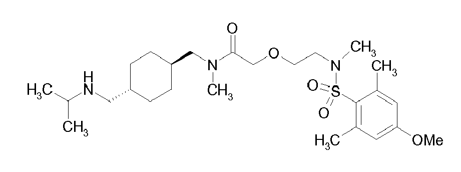

C₂₆H₄₅N₃O₅S x HCl (548.18)
[M+H]+ = 512
HPLC (Methode 5): Retentionszeit = 1.56 min

### Beispiel 392

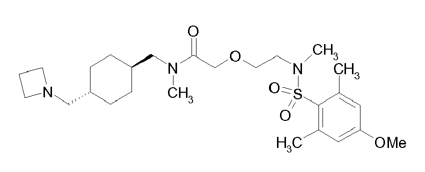

C₂₆H₄₃N₃O₅S x HCl (546.16)
[M+H]+ = 510
DC: Kieselgel, Dichlormethan / Methanol 9:1, Rf-Wert = 0.05

### Beispiel 393

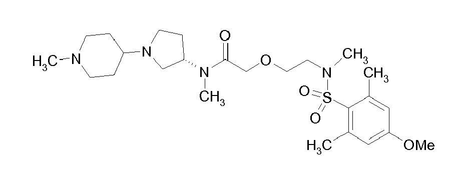

C₂₅H₄₂N₄O₅S x 2HCl (583.61)
[M+H]+ = 511
DC: Kieselgel, Dichlormethan / Ethanol / Ammoniak 9:1:0.1, Rf-Wert = 0.22

### Beispiel 394

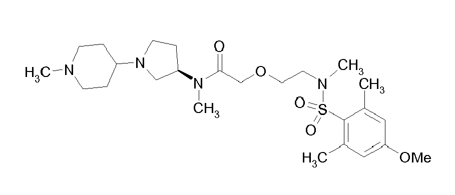

C₂₅H₄₂N₄O₅S x 2HCl (583.61)
[M+H]+ = 511
HPLC (Methode 5): Retentionszeit = 1.37 min

### Beispiel 395

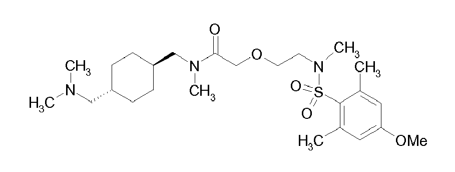

C₂₅H₄₃N₃O₅S x HCl (534.15)
[M+H]+ = 498
DC: Kieselgel, Dichlormethan / Methanol / Ammoniak 8:2:0.01, Rf-Wert = 0.58

### Beispiel 396

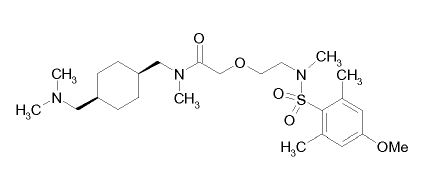

C₂₅H₄₃N₃O₅S x HCl (534.15)
[M+H]+ = 498
DC: Kieselgel, Dichlormethan / Methanol / Ammoniak 8:2:0.01, Rf-Wert = 0.57

### Beispiel 397

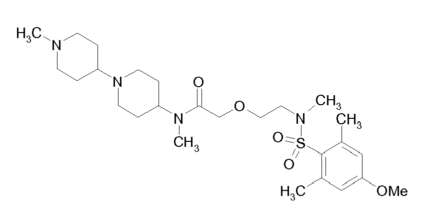

C₂₆H₄₄N₄O₅S x 2HCl (597.64)
[M+H]+ = 525
DC: Kieselgel, Dichlormethan / Methanol / Ammoniak 8:2:0.2, Rf-Wert = 0.52

### Beispiel 398

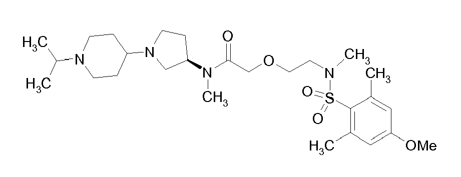

C₂₇H₄₆N₄O₅S x 2HCl (611.67)
[M+H]+ = 539
HPLC (Methode 12): Retentionszeit = 2.45 min

### Beispiel 399

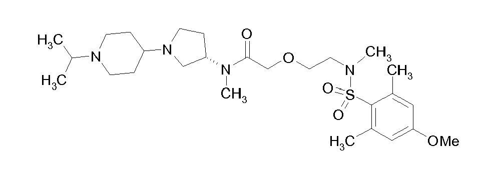

C₂₇H₄₆N₄O₅S x 2HCl (611.67)
[M+H]+ = 539
HPLC (Methode 12): Retentionszeit = 2.35 min

### Beispiel 400

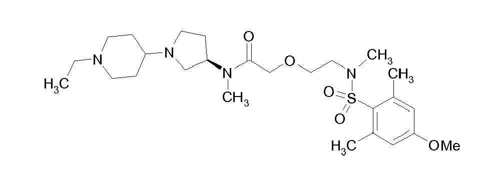

C₂₆H₄₄N₄O₅S x 2HCl (597.64)
[M+H]+ = 525
HPLC (Methode 12): Retentionszeit = 2.3 min

### Beispiel 401

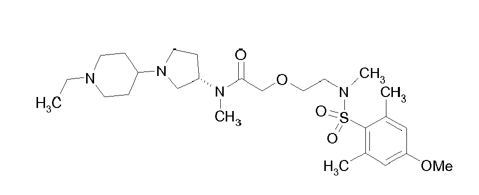

C₂₆H₄₄N₄O₅S x 2HCl (597.64)
[M+H]+ = 525
HPLC (Methode 12): Retentionszeit = 2.3 min

### Beispiel 402

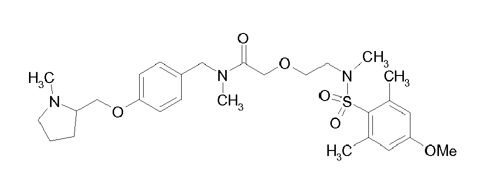

C₂₈H₄₁N₃O₆S (547.71)
[M+H]+ = 548
HPLC (Methode 9): Retentionszeit = 1.7 min

### Beispiel 403

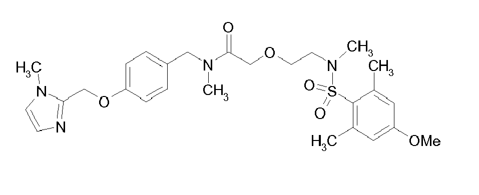

C₂₇H₃₆N₄O₆S (544.66)
[M+H]+ = 545
HPLC (Methode 9): Retentionszeit = 1.69 min

### Beispiel 404

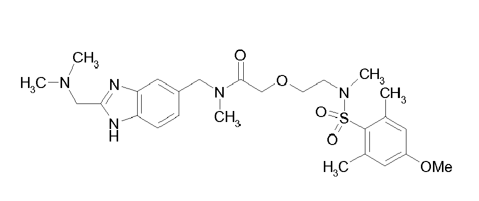

C₂₆H₃₇N₅O₅S (531.67)
[M+H]+ = 532
HPLC (Methode 6): Retentionszeit = 1.56 min

### Beispiel 405

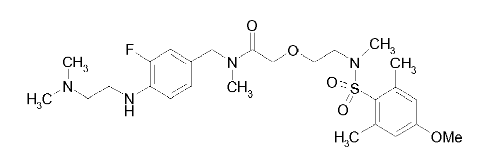

C₂₆H₃gFN₄O₅S (538.68)
[M+H]+ = 539
HPLC (Methode 6): Retentionszeit = 2.60 min

### Beispiel 406

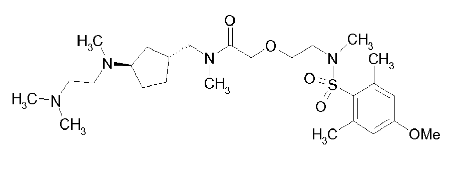

C₂₆H₄₆N₄O₅S x 2HCl (599.65)
[M+H]+ = 527
HPLC (Methode 7): Retentionszeit = 1.78 min

### Beispiel 407

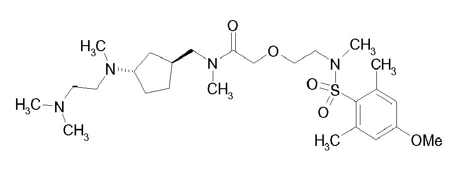

C₂₆H₄₆N₄O₅S x 2HCl (599.65)
[M+H]+ = 527
HPLC (Methode 7): Retentionszeit = 1.77 min

### Beispiel 408

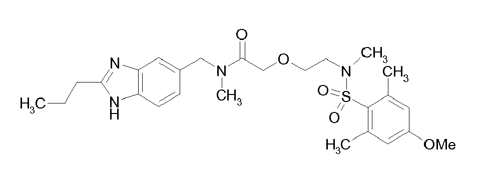

C₂₆H₃₆N₄O₅S (516.65)
[M+H]+ = 517
HPLC (Methode 9): Retentionszeit = 1.65 min

### Beispiel 409

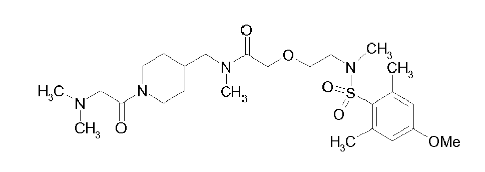

C₂₅H₄₂N₄O₆S x C₂HF₃O₂ (640.71)
[M+H]+ = 527
HPLC (Methode 9): Retentionszeit = 1.55 min

### Beispiel 410

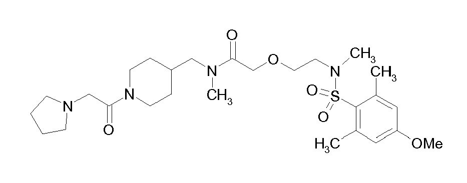

C₂₇H₄₄N₄O₆S x C₂HF₃O₂ (666.75)
[M+H]+ = 553
HPLC (Methode 9): Retentionszeit = 1.57 min

### Beispiel 411

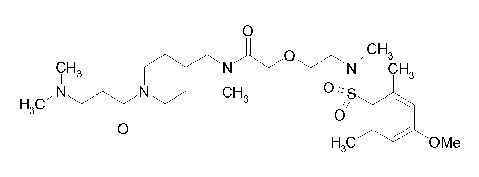

C₂₆H₄₄N₄O₆S x C₂HF₃O₂ (654.74)
[M+H]+ = 541
HPLC (Methode 9): Retentionszeit = 1.57 min

### Beispiel 412

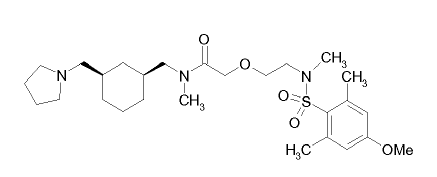

C₂₇H₄₅N₃O₅S x C₂HF₃O₂ (637.75)
[M+H]+ = 524
HPLC (Methode 6): Retentionszeit = 1.71 min

### Beispiel 413

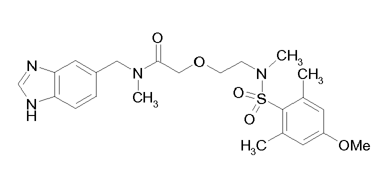

C₂₃H₃₀N₄O₅S (474.57)
[M+H]+ = 475
HPLC (Methode 9): Retentionszeit = 1.53 min

### Beispiel 414

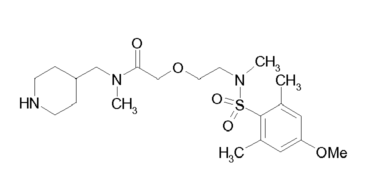

C₂₁H₃₅N₃O₅S (441.59)
[M+H]+ = 442
HPLC (Methode 9): Retentionszeit = 1.48 min

### Beispiel 415

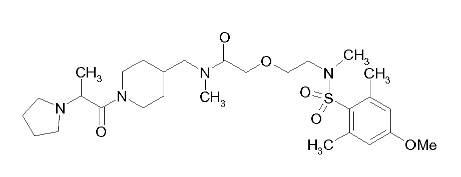

C₂₈H₄₆N₄O₆S x C₂HF₃O₂ (680.78)
[M+H]+ = 567
HPLC (Methode 9): Retentionszeit = 1.60 min

### Beispiel 416

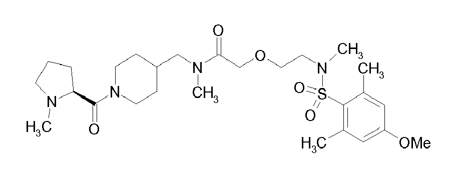

C₂₇H₄₄N₄O₆S x C₂HF₃O₂ (666.75)
[M+H]+ = 553
HPLC (Methode 9): Retentionszeit = 1.57 min

### Beispiel 417

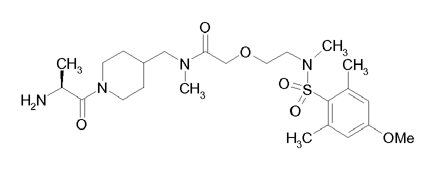

C₂₄H₄₀N₄O₆S x C₂HF₃O₂ (626.69)
[M+H]+ = 513
HPLC (Methode 9): Retentionszeit = 1.53 min

### Beispiel 418

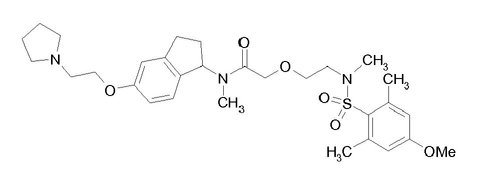

C₃₀H₄₃N₃O₆S x CH₂O₂ (619.77)
[M+H]+ = 574
HPLC (Methode 9): Retentionszeit = 1.73 min

### Beispiel 419

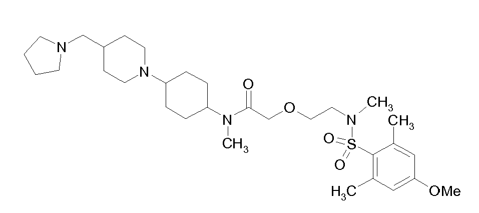

C₃₁H₅₂N₄O₅S x C₂HF₃O₂ (706.86)
[M+H]+ = 593
HPLC (Methode 9): Retentionszeit = 1.34 min

### Beispiel 420

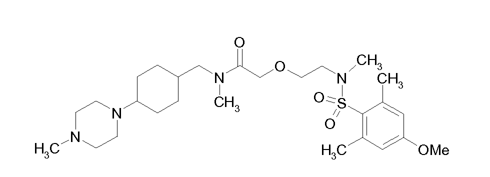

C₂₇H₄₆N₄O₅S (538.74)
[M+H]+ = 539
HPLC (Methode 9): Retentionszeit = 1.43 min

### Beispiel 421

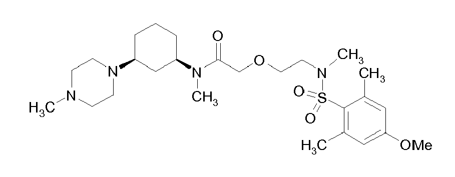

C₂₆H₄₄N₄O₅S x C₂HF₃O₂ (638.74)
[M+H]+ = 525
HPLC (Methode 6): Retentionszeit = 1.53 min

### Beispiel 422

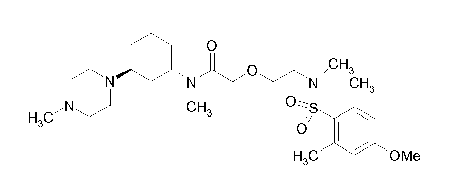

C₂₆H₄₄N₄O₅S x C₂HF₃O₂ (638.74)
[M+H]+ = 525
HPLC (Methode 6): Retentionszeit = 1.41 min

### Beispiel 423

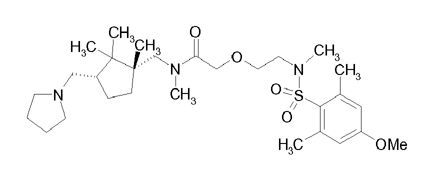

C₂₉H₄₉N₃O₅S x C₂HF₃O₂ (665.81)
[M+H]+ = 552
HPLC (Methode 9): Retentionszeit = 1.74 min

### Beispiel 424

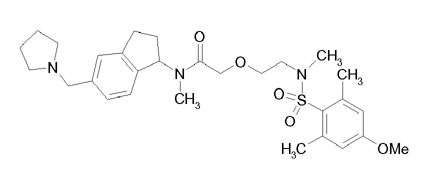

C₂₉H₄₁N₃O₅S (543.72)
[M+H]+ = 544
HPLC (Methode 6): Retentionszeit = 1.73 min

### Beispiel 425

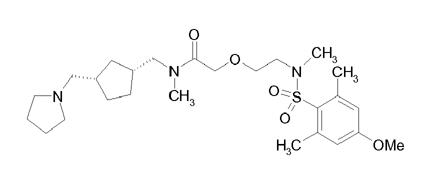

C₂₆H₄₃N₃O₅S x C₂HF₃O₂ (623.73)
[M+H]+ = 510
HPLC (Methode 9): Retentionszeit = 1.63 min

### Beispiel 426

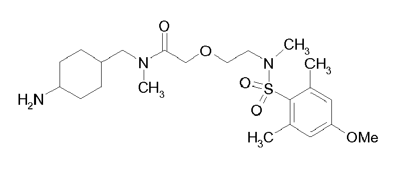

C₂₂H₃₇N₃O₅S (455.61)
[M+H]+ = 456
HPLC (Methode 6): Retentionszeit = 1.55 min

### Beispiel 427

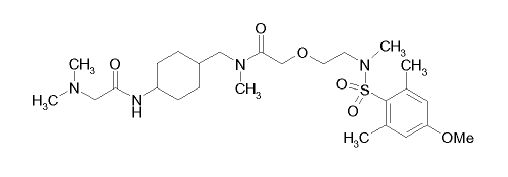

C₂₆H₄₄N₄O₆S (540.72)
[M+H]+ = 541
HPLC (Methode 6): Retentionszeit = 1.59 min

### Beispiel 428

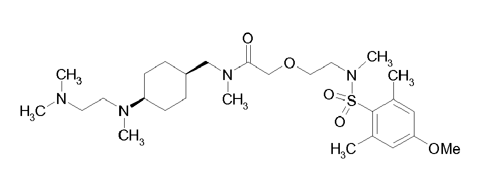

C₂₇H₄₈N₄O₅S x 2HCl (613.68)
[M+H]+ = 541
HPLC (Methode 7): Retentionszeit = 1.65 min

### Beispiel 429

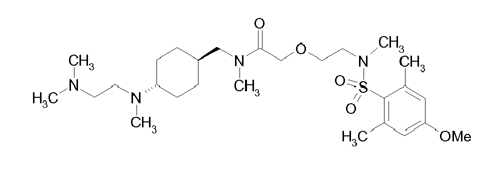

C₂₇H₄₈N₄O₅S x 2HCl (613.68)
[M+H]+ = 541
HPLC (Methode 11): Retentionszeit = 1.64 min

### Beispiel 430

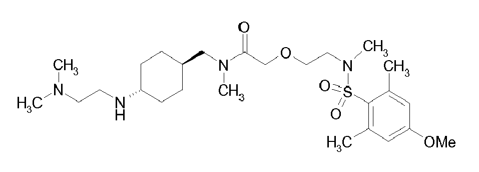

C₂₆H₄₆N₄O₅S x 2C₂HF₃O₂ (754.78)
[M+H]+ = 527
HPLC (Methode 5): Retentionszeit = 1.16 min

### Beispiel 431

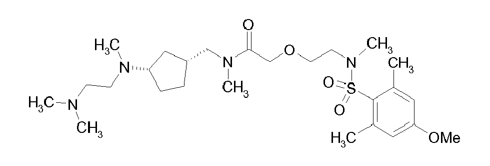

C₂₆H₄₆N₄O₅S x 2HCl (599.65)
[M+H]+ = 527
HPLC (Methode 7): Retentionszeit = 1.82 min

### Beispiel 432

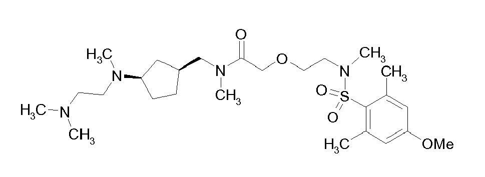

C₂₆H₄₆N₄O₅S x 2HCl (599.65)
[M+H]+ = 527
HPLC (Methode 7): Retentionszeit = 1.82 min

### Beispiel 433

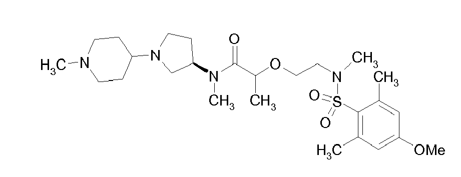

C₂₆H₄₄N₄O₅S x 2HCl (597.64)
[M+H]+ = 525
HPLC (Methode 12): Retentionszeit = 2.3 min

### Beispiel 434

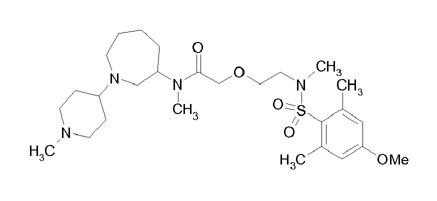

C₂₇H₄₆N₄O₅S x 2HCl (611.67)
[M+H]+ = 539
DC: Kieselgel, Dichlormethan / Methanol / Ammoniak 8:2:0.2, Rf-Wert = 0.65

### Beispiel 435

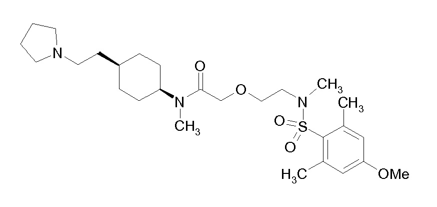

C₂₇H₄₅N₃O₅S (523.73)
[M+H]+ = 524
HPLC (Methode 6): Retentionszeit = 1.29 min

### Beispiel 436

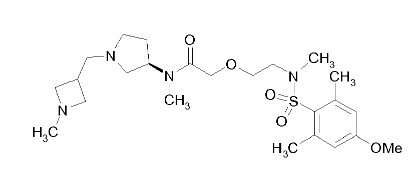

C₂₄H₄₀N₄O₅S x CH₂O₂ (542.69)
[M+H]+ = 497
HPLC (Methode 9): Retentionszeit = 1.25 min

### Beispiel 437

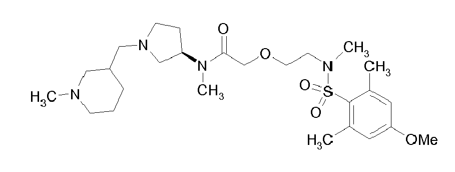

C₂₆H₄₄N₄O₅S x CH₂O₂ (570.74)
[M+H]+ = 525
HPLC (Methode 9): Retentionszeit = 1.31 min

### Beispiel 438

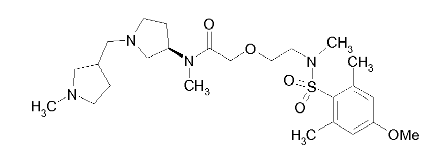

C₂₅H₄₂N₄O₅S x CH₂O₂ (556.72)
[M+H]+ = 511
HPLC (Methode 9): Retentionszeit = 1.31 min

### Beispiel 439

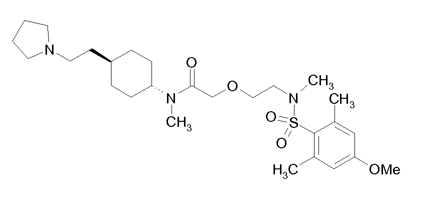

C₂₇H₄₅N₃O₅S (523.73)
[M+H]+ = 524
HPLC (Methode 6): Retentionszeit = 1.67 min

### Beispiel 440

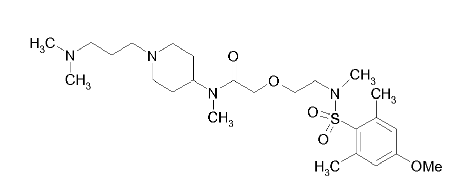

C₂₅H₄₄N₄O₅S x C₂HF₃O₂ (626.73)
[M+H]+ = 513
HPLC (Methode 9): Retentionszeit = 1.29 min

### Beispiel 441

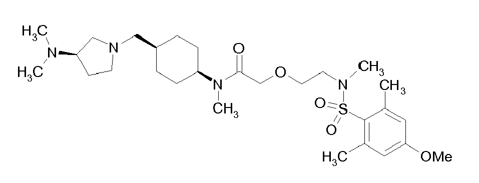

C₂₈H₄₈N₄O₅S x 2 HCl (625.69)
[M+H]+ = 553
HPLC (Methode 9): Retentionszeit = 1.35 min

### Beispiel 442

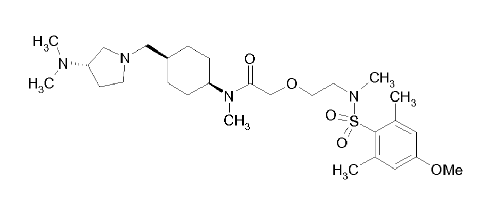

C₂₈H₄₈N₄O₅S x 2 HCl (625.69)
[M+H]+ = 553
HPLC (Methode 9): Retentionszeit = 1.32 min

### Beispiel 443

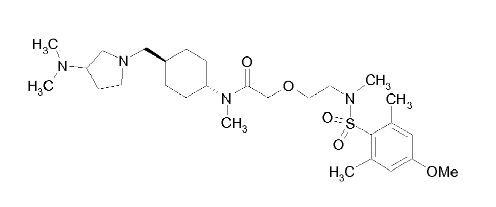

C₂₈H₄₈N₄O₅S (552.77)
[M+H]+ = 553
HPLC (Methode 9): Retentionszeit = 1.37 min

### Beispiel 444

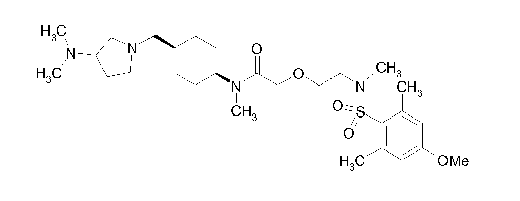

C₂₈H₄₈N₄O₅S x CH₂O₂ (598.80)
[M+H]+ = 553
HPLC (Methode 9): Retentionszeit = 1.35 min

### Beispiel 445

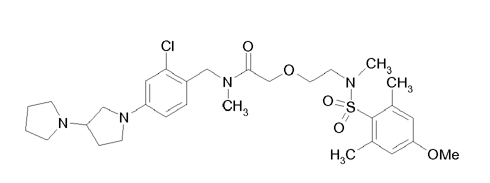

C₃₀H₄₃ClN₄O₅S (607.21)
[M+H]+ = 608
HPLC (Methode 6): Retentionszeit = 1.80 min

### Beispiel 446

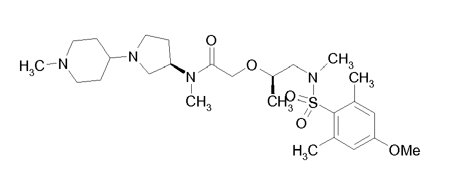

C₂₆H₄₄N₄O₅S x 2HCl (597.64)
[M+H]+ = 525
HPLC (Methode 12): Retentionszeit = 2.4 min

### Beispiel 447

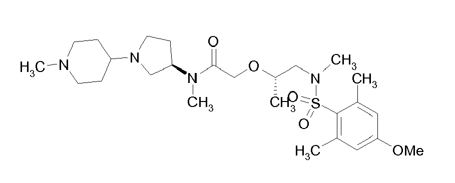

C₂₆H₄₄N₄O₅S x 2HCl (597.64)
[M+H]+ = 525
HPLC (Methode 12): Retentionszeit = 2.4 min

### Beispiel 448

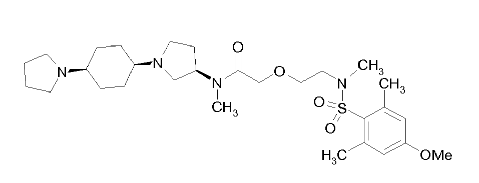

C₂₉H₄₈N₄O₅S x 2HCl (637.70)
[M+H]+ = 565
HPLC (Methode 12): Retentionszeit = 2.3 min

### Beispiel 449

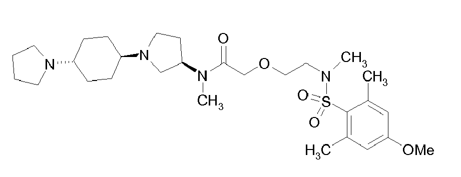

C₂₉H₄₈N₄O₅S x 2HCl (637.70)
[M+H]+ = 565
HPLC (Methode 12): Retentionszeit = 2.92 min

### Beispiel 450

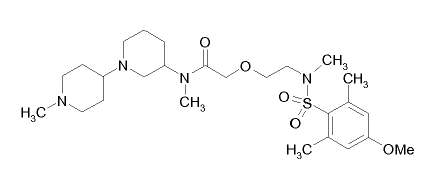

C₂₆H₄₄N₄O₅S x 2HCl (597.64)
[M+H]+ = 525
DC: Kieselgel, Dichlormethan / Methanol / Ammoniak 8:2:0.2, Rf-Wert = 0.60

### Beispiel 451

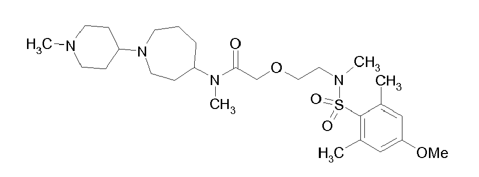

C₂₇H₄₆N₄O₅S x 2HCl (611.67)
[M+H]+ = 539
DC: Kieselgel, Dichlormethan / Methanol / Ammoniak 8:2:0.2, Rf-Wert = 0.62

### Beispiel 452

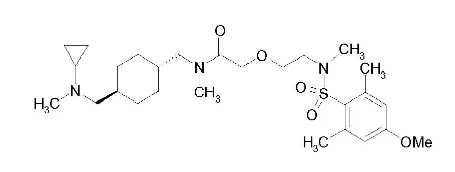

C₂₇H₄₅N₃O₅S x HCl (560.19)
[M+H]+ = 524
HPLC (Methode 12): Retentionszeit = 3.01 min

### Beispiel 453

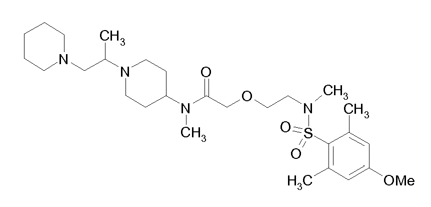

C₂₈H₄₈N₄O₅S x 2HCl (625.69)
[M+H]+ = 553
HPLC (Methode 12): Retentionszeit = 2.45 min

### Beispiel 454

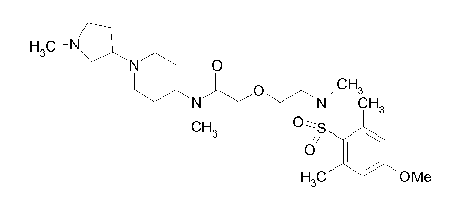

C₂₅H₄₂N₄O₅S x 2HCl (583.61)
[M+H]+ = 511
HPLC (Methode 12): Retentionszeit = 2.33 min

### Beispiel 455

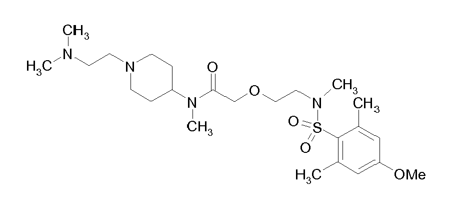

C₂₄H₄₂N₄O₅S x C₂HF₃O₂ (612.70)
[M+H]+ = 499
HPLC (Methode 9): Retentionszeit = 1.32 min

### Beispiel 456

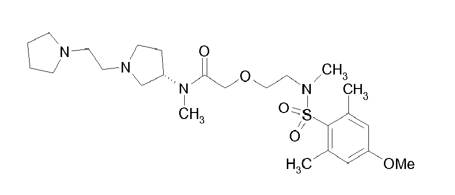

C₂₅H₄₂N₄O₅S x 2C₂HF₃O₂ (738.74)
[M+H]+ = 511
HPLC (Methode 9): Retentionszeit = 1.24 min

### Beispiel 457

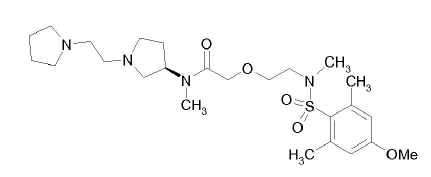

C₂₅H₄₂N₄O₅S x 2C₂HF₃O₂ (738.74)
[M+H]+ = 511
HPLC (Methode 9): Retentionszeit = 1.27 min

### Beispiel 458

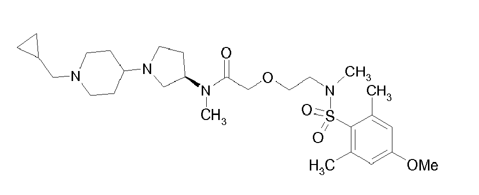

C₂₈H₄₆N₄O₅S x CH₂O₂ (596.78)
[M+H]+ = 551
HPLC (Methode 6): Retentionszeit = 1.31 min

### Beispiel 459

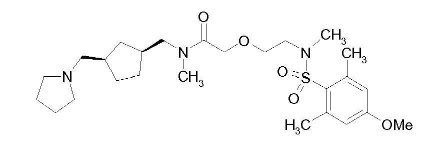

C₂₆H₄₃N₃O₅S x C₂HF₃O₂ (623.73)
[M+H]+ = 510
HPLC (Methode 9): Retentionszeit = 1.62 min

### Beispiel 460

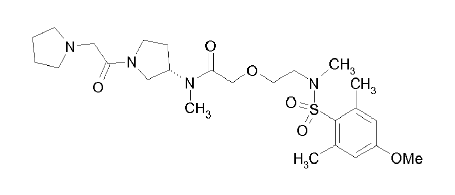

C₂₅H₄₀N₄O₆S x C₂HF₃O₂ (638.70)
[M+H]+ = 525
HPLC (Methode 9): Retentionszeit = 1.49 min

### Beispiel 461

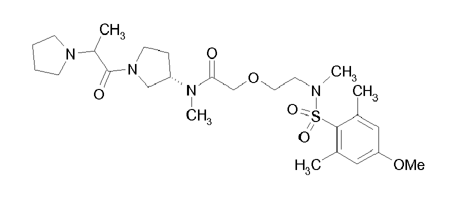

C₂₆H₄₂N₄O₆S x C₂HF₃O₂ (652.72)
[M+H]+ = 539
HPLC (Methode 9): Retentionszeit = 1.51 min

### Beispiel 462

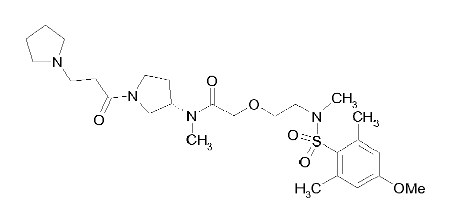

C₂₆H₄₂N₄O₆S x C₂HF₃O₂ (652.72)
[M+H]+ = 539
HPLC (Methode 9): Retentionszeit = 1.52 min

### Beispiel 463

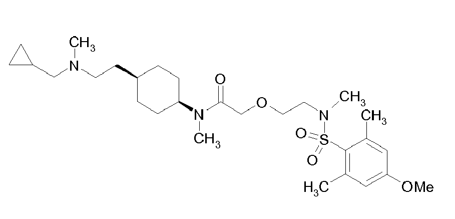

C₂₈H₄₇N₃O₅S (537.76)
[M+H]+ = 538
HPLC (Methode 9): Retentionszeit = 1.71 min

### Beispiel 464

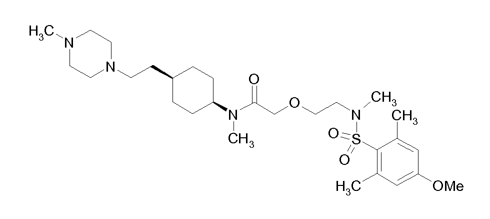

C₂₈H₄₈N₄O₅S (552.77)
[M+H]+ = 553
HPLC (Methode 9): Retentionszeit = 1.39 min

### Beispiel 465

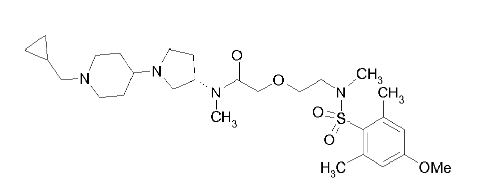

C₂₈H₄₆N₄O₅S x CH₂O₂ (596.78)
[M+H]+ = 551
HPLC (Methode 6): Retentionszeit = 1.32 min

### Beispiel 466

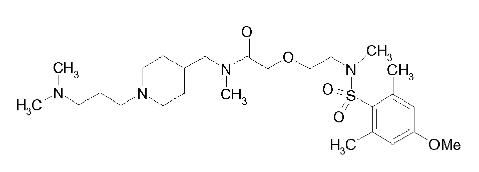

C₂₆H₄₆N₄O₅S (526.73)
[M+H]+ = 527
HPLC (Methode 9): Retentionszeit = 1.27 min

### Beispiel 467

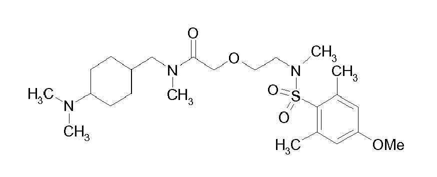

C₂₄H₄₁N₃O₅S (483.67)
[M+H]+ = 484
HPLC (Methode 6): Retentionszeit = 1.60 min

### Beispiel 468

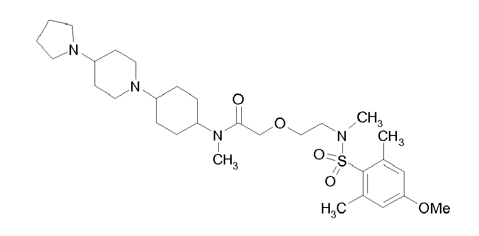

C₃₀H₅₀N₄O₅S x CH₂O₂ (624.83)
[M+H]+ = 579
HPLC (Methode 6): Retentionszeit = 1.31 min

### Beispiel 469

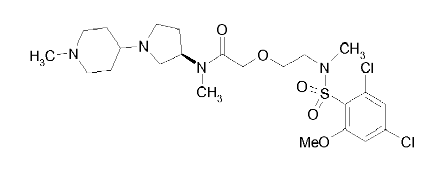

C₂₃H₃₆Cl₂N₄O₅S (551.53)
[M+H]+ = 551/553/555
HPLC (Methode 9): Retentionszeit = 1.30 min

### Beispiel 470

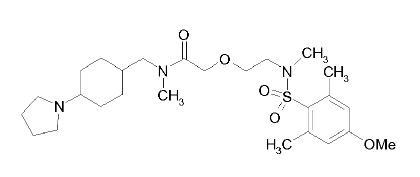

C₂₆H₄₃N₃O₅S (509.70)
[M+H]+ = 510
HPLC (Methode 6): Retentionszeit = 1.67 min

### Beispiel 471

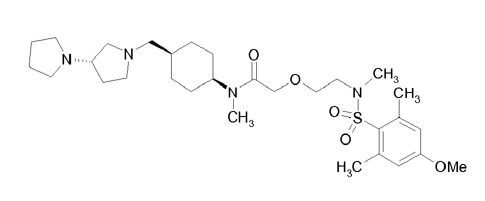

C₃₀H₅₀N₄O₅S x C₂HF₃O₂ (692.83)
[M+H]+ = 579
HPLC (Methode 9): Retentionszeit = 1.39 min

### Beispiel 472

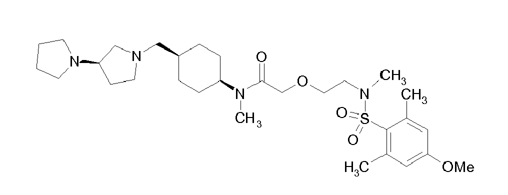

C₃₀H₅₀N₄O₅S x C₂HF₃O₂ (692.83)
[M+H]+ = 579
HPLC (Methode 9): Retentionszeit = 1.39 min

### Beispiel 473

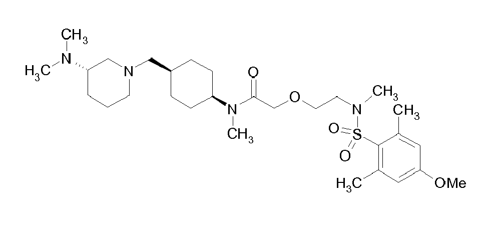

C₂₉H₅₀N₄O₅S x C₂HF₃O₂ (680.82)
[M+H]+ = 567
HPLC (Methode 9): Retentionszeit = 1.40 min

### Beispiel 474

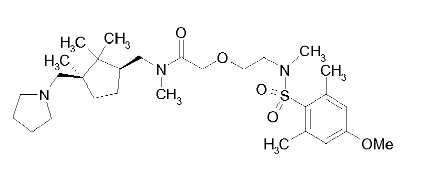

C₂₉H₄₉N₃O₅S (551.78)
[M+H]+ = 552
HPLC (Methode 9): Retentionszeit = 1.72 min

### Beispiel 475

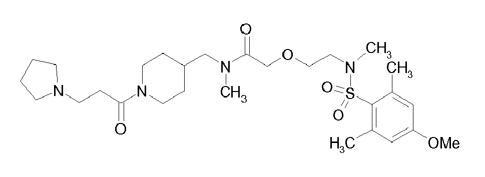

C₂₈H₄₆N₄O₆S x C₂HF₃O₂ (680.78)
[M+H]+ = 567
HPLC (Methode 9): Retentionszeit = 1.60 min

### Beispiel 476

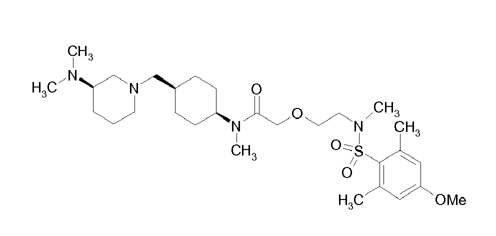

C₂₉H₅₀N₄O₅S x C₂HF₃O₂ (680.82)
[M+H]+ = 567
HPLC (Methode 9): Retentionszeit = 1.41 min

### Beispiel 477

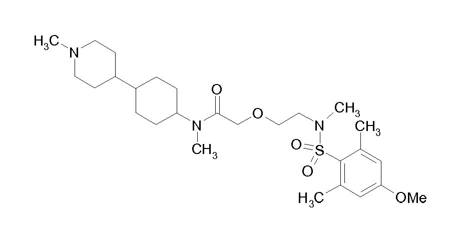

C₂₇H₄₅N₃O₅S x C₂HF₃O₂ (637.75)
[M+H]+ = 524
HPLC (Methode 9): Retentionszeit = 1.69 min

### Beispiel 478

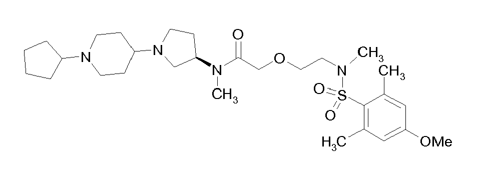

C₂₉H₄₈N₄O₅S x 2HCl (637.70)
[M+H]+ = 565
HPLC (Methode 12): Retentionszeit = 2.4 min

### Beispiel 479

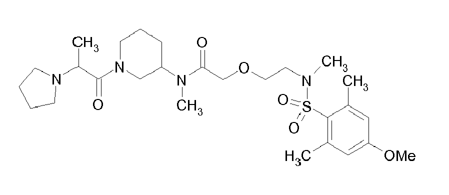

C₂₇H₄₄N₄O₆S x C₂HF₃O₂ (666.75)
[M+H]+ = 553
HPLC (Methode 9): Retentionszeit = 1.61 min

### Beispiel 480

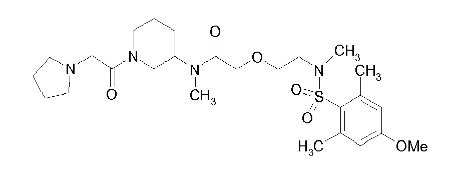

C₂₆H₄₂N₄O₆S x C₂HF₃O₂ (652.72)
[M+H]+ = 539
HPLC (Methode 9): Retentionszeit = 1.60 min

### Beispiel 481

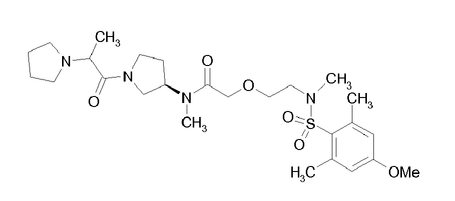

C₂₆H₄₂N₄O₆S (538.70)
[M+H]+ = 539
HPLC (Methode 9): Retentionszeit = 1.47 min

### Beispiel 482

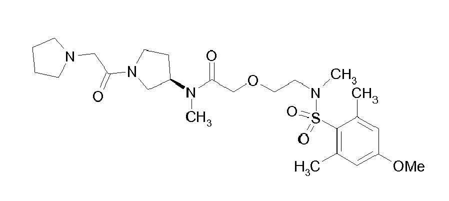

C₂₅H₄₀N₄O₆S (524.67)
[M+H]+ = 525
HPLC (Methode 9): Retentionszeit = 1.48 min

### Beispiel 575

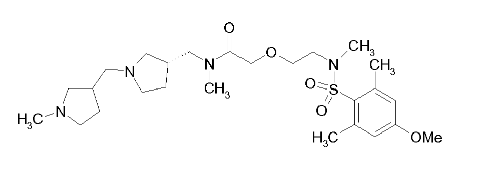

C₂₆H₄₄N₄O₅S (524.72)
[M+H]+ = 525
HPLC (Methode 9): Retentionszeit = 1.30 min

### Beispiel 576

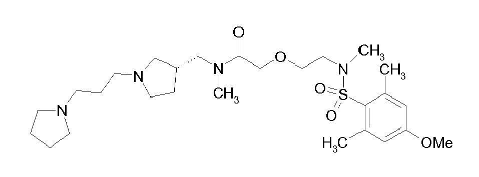

C₂₇H₄₆N₄O₅S (538.74)
[M+H]+ = 539
HPLC (Methode 9): Retentionszeit = 1.34 min

### Beispiel 577

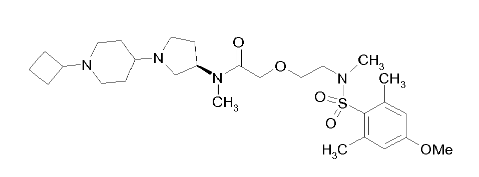

C₂₈H₄₆N₄O₅S x 2HCl (623.68)
[M+H]+ = 551
DC: Kieselgel, Dichlormethan / Methanol / Ammoniak 8:2:0.2, Rf-Wert = 0.71

### Beispiel 578

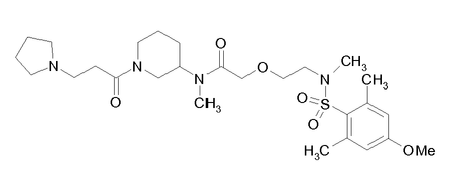

C₂₇H₄₄N₄O₆S x C₂HF₃O₂ (666.75)
[M+H]+ = 553
HPLC (Methode 9): Retentionszeit = 1.62 min

### Beispiel 579

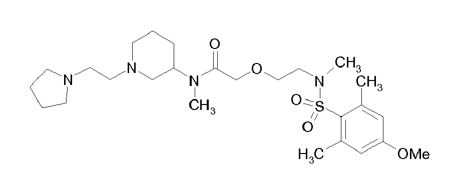

C₂₆H₄₄N₄O₅S x 2C₂HF₃O₂ (752.76)
[M+H]+ = 525
HPLC (Methode 9): Retentionszeit = 1.33 min

### Beispiel 580

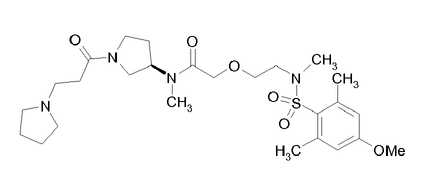

C₂₆H₄₂N₄O₆S x C₂HF₃O₂ (652.72)
[M+H]+ = 539
HPLC (Methode 9): Retentionszeit = 1.50 min

### Beispiel 581

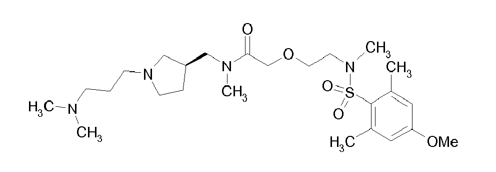

C₂₅H₄₄N₄O₅S (512.71)
[M+H]+ = 513
HPLC (Methode 9): Retentionszeit = 1.27 min

### Beispiel 582

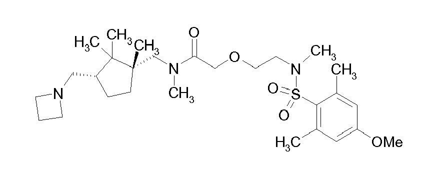

C₂₈H₄₇N₃O₅S x C₂HF₃O₂ (651.78)
[M+H]+ = 538
HPLC (Methode 9): Retentionszeit = 1.71 min

### Beispiel 583

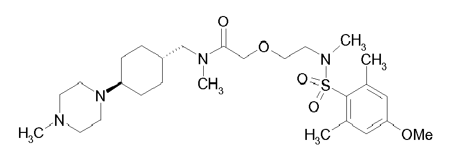

C₂₇H₄₆N₄O₅S (538.74)
[M+H]+ = 539
HPLC (Methode 9): Retentionszeit = 1.37 min

### Beispiel 584

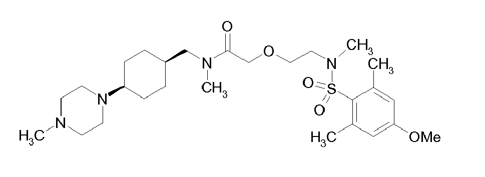

C₂₇H₄₆N₄O₅S (538.74)
[M+H]+ = 539
HPLC (Methode 9): Retentionszeit = 1.44 min

### Beispiel 585

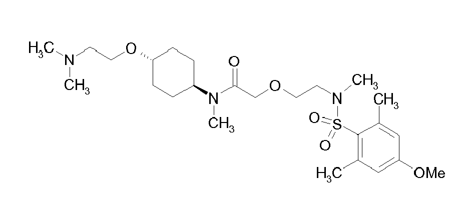

C₂₅H₄₃N₃O₆S x HCl (550.15)
[M+H]+ = 514
HPLC (Methode 5): Retentionszeit = 1.34 min

### Beispiel 586

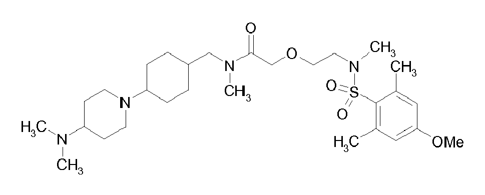

C₂₉H₅₀N₄O₅S x 2HCl (639.72)
[M+H]+ = 567
HPLC (Methode 5): Retentionszeit = 1.21 min

### Beispiel 587

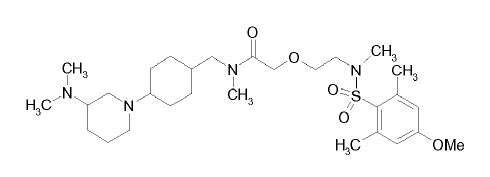

C₂₉H₅₀N₄O₅S x 2HCl (639.72)
[M+H]+ = 567
HPLC (Methode 5): Retentionszeit = 1.20 min

### Beispiel 588

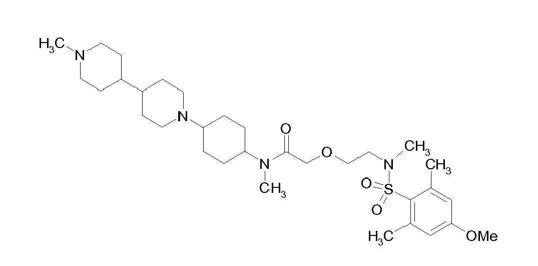

C₃₂H₅₄N₄O₅S x C₂HF₃O₂ (720.88)
[M+H]+ = 607
HPLC (Methode 9): Retentionszeit = 1.37 min

### Beispiel 589

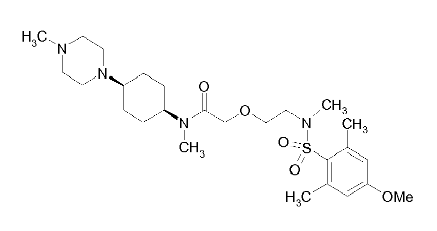

C₂₆H₄₄N₄O₅S x 2C₂HF₃O₂ (752.76)
[M+H]+ = 525
HPLC (Methode 9): Retentionszeit = 1.41 min

### Beispiel 590

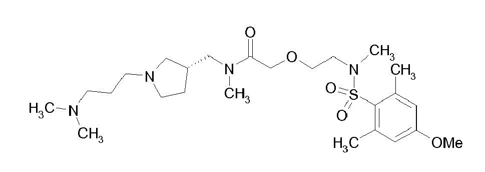

C₂₅H₄₄N₄O₅S (512.71)
[M+H]+ = 513
HPLC (Methode 9): Retentionszeit = 1.35 min

### Beispiel 591

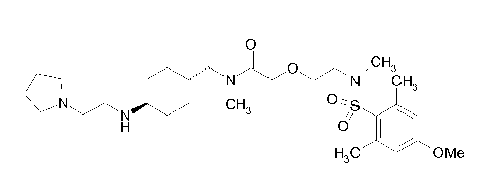

C₂₈H₄₈N₄O₅S x 2C₂HF₃O₂ (780.82)
[M+H]+ = 553
HPLC (Methode 9): Retentionszeit = 1.40 min

### Beispiel 592

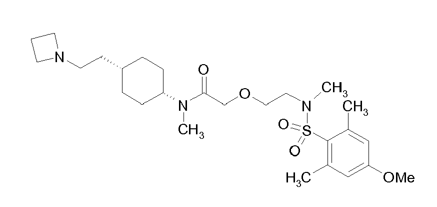

C₂₆H₄₃N₃O₅S (509.70)
[M+H]+ = 510
HPLC (Methode 9): Retentionszeit = 1.70 min

### Beispiel 612

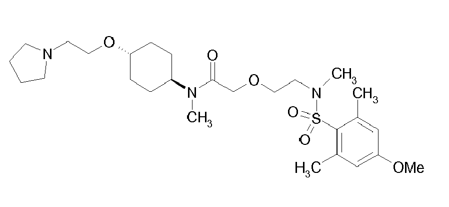

C₂₇H₄₅N₃O₆S x HCl (576.19)
[M+H]+ = 540
HPLC (Methode 5): Retentionszeit = 1.37 min

### Beispiel 613

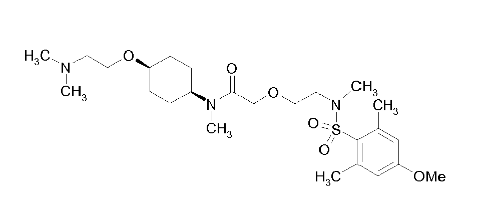

C₂₅H₄₃N₃O₆S x HCl (550.15)
[M+H]+ = 514
HPLC (Methode 5): Retentionszeit = 1.36 min

### Beispiel 614

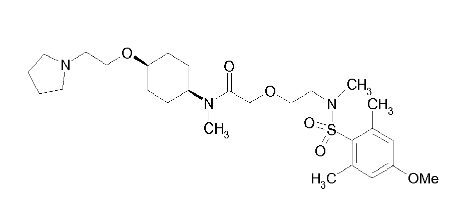

C₂₇H₄₅N₃O₆S x HCl (576.19)
[M+H]+ = 540
HPLC (Methode 5): Retentionszeit = 1.38 min

### Beispiel 615

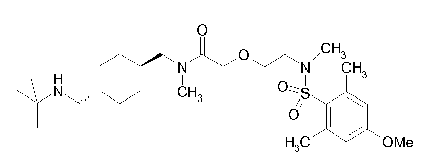

C₂₇H₄₇N₃O₅S x HCl (562.21)
[M+H]+ = 526
HPLC (Methode 4): Retentionszeit = 3.0 min

### Beispiel 616

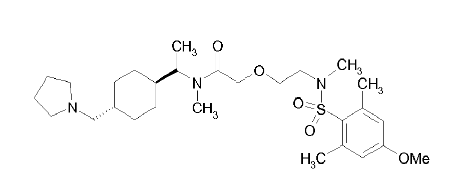

C₂₈H₄₇N₃O₅S x HCl (574.22)
[M+H]+ = 538
HPLC (Methode 4): Retentionszeit = 3.1 min

### Beispiel 617

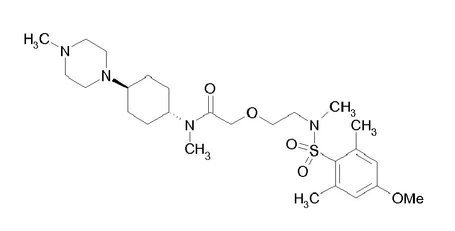

C₂₆H₄₄N₄O₅S (524.72)
[M+H]+ = 525
HPLC (Methode 9): Retentionszeit = 1.32 min

### Beispiel 618

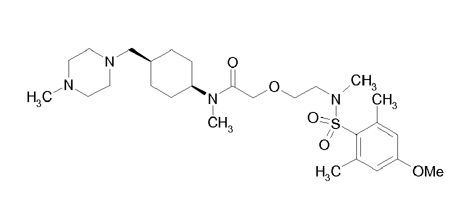

C₂₇H₄₆N₄O₅S (538.74)
[M+H]+ = 539
HPLC (Methode 9): Retentionszeit = 1.57 min

### Beispiel 619

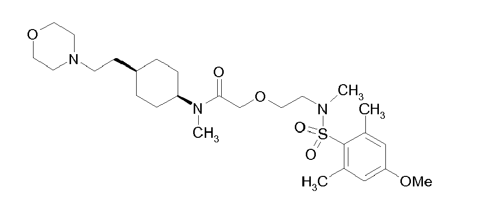

C₂₇H₄₅N₃O₆S (539.73)
[M+H]+ = 540
HPLC (Methode 9): Retentionszeit = 1.65 min

### Beispiel 620

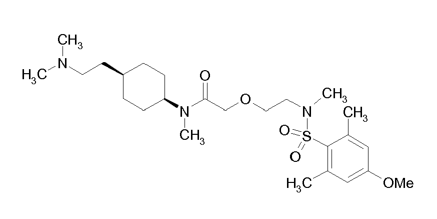

C₂₅H₄₃N₃O₅S (497.69)
[M+H]+ = 498
HPLC (Methode 9): Retentionszeit = 1.65 min

### Beispiel 621

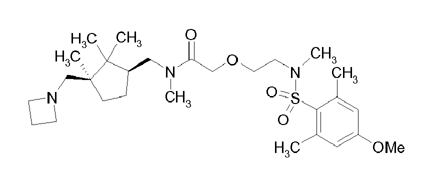

C₂₈H₄₇N₃O₅S x HCl (574.22)
[M+H]+ = 538
HPLC (Methode 9): Retentionszeit = 1.68 min

### Beispiel 622

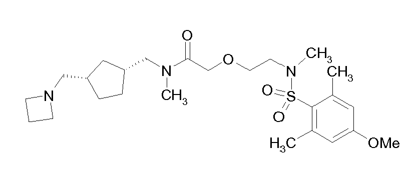

C₂₅H₄₁N₃O₅S x C₂HF₃O₂ (609.70)
[M+H]+ = 496
HPLC (Methode 9): Retentionszeit = 1.62 min

### Beispiel 623

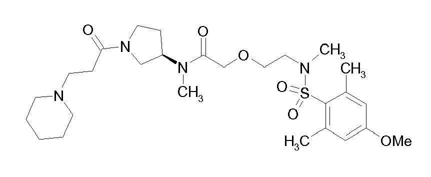

C₂₇H₄₄N₄O₆S x C₂HF₃O₂ (666.76)
[M+H]+ = 553
HPLC (Methode 9): Retentionszeit = 1.57 min

### Beispiel 624

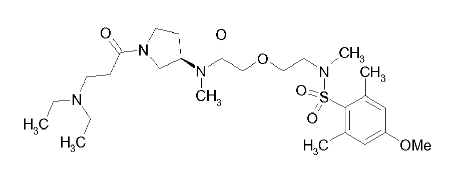

C₂₆H₄₄N₄O₆S x C₂HF₃O₂ (654.75)
[M+H]+ = 541
HPLC (Methode 9): Retentionszeit = 1.56 min

### Beispiel 625

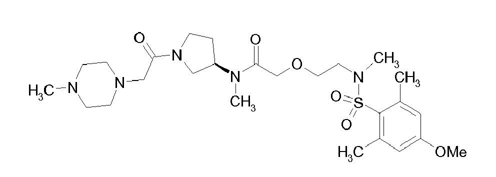

C₂₆H₄₃N₅O₆S x C₂HF₃O₂ (667.74)
[M+H]+ = 554
HPLC (Methode 9): Retentionszeit = 1.00 min

### Beispiel 626

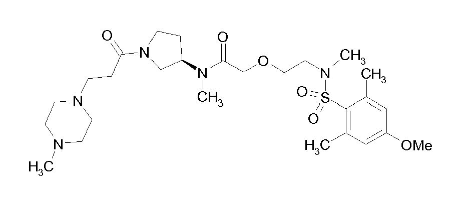

C₂₇H₄₅N₅O₆S x C₂HF₃O₂ (681.77)
[M+H]+ = 568
HPLC (Methode 9): Retentionszeit = 1.03 min

### Beispiel 627

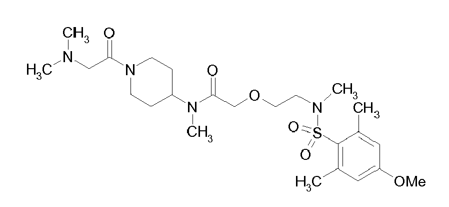

C₂₄H₄₀N₄O₆S x C₂HF₃O₂ (626.69)
[M+H]+ = 513
HPLC (Methode 9): Retentionszeit = 1.50 min

### Beispiel 628

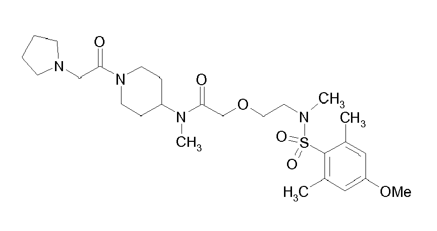

C₂₆H₄₂N₄O₆S x C₂HF₃O₂ (654.72)
[M+H]+ = 539
HPLC (Methode 9): Retentionszeit = 1.55 min

### Beispiel 629

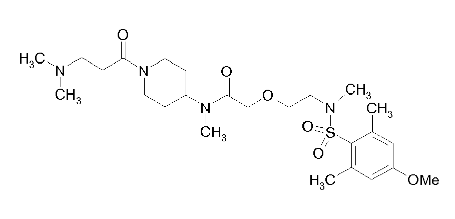

C₂₅H₄₂N₄O₆S x C₂HF₃O₂ (640.71)
[M+H]+ = 527
HPLC (Methode 9): Retentionszeit = 1.63 min

### Beispiel 630

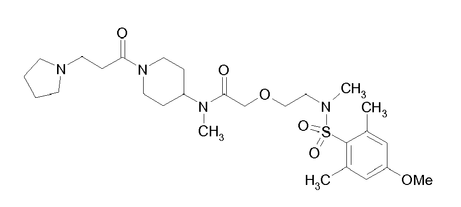

C₂₇H₄₄N₄O₆S (552.73)
[M+H]+ = 553
HPLC (Methode 9): Retentionszeit = 1.65 min

### Beispiel 638

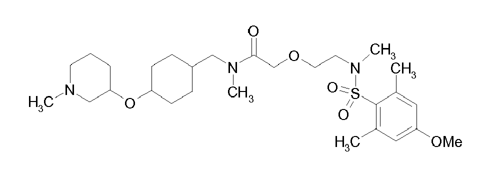

C₂₈H₄₇N₃O₆S x HCl (590.22)
[M+H]+ = 554
HPLC (Methode 5): Retentionszeit = 1.44 min

### Beispiel 639

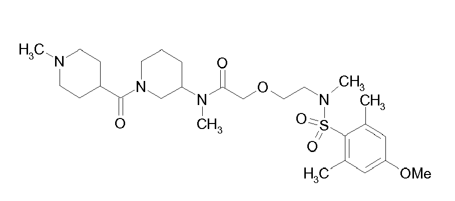

C₂₇H₄₄N₄O₆S x C₂HF₃O₂ (666.75)
[M+H]+ = 553
HPLC (Methode 9): Retentionszeit = 1.59 min

### Beispiel 640

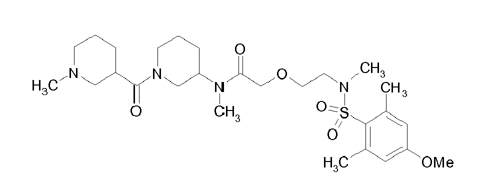

C₂₇H₄₄N₄O₆S x C₂HF₃O₂ (666.75)
[M+H]+ = 553
HPLC (Methode 9): Retentionszeit = 1.61 min

### Beispiel 641

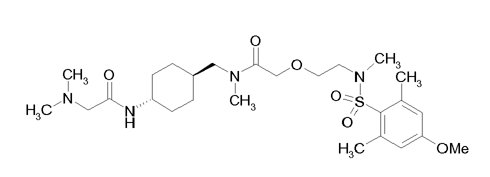

C₂₆H₄₄N₄O₆S x C₂HF₃O₂ (590.22)
[M+H]+ = 541
HPLC (Methode 9): Retentionszeit = 1.56 min

### Beispiel 642

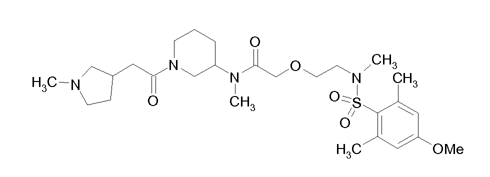

C₂₇H₄₄N₄O₆S x C₂HF₃O₂ (666.75)
[M+H]+ = 553
HPLC (Methode 9): Retentionszeit = 1.55 min

### Beispiel 643

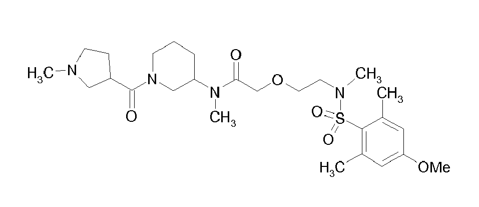

C₂₆H₄₂N₄O₆S x C₂HF₃O₂ (652.72)
[M+H]+ = 539
HPLC (Methode 9): Retentionszeit = 1.55 min

### Beispiel 644

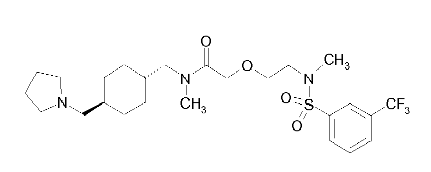

C₂₅H₃₈F₃N₃O₄S (533.66)
[M+H]+ = 534

### Beispiel 645

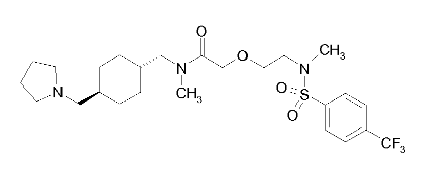

C₂₅H₃₈F₃N₃O₄S (533.66)
[M+H]+ = 534

### Beispiel 646

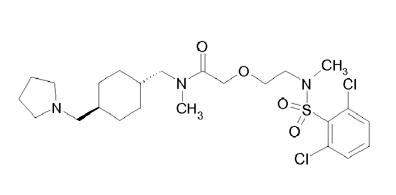

C₂₄H₃₇Cl₂N₃O₄S (534.55)
[M+H]+ = 535

### Beispiel 647

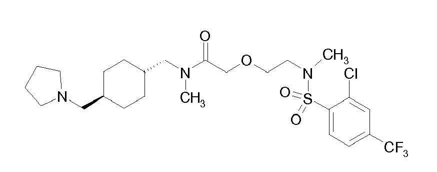

C₂₅H₃₇ClF₃N₃O₄S (568.10)
[M+H]+ = 569

Die folgenden Verbindungen wurden analog zu Beispiel 121 hergestellt: Beispiel 483 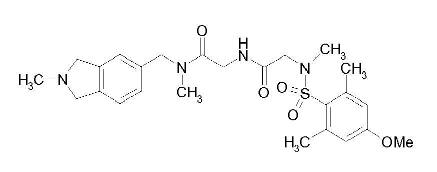 C₂₅H₃₄N₄O₅S x C₂HF₃O₂ (616.65)
[M+H]+ = 503
HPLC (Methode 6): Retentionszeit = 2.30 min

### Beispiel 484

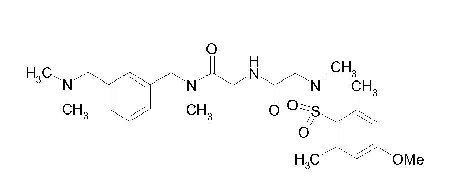

C₂₅H₃₆N₄O₅S x HCl (541.10)
[M+H]+ = 505
HPLC (Methode 5): Retentionszeit = 1.49 min

### Beispiel 485

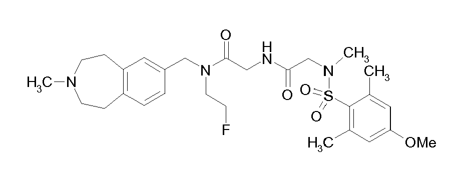

C₂₈H₃₉FN₄O₅S x C₂HF₃O₂ (676.72)
[M+H]+ = 563
HPLC (Methode 5): Retentionszeit = 1.55 min

### Beispiel 486

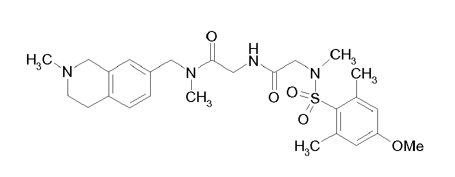

C₂₆H₃₆N₄O₅S x HCl (553.11)
[M+H]+ = 517
HPLC (Methode 11): Retentionszeit = 1.73 min

### Beispiel 487

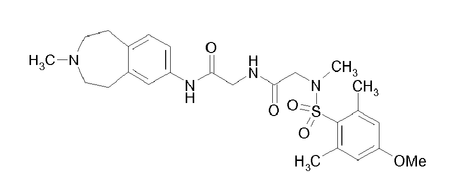

C₂₅H₃₄N₄O₅S x C₂HF₃O₂ (616.65)
[M+H]+ = 503
HPLC (Methode 5): Retentionszeit = 1.49 min

### Beispiel 488

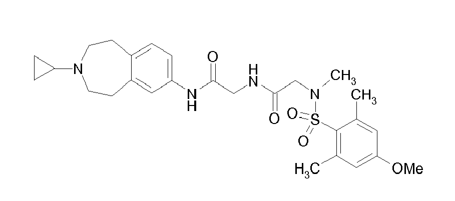

C₂₇H₃₆N₄O₅S x C₂HF₃O₂ (642.69)
[M+H]+ = 529
HPLC (Methode 5): Retentionszeit = 1.50 min

### Beispiel 489

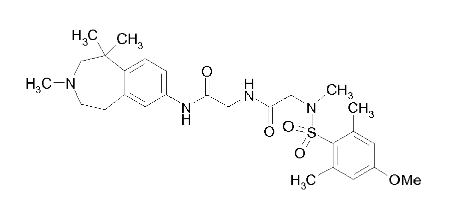

C₂₇H₃₈N₄O₅S x C₂HF₃O₂ (644.70)
[M+H]+ = 531
HPLC (Methode 5): Retentionszeit = 1.54 min

### Beispiel 490

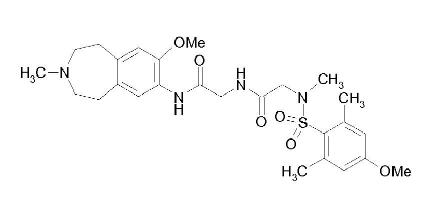

C₂₆H₃₆N₄O₆S x C₂HF₃O₂ (646.68)
[M+H]+ = 533
HPLC (Methode 5): Retentionszeit = 1.51 min

### Beispiel 491

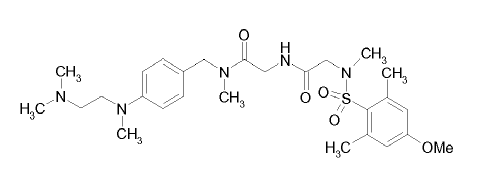

C₂₇H₄₁N₅O₅S x HCl (584.17)
[M+H]+ = 548
HPLC (Methode 5): Retentionszeit = 1.53 min

### Beispiel 492

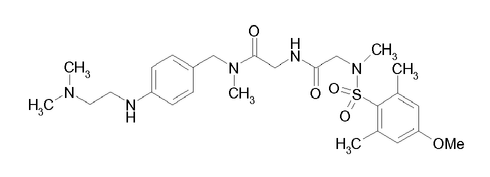

C₂₆H₃₉N₅O₅S x HCl (570.15)
[M+H]+ = 534
HPLC (Methode 5): Retentionszeit = 1.52 min

### Beispiel 493

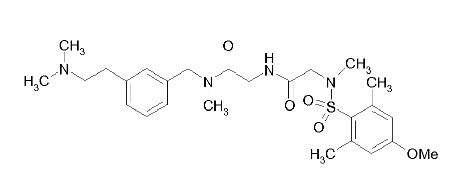

C₂₆H₃₈N₄O₅S x HCl (555.13)
[M+H]+ = 519
HPLC (Methode 5): Retentionszeit = 1.51 min

### Beispiel 494

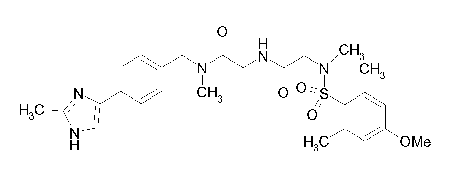

C₂₆H₃₃N₅O₅S (527.64)
[M+H]+ = 528
HPLC (Methode 4): Retentionszeit = 3.0 min

### Beispiel 495

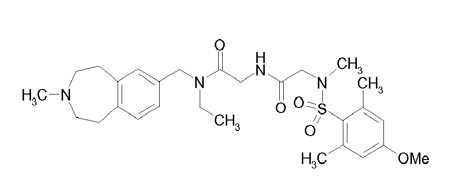

C₂₈H₄₀N₄O₅S x HCl (581.17)
[M+H]+ = 545
HPLC (Methode 4): Retentionszeit = 3.1 min

### Beispiel 496

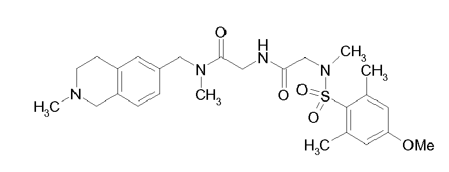

C₂₆H₃₆N₄O₅S x HCl (553.11)
[M+H]+ = 517
HPLC (Methode 5): Retentionszeit = 1.48 min

### Beispiel 497

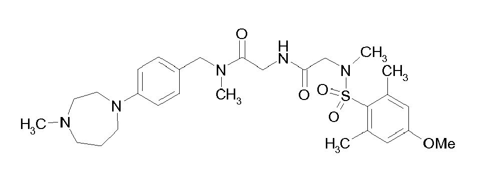

C₂₈H₄₁N₅O₅S x HCl (596.18)
[M+H]+ = 560
HPLC (Methode 5): Retentionszeit = 1.52 min

### Beispiel 498

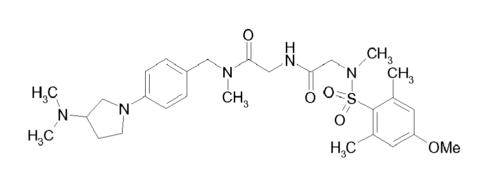

C₂₈H₄₁N₅O₅S x HCl (596.18)
[M+H]+ = 560
HPLC (Methode 5): Retentionszeit = 1.52 min

### Beispiel 499

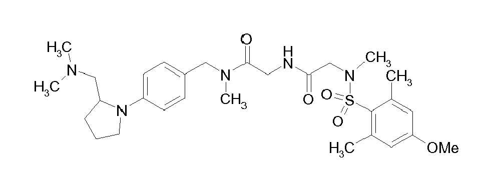

C₂₉H₄₃N₅O₅S x HCl (610.21)
[M+H]+ = 574
HPLC (Methode 5): Retentionszeit = 1.57 min

### Beispiel 500

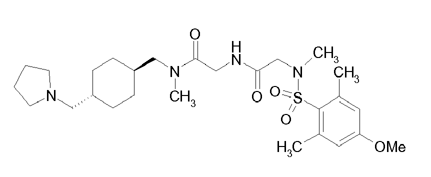

C₂₇H₄₄N₄O₅S x HCl (573.19)
[M+H]+ = 537
DC: Kieselgel, Dichlormethan / Methanol / Ammoniak 8:2:0.01, Rf-Wert = 0.66

### Beispiel 501

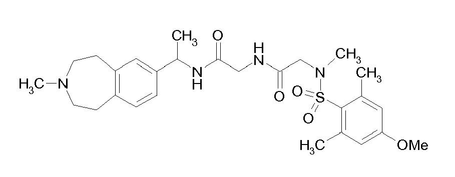

C₂₇H₃₈N₄O₅S x HCl (567.14)
[M+H]+ = 531
HPLC (Methode 4): Retentionszeit = 3.0 min

### Beispiel 502

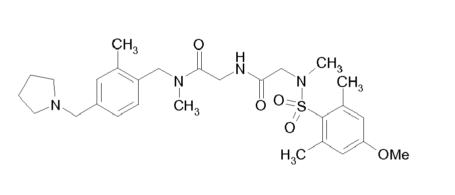

C₂₈H₄₀N₄O₅S x HCl (581.17)
[M+H]+ = 545
DC: Kieselgel, Dichlormethan / Methanol 9:1, Rf-Wert = 0.18

### Beispiel 503

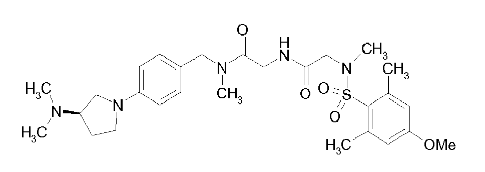

C₂₈H₄₁N₅O₅S x HCl (596.18)
[M+H]+ = 560
DC: Kieselgel, Ethylacetat / Methanol / Ammoniak 9:1:0.1, Rf-Wert = 0.42

### Beispiel 504

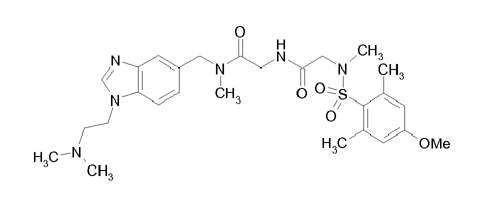

C₂₇H₃₈N₆O₅S (558.69)
[M+H]+ = 559
HPLC (Methode 6): Retentionszeit = 2.23 min

### Beispiel 505

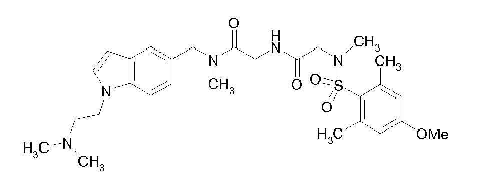

C₂₈H₃₉N₅O₅S (557.71)
[M+H]+ = 558
HPLC (Methode 6): Retentionszeit = 2.35 min

### Beispiel 506

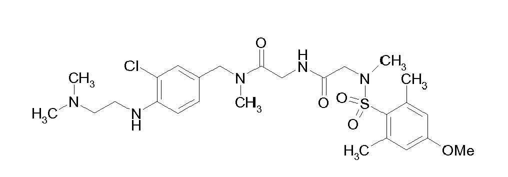

C₂₆H₃₈ClN₅O₅S x CH₂O₂ (614.16)
[M+H]+ = 568/570
HPLC (Methode 6): Retentionszeit = 2.55 min

### Beispiel 507

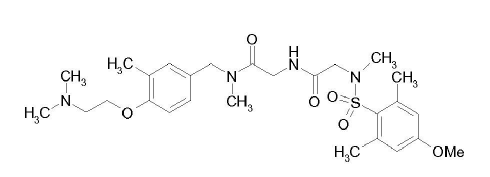

C₂₇H₄₀N₄O₆S (548.70)
[M+H]+ = 549
HPLC (Methode 6): Retentionszeit = 2.49 min

### Beispiel 508

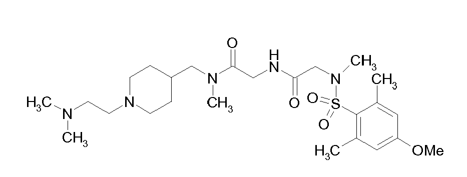

C₂₅H₄₃N₅O₅S (525.71)
[M+H]+ = 526
HPLC (Methode 6): Retentionszeit = 1.85 min

### Beispiel 509

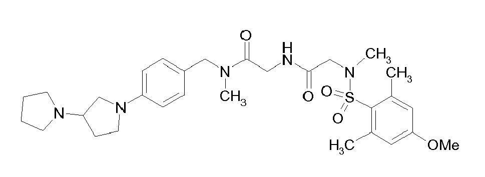

C₃₀H₄₃N₅O₅S (585.76)
[M+H]+ = 586
HPLC (Methode 6): Retentionszeit = 2.66 min

### Beispiel 510

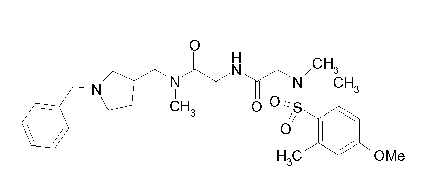

C₂₇H₃₈N₄O₅S (530.68)
[M+H]+ = 531
HPLC (Methode 6): Retentionszeit = 2.52 min

### Beispiel 511

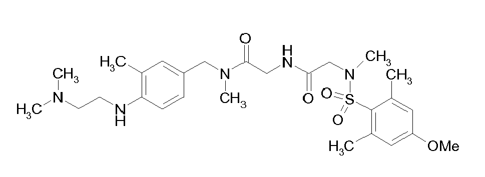

C₂₇H₄₁N₅O₅S (547.71)
[M+H]+ = 548
HPLC (Methode 6): Retentionszeit = 2.44 min

### Beispiel 512

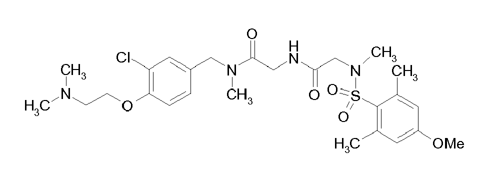

C₂₆H₃₇ClN₄O₆S (569.11)
[M+H]+ = 570
HPLC (Methode 6): Retentionszeit = 2.56 min

### Beispiel 513

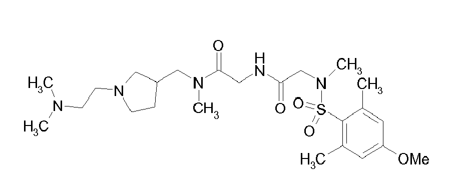

C₂₄H₄₁N₅O₅S (511.68)
[M+H]+ = 512
HPLC (Methode 6): Retentionszeit = 1.80 min

### Beispiel 514

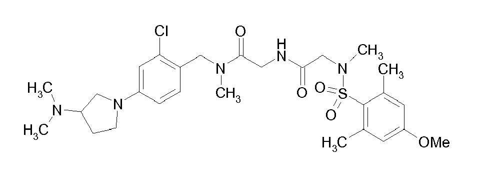

C₂₈H₄₀ClN₅O₅S (594.17)
[M+H]+ = 594/596
HPLC (Methode 6): Retentionszeit = 2.68 min

### Beispiel 515

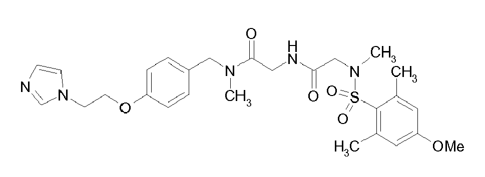

C₂₇H₃₅N₅O₆S (557.66)
[M+H]+ = 558
HPLC (Methode 6): Retentionszeit = 2.51 min

### Beispiel 516

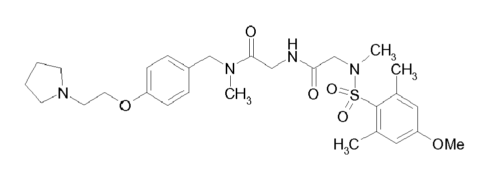

C₂₈H₄₀N₄O₆S (560.71)
[M+H]+ = 561
HPLC (Methode 6): Retentionszeit = 2.51 min

### Beispiel 517

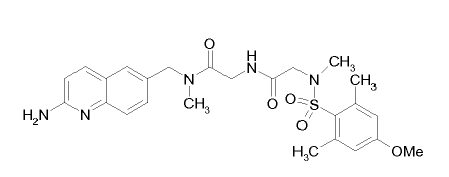

C₂₅H₃₁N₅O₅S (513.61)
[M+H]+ = 514
HPLC (Methode 6): Retentionszeit = 2.35 min

### Beispiel 518

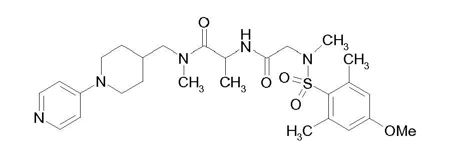

C₂₇H₃₉N₅O₅S x C₂HF₃O₂ (659.72)
[M+H]+ = 546
HPLC (Methode 6): Retentionszeit = 2.44 min

### Beispiel 519

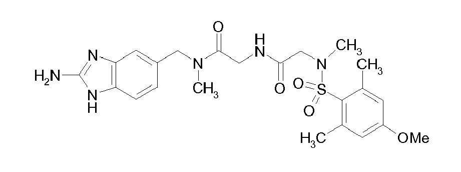

C₂₃H₃₀N₆O₅S (502.59)
[M+H]+ = 503
HPLC (Methode 6): Retentionszeit = 2.37 min

### Beispiel 520

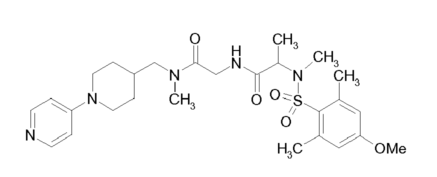

C₂₇H₃₉N₅O₅S x C₂HF₃O₂ (659.72)
[M+H]+ = 546
HPLC (Methode 6): Retentionszeit = 2.52 min

### Beispiel 521

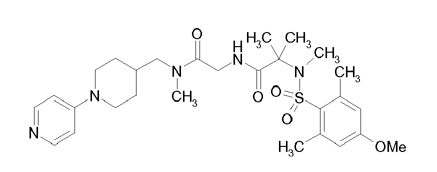

C₂₈H₄₁N₅O₅S x C₂HF₃O₂ (673.75)
[M+H]+ = 560
HPLC (Methode 6): Retentionszeit = 2.58 min

### Beispiel 522

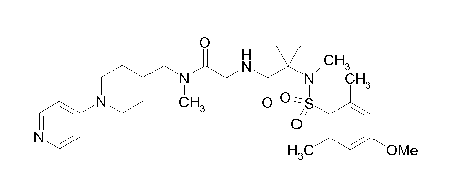

C₂₈H₃₉N₅O₅S x C₂HF₃O₂ (671.73)
[M+H]+ = 558
HPLC (Methode 6): Retentionszeit = 2.57 min

### Beispiel 523

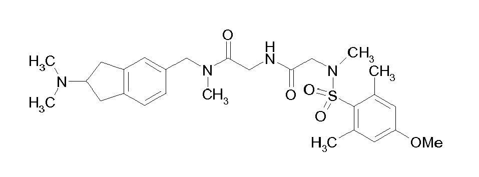

C₂₇H₃₈N₄O₅S x C₂HF₃O₂ (644.70)
[M+H]+ = 531
HPLC (Methode 6): Retentionszeit = 2.45 min

### Beispiel 524

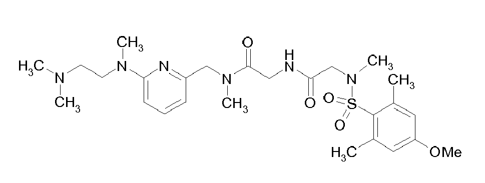

C₂₆H₄₀N₆O₅S (548.70)
[M+H]+ = 549
HPLC (Methode 6): Retentionszeit = 2.52 min

### Beispiel 525

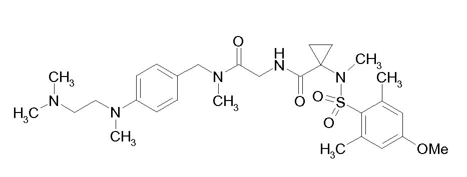

C₂₉H₄₃N₅O₅S x C₂HF₃O₂ (687.77)
[M+H]+ = 574
HPLC (Methode 6): Retentionszeit = 2.72 min

### Beispiel 526

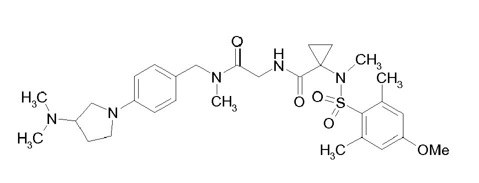

C₃₀H₄₃N₅O₅S (585.76)
[M+H]+ = 586
HPLC (Methode 6): Retentionszeit = 2.70 min

### Beispiel 527

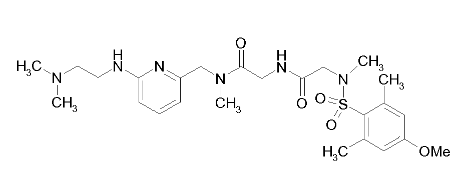

C₂₅H₃₈N₆O₅S x CH₂O₂ (580.70)
[M+H]+ = 535
HPLC (Methode 6): Retentionszeit = 2.16 min

### Beispiel 528

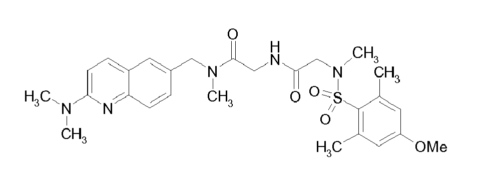

C₂₇H₃₅N₅O₅S x C₂HF₃O₂ (655.69)
[M+H]+ = 542
HPLC (Methode 6): Retentionszeit = 2.48 min

### Beispiel 529

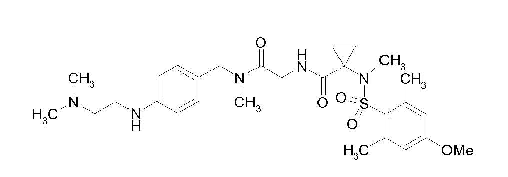

C₂₈H₄₁N₅O₅S x C₂HF₃O₂ (673.75)
[M+H]+ = 560
HPLC (Methode 6): Retentionszeit = 2.61 min

### Beispiel 530

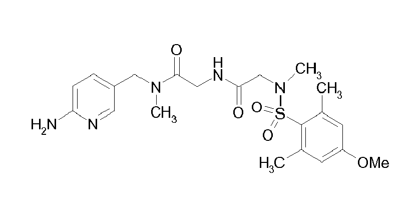

C₂₁H₂₉N₅O₅S (463.55)
[M+H]+ = 464
HPLC (Methode 9): Retentionszeit = 1.47 min

### Beispiel 531

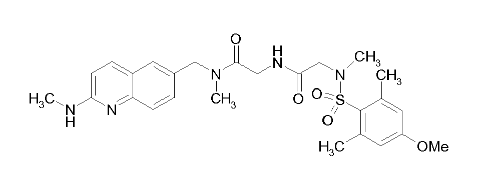

C₂₆H₃₃N₅O₅S (527.64)
[M+H]+ = 528
HPLC (Methode 9): Retentionszeit = 1.60 min

### Beispiel 532

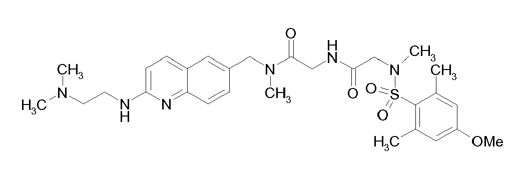

C₂₉H₄₀N₆O₅S (584.73)
[M+H]+ = 585
HPLC (Methode 9): Retentionszeit = 1.37 min

### Beispiel 533

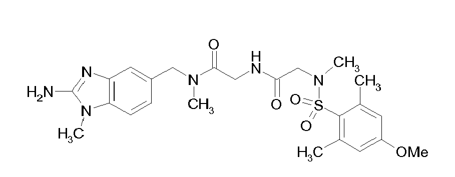

C₂₄H₃₂N₆O₅S x C₂HF₃O₂ (630.64)
[M+H]+ = 517
HPLC (Methode 9): Retentionszeit = 1.58 min

### Beispiel 534

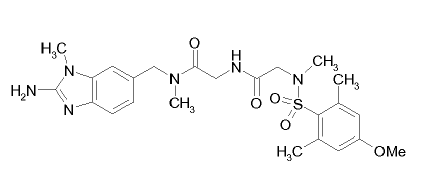

C₂₄H₃₂N₆O₅S x C₂HF₃O₂ (630.64)
[M+H]+ = 517
HPLC (Methode 9): Retentionszeit = 1.57 min

### Beispiel 535

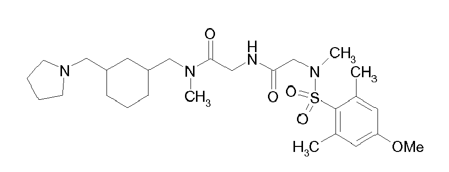

C₂₇H₄₄N₄O₅S x C₂HF₃O₂ (650.75)
[M+H]+ = 537
HPLC (Methode 9): Retentionszeit = 1.68 min

### Beispiel 536

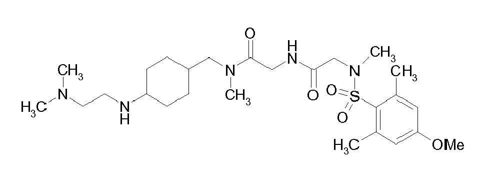

C₂₆H₄₅N₅O₅S x 2HCl (612.65)
[M+H]+ = 540
HPLC (Methode 5): Retentionszeit = 1.39 min

### Beispiel 537

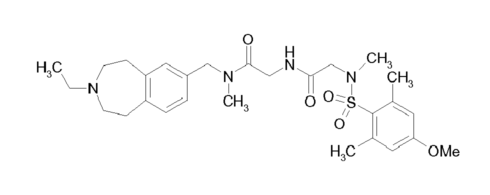

C₂₈H₄₀N₄O₅S x C₂HF₃O₂ (658.73)
[M+H]+ = 545
HPLC (Methode 5): Retentionszeit = 1.53 min

### Beispiel 538

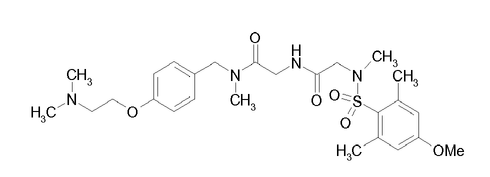

C₂₆H₃₈N₄O₆S x C₂HF₃O₂ (648.69)
[M+H]+ = 535
HPLC (Methode 5): Retentionszeit = 1.43 min

### Beispiel 539

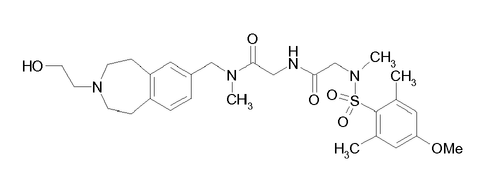

C₂₈H₄₀N₄O₆S (560.71)
[M+H]+ = 561
HPLC (Methode 5): Retentionszeit = 1.50 min

### Beispiel 540

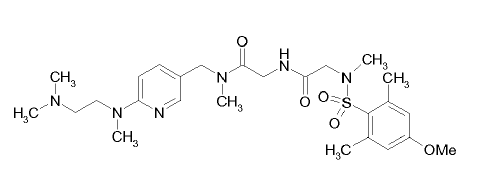

C₂₆H₄₀N₆O₅S (548.70)
[M+H]+ = 549
HPLC (Methode 5): Retentionszeit = 1.39 min

### Beispiel 541

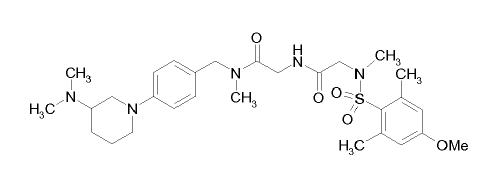

C₂₉H₄₃N₅O₅S x HCl (610.21)
[M+H]+ = 574
HPLC (Methode 7): Retentionszeit = 1.88 min

### Beispiel 542

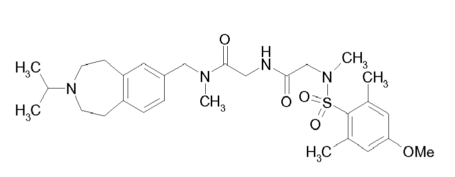

C₂₉H₄₂N₄O₅S x C₂HF₃O₂ (672.76)
[M+H]+ = 559
HPLC (Methode 5): Retentionszeit = 1.55 min

### Beispiel 543

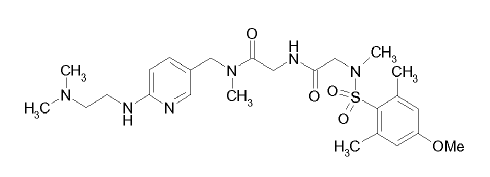

C₂₅H₃₈N₆O₅S x 2HCl (607.59)
[M+H]+ = 535
HPLC (Methode 5): Retentionszeit = 1.39 min

### Beispiel 544

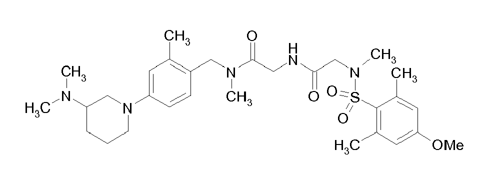

C₃₀H₄₅N₅O₅S x C₂HF₃O₂ (701.80)
[M+H]+ = 588
HPLC (Methode 5): Retentionszeit = 1.55 min

### Beispiel 545

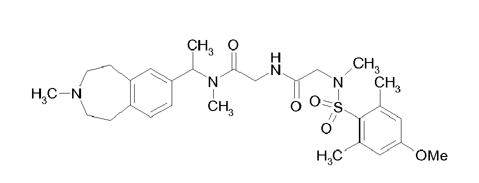

C₂₈H₄₀N₄O₅S x HCl (581.17)
[M+H]+ = 545
HPLC (Methode 4): Retentionszeit = 3.3 min

### Beispiel 546

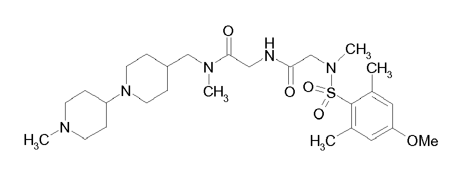

C₂₇H₄₅N₅O₅S x 2HCL (624.66)
[M+H]+ = 552
DC: Kieselgel, Dichlormethan / Methanol / Ammoniak 8:2:0.01, Rf-Wert = 0.25

### Beispiel 547

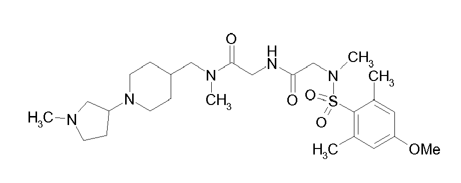

C₂₆H₄₃N₅O₅S x HCl (574.18)
[M+H]+ = 538
DC: Kieselgel, Dichlormethan / Methanol / Ammoniak 8:2:0.01, Rf-Wert = 0.10

### Beispiel 548

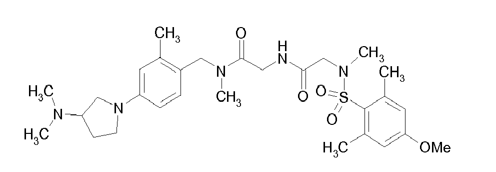

C₂₉H₄₃N₅O₅S x HCl (610.21)
[M+H]+ = 574
DC: Kieselgel, Dichlormethan / Methanol 9:1, Rf-Wert = 0.25

### Beispiel 549

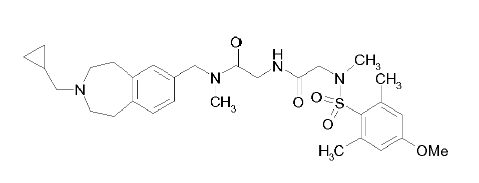

C₃₁H₄₄N₄O₅S x HCl (621.23)
[M+H]+ = 585
HPLC (Methode 12): Retentionszeit = 3.0 min

### Beispiel 550

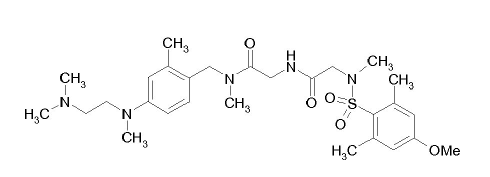

C₂₈H₄₃N₅O₅S x HCl (598.20)
[M+H]+ = 562
DC: Kieselgel, Dichlormethan / Methanol 9:1, Rf-Wert = 0.14

### Beispiel 551

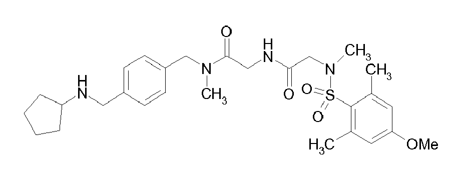

C₂₉H₄₂N₄O₅S x HCl (595.19)
[M+H]+ = 559
DC: Kieselgel, Dichlormethan / Methanol / Ammoniak 9:1:0.1, Rf-Wert = 0.32

### Beispiel 552

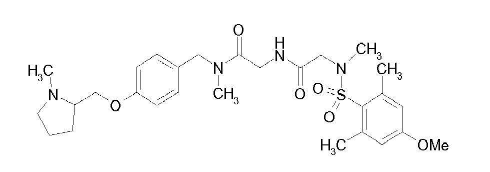

C₂₈H₄₀N₄O₆S (560.71)
[M+H]+ = 561
HPLC (Methode 9): Retentionszeit = 1.66 min

### Beispiel 553

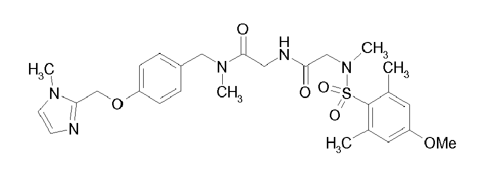

C₂₇H₃₅N₅O₆S (557.66)
[M+H]+ = 558
HPLC (Methode 9): Retentionszeit = 1.64 min

### Beispiel 554

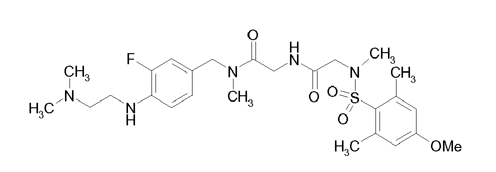

C₂₆H₃₈FN₅O₅S (551.68)
[M+H]+ = 552
HPLC (Methode 6): Retentionszeit = 2.30 min

### Beispiel 555

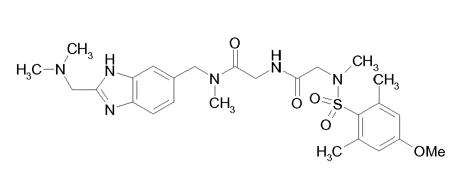

C₂₆H₃₆N₆O₅S (544.67)
[M+H]+ = 545
HPLC (Methode 6): Retentionszeit = 1.48 min

### Beispiel 556

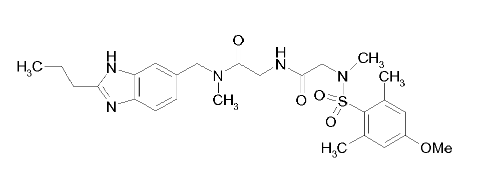

C₂₆H₃₅N₅O₅S (529.65)
[M+H]+ = 530
HPLC (Methode 9): Retentionszeit = 1.61 min

### Beispiel 557

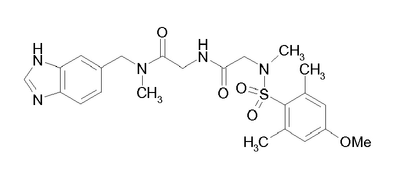

C₂₃H₂₉N₅O₅S (487.57)
[M+H]+ = 488
HPLC (Methode 9): Retentionszeit = 1.53 min

### Beispiel 558

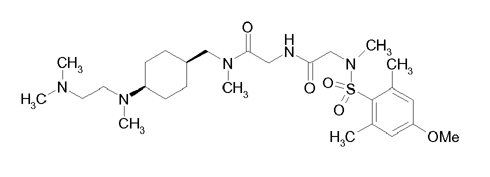

C₂₇H₄₇N₅O₅S x 2HCl (626.68)
[M+H]+ = 554
HPLC (Methode 7): Retentionszeit = 1.61 min

### Beispiel 559

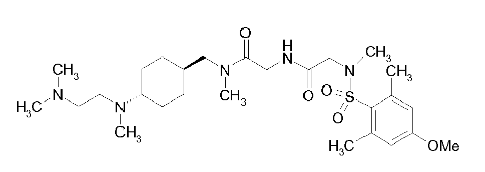

C₂₇H₄₇N₅O₅S x 2HCl (626.68)
[M+H]+ = 554
HPLC (Methode 7): Retentionszeit = 1.61 min

### Beispiel 560

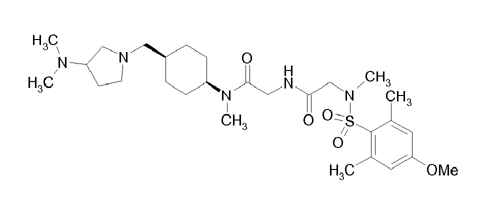

C₂₈H₄₇N₅O₅S x CH₂O₂ (611.80)
[M+H]+ = 566
HPLC (Methode 9): Retentionszeit = 1.33 min

### Beispiel 561

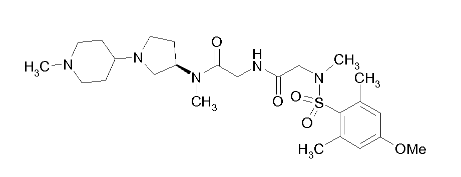

C₂₅H₄₁N₅O₅S x 2HCl (596.61)
[M+H]+ = 524
HPLC (Methode 12): Retentionszeit = 2.3 min

### Beispiel 562

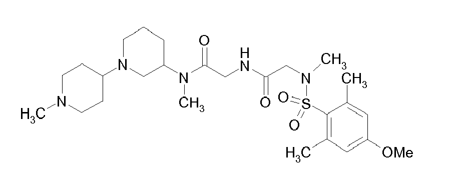

C₂₆H₄₃N₅O₅S x 2HCl (610.64)
[M+H]+ = 538
DC: Kieselgel, Dichlormethan / Methanol / Ammoniak 8:2:0.2, Rf-Wert = 0.53

### Beispiel 563

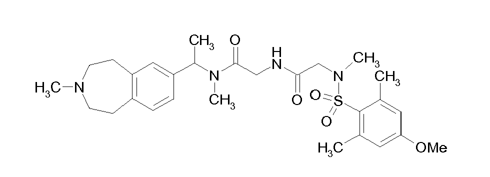

C₂₉H₄₂N₄O₅S x HCl (595.19)
[M+H]+ = 559
HPLC (Methode 12): Retentionszeit = 2.8 min

### Beispiel 564

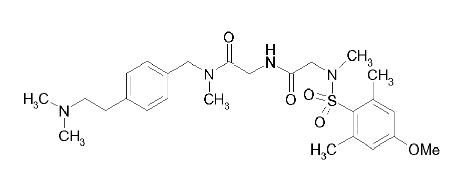

C₂₆H₃₈N₄O₅S x C₂HF₃O₂ (632.69)
[M+H]+ = 519
HPLC (Methode 9): Retentionszeit = 1.61 min

### Beispiel 565

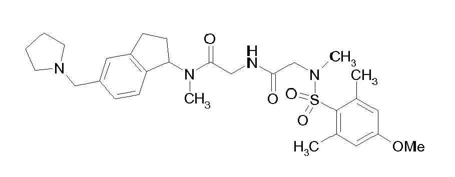

C₂₉H₄₀N₄O₅S (556.72)
[M+H]+ = 557
HPLC (Methode 9): Retentionszeit = 1.69 min

### Beispiel 566

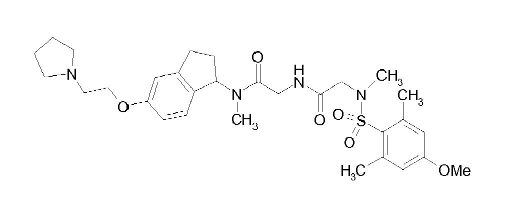

C₃₀H₄₂N₄O₆S (586.74)
[M+H]+ = 587
HPLC (Methode 9): Retentionszeit = 1.74 min

### Beispiel 603

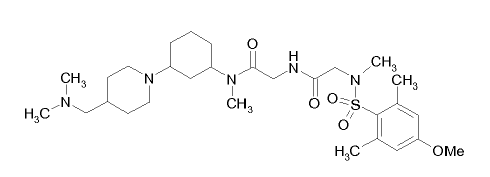

C₂₉H₄₉N₅O₅S x 2HCl (652.72)
[M+H]+ = 580
HPLC (Methode 10): Retentionszeit = 1.11 min

### Beispiel 604

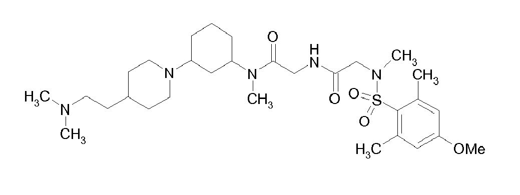

C₃₀H₅₁N₅O₅S x 2HCl (666.74)
[M+H]+ = 594
HPLC (Methode 10): Retentionszeit = 1.11 min

### Beispiel 605

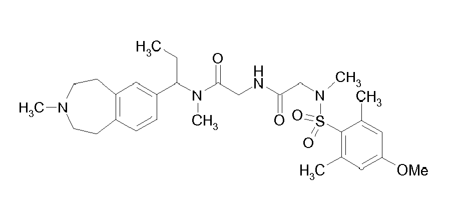

C₂₉H₄₂N₄O₅S x HCl (595.19)
[M+H]+ = 559
HPLC (Methode 12): Retentionszeit = 3.1 min

### Beispiel 606

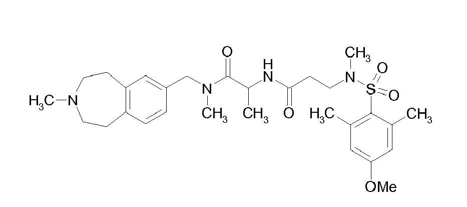

C₂₉H₄₂N₄O₅S x HCl (595.19)
[M+H]+ = 559
DC: Kieselgel, Dichlormethan / Methanol / Ammoniak 8:2:0.01, Rf-Wert = 0.68

### Beispiel 631

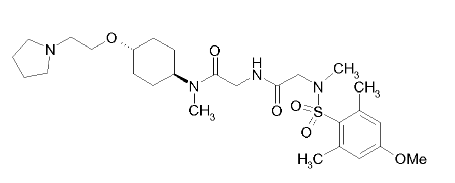

C₂₇H₄₄N₄O₆S x HCl (589.19)
[M+H]+ = 553
HPLC (Methode 5): Retentionszeit = 1.35 min

### Beispiel 632

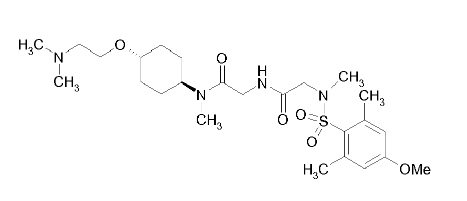

C₂₅H₄₂N₄O₆S x HCl (563.15)
[M+H]+ = 527
HPLC (Methode 5): Retentionszeit = 1.38 min

### Beispiel 633

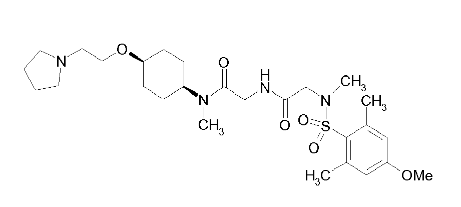

C₂₇H₄₄N₄O₆S x HCl (589.19)
[M+H]+ = 553
HPLC (Methode 5): Retentionszeit = 1.35 min

### Beispiel 634

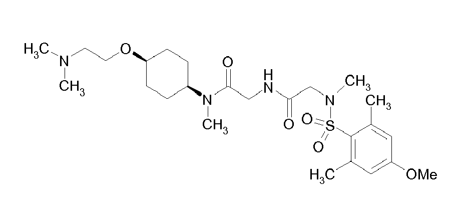

C₂₅H₄₂N₄O₆S x HCl (563.15)
[M+H]+ = 527
HPLC (Methode 5): Retentionszeit = 1.32 min

Die folgenden Verbindungen wurden analog zu Beispiel 130 hergestellt:

### Beispiel 567

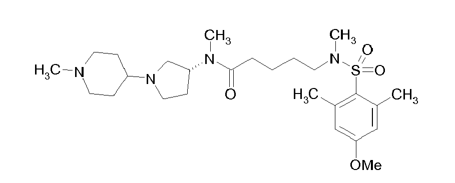

C₂₆H₄₄N₄O₄S x 2C₂HF₃O₂ (736.76)
[M+H]+ = 509
HPLC (Methode 6): Retentionszeit = 1.36 min

### Beispiel 568

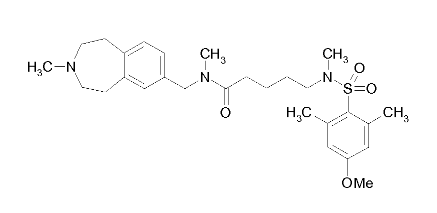

C₂₈H₄₁N₃O₄S x C₂HF₃O₂ (629.73)
[M+H]+ = 516
HPLC (Methode 6): Retentionszeit = 1.77 min

Die folgenden Verbindungen wurden analog zu Beispiel 136 hergestellt: Beispiel 569 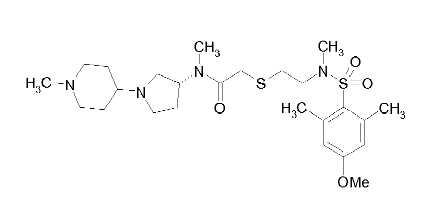 C₂₅H₄₂N₄O₄S₂ x C₂HF₃O₂ (640.78)
[M+H]+ = 527
HPLC (Methode 6): Retentionszeit = 1.35 min

### Beispiel 570

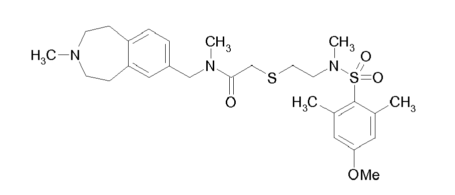

C₂₇H₃₉N₃O₄S₂ (533.75)
[M+H]+ = 534
HPLC (Methode 6): Retentionszeit = 1.74 min

Die folgende Verbindung wurde analog zu Beispiel 138 hergestellt:

### Beispiel 571

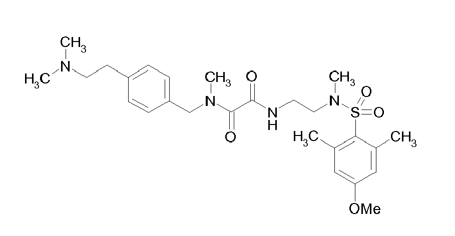

C₂₆H₃₈N₄O₅S x CH₂O₂ (564.70)
[M+H]+ = 519
HPLC (Methode 6): Retentionszeit = 2.33 min

### Beispiel 572

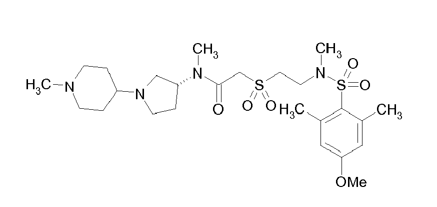

572a) 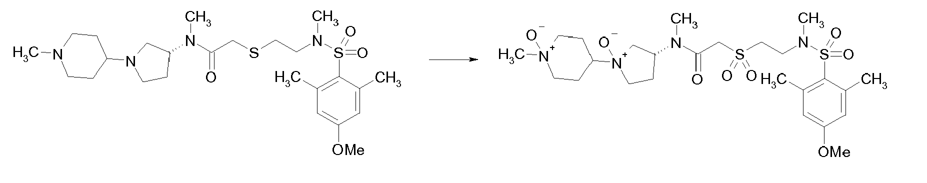 Eine Mischung aus 88.0 mg (0.167 mMol) 569, 0.15 g (0.61 mMol) 70 %ige *m*-Chlorperbenzoesäure (Fluka) und 3 ml Dichlormethan wird 30 Minuten bei Raumtemperatur gerührt und danach im Vakuum bis zur Trockene eingeengt. Der Rückstand wird in Methanol gelöst und membranfiltriert. Das Produkt wird anschließend durch präparative HPLC aus dem Filtrat gewonnen.
   C₂₅H₄₂N₄O₈S₂ (590.76)
   [M+H]+ = 591
572b) 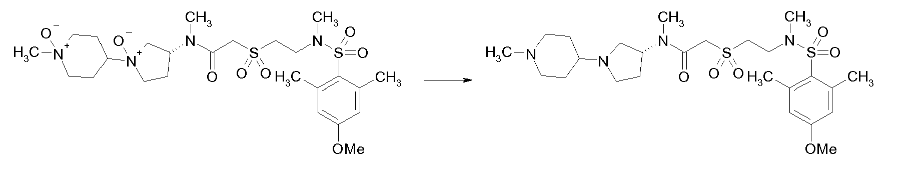 Eine Mischung aus 90.0 mg (0.15 mMol) Produkt aus 572a, 20.0 mg Raney-Nickel und 10 ml THF wird im Autoklaven eine Stunde bei Raumtemperatur gerührt. Anschließend wird der Katalysator abfiltriert und das Filtrat im Vakuum bis zur Trockene eingeengt. Das so erhaltene Rohpodukt wird durch präparative HPLC gereinigt.
   C₂₅H₄₂N₄O₆S₂ x CH₂O₂ (604.78)
   [M+H]+ = 559
   HPLC (Methode 6): Retentionszeit = 1.33 min

### Beispiel 573

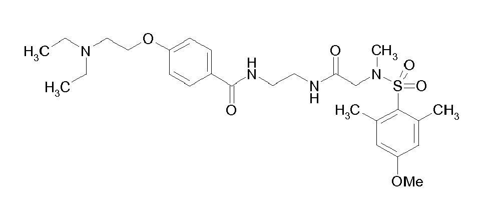

573a) 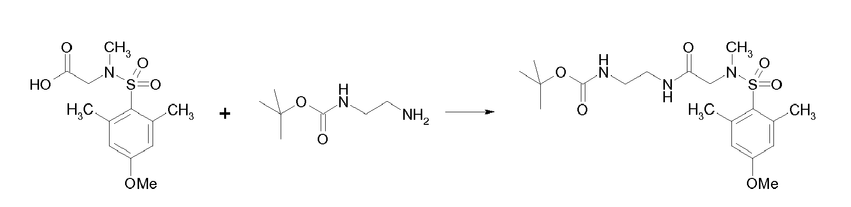 573a wird analog zu 1f aus 2.16 g (7.50 mMol) Produkt aus 121b, 1.20 g (7.50 mMol) N-Boc-ethylendiamin (Fluka), 3.14 ml (22.50 mMol) Triethylamin und 2.41 g (7.50 mMol) TBTU in 28 ml THF und 4 ml DMF hergestellt.
   C₁₉H₃₁N₃O₆S (429.53)
   DC: Kieselgel, Dichlormethan / Ethanol 19:1, Rf-Wert = 0.35
573b) 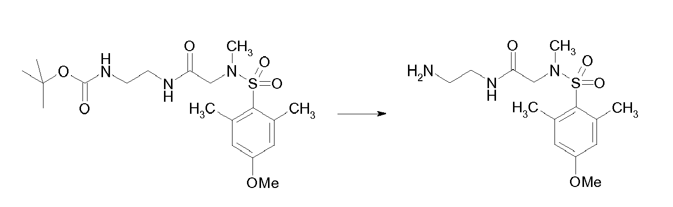 573b wird analog zu 28d aus 2.70 g (6.29 mMol) Produkt aus 573a und 7 ml TFA in 50 ml Dichlormethan hergestellt.
   C₁₄H₂₃N₃O₄ (329.42)
   DC: Kieselgel, Dichlormethan / Ethanol 9:1, Rf-Wert = 0.15
573c)

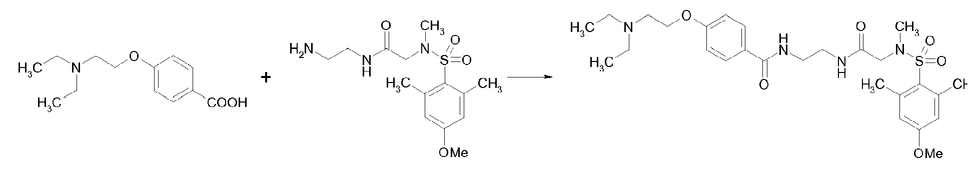

Beispiel 573 wird analog zu 1f aus 0.119 g (0.50 mMol) 4-(2-Diethylaminoethoxy)-benzoesäure (J. Med. Chem. 14, 1971, 836-842), 0.165 g (0.50 mMol) Produkt aus 573b, 0.21 ml (1.50 mMol) Triethylamin und 0.16 g (0.50 mMol) TBTU in 7 ml THF und 1 ml DMF hergestellt.
C₂₇H₄₀N₄O₆S x HCl (585.16)
HPLC (Methode 5): Retentionszeit = 1.44 min

Die folgenden Verbindungen wurde analog zu Beispiel 573 hergestellt:

### Beispiel 574

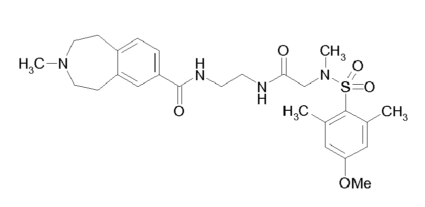

C₂₆H₃₆N₄O₅S x HCl (553.11)
[M+H]+ = 517
HPLC (Methode 5): Retentionszeit = 1.40 min

### Beispiel 607

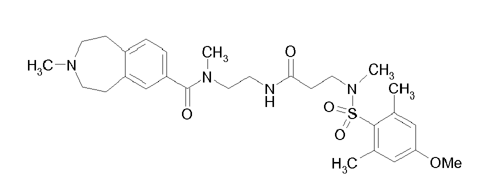

C₂₈H₄₀N₄O₅S x HCl (581.17)
[M+H]+ = 545
HPLC (Methode 12): Retentionszeit = 3.51 min

### Beispiel 635

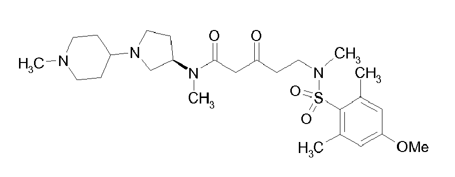

635a) 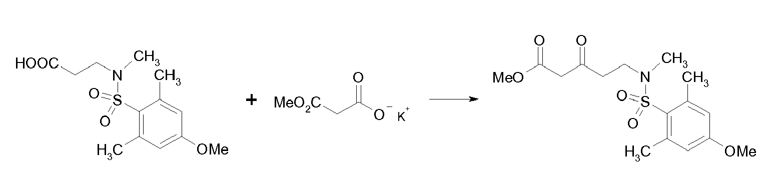 Eine Mischung aus 0.78 g (4.98 mMol) Monomethylmalonat Kaliumsalz (Fluka), 0.52 g (5.47 mMol) Magnesiumchlorid und 30 ml THF wird vier Stunden bei 50°C gerührt. Eine zweite Mischung aus 1.00 g (3.32 mMol) Produkt aus 22c, 0.65 g (3.98 mMol) CDI und 20 ml THF wird zuerst eine Stunde bei RT gerührt und anschließend zur ersten Mischung hinzu gegeben. Man lässt über Nacht bei RT rühren und filtriert danach den entstandenen Niederschlag ab. Das Filtrat wird im Vakuum bis zur Trockene eingeengt. Das so erhaltene Rohprodukt wird mit Wasser verrieben, abfiltriert und im Vakuumtrockenschrank bei 45°C getrocknet.
   C₁₆H₂₃NO₆S (357.42)
   [M+H]+ = 358
   HPLC (Methode 9): Retentionszeit = 2.19 min
635b) 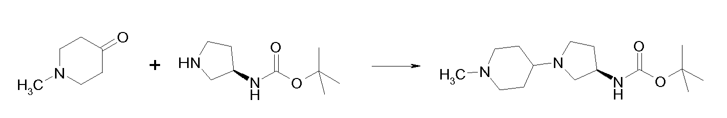 635b wird analog zu 60a aus 0.47 ml (4.07 mMol) 1-Methyl-4-piperidon (Fluka), 0.76 g .(4.07 mMol) (R)-3-(Boc-amino)-pyrrolidin (Fluka), 1.72 g (8.13 mMol) Natriumtriacetoxyborhydrid und 0.23 ml (4.07 mMol) Essigsäure in 10 ml Dichlormethan hergestellt.
   C₁₅H₂₉N₃O₂ (283.41)
   [M+H]+ = 284
635c) 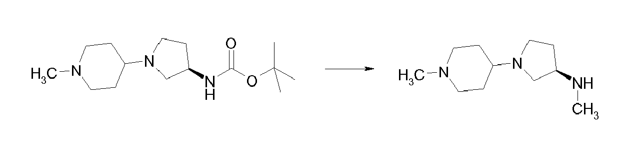 635c wird analog zu 38f aus 0.90 g (3.18 mMol) Produkt aus 635b und 5.0 ml (10.00 mMol) Lithiumaluminiumhydrid (2M in THF) in 15 ml THF hergestellt.
   C₁₁H₂₃N₃ (197.32)
   [M+H]+ = 198
635d) 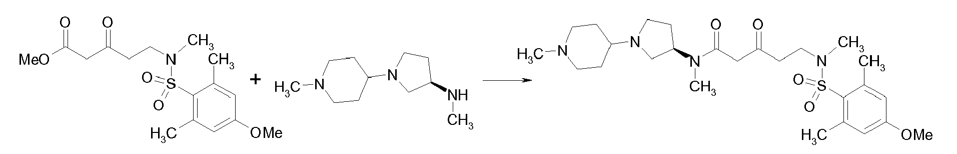 Eine Mischung aus 0.56 g (1.58 mMol) Produkt aus 635a, 0.54 g (2.73 mMol) Produkt aus 635c und 5 ml Toluol wird 24 Stunden auf 120°C erhitzt. Anschließend wird die Reaktionsmischung im Vakuum bis zur Trockene eingeengt. Das so erhaltene Rohprodukt wird durch präparative HPLC gereinigt.
   C₂₆H₄₂N₄O₅S (522.70)
   [M+H]+ = 523
   HPLC (Methode 9): Retentionszeit = 1.33 min

Analog zu den voranstehend genannten Beispielen wurden erhalten:#

### Beispiel 651

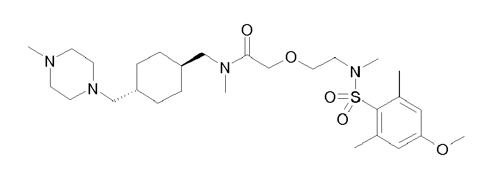

### Beispiel 652

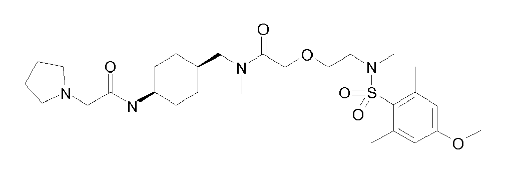

### Beispiel 653

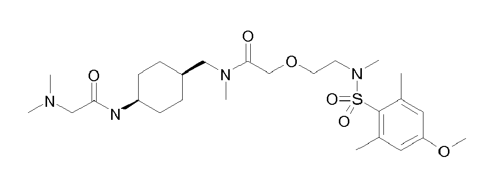

### Beispiel 654

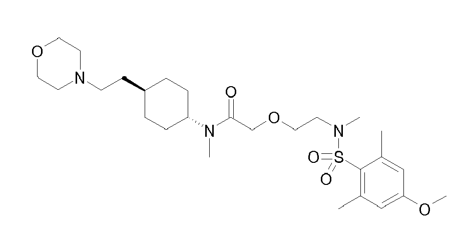

### Beispiel 655

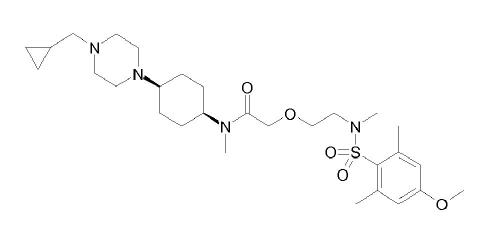

### Beispiel 656

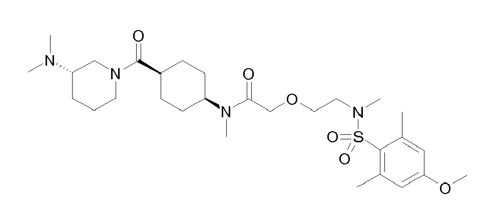

### Beispiel 657

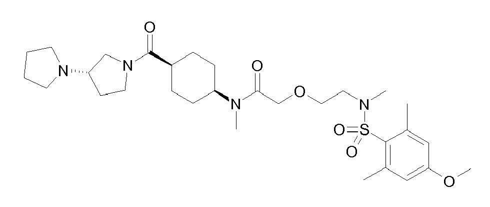

### Beispiel 658

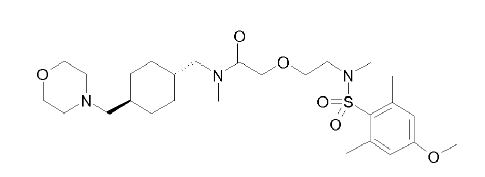

### Beispiel 659

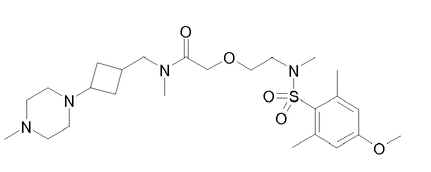

### Beispiel 660

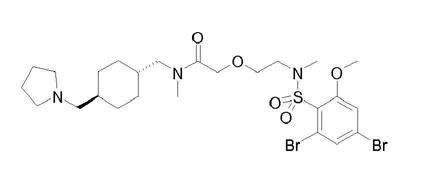

### Beispiel 661

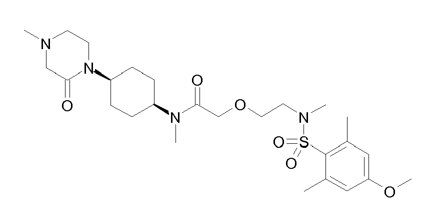

### Beispiel 662

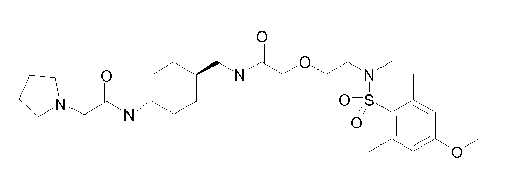

### Beispiel 663

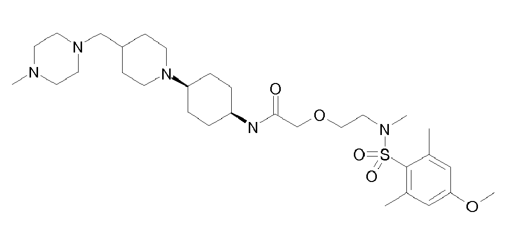

### Beispiel 664

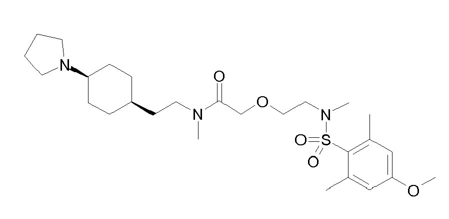

### Beispiel 665

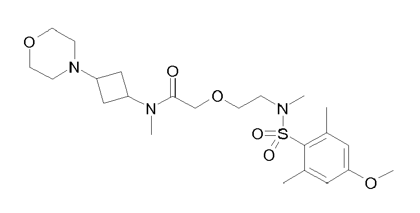

### Beispiel 666

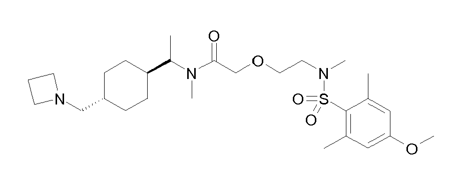

### Beispiel 667

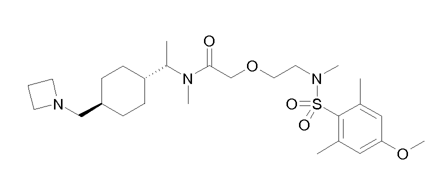

### Beispiel 668

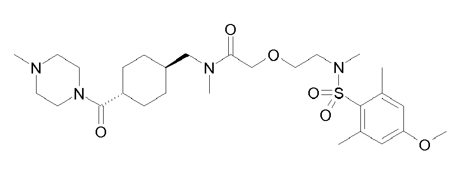

### Beispiel 669

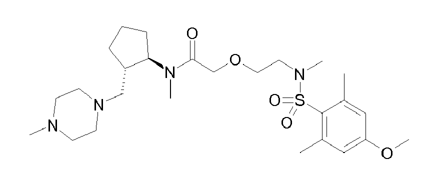

### Beispiel 670

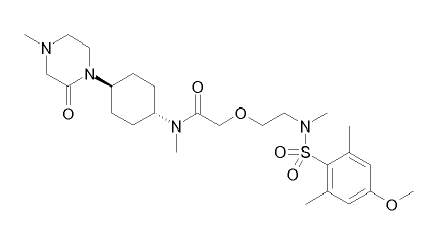

### Beispiel 671

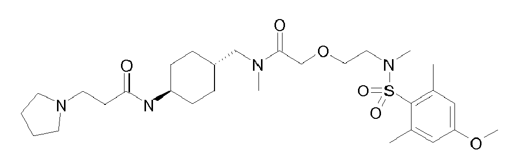

### Beispiel 672

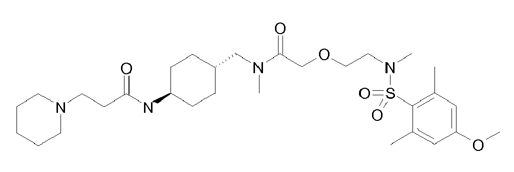

### Beispiel 673

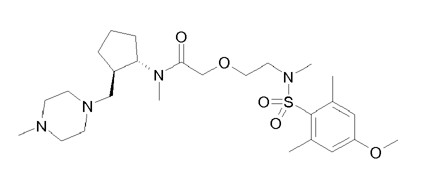

### Beispiel 674

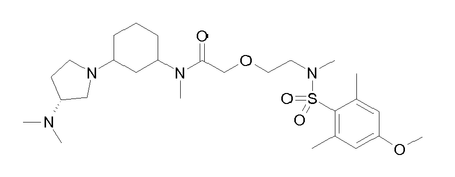

### Beispiel 675

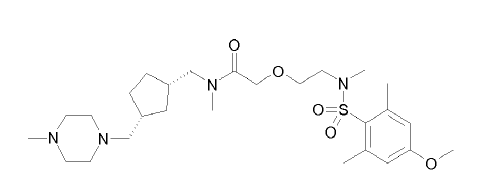

### Beispiel 676

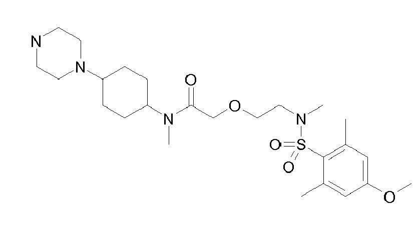

### Beispiel 677

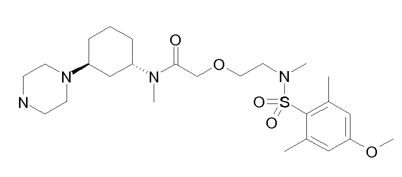

### Beispiel 678

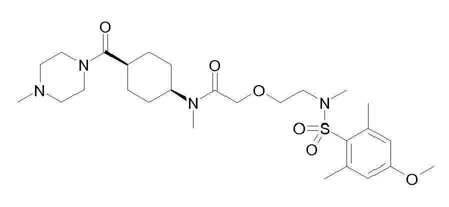

### Beispiel 679

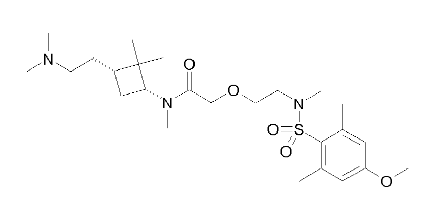

### Beispiel 680

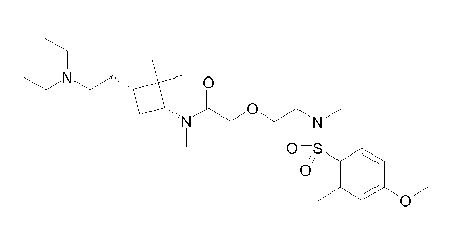

### Beispiel 681

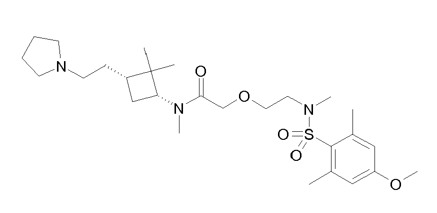

### Beispiel 682

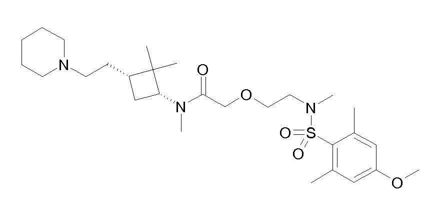

### Beispiel 683

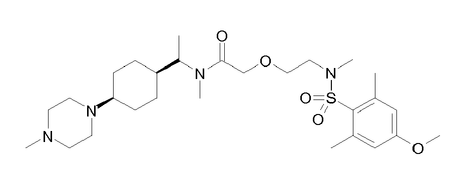

### Beispiel 684

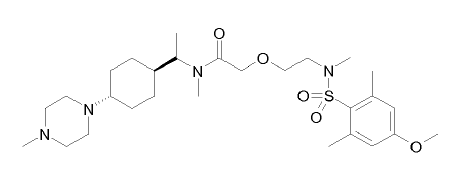

### Beispiel 685

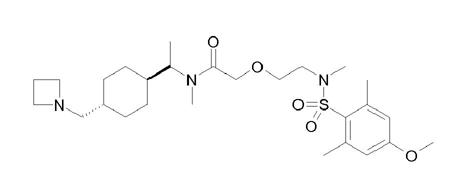

### Beispiel 686

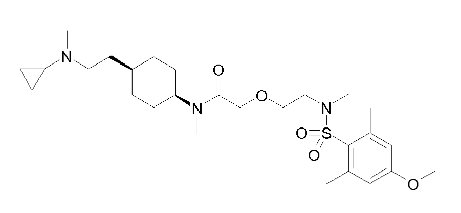

### Beispiel 687

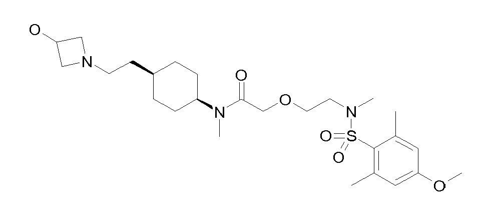

### Beispiel 688

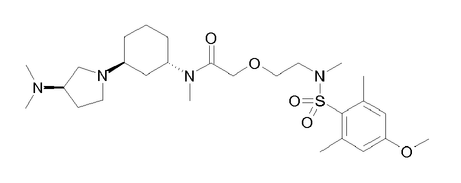

### Beispiel 689

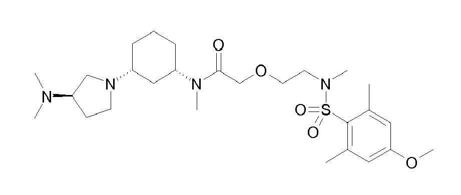

### Beispiel 690

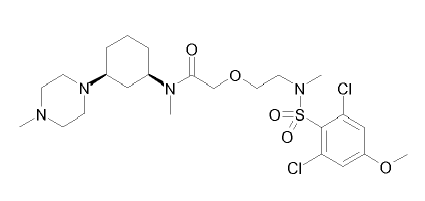

### Beispiel 691

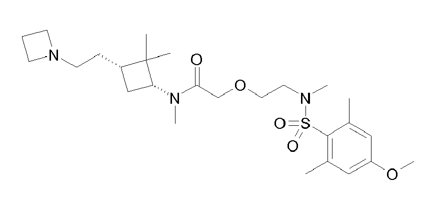

### Beispiel 692

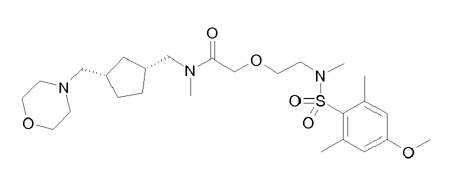

### Beispiel 693

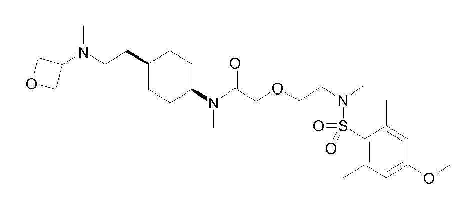

### Beispiel 694

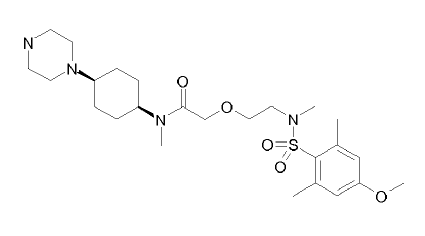

### Beispiel 695

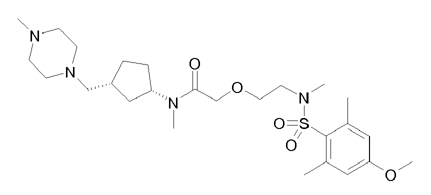

### Beispiel 696

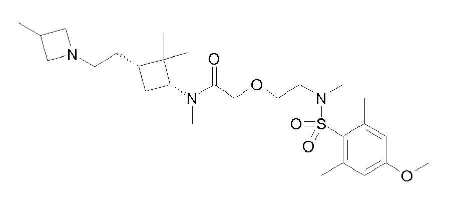

### Beispiel 697

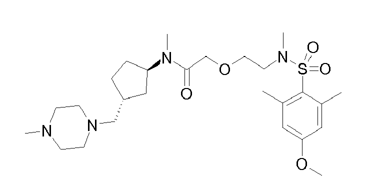

### Beispiel 698

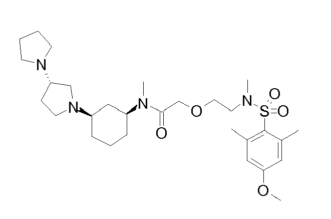

### Beispiel 699

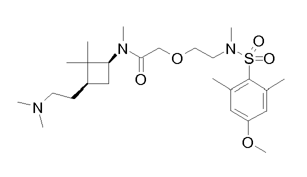

### Beispiel 700

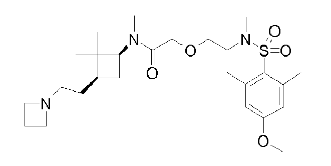

### Beispiel 701

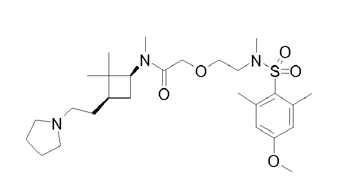

### Beispiel 702

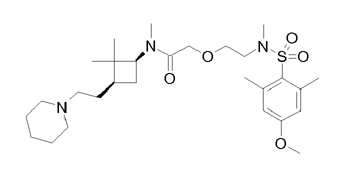

### Beispiel 703

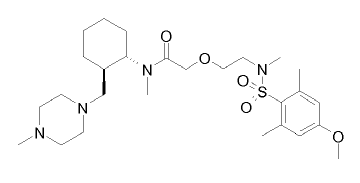

### Beispiel 704

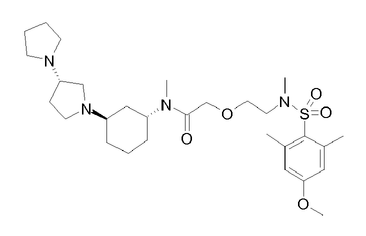

### Beispiel 705

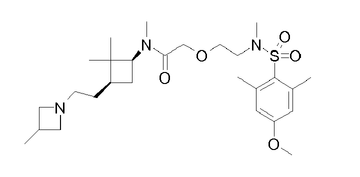

### Beispiel 706

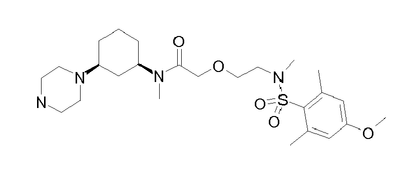

### Beispiel 707

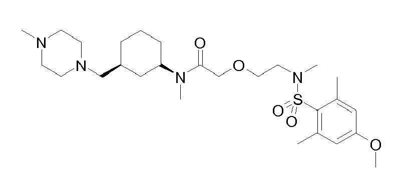

Die nachfolgenden Beispiele beschreiben pharmazeutische Darreichungsformen, die als Wirkstoff eine beliebige Verbindung der allgemeinen Formel I enthalten:

### Beispiel I

Trockenampulle mit 75 mg Wirkstoff pro 10 ml

Zusammensetzung:

| | |
|---|---|
| Wirkstoff | 75.0 mg |
| Mannitol | 500 mg |
| Wasser für Injektionszwecke ad | 10.0 ml |

Herstellung:
Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

### Tablette mit 50 mg Wirkstoff

Zusammensetzung:

| | | |
|---|---|---|
| (1) | Wirkstoff | 50.0 mg |
| (2) | Milchzucker | 98.0 mg |
| (3) | Maisstärke | 50.0 mg |
| (4) | Polyvinylpyrrolidon | 15.0 mg |
| (5) | Magnesiumstearat | 2.0 mg |
| | | 215,0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
   Durchmesser der Tabletten: 9 mm.

### Beispiel III

### Tablette mit 350 mg Wirkstoff

Zusammensetzung:

| | | |
|---|---|---|
| (1) | Wirkstoff | 350.0 mg |
| (2) | Milchzucker | 136.0 mg |
| (3) | Maisstärke | 80.0 mg |
| (4) | Polyvinylpyrrolidon | 30.0 mg |
| (5) | Magnesiumstearat | 4.0 mg |
| | | 600.0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
   Durchmesser der Tabletten: 12 mm.

### Beispiel IV

### Kapseln mit 50 mg Wirkstoff

Zusammensetzung:

| | | |
|---|---|---|
| (1) | Wirkstoff | 50.0 mg |
| (2) | Maisstärke getrocknet | 58.0 mg |
| (3) | Milchzucker pulverisiert | 50.0 mg |
| (4) | Magnesiumstearat | 2.0 mg |
| | | 160.0 mg |

Herstellung:
(1) wird mit (3) verrieben. Diese Verreibung wird der Mischung aus (2) und (4) unter intensiver Mischung zugegeben.
   Diese Pulvermischung wird auf einer Kapselabfüllmaschine in Hartgelatine-Steckkapseln Größe 3 abgefüllt.

### Beispiel V

### Kapseln mit 350 mg Wirkstoff

Zusammensetzung:

| | | |
|---|---|---|
| (1) | Wirkstoff | 350.0 mg |
| (2) | Maisstärke getrocknet | 46.0 mg |
| (3) | Milchzucker pulverisiert | 30.0 mg |
| (4) | Magnesiumstearat | 4.0 mg |
| | | 430.0 mg |

Herstellung:
(1) wird mit (3) verrieben. Diese Verreibung wird der Mischung aus (2) und (4) unter intensiver Mischung zugegeben.
   Diese Pulvermischung wird auf einer Kapselabfüllmaschine in Hartgelatine-Steckkapseln Gr6Be 0 abgefüllt.

### Beispiel VI

### Suppositorien mit 100 mg Wirkstoff

1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel I 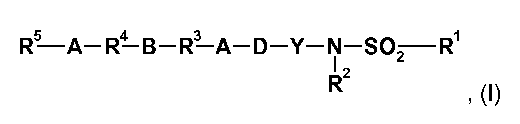 in der
**A** eine Bindung, C₁₋₄-Alkylen oder-CH₂-C(O)-,
**B** eine Bindung, C₁₋₃-Alkylen, -O- oder-C(O)-,
**D** eine Gruppe der allgemeinen Formeln **II** 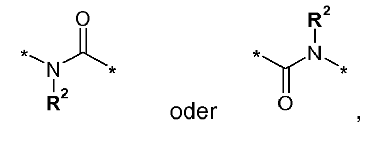
**Y** eine gegebenenfalls durch den Rest **R²** substituierte C₁₋₆-Alkylen-Gruppe, wobei eine Methylengruppe zusätzlich durch **Y¹** ersetzt sein kann und
**Y¹** -O-, -S-, -S(O)-, -N(**R²**)-, -N(**R²**)-C(O)-, -C(O)-N(**R²**)-, -C(O)-, -CH(Aryl)-, C₃₋₆-Cycloalkylen oder -S(O)₂- darstellt,
**R¹** C₃₋₇-Cycloalkyl- oder Aryl-, Heteroaryl- oder Aryl-C₁₋₃-alkyl-, die jeweils durch einen, zwei, drei oder vier Reste **R^{1.1}** substituiert sein können, wobei die Reste **R^{1.1}** gleich oder verschieden sein können und
**R^{1.1}** H, F, Cl, Br, I, C₁₋₃-Alkyl-, F₃C-, HO-, C₁₋₃-Alkyl-O- oder C₁₋₃-Alkyl-O-C₂₋₄-alkylen-O- darstellt,
**R²** H oder C₁₋₃-Alkyl-, wobei jede Methylengruppe mit bis zu zwei und jede Methylgruppe mit bis zu drei Fluoratomen substituiert sein kann, oder auch H₃C-C(O)-,
**R³** a) C₁₋₆-Alkylen,
b) eine durch **R^{3.1}** einfach, zweifach oder dreifach substituierte C₃₋₆-Cycloalkylengruppe,
c) eine durch **R^{3.1}** einfach oder zweifach substituierte C₅₋₇-Cycloalkenylengruppe, die über die ungesättigte Bindung mit einem Phenylring kondensiert ist,
d) -N(**R²**)-,
e) eine durch **R^{3.1}** einfach oder zweifach substituierte Arylengruppe,
f) eine durch **R^{3.1}** einfach oder zweifach substituierte Heteroarylengruppe,
g) einen durch **R^{3.1}** einfach oder zweifach substituierten gesättigten 4- bis 7-gliedrigen heterocyclischen Ring,
h) einen durch **R^{3.1}** einfach oder zweifach substituierten ungesättigten 5- bis 7-gliedrigen heterocyclischen Ring, der über die ungesättigten Bindungen mit einem oder zwei Phenylringen kondensiert ist, oder
i) einen durch **R^{3.1}** einfach oder zweifach substituierten gesättigten 8- bis 10-gliedrigen Aza-Heterobicyclus,
wobei die Reste **R^{3.1}** jeweils gleich oder verscheiden sein können und
**R^{3.1}** H, F, Cl, Br, I, C₁₋₃-Alkyl-, HO-, C₁₋₃-Alkyl-O- oder C₁₋₃-Alkyl-O-C₂₋₄-alkylen-O- darstellt, oder
**R³** auch -O-, wenn **B** nicht die Gruppe -O- darstellt,
**R⁴**
a) -O-,
b) -C(O)O-,
c) -C(O)N**R²**-,
d) -N**R²-,**
e) -N**R²**-N**R²**-,
f) C₃₋₇-Cycloalkylen,
g) C₁₋₆-Alkylen,
h) eine durch **R^{4.1}** einfach oder zweifach substituierte Arylengruppe,
i) eine durch **R^{4.1}** einfach oder zweifach substituierte Heteroarylengruppe,
j) einen durch **R^{4.1}** einfach oder zweifach substituierten 4- bis 7-gliedrigen gesättigten heterocyclischen Ring,
k) einen durch **R^{4.1}** einfach oder zweifach substituierten gesättigten 8- bis 10-gliedrigen Diaza-Heterobicyclus,
l) einen durch **R^{4.1}** einfach oder zweifach substituierten 5- bis 7-gliedrigen ungesättigten heterocyclischen Ring, der über die ungesättigten Bindungen mit einem oder zwei Phenylringen kondensiert ist, oder
m) einen gesättigten 9- bis 11-gliedrigen Diaza-Spirocyclus,
wobei die Reste **R^{4.1}** jeweils gleich oder verscheiden sein können und
**R^{4.1}** H, F, Cl, Br, I, C₁₋₃-Alkyl-, HO-, C₁₋₃-Alkyl-O- oder C₁₋₃-Alkyl-O-C₂₋₄-alkylen-O- darstellt,
**R⁵** H, HO-, C₁₋₈-Alkyl-, eine gegebenenfalls durch C₁₋₃-Alkyl- substituierte C₃₋₇-Cycloalkylgruppe, H₂N-, C₁₋₄-Alkyl-NH-, (C₃₋₆-Cycloalkyl)-NH-, (C₁₋₄-Alkyl)₂N-, (C₁₋₄-Alkyl)(C₃₋₆-Cycloalkyl)N-, (Cyclopropylmethyl)(methyl)N-, H₂N-C(O)-, eine durch **R^{5.1}** einfach oder zweifach substituierte Phenylgruppe, eine durch **R^{5.1}** einfach oder zweifach substituierte Heteroarylgruppe oder einen durch **R^{5.1}** einfach oder zweifach substituierten 4- bis 7-gliedrigen gesättigten heterocyclischen Ring, wobei die Reste **R^{5.1}** jeweils gleich oder verscheiden sein können und
**R^{5.1}** H, F, Cl, Br, I, C₁₋₃-Alkyl-, HO-, C₁₋₃-Alkyl-O-, (C₁₋₃-Alkyl)₂N- oder C₁₋₃-Alkyl-O-C₂₋₄-alkylen-O- darstellt,
bedeutet
deren Enantiomere, deren Diastereomere, deren Gemische und deren Salze, insbesondere deren physiologisch verträgliche Salze mit organischen oder anorganischen Säuren oder Basen.

2. Verbindungen der allgemeinen Formel **I** gemäß Anspruch 1, in denen
**A** eine Bindung, C₁₋₄-Alkylen oder-CH₂-C(O)-,
**B** eine Bindung, C₁₋₂Alkylen, -O- oder -C(O)-,
**D** eine Gruppe der allgemeinen Formel **II** 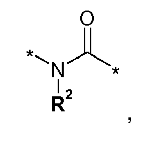
**Y** C₁₋₄-Alkylen oder eine Gruppe ausgewählt aus 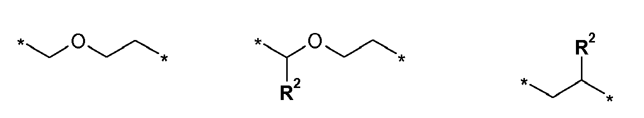 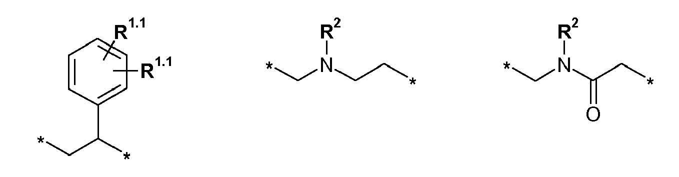 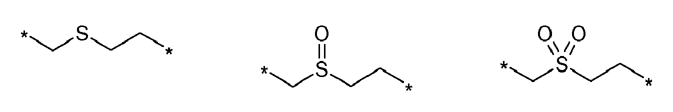 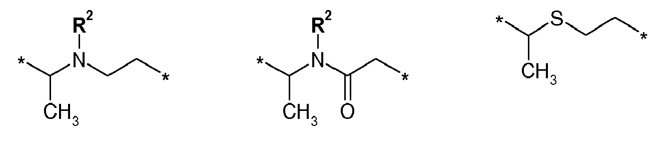 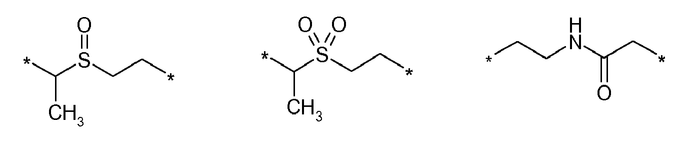 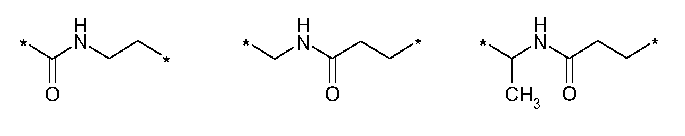 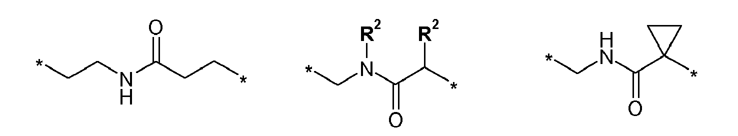 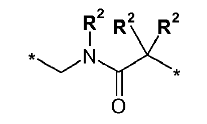
**R¹** Aryl- oder Heteroaryl-, die jeweils durch einen, zwei, drei oder vier Reste **R^{1.1}** substituiert sein können, wobei die Reste **R^{1.1}** gleich oder verschieden sein können und
**R^{1.1}** H, F, Cl, Br, I, C₁₋₃-Alkyl-, F₃C-, HO-, C₁₋₃-Alkyl-O- oder C₁₋₃-Alkyl-O-C₂₋₄-alkylen-O- darstellt,
**R²** H, H₃C-, H₅C₂-, Isopropyl, F₃C-CH₂-, F₂CH-CH₂- oder FH₂C-H₂C-,
**R³** C₁₋₄-Alkylen, -N(**R**²)- oder eine Gruppe ausgewählt aus 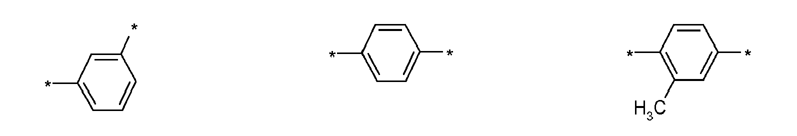 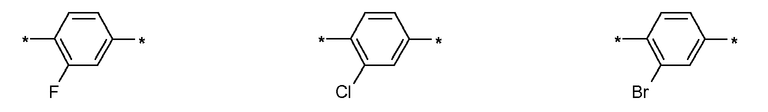 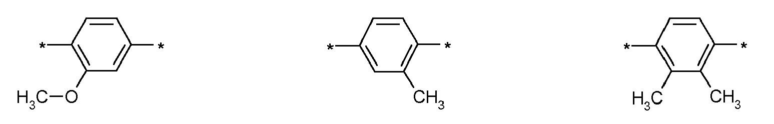 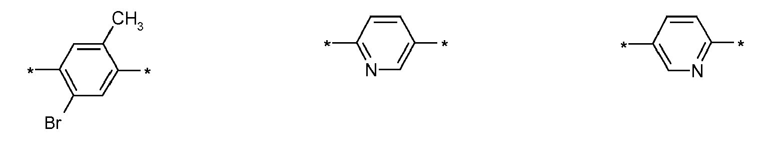 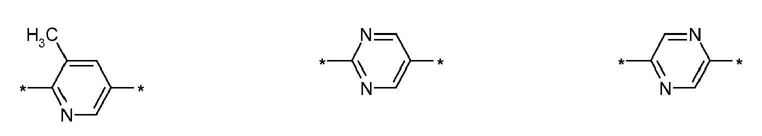 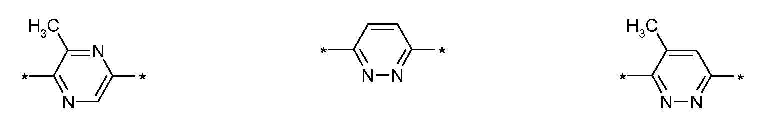 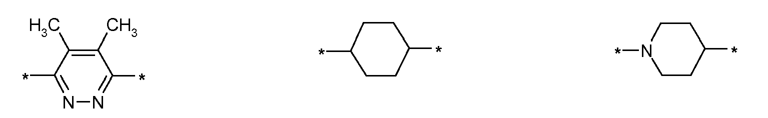 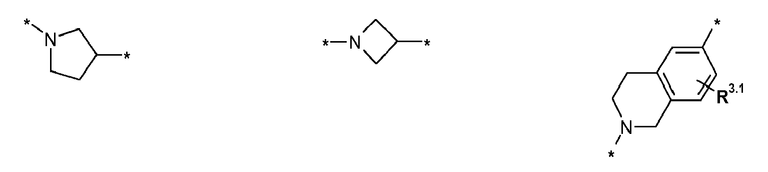 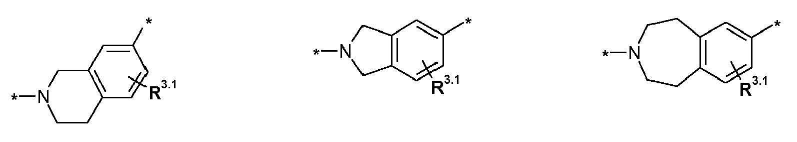 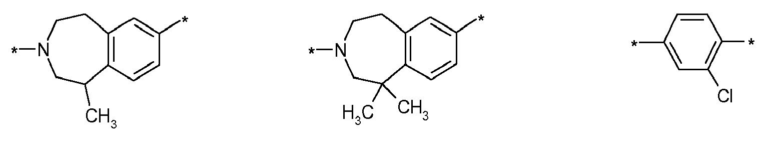 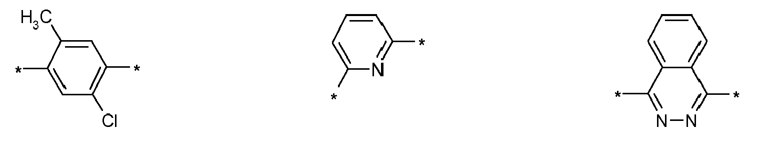 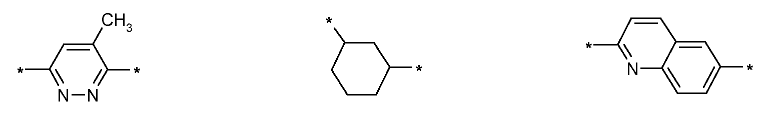 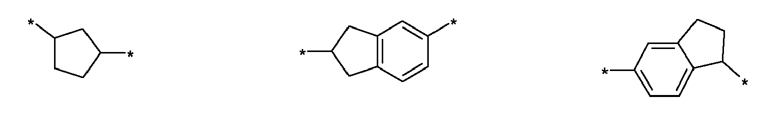 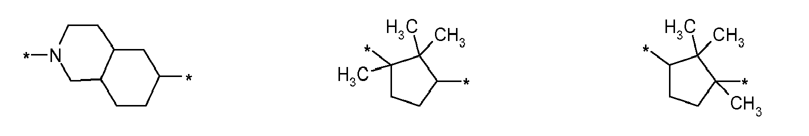 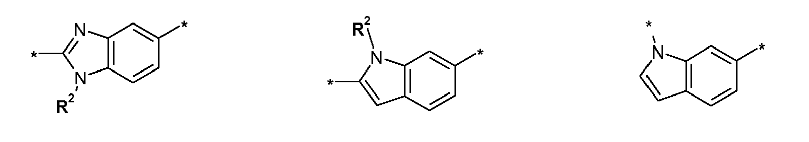 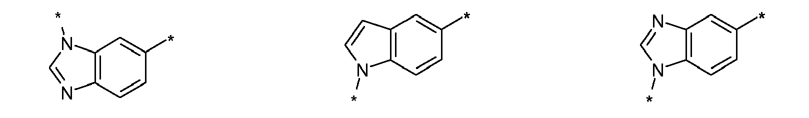 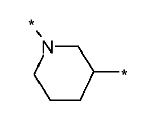 worin
**R^{3.1}** H, F, Cl, Br, I, C₁₋₃-Alkyl-, HO-, C₁₋₃-Alkyl-O- oder C₁₋₃-Alkyl-O-C₂₋₄-alkylen-O- darstellt, oder
**R³** auch -O-, wenn B nicht die Gruppe -O- darstellt,
**R⁴** C₁₋₄-Alkylen, C₃₋₇-Cycloalkylen, -O- oder eine Gruppe ausgewählt aus 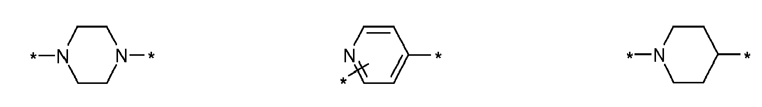 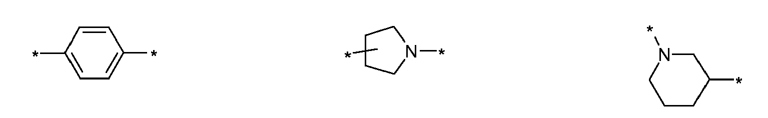 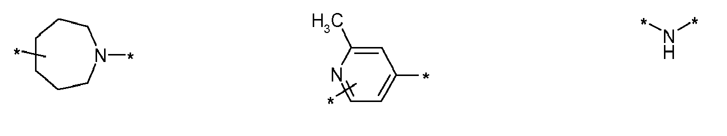 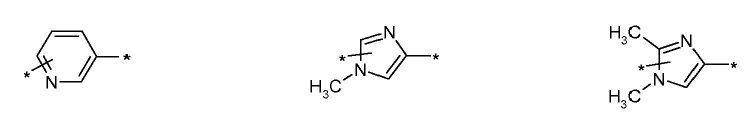 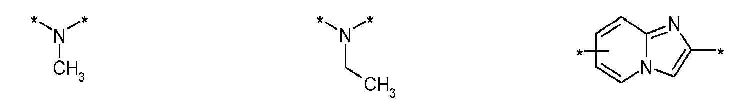 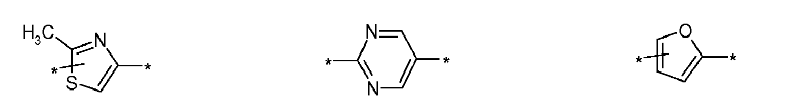 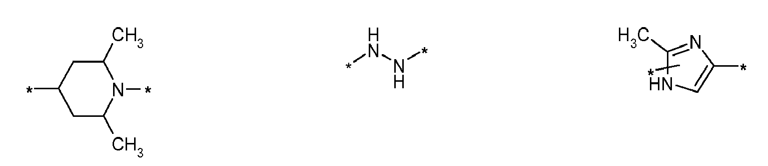 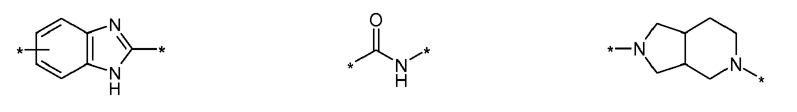 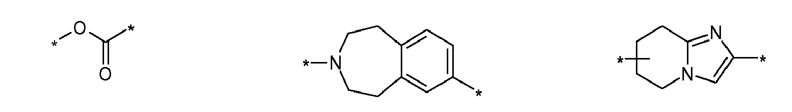 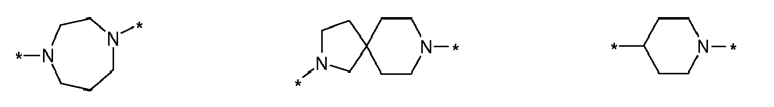 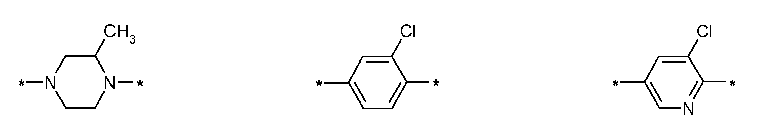 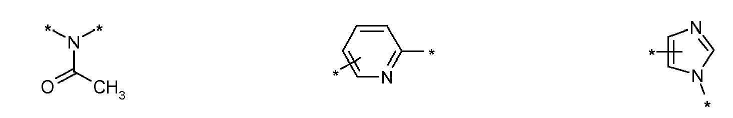 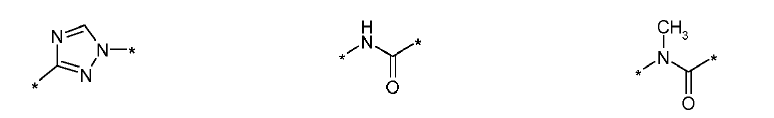 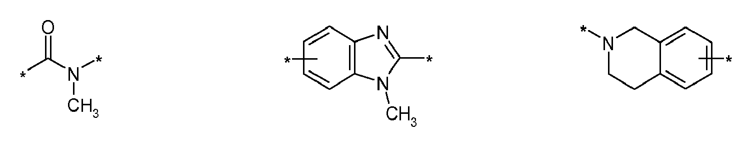
**R⁵** H, C₁₋₈-Alkyl, C₃₋₇-Cycloalkyl-, HO-, (C₁₋₃-Alkyl)-O-, (C₁₋₄-Alkyl)-NH-, (C₃₋₆-Cycloalkyl)-NH-, (C₁₋₄-Alkyl)₂N-, (C₁₋₄-Alkyl)(C₃₋₆-Cycloalkyl)N-, (Cyclopropylmethyl)(methyl)N-, H₂N-C(O)-, oder
**R⁵** eine Gruppe ausgewählt aus 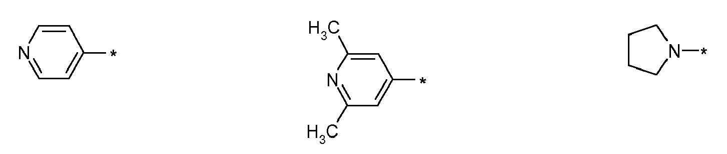 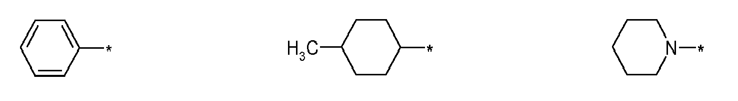 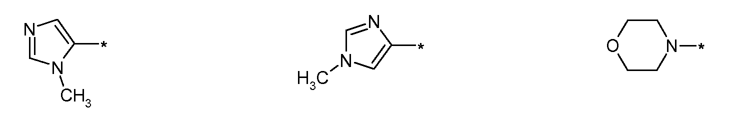 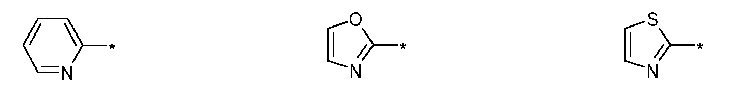 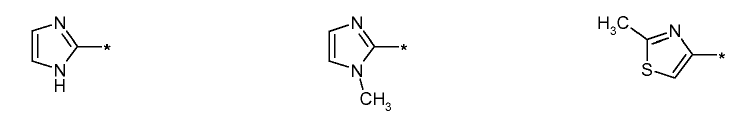 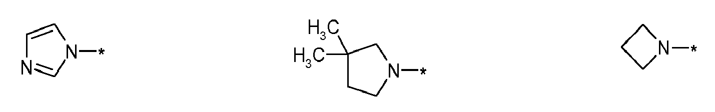 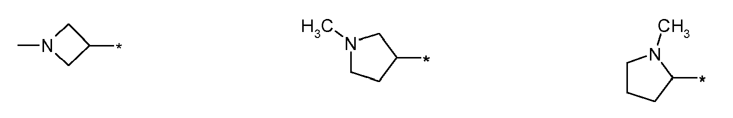 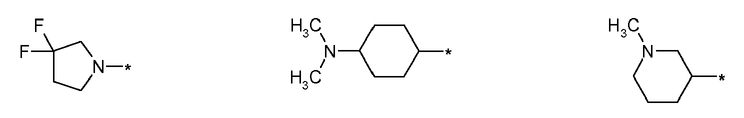 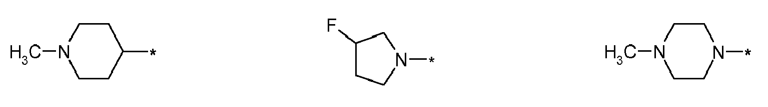
bedeutet,
deren Enantiomere, deren Diastereomere, deren Gemische und deren Salze, insbesondere deren physiologisch verträgliche Salze mit organischen oder anorganischen Säuren oder Basen.

3. Verbindungen der allgemeinen Formel **I** gemäß Anspruch 1, in denen
**A** eine Bindung, C₁₋₃-Alkylen oder-CH₂-C(O)-,
**B** eine Bindung, C₁₋₂-Alkylen, -O- oder -C(O)-,
**D** eine Gruppe der allgemeinen Formel **II** 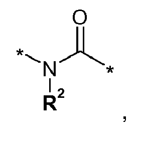
**Y** eine Gruppe ausgewählt aus 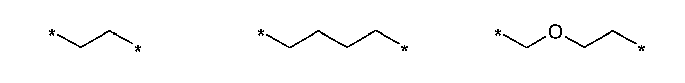 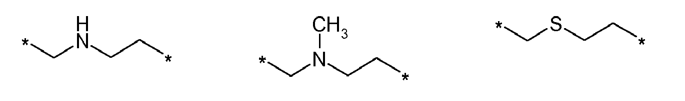 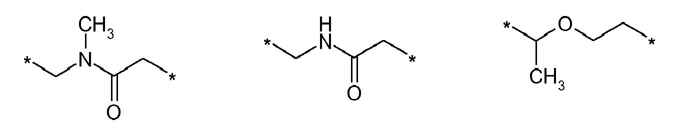 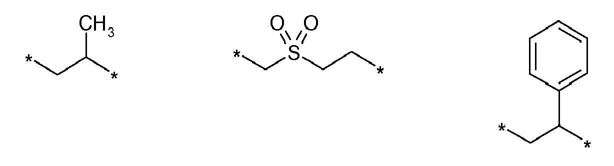 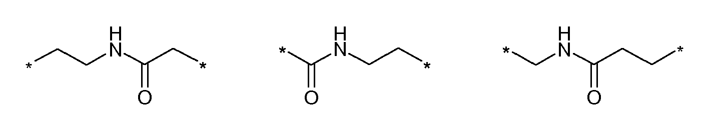 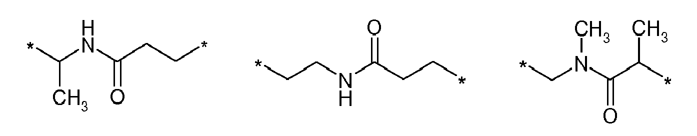 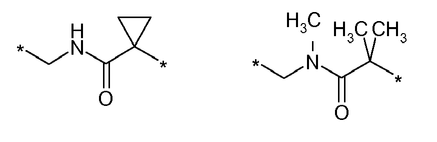
**R¹** eine Gruppe ausgewählt aus 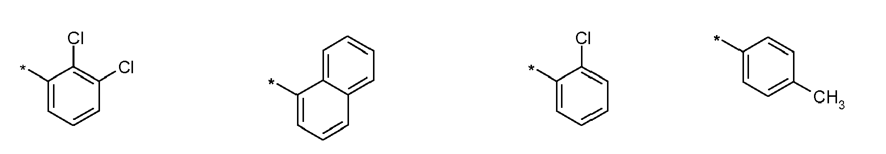 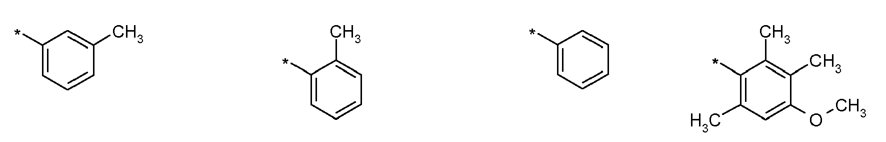 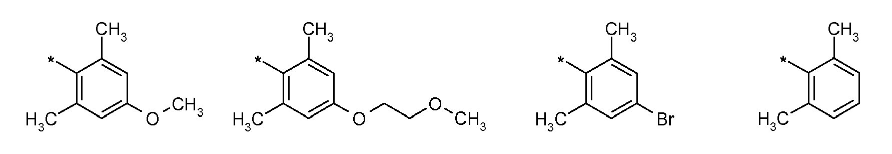 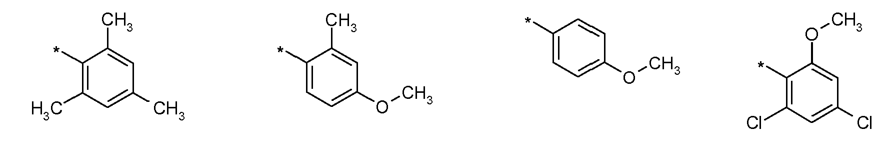 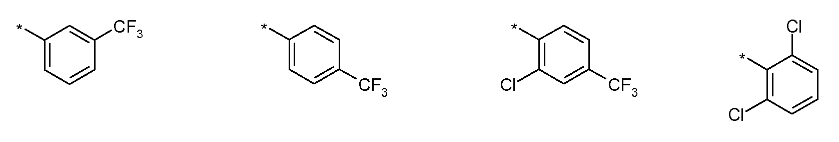
**R²** H, H₃C-, H₅C₂- oder FH₂C-H₂C-,
**R³** C₁₋₄-Alkylen, -NH-, -N(CH₃)- oder eine Gruppe ausgewählt aus 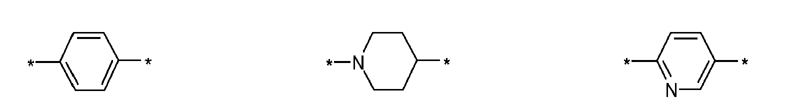 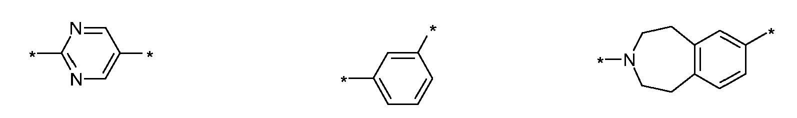 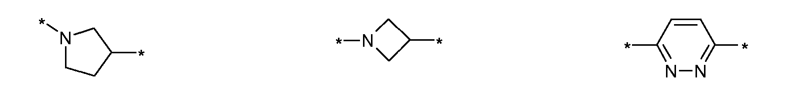 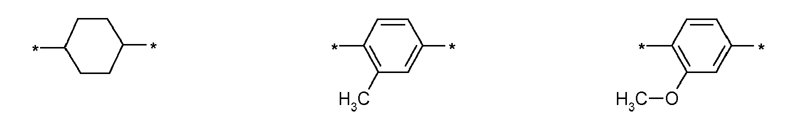 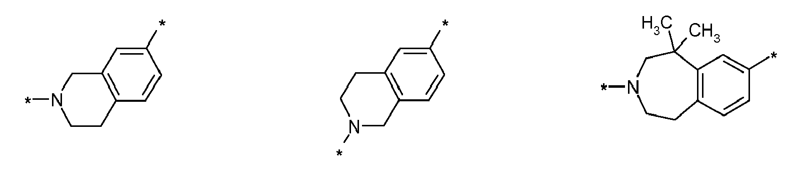 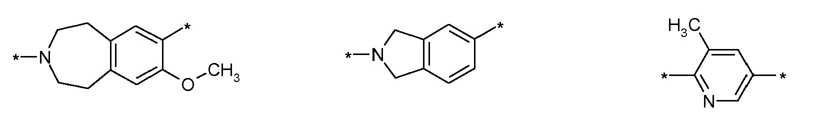 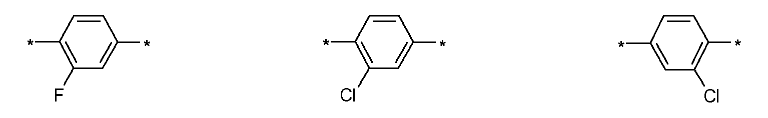 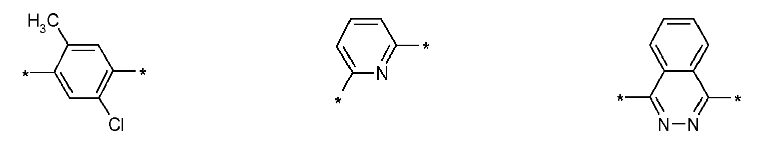 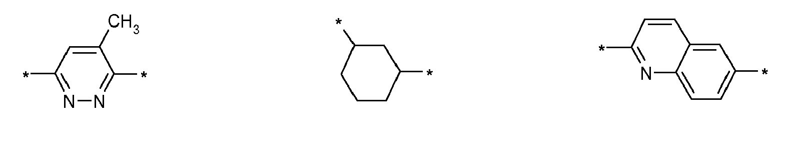 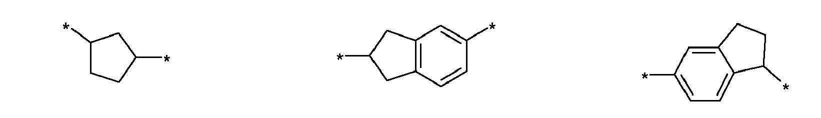 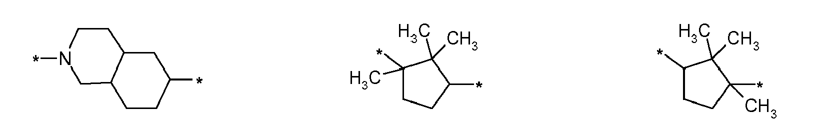 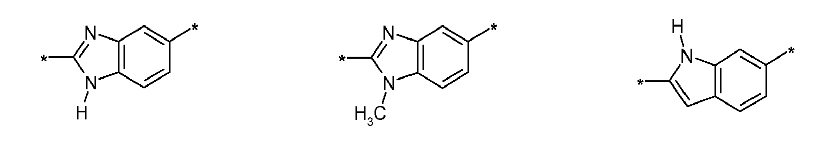 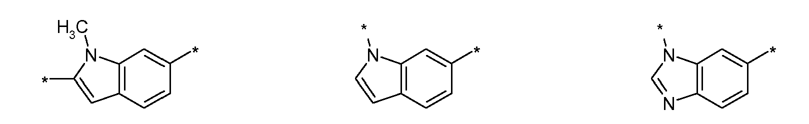 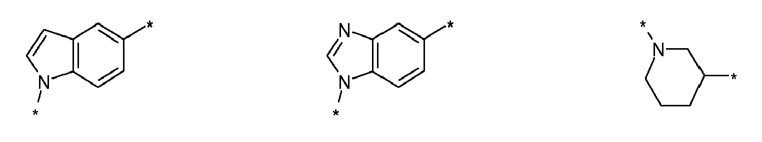 oder
**R³** auch -O-, wenn B nicht die Gruppe -O- darstellt,
**R⁴** C₁₋₄-Alkylen, C₃₋₇-Cycloalkylen, -O- oder eine Gruppe ausgewählt aus 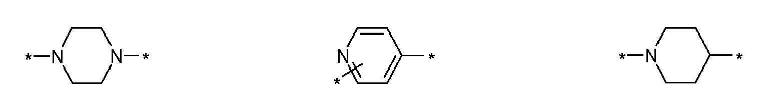 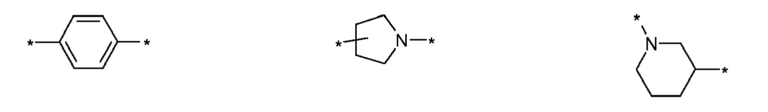 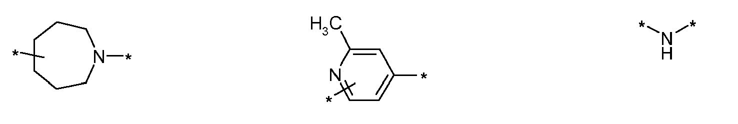 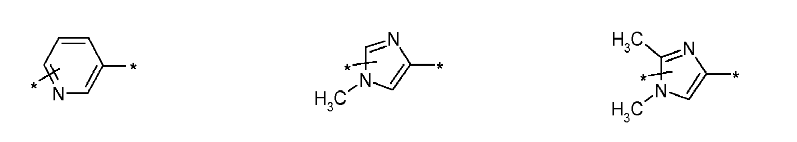 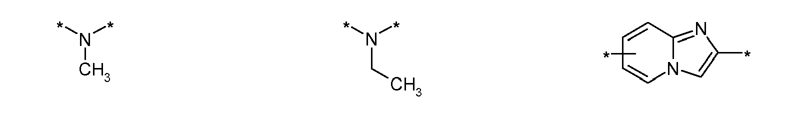 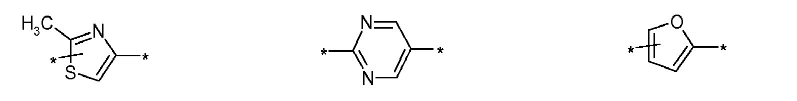 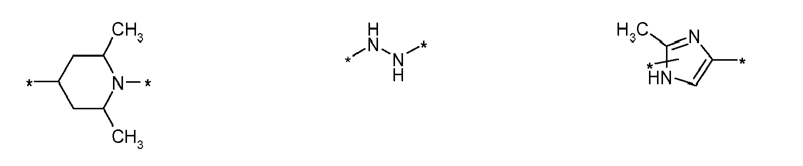 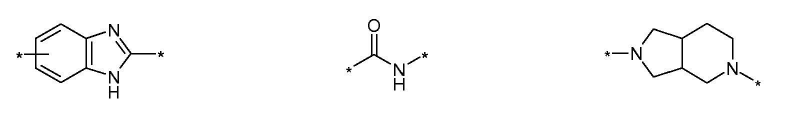 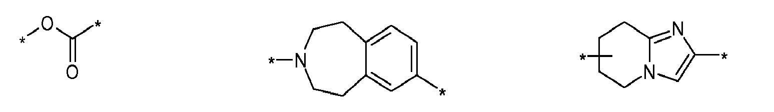 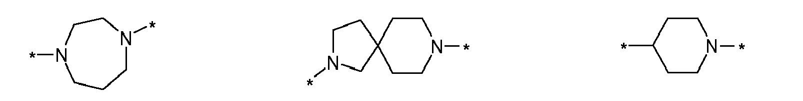 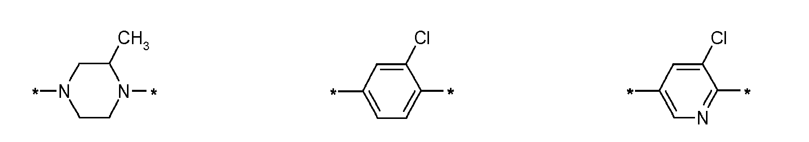 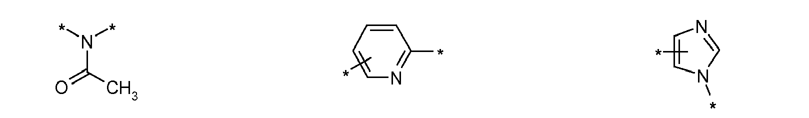 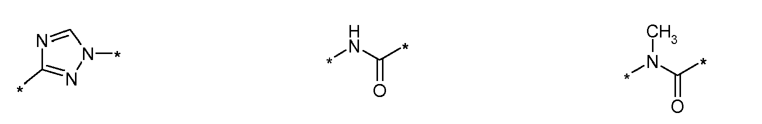 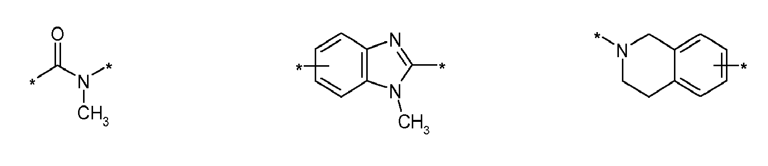
**R⁵** H, HO-, C₁₋₅-Alkyl, C₃₋₅-Cycloalkyl-, H₂N-, (C₁₋₂-Alkyl)-NH-, (C₃₋₆-Cycloalkyl)-NH-, (C₁₋₂-Alkyl)₂N-, (C₁₋₄-Alkyl)(C₃₋₆-Cycloalkyl)N-, (Cyclopropylmethyl)(methyl)N-, H₂N-C(O)-, oder
**R⁵** eine Gruppe ausgewählt aus 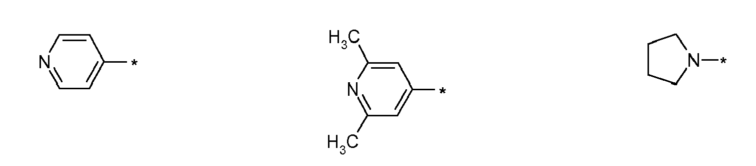 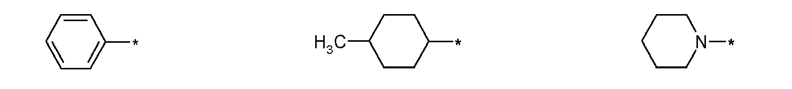 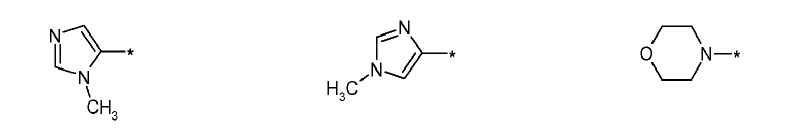 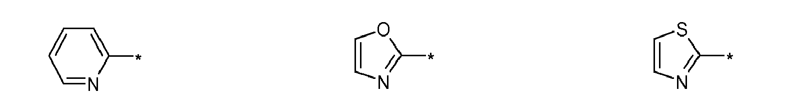 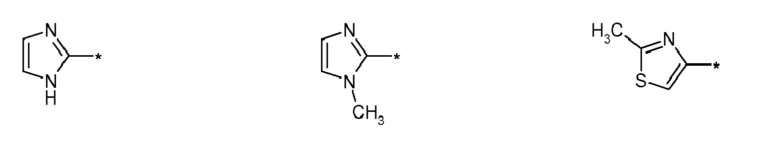 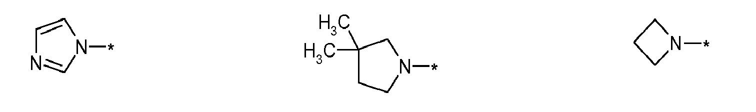 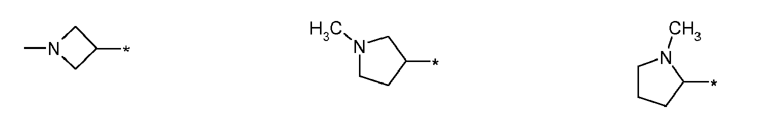 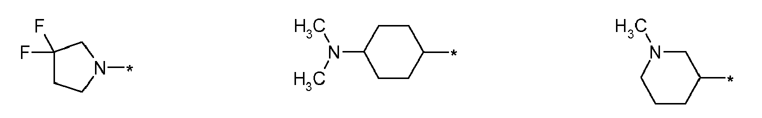 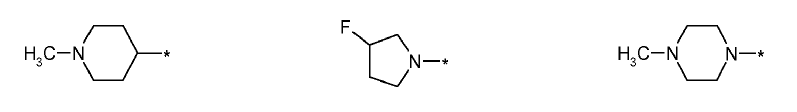
bedeutet,
deren Enantiomere, deren Diastereomere, deren Gemische und deren Salze, insbesondere deren physiologisch verträgliche Salze mit organischen oder anorganischen Säuren oder Basen.

4. Verbindungen der allgemeinen Formel **I** gemäß Anspruch 1, in denen
**A, B, D, Y, R², R³, R⁴** und **R⁵** wie in Anspruch 1 definiert sind und
**R¹** die Gruppe 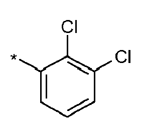 bedeutet,
deren Enantiomere, deren Diastereomere, deren Gemische und deren Salze, insbesondere deren physiologisch verträgliche Salze mit organischen oder anorganischen Säuren oder Basen.

5. Verbindungen der allgemeinen Formel **I** gemäß Anspruch 1, in denen
**A, B, D, Y, R², R³, R⁴** und **R⁵** wie in Anspruch 1 definiert sind und
**R¹** die Gruppe 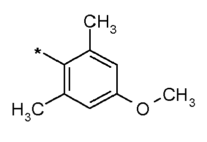 bedeutet,
deren Enantiomere, deren Diastereomere, deren Gemische und deren Salze, insbesondere deren physiologisch verträgliche Salze mit organischen oder anorganischen Säuren oder Basen.

6. Verbindungen der allgemeinen Formel **I** gemäß Anspruch 1, in denen
**A, B, R¹, R³, R⁴** und **R⁵** wie in Anspruch 1 definiert sind und
**-D-Y-** zusammen eine Gruppe ausgewählt aus 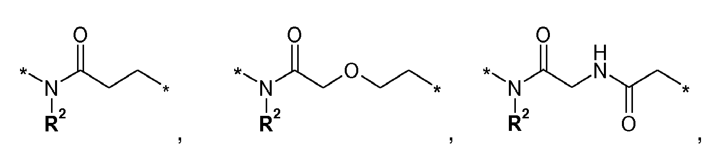 und
**R²** H oder C₁₋₃-Alkyl-,
bedeutet,
deren Enantiomere, deren Diastereomere, deren Gemische und deren Salze, insbesondere deren physiologisch verträgliche Salze mit organischen oder anorganischen Säuren oder Basen.

7. Folgende Verbindungen der allgemeinen Formel **I** gemäß Anspruch 1:
| **Beispiel** | **Struktur** |
|---|---|
| (1) | 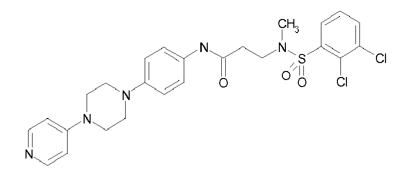 |
| (2) | 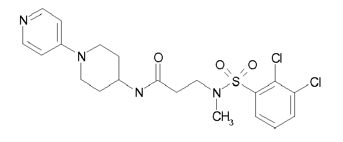 |
| (3) | 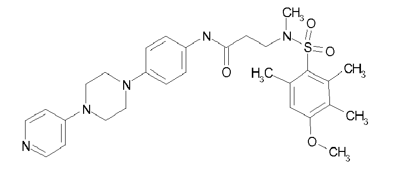 |
| (4) | 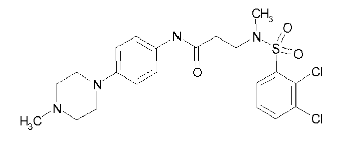 |
| (5) | 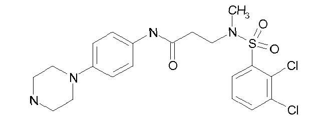 |
| (6) | 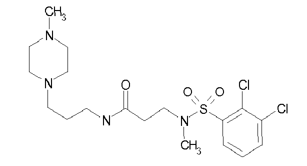 |
| (7) | 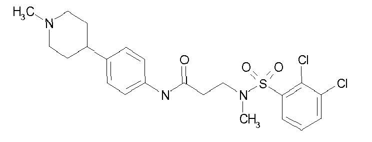 |
| (8) | 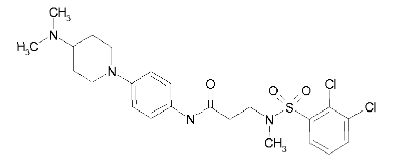 |
| (9) | 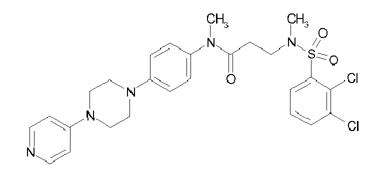 |
| (10) | 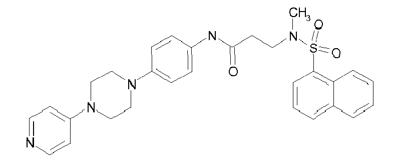 |
| (11) | 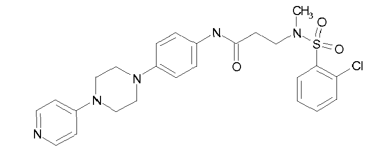 |
| (12) | 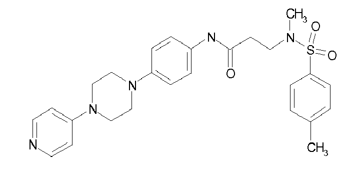 |
| (13) | 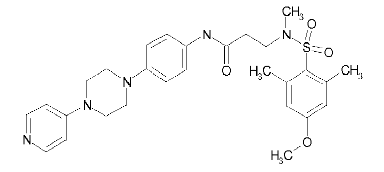 |
| (14) | 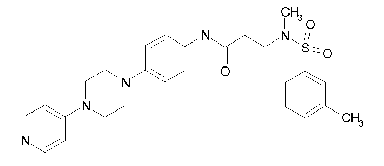 |
| (15) | 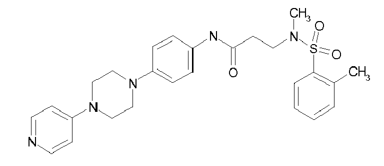 |
| (16) | 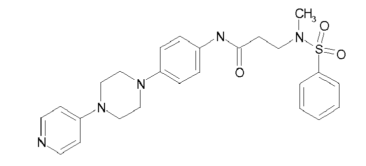 |
| (17) | 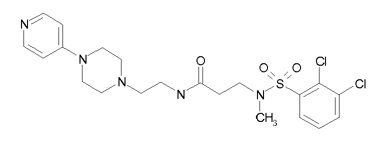 |
| (18) | 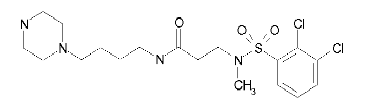 |
| (19) | 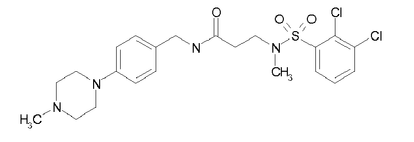 |
| (20) | 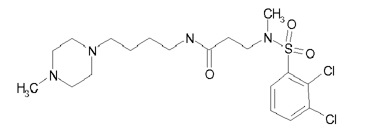 |
| (21) | 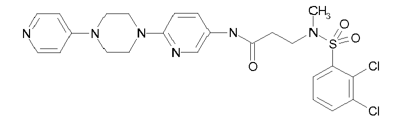 |
| (22) | 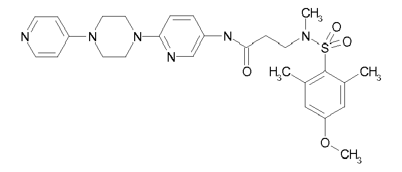 |
| (23) | 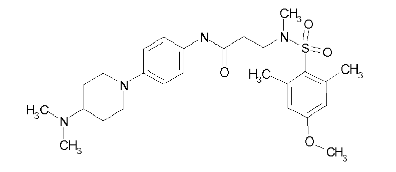 |
| (24) | 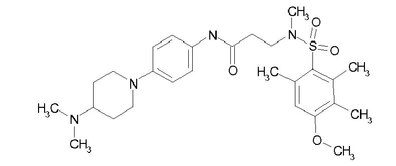 |
| (25) | 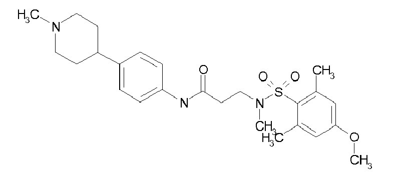 |
| (26) | 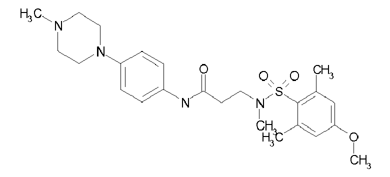 |
| (27) | 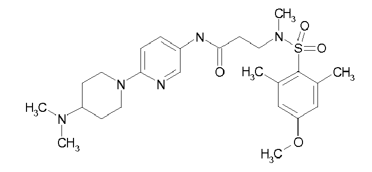 |
| (28) | 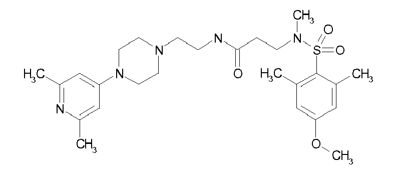 |
| (29) | 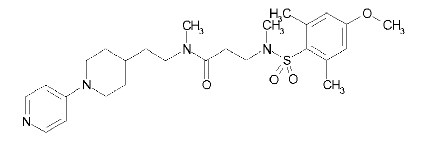 |
| (30) | 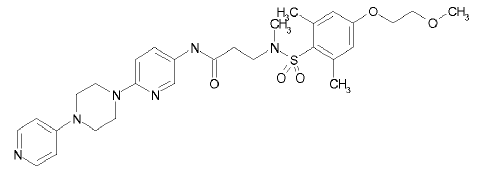 |
| (31) | 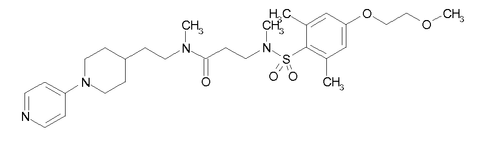 |
| (32) | 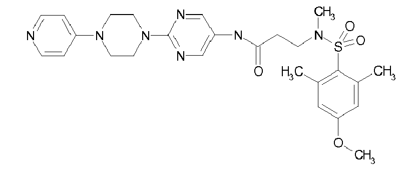 |
| (33) | 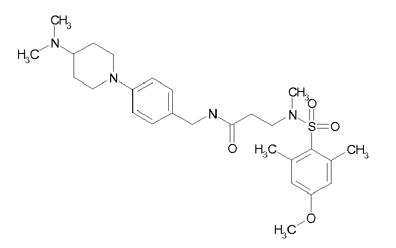 |
| (34) | 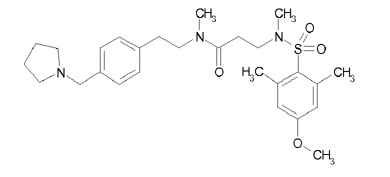 |
| (35) | 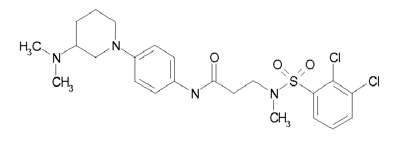 |
| (36) | 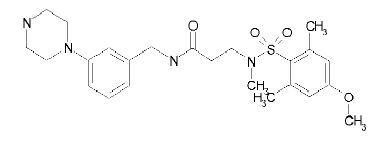 |
| (37) | 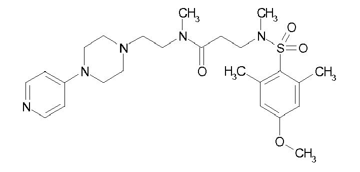 |
| (38) | 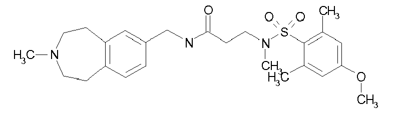 |
| (39) | 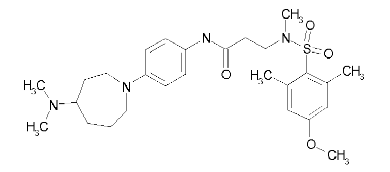 |
| (40) | 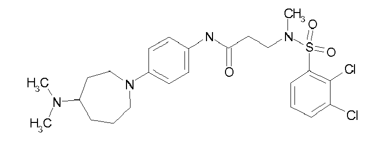 |
| (41) | 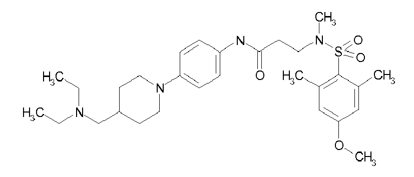 |
| (42) | 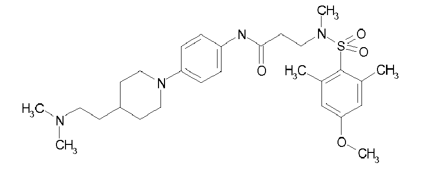 |
| (43) | 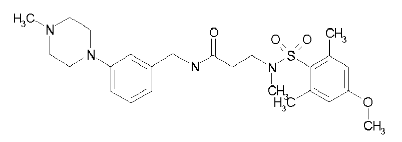 |
| (44) | 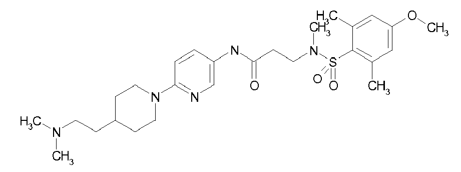 |
| (45) | 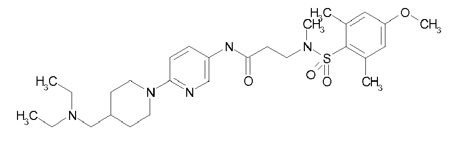 |
| (46) | 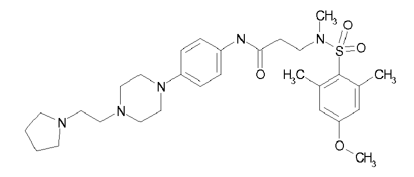 |
| (47) | 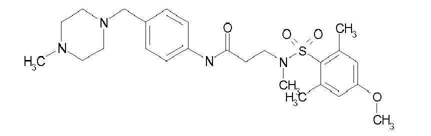 |
| (48) | 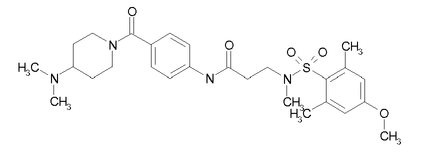 |
| (49) | 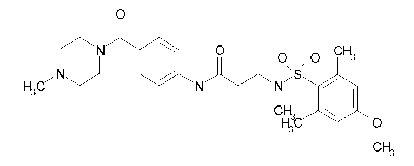 |
| (50) | 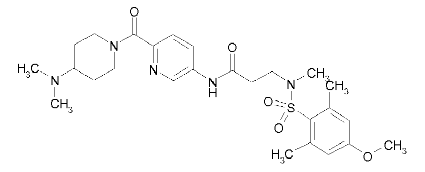 |
| (51) | 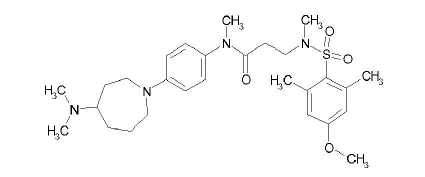 |
| (52) | 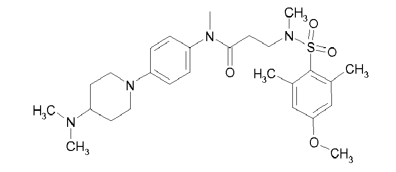 |
| (53) | 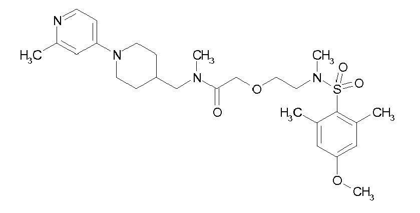 |
| (54) | 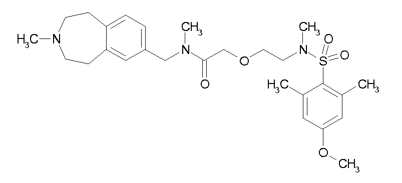 |
| (55) | 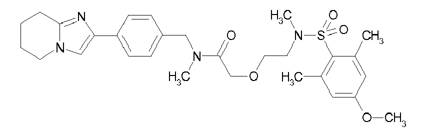 |
| (56) | 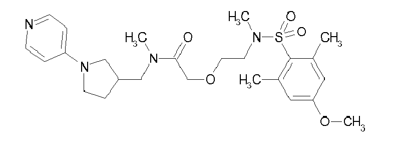 |
| (57) | 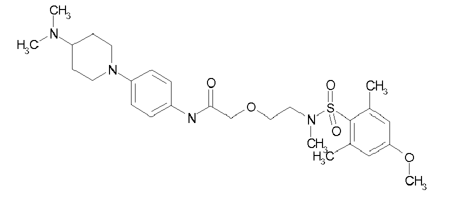 |
| (58) | 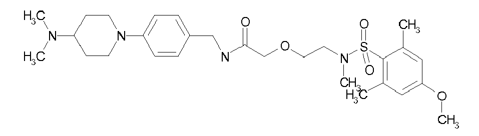 |
| (59) | 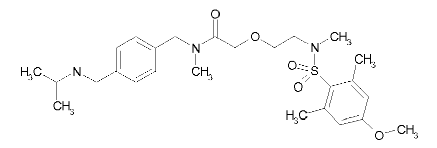 |
| (60) | 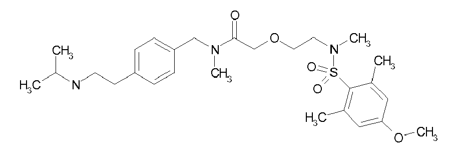 |
| (61) | 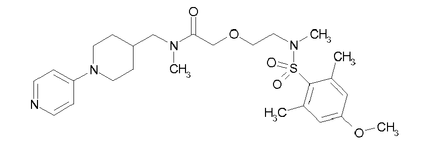 |
| (62) | 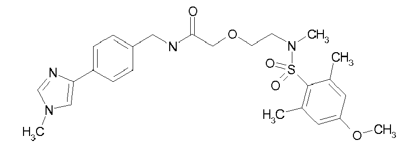 |
| (63) | 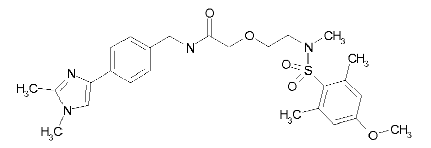 |
| (64) | 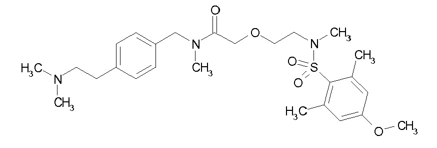 |
| (65) | 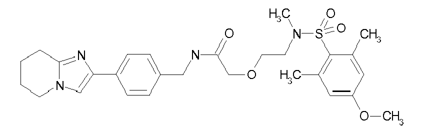 |
| (66) | 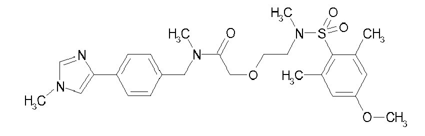 |
| (67) | 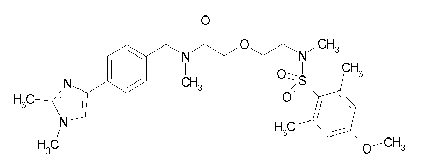 |
| (68) | 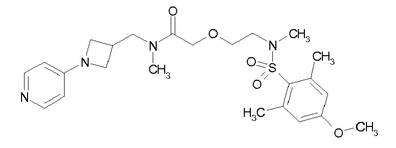 |
| (69) | 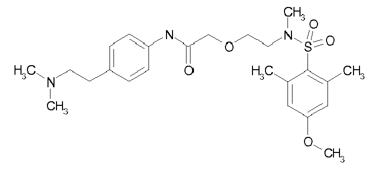 |
| (70) | 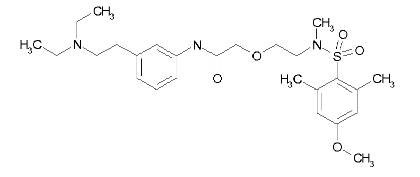 |
| (71) | 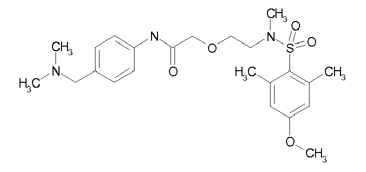 |
| (72) | 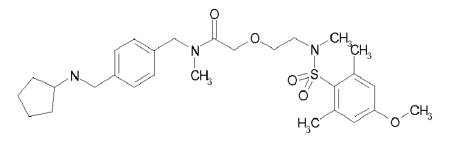 |
| (73) | 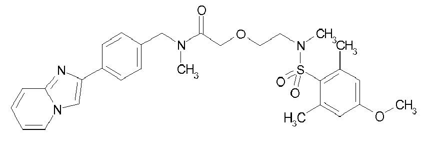 |
| (74) | 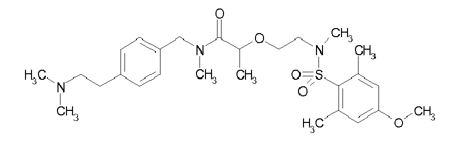 |
| (75) | 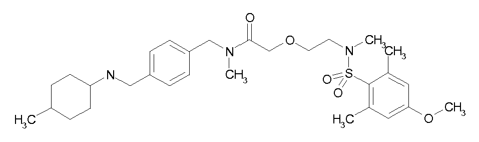 |
| (76) | 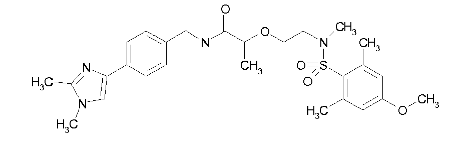 |
| (77) | 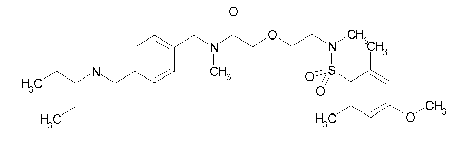 |
| (78) | 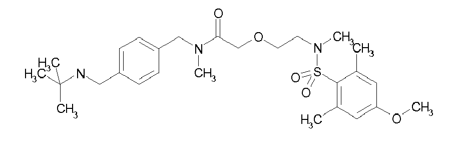 |
| (79) | 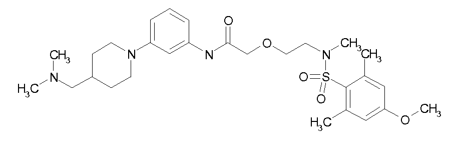 |
| (80) | 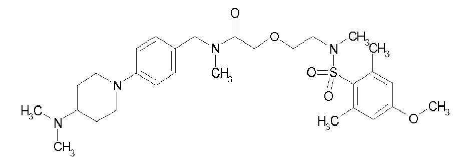 |
| (81) | 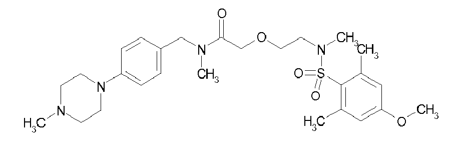 |
| (82) | 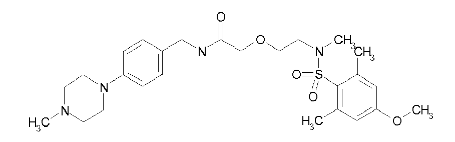 |
| (83) | 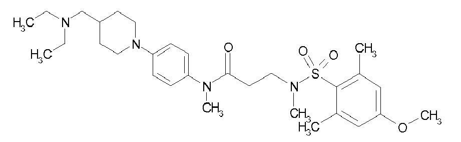 |
| (84) | 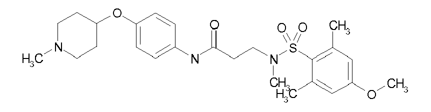 |
| (85) | 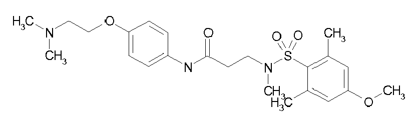 |
| (86) | 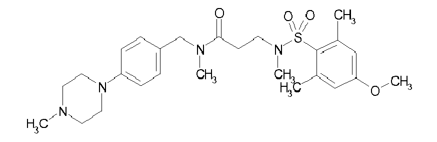 |
| (87) | 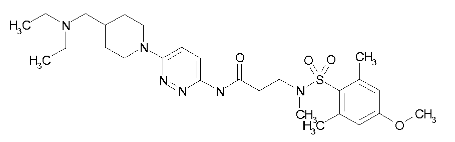 |
| (88) | 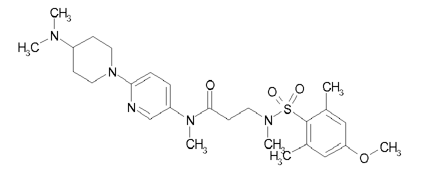 |
| (89) | 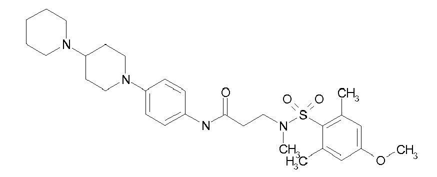 |
| (90) | 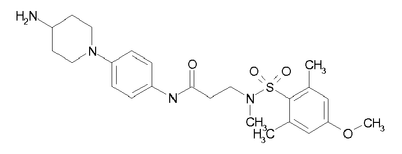 |
| (91) | 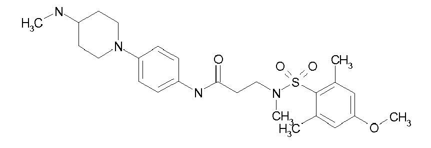 |
| (92) | 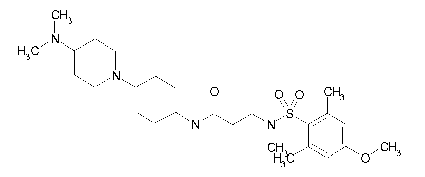 |
| (93) | 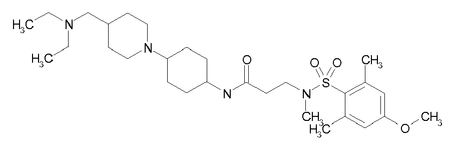 |
| (94) | 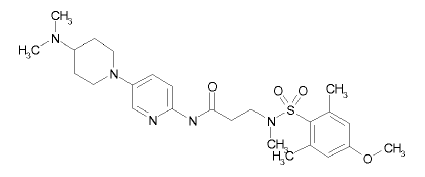 |
| (95) | 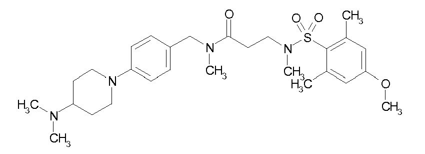 |
| (96) | 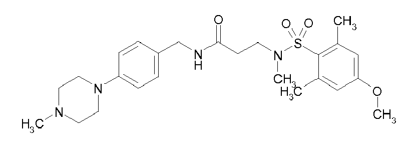 |
| (97) | 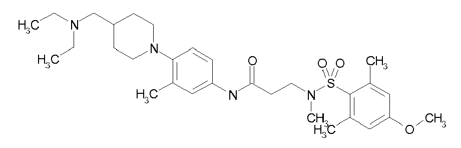 |
| (98) | 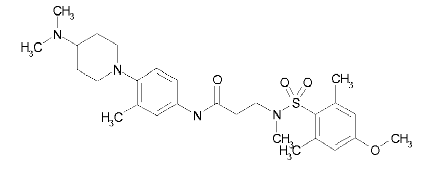 |
| (99) | 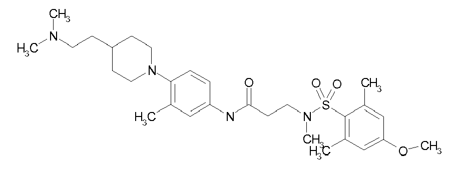 |
| (100) | 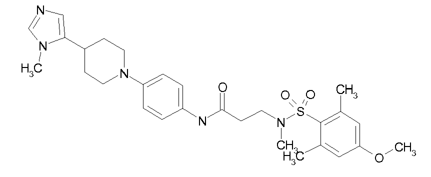 |
| (101) | 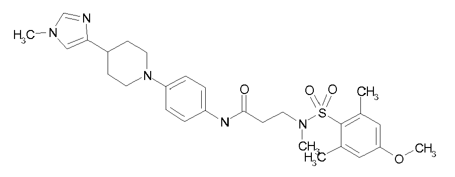 |
| (102) | 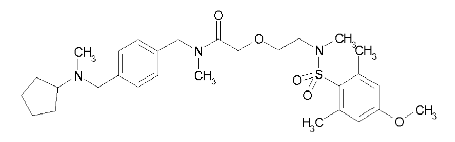 |
| (103) | 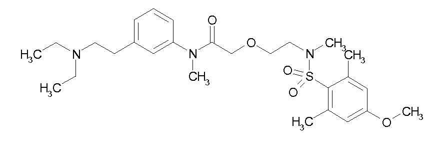 |
| (104) | 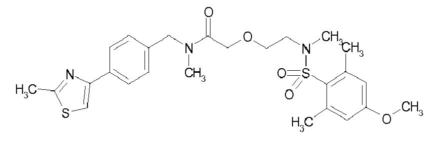 |
| (105) | 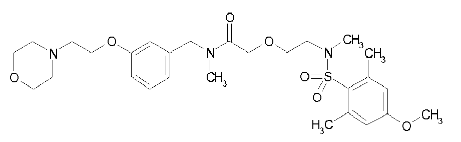 |
| (106) | 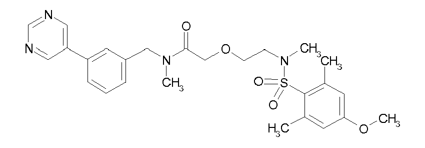 |
| (107) | 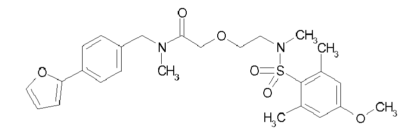 |
| (108) | 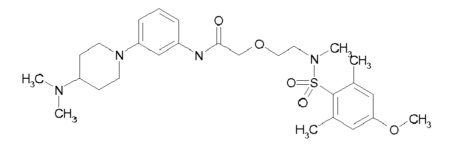 |
| (109) | 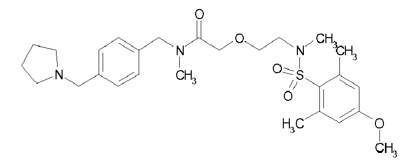 |
| (110) | 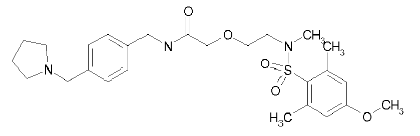 |
| (111) | 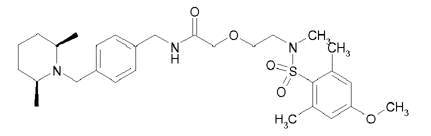 |
| (112) | 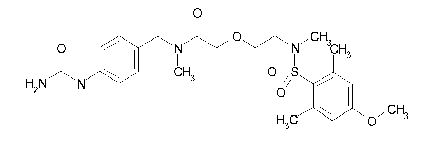 |
| (113) | 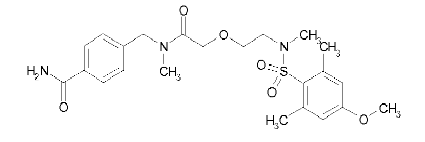 |
| (114) | 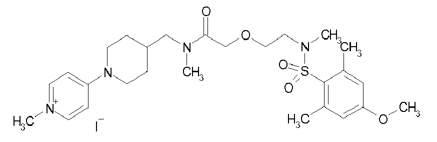 |
| (115) | 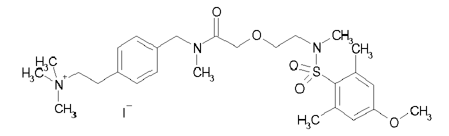 |
| (116) | 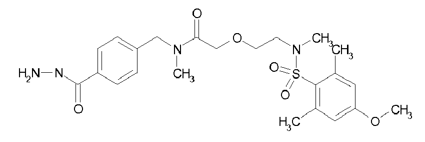 |
| (117) | 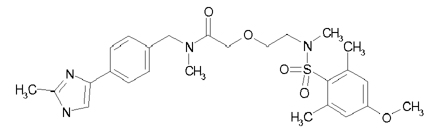 |
| (118) | 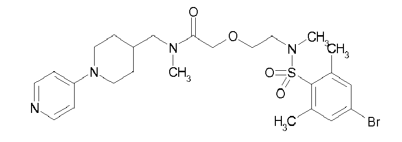 |
| (119) | 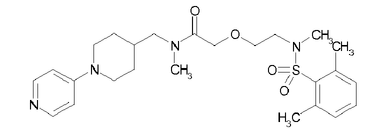 |
| (120) | 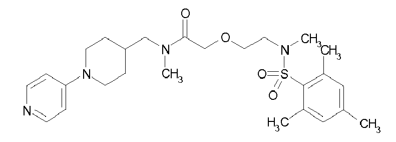 |
| (121) | 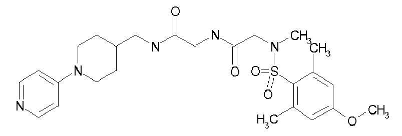 |
| (122) | 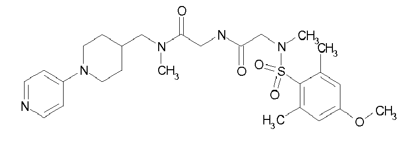 |
| (123) | 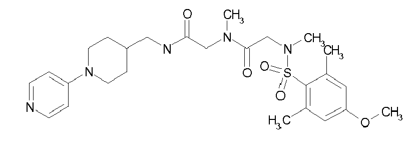 |
| (124) | 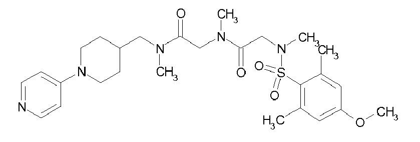 |
| (125) | 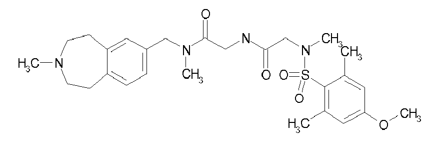 |
| (126) | 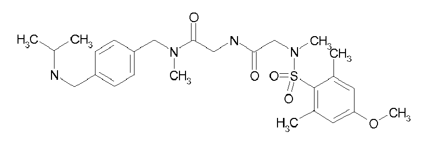 |
| (127) | 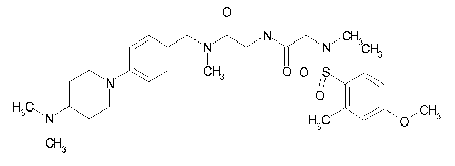 |
| (128) | 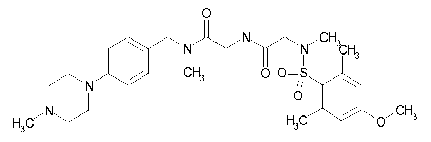 |
| (129) | 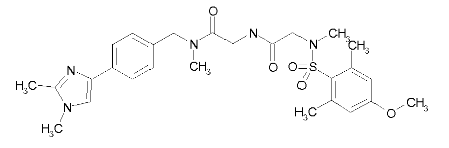 |
| (130) | 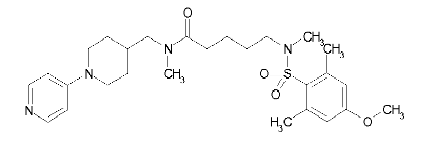 |
| (131) | 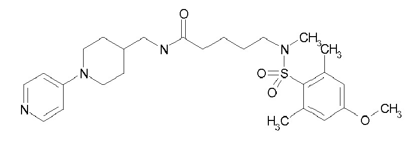 |
| (132) | 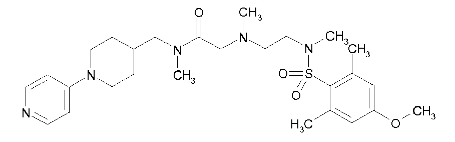 |
| (133) | 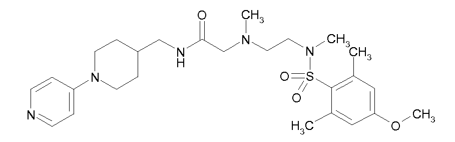 |
| (134) | 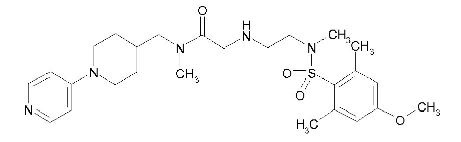 |
| (135) | 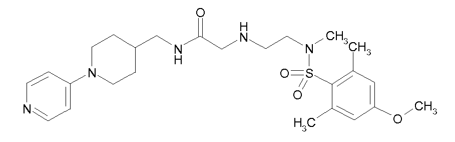 |
| (136) | 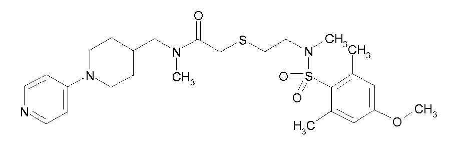 |
| (137) | 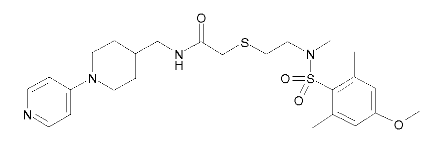 |
| (138) | 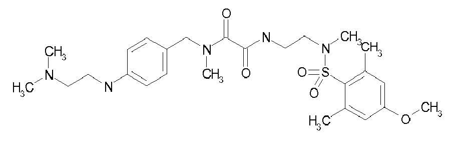 |
| (139) | 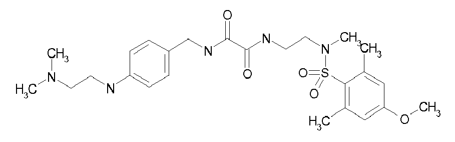 |
| (140) | 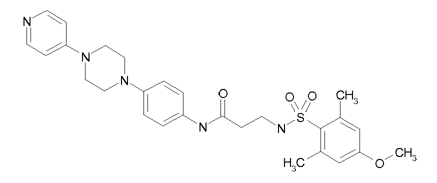 |
| (141) | 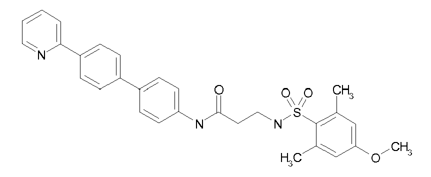 |
| (142) | 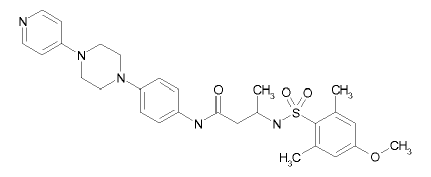 |
| (143) | 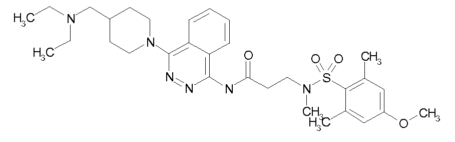 |
| (144) | 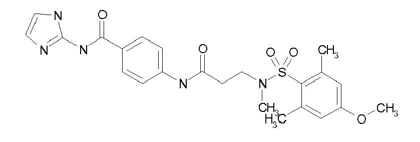 |
| (145) | 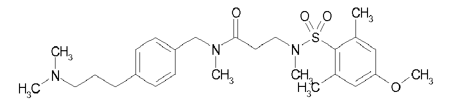 |
| (146) | 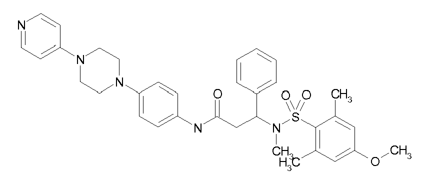 |
| (147) | 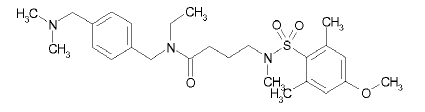 |
| (148) | 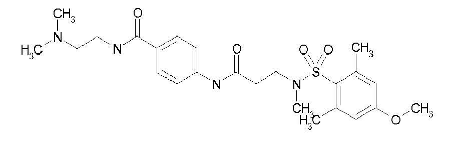 |
| (149) | 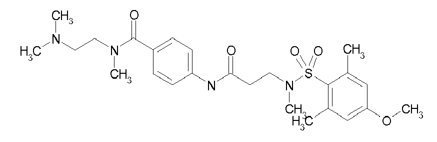 |
| (150) | 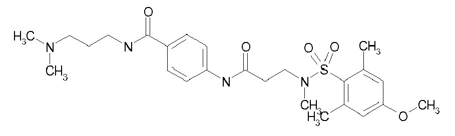 |
| (151) | 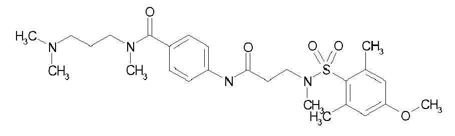 |
| (152) | 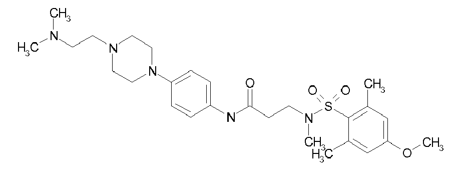 |
| (153) | 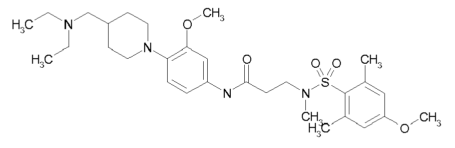 |
| (154) | 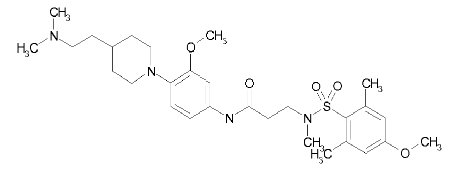 |
| (155) | 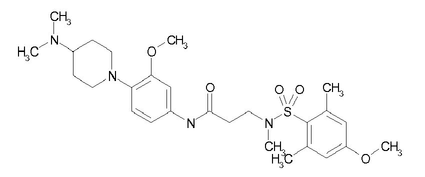 |
| (156) | 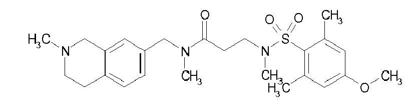 |
| (157) | 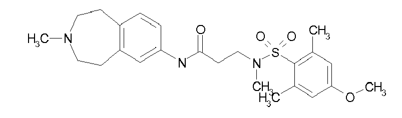 |
| (158) | 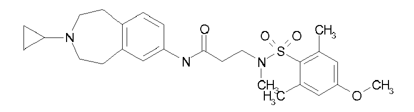 |
| (159) | 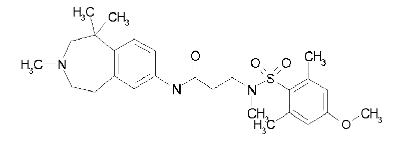 |
| (160) | 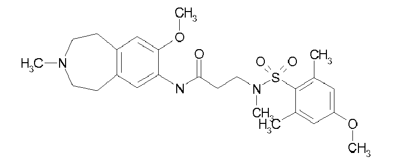 |
| (161) | 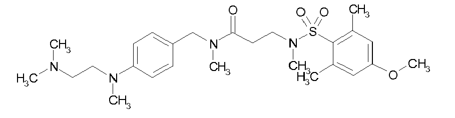 |
| (162) | 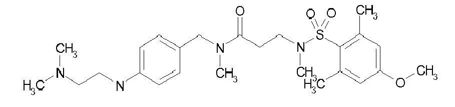 |
| (163) | 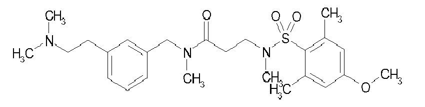 |
| (164) | 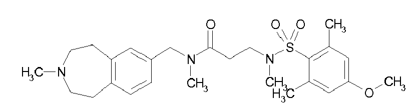 |
| (165) | 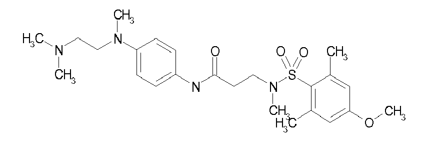 |
| (166) | 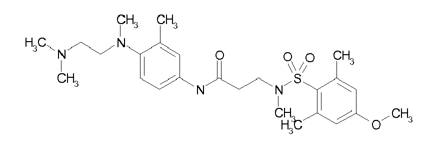 |
| (167) | 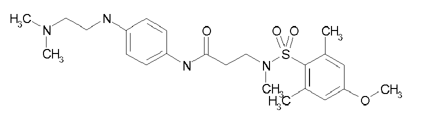 |
| (168) | 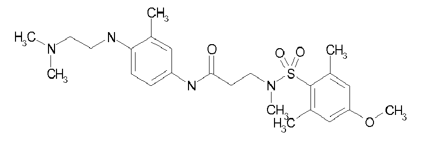 |
| (169) | 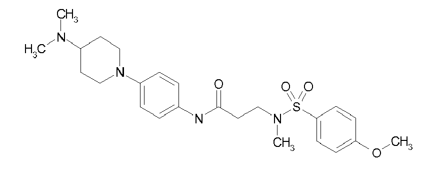 |
| (170) | 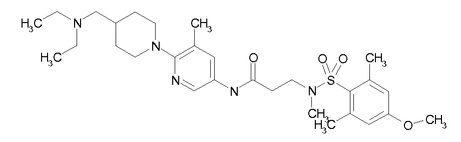 |
| (171) | 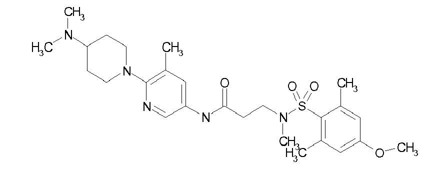 |
| (172) | 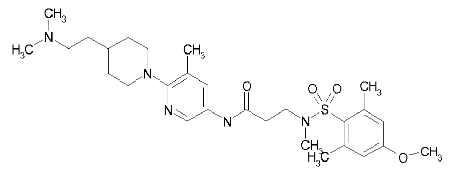 |
| (173) | 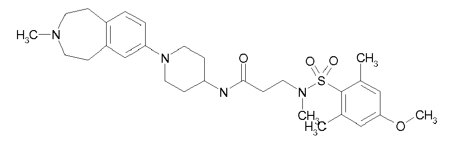 |
| (174) | 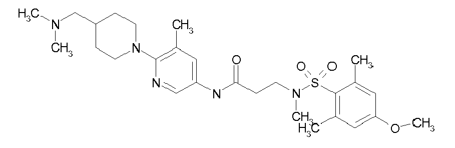 |
| (175) | 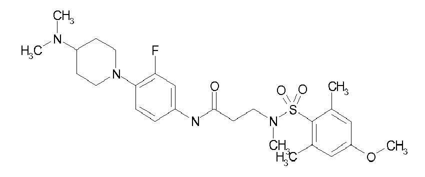 |
| (176) | 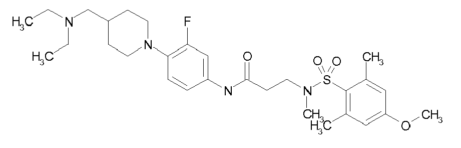 |
| (177) | 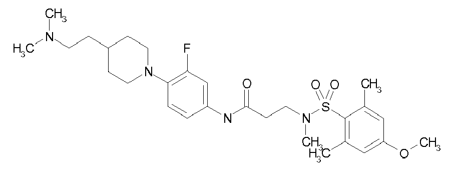 |
| (178) | 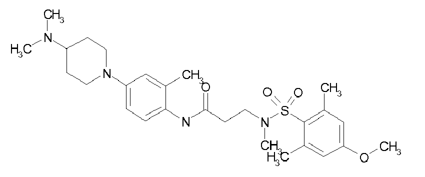 |
| (179) | 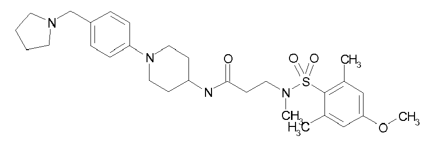 |
| (180) | 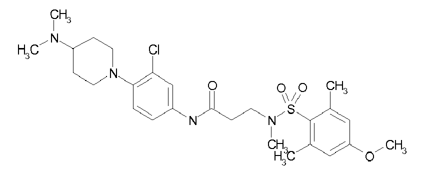 |
| (181) | 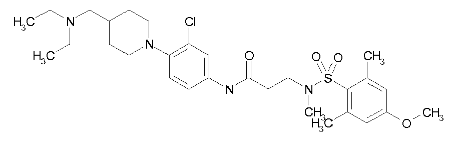 |
| (182) | 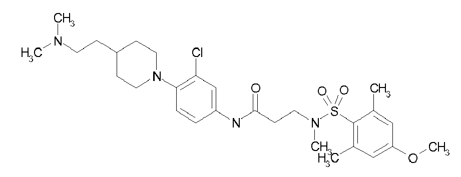 |
| (183) | 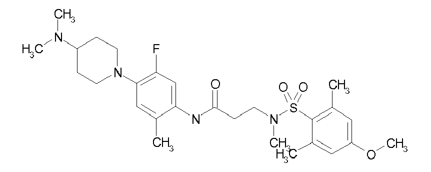 |
| (184) | 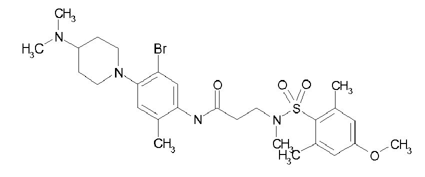 |
| (185) | 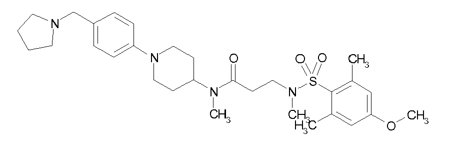 |
| (186) | 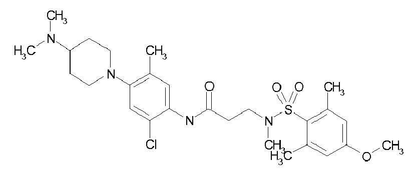 |
| (187) | 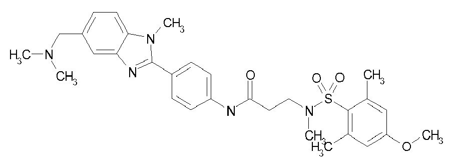 |
| (188) | 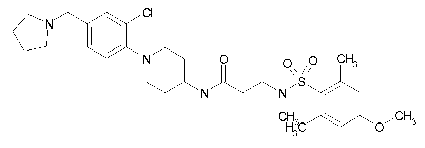 |
| (189) | 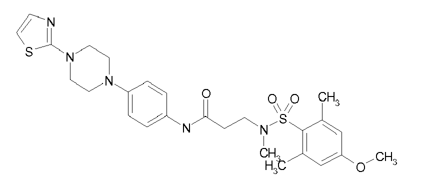 |
| (190) | 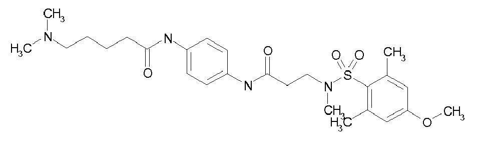 |
| (191) | 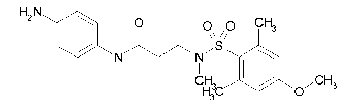 |
| (192) | 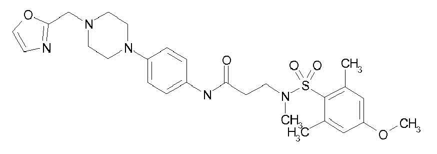 |
| (193) | 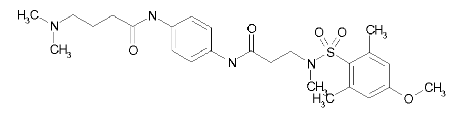 |
| (194) | 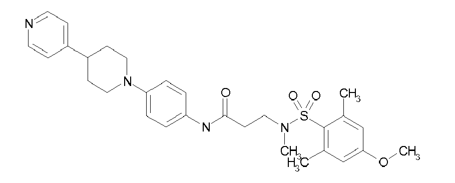 |
| (195) | 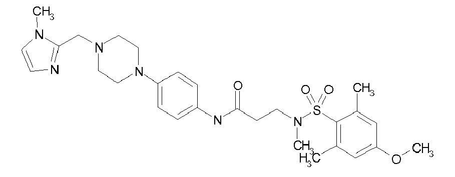 |
| (196) | 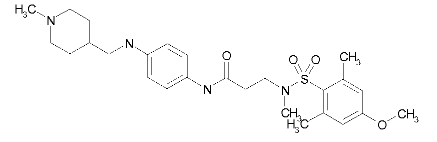 |
| (197) | 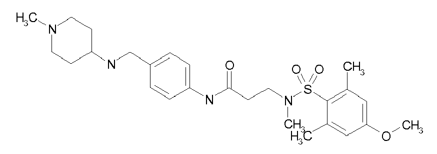 |
| (198) | 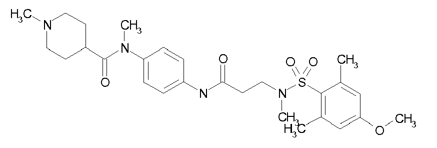 |
| (199) | 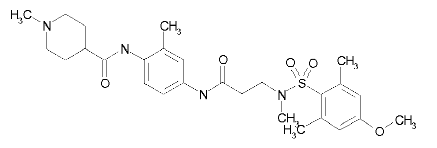 |
| (200) | 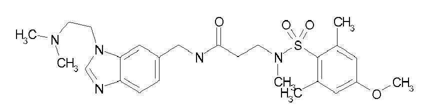 |
| (201) | 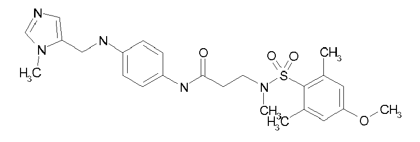 |
| (202) | 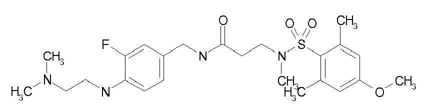 |
| (203) | 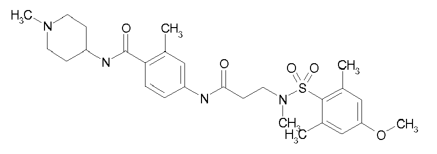 |
| (204) | 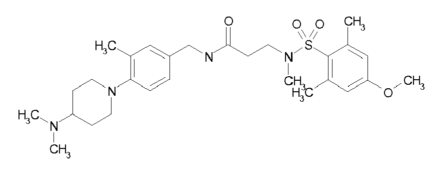 |
| (205) | 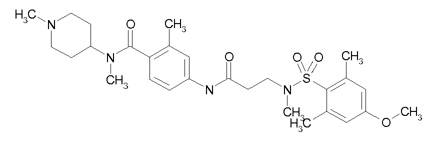 |
| (206) | 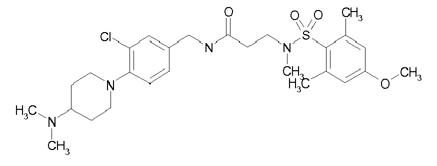 |
| (207) | 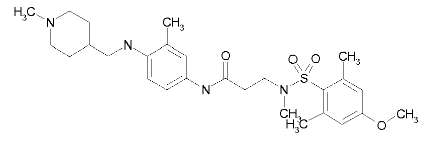 |
| (208) | 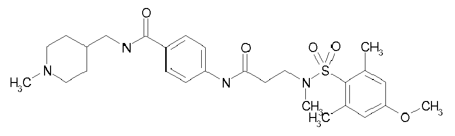 |
| (209) | 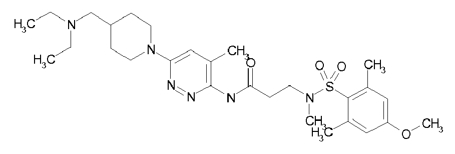 |
| (210) | 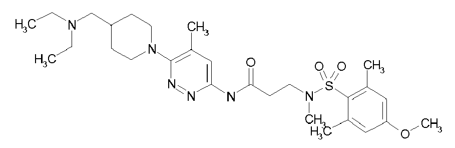 |
| (211) | 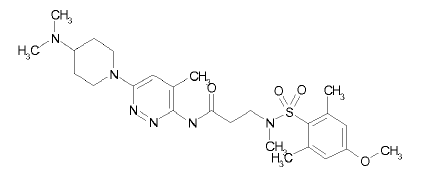 |
| (212) | 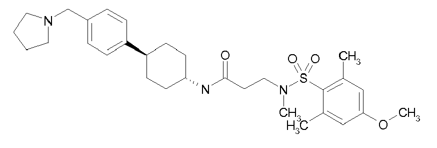 |
| (213) | 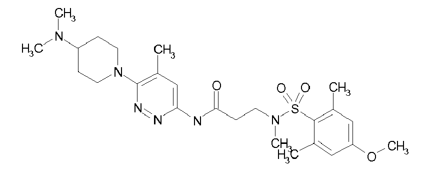 |
| (214) | 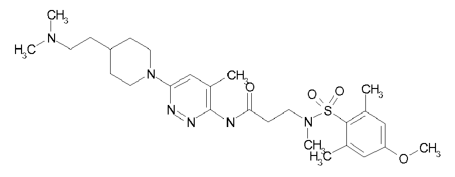 |
| (215) | 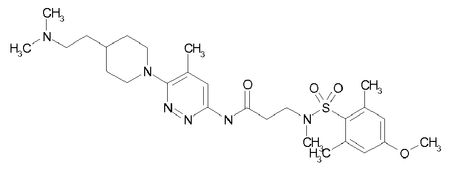 |
| (216) | 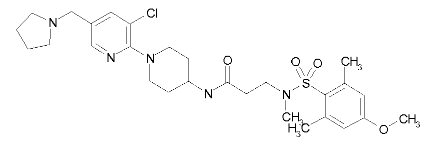 |
| (217) | 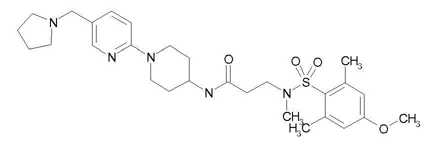 |
| (218) | 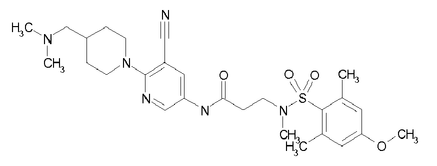 |
| (219) | 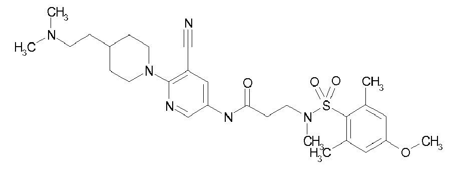 |
| (220) | 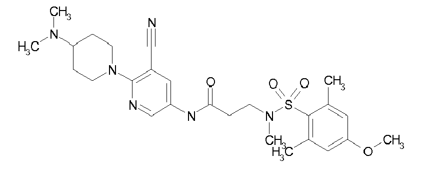 |
| (221) | 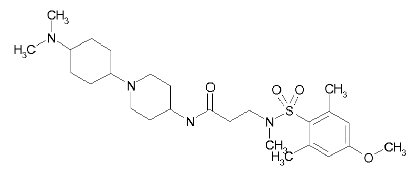 |
| (222) | 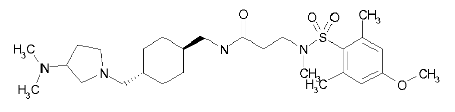 |
| (223) | 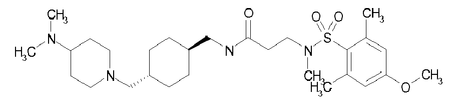 |
| (224) | 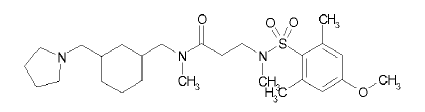 |
| (225) | 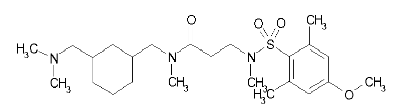 |
| (226) | 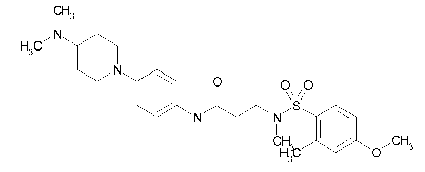 |
| (227) | 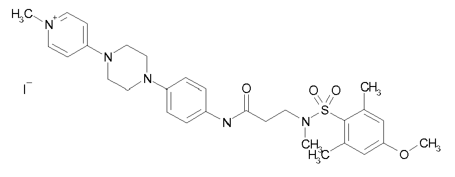 |
| (228) | 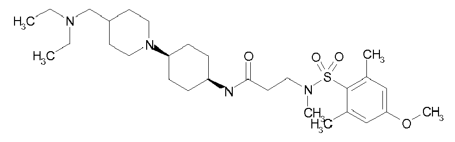 |
| (229) | 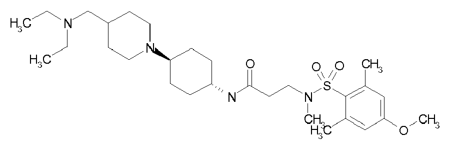 |
| (230) | 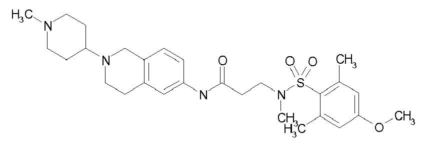 |
| (231) | 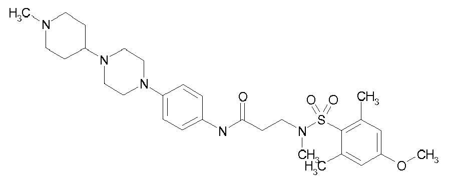 |
| (232) | 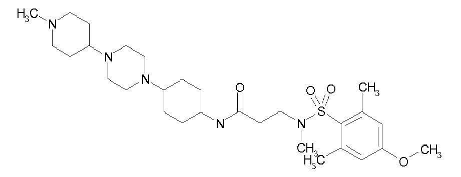 |
| (233) | 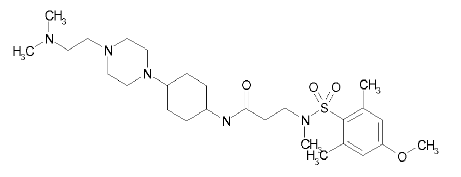 |
| (234) | 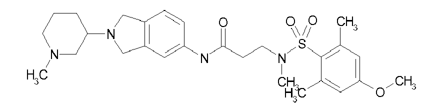 |
| (235) | 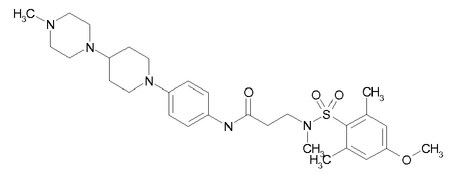 |
| (236) | 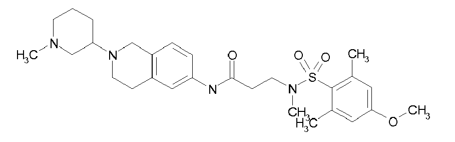 |
| (237) | 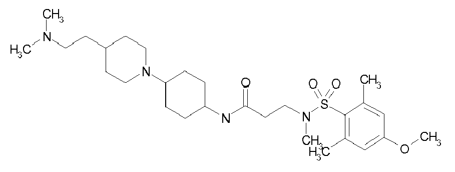 |
| (238) | 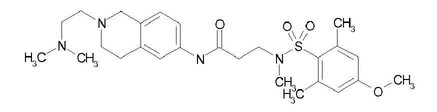 |
| (239) | 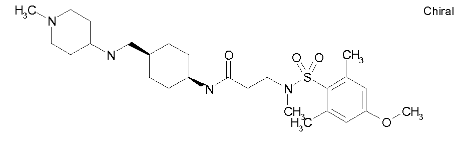 |
| (240) | 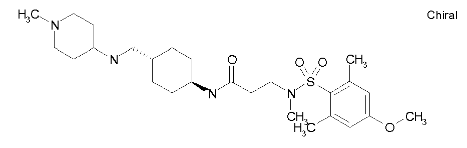 |
| (241) | 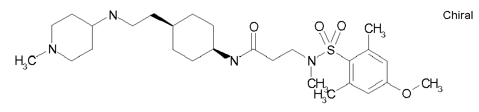 |
| (242) | 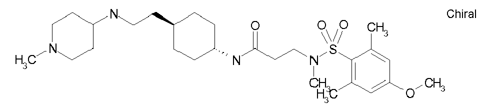 |
| (243) | 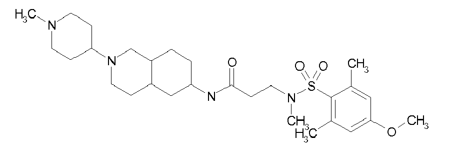 |
| (244) | 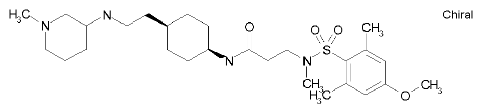 |
| (245) | 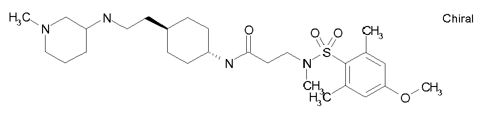 |
| (246) | 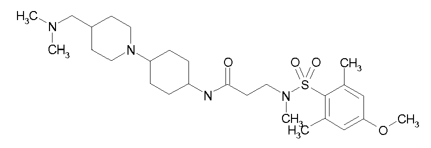 |
| (247) | 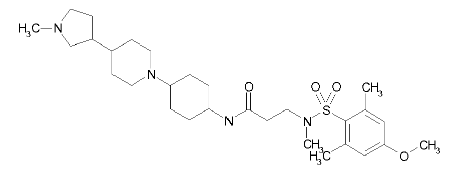 |
| (248) | 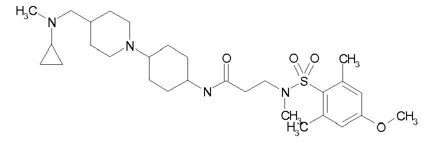 |
| (249) | 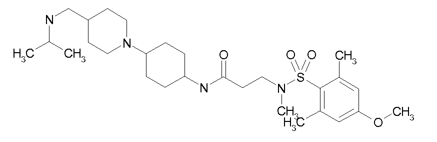 |
| (250) | 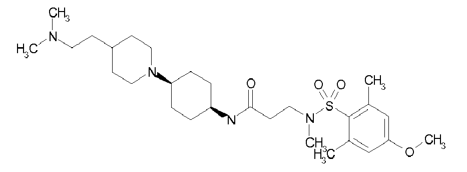 |
| (251) | 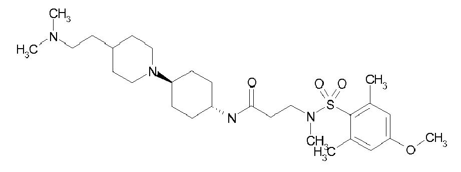 |
| (252) | 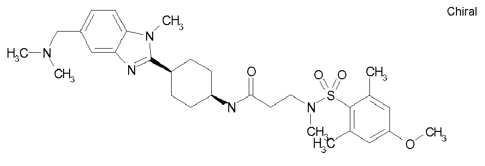 |
| (253) | 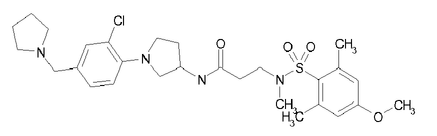 |
| (254) | 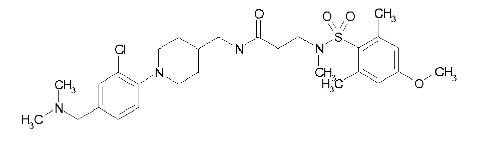 |
| (255) | 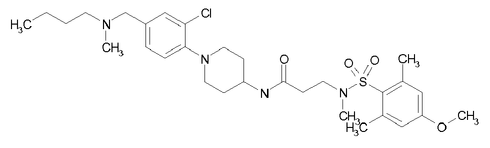 |
| (256) | 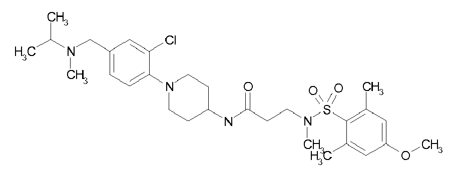 |
| (257) | 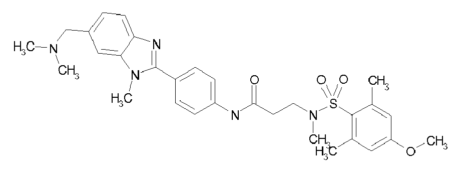 |
| (258) | 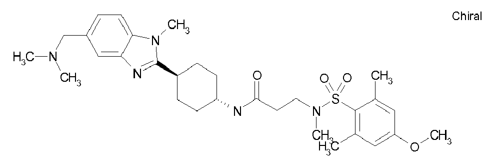 |
| (259) | 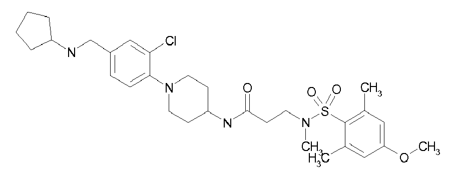 |
| (260) | 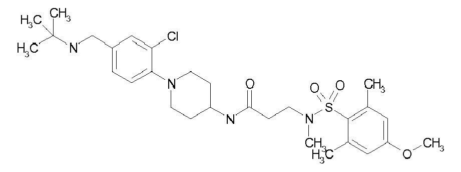 |
| (261) | 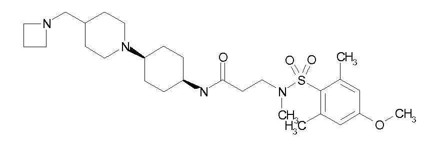 |
| (262) | 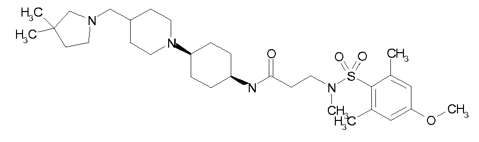 |
| (263) | 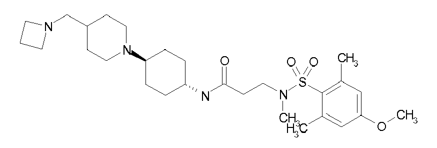 |
| (264) | 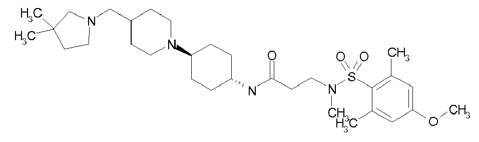 |
| (265) | 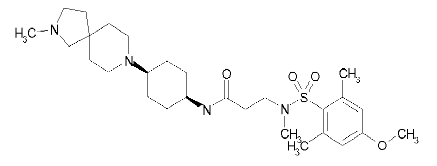 |
| (266) | 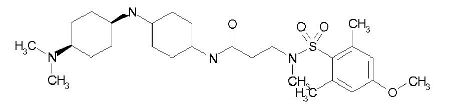 |
| (267) | 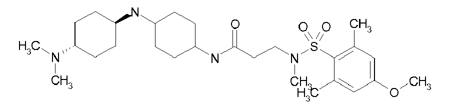 |
| (268) | 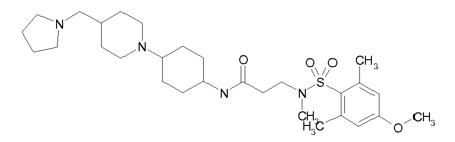 |
| (269) | 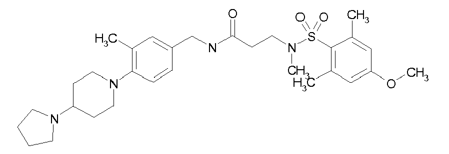 |
| (270) | 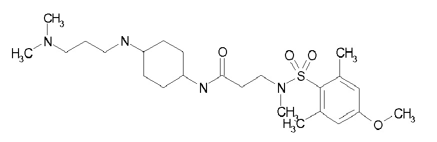 |
| (271) | 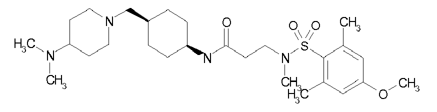 |
| (272) | 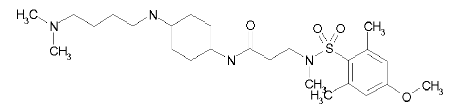 |
| (273) | 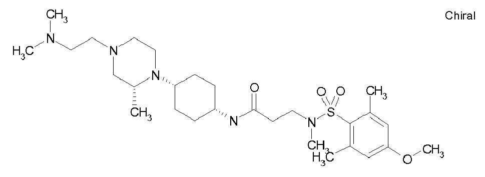 |
| (274) | 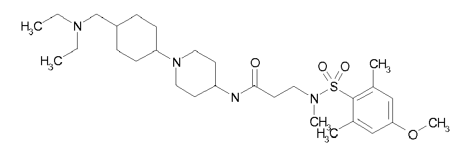 |
| (275) | 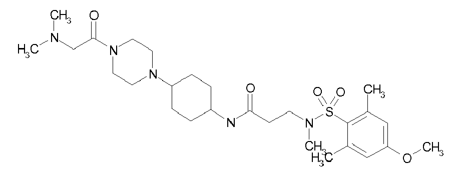 |
| (276) | 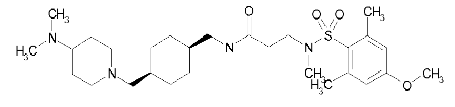 |
| (277) | 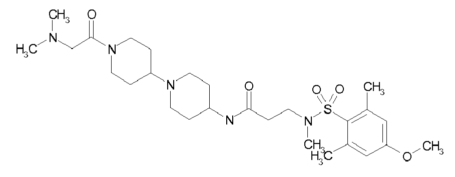 |
| (278) | 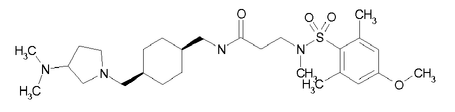 |
| (279) | 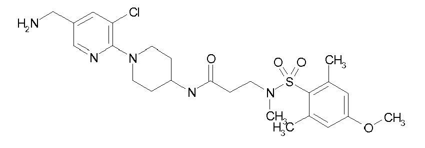 |
| (280) | 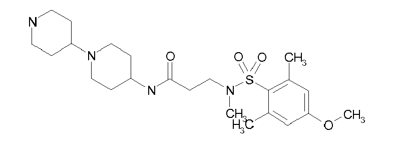 |
| (281) | 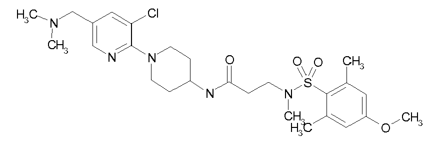 |
| (282) | 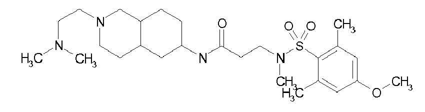 |
| (283) | 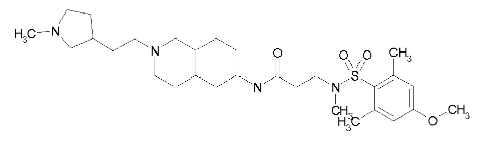 |
| (284) | 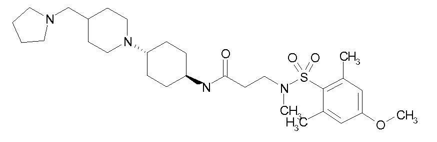 |
| (285) | 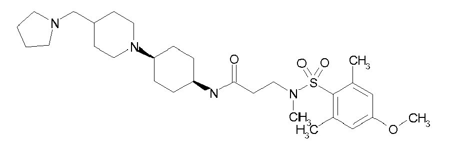 |
| (286) | 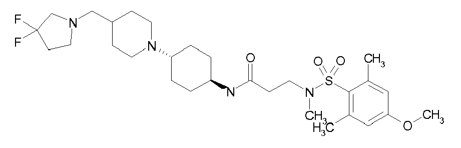 |
| (287) | 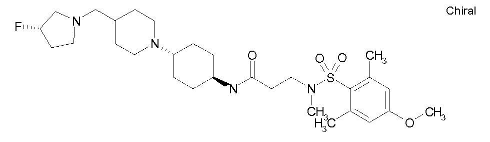 |
| (288) | 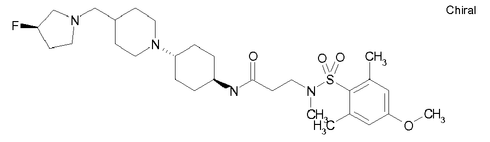 |
| (289) | 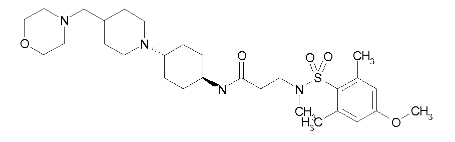 |
| (290) | 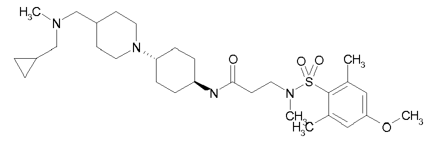 |
| (291) | 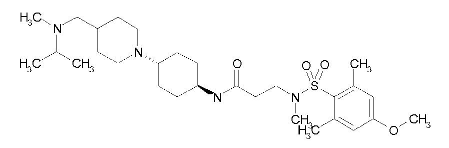 |
| (292) | 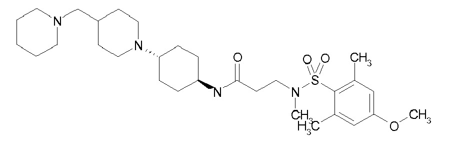 |
| (293) | 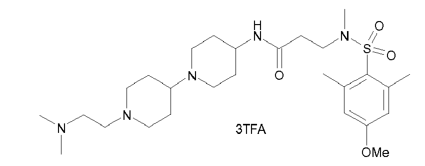 |
| (294) | 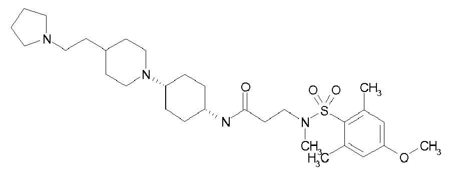 |
| (295) | 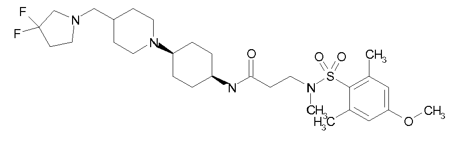 |
| (296) | 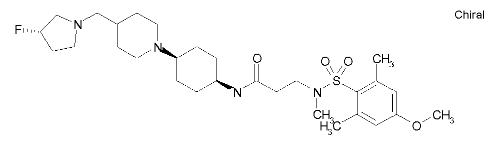 |
| (297) | 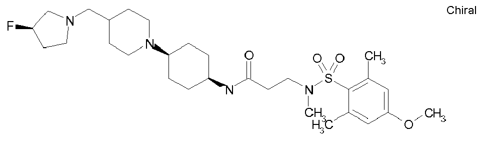 |
| (298) | 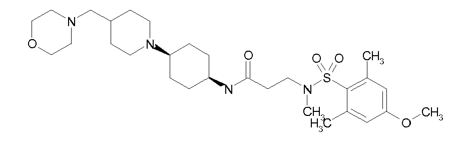 |
| (299) | 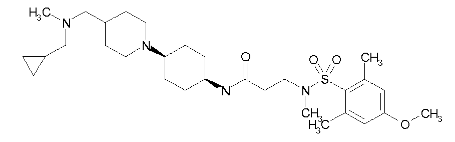 |
| (300) | 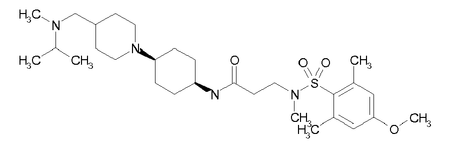 |
| (301) | 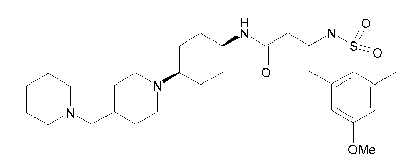 |
| (302) | 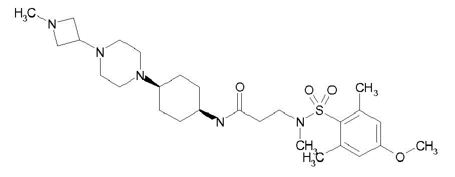 |
| (303) | 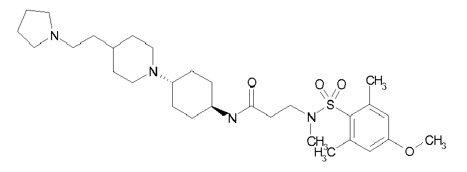 |
| (304) | 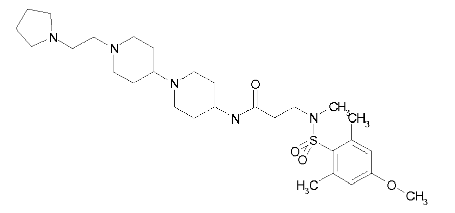 |
| (305) | 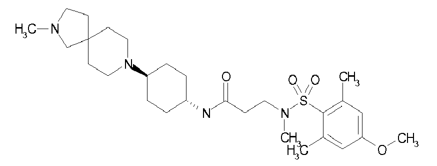 |
| (306) | 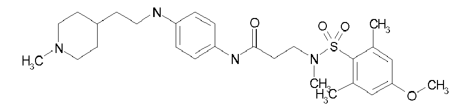 |
| (307) | 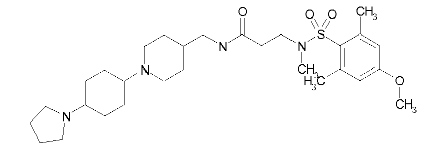 |
| (308) | 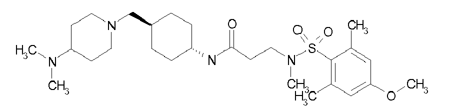 |
| (309) | 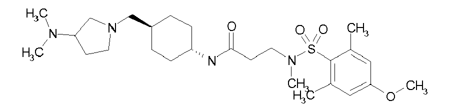 |
| (310) | 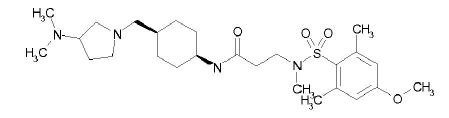 |
| (311) | 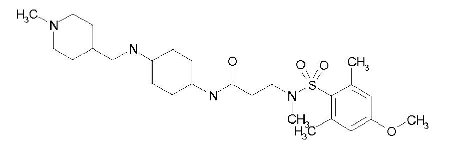 |
| (312) | 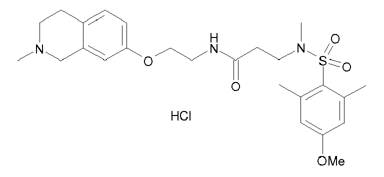 |
| (313) | 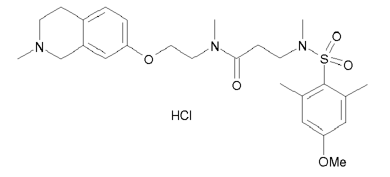 |
| (314) | 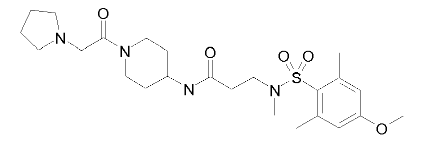 |
| (315) | 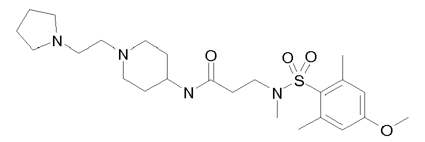 |
| (316) | 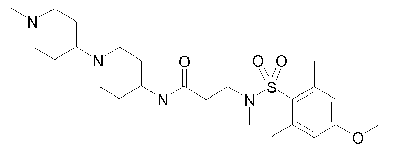 |
| (317) | 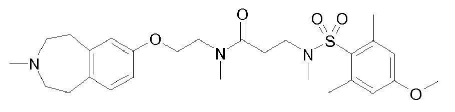 |
| (318) | 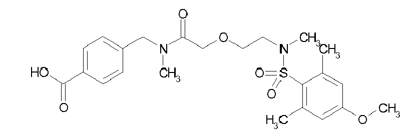 |
| (319) | 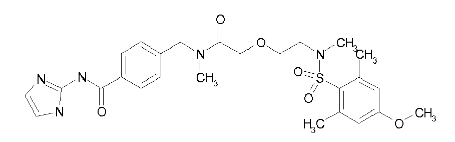 |
| (320) | 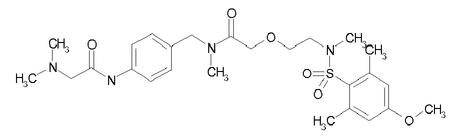 |
| (321) | 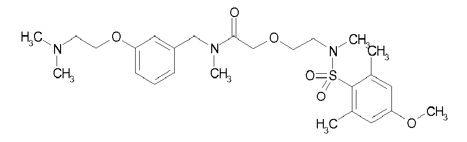 |
| (322) | 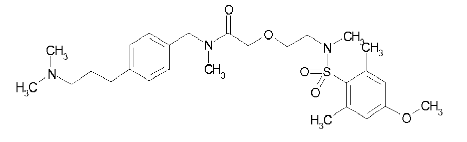 |
| (323) | 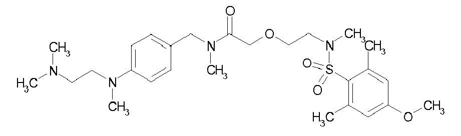 |
| (324) | 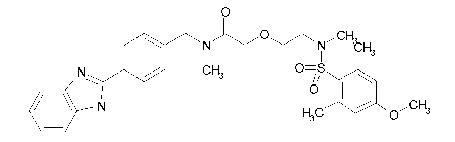 |
| (325) | 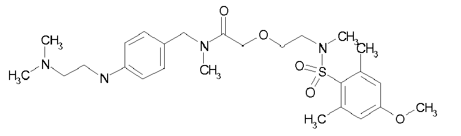 |
| (326) | 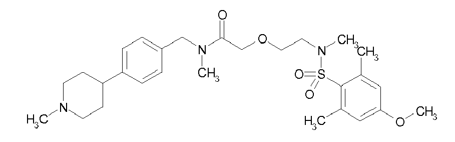 |
| (327) | 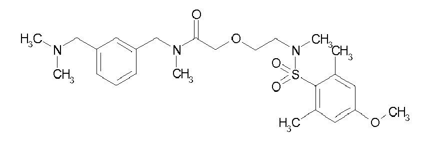 |
| (328) | 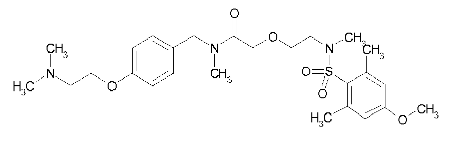 |
| (329) | 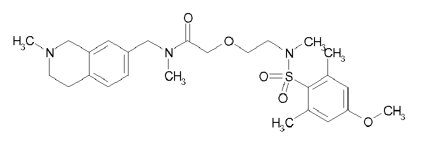 |
| (330) | 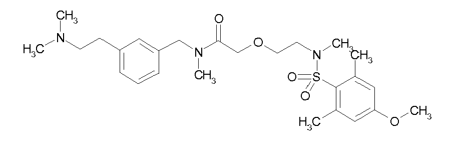 |
| (331) | 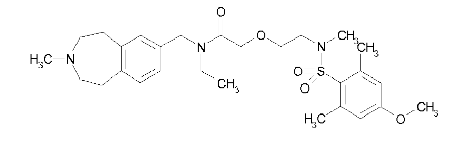 |
| (332) | 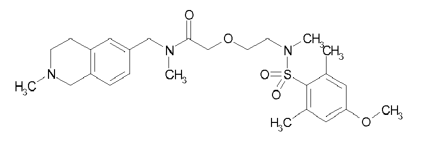 |
| (333) | 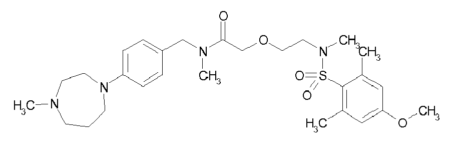 |
| (334) | 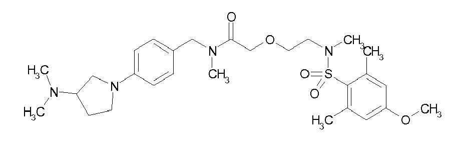 |
| (335) | 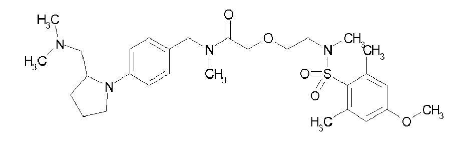 |
| (336) | 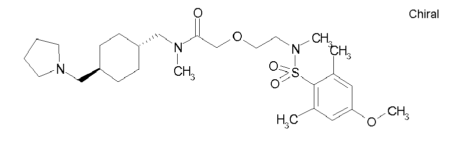 |
| (337) | 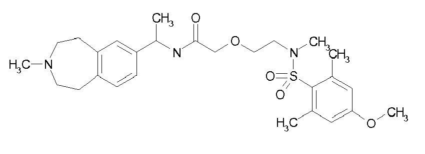 |
| (338) | 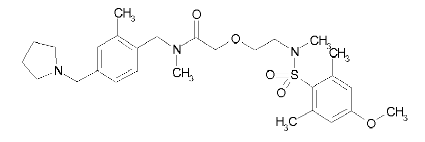 |
| (339) | 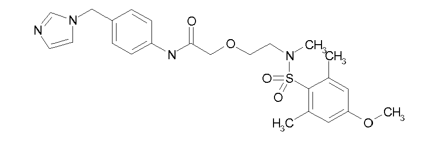 |
| (340) | 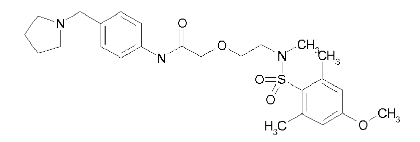 |
| (341) | 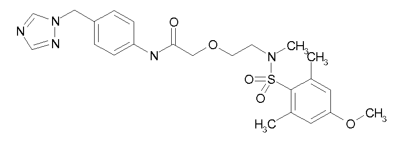 |
| (342) | 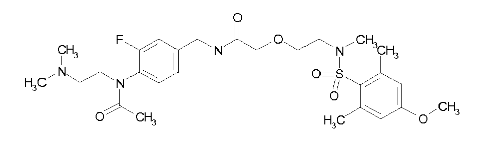 |
| (343) | 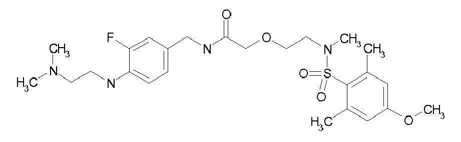 |
| (344) | 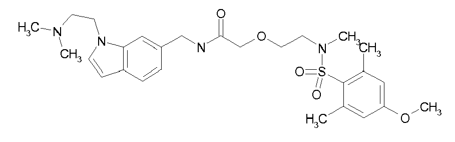 |
| (345) | 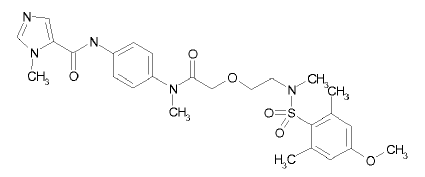 |
| (346) | 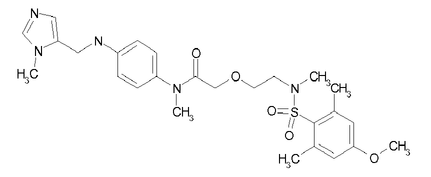 |
| (347) | 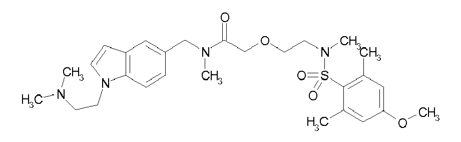 |
| (348) | 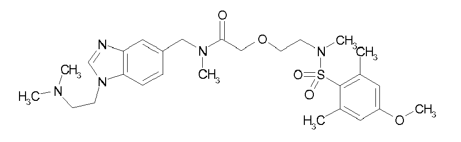 |
| (349) | 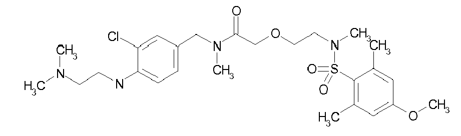 |
| (350) | 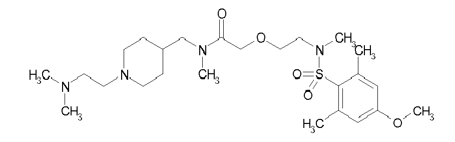 |
| (351) | 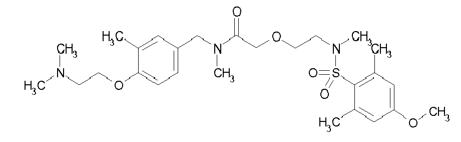 |
| (352) | 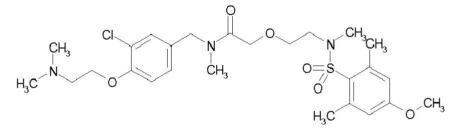 |
| (353) | 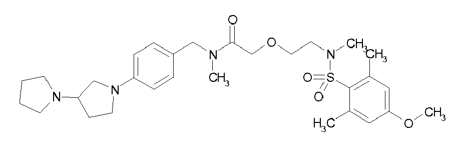 |
| (354) | 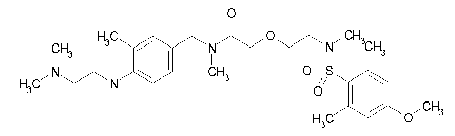 |
| (355) | 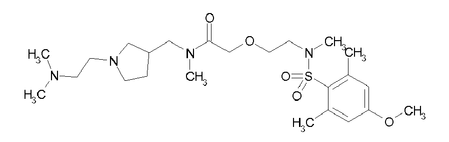 |
| (356) | 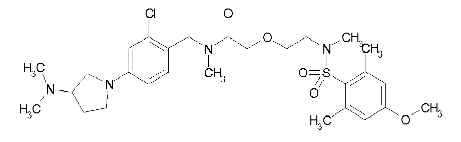 |
| (357) | 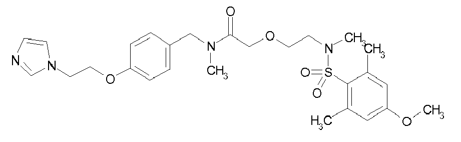 |
| (358) | 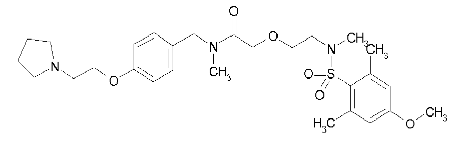 |
| (359) | 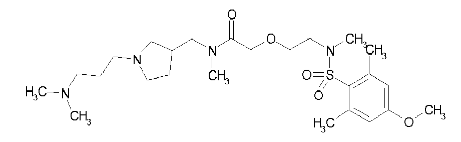 |
| (360) | 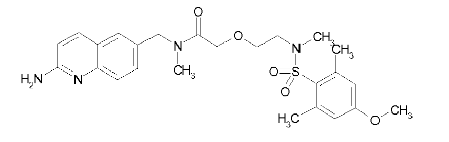 |
| (361) | 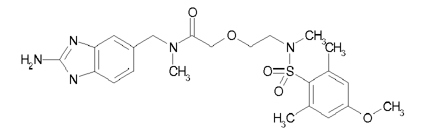 |
| (362) | 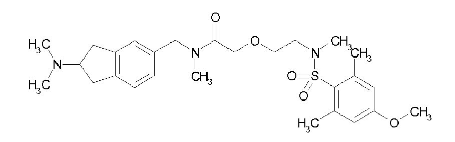 |
| (363) | 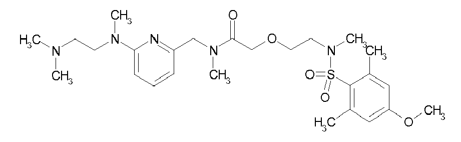 |
| (364) | 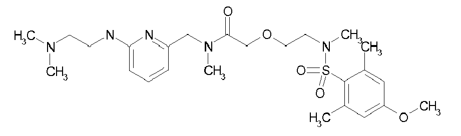 |
| (365) | 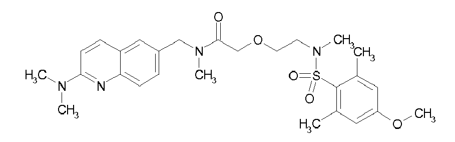 |
| (366) | 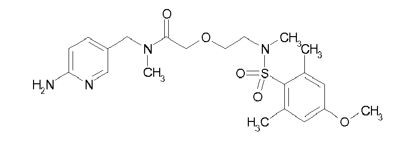 |
| (367) | 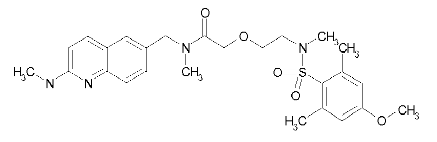 |
| (368) | 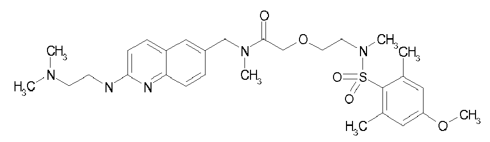 |
| (369) | 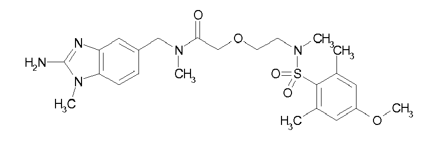 |
| (370) | 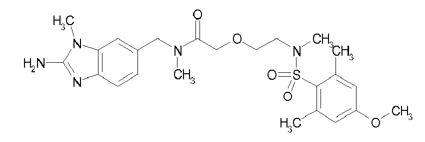 |
| (371) | 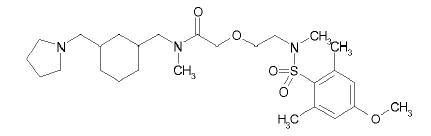 |
| (372) | 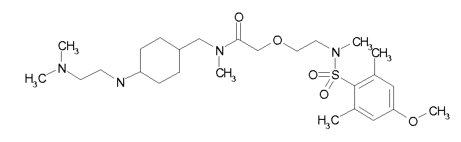 |
| (373) | 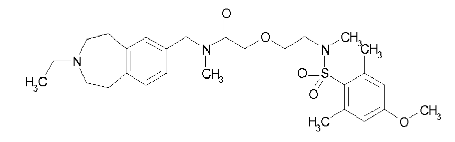 |
| (374) | 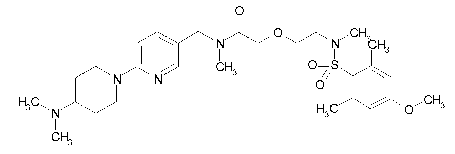 |
| (375) | 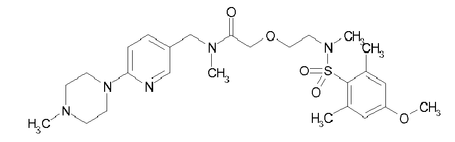 |
| (376) | 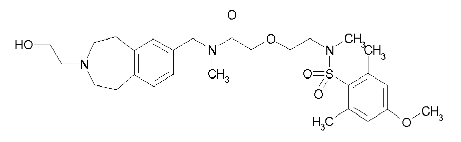 |
| (377) | 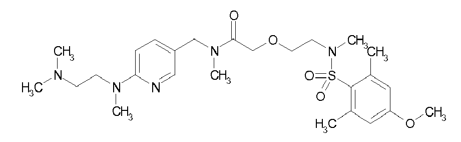 |
| (378) | 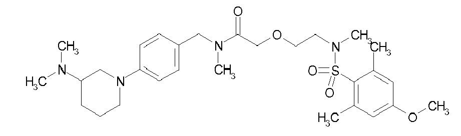 |
| (379) | 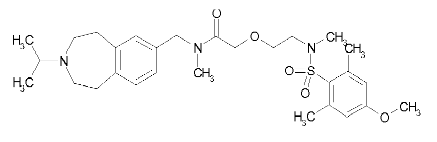 |
| (380) | 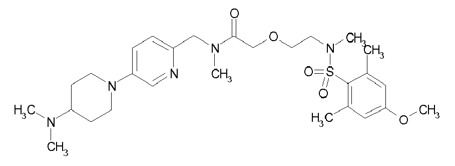 |
| (381) | 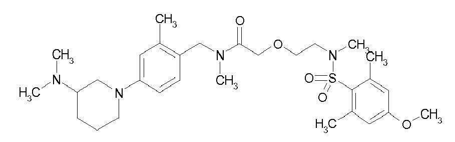 |
| (382) | 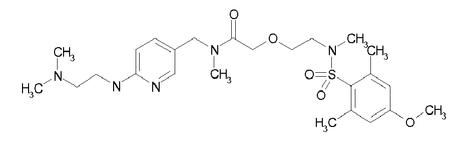 |
| (383) | 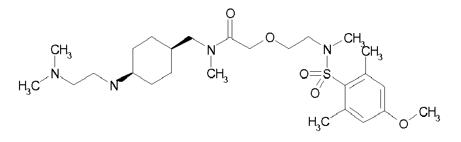 |
| (384) | 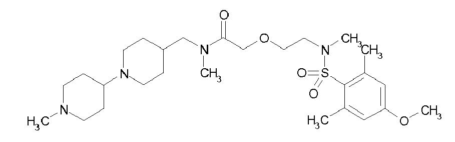 |
| (385) | 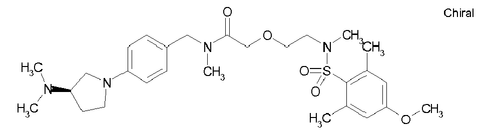 |
| (386) | 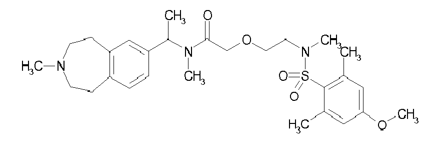 |
| (387) | 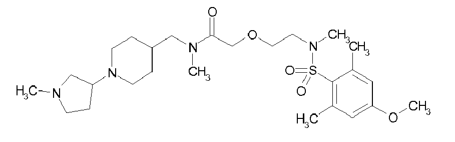 |
| (388) | 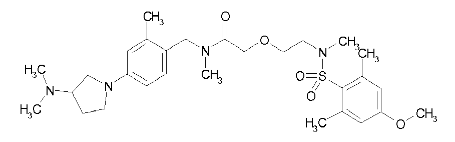 |
| (389) | 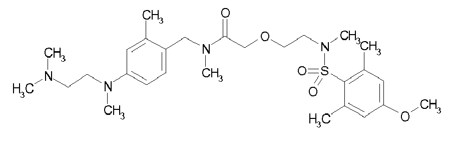 |
| (390) | 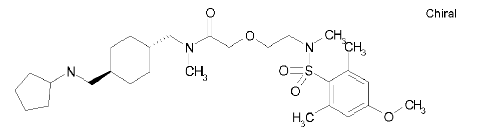 |
| (391) | 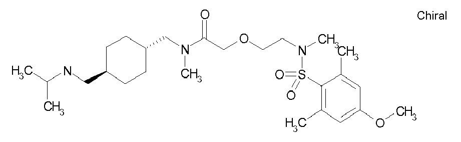 |
| (392) | 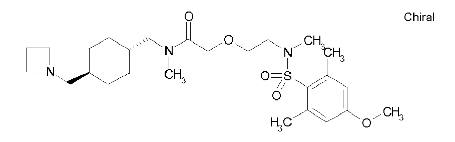 |
| (393) | 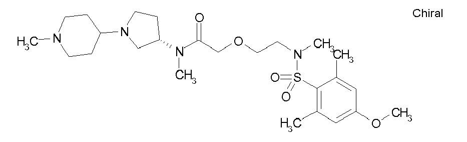 |
| (394) | 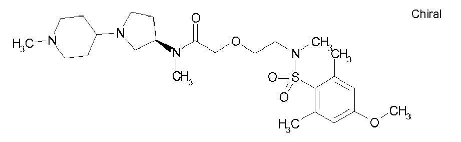 |
| (395) | 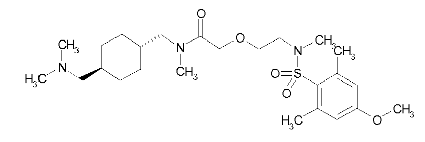 |
| (396) | 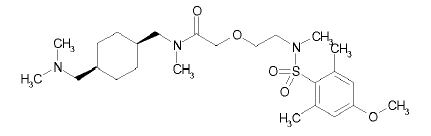 |
| (397) | 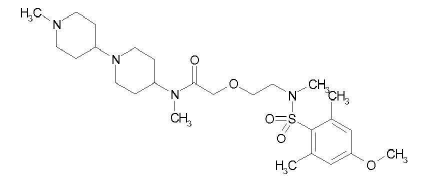 |
| (398) | 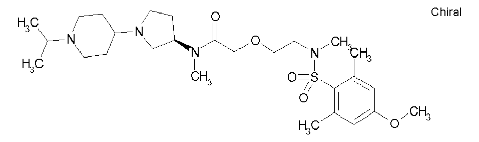 |
| (399) | 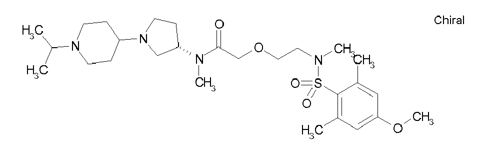 |
| (400) | 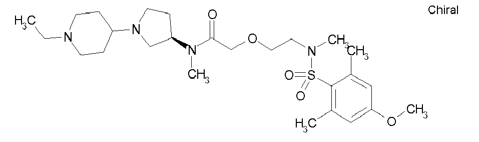 |
| (401) | 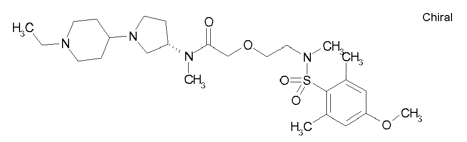 |
| (402) | 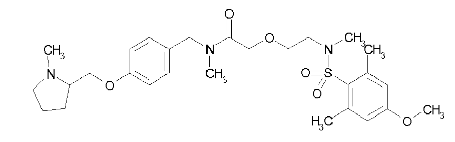 |
| (403) | 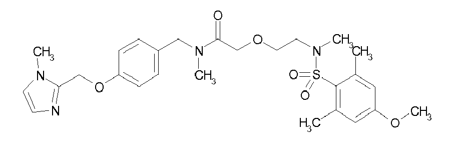 |
| (404) | 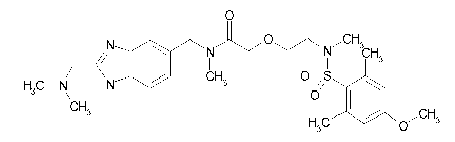 |
| (405) | 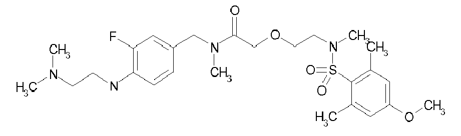 |
| (406) | 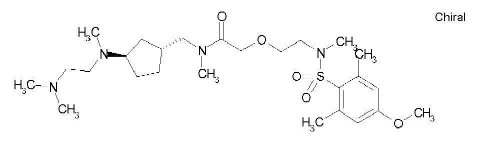 |
| (407) | 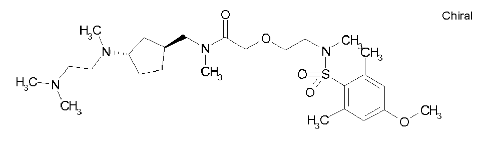 |
| (408) | 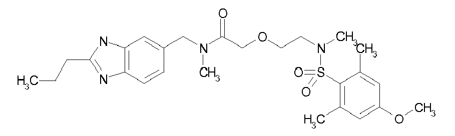 |
| (409) | 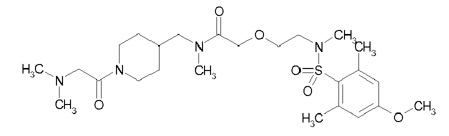 |
| (410) | 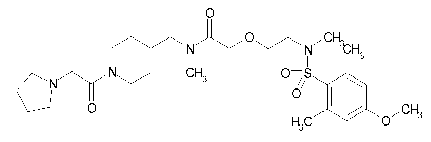 |
| (411) | 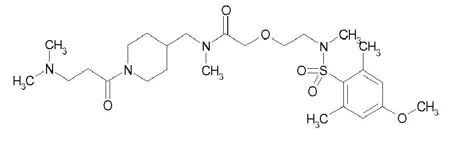 |
| (412) | 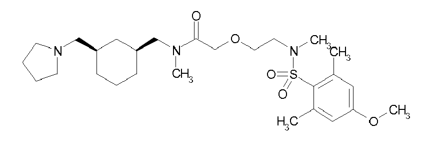 |
| (413) | 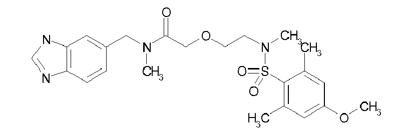 |
| (414) | 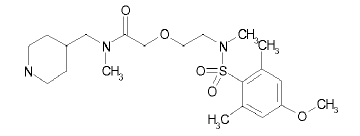 |
| (415) | 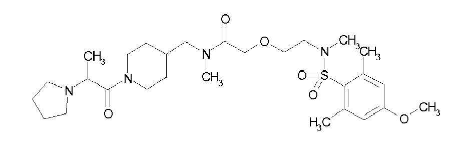 |
| (416) | 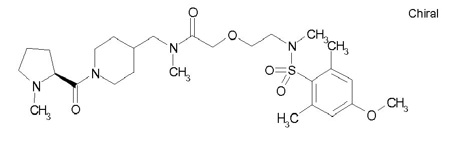 |
| (417) | 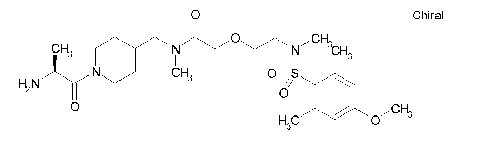 |
| (418) | 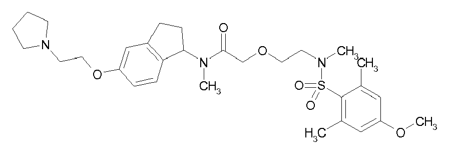 |
| (419) | 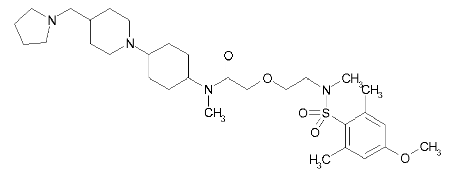 |
| (420) | 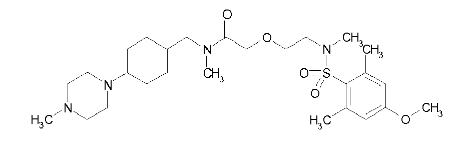 |
| (421) | 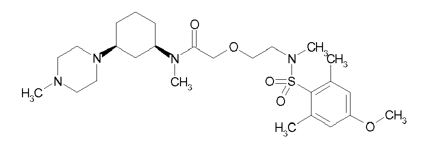 |
| (422) | 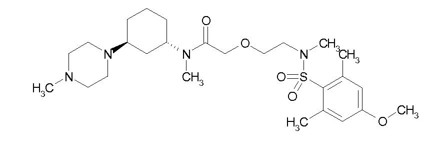 |
| (423) | 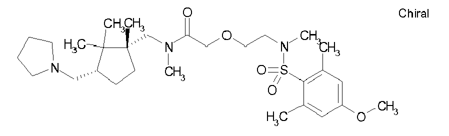 |
| (424) | 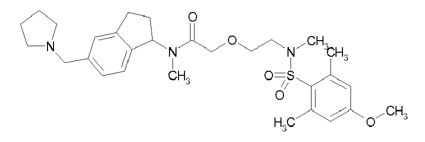 |
| (425) | 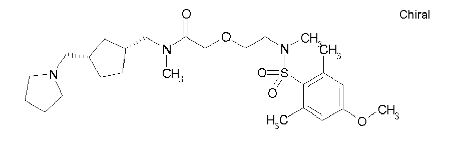 |
| (426) | 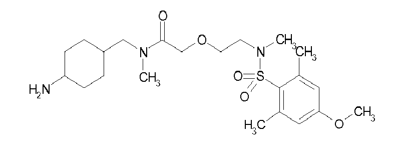 |
| (427) | 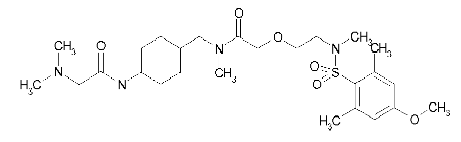 |
| (428) | 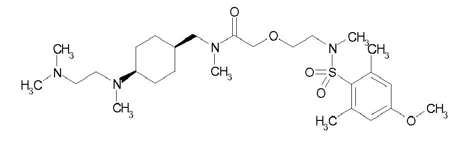 |
| (429) | 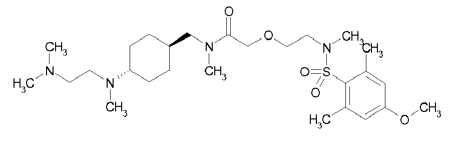 |
| (430) | 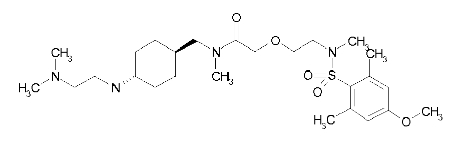 |
| (431) | 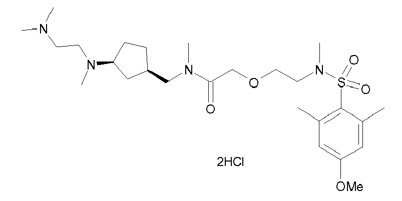 |
| (432) | 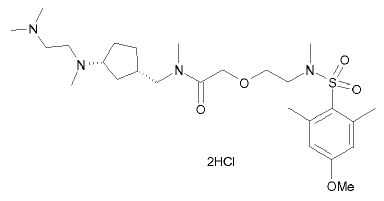 |
| (433) | 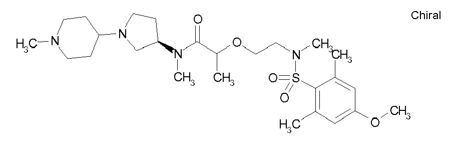 |
| (434) | 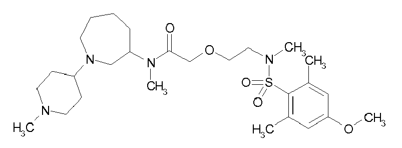 |
| (435) | 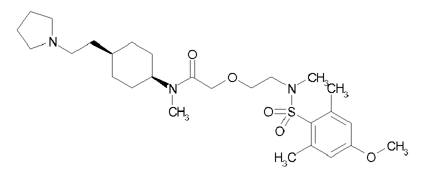 |
| (436) | 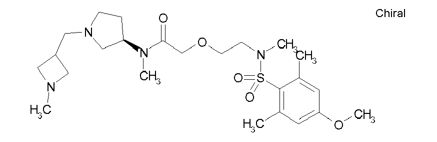 |
| (437) | 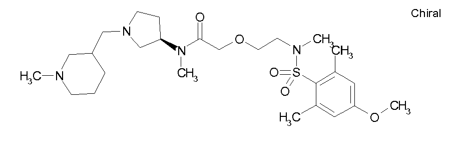 |
| (438) | 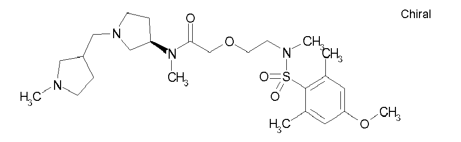 |
| (439) | 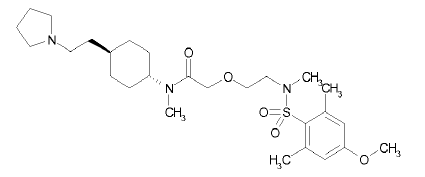 |
| (440) | 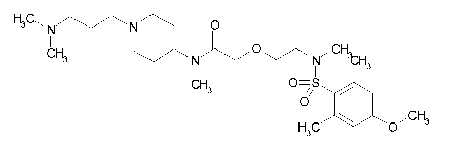 |
| (441) | 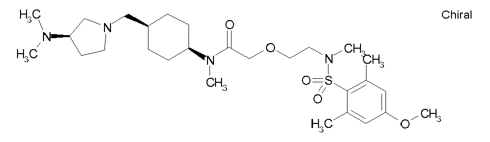 |
| (442) | 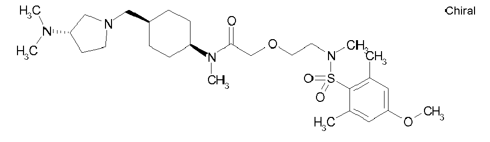 |
| (443) | 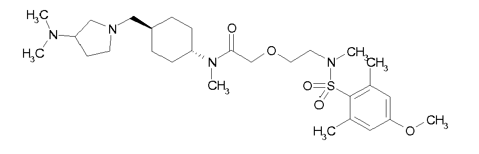 |
| (444) | 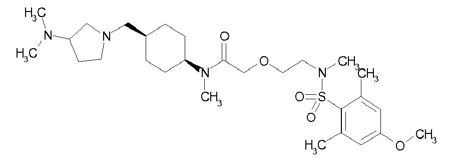 |
| (445) | 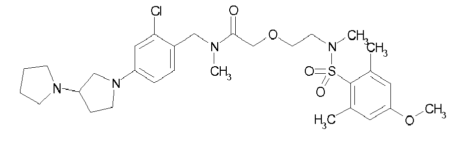 |
| (446) | 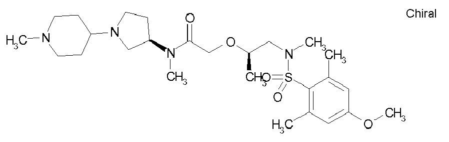 |
| (447) | 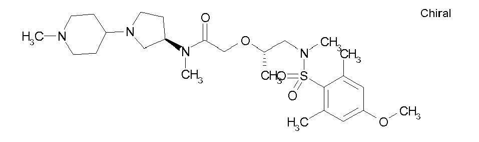 |
| (448) | 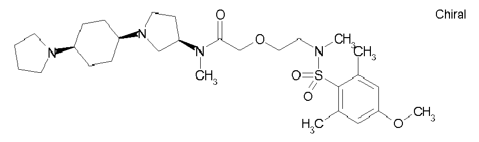 |
| (449) | 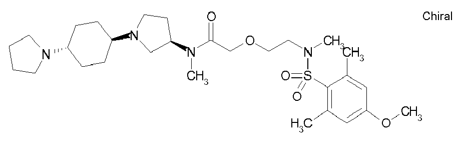 |
| (450) | 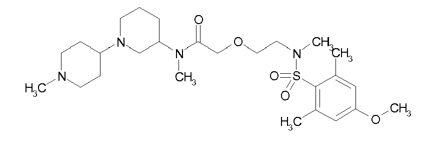 |
| (451) | 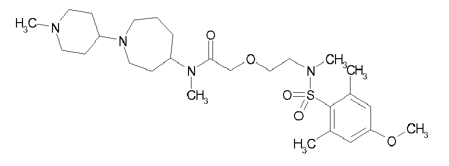 |
| (452) | 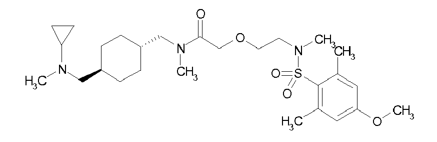 |
| (453) | 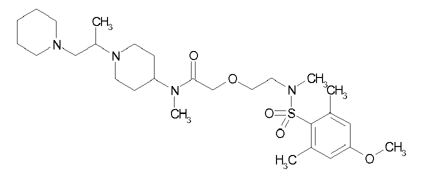 |
| (454) | 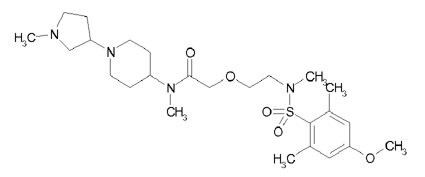 |
| (455) | 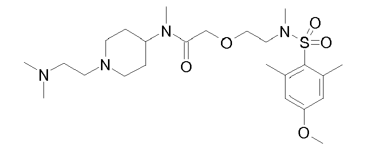 |
| (456) | 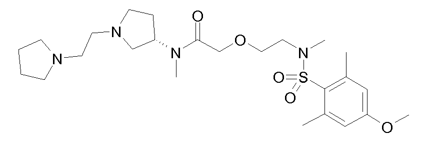 |
| (457) | 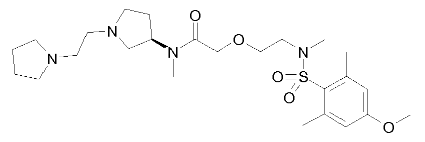 |
| (458) | 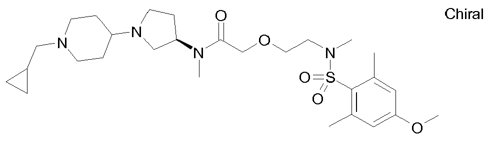 |
| (459) | 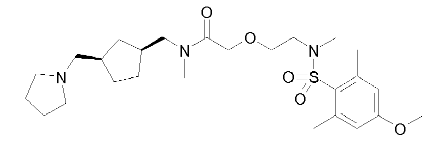 |
| (460) | 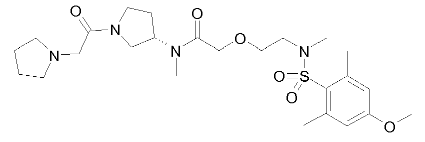 |
| (461) | 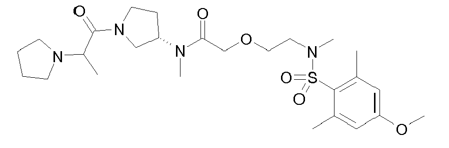 |
| (462) | 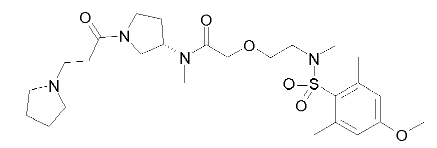 |
| (463) | 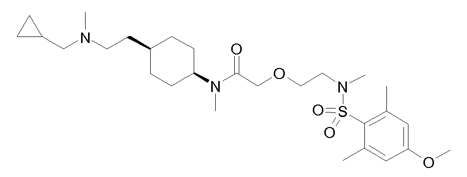 |
| (464) | 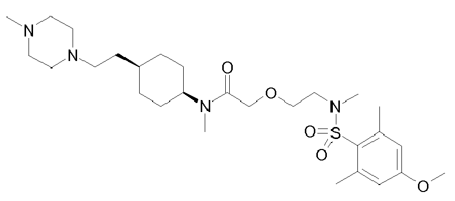 |
| (465) | 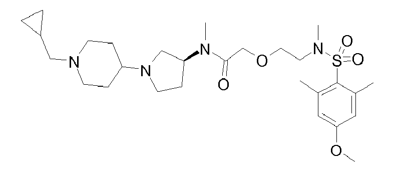 |
| (466) | 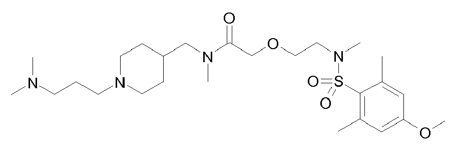 |
| (467) | 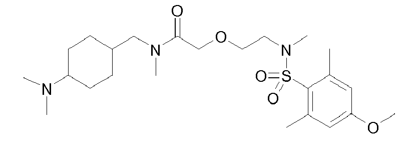 |
| (468) | 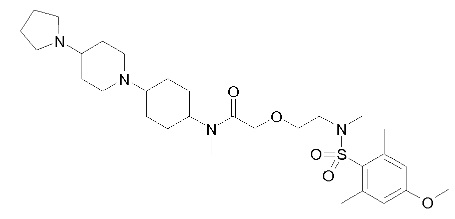 |
| (469) | 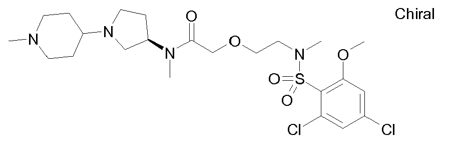 |
| (470) | 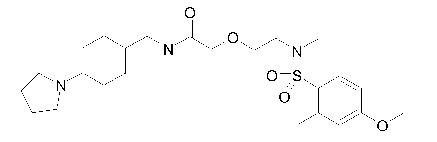 |
| (471) | 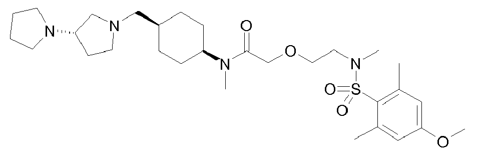 |
| (472) | 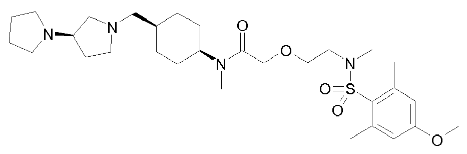 |
| (473) | 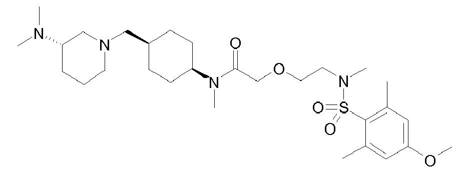 |
| (474) | 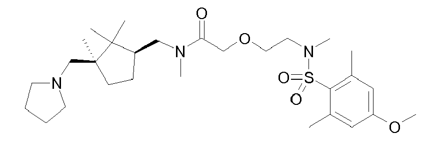 |
| (475) | 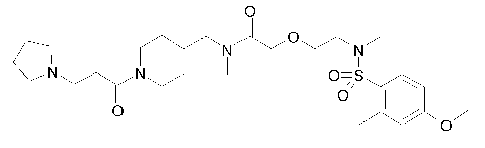 |
| (476) | 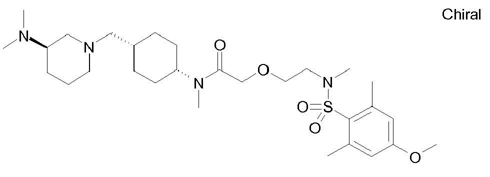 |
| (477) | 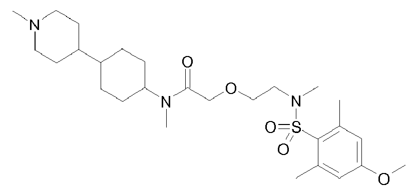 |
| (478) | 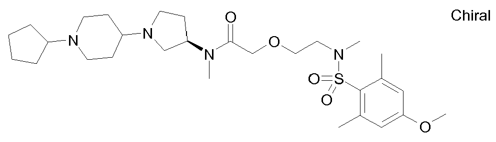 |
| (479) | 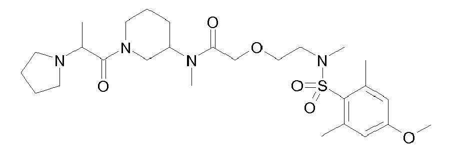 |
| (480) | 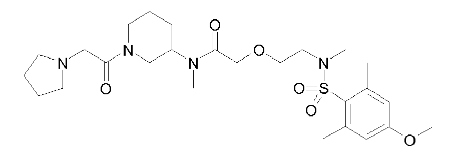 |
| (481) | 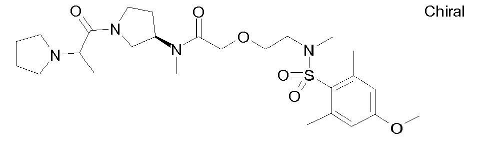 |
| (482) | 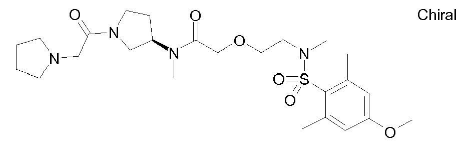 |
| (483) | 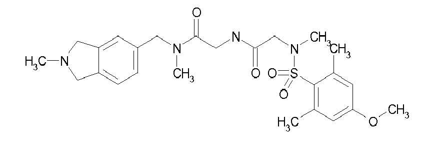 |
| (484) | 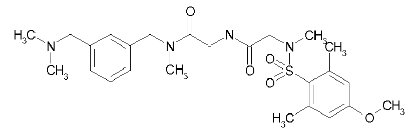 |
| (485) | 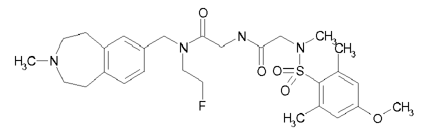 |
| (486) | 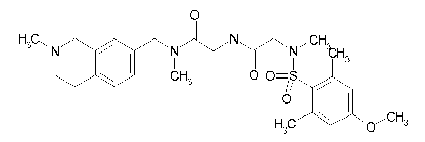 |
| (487) | 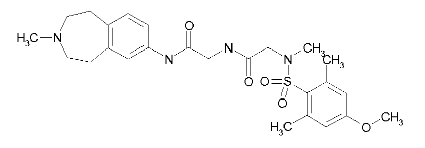 |
| (488) | 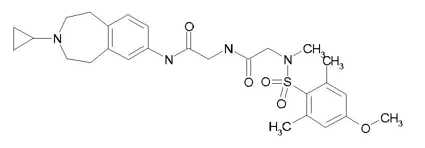 |
| (489) | 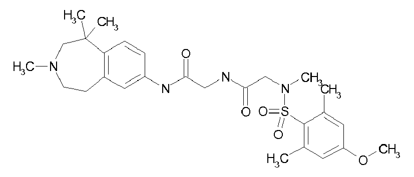 |
| (490) | 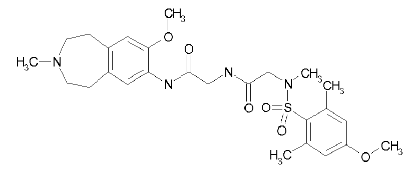 |
| (491) | 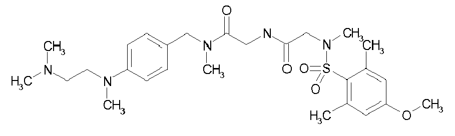 |
| (492) | 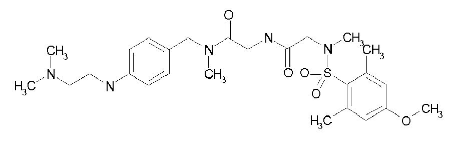 |
| (493) | 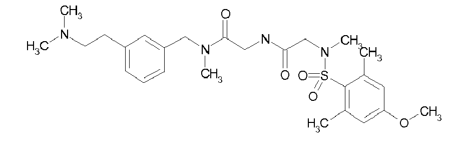 |
| (494) | 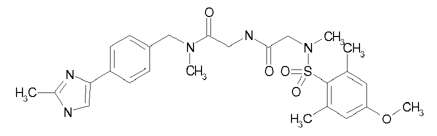 |
| (495) | 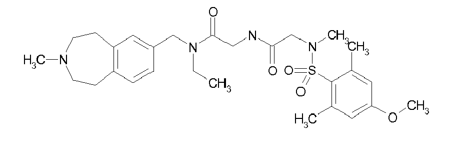 |
| (496) | 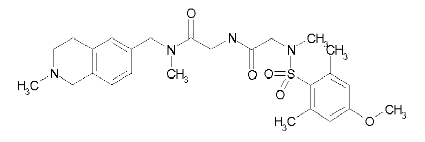 |
| (497) | 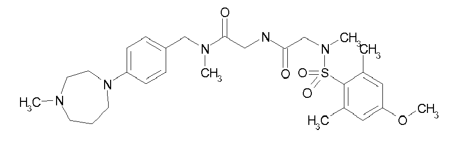 |
| (498) | 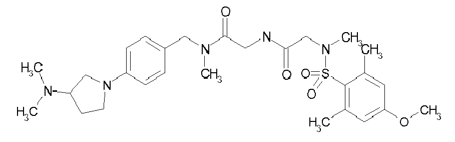 |
| (499) | 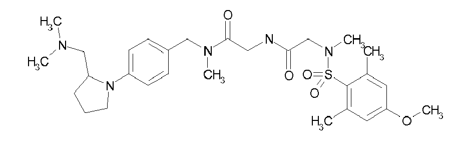 |
| (500) | 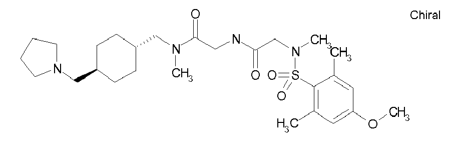 |
| (501) | 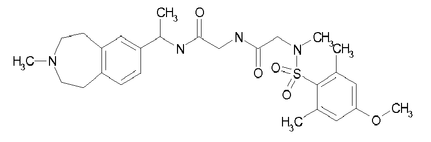 |
| (502) | 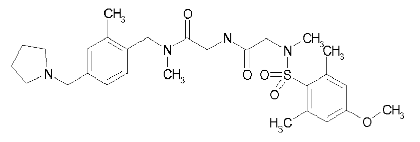 |
| (503) | 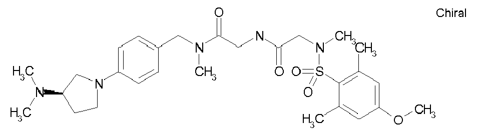 |
| (504) | 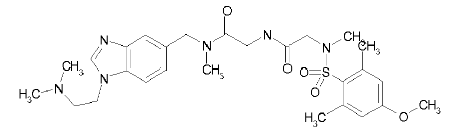 |
| (505) | 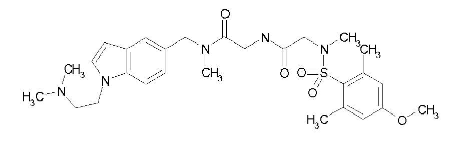 |
| (506) | 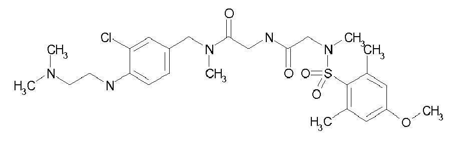 |
| (507) | 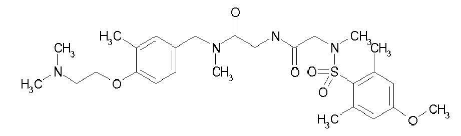 |
| (508) | 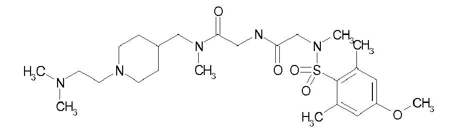 |
| (509) | 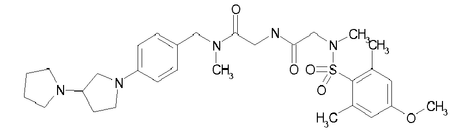 |
| (510) | 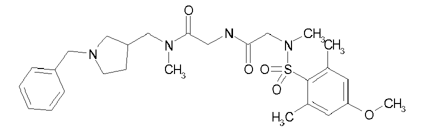 |
| (511) | 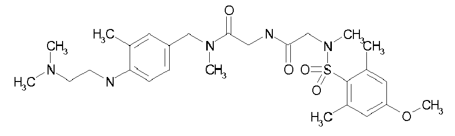 |
| (512) | 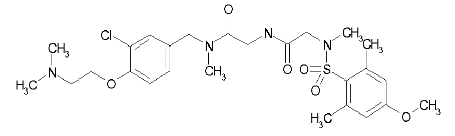 |
| (513) | 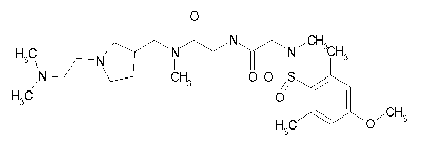 |
| (514) | 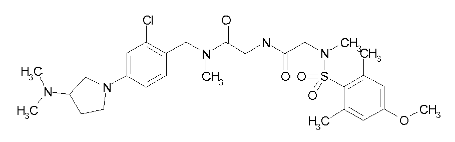 |
| (515) | 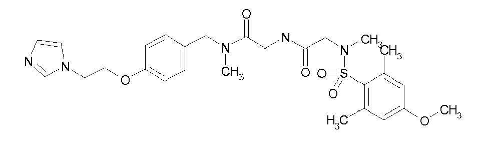 |
| (516) | 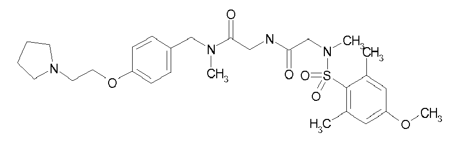 |
| (517) | 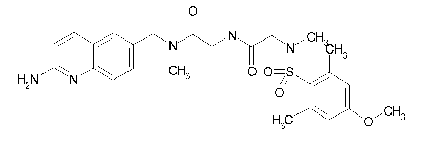 |
| (518) | 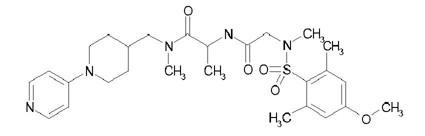 |
| (519) | 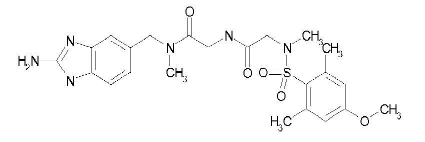 |
| (520) | 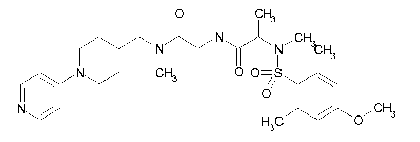 |
| (521) | 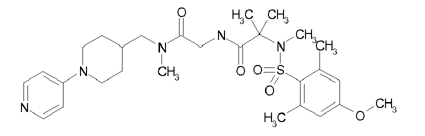 |
| (522) | 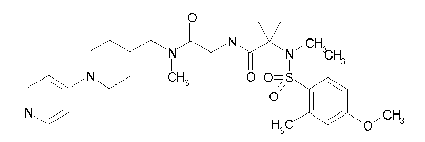 |
| (523) | 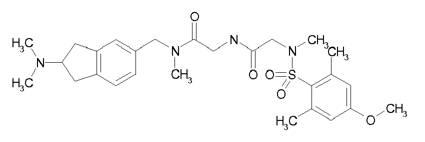 |
| (524) | 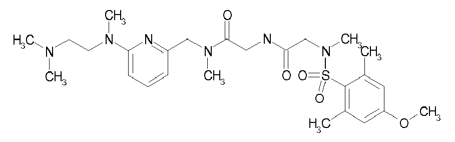 |
| (525) | 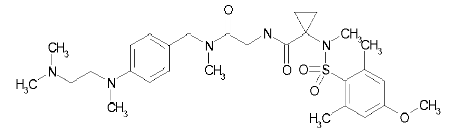 |
| (526) | 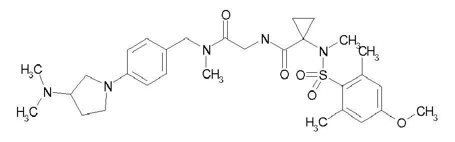 |
| (527) | 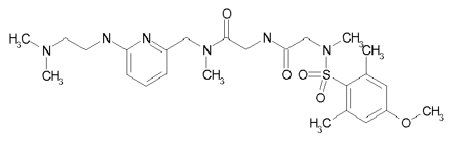 |
| (528) | 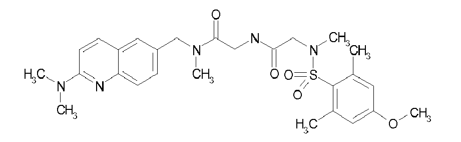 |
| (529) | 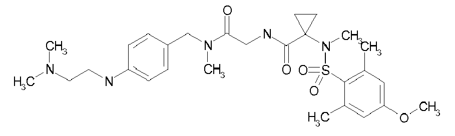 |
| (530) | 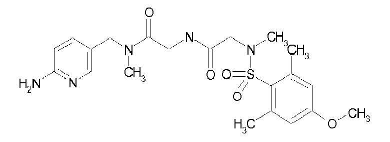 |
| (531) | 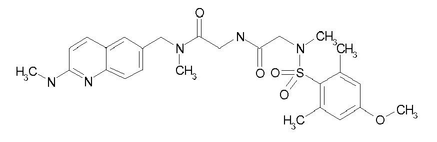 |
| (532) | 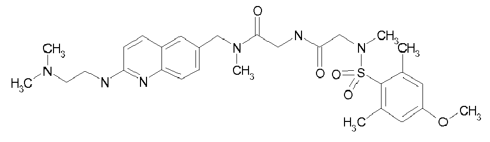 |
| (533) | 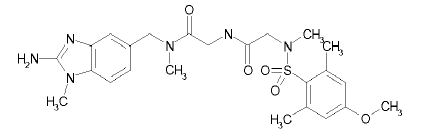 |
| (534) | 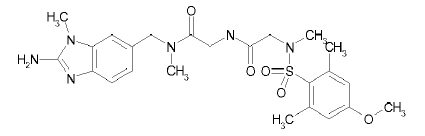 |
| (535) | 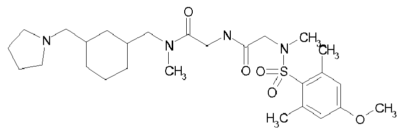 |
| (536) | 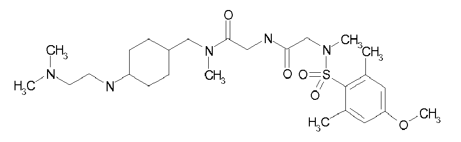 |
| (537) | 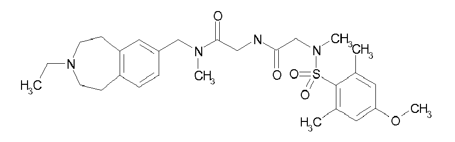 |
| (538) | 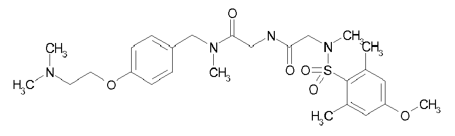 |
| (539) | 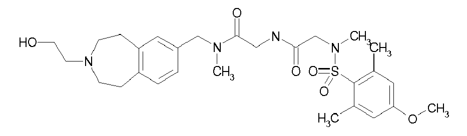 |
| (540) | 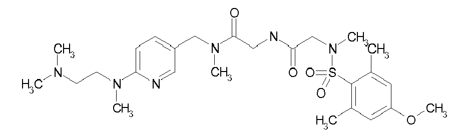 |
| (541) | 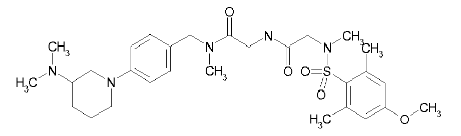 |
| (542) | 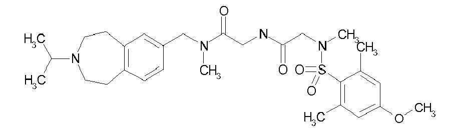 |
| (543) | 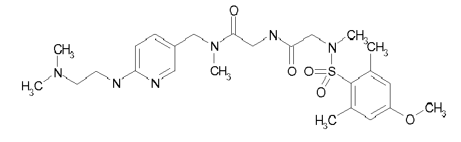 |
| (544) | 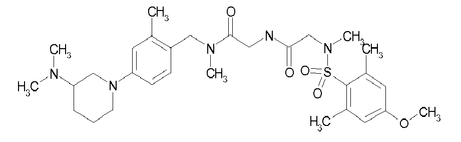 |
| (545) | 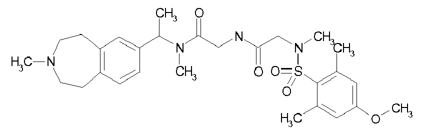 |
| (546) | 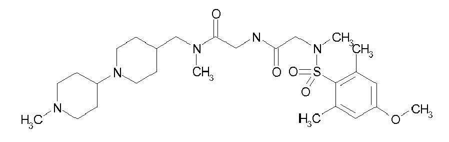 |
| (547) | 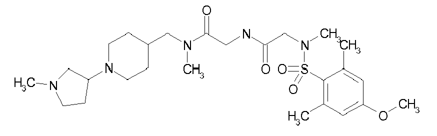 |
| (548) | 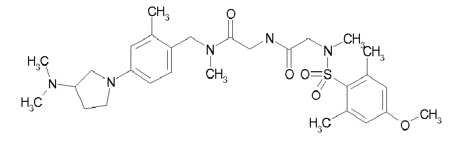 |
| (549) | 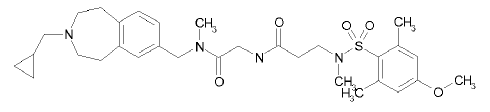 |
| (550) | 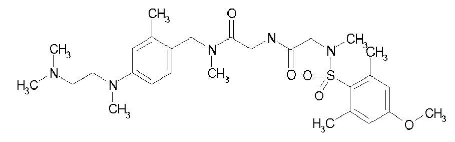 |
| (551) | 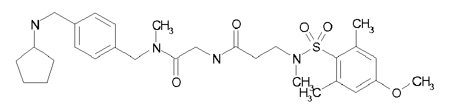 |
| (552) | 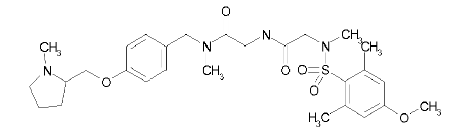 |
| (553) | 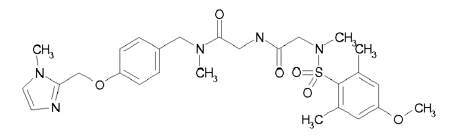 |
| (554) | 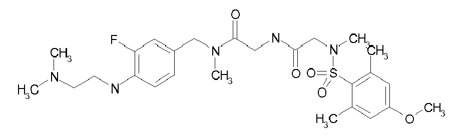 |
| (555) | 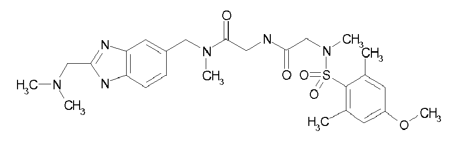 |
| (556) | 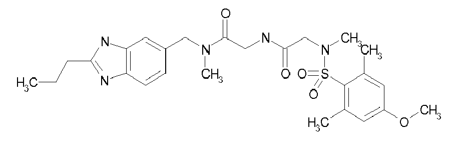 |
| (557) | 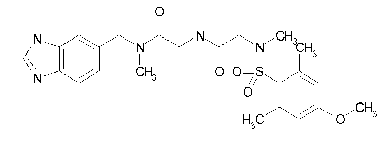 |
| (558) | 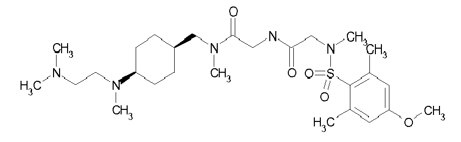 |
| (559) | 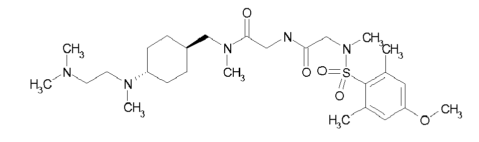 |
| (560) | 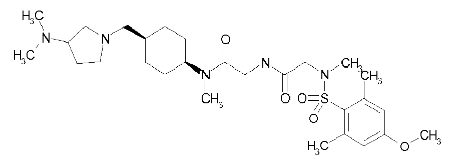 |
| (561) | 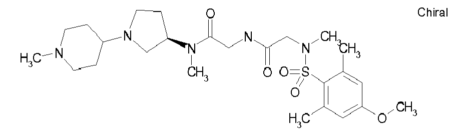 |
| (562) | 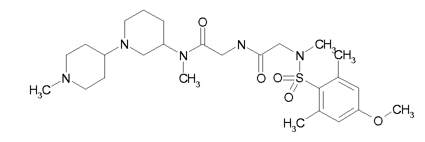 |
| (563) | 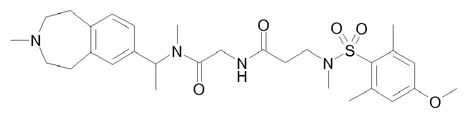 |
| (564) | 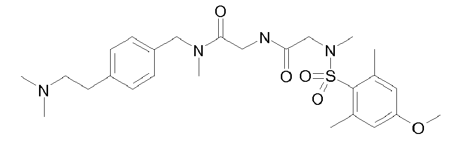 |
| (565) | 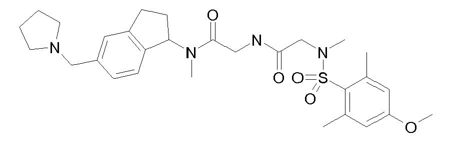 |
| (566) | 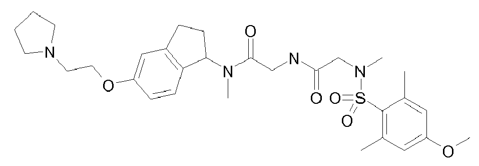 |
| (567) | 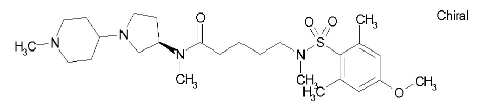 |
| (568) | 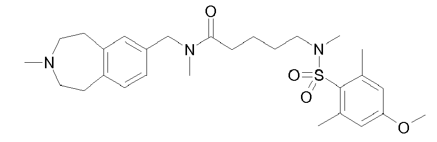 |
| (569) | 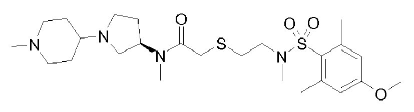 |
| (570) | 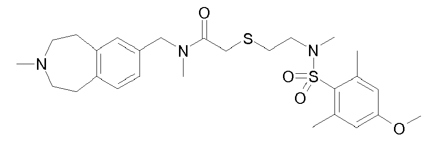 |
| (571) | 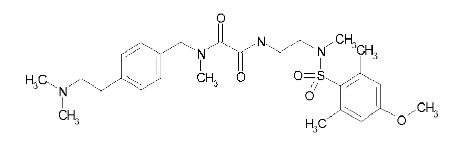 |
| (572) | 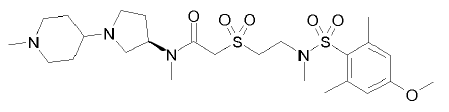 |
| (573) | 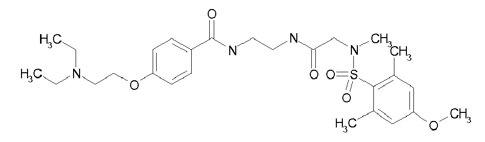 |
| (574) | 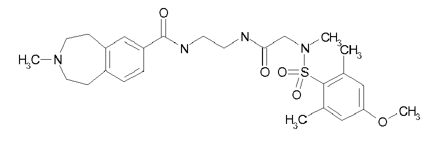 |
| (575) | 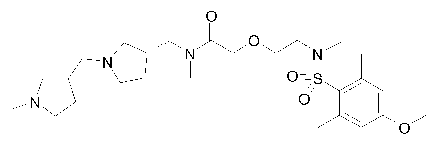 |
| (576) | 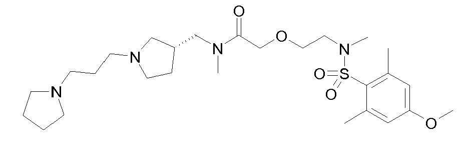 |
| (577) | 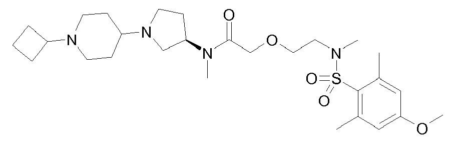 |
| (578) | 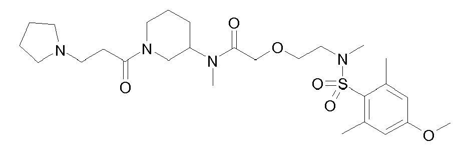 |
| (579) | 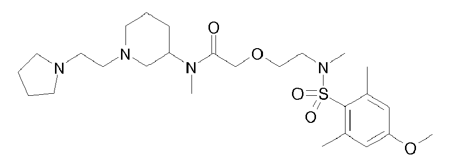 |
| (580) | 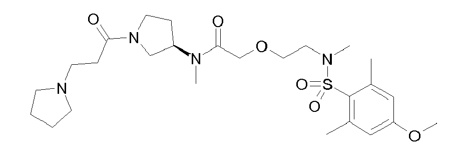 |
| (581) | 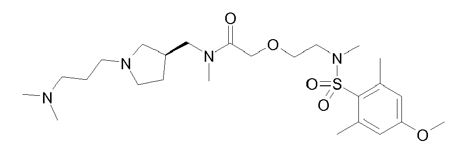 |
| (582) | 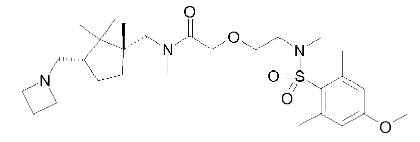 |
| (583) | 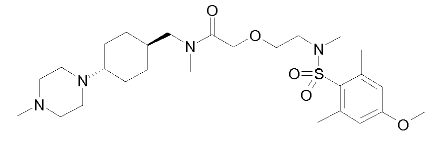 |
| (584) | 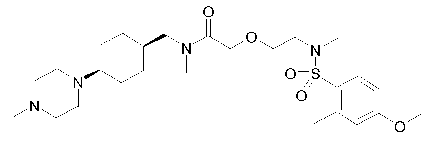 |
| (585) | 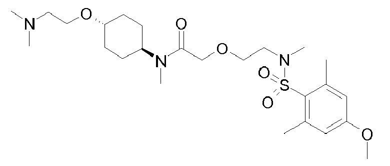 |
| (586) | 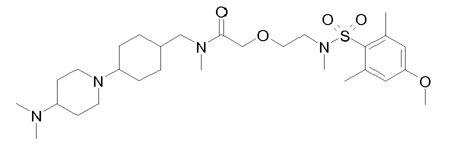 |
| (587) | 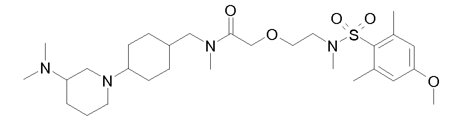 |
| (588) | 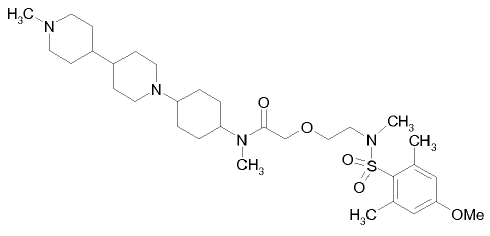 |
| (589) | 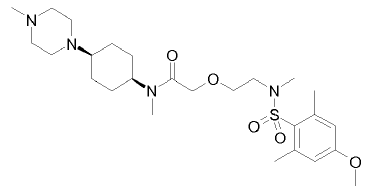 |
| (590) | 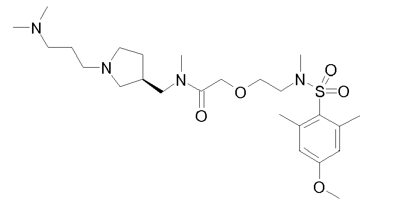 |
| (591) | 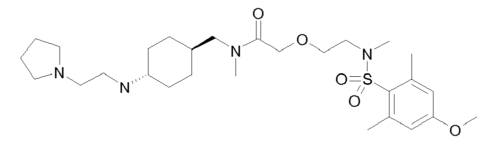 |
| (592) | 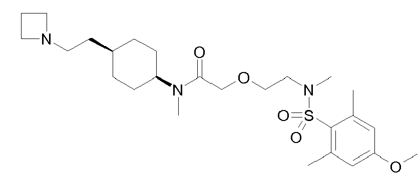 |
| (593) | 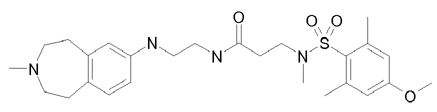 |
| (594) | 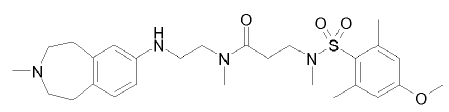 |
| (595) | 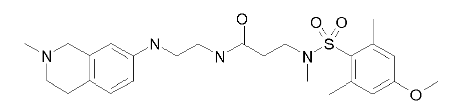 |
| (596) | 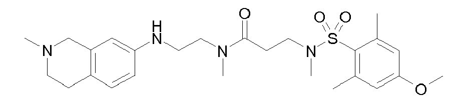 |
| (597) | 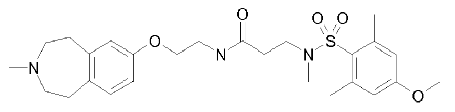 |
| (598) | 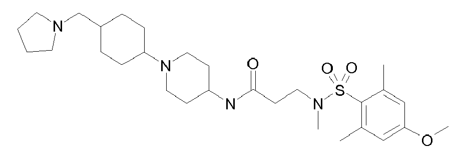 |
| (599) | 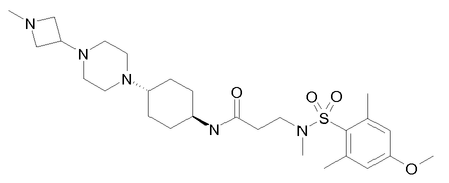 |
| (600) | 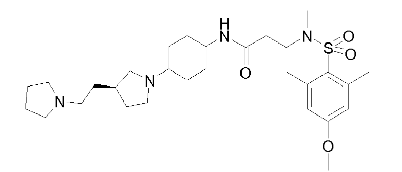 |
| (601) | 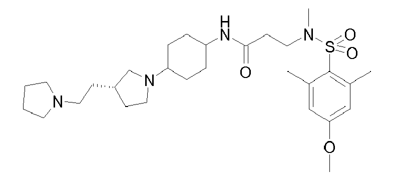 |
| (602) | 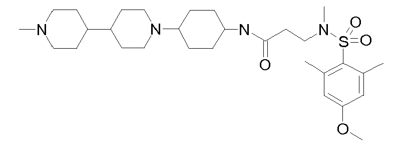 |
| (603) | 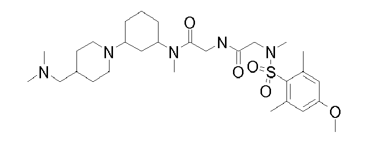 |
| (604) | 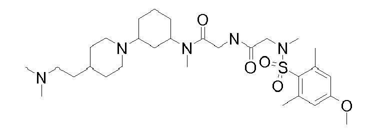 |
| (605) | 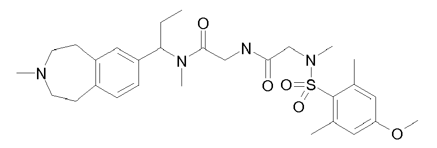 |
| (606) | 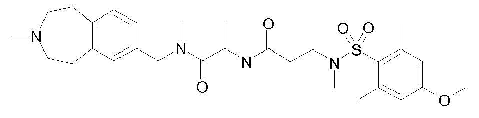 |
| (607) | 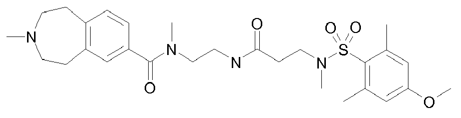 |
| (608) | 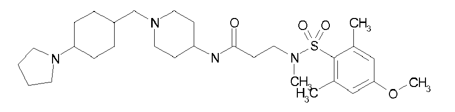 |
| (609) | 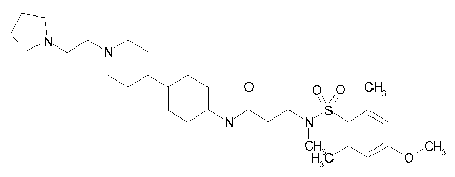 |
| (610) | 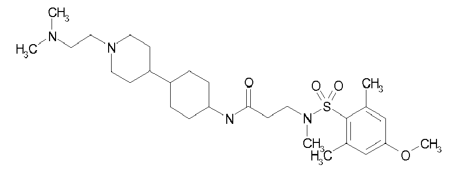 |
| (611) | 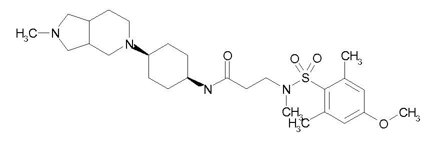 |
| (612) | 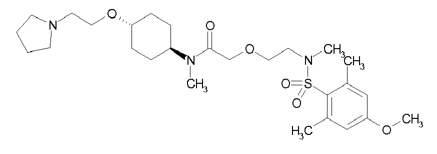 |
| (613) | 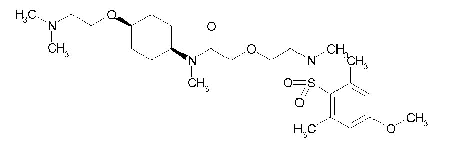 |
| (614) | 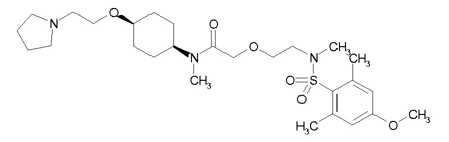 |
| (615) | 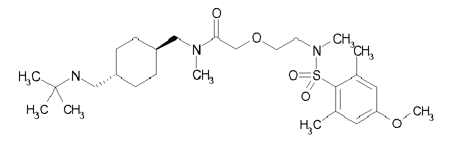 |
| (616) | 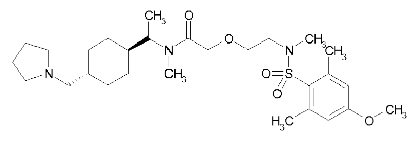 |
| (617) | 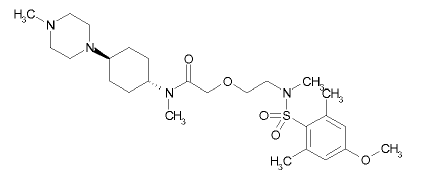 |
| (618) | 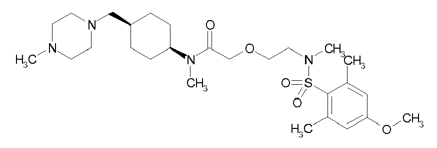 |
| (619) | 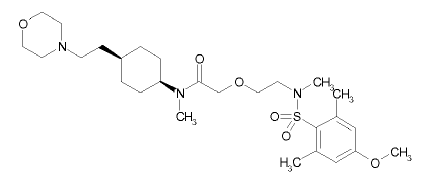 |
| (620) | 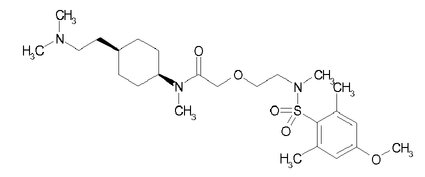 |
| (621) | 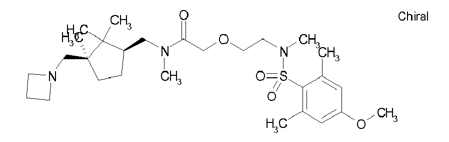 |
| (622) | 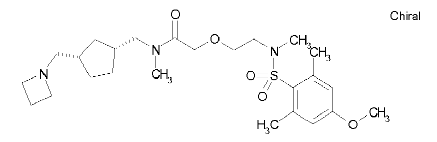 |
| (623) | 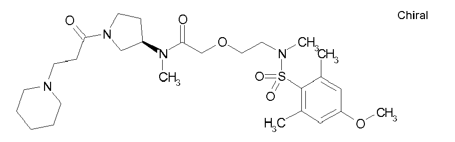 |
| (624) | 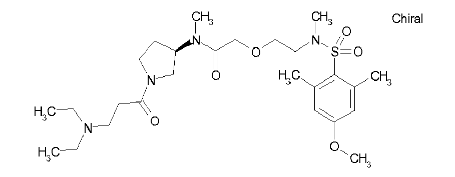 |
| (625) | 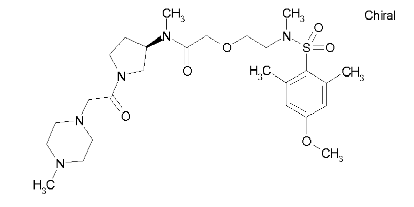 |
| (626) | 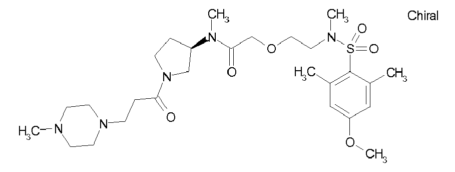 |
| (627) | 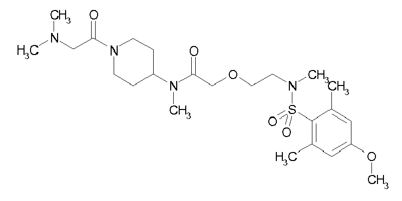 |
| (628) | 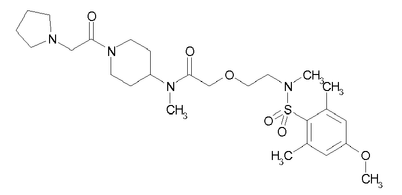 |
| (629) | 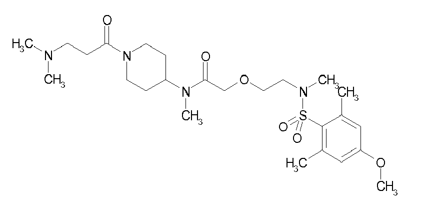 |
| (630) | 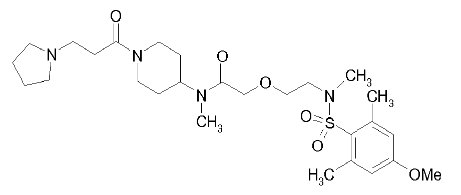 |
| (631) | 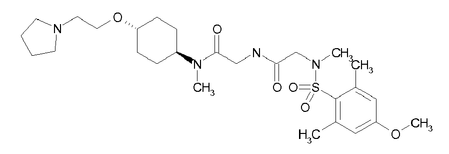 |
| (632) | 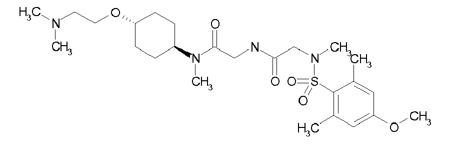 |
| (633) | 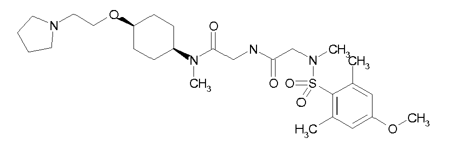 |
| (634) | 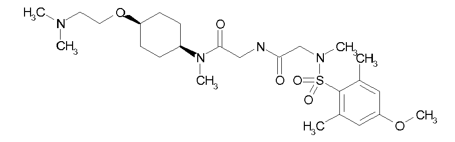 |
| (635) | 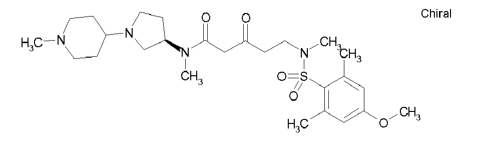 |
| (636) | 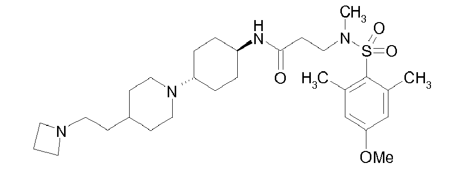 |
| (637) | 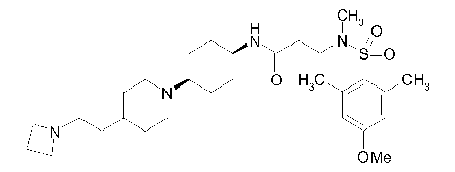 |
| (638) | 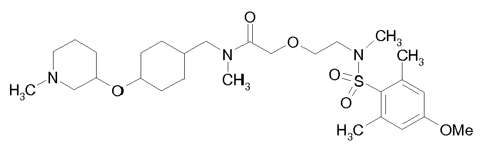 |
| (639) | 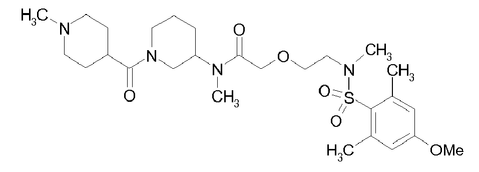 |
| (640) | 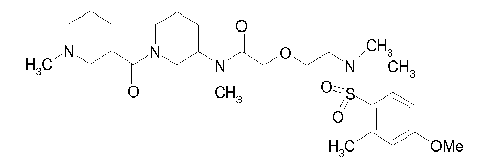 |
| (641) | 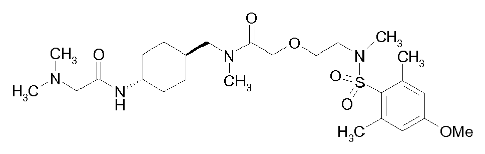 |
| (642) | 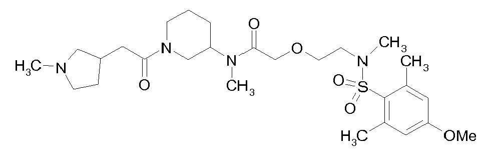 |
| (643) | 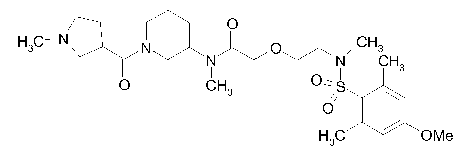 |
| (644) | 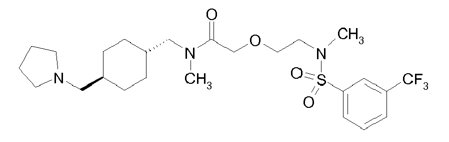 |
| (645) | 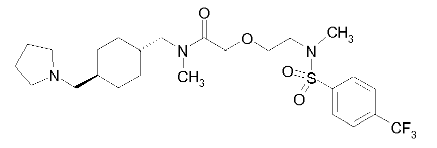 |
| (646) | 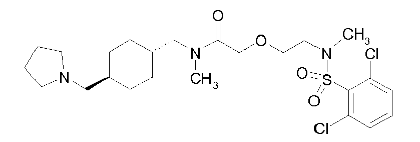 |
| (647) | 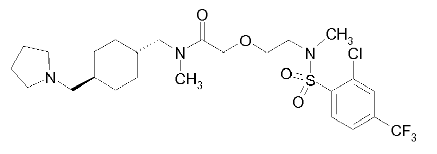 |
| (648) | 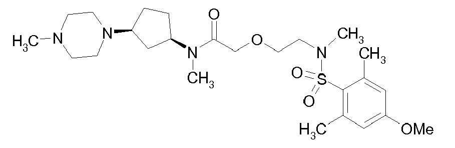 |
| (649) | 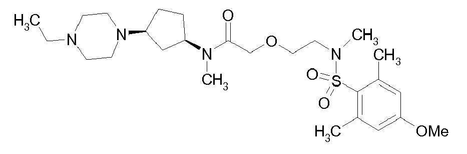 |
| (650) | 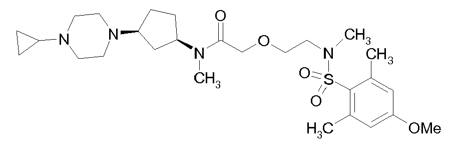 |
| (651) | 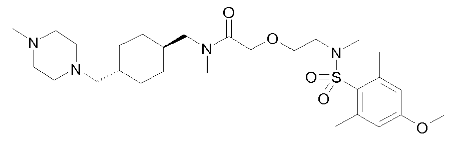 |
| (652) | 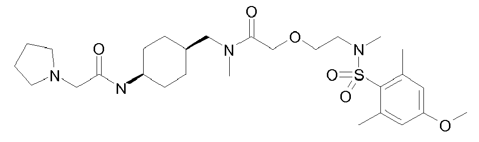 |
| (653) | 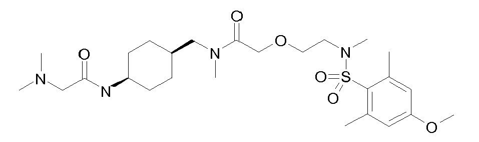 |
| (654) | 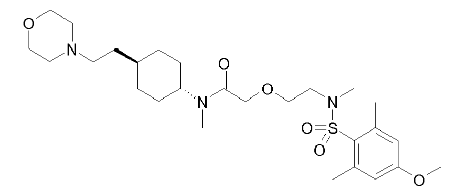 |
| (655) | 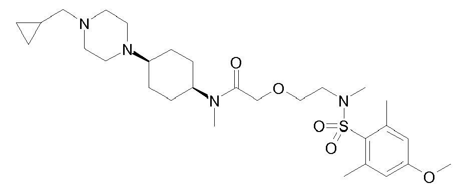 |
| (656) | 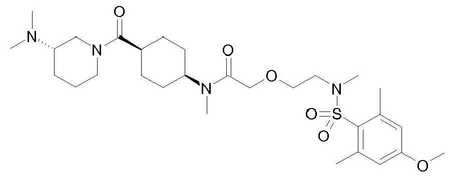 |
| (657) | 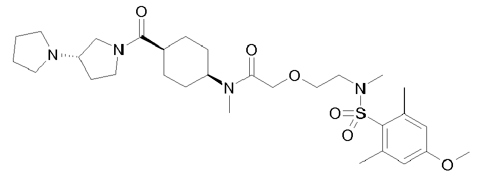 |
| (658) | 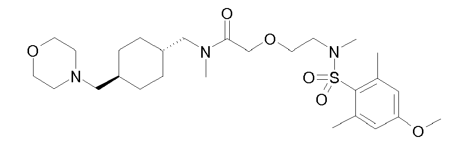 |
| (659) | 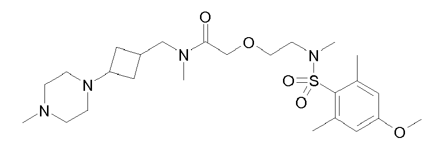 |
| (660) | 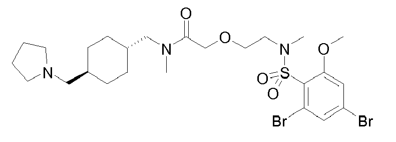 |
| (661) | 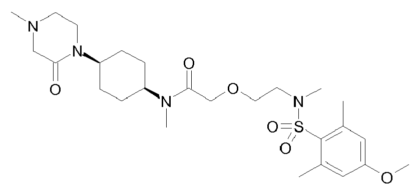 |
| (662) | 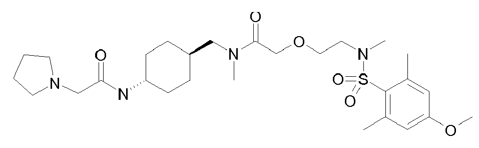 |
| (663) | 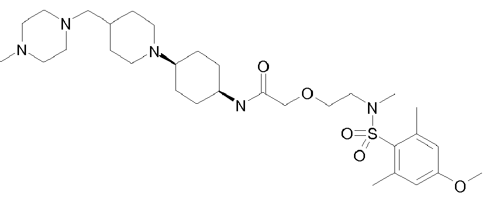 |
| (664) | 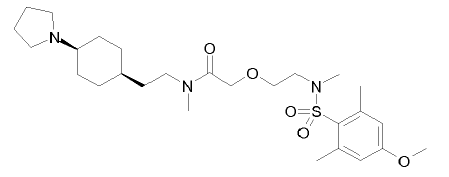 |
| (665) | 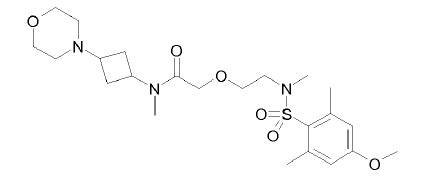 |
| (666) | 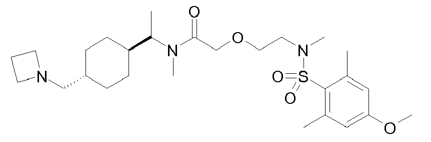 |
| (667) | 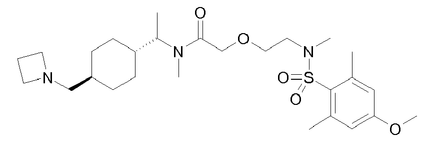 |
| (668) | 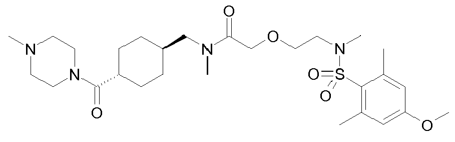 |
| (669) | 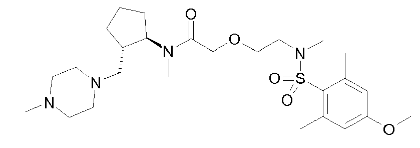 |
| (670) | 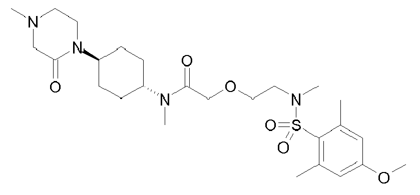 |
| (671) | 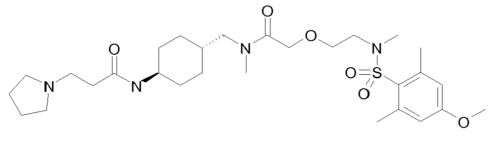 |
| (672) | 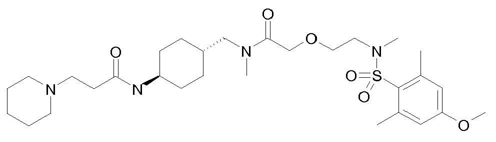 |
| (673) | 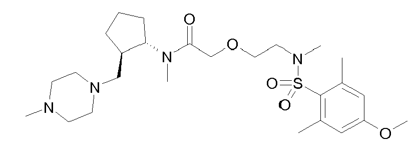 |
| (674) | 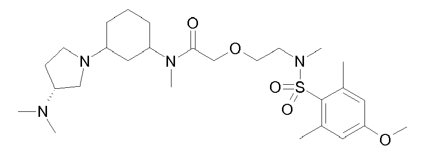 |
| (675) | 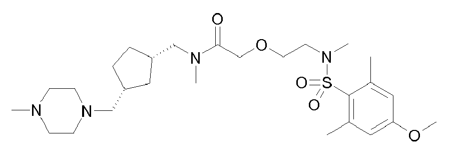 |
| (676) | 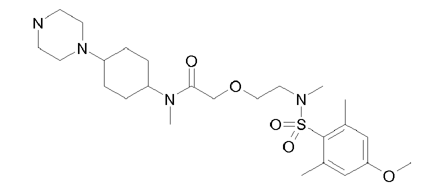 |
| (677) | 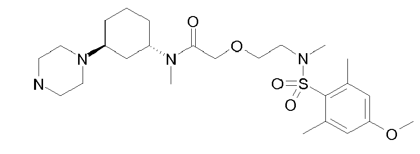 |
| (678) | 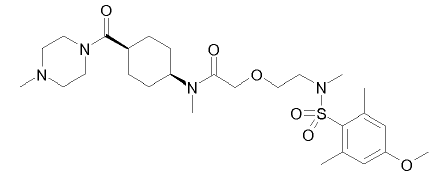 |
| (679) | 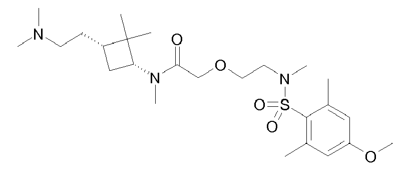 |
| (680) | 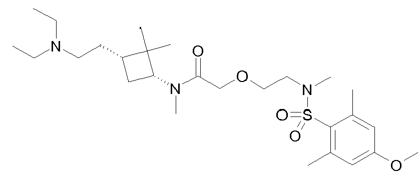 |
| (681) | 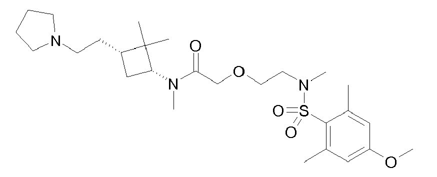 |
| (682) | 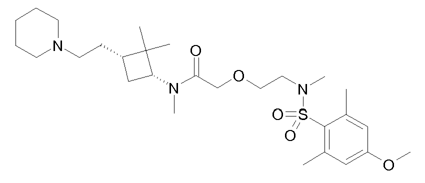 |
| (683) | 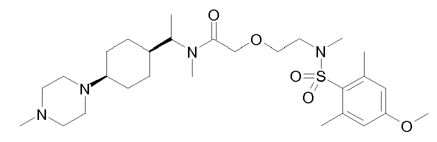 |
| (684) | 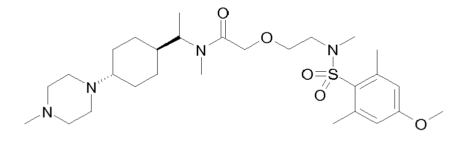 |
| (685) | 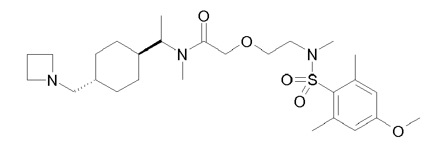 |
| (686) | 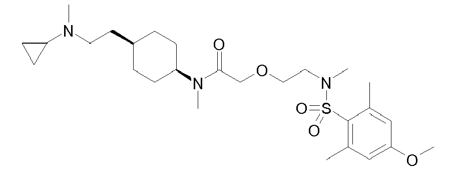 |
| (687) | 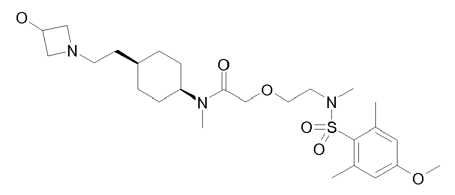 |
| (688) | 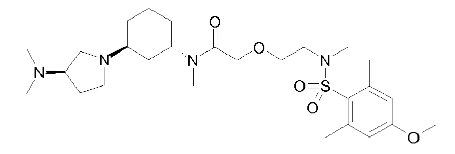 |
| (689) | 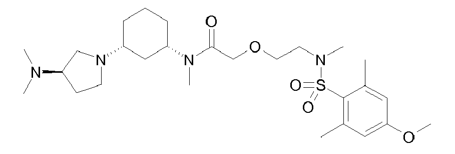 |
| (690) | 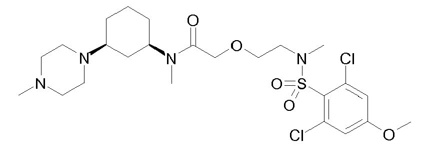 |
| (691) | 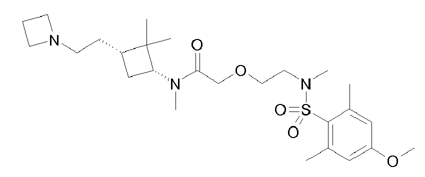 |
| (692) | 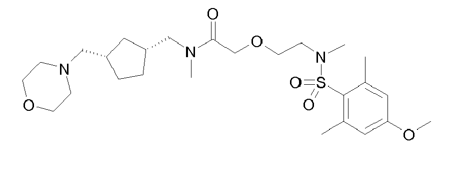 |
| (693) | 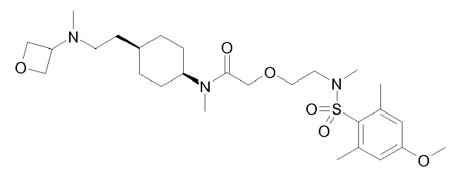 |
| (694) | 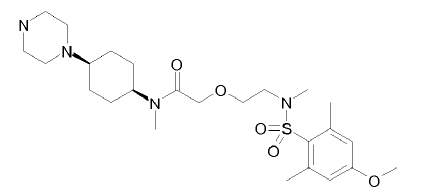 |
| (695) | 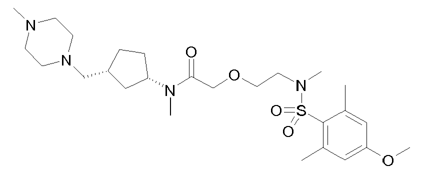 |
| (696) | 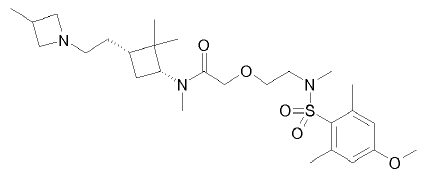 |
| (697) | 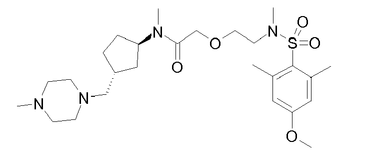 |
| (698) | 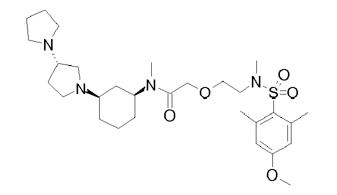 |
| (699) | 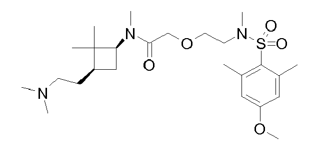 |
| (700) | 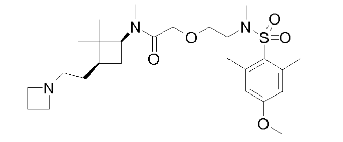 |
| (701) | 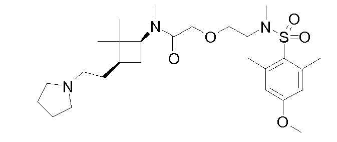 |
| (702) | 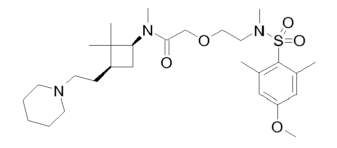 |
| (703) | 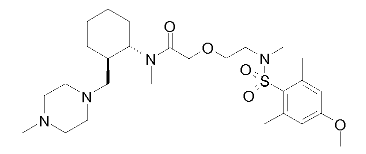 |
| (704) | 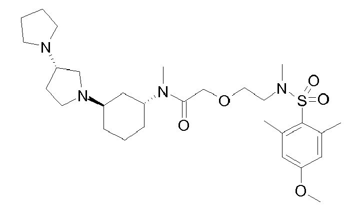 |
| (705) | 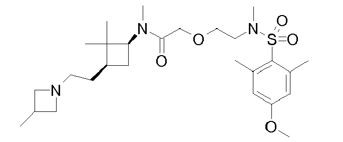 |
| (705) | 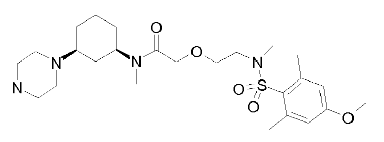 |
| (707) | 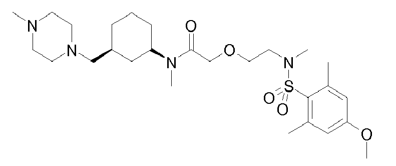 |
deren Enantiomere, deren Diastereomere, deren Gemische und deren Salze, insbesondere deren physiologisch verträgliche Salze mit organischen oder anorganischen Säuren oder Basen.

8. Physiologisch verträgliche Salze der Verbindungen nach einem der Ansprüche 1 bis 7 mit anorganischen oder organischen Säuren oder Basen.

9. Arzneimittel, enthaltend eine Verbindung nach mindestens einem der Ansprüche 1 bis 7 oder ein physiologisch verträgliches Salz gemäß Anspruch 8 neben gegebenenfalls einem oder mehreren inerten Trägerstoffen und/oder Verdünnungsmitteln.

10. Verwendung einer Verbindung nach mindestens einem der Ansprüche 1 bis 8 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

11. Verfahren zur Herstellung eines Arzneimittels gemäß Anspruch 9, **dadurch gekennzeichnet, dass** auf nichtchemischem Weg eine Verbindung nach mindestens einem der Ansprüche 1 bis 8 in einen oder mehrere inerte Trägerstoffe und/oder Verdünnungsmittel eingearbeitet wird.
